# EUROPÄISCHE PATENTSCHRIFT

(11) **EP 2 356 113 B1**
(45) Veröffentlichungstag und Bekanntmachung des Hinweises auf die Patenterteilung: **07.01.2015**
(21) Anmeldenummer: 09744351.9
(22) Anmeldetag: 31.10.2009
(51) Int. Cl.: C07D 413/04, C07D 413/14, C07D 471/08, C07D 487/08, A61K 31/4245, A61K 31/454, A61K 31/4545, A61K 31/497, A61P 35/00

(54) **HETEROCYCLISCH SUBSTITUIERTE ARYL-VERBINDUNGEN ALS HIF-INHIBITOREN**
HETEROCYCLICALLY SUBSTITUTED ARYL COMPOUNDS AS HIF INHIBITORS
COMPOSÉS ARYLES SUBSTITUÉS PAR HÉTÉROCYCLE COMME INHIBITEURS DE HIF

(30) Priorität: 14.11.2008 DE 102008057343; 11.09.2009 DE 102009041242
(43) Veröffentlichungstag der Anmeldung: 17.08.2011
(73) Patentinhaber: Bayer Intellectual Property GmbH, 40789 Monheim (DE)
(72) Erfinder: HÄRTER, Michael, 51375 Leverkusen (DE); BECK, Hartmut, 50733 Köln (DE); ELLINGHAUS, Peter, 49324 Melle (DE); BERHOERSTER, Kerstin, 45239 Essen (DE); GRESCHAT, Susanne, 49419 Wagenfeld (DE); THIERAUCH, Karl-Heinz, 14169 Berlin (DE); SÜSSMEIER, Frank, 80992 München (DE)
(74) Vertreter: BIP Patents
(86) Internationale Anmeldenummer: PCT/EP2009/007806
(87) Internationale Veröffentlichungsnummer: WO 2010/054763

(56) Entgegenhaltungen:
- WO-A2-2005/030121
- WO-A2-2007/065010

## Beschreibung

Die vorliegende Anmeldung betrifft neue heterocyclisch substituierte Aryl-Verbindungen, Verfahren zu ihrer Herstellung, diese Verbindungen zur Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von hyperproliferativen und angiogenen Erkrankungen sowie solcher Erkrankungen, die durch eine metabolische Adaptation an hypoxische Zustände entstehen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Im Jahr 2002 wurden weltweit 4,4 Millionen Menschen mit Tumorerkrankungen der Brust, des Darms, der Eierstöcke, der Lunge oder der Prostata diagnostiziert. Für das gleiche Jahr wurden ca. 2,5 Millionen Todesfälle als Folge dieser Erkrankungen angenommen (Globocan 2002 Report). In den USA allein wurden für das Jahr 2005 über 1,25 Millionen neue Fälle und über 500.000 Todesfälle aufgrund von Krebserkrankungen prognostiziert. Die Mehrzahl dieser neuen Fälle betrifft Krebserkrankungen von Darm (∼ 100.000), Lunge (∼ 170.000), Brust (∼ 210.000) und Prostata (∼ 230.000). Es wird von einer weiteren Zunahme der Krebserkrankungen von ca. 15% über die nächsten 10 Jahre ausgegangen (American Cancer Society, Cancer Facts and Figures 2005).

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Chemotherapien setzen sich häufig aus Kombinationen von zytotoxischen Arzneimitteln zusammen. Die Mehrheit dieser Substanzen haben als Wirkmechanismus eine Bindung an Tubulin, oder es handelt sich um Verbindungen, die mit der Bildung und Prozessierung von Nukleinsäuren interagieren. In neuerer Zeit zählen dazu auch Enzym-Inhibitoren, die mit der epigenetischen DNA-Modifikation oder der Zellzyklusprogression interferieren (z.B. Histon-Deacetylase-Inhibitoren, Aurora-Kinase-Inhibitoren). Da solche Therapien toxisch sind, setzt man in neuerer Zeit vermehrt auf gezielte Therapien, bei denen spezielle Prozesse in der Zelle blockiert werden, ohne dass eine hohe toxische Belastung erfolgt. Dazu zählen insbesondere Inhibitoren von Kinasen, welche die Phosphorylierung von Rezeptoren und Signalübertragungsmolekülen hemmen. Ein Beispiel hierfür ist Imatinib, das sehr erfolgreich zur Behandlung von chronisch-myeloischer Leukämie (CML) und gastrointestinalen stromalen Tumoren (GIST) eingesetzt wird. Weitere Beispiele sind EGFR-Kinase- und HER2-blockierende Substanzen wie Erlotinib sowie VEGFR-Kinase-Inhibitoren wie Sorafenib und Sunitinib, welche bei Nierenzellkarzinomen, Leberkarzinomen bzw. fortgeschrittenen Stadien von GIST eingesetzt werden.

Mit einem gegen VEGF gerichteten Antikörper ist es gelungen, die Lebenserwartung von Kolorektalkarzinom-Patienten zu verlängern. Bevacizumab hemmt das Blutgefäßwachstum, was der schnellen Ausdehnung eines Tumors im Wege steht, da dieser für eine kontinuierlich funktionierende Ver- und Entsorgung einen Anschluß an das Blutgefäßsystem benötigt.

Ein Stimulus für die Angiogenese ist die Hypoxie, welche bei soliden Tumoren immer wieder auftritt, da die Blutversorgung aufgrund des ungeregelten Wachstums unzureichend ist. Bei Sauerstoffarmut stellen die Zellen ihren Stoffwechsel von der oxidativen Phosphorylierung auf die Glykolyse um, damit der ATP-Spiegel in der Zelle stabilisiert wird. Dieser Prozess wird durch einen Transkriptionsfaktor gesteuert, der abhängig vom Sauerstoffgehalt in der Zelle hochreguliert wird. Dieser "Hypoxie-induzierter Faktor" (HIF) genannte Transkriptionsfaktor wird normalerweise post-translational durch einen schnellen Abbau entfernt und am Transport in den Zellkern gehindert. Dies geschieht durch die Hydroxylierung zweier Prolin-Einheiten in der sauerstoffabbaubaren Domäne (ODD) und einer Asparagin-Einheit in der Nähe des C-Terminus durch die Enzyme Prolyl-Dehydrogenase und FIH ("factor inhibiting HIF"). Nach der Modifikation der Prolin-Einheiten kann HIF vermittels des Hippel-Lindau-Proteins (Teil eines Ubiquitin-E3-Ligase-Komplexes) über den Proteasomenapparat abgebaut werden (Maxwell, Wiesener *et al.,* 1999). Bei Sauerstoffmangel unterbleibt der Abbau, das Protein wird hochreguliert und führt zur Transkription bzw. zur Blockade der Transkription zahlreicher (mehr als 100) anderer Proteine (Semenza und Wang, 1992; Wang und Semenza, 1995).

Der Transkriptionsfaktor HIF wird durch die regulierte α- und eine konstitutiv vorhandene β-Untereinheit (ARNT, aryl hydrocarbon receptor nuclear translocator) gebildet. Von der α-Untereinheit gibt es drei verschiedene Spezies 1α, 2α und 3α, wobei die letzte eher als Suppressor anzunehmen ist (Makino, Cao *et al.,* 2001). Bei den HIF-Untereinheiten handelt es sich um bHLH (basic helix loop helix)-Proteine, die über ihre HLH- und PAS (Per-Arnt-Sim)-Domäne dimerisieren, was ihre Transaktivierungsaktivität startet (Jiang, Rue *et al.,* 1996).

In den wichtigsten Tumorentitäten wird die Überexpression des HIF1α-Proteins mit zunehmender Blutgefäßdichte und verstärkter VEGF-Expression korreliert (Hirota und Semenza, 2006). Gleichzeitig wird der Glukosestoffwechsel hin zur Glykolyse verändert, und der Krebs-Zyklus wird zugunsten der Produktion von Zellbausteinen reduziert. Dies impliziert auch eine Änderung des Fettstoffwechsels. Solche Änderungen scheinen das Überleben der Tumore zu gewährleisten. Wird nun andererseits die Aktivität von HIF gehemmt, so könnte man folglich die Entwicklung von Tumoren unterdrücken. Dies wurde bereits in verschiedenen experimentellen Modellen beobachtet (Chen, Zhao *et al.,* 2003; Stoeltzing, McCarty *et al.,* 2004; Li, Lin *et al.,* 2005; Mizukami, Jo *et al.,* 2005; Li, Shi *et al.,* 2006). Spezifische Inhibitoren des von HIF gesteuerten Metabolismus sollten sich daher als Tumortherapeutika eignen.

Aufgabe der vorliegenden Erfindung war somit die Bereitstellung neuer Verbindungen, welche als Inhibitoren der transaktivierenden Wirkung des Transkriptionsfaktors HIF agieren und als solche zur Behandlung und/oder Prävention von Erkrankungen, insbesondere von hyperproliferativen und angiogenen Erkrankungen wie Krebserkrankungen, eingesetzt werden können.

Substituierte multicyclische Heteroaryl-Verbindungen mit Pyrrol-, Pyrazol- und/oder Oxadiazol-Partialstrukturen sowie die Verwendung dieser Verbindungen zur Behandlung verschiedenartiger Erkrankungen sind in zahlreicher Form in der Patentliteratur beschrieben, so unter anderem in EP 0 908 456-A1, WO 97/36881-A1, WO 01/12627-A1, WO 01/85723-A1, WO 02/100826-A2, WO 2004/014370-A2, WO 2004/014881-A2, WO 2004/014902-A2, WO 2004/035566-A1, WO 2004/058176-A2, WO 2004/089303-A2, WO 2004/089308-A2, WO 2005/070925-A1, WO 2006/114313-A1, WO 2007/002559-A1, WO 2007/034279-A2, WO 2008/004096-A1, WO 2008/024390-A2 und WO 2008/114157-A1. In WO 2005/030121-A2 und WO 2007/065010-A2 wird die Verwendung bestimmter Pyrazol-Derivate zur Inhibition der Expression von HIF und HIF-regulierten Genen in Tumorzellen beansprucht. In WO 2008/141731-A2 werden Heteroaryl-substituierte *N*-Benzylpyrazole als Inhibitoren des HIF-Regulationsweges zur Behandlung von Krebserkrankungen beschrieben. Heteroaryl-substituierte 5-(1*H*-Pyrazol-3-yl)-1,2,4-oxadiazole als Cannabinoid-Rezeptor-Modulatoren für die Behandlung verschiedenartiger Erkrankungen werden in US 2008/0255211-A1 offenbart. Weitere Diaryl-substituierte Isoxazol- und 1,2,4-Oxadiazol-Derivate werden in WO 2009/029632-A1 als Inhibitoren der Monoamin-Oxidase B zur Behandlung psychiatrischer Erkrankungen beschrieben.

Gegenstand der vorliegenden Erfindung sind Verbindungen der allgemeinen Formel (I) 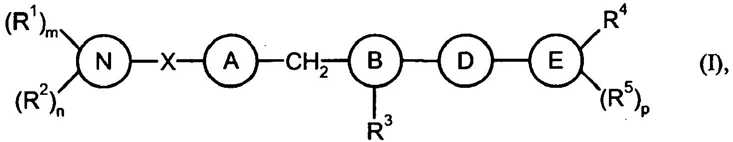 in welcher
der Ring 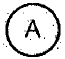 für einen Phenyl- oder Pyridyl-Ring steht, der Ringe 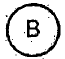 mit dem Substituenten R³ für einen Heteroaryl-Ring der Formel 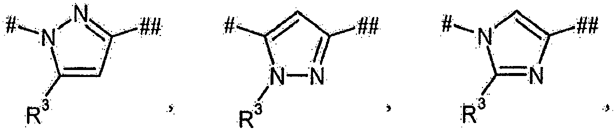 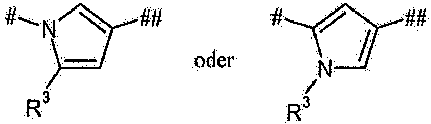 steht, worin
- #: die Verknüpfungsstelle mit der angrenzenden CH₂-Gruppe
und
- ##: die Verknüpfungsstelle mit dem Ring bezeichnen, 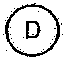 bezeichnen,
der Ring 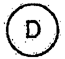 für einen Heteroaryl-Ring der Formel 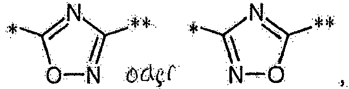 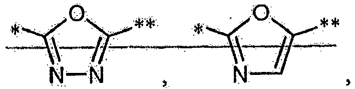 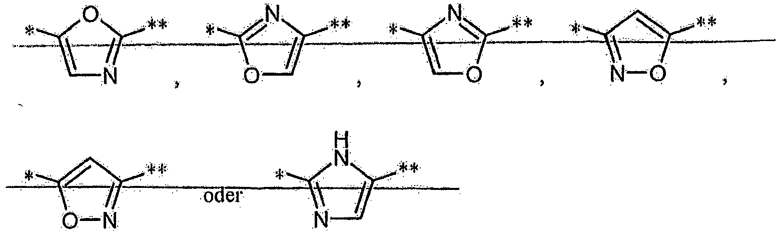 steht, worin
- *: die Verknüpfungsstelle mit dem Ring 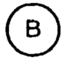 und
- **: die Verknüpfungsstelle mit dem Ring 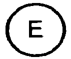 bezeichnen,
der Ring 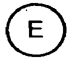 für einen Pheriyl- oder Pyridyl-Ring steht,
der Ring 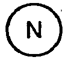 für einen gesättigten 4- bis 10-gliedrigen Aza-Heterocyclus steht, der mindestens ein N-Atom als Ringglied enthält und darüber hinaus ein oder zwei weitere Hetero-Ringglieder aus der Reihe N, O, S und/oder S(O)₂ enthalten kann,
- X: für eine Bindung oder für ◆-(CH₂)_{q}-N(R⁶)-◆◆, ◆-N(R⁶)-(CH₂)_{q}-◆◆, -O-, -S-, -C(=O)-, -S(=O)₂-, ◆-C(=O)-N(R⁶)-◆◆ oder ◆-N(R⁶)-C(=O)-◆◆ steht, worin
- ◆: die Verknüpfungsstelle mit dem Ring 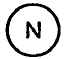 und
- ◆◆: die Verknüpfungsstelle mit dem Ring 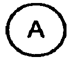 bezeichnen, q die Zahl 0, 1 oder 2 bedeutet
und
- R⁶: Wasserstoff, (C₁-C₆)-Alkyl oder (C₃-C₆)-Cycloalkyl bedeutet, wobei (C₁-C₆)-Alkyl und (C₃-C₆)-Cycloalkyl jeweils mit Hydroxy oder (C₁-C₄)-Alkoxy substituiert sein können,
- R¹: für einen an ein Kohlenstoffatom des Ringes Ⓝ gebundenen Substituenten ausgewählt aus der Reihe Fluor, Cyano, (C₁-C₆)-Alkyl, Hydroxy, (C₁-C₆)-Alkoxy, Oxo, Amino, Mono-(C₁-C₆)-alkylamino, Di-(C₁-C₆)-alkylamino und (C₃-C₆)-Cycloalkyl steht, wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann und (C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1, 2, 3 oder 4 steht, wobei im Fall, dass der Substituent R¹ mehrfach auftritt, seine Bedeutungen gleich oder verschieden sein können,
- R²: für einen an ein Stickstoffatom des Ringes Ⓝ gebundenen Substituenten ausgewählt aus der Reihe (C₁-C₆)-Alkyl, (C₁-C₆)-Alkylcarbonyl, (C₁-C₆)-Alkoxycarbonyl, (C₁-C₆)-Alkylsulfonyl und (C₃-C₆)-Cycloalkyl steht, wobei die Alkyl-Gruppe in (C₁-C₆)-Alkyl, (C₁-C₆)-Alkylcarbonyl, (C₁-C₆)-Alkoxycarbonyl und (C₁-C₆)-Alkylsulfonyl ihrerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cycloalkyl und 4- bis 6-gliedriges Heterocyclyl substituiert sein kann und (C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- n: für die Zahl 0 oder 1 oder auch, sofern der Aza-Heterocyclus Ⓝ weitere N-Atome als Ringglieder enthält, die Zahl 2 steht, wobei im Fall, dass der Substituent R² zweifach auftritt, seine Bedeutungen gleich oder verschieden sein können,
- R³: für Methyl, Ethyl oder Trifluormethyl steht,
- R⁴: für Wasserstoff oder einen Substituenten ausgewählt aus der Reihe Halogen, Cyano, Pentafluorthio, (C₁-C₆)-Alkyl, Tri-(C₁-C₄)-alkylsilyl, -OR⁷, -NR⁷R⁸, -N(R⁷)-C(=O)-R⁸, -N(R⁷)-C(=O)-OR⁷, -N(R⁷)-S(=O)₂-R⁸, -C(=O)-OR⁷, -C(=O)-NR⁷R⁸, -SR⁷, -S(=O)-R⁷, -S(=O)₂-R⁷, -S(=O)₂-NR⁷R⁸, -S(=O)(=NH)-R⁷, -S(=O)(=NCH₃)-R⁷, (C₃-C₆)-Cycloalkyl, 4- bis 6-gliedriges Heterocyclyl und 5- oder 6-gliedriges Heteroaryl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -N(R⁷)-C(=O)-OR⁸, -C(=O)-OR⁷, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo, Amino, Mono-(C₁-C₄)-alkylamino, Di-(C₁-C₄)-alkylamino, (C₁-C₄)-Alkylcarbonylamino, (C₁-C₄)-Alkoxycarbonylamino, (C₁-C₄)-Alkylcarbonyl und (C₁-C₄)-Alkoxycarbonyl
sowie
die genannten Heteroaryl-Gruppen ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy
substituiert sein können, wobei die hierin genannten (C₁-C₄)-Alkyl-Substituenten ihrerseits mit Hydroxy, (C₁-C₄)-Alkoxy, Trifluormethoxy, (C₁-C₄)-Alkylcarbonyloxy, Aminocarbonyl, Mono-(C₁-C₄)-alkylaminocarbonyl oder Di-(C₁-C₄)-alkylaminocarbonyl oder bis zu dreifach mit Fluor substituiert sein können,
und worin
- R⁷ und R⁸: unabhängig voneinander bei jedem einzelnen Auftreten Wasserstoff, (C₁-C₆)-Alkyl, (C₃-C₆)-Cycloalkyl oder 4- bis 6-gliedriges Heterocyclyl bedeuten, wobei (C₁-C₆)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, Amino, Mono-(C₁-C₄)-alkylamino, Di-(C₁-C₄)-alkylamino, (C₁-C₄)-Alkoxycarbonyl, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, (C₁-C₄)-Alkylcarbonyl und (C₁-C₄)-Alkoxycarbonyl substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino, (C₁-C₄)-Alkylcarbonyl und (C₁-C₄)-Alkoxycarbonyl substituiert sein kann,
- R⁵: für einen Substituenten ausgewählt aus der Reihe Fluor, Chlor, Cyano, Methyl, Trifluormethyl und Hydroxy stehtund
- p: für die Zahl 0, 1 oder 2 steht,
wobei im Fall, dass der Substituent R⁵ zweifach auftritt, seine Bedeutungen gleich oder verschieden sein können,
sowie ihre Salze, Solvate und Solvate der Salze.

Eine alternative Ausführungsform innerhalb des zuvor beschriebenen Gegenstands der Erfindung umfasst Verbindungen der Formel (I), in welcher
- R¹: für einen an ein Kohlenstoffatom des Ringes 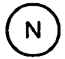 gebundenen Substituenten ausgewählt aus der Reihe Fluor, (C₁-C₆)-Alkyl, Hydroxy, (C₁-C₆)-Alkoxy, Oxo, Amino, Mono-(C₁-C₆)-alkylamino, Di-(C₁-C₆)-alkylamino und (C₃-C₆)-Cycloalkyl steht, wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1, 2, 3 oder 4 steht, wobei im Fall, dass der Substituent R¹ mehrfach auftritt, seine Bedeutungen gleich oder verschieden sein können, und
- R⁴: für Wasserstoff oder einen Substituenten ausgewählt aus der Reihe Halogen, Cyano, Pentafluorthio, (C₁-C₆)-Alkyl, Tri-(C₁-C₄)-alkylsilyl, -OR⁷, -NR⁷R⁸, -N(R⁷)-C(=O)-R⁸, -N(R⁷)-C(=O)-OR⁸, -N(R⁷)-S(=O)₂-R⁸, -C(=O)-OR⁷, -C(=O)-NR⁷R⁸, -SR⁷, -S(=O)-R⁷, -S(=O)₂-R⁷, -S(=O)₂-NR⁷R⁸, (C₃-C₆)-Cycloalkyl, 4-bis 6-gliedriges Heterocyclyl und 5- oder 6-gliedriges Heteroaryl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁷, -N(R⁷)-C(=O)-OR⁸, -C(=O)-OR⁷, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo, Amino, Mono-(C₁-C₄)-alkyl-amino, Di-(C₁-C₄)-alkylamino, (C₁-C₄)-Alkylcarbonylamino, (C₁-C₄)-Alkoxycarbonyl-amino, (C₁-C₄)-Alkylcarbonyl und (C₁-C₄)-Alkoxycarbonyl
sowie
die genannten Heteroaryl-Gruppen ihrerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein können,
und worin R⁷ und R⁸ die zuvor angegebenen Bedeutungen haben,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure, Benzoesäure und 4-Sulfamoylbenzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die *N*-Oxide von in erfindungsgemäßen Verbindungen enthaltenen Pyridyl-Ringen und tertiären cyclischen Amin-Gruppierungen sind gleichfalls von der vorliegenden Erfindung umfasst.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, *n*-Pentyl, 2-Pentyl, 3-Pentyl, Neopentyl, *n*-Hexyl, 2-Hexyl und 3-Hexyl.

(C₁-C₆)-Alkylcarbonyl und (C₁-C₄)-Alkylcarbonyl stehen im Rahmen der Erfindung für einen geradkettigen oder verzweigten Alkylrest mit 1 bis 6 bzw. 1 bis 4 Kohlenstoffatomen, der über eine Carbonyl-Gruppe [-C(=O)-] verknüpft ist. Bevorzugt ist eine geradkettige oder verzweigte Alkylcarbonyl-Gruppe mit 1 bis 4 Kohlenstoffatomen im Alkylrest. Beispielhaft und vorzugsweise seien genannt: Acetyl, Propionyl, *n*-Butyryl, *iso*-Butyryl, *n*-Pentanoyl, Pivaloyl, *n*-Hexanoyl und *n-*Heptanoyl.

(C₁-C₆)-Alkylsulfonyl und (C₁-C₄)-Alkylsulfonyl stehen im Rahmen der Erfindung für einen geradkettigen oder verzweigten Alkylrest mit 1 bis 6 bzw. 1 bis 4 Kohlenstoffatomen, der über eine Sulfonyl-Gruppe [-S(=O)₂-] verknüpft ist. Bevorzugt ist eine geradkettige oder verzweigte Alkyl-sulfonyl-Gruppe mit 1 bis 4 Kohlenstoffatomen im Alkylrest. Beispielhaft und vorzugsweise seien genannt: Methylsulfonyl, Ethylsulfonyl, *n*-Propylsulfonyl, Isopropylsulfonyl, *n*-Butylsulfonyl, *tert*.-Butylsulfonyl, *n*-Pentylsulfonyl und *n*-Hexylsulfonyl.

Tri-(C₁-C₄)-alkylsilyl steht im Rahmen der Erfindung für eine Silyl-Gruppe mit drei gleichen oder verschiedenen geradkettigen oder verzweigten Alkylsubstituenten, die jeweils 1 bis 4 Kohlenstoffatome aufweisen. Beispielhaft und vorzugsweise seien genannt: Trimethylsilyl, *tert*.-Butyl-dimethylsilyl und Triisopropylsilyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *iso*-Butoxy, *sec*.-Butoxy, *tert.-*Butoxy, *n*-Pentoxy, 2-Pentoxy, 3-Pentoxy, Neopentoxy, *n*-Hexoxy, 2-Hexoxy und 3-Hexoxy.

(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonyl-Gruppe [-C(=O)-] verknüpft ist. Bevorzugt ist eine geradkettige oder verzweigte Alkoxycarbonyl-Gruppe mit 1 bis 4 Kohlenstoffatomen im Alkoxyrest. Beispielhaft und vorzugsweise seien genannt: Methoxycarbonyl, Ethoxycarbonyl, *n*-Propoxycarbonyl, Isopropoxycarbonyl, *n*-Butoxycarbonyl, *tert*.-Butoxycarbonyl, *n*-Pentoxycarbonyl und *n*-Hexoxycarbonyl.

Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.

Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, *N*-Isopropyl-*N*-methylamino, *N*-Isopropyl-*N*-*n*-propylamino, *N,N*-Diisopropylamino, *N*-*n*-Butyl-*N*-methylamino, *N*-*tert*.-Butyl-*N*-methylamino, *N*-Methyl-*N*-*n-*pentylamino und *N*-*n*-Hexyl-*N*-methylamino.

Mono- bzw. Di-(C₁-C₄)-alkylaminocarbonyl steht im Rahmen der Erfindung für eine Amino-Gruppe, die über eine Carbonyl-Gruppe [-C(=O)-] verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielhaft und vorzugsweise seien genannt:

Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N,N*-Dimethylaminocarbonyl, *N,N*-Diethylaminocarbonyl, *N*-Ethyl-*N*-methylaminocarbonyl, *N*-Methyl-*N*-*n*-propylaminocarbonyl, *N*-Isopropyl-*N*-methylaminocarbonyl, *N,N*-Diisopropylaminocarbonyl, *N*-*n*-Butyl-*N*-methylaminocarbonyl und *N*-tert.-Butyl-*N*-methylaminocarbonyl.

(C₁-C₄)-Alkylcarbonylamino steht im Rahmen der Erfindung für eine Amino-Gruppe mit einem geradkettigen oder verzweigten Alkylcarbonyl-Substituenten, der 1 bis 4 Kohlenstoffatome im Alkylrest aufweist und über die-Carbonylgruppe mit dem N-Atom verknüpft ist. Beispielhaft und vorzugsweise seien genannt: Acetylamino, Propionylamino, *n*-Butyrylamino, *iso*-Butyrylamino, *n-*Pentanoylamino und Pivaloylamino.

(C₁-C₄)-Alkylcarbonyloxy steht im Rahmen der Erfindung für einen Oxyrest mit einem geradkettigen oder verzweigten Alkylcarbonyl-Substituenten, der 1 bis 4 Kohlenstoffatome im Alkylrest aufweist und über die Carbonylgruppe mit dem O-Atom verknüpft ist. Beispielhaft und vorzugsweise seien genannt: Acetoxy, Propionoxy, *n*-Butyroxy, *iso*-Butyroxy, *n*-Pentanoyloxy und Pivaloyloxy.

(C₁-C₄)-Alkoxycarbonylamino steht im Rahmen der Erfindung für eine Amino-Gruppe mit einem geradkettigen oder verzweigten Alkoxycarbonyl-Substituenten, der 1 bis 4 Kohlenstoffatome im Alkoxyrest aufweist und über die Carbonylgruppe mit dem N-Atom verknüpft ist. Beispielhaft und vorzugsweise seien genannt: Methoxycarbonylamino, Ethoxycarbonylamino, *n*-Propoxycarbonylamino, Isopropoxycarbonylamino, *n*-Butoxycarbonylamino und *tert*.-Butoxycarbonylamino.

(C₁-C₆)-Cycloalkyl und (C₃-C₅)-Cycloalkyl stehen im Rahmen der Erfindung für eine monocyclische, gesättigte Cycloalkylgruppe mit 3 bis 6 bzw. 3 bis 5 Ring-Kohlenstoffatomen. Beispielhaft und vorzugsweise seien genannt: Cyclopropyl, Cyclobutyl, Cyclopentyl und Cyclohexyl.

4- bis 6-gliedriges Heterocyclyl und 4- oder 5-gliedriges Heterocyclyl stehen im Rahmen der Erfindung für einen monocyclischen, gesättigten Heterocyclus mit insgesamt 4 bis 6 bzw. 4 oder 5 Ringatomen, welcher ein oder zwei Ring-Heteroatome aus der Reihe N, O, S und/oder S(O)₂ enthält und über ein Ring-Kohlenstoffatom oder gegebenenfalls über ein Ring-Stickstoffatom verknüpft ist. Bevorzugt ist 4- oder 5-gliedriges Heterocyclyl mit einem Ring-Heteroatom aus der Reihe N, O oder S sowie 6-gliedriges Heterocyclyl mit einem oder zwei Ring-Heteroatomen aus der Reihe N, O und/oder S. Beispielhaft seien genannt: Azetidinyl, Oxetanyl, Thietanyl, Pyrrolidinyl, Pyrazolidinyl, Tetrahydrofuranyl, Thiolanyl, 1,1-Dioxidothiolanyl, 1,3-Oxazolidinyl, 1,3-Thiazolidinyl, Piperidinyl, Piperazinyl, Tetrahydropyranyl, Tetrahydrothiopyranyl, 1,3-Dioxanyl, 1,4-Dioxanyl, Morpholinyl, Thiomorpholinyl und 1,1-Dioxidothiomorpholinyl. Bevorzugt sind Azetidinyl, Oxetanyl, Pyrrolidinyl, Tetrahydrofuranyl, Piperidinyl, Piperazinyl, Tetrahydropyranyl, Morpholinyl und Thiomorpholinyl.

Ein 4- bis 10-gliedriger Aza-Heterocyclus steht im Rahmen der Erfindung für einen mono- oder gegebenenfalls bicyclischen, gesättigten Heterocyclus mit insgesamt 4 bis 10 Ringatomen, der mindestens ein Ring-Stickstoffatom enthält und darüber hinaus ein oder zwei weitere Ring-Heteroatome aus der Reihe N, O, S und/oder S(O)₂ enthalten kann und der über ein Ring-Kohlenstoffatom oder gegebenenfalls über ein Ring-Stickstoffatom verknüpft ist. Bevorzugt ist ein 4- bis 10-gliedriger Aza-Heterocyclus, der mindestens ein Ring-Stickstoffatom enthält und darüber hinaus ein weiteres Ring-Heteroatom aus der Reihe N, O, S oder S(O)₂ enthalten kann. Beispielhaft und vorzugsweise seien genannt: Azetidinyl, Pyrrolidinyl, Pyrazolidinyl, 1,3-Oxazolidinyl, 1,3-Thiazolidinyl, Piperidinyl, Piperazinyl, Morpholinyl, Thiomorpholinyl, 1,1-Dioxidothiomorpholinyl, Hexahydroazepinyl, Hexahydro-1,4-diazepinyl, Octahydroazocinyl, Octahydropyrrolo[3,4-b]-pyrrolyl, Octahydroisoindolyl, Octahydropyrrolo[3,2-b]pyridyl, Octahydropyrrolo[3,4-b]pyridyl, Octahydropyrrolo[3,4-c]pyridyl, Octahydropyrrolo[1,2-a]pyrazinyl, Decahydroisochinolinyl, Octa-hydropyrido[1,2-a]pyrazinyl, 7-Azabicyclo[2.2.1]heptyl, 3-Azabicyclo[3.2.0]heptyl, 2-Oxa-6-aza-spiro[3.3]heptyl, 3-Azabicyclo[3.2.1]octyl, 8-Azabicyclo[3.2.1]octyl, 8-Oxa-3-azabicyclo[3.2.1]-octyl und 9-Azabicyclo[3.3.1]nonyl.

5- oder 6-gliedriges Heteroaryl steht im Rahmen der Erfindung für einen aromatischen Heterocyclus (Heteroaromaten) mit insgesamt 5 bzw. 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind 5- oder 6-gliedrige Heteroaryl-Reste mit bis zu zwei Ring-Heteroatomen aus der Reihe N, O und/oder S, wie beispielsweise Furyl, Pyrrolyl, Thienyl, Thiazolyl, Oxazolyl, Isothiazolyl, Isoxazolyl, Pyrazolyl, Imidazolyl, Pyridyl, Pyrimidinyl, Pyridazinyl und Pyrazinyl.

Halogen schließt im Rahmen der Erfindung Fluor, Chlor, Brom und Iod ein. Bevorzugt sind Chlor, Fluor oder Brom, besonders bevorzugt Fluor oder Chlor.

Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder mit zwei oder drei gleichen oder verschiedenen Substituenten ist bevorzugt. Besonders bevorzugt ist die Substitution mit einem oder mit zwei gleichen oder verschiedenen Substituenten.

Gegenstand der vorliegenden Erfindung sind insbesondere solche Verbindungen der allgemeinen Formel (I), in denen
der Ring 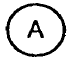 für einen Phenyl- oder Pyridyl-Ring steht und die angrenzenden Gruppen X und CH₂ in 1,3- oder 1,4-Relation zueinander an Ring-Kohlenstoffatome von 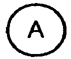 gebunden sind
und
der Ring 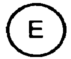 mit den Substituenten R⁴ und R⁵ für einen Phenyl-Ring der Formel 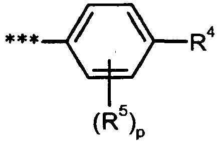 steht, worin
- ***: die Verknüpfungsstelle mit dem Ring 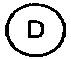 bezeichnet,
sowie ihre Salze, Solvate und Solvate der Salze.

Bevorzugt im Rahmen der vorliegenden Erfindung sind Verbindungen der Formel (I), in welcher der Ring 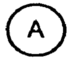 für einen Pyridyl-Ring steht und die angrenzenden Gruppen X und CH₂ in 1,3-oder 1,4-Relation zueinander an Ring-Kohlenstoffatome dieses Pyridyl-Rings gebunden sind
und
der Ring 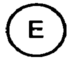 mit den Substituenten R⁴ und R⁵ für einen Phenyl-Ring der Formel 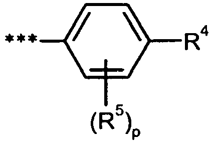 steht, worin
- ***: die Verknüpfungsstelle mit dem Ring 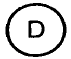 bezeichnet,
sowie ihre Salze, Solvate und Solvate der Salze.

Bevorzugt sind auch Verbindungen der Formel (I), in welcher der Ring 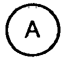 für einen Phenyl-Ring steht und die angrenzenden Gruppen X und CH₂ in 1,3-oder 1,4-Relation zueinander an diesen Phenyl-Ring gebunden sind,
der Ring 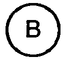 mit dem Substituenten R³ für einen Heteroaryl-Ring der Formel 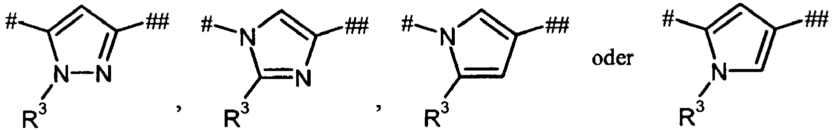 steht, worin
- #: die Verknüpfungsstelle mit der angrenzenden CH₂-Gruppe
und
- ##: die Verknüpfungsstelle mit dem Ring 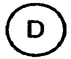 bezeichnen,
und
der Ring 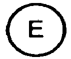 mit den Substituenten R⁴ und R⁵ für einen Phenyl-Ring der Formel 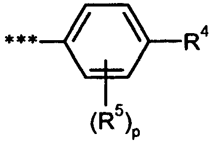 steht, worin
- ***: die Verknüpfungsstelle mit dem Ring 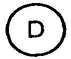 bezeichnet,
sowie ihre Salze, Solvate und Solvate der Salze.

Gleichfalls bevorzugt sind Verbindungen der Formel (I), in welcher der Ring 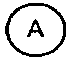 für einen Phenyl-Ring steht und die angrenzenden Gruppen X und CH₂ in 1,3-oder 1,4-Relation zueinander an diesen Phenyl-Ring gebunden sind, der Ring 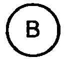 mit dem Substituenten R³ für einen Heteroaryl-Ring der Formel 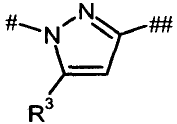 steht, worin
- #: die Verknüpfungsstelle mit der angrenzenden CH₂-Gruppe
und
- ##: die Verknüpfungsstelle mit dem Ring 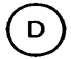 bezeichnen,
der Ring 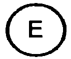 mit den Substituenten R⁴ und R⁵ für einen Phenyl-Ring der Formel 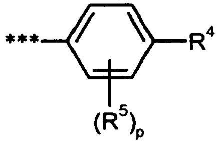 steht, worin
- ***: die Verknüpfungsstelle mit dem Ring 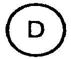 bezeichnet,
- R¹: für einen an ein Kohlenstoffatom des Ringes 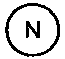 gebundenen Substituenten ausgewählt aus der Reihe Cyano, (C₁-C₆)-Alkyl, Oxo und (C₃-C₆)-Cycloalkyl steht, wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann und (C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- R²: für einen an ein Stickstoffatom des Ringes Ⓝ gebundenen Substituenten ausgewählt aus der Reihe (C₁-C₆)-Alkyl, (C₁-C₆)-Alkylcarbonyl, (C₁-C₆)-Alkoxycarbonyl, (C₁-C₆)-Alkylsulfonyl und (C₃-C₆)-Cycloalkyl steht,
wobei die Alkyl-Gruppe in (C₁-C₆)-Alkyl, (C₁-C₆)-Alkylcarbonyl, (C₁-C₆)-Alkoxycarbonyl und (C₁-C₆)-Alkylsulfonyl ihrerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cycloalkyl und 4- bis 6-gliedriges Heterocyclyl substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1, 2, 3 oder 4 steht, wobei im Fall, dass der Substituent R¹ mehrfach auftritt, seine Bedeutungen gleich oder verschieden sein können,
und
- n: für die Zahl 0 oder 1 oder auch, sofern der Aza-Heterocyclus 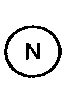 weitere N-Atome als Ringglieder enthält, die Zahl 2 steht,
wobei im Fall, dass der Substituent R² zweifach auftritt, seine Bedeutungen gleich oder verschieden sein können,
wobei die Summe aus m und n ungleich der Zahl 0 ist,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 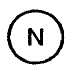 gebundenen Substituenten ausgewählt aus der Reihe (C₁-C₆)-Alkyl, Oxo und (C₃-C₆)-Cycloalkyl steht, wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1, 2, 3 oder 4 steht, wobei im Fall, dass der Substituent R¹ mehrfach auftritt, seine Bedeutungen gleich oder verschieden sein können, und
- n: für die Zahl 0 oder 1 oder auch, sofern der Aza-Heterocyclus 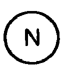 weitere N-Atome als Ringglieder enthält, die Zahl 2 steht,
wobei im Fall, dass der Substituent R² zweifach auftritt, seine Bedeutungen gleich oder verschieden sein können,
wobei die Summe aus m und n ungleich der Zahl 0 ist,
sowie ihre Salze, Solvate und Solvate der Salze.

Besonders bevorzugt im Rahmen der vorliegenden Erfindung sind Verbindungen der Formel (I), in welcher der Ring 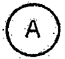 für einen Pyridyl-Ring steht und die angrenzenden Gruppen X und CH₂ in 1,3-oder 1,4-Relation zueinander an Ring-Kohtenstoffatome dieses Pyridyl-Rings gebunden sind,
der Ring 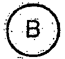 mit dem Substituenten R³ für einen Heteroaryl-Ring der Formel 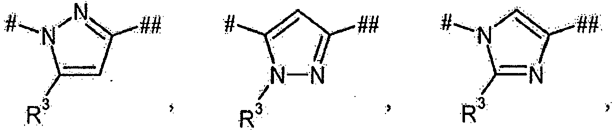 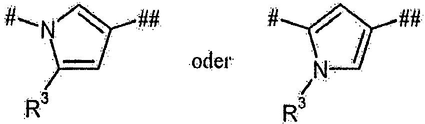 steht, worin
- #: die Verknüpfungsstelle mit der angrenzenden CH₂-Gruppe
und
- ##: die Verknüpfungsstelle mit dem Ring 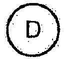 bezeichnen,
der Ring 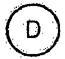 für einen Heteroaryl-Ring der Formel 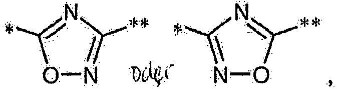 steht, worin
- *: die Verknüpfungsstelle mit dem Ring 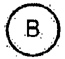 und
- **: die Verknüpfungsstelle mit dem Ring 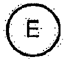 bezeichnen,
der Ring 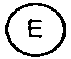 mit den Substituenten R⁴ und R⁵ für einen Phenyl-Ring der Formel 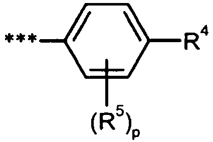 steht, worin
- ***: die Verknüpfungsstelle mit dem Ring 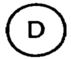 bezeichnet,
der Ring 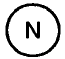 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -O-, -S-, -C(=O)-, -S(=O)₂- oder ◆-N(R⁶)-C(=O)-◆◆ steht, worin
◆ die Verknüpfungsstelle mit dem Ring 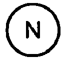 und
◆◆ die Verknüpfungsstelle mit dem Ring 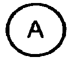 bezeichnen,
q die Zahl 0, 1 oder 2 bedeutet
und
R⁶ Wasserstoff, (C₁-C₄)-Alkyl oder (C₃-C₆)-Cycloalkyl bedeutet,
- R¹: für einen an ein Kohlenstoffatom des Ringes 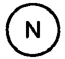 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht,
wobei im Fall, dass der Substituent R¹ zweifach auftritt, seine Bedeutungen gleich oder verschieden sein können,
- R²: für einen an ein Stickstoffatom des Ringes 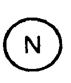 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl und (C₃-C₆)-Cycloalkyl steht, wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cyclo-alkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- n: für die Zahl 0 oder 1 steht,
- R³: für Methyl, Ethyl oder Trifluormethyl steht,
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, -S(=O)(=NH)-R⁷ -S(=O)(=NCH₃)-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann und wobei die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy
substituiert sein können,
wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können,
und worin
- R⁷ und R⁸: unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten,
wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
- R⁵: für einen Substituenten ausgewählt aus der Reihe Fluor, Chlor und Methyl steht
und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 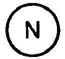 gebundenen Substituenten ausgewählt aus der Reihe Fluor, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht, wobei im Fall, dass der Substituent R¹ zweifach auftritt, seine Bedeutungen gleich oder verschieden sein können,
und
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, (C₃-C₆)-Cycloalkyl und 4- bis 6-gliedriges Heterocyclyl steht, wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl
sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein können,
und worin R⁷ und R⁸ die in dieser zuletzt beschriebenen Ausführungsform genannten Bedeutungen haben, sowie ihre Salze, Solvate und Solvate der Salze.

Besonders bevorzugt sind auch Verbindungen der Formel (I), in welcher der Ring 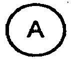 für einen Phenyl-Ring steht und die angrenzenden Gruppen X und CH₂ in 1,3-oder 1,4-Relation zueinander an diesen Phenyl-Ring gebunden sind, der Ring 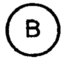 mit dem Substituenten R³ für einen Heteroaryl-Ring der Formel 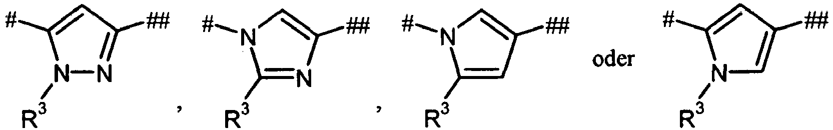 steht, worin
- #: die Verknüpfungsstelle mit der angrenzenden CH₂-Gruppe
und
- ##: die Verknüpfungsstelle mit dem Ring 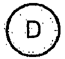 bezeichnen,
der Ring 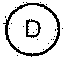 für einen Heteroaryl-Ring der Formel 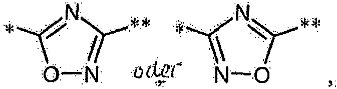 steht, worin
- *: die Verknüpfungsstelle mit dem Ring 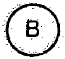 und
- **: die Verknüpfungsstelle mit dem Ring 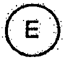 bezeichnen,
der Ring 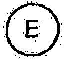 mit den Substituenten R⁴ und R⁵ für einen Phenyl-Ring der Formel 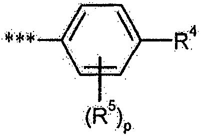 steht, worin
- ***: die Verknüpfungsstelle mit dem Ring 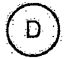 bezeichnet,
der Ring 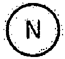 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -O-, -S-, -C(=O)-, -S(=O)₂- oder ◆-N(R⁶)-C(=O)-◆◆ steht, worin
◆ die Verknüpfungsstelle mit dem Ring 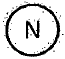 und
◆◆ die Verknüpfungsstelle mit dem Ring 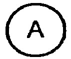 bezeichnen, q die Zahl 0, 1 oder 2 bedeutet und
R⁶ Wasserstoff, (C₁-C₄)-Alkyl oder (C₃-C₆)-Cycloalkyl bedeutet,
- R¹: für einen an ein Kohlenstoffatom des Ringes 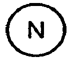 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht, wobei im Fall, dass der Substituent R¹ zweifach auftritt, seine Bedeutungen gleich oder verschieden sein können,
- R²: für einen an ein Stickstoffatom des Ringes 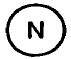 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl und (C₃-C₆)-Cycloalkyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cyclo-alkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- n: für die Zahl 0 oder 1 steht,
- R³: für Methyl, Ethyl oder Trifluormethyl steht,
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷ -S(=O)₂-R⁷, -S(=O)(=NH)-R⁷, -S(=O)(=NCH₃)-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy
substituiert sein können, wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können,
und worin
- R⁷ und R⁸: unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten,
wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
- R⁵: für einen Substituenten ausgewählt aus der Reihe Fluor, Chlor und Methyl steht und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 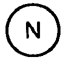 gebundenen Substituenten ausgewählt aus der Reihe Fluor, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann und (C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht, wobei im Fall, dass der Substituent R¹ zweifach auftritt, seine Bedeutungen gleich oder verschieden sein können, und
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷ , -S(=O)-R⁷, -S(=O)₂-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy
substituiert sein können,
und worin R⁷ und R⁸ die in dieser zuletzt beschriebenen Ausführungsform genannten Bedeutungen haben,
sowie ihre Salze, Solvate und Solvate der Salze.

Gleichfalls besonders bevorzugt sind Verbindungen der Formel (I), in welcher der Ring 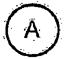 für einen Phenyl-Ring steht und die angrenzenden Gruppen X und CH₂ in 1,3-oder 1,4-Relation zueinander an diesen Phenyl-Ring gebunden sind,
der Ring 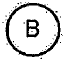 mit dem Substituenten R³ für einen Heteroaryl-Ring der Formel 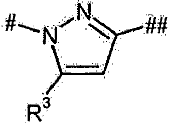 steht, worin
- #: die Verknüpfungsstelle mit der angrenzenden CH₂-Gruppe *
und
- ##: die Verknüpfungsstelle mit dem Ring 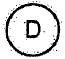 bezeichnen,
der Ring 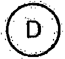 für einen Heteroaryl-Ring der Formel 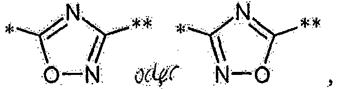 steht, worin
- *: die Verknüpfungsstelle mit dem Ring 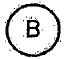 und
- **: die Verknüpfungsstelle mit dem Ring 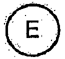 bezeichnen,der Ring 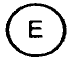 mit den Substituenten R⁴ und R⁵ für einen Phenyl-Ring der Formel 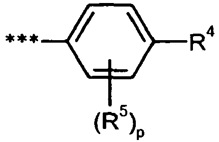 steht, worin
- ***: die Verknüpfungsstelle mit dem Ring 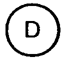 bezeichnet,
der Ring 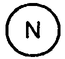 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -O-, -S-, -C(=O)-, -S(=O)₂- oder ◆-N(R⁶)-C(=O)-◆◆ steht, worin
◆ die Verknüpfungsstelle mit dem Ring 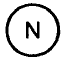 und
◆◆ die Verknüpfungsstelle mit dem Ring 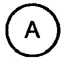 bezeichnen,
q die Zahl 0, 1 oder 2 bedeutet und
R⁶ Wasserstoff, (C₁-C₄)-Alkyl oder (C₃-C₆)-Cycloalkyl bedeutet,
- R¹: für einen an ein Kohlenstoffatom des Ringes 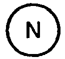 gebundenen Substituenten ausgewählt aus der Reihe Cyano, (C₁-C₄)-Alkyl, Oxo und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- R²: für einen an ein Stickstoffatom des Ringes Ⓝ gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl und (C₃-C₆)-Cycloalkyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cyclo-alkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht, wobei im Fall, dass der Substituent R¹ zweifach auftritt, seine Bedeutungen gleich oder verschieden sein können,
- n: für die Zahl 0 oder 1 steht,
wobei die Summe aus m und n gleich der Zahl 1, 2 oder 3 ist,
- R³: für Methyl, Ethyl oder Trifluormethyl steht,
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, -S(=O)(=NH)-R⁷, -S(=O)(=NCH₃)-R⁷, (C₃-C₆)-Cycloalkyl und 4- bis 6-gliedriges Heterocyclyl steht, wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy
substituiert sein können,
wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können,
und worin
R⁷ und R⁸ unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten,
wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl substituiert sein können,
oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkyl-carbonyl substituiert sein kann,
- R⁵: für einen Substituenten ausgewählt aus der Reihe Fluor, Chlor und Methyl steht und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 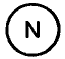 gebundenen Substituenten ausgewählt aus der Reihe (C₁-C₄)-Alkyl, Oxo und (C₃-C₆)-Cycloalkyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
- m: für die Zahl 0, 1 oder 2 steht,
wobei im Fall, dass der Substituent R¹ zweifach auftritt, seine Bedeutungen gleich oder verschieden sein können,
- n: für die Zahl 0 oder 1 steht,
wobei die Summe aus m und n gleich der Zahl 1, 2 oder 3 ist,
und
- R⁴: für einen Substituenten ausgewählt aus der Reihe Fluor, Chlor, Cyano, Pentafluorthio, (C₁-C₆)-Alkyl, Tri-(C₁-C₄)-alkylsilyl, -OR₇, -NR⁷R⁸, -SR7, -S(=O)-R⁷, -S(=O)₂-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei

die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl sowie die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, Trifluormethyl,
(C₁-C₄)-Alkoxy und Trifluormethoxy substituiert sein können,
und worin R⁷ und R⁸ die in dieser zuletzt beschriebenen Ausführungsform genannten Bedeutungen haben,
sowie ihre Salze, Solvate und Solvate der Salze.

Ganz besonders bevorzugt im Rahmen der vorliegenden Erfindung sind Verbindungen der Formel (I), in welcher der Ring 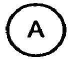 für einen Pyridyl-Ring der Formel 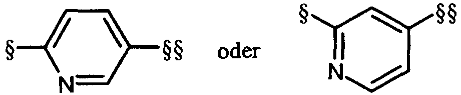 steht, worin
- §: die Verknüpfungsstelle mit der angrenzenden Gruppe X
und
- §§: die Verknüpfungsstelle mit der angrenzenden CH₂-Gruppe bezeichnen,
der Ring 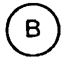 mit dem Substituenten R³ für einen Heteroaryl-Ring der Formel 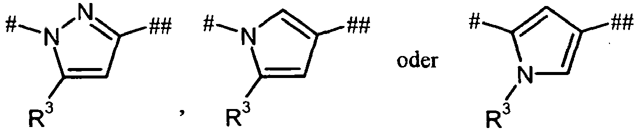 steht, worin
- #: die Verknüpfungsstelle mit der angrenzenden CH₂-Gruppe
und
- ##: die Verknüpfungsstelle mit dem Ring 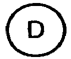 bezeichnen,
der Ring 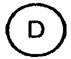 für einen Heteroaryl-Ring der Formel 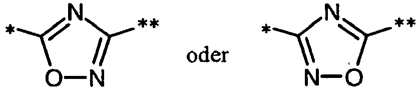 steht, worin
- *: die Verknüpfungsstelle mit dem Ring 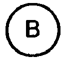 und
- **: die Verknüpfungsstelle mit dem Ring 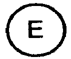 bezeichnen,
der Ring 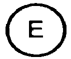 mit den Substituenten R⁴ und R⁵ für einen Phenyl-Ring der Formel 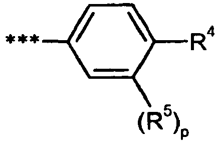 steht, worin
- ***: die Verknüpfungsstelle mit dem Ring 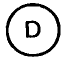 bezeichnet,der Ring 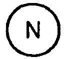 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 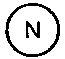 und
◆◆ die Verknüpfungsstelle mit dem Ring 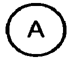 bezeichnen,
q die Zahl 0, 1 oder 2 bedeutet und
R⁶ Wasserstoff, Methyl, Ethyl, Isopropyl, Cyclopropyl oder Cyclobutyl bedeutet,
- R¹: für einen an ein Kohlenstoffatom des Ringes 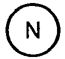 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht,
- R²: für einen an ein Stickstoffatom des Ringes 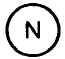 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cyclo-alkyl und 4- oder 5-gliedriges Heterocyclyl sowie bis zu dreifach mit Fluor substituiert sein kann,
- n: für die Zahl 0 oder 1 steht,
- R³: für Methyl steht,
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cyclo-alkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der genannte (C₁-C₄)-Alkyl-Substituent seinerseits mit Methoxy, Trifluormethoxy oder Ethoxy substituiert sein kann,
und worin
R⁷ und R⁸ unabhängig voneinander bei jedem einzelnen Auftreten Wasserstoff, (C₁-C₄)-Alkyl oder (C₃-C₆)-Cycloalkyl bedeuten,
wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können,
oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
- R⁵: für Fluor steht und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 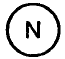 gebundenen Substituenten ausgewählt aus der Reihe Fluor, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht, und
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, (C₃-C₆)-Cycloalkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
und worin R⁷ und R⁸ die in dieser zuletzt beschriebenen Ausführungsform genannten Bedeutungen haben,
sowie ihre Salze, Solvate und Solvate der Salze.

Ganz besonders bevorzugt sind auch Verbindungen der Formel (I), in welcher der Ring 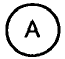 für einen Phenyl-Ring steht und die angrenzenden Gruppen X und CH₂ in 1,3- oder 1,4-Relation zueinander an diesen Phenyl-Ring gebunden sind, der Ring 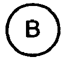 mit dem Substituenten R³ für einen Heteroaryl-Ring der Formel 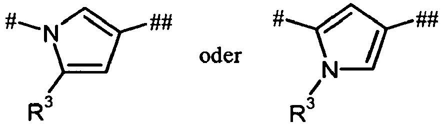 steht, worin
- #: die Verknüpfungsstelle mit der angrenzenden CH₂-Gruppe
und
- ##: die Verknüpfungsstelle mit dem Ring 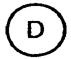 bezeichnen, der Ring 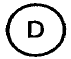 für einen Heteroaryl-Ring der Formel 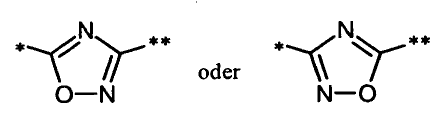 steht, worin
- *: die Verknüpfungsstelle mit dem Ring 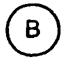 und
- **: die Verknüpfungsstelle mit dem Ring 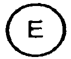 bezeichnen,
der Ring 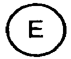 mit den Substituenten R⁴ und R⁵ für einen Phenyl-Ring der Formel 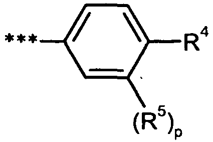 steht, worin
- ***: die Verknüpfungsstelle mit dem Ring 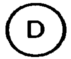 bezeichnet,
der Ring 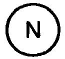 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 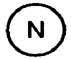 und
◆◆ die Verknüpfungsstelle mit dem Ring 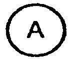 bezeichnen,
q die Zahl 0, 1 oder 2 bedeutet
und
R⁶ Wasserstoff, Methyl, Ethyl, Isopropyl, Cyclopropyl oder Cyclobutyl bedeutet,
- R¹: für einen an ein Kohlenstoffatom des Ringes 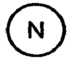 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht,
- R²: für einen an ein Stickstoffatom des Ringes 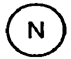 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cyclo-alkyl und 4- oder 5-gliedriges Heterocyclyl sowie bis zu dreifach mit Fluor substituiert sein kann,
- n: für die Zahl 0 oder 1 steht,
- R³: für Methyl steht,
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cyclo-alkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können, wobei der genannte (C₁-C₄)-Alkyl-Substituent seinerseits mit Methoxy, Trifluormethoxy oder Ethoxy substituiert sein kann,
und worin
- R⁷ und R⁸: unabhängig voneinander bei jedem einzelnen Auftreten Wasserstoff, (C₁-C₄)-Alkyl oder (C₃-C₆)-Cycloalkyl bedeuten,
wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkyl-carbonyl substituiert sein kann,
- R⁵: für Fluor steht
und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 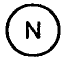 gebundenen Substituenten ausgewählt aus der Reihe Fluor, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht, und
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, (C₃-C₆)-Cycloalkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
und worin R⁷ und R⁸ die in dieser zuletzt beschriebenen Ausführungsform genannten Bedeutungen haben,
sowie ihre Salze, Solvate und Solvate der Salze.

Gleichfalls ganz besonders bevorzugt sind Verbindungen der Formel (I), in welcher der Ring 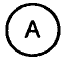 für einen Phenyl-Ring steht und die angrenzenden Gruppen X und CH₂ in 1,3- oder 1,4-Relation zueinander an diesen Phenyl-Ring gebunden sind, der Ring 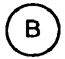 mit dem Substituenten R³ für einen Heteroaryl-Ring der Formel 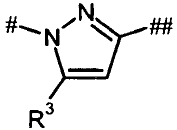 steht, worin
- #: die Verknüpfungsstelle mit der angrenzenden CH₂-Gruppe und
- ##: die Verknüpfungsstelle mit dem Ring 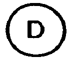 bezeichnen,
der Ring 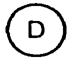 für einen Heteroaryl-Ring der Formel 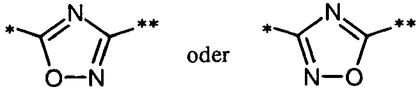 steht, worin
- *: die Verknüpfungsstelle mit dem Ring 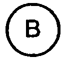 und
- **: die Verknüpfungsstelle mit dem Ring 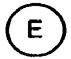 bezeichnen,
der Ring 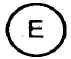 mit den Substituenten R⁴ und R⁵ für einen Phenyl-Ring der Formel 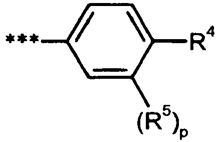 steht, worin
- ***: die Verknüpfungsstelle mit dem Ring 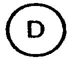 bezeichnet,
der Ring 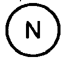 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 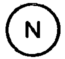 und
◆◆ die Verknüpfungsstelle mit dem Ring 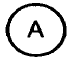 bezeichnen,
q die Zahl 0, 1 oder 2 bedeutet
und
R⁶ Wasserstoff, Methyl, Ethyl, Isopropyl, Cyclopropyl oder Cyclobutyl bedeutet,
- R¹: für einen an ein Kohlenstoffatom des Ringes 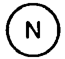 gebundenen Substituenten ausgewählt aus der Reihe Cyano, (C₁-C₄)-Alkyl, Oxo, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- R²: für einen an ein Stickstoffatom des Ringes 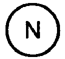 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht, wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cyclo-alkyl und 4- oder 5-gliedriges Heterocyclyl sowie bis zu dreifach mit Fluor substituiert sein kann,
- m: für die Zahl 0 oder 1 steht,
- n: für die Zahl 0 oder 1 steht,
wobei die Summe aus m und n gleich der Zahl 1 oder 2 ist,
- R³: für Methyl steht,
- R⁴: für einen Substituenten ausgewählt aus der Reihe Chlor, Pentafluorthio, (C₁-C₆)-Alkyl, Trimethylsilyl, -OR¹, -SR⁷, -S(=O)-R⁷, -S(=O)₂-R⁷, -S(=O)(=NCH₃)-CF₃, (C₃-C₆)-Cyclo-alkyl und 4- bis 6-gliedriges Heterocyclyl steht,
wobei (C₁-C₆)-Alkyl seinerseits mit einem Rest ausgewählt aus der Reihe -OR¹, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die gennanten Cycloalkyl- und Heterocyclyl-Grupprn ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der gennannte (C₁-C₄)-Alkyl-Substituent seinerseits mit Methoxy, Trifluormethoxy oder Ethoxy substituiert kann,
und worin
- R⁷ und R⁸: unabhängig voneinander bei jedem einzelnen Auftreten Wasserstoff, (C₁-C₄)-Alkyl oder (C₃-C₆)-Cycloalkyl bedeuten,

wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können,
oder 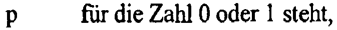
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkyl-carbonyl substituiert sein kann,
- R⁵: für Fluor steht
und
- p: für die Zahl 0 oder 1 steht,
sowie ihre Salze, Solvate und Solvate der Salze.

Eine alternative Ausführungsform innerhalb der zuletzt beschriebenen Ausführungsform umfasst Verbindungen der Formel (I), in welcher
- R¹: für einen an ein Kohlenstoffatom des Ringes 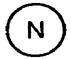 gebundenen Substituenten ausgewählt aus der Reihe (C₁-C₄)-Alkyl, Oxo, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht,
- n: für die Zahl 0 oder 1 steht,
wobei die Summe aus m und n gleich der Zahl 1 oder 2 ist, und
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷ -S(=O)₂-R⁷, (C₃-C₆)-Cycloalkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können, und worin R⁷ und R⁸ die in dieser zuletzt beschriebenen Ausführungsform genannten Bedeutungen haben,
sowie ihre Salze, Solvate und Solvate der Salze.

Eine besondere Ausführungsform der vorliegenden Erfindung betrifft Verbindungen der Formel (I), in welchen in der Definition der Gruppe X
- q: für die Zahl 0 oder 1 steht,
sowie ihre Salze, Solvate und Solvate der Salze.

Insbesondere bevorzugt im Rahmen der vorliegenden Erfindung sind Verbindungen der Formel (I), in welcher der Ring 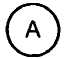 für einen Pyridyl-Ring der Formel 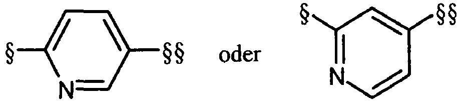 steht, worin
- §: die Verknüpfungsstelle mit der angrenzenden Gruppe X und
- §§: die Verknüpfungsstelle mit der angrenzenden CH₂-Gruppe bezeichnen,
der Ring 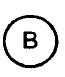 mit dem Substituenten R³ für einen Heteroaryl-Ring der Formel 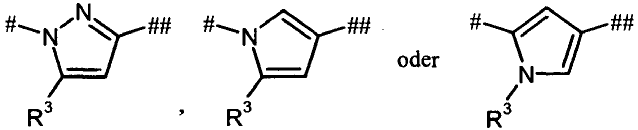 steht, worin
- #: die Verknüpfungsstelle mit der angrenzenden CH₂-Gruppe und
- ##: die Verknüpfungsstelle mit dem Ring 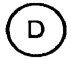 bezeichnen,
der Ring 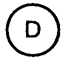 für einen Heteroaryl-Ring der Formel 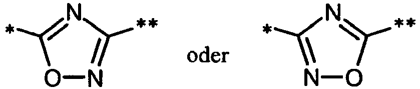 steht, worin
- *: die Verknüpfungsstelle mit dem Ring 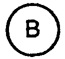 und
- **: die Verknüpfungsstelle mit dem Ring 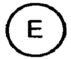 bezeichnen,
der Ring 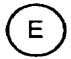 mit den Substituenten R⁴ und R⁵ für einen Phenyl-Ring der Formel 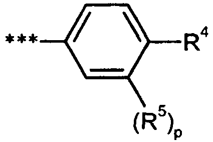 steht, worin
- ***: die Verknüpfungsstelle mit dem Ring 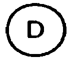 bezeichnet,
der Ring 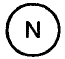 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 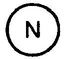 und
◆◆ die Verknüpfungsstelle mit dem Ring 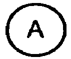 bezeichnen,
q die Zahl 0 oder 1 bedeutet und
R⁶ Wasserstoff, Methyl, Ethyl, Isopropyl, Cyclopropyl oder Cyclobutyl bedeutet,
- R¹: für einen an ein Kohlenstoffatom des Ringes 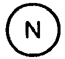 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht,
- R²: für einen an ein Stickstoffatom des Ringes 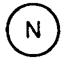 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cycloalkyl und 4- oder 5-gliedriges Heterocyclyl sowie bis zu dreifach mit Fluor substituiert sein kann,
- n: für die Zahl 0 oder 1 steht,
- R³: für Methyl steht,
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cyclo-alkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der genannte (C₁-C₄)-Alkyl-Substituent seinerseits mit Methoxy, Trifluormethoxy oder Ethoxy substituiert sein kann, und worin
- R⁷ und R⁸: unabhängig voneinander bei jedem einzelnen Auftreten Wasserstoff, (C₁-C₄)-Alkyl oder (C₃-C₆)-Cycloalkyl bedeuten,
wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
- R⁵: für Fluor steht
und
- p: für die Zahl 0 oder 1 steht,
sowie ihre Salze, Solvate und Solvate der Salze.

Insbesondere bevorzugt sind auch Verbindungen der Formel (I), in welcher der Ring 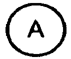 für einen Phenyl-Ring steht und die angrenzenden Gruppen X und CH₂ in 1,3- oder 1,4-Relation zueinander an diesen Phenyl-Ring gebunden sind, der Ring 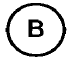 mit dem Substituenten R³ für einen Heteroaryl-Ring der Formel 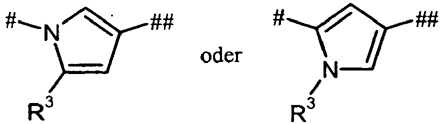 steht, worin
- #: die Verknüpfungsstelle mit der angrenzenden CH₂-Gruppe
und
- ##: die Verknüpfungsstelle mit dem Ring 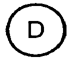 bezeichnen,
der Ring 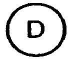 für einen Heteroaryl-Ring der Formel 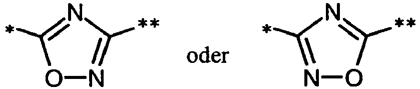 steht, worin
- *: die Verknüpfungsstelle mit dem Ring 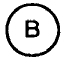 und
- **: die Verknüpfungsstelle mit dem Ring 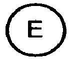 bezeichnen,
der Ring 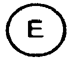 mit den Substituenten R⁴ und R⁵ für einen Phenyl-Ring der Formel 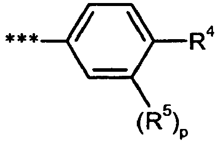 steht, worin
- ***: die Verknüpfungsstelle mit dem Ring 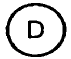 bezeichnet,
der Ring 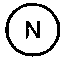 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 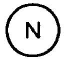 und
◆◆ die Verknüpfungsstelle mit dem Ring 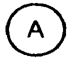 bezeichnen,
q die Zahl 0 oder 1 bedeutet und
R⁶ Wasserstoff, Methyl, Ethyl, Isopropyl, Cyclopropyl oder Cyclobutyl bedeutet,
- R¹: für einen an ein Kohlenstoffatom des Ringes 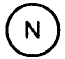 gebundenen Substituenten ausgewähl taus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- m: für die Zahl 0 oder 1 steht,
- R²: für einen an ein Stickstoffatom des Ringes 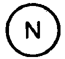 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cyclo-alkyl und 4- oder 5-gliedriges Heterocyclyl sowie bis zu dreifach mit Fluor substituiert sein kann,
- n: für die Zahl 0 oder 1 steht,
- R³: für Methyl steht,
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cyclo-alkyl und 4- bis 6-gliedriges Heterocyclyl steht, wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können, wobei der genannte (C₁-C₄)-Alkyl-Substituent seinerseits mit Methoxy, Trifluormethoxy oder Ethoxy substituiert sein kann,
und worin
- R⁷ und R⁸: unabhängig voneinander bei jedem einzelnen Auftreten Wasserstoff, (C₁-C₄)-Alkyl oder (C₃-C₆)-Cycloalkyl bedeuten, wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
- R⁵: für Fluor steht
und
- p: für die Zahl 0 oder 1 steht,
sowie ihre Salze, Solvate und Solvate der Salze.

Insbesondere bevorzugt sind auch Verbindungen der Formel (I), in welcher der Ring 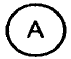 für einen Phenyl-Ring steht und die angrenzenden Gruppen X und CH₂ in 1,3- oder 1,4-Relation zueinander an diesen Phenyl-Ring gebunden sind, der Ring 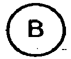 mit dem Substituenten R³ für einen Heteroaryl-Ring der Formel 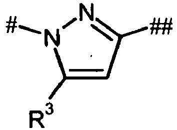 steht, worin
- #: die Verknüpfungsstelle mit der angrenzenden CH₂-Gruppe und
- ##: die Verknüpfungsstelle mit dem Ring 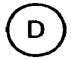 bezeichnen,
der Ring 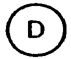 für einen Heteroaryl-Ring der Formel 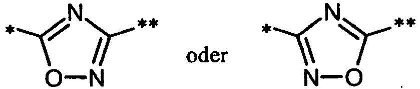 steht, worin
- *: die Verknüpfungsstelle mit dem Ring 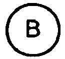 und
- **: die Verknüpfungsstelle mit dem Ring 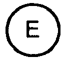 bezeichnen,
der Ring 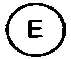 mit den Substituenten R⁴ und R⁵ für einen Phenyl-Ring der Formel 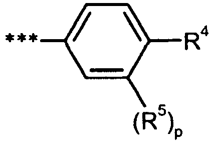 steht, worin
- ***: die Verknüpfungsstelle mit dem Ring 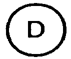 bezeichnet,
der Ring 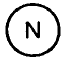 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
- X: für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 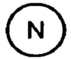 und
◆◆ die Verknüpfungsstelle mit dem Ring 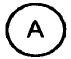 bezeichnen,
q die Zahl 0 oder 1 bedeutet und
R⁶ Wasserstoff, Methyl, Ethyl, Isopropyl, Cyclopropyl oder Cyclobutyl bedeutet,
- R¹: für einen an ein Kohlenstoffatom des Ringes 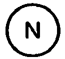 gebundenen Substituenten ausgewählt aus der Reihe Cyano, (C₁-C₄)-Alkyl, Oxo, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
- R²: für einen an ein Stickstoffatom des Ringes 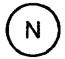 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cycloalkyl und 4- oder 5-gliedriges Heterocyclyl sowie bis zu dreifach mit Fluor substituiert sein kann,
- m: für die Zahl 0 oder 1 steht,
- n: für die Zahl 0 oder 1 steht,
wobei die Summe aus m und n gleich der Zahl 1 oder 2 ist,
- R³: für Methyl steht,
- R⁴: für einen Substituenten ausgewählt aus der Reihe Chlor, Pentafluorthio, (C₁-C₆)-Alkyl, Trimethylsilyl, -OR⁷, -SR⁷, -S(=O)-R⁷ -S(=O)₂-R⁷, -S(=O)(=NCH₃)-CF₃, (C₃-C₆)-Cyclo-alkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der genannte (C₁-C₄)-Alkyl-Substituent seinerseits mit Methoxy, Trifluormethoxy oder Ethoxy substituiert sein kann, und worin
- R⁷ und R⁸: unabhängig voneinander bei jedem einzelnen Auftreten Wasserstoff, (C₁-C₄)-Alkyl oder (C₃-C₆)-Cycloalkyl bedeuten, wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
oder
- R⁷ und R⁸: im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
und
- p: für di Zahl 0 oder 1 steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen Verbindungen können auf vielfältige Art und Weise hergestellt werden. Zur Anwendung kamen hier insbesondere die im Folgenden als Verfahren A, B, C und D bezeichneten prinzipiellen Methoden, die in unterschiedlichen Varianten ausgeführt werden können.

Es gilt für alle Verfahren und Varianten von Verfahren, die im Folgenden beschrieben werden, dass, wenn der an ein Stickstoffatom des Ringes N gebundene Rest R² für Wasserstoff steht (d.h. n = 0 ist), je nach Reaktionstyp anstelle dieses Wasserstoffatoms eine Amino-Schutzgruppe eingesetzt wird, die nach erfolgter Reaktion oder am Ende der Reaktionssequenz wieder abgespalten wird, um die Zielverbindungen der Formel (I) zu erhalten. Dies ist immer dann der Fall, wenn ein an ein Stickstoffatom gebundenes Wasserstoffatom nicht kompatibel mit den angewendeten Reaktionsbedingungen ist. Dieses Vorgehen, einschließlich der Kenntnis darüber, wann entsprechende Reaktionsbedingungen nicht kompatibel sind, und die hierfür zweckmäßigen Amino-Schutzgruppen, einschließlich der Verfahren zu ihrer Einführung und Abspaltung, sind dem Fachmann an sich bekannt und geläufig. Beispiele für solche Amino-Schutzgruppen sind tert.-Butoxycarbonyl und Benzyloxycarbonyl. Detaillierte Beschreibungen solcher Schutzgruppen-Operationen finden sich in den Versuchsvorschriften zur Herstellung der Ausgangsmaterialien und Intermediate sowie der Ausführungsbeispiele im Experimentellen Teil. Zur besseren Verständlichkeit wird in der folgenden Beschreibung der Herstellverfahren zu den erfindungsgemäßen Verbindungen auf Schutzgruppen und Schutzgruppen-Operationen dieser Art nicht weiter eingegangen.

Verfahren A (mit den Varianten A.1, A.2 und A.3; siehe Schemata 1-3) ist dadurch gekennzeichnet, dass Verbindungen der Formel (V), in welcher B, D, E, R³, R⁴, R⁵ und p die oben beschriebenen Bedeutungen haben und in der das angezeigte Wasserstoffatom an ein Stickstoffatom des Ringes B gebunden ist, mit einer Verbindung der Formel (II), (III) oder (IV) umgesetzt werden, in denen A, N, X, R¹, R², m und n die oben beschriebenen Bedeutungen haben und in denen Y ganz allgemein für ein Atom oder eine Gruppierung steht, mit deren Hilfe sich die Verbindungsgruppe X aufbauen und der Ring N (einschließlich seiner Substituenten R¹ und R²) verknüpfen läßt, und in denen Z für eine Abgangsgruppe steht. Beispiele für Y sind Chlor, Brom, Iod, Cyano, Nitro, Hydroxy, Formyl, Carboxyl und Alkoxycarbonyl; Beispiele für Z sind Chlor, Brom, Iod, Methansulfonat (Mesylat), Trifluormethansulfonat (Triflat) und 4-Methylbenzolsulfonat (Tosylat). 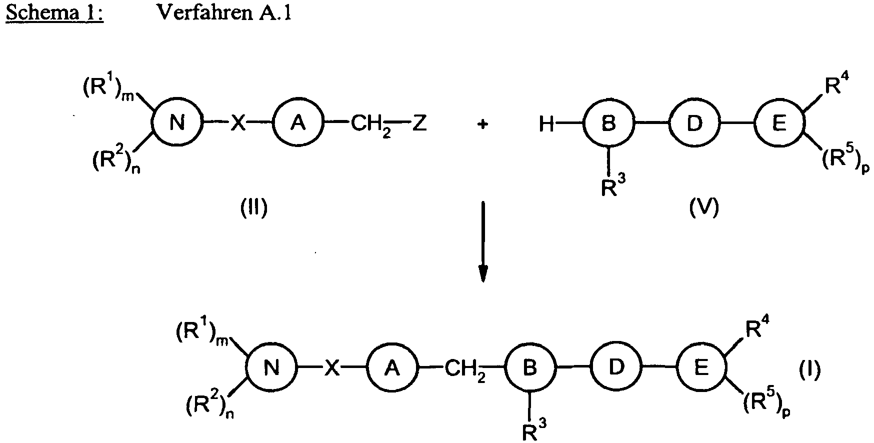 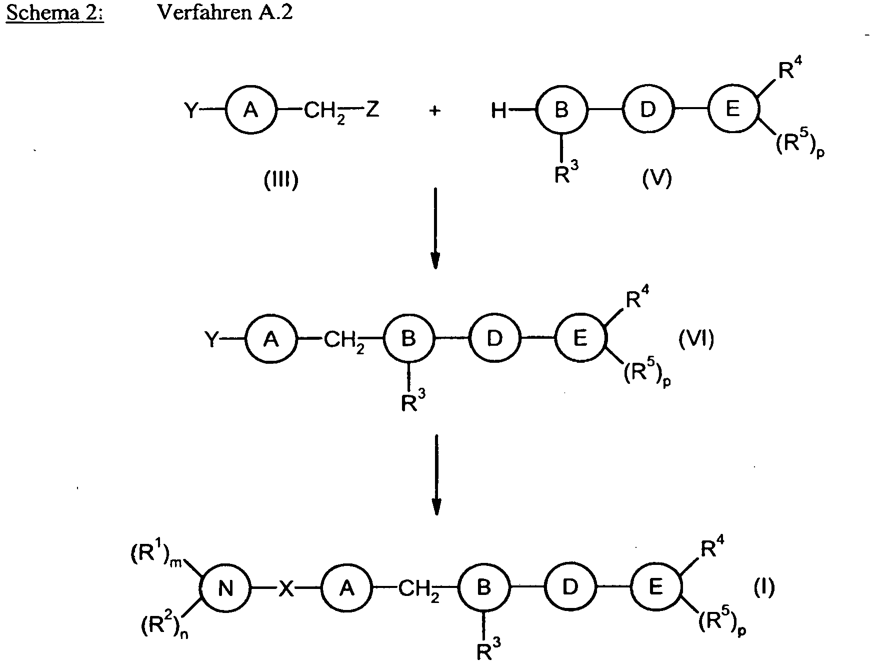 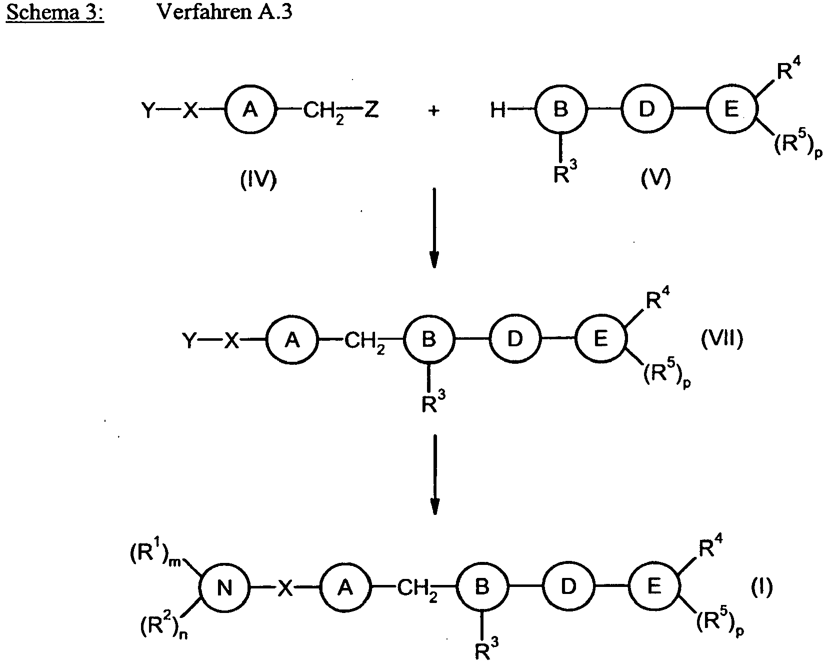 Die Umsetzung der Verbindungen der Formel (II), (III) bzw. (IV) mit den Verbindungen der Formel (V) erfolgt in Gegenwart einer starken Base, wie zum Beispiel und vorzugsweise Kalium-*tert*.-butylat, in einem geeigneten Lösungsmittel, wie zum Beispiel und vorzugsweise Tetrahydrofuran, in einem Temperaturbereich zwischen -10°C und +50°C, vorzugsweise zwischen 0°C und Raumtemperatur. Die anschließende Umsetzung der Intermediate der Formeln (VI) und (VII) zu den Produkten der Formel (I) variiert und hängt insbesondere von der Natur der Gruppe X und des Ringes A ab. Diese Folgereaktionen werden weiter unten beschrieben.

Im Verfahren B wird der Ring D aufgebaut, wobei der Ring D hier für ein 1,2,4-Oxadiazol steht. Auch das Verfahren B kommt in verschiedenen Abwandlungen zur Anwendung. Die Varianten des Verfahrens B (Varianten B.1, B.2 und B.3) ähneln den unterschiedlichen Varianten des Verfahrens A bezüglich der verwendeten Edukte und der Teilreaktionen, die auf den Ringschluss zum Oxadiazol folgen. Im Folgenden soll deshalb nur die Variante B.1 detaillierter dargestellt werden (Schema 4). Verbindungen der Formel (VIII), in der A, B, N, X, R¹, R², R³, m und n die oben beschriebenen Bedeutungen haben, werden hierbei mit Hydroxyamidinen der Formel (IX), in welcher E, R⁴, R⁵ und p die oben angegebenen Bedeutungen haben, zu den Produkten der Formel (I-A) umgesetzt.

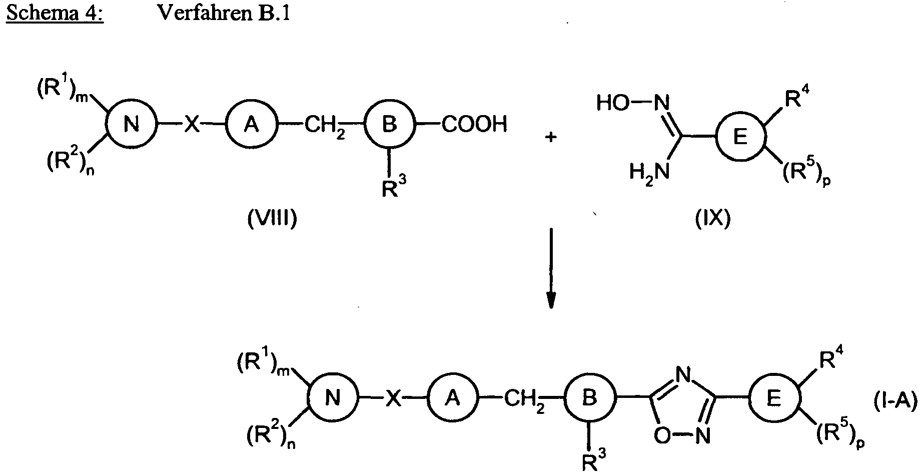

Die Umsetzung der Verbindungen der Formel (VIII) mit den Verbindungen der Formel (IX) erfolgt in Gegenwart von Kupplungsreagenzien, wie zum Beispiel 1*H*-Benzotriazol-1-ol und *N*-[3-(Di-methylamino)propyl]-*N*'-ethylcarbodiimid-Hydrochlorid, in Gegenwart von tertiären Aminbasen, wie zum Beispiel Triethylamin, in geeigneten Lösungsmitteln wie zum Beispiel *N*,*N*-Dimethyl-formamid. Die Reaktionspartner werden zunächst einige Zeit bei Raumtemperatur miteinander umgesetzt, bevor das Gemisch dann auf Temperaturen im Bereich von +80°C bis +140°C erhitzt wird. Alternativ können die Verbindungen der Formel (VIII) zunächst in die entsprechenden Carbonsäurechloride überführt werden. Dazu werden Chlorierungsreagenzien, wie zum Beispiel Oxalylchlorid oder Thionylchlorid, in inerten Lösungsmitteln, wie zum Beispiel Dichlormethan oder Chloroform, eingesetzt. Die Reaktion erfolgt bevorzugt bei Raumtemperatur und in Gegenwart einer katalytischen Menge *N*,*N*-Dimethylformamids. Das so erhaltene Säurechlorid wird anschließend mit den Verbindungen der Formel (IX) zur Reaktion gebracht. Das Produkt dieser Reaktion wird dann in inerten Lösungsmitteln, wie zum Beispiel Dimethylsulfoxid oder *N,N*-Dimethylformamid, auf Temperaturen im Bereich von +80°C bis +140°C erhitzt.

In den übrigen Varianten des Verfahrens B kommen anstelle von Verbindungen der Formel (VIII) Carbonsäuren der Formel (X) (Verfahren B.2) oder (XI) (Verfahren B.3) zum Einsatz, in denen jeweils A, B, X, Y und R³ die oben beschriebenen Bedeutungen haben.

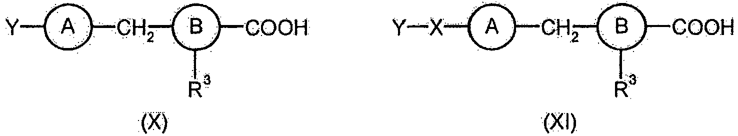

Verfahren D beschreibt die Herstellung von Verbindungen der Formel (I), in welcher der Ring D für ein 1,2,4-Oxadiazol steht, das im Vergleich zu den in Verfahren B beschriebenen Oxadiazol-Derivaten in seitenvertauschter Weise mit den angrenzenden Gruppen verknüpft ist. Analog zu den Verfahren A, B und C kann Verfahren D in den unterschiedlichen Varianten D.1, D.2 und D.3 ausgeführt werden; wie für die Verfahren B und C wird im Folgenden nur die Variante D.1 näher erläutert (Schema 6). Die Carbonsäuren der Formel (VIII) werden hierbei zunächst in die primären Amidc der Formel (XV) überführt, aus denen dann die Nitrile der Formel (XVI) hergestellt werden. Durch Reaktion mit Hydroxylamin werden diese in die Hydroxyamidine der Formel (XVII) überführt, aus denen durch Kupplung mit den Säurechloriden der Formel (XVIII) und anschlieβende Cyclisierung die Produkte der Formel (I-C) erhalten werden. A, B, E, N, X, R¹, R², R³, R⁴, R⁵, m, n und p haben jeweils die oben beschriebenen Bedeutungen.

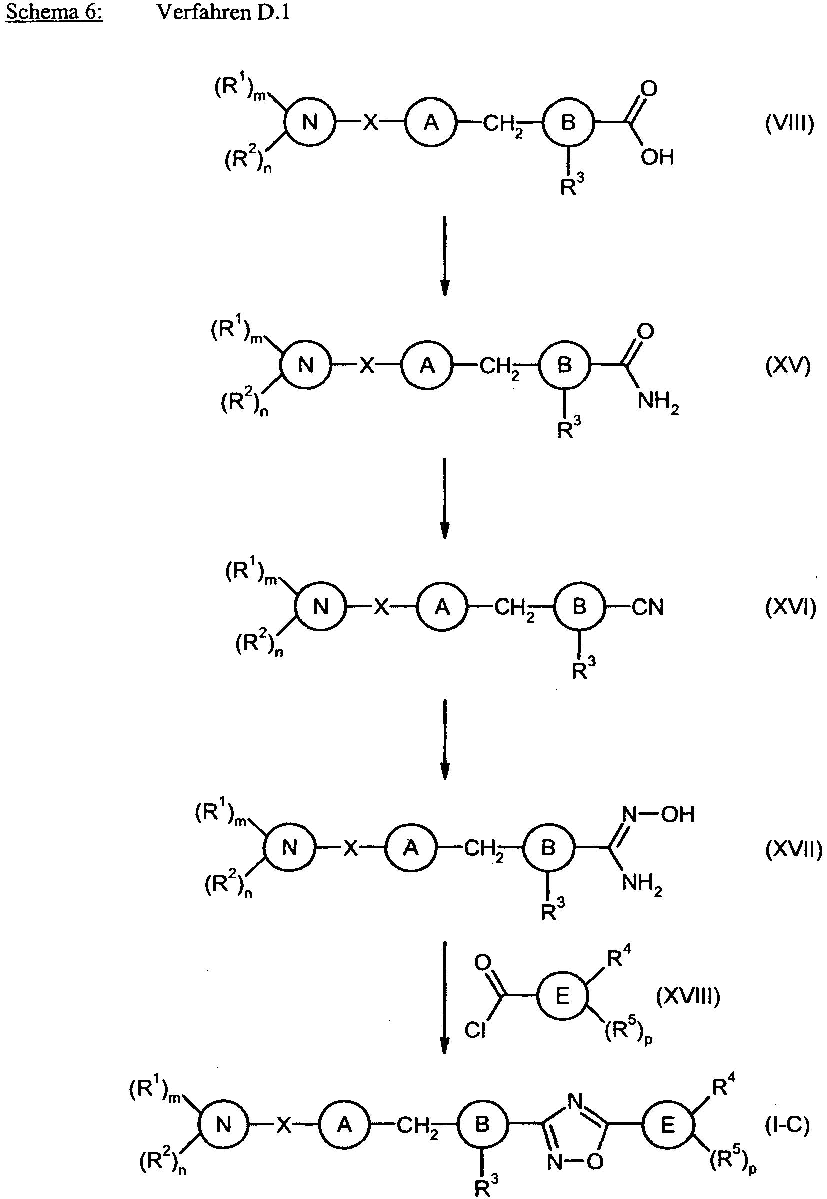

Die Umsetzung der Carbonsäuren der Formel (VIII) zu den Amiden der Formel (XV) erfolgt in zwei Stufen: Zunächst durch Reaktion mit Chlorierungsreagenzien, wie zum Beispiel Oxalylchlorid oder Thionylchlorid, in inerten Lösungsmitteln, wie zum Beispiel Dichlormethan oder Chloroform, und anschließend durch Umsetzung der so erhaltenen Carbonsäurechloride mit Lösungen von Ammoniak in Methanol oder Wasser in einem geeigneten Ko-Solvens wie zum Beispiel Tetrahydrofuran oder 1,4-Dioxan. Die Dehydratisierung der primären Amide der Formel

(XV) zu den Nitrilen der Formel (XVI) erfolgt durch Reaktion mit Anhydriden oder Chloriden starker Säuren, wie beispielsweise und bevorzugt von Trifluormethansulfonsäure oder Trifluoressigsäure, in Gegenwart eines Überschusses einer Base, wie zum Beispiel Triethylamin oder *N,N-*Diisopropylethylamin, in inerten Lösungsmitteln wie zum Beispiel Dichlormethan. Die Reaktion erfolgt bevorzugt im Temperaturbereich zwischen 0°C und Raumtemperatur. Die anschließende Umsetzung mit Hydroxylamin erfolgt bevorzugt in alkoholischen Lösungsmitteln, wie zum Beispiel Ethanol, am Siedepunkt des Lösungsmittels. Die so erhaltenen Hydroxyamidine der Formeln (XVII) werden mit den Säurechloriden der Formel (XVIII) in Gegenwart von Basen, wie zum Beispiel Triethylamin oder *N,N*-Diisopropylethylamin, in inerten Lösungsmitteln, wie zum Beispiel Dichlormethan oder Ethylacetat, bei Temperaturen zwischen -10°C und Raumtemperatur umgesetzt. Die dabei erhaltenen Zwischenprodukte werden in inerten Lösungsmitteln, wie zum Beispiel Dimethylsulfoxid oder *N,N-*Dimethylformamid, bei Temperaturen zwischen +80°C und +160°C zu den Produkten der Formel (I-C) cyclisiert.

Im Folgenden werden die Reaktionen, die von den Intermediaten der Formel (VI) (Verfahren A.2, Schema 2) zu den Produkten der Formel (I) führen, in Abhängigkeit von der Gruppe X und der Art des Ringes A beschrieben. Diese Reaktionen finden entsprechend auch Anwendung in den Verfahren B.2 und D.2.
*a)* Wenn X für ◆-(CH₂)_{q}-NR⁶-◆◆, O oder S steht, worin R⁶, q, ◆ und ◆◆ die oben beschriebenen Bedeutungen haben, und der Ring A für einen Pyridinring steht, und die Gruppe Y an ein Kohlenstoffatom dieses Pyridinrings gebunden ist, das sich in direkter Nachbarschaft zu dem Pyridin-Stickstoffatom befindet, und Y für Halogen oder ein Sulfonat steht, dann werden gemäß Schema 7 Verbindungen der Formel (VI) mit entsprechenden Verbindungen der Formel (XIX) umgesetzt. Die Reaktion erfolgt in Gegenwart eines Überschusses der Verbindung der Formel (XIX), und wenn X für O oder S steht, zusätzlich in Gegenwart einer Base, wie zum Beispiel Natriumhydrid. Die Umsetzung findet in Lösungsmitteln wie Diethylenglykoldimethylether oder *N-*Methylpyrrolidinon oder, wenn X für ◆-(CH₂)_{q}-NR⁶-◆◆ steht, in tertiären Aminbasen wie *N*,*N-*Diisopropylethylamin statt, oder die Verbindungen der Formel (XIX) dienen selbst als Lösungsmittel. Die Reaktion wird bei erhöhter Temperatur ausgeführt, vorzugsweise in einem Temperatur-bereich zwischen +80°C und +200°C. Reaktionen im oberen Bereich des genannten Temperatur-intervalls werden bevorzugt in geschlossenen Druckgefäßen in einem Mikrowellengerät durchgeführt. 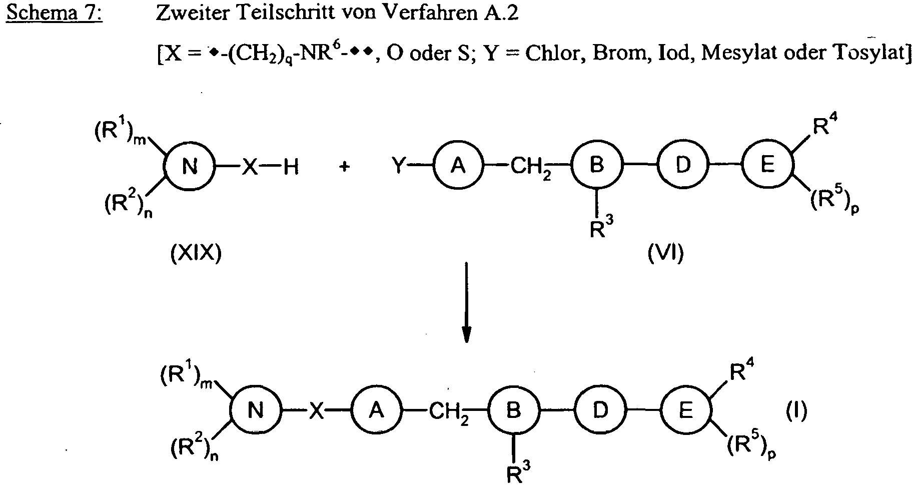
*b)* Wenn X für ◆-(CH₂)_{q}-NR⁶-◆◆, O oder S steht und die Gruppe Y für Halogen oder ein Sulfonat steht und an ein Kohlenstoffatom eines Pyridinrings A gebunden ist, das sich in beliebiger Position in Relation zu dem Pyridin-Stickstoffatom befindet, oder es sich bei Ring A um einen Phenylring handelt, dann werden die Verbindungen der Formel (VI) und die Verbindungen der Formel (XIX) gemäß Schema 7 in Gegenwart von Palladium-Katalysatoren miteinander umgesetzt. Geeignete Palladium-Quellen sind zum Beispiel Palladium(II)acetat oder Tris(dibenzylidenaceton)dipalladium(O). Als Liganden können zum Beispiel 2,2'-Bis(diphenylphosphino)-1,1'-binaphthyl, 1-[2-(Dicyclohexylphosphino)ferrocenyl]ethyldi-*tert*.-butylphosphin oder Bis(diphenylphosphino)ferrocen verwendet werden. Die Reaktionen verlaufen in Gegenwart von Basen wie zum Beispiel Triethylamin oder Natrium-*tert*.-butylat. Geeignete Lösungsmittel sind zum Beispiel Toluol, *N-*Methylpyrrolidinon oder 1,2-Dimethoxyethan. Die Reaktionen erfolgen für gewöhnlich im Temperaturintervall zwischen +60°C und dem jeweiligen Siedepunkt des Lösungsmittels.
*c*) Wenn X für ◆-NR⁶-C(=O)-◆◆ steht, worin R⁶, ◆ und ◆◆ die oben beschriebenen Bedeutungen haben, werden Verbindungen der Formel (VI), in welcher Y für eine AlkoxycarbonylGruppe oder Cyano steht, zunächst durch Behandeln mit wässrigem Alkali in die korrespondierenden Carbonsäuren überführt und anschließend mit Verbindungen der Formel (XX) zu den Produkten der Formel (I) umgesetzt (siehe Schema 8). Diese Umsetzung erfolgt entweder direkt aus der Carbonsäure in Gegenwart von Kupplungsreagenzien wie zum Beispiel 1*H*-Benzotriazol-1-ol und *N-*[3-(Dimethylamino)propyl]-*N*'-ethylcarbodiimid-Hydrochlorid, oder indem die Carbonsäure beispielsweise mit Hilfe von Thionylchlorid oder Oxalylchlorid in das entsprechende Säurechlorid überführt und anschließend mit der Aminkomponente (XX) umgesetzt wird.

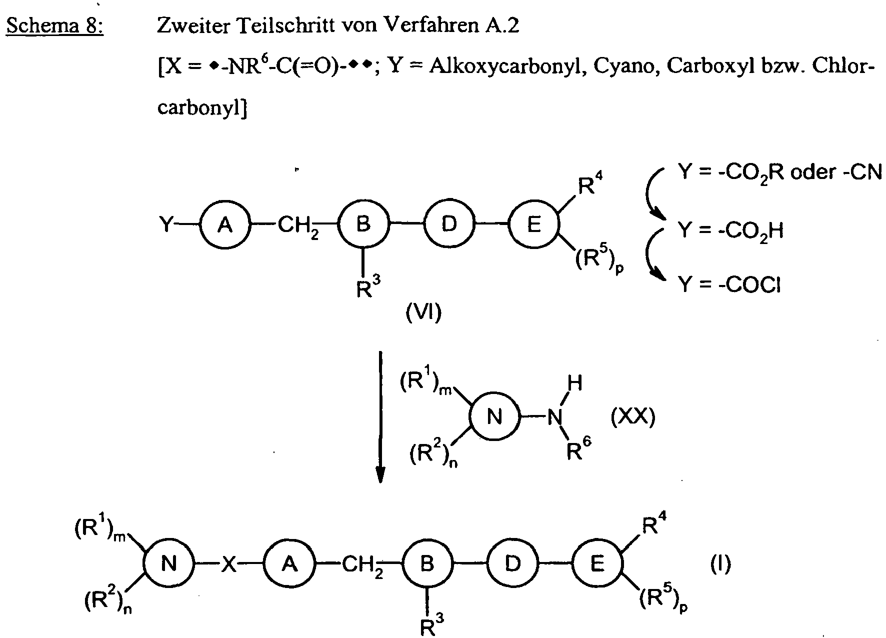

Die Hydrolyse der Ester (VI) [Y = Alkoxycarbonyl] erfolgt vorzugsweise mit wässrigen Lösungen von Lithium-, Natrium- oder Kaliumhydroxid in Gegenwart von mit Wasser mischbaren inerten Lösungsmitteln wie zum Beispiel Methanol, Ethanol oder Tetrahydrofuran. Die Reaktion erfolgt im Allgemeinen im Temperaturintervall zwischen Raumtemperatur und +60°C, vorzugsweise bei Raumtemperatur. Die Hydrolyse der Nitrile (VI) [Y = Cyano] erfolgt ebenfalls mit wässrigem Alkali, vorzugsweise mit wässrigem Kaliumhydroxid in Ethanol beim Siedepunkt des Lösungsmittels. Die anschließende Überführung der so erhaltenen Carbonsäuren in die entsprechenden Säurechloride erfolgt mit Chlorierungsreagenzien, wie zum Beispiel und vorzugsweise Oxalylchlorid oder Thionylchlorid, in inerten Lösungsmitteln wie zum Beispiel Dichlormethan. Die Reaktion wird im Temperaturbereich zwischen 0°C und dem Siedepunkt des Lösungsmittels durchgeführt, vorzugsweise bei Raumtemperatur. Die abschließende Reaktion der Amine der Formel (XX) mit den Säurechloriden der Formel (VI) [Y = Chlorcarbonyl] erfolgt in Gegenwart von Basen, wie zum Beispiel Triethylamin, *N,N*-Diisopropylethylamin oder Kaliumcarbonat, in inerten Lösungsmitteln wie zum Beispiel Dichlormethan oder Ethylacetat. Die Reaktion wird im Temperaturbereich von 0°C bis Raumtemperatur durchgeführt. Die Umsetzung der Amine der Formel (XX) mit den Carbonsäuren der Formel (VI) [Y = Carboxyl] erfolgt mit Hilfe üblicher Kupplungsreagenzien, wie zum Beispiel 1*H-*Benzotriazol-1-ol und *N*-[3-(Dimethylamino)propyl]-*N*'-ethylcarbodiimid-Hydrochlorid, in geeigneten Lösungsmitteln, wie zum Beispiel *N*,*N*-Dimethylformamid, und in Gegenwart von tertiären Aminbasen wie zum Beispiel Triethylamin. Die Reaktion wird bevorzugt bei Raumtemperatur ausgeführt.
*d)* Wenn X für -C(=O)- steht und der Ring N über ein Stickstoffatom an X gebunden ist, werden Verbindungen der Formel (VI), in welcher Y für Carboxyl oder Chlorcarbonyl steht (siehe Schema 8) mit Verbindungen der Formel (XXI), in welcher das angezeigte Wasserstoffatom an ein Stickstoffatom des Ringes N gebunden ist, zu den Produkten der Formel (I) umgesetzt.

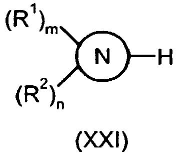

Die Reaktionsbedingungen hierzu sind völlig analog zu den unter Abschnitt c) beschriebenen.
*e)* Wenn X für ◆-C(=O)-NH-◆◆ steht, worin ◆ und ◆◆ die oben beschriebenen Bedeutungen haben, werden Verbindungen der Formel (VI), in welcher Y für eine Nitrogruppe steht, zunächst zu den entsprechenden Aminen [Y = NH₂] reduziert und anschließend mit Verbindungen der Formel (XXII) oder (XXIII) zu den Produkten der Formel (I) umgesetzt (siehe Schema 9). Diese Umsetzung erfolgt im Falle der Carbonsäuren (XXII) in Gegenwart von üblichen Kupplungsreagenzien, wie zum Beispiel 1*H-*Benzotriazol-1-ol und *N*-[3-(Dimethylamino)propyl]-*N*'-ethylcarbodiimid-Hydrochlorid, und im Falle der Säurechloride (XXXII) direkt in Gegenwart von tertiären Aminbasen wie Triethylamin oder *N,N*-Diisopropylethylamin.

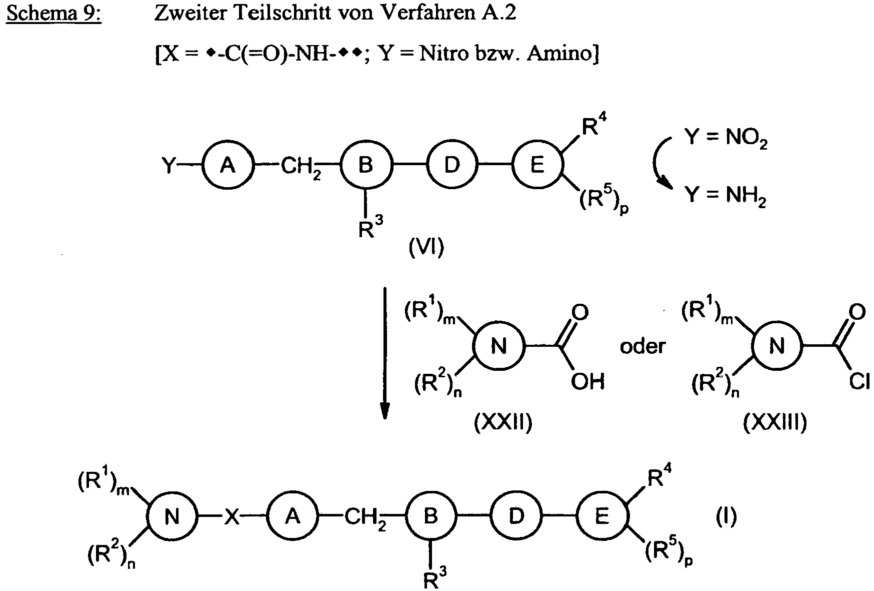

Die Reduktion der Nitrogruppe gelingt zum Beispiel durch katalytische Hydrierung mit Hilfe von Edelmetall-Katalysatoren, wie zum Beispiel Palladium auf Kohle, in inerten Lösungsmitteln, wie zum Beispiel Ethanol, in Gegenwart von Wasserstoff mit einem Druck von 1 bis 50 bar, vorzugsweise von 1 bis 5 bar. Die Reaktion erfolgt typischerweise bei Raumtemperatur. Die anschließende Umsetzung mit den Carbonsäuren (XXII) oder Säurechloriden (XXIII) erfolgt entweder mit Hilfe von Kupplungsreagenzien oder direkt in Gegenwart von tertiären Aminbasen, wie oben bereits beschrieben wurde.
*f*) Wenn X für Sauerstoff steht, können alternativ auch Verbindungen der Formel (XXIV), in welcher Z für eine Abgangsgruppe wie zum Beispiel Chlor, Brom oder Methansulfonat steht, und Verbindungen der Formel (VI), in welcher Y für Hydroxy steht, miteinander umgesetzt werden. Letztere sind beispielsweise über entsprechende Silylether erhältlich (siehe Schema 10).

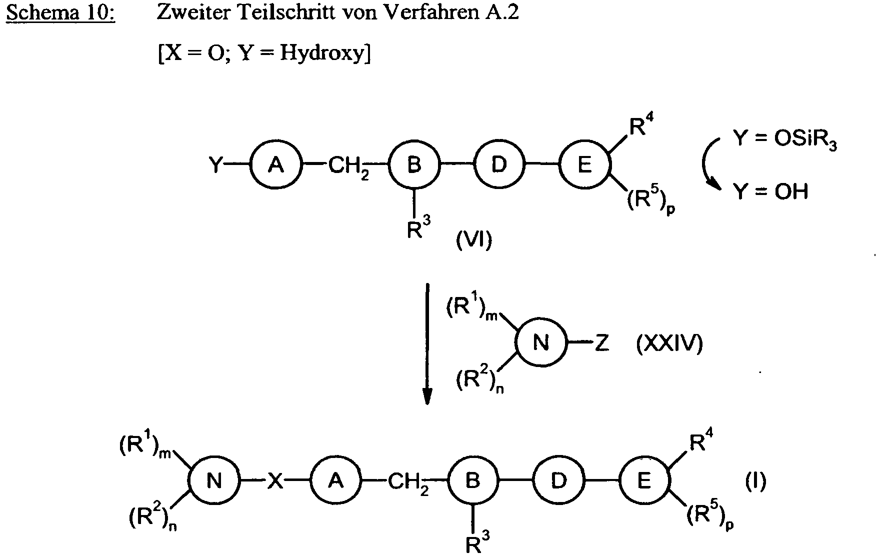

Die Umsetzung der Verbindungen der Formel (VI), in welcher Y für einen Silylether steht, zu den freien Hydroxy-Verbindungen der Formel (VI) [Y = OH] erfolgt zum Beispiel durch Behandlung mit einer Fluorid-Quelle wie Tetra-n-butylammoniumfluorid in Lösungsmitteln wie Tetrahydrofuran bei Temperaturen bevorzugt zwischen 0°C und Raumtemperatur. Die anschließende Reaktion mit den Verbindungen der Formel (XXIV) erfolgt in inerten Lösungsmitteln, wie beispielsweise und bevorzugt *N,N-*Dimethylformamid, in Gegenwart von Basen, wie zum Beispiel Natriumhydrid oder Cäsiumcarbonat, bei Temperaturen zwischen Raumtemperatur und +140°C.
*g*) Ein ähnliches Verfahren wie unter f) beschrieben kann angewandt werden, wenn X für NH steht. Die in Schema 9 gezeigten Verbindungen der Formel (VI), in welcher Y für NH₂ steht, werden zunächst zum Beispiel mit Di-*tert*.-butyldicarbonat oder mit Benzyloxycarbonylchlorid in die entsprechenden Carbamate überführt, die dann mit Verbindungen der Formel (XXIV) (siehe Schema 10), in welcher Z für eine Abgangsgruppe wie Chlor, Brom oder Methansulfonat steht, umgesetzt werden. In der abschließenden Reaktion wird die Carbamat-Schutzgruppe wieder entfernt, um so die Produkte der Formel (I) zu erhalten, in der X für NH steht. Die Verfahren zur Einführung und Abspaltung der Carbamat-Schutzgruppen sind in der chemischen Literatur beschrieben und dem Fachmann bekannt. Die Reaktion der Verbindungen der Formel (XXIV) mit den aus Verbindungen der Formel (VI) [Y = NH₂] abgeleiteten Carbamaten wird unter ähnlichen Bedingungen durchgeführt wie unter f) beschrieben.
*h*) Wenn X für eine Bindung steht, der Ring N über ein Ring-Stickstoffatom an den Ring A gebunden ist, der Ring A für einen Pyridinring steht und die Gruppe Y an ein Kohlenstoffatom dieses Pyridinrings gebunden ist, das sich in direkter Nachbarschaft zu dem Pyridin-Stickstoffatom befindet, und Y für Halogen oder ein Sulfonat steht, dann werden Verbindungen der Formel (VI) mit Verbindungen der Formel (XXI), in welcher das angezeigte Wasserstoffatom an ein Stickstoffatom des Ringes N gebunden ist, umgesetzt (siehe Schema 11). Die Reaktion erfolgt in Gegenwart eines Überschusses der Verbindung der Formel (XXI) und gegebenenfalls in Gegenwart einer tertiären Aminbase wie beispielsweise *N,N-*Diisopropylethylamin. Die Umsetzung findet in Lösungsmitteln wie Diethylenglykoldimethylether oder *N*-Methylpyrrolidinon statt, oder die tertiäre Aminbase oder die Verbindungen der Formel (XXI) dienen selbst als Lösungsmittel. Die Reaktion wird bei erhöhter Temperatur ausgeführt, vorzugsweise in einem Temperaturbereich zwischen +80°C und +200°C. Reaktionen im oberen Bereich des genannten Temperaturintervalls werden bevorzugt in geschlossenen Druckgefäßen in einem Mikrowellengerät durchgeführt. 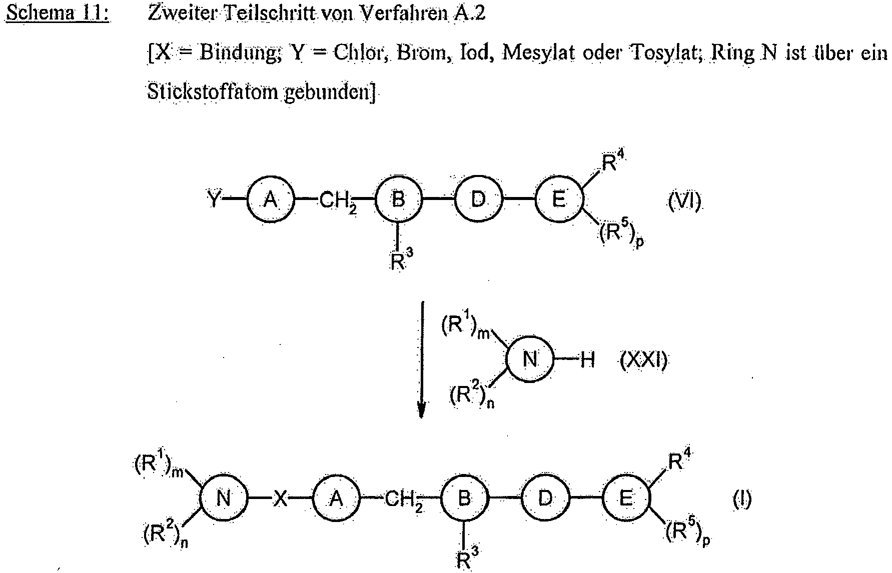
*i*) Wenn X für eine Bindung steht, der Ring N über ein Ring-Stickstoffatom an den Ring A gebunden ist und die Gruppe Y für Halogen, oder ein Sulfonat steht und an ein Kohlenstoffatom eines Pyridinrings A gebunden ist, das sich in beliebiger Position in Relation zu dem Pyridin-Stickstoffatom befindet, oder es sich bei Ring A um einen Phenylring handelt, dann werden die Verbindungen der Formel (VI) und die Verbindungen der Formel (XXI) gemäß Schema 11 in Gegenwart von Palladium-Katalysatoren miteinander umgesetzt. Geeignete Palladium-Quellen sind zum Beispiel Palladium(II)acetat oder Tris(dibenzylidenaceton)dipalladium(0). Als Liganden können zum Beispiel 2,2'-Bis(diphenylphosphino)-1,1'-binaphthyl, 1-[2-(Dicyclohexylphosphino)-ferrocenyl]ethyldi-*tert*.-butylphosphin oder Bis(diphenylphosphino)ferrocen verwendet werden. Die Reaktionen erfolgen in Gegenwart von Basen wie zum Beispiel Triethylamin oder Natrium-*tert.*-butylat. Geeignete Lösungsmitteln sind zum Beispiel Toluol, *N*-Methylpyrrolidinon oder 1,2-Dimethoxyethan. Die Reaktionen werden für gewöhnlich im Temperaturintervall zwischen +60°C und dem jeweiligen Siedepunkt des Lösungsmittels durchgeführt.

Im Folgenden werden Reaktionen, die von den Intermediaten des Typs der Formel (VII) (Verfahren A.3, Schema 3) zu den Produkten der Formel (I) führen, in Abhängigkeit von der Natur der Gruppen Y und Z beschrieben. Diese Reaktionen finden entsprechend auch Anwendung in den Verfahren B.3 und D.3.
*j)* Die Verbindungen der Formel (XXV), in welcher Y für Hydroxy steht, werden zunächst in Verbindungen der Formel (XXVI), in welcher Z für eine Abgangsgruppe wie zum Beispiel Chlor, Brom oder Methansulfonat steht, überführt und anschließend mit Aminen der Formel (XX) zu den Produkten der Formel (I) umgesetzt, in welcher X für die Gruppe ◆-NR⁶-CH₂-◆◆ steht (siehe Schema 12).

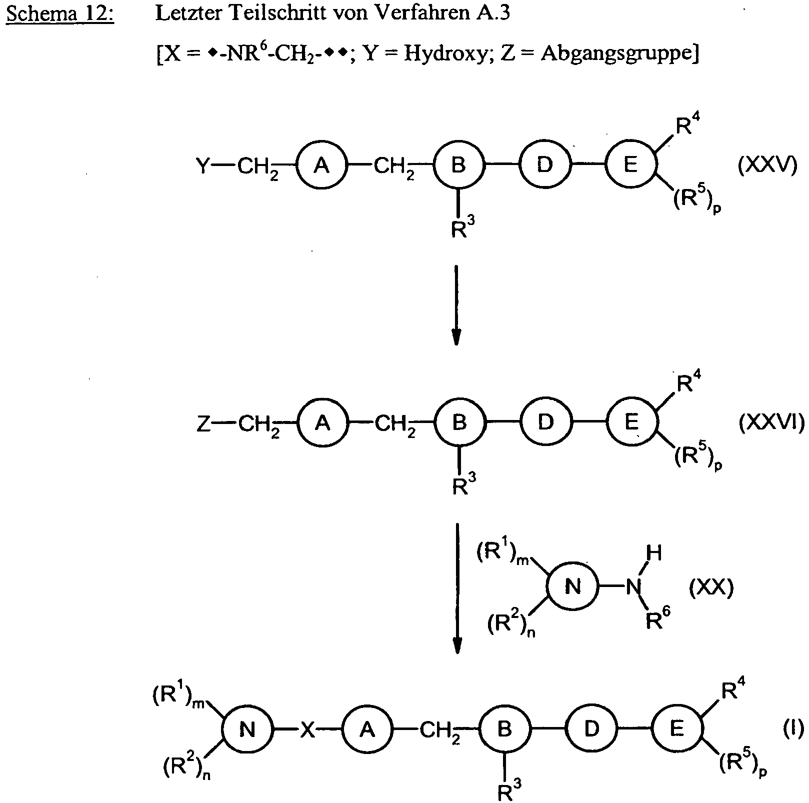

Die Verbindungen der Formel (XXV), in welcher Y für Hydroxy steht, werden zu Verbindungen der Formel (XXVI) umgewandelt, indem sie zum Beispiel mit Brom in Gegenwart von Triphenylphosphin in geeigneten Lösungsmitteln, wie zum Beispiel Tetrahydrofuran, bei Raumtemperatur zu den entsprechenden Bromiden (XXVI) [Z = Br] umgesetzt werden. Die Umwandlung kann beispielsweise und bevorzugt auch mit Hilfe von Trifluormethansulfonsäureanhydrid oder Methansulfonsäureanhydrid in Gegenwart von Basen, wie zum Beispiel Triethylamin oder 2,6-Dimethylpyridin, erfolgen. Diese Reaktionen werden bevorzugt in Dichlormethan oder Tetrahydrofuran bei tiefen Temperaturen von ca. -78°C durchgeführt. Erhalten werden so Verbindungen der Formel (XXVI), in welcher Z für Trifluormethansulfonat (Triflat) bzw. Methansulfonat (Mesylat) steht. Die Verbindungen der Formel (XXVI) werden dann mit Aminen der Formel (XX) zu den Produkten der Formel (I) umgesetzt, indem die Reaktanden beispielsweise in Dichlormethan oder Tetrahydrofuran in Gegenwart von tertiären Aminbasen, wie zum Beispiel Triethylamin oder 2,6-Dimethylpyridin, bei Temperaturen zwischen -78°C und Raumtemperatur umgesetzt werden. Wenn Z für Trifluormethansulfonat oder Methansulfonat steht, kann die Reaktionssequenz ausgehend von Verbindungen der Formel (XXV) [Y = OH] auch als Eintopfverfahren durchgeführt werden.

Die Ausgangsverbindungen der Formeln (II), (III), (IV), (V), (VIII), (IX), (X), (XI), (XII), (XVIII), (XIX), (XX), (XXI), (XXII), (XXIII) und (XXIV) sind entweder kommerziell erhältlich oder als solche in der Literatur beschrieben, oder sie können auf für den Fachmann offensichtlichem Wege analog zu in der Literatur publizierten Methoden hergestellt werden. So lassen sich beispielsweise Verbindungen der Formel (V), in welcher der Ring D für ein 1,2,4-Oxadiazol oder ein 1,3-Oxazol steht, in Analogie zu den oben beschriebenen Verfahrensmethoden B, C und D herstellen, und Verbindungen der Formeln (II), (VIII), (X) und (XI) können analog zu den Verfahrensvarianten A.1, A.2 und A.3 mit den in den Schemata 7-12 beschriebenen Teilschritten erhalten werden.

Beispielsweise können erfindungsgemäße Verbindungen der Formel (I-D) 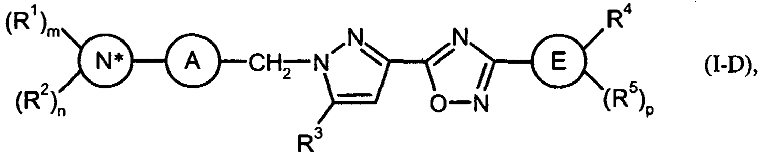 in welcher die Ringe A und E sowie R¹, R², R³, R⁴, R⁵, m, n und p jeweils die oben angegebenen Bedeutungen haben
und
der Ring N* für einen über ein Ring-Stickstoffatom an den Ring A gebundenen Ring N, welcher wie oben definiert ist, steht,
dadurch hergestellt werden, dass man ein *N*'-Hydroxyamidin der Formel (IX) 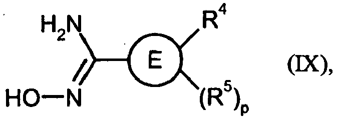 in welcher der Ring E sowie R⁴, R⁵ und p die oben angegebenen Bedeutungen haben,
zunächst entweder
[A] mit einer Pyrazolcarbonsäure der Formel (XXVII) 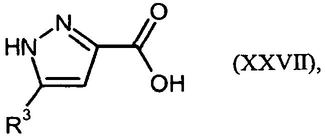 in welcher R³ die oben angegebene Bedeutung hat,
   zu einem 1,2,4-Oxadiazol-Derivat der Formel (XXVIII) 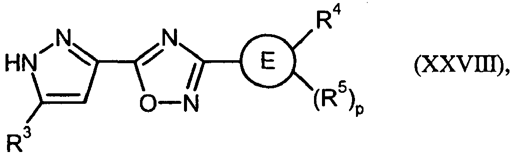 in welcher der Ring E sowie R³, R⁴, R⁵ und p die oben angegebenen Bedeutungen haben,
   kondensiert und dieses dann in Gegenwart einer Base mit einer Verbindung der Formel (III) 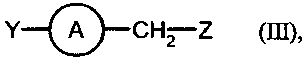 in welcher der Ring A die oben angegebene Bedeutung hat,
   - Y: für Chlor, Brom oder Iod steht
   und
   - Z: für Chlor, Brom, Iod, Mesylat, Triflat oder Tosylat steht,
   zu einer Verbindung der Formel (XXIX) 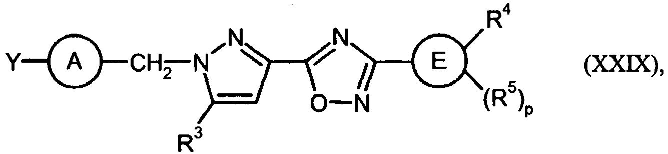 in welcher die Ringe A und E sowie R³, R⁴, R⁵, p und Y die oben angegebenen Bedeutungen haben,
   alkyliert
   oder
[B] mit einer Pyrazolcarbonsäure der Formel (XXX) 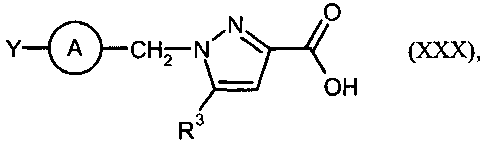 in welcher der Ring A sowie R³ die oben angegebenen Bedeutungen haben und
   - Y: für Chlor, Brom oder Iod steht,
zu der Verbindung der Formel (XXIX) 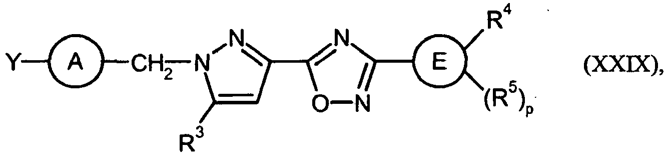 in welcher die Ringe A und E sowie R³, R⁴, R⁵, p und Y die oben angegebenen Bedeutungen haben,
kondensiert
und anschließend die so erhaltene Verbindung der Formel (XXIX) gegebenenfalls in Gegenwart eines Palladium-Katalysators und/oder einer Base mit einer Verbindung der Formel (XXXI) 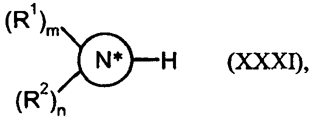 in welcher der Ring N* sowie R¹, R², m und n die oben angegebenen Bedeutungen haben und das angezeigte Wasserstoffatom an ein Stickstoffatom des Ringes N* gebunden ist,
umsetzt (vgl. hierzu das zuvor beschriebene Verfahren A.2 in Verbindung mit der in Schema 11 dargestellten Variante des zweiten Teilschritts sowie die Verfahren B.1 und B.2 mit den jeweils dort angegebenen Reaktionsparametem).

Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen hochpotente Inhibitoren des HIF-Regulationsweges dar und weisen eine gute Bioverfügbarkeit nach peroraler Gabe auf.

Die erfindungsgemäßen Verbindungen eignen sich aufgrund ihres Wirkprofils insbesondere zur Behandlung von hyperproliferativen Erkrankungen beim Menschen und bei Säugetieren allgemein. Die Verbindungen können die Zellproliferation und Zellteilung hemmen, blockieren, verringern oder senken und andererseits die Apoptose verstärken.

Zu den hyperproliferativen Erkrankungen, zu deren Behandlung die erfindungsgemäßen Verbindungen eingesetzt werden können, zählen unter anderem Psoriasis, Keloide, Narbenbildungen und andere proliferative Erkrankungen der Haut, benigne Erkrankungen wie die benigne Prostatahyperplasie (BPH), sowie insbesondere die Gruppe der Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (ductale und lobuläre Formen, auch *in situ),* Atemwegstumore (kleinzelliges und nicht-kleinzelliges Karzinom, Bronchialkarzinom), Hirntumore (z.B. des Hirnstamms und des Hypothalamus, Astrocytoma, Medulloblastoma, Ependymoma sowie neuro-ectodermale und pineale Tumore), Tumore der Verdauungsorgane (Speiseröhre, Magen, Gallenblase, Dünndarm, Dickdarm, Rektum), Lebertumore (u.a. hepatozelluläres Karzinom, Cholangiokarzinom und gemischt-hepatozelluläres Cholangiokarzinom), Tumore des Kopf- und Halsbereiches (Larynx, Hypopharynx, Nasopharynx, Oropharynx, Lippen und Mundhöhle), Hauttumore (Plattenepithelkarzinom, Kaposi-Sarkom, malignes Melanom, Merkel-zell-Hautkrebs und nicht-melanomartiger Hautkrebs), Tumore der Weichteile (u.a. Weichteilsarkome, Osteosarkome, maligne fibröse Histiozytome, Lymphosarkome und Rhabdomyosarkome), Tumore der Augen (u.a. intraokuläres Melanom und Retinoblastom), Tumore der endokrinen und exokrinen Drüsen (z.B. thyroide und parathyroide Drüsen, Bauchspeicheldrüse und Speicheldrüse), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Bluterkrankungen in solider Form und als zirkulierende Blutzellen, wie Lymphome, Leukämien und myeloproliferative Erkrankungen, z.B. akute myeloide, akute lymphoblastische, chronisch-lymphozytische, chronisch-myelogene und Haarzell-Leukämie, sowie AIDSkorrelierte Lymphome, Hodgkin-Lymphome, Non-Hodgkin-Lymphome, kutane T-Zell-Lymphome, Burkitt-Lymphome und Lymphome im zentralen Nervensystem.

Diese gut beschriebenen Krankheiten des Menschen können mit vergleichbarer Ätiologie auch in anderen Säugetieren vorkommen und dort mit den Verbindungen der vorliegenden Erfindung behandelt werden.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Die erfindungsgemäßen Verbindungen wirken als Modulatoren des HIF-Regulationsweges und eignen sich daher auch zur Behandlung von Erkrankungen, welche mit einer schädlichen Expression des HIF-Transkriptionsfaktors assoziiert sind. Dies betrifft insbesondere die Transkriptionsfaktoren HIF-1α und HIF-2α. Der Begriff "schädliche Expression von HIF" bedeutet hierbei ein nicht-normal-physiologisches Vorhandensein von HIF-Protein. Dies kann bedingt sein durch übermäßige Synthese des Proteins (mRNA- oder translationsbedingt), durch verringerten Abbau oder durch unzureichende Gegenregulation bei der Funktion des Transkriptionsfaktors.

HIF-1α und HIF-2α regulieren mehr als 100 Gene. Dies betrifft Proteine, die bei der Angiogenese eine Rolle spielen und daher direkt tumorrelevant sind, und auch solche, die den Glukose-, Aminosäure- und Lipid-Stoffwechsel sowie Zellmigration, Metastase und DNA-Reparatur beeinflussen oder durch Unterdrückung der Apoptose das Überleben der Tumorzellen verbessern. Andere wirken eher indirekt über die Hemmung der Immunreaktion und Hochregulierung von angiogenen Faktoren in Entzündungszellen. Eine wichtige Rolle spielt HIF auch bei den Stammzellen, hier insbesondere den Tumorstammzellen, von denen berichtet wird, dass sie erhöhte HIF-Spiegel aufweisen. Durch die Hemmung des HIF-Regulationsweges durch die Verbindungen der vorliegenden Erfindung werden damit auch Tumorstammzellen therapeutisch beeinflusst, die keine hohe Proliferationsrate aufweisen und daher von zytotoxischen Substanzen nur unzureichend betroffen sind (vgl. Semenza, 2007; Weidemann und Johnson, 2008).

Veränderungen des Zellmetabolismus durch HIF sind nicht exklusiv für Tumore, sondern treten auch bei anderen hypoxischen pathophysiologischen Prozessen auf, mögen sie chronisch oder transient sein. HIF-Inhibitoren - wie die Verbindungen der vorliegenden Erfindung - sind in solchen Zusammenhängen therapeutisch hilfreich, in denen beispielsweise durch eine Adaptation von Zellen an hypoxische Situationen zusätzlicher Schaden entsteht, da geschädigte Zellen, wenn sie nicht wie vorgesehen funktionieren, weitere Schäden hervorrufen können. Ein Beispiel hierfür ist die Bildung von epileptischen Herden in partiell zerstörtem Gewebe nach Schlaganfällen. Ähnliches findet man bei Herz-Kreislauf-Erkrankungen, wenn als Folge von thromboembolischen Ereignissen, Entzündungen, Verwundungen, Intoxikationen oder anderen Ursachen ischämische Prozesse im Herzen oder im Gehirn auftreten. Diese können zu Schäden führen wie einem lokal verlangsamten Aktionspotential, welches seinerseits Arrhythmien oder ein chronisches Herzversagen nach sich ziehen kann. In transienter Form, z.B. durch Apnoe, kann es unter Umständen zu einer essentiellen Blutdruckerhöhung kommen, was zu bekannten Folgeerkrankungen wie beispielsweise Schlaganfall und Herzinfarkt führen kann.

Die Hemmung des HIF-Regulationsweges, wie sie durch die erfindungsgemäßen Verbindungen erreicht wird, kann daher auch bei Erkrankungen wie Herzinsuffizienz, Arrhythmie, Herzinfarkt, Apnoe-induzierte Hypertonie, pulmonale Hypertonie, Transplantationsischämie, Reperfusionsschäden, Schlaganfall und Makuladegeneration sowie zur Wiedergewinnung der Nervenfunktion nach traumatischer Schädigung oder Durchtrennung hilfreich sein.

Da HIF einer der Faktoren ist, welche den Übergang von einem epithelialen zu einem mesenchymalen Zelltyp steuern, was im Speziellen für die Lunge und die Niere von Bedeutung ist, können die erfindungsgemäßen Verbindungen auch eingesetzt werden, um mit HIF assoziierte Fibrosen von Lunge und Niere zu verhindern oder einzudämmen.

Weitere Erkrankungen, zu deren Behandlung die erfindungsgemäßen Verbindungen verwendet werden können, sind entzündliche Gelenkerkrankungen, wie verschiedene Formen der Arthritis, sowie entzündliche Darmerkrankungen, wie beispielsweise Morbus Crohn.

Die Chugwash-Polyzythämie wird durch HIF-2α-Aktivität während der Erythropoese unter anderem in der Milz vermittelt. Die erfindungsgemäßen Verbindungen, als Hemmstoffe des HIF-Regulationsweges, sind daher auch geeignet, hier die exzessive Erythrozytenbildung zu unterdrücken und damit die Auswirkungen dieser Erkrankung zu mildern.

Die Verbindungen der vorliegenden Erfindung können ferner verwendet werden zur Behandlung von Erkrankungen, die mit exzessiver oder anormaler Angiogenese verbunden sind. Dazu gehören unter anderem diabetische Retinopathie, ischämische Retinalvenenocclusion und Retinopathie bei Frühgeburt (vgl. Aiello *et al.,* 1994; Peer *et al.,* 1995), altersabhängige Makuladegeneration (AMD; vgl. Lopez *et al.,* 1996), neovaskuläres Glaukom, Psoriasis, retrolentale Fibroplasie, Angiofibrom, Entzündung, rheumatische Arthritis (RA), Restenose, *in-stent*-Restenose sowie Restenose nach Gefäßimplantation.

Eine gesteigerte Blutversorgung ist außerdem mit kanzerösem, neoplastischem Gewebe assoziiert und führt hier zu einem beschleunigten Tumorwachstum. Zudem erleichtert das Wachstum neuer Blut- und Lymphgefäße die Bildung von Metastasen und damit die Verbreitung des Tumors. Neue Lymph- und Blutgefäße sind auch schädlich für Allografts in immunprivilegierten Geweben, wie dem Auge, was zum Beispiel die Anfälligkeit für Abstoßungsreaktionen erhöht. Verbindungen der vorliegenden Erfindung können daher auch eingesetzt werden, um eine der vorgenannten Erkrankungen zu therapieren, z.B. durch eine Hemmung des Wachstums oder eine Verringerung der Anzahl von Blutgefäßen. Dies kann über eine Hemmung der Endothelzellproliferation oder andere Mechanismen zur Verhinderung oder Abschwächung der Gefäßbildung und über eine Reduktion von neoplastischen Zellen durch Apoptose erreicht werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist deshalb besonders angezeigt, da hypoxische Regionen eines Tumors nur wenig auf die genannten konventionellen Therapien ansprechen, wohingegen die Verbindungen der vorliegenden Erfindung insbesondere dort ihre Aktivität entfalten.

Als geeignete Kombinationswirkstoffe seien beispielhaft genannt:

Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyhamstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, IntronA, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, MitomycinC, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, BAY 43-9006 (Sorafenib), CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnas, 13-*cis*-Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agentien kombiniert werden, welche beispielhaft - ohne dass diese Aufzählung abschließend wäre - sein können:

Aminoglutethimid, L-Asparaginase, Azathioprin, 5-Azacytidin, Bleomycin, Busulfan, Camptothecin, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, *N*-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) und rekombinanten Proteinen kombinieren, welche additiv oder synergistisch die Effekte der Hemmung der HIF-Signalwegsübertragung verstärken.

Inhibitoren des HIF-Regulationsweges wie die erfindungsgemäßen Verbindungen können auch in Kombination mit anderen, gegen die Angiogenese gerichteten Therapien positive Effekte erzielen, wie zum Beispiel mit Avastin, Axitinib, DAST, Recentin, Sorafenib oder Sunitinib. Kombinationen mit Inhibitoren des Proteasoms und von mTOR sowie Antihormone und steroidale metabolische Enzyminhibitoren sind wegen ihres günstigen Nebenwirkungsprofils besonders geeignet.

Generell können mit der Kombination von Verbindungen der vorliegenden Erfindung mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Verbindung mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- aq.: wässrig
- Boc: *tert*.-Butoxycarbonyl
- Bsp.: Beispiel
- Bu: Butyl
- ca.: *circa,* ungefähr
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N,N*-Dimethylaminopyridin
- DME: 1,2-Dimethoxyethan
- DMF: Dimethylformamid
- DMSO: Dimethylsulfoxid
- dt: Dublett von Triplett (bei NMR)
- d. Th.: der Theorie (bei chemischer Ausbeute)
- EDC: *N*'-(3-Dimethylaminopropyl)-*N*-ethylcarbodiimid-Hydrochlorid
- ee: Enantiomerenüberschuss
- EI: Elektronenstoß-Ionisation (bei MS)
- eq.: Äquivalent(e)
- ESI: Elektrospray-Ionisation (bei MS)
- Et: Ethyl
- GC: Gaschromatographie
- h: Stunde(n)
- HOBt: 1-Hydroxy-1*H-*benzotriazol-Hydrat
- HPLC: Hochdruck-, Hochleistungsflüssigchromatographie
- ⁱPr: Isopropyl
- LC-MS: Flüssigchromatographie-gekoppelte Massenspektrometrie
- m: Multiplett (bei NMR)
- min: Minute(n)
- MPLC: Mitteldruckflüssigchromatographie (über Kieselgel; auch "flashChromatographie" genannt)
- MS: Massenspektrometrie
- NMP: *N*-Methyl-2-pyrrolidon
- NMR: Kemresonanzspektrometrie
- Pd/C: Palladium auf Aktivkohle
- PEG: Polyethylenglykol
- Pr: Propyl
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- sept: Septett (bei NMR)
- t: Triplett (bei NMR)
- ^{t}Bu: *tert*.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- zus.: zusammen

### HPLC-Methoden:

### Methode A

Instrument: HP 1100 mit DAD-Detektion; Säule: Kromasil 100 RP-18, 60 mm x 2.1 mm, 3.5 µm; Eluent A: 5 ml Perchlorsäure (70%-ig) / L Wasser, Eluent B: Acetonitril; Gradient: 0 min 2% B→ 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode B

Instrument: HP 1100 mit DAD-Detektion; Säule: Kromasil 100 RP-18, 60 mm x 2.1 mm, 3.5 µm; Eluent A: 5 ml Perchlorsäure (70%-ig) / L Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### LC/MS-Methoden:

### Methode C

Gerätetyp MS: Micromass ZQ; Gerätetyp HPLC: HP 1100 Series; UV DAD; Säule: Phenomenex Gemini 3µ, 30 mm x 3.00 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode D

Gerätetyp MS: Waters Micromass Quattro Micro; Gerätetyp HPLC: Agilent 1100 Serie; Säule: Thermo Hypersil GOLD 3µ, 20 mm x 4 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 100% A → 3.0 min 10% A → 4.0 min 10% A → 4.01 min 100% A → 5.00 min 100% A; Ofen: 50°C; Fluss: 2 ml/min; UV-Detektion: 210 nm.

### Methode E

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode F

Instrument: Micromass Quattro Premier mit Waters UPLC Acquity; Säule: Thermo Hypersil GOLD 1.9µ, 50 mm x 1 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode G

Instrument: Micromass Platform LCZ mit HPLC Agilent Serie 1100; Säule: Thermo Hypersil GOLD 3µ, 20 mm x 4 mm; Eluent A: 1 L Wasser + 0.5 ml 50%-ige Ameisensäure, Eluent B: 1 L

Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode H

Instrument: Micromass Quattro LCZ mit HPLC Agilent Serie 1100; Säule: Phenomenex Synergi 2.5µ MAX-RP 100A Mercury 20 mm x 4 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Methode I

Instrument: Waters Acquity SQD UPLC System; Säule: Waters Acquity UPLC HSS T3 1.8 µm, 50 mm x 1 mm; Eluent A: 1 L Wasser + 0.25 ml 99%-ige Ameisensäure, Eluent B: 1 L Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode J

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode K

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98% A → 0.9 min 25% A → 1.0 min 5% A → 1.4 min 5% A → 1.41 min 98% A → 1.5 min 98% A; Ofen: 40°C; Fluss: 0.60 ml/min; UV-Detektion: DAD, 210 nm.

### GC/MS-Methoden:

### Methode L

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode M

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (12 min halten).

### präparative HPLC-Methoden:

### Methode N

Säule: GROM-SIL 120 ODS-4 HE, 10 µm, 250 mm x 30 mm; Laufmittel: und Gradientenprogramm: Acetonitril/0.1% aq. Ameisensäure 10:90 (0-3 min), Acetonitril/0.1% aq. Ameisensäure 10:90 → 95:5 (3-27 min), Acetonitril/0.1 % aq. Ameisensäure 95:5 (27-34 min), Acetonitril/ 0.1% aq. Ameisensäure 10:90 (34-38 min); Fluss: 50 ml/min; Temperatur: 22°C; UV-Detektion: 254 nm.

### Methode O

Säule: Reprosil C18, 10 µm, 250 mm x 30 mm; Laufmittel und Gradientenprogramm: Acetonitril/ 0.1% aq. Trifluoressigsäure 10:90 (0-2 min), Acetonitril/0.1% aq. Trifluoressigsäure 10:90 → 90:10 (2-23 min), Acetonitril/0.1% aq. Trifluoressigsäure 90:10 (23-28 min), Acetonitril/0.1% aq. Trifluoressigsäure 10:90 (28-30 min); Fluss: 50 ml/min; Temperatur: 22°C; UV-Detektion: 210 nm.

### Methode P

Säule: Reprosil C18, 10 µm, 250 mm x 30 mm; Laufmittel und Gradientenprogramm: Acetonitril/ 0.1% aq. Ammoniak 20:80 (0-3 min), Acetonitril/0.1% aq. Ammoniak 20:80 → 98:2 (3-35 min), Acetonitril/0.1% aq. Ammoniak 98:2 (35-40 min); Fluss: 50 ml/min; Temperatur: 22°C; UV-Detektion: 210 nm.

### LC/MS-Methode:

### Methode O

Gerätetyp MS: Waters ZQ; Gerätetyp HPLC: Agilent 1100 Serie; UV DAD; Säule: Thermo Hypersil GOLD 3µ, 20 mm x 4 mm; Eluent A: 1 L Wasser + 0.5 ml 50%-ige Ameisensäure, Eluent B: 1 L Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### N'-Hydroxy-4-(1,1,1-trifluor-2-methylpropan-2-yl)benzolcarboximidamid

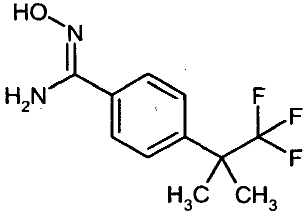

### Schritt 1: 2-(4-Bromphenyl)-1,1,1-trifluorpropan-2-ol

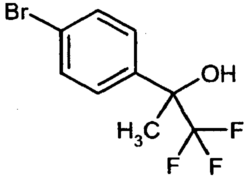

Zunächst wurde eine Suspension von Dichlor(dimethyl)titan in einem Heptan/Dichlormethan-Gemisch wie folgt hergestellt: Man kühlte 100 ml (100 mmol) einer 1 M Lösung von Titantetrachlorid in Dichlormethan auf -30°C, tropfte 100 ml (100 mmol) einer 1 M Lösung von Dimethylzink in Heptan hinzu und rührte 30 min bei -30°C nach. Anschließend wurde diese Suspension auf -40°C abgekühlt und eine Lösung von 10 g (39.5 mmol) 1-(4-Bromphenyl)-2,2,2-trifluorethanon in 50 ml Dichlormethan hinzugegeben. Man rührte 5 min bei -405°C nach, ließ dann die Temperatur auf RT kommen und rührte weitere 2 h bei RT. Unter Eiskühlung ließ man langsam 50 ml Wasser hinzutropfen und verdünnte anschließend mit weiteren 300 ml Wasser. Man extrahierte zweimal mit Dichlormethan, wusch die vereinigten Dichlormethan-Phasen einmal mit Wasser, trocknete über wasserfreiem Magnesiumsulfat, filtrierte und entfernte das Lösungsmittel am Rotationsverdampfer. Der Rückstand wurde säulenchromatographisch an Kieselgel gereinigt (Laufmittel:: Cyclohexan/Ethylacetat 85:15). Es wurden 10.5 g (100% d. Th.) der Titelverbindung erhalten, wobei laut ¹H-NMR noch Reste von Lösungsmittel enthalten waren.

¹H-NMR (400 MHz, CDCl₃, δ/ppm): 7.52 (d, 2H), 7.47 (d, 2H), 1.76 (s, 3H).

LC/MS (Methode C, ESIpos): Rₜ = 2.27 min, m/z = 268 [M+H]⁺.

### Schritt 2: 2-(4-Bromphenyl)-1,1,1-trifluorpropan-2-ylmethansulfonat

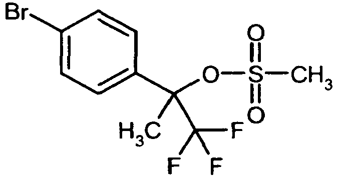

Man legte 3.12 g (78.05 mmol, 60%-ig in Mineralöl) Natriumhydrid in 45 ml THF unter Argon vor und tropfte eine Lösung von 10.5 g (39.03 mmol) der in Beispiel 1A / Schritt 1 erhaltenen Verbindung in 20 ml THF bei RT hinzu. Nachdem man 1 h bei RT und 30 min bei 40°C gerührt hatte, wurde eine Lösung von 8.94 g (78.05 mmol) Methansulfonylchlorid in 45 ml THF hinzugetropft und das Reaktionsgemisch weitere 60 min bei 40°C gerührt. Anschließend tropfte man langsam 50 ml Wasser zum Gemisch hinzu, verdünnte mit gesättigter wässriger Natriumhydrogencarbonat-Lösung und extrahierte zweimal mit Ethylacetat. Man trocknete die vereinigten Ethylacetat-Phasen über wasserfreiem Magnesiumsulfat, filtrierte und entfernte das Lösungsmittel am Rotationsverdampfer. Der Rückstand wurde in Hexan verrührt und der erhaltene Feststoff abfiltriert und im Vakuum getrocknet. Es wurden 12.4 g (92% d. Th.) der Titelverbindung erhalten.

¹H-NMR (400 MHz, CDCl₃, δ/ppm): 7.58 (d, 2H), 7.43 (d, 2H), 3.16 (s, 3H), 2.28 (s, 3H).

LC/MS (Methode D, ESIpos): Rₜ = 2.32 min, m/z = 364 [M+NH₄]⁺.

### Schritt 3: 1-Brom-4-(1,1,1-trifluor-2-methylpropan-2-yl)benzol

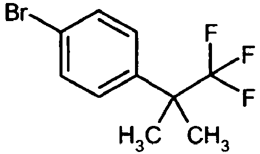

Man legte 12.4 g (35.72 mmol) der in Beispiel 1A / Schritt 2 erhaltenen Verbindung in 250 ml Dichlormethan vor und kühlte auf 0°C ab. Dann tropfte man langsam unter Rühren 35.7 ml (71.44 mmol) einer 2 M Lösung von Trimethylaluminium bei 0°C hinzu, ließ das Gemisch anschließend auf RT kommen und rührte weitere 1.5 h bei RT nach. Zu dem Gemisch tropfte man langsam 120 ml einer gesättigten wässrigen Natriumhydrogencarbonat-Lösung und danach 40 ml einer gesättigten wässrigen Natriumchlorid-Lösung hinzu. Man filtrierte über Kieselgur und wusch das Kieselgur zweimal mit Dichlormethan nach. Man wusch die vereinigten Dichlormethan-Phasen einmal mit gesättigter wässriger Natriumchlorid-Lösung, trocknete über wasserfreiem Magnesium-sulfat und entfernte das Lösungsmittel am Rotationsverdampfer. Es wurden 8.69 g (87% d. Th.) der Titelverbindung in 95%-iger Reinheit erhalten.

¹H-NMR (400 MHz, CDCl₃, δ/ppm): 7.49 (d, 2H), 7.33 (d, 2H), 1.55 (s, 6H).

LC/MS (Methode E, ESIpos): Rₜ = 2.54 min, keine Ionisierung.

GC/MS (Methode L, EI): Rₜ = 3.48 min, m/z = 266 [M]⁺.

### Schritt 4: 4-(1,1,1-Trifluor-2-methylpropan-2-yl)benzolcarbonitril

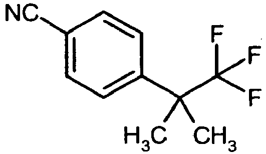

Man legte 3.34 g (12.50 mmol) der in Beispiel 1A / Schritt 3 erhaltenen Verbindung in 2.5 ml entgastem DMF unter Argon vor, gab 881 mg (7.50 mmol) Zinkcyanid sowie 867 mg (0.75 mmol) Tetrakis(triphenylphosphin)palladium(0) hinzu und rührte über Nacht bei 80°C. Nach Abkühlen auf RT verdünnte man das Reaktionsgemisch mit Ethylacetat und filtrierte feste Bestandteile ab. Das Filtrat wurde zweimal mit 2 N wässriger Ammoniak-Lösung und einmal mit gesättigter wässriger Natriumchlorid-Lösung gewaschen, über wasserfreiem Magnesiumsulfat getrocknet und am Rotationsverdampfer vom Lösungsmittel befreit. Der Rückstand wurde säulenchromatographisch an Kieselgel gereinigt (Laufmittel:: Cyclohexan/Ethylacetat 85:15). Es wurden 2.08 g (78% d. Th.) der Titelverbindung erhalten.

¹H-NMR (400 MHz, CDCl₃, δ/ppm): 7.68 (d, 2H), 7.62 (d, 2H), 1.60 (s, 6H).

GC/MS (Methode L, EI): Rₜ = 3.83 min, m/z = 213 [M]⁺.

### Schritt 5: N'-Hydroxy-4-(1,1,1-trifluor-2-methylpropan-2-yl)benzolcarboximidamid

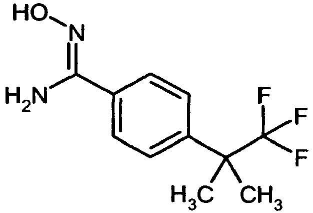

Ein Gemisch aus 2.40 g (11.26 mmol) der Verbindung aus Beispiel 1A / Schritt 4, 1.72 g (24.77 mmol) Hydroxylamin-Hydrochlorid und 3.45 ml (24.77 mmol) Triethylamin in 60 ml Ethanol wurde 1 h unter Rückfluss gerührt. Nach Abkühlen auf RT wurde das Lösungsmittel am Rotationsverdampfer entfernt. Man versetzte den Rückstand mit Ethylacetat und filtrierte den vorhandenen Feststoff ab. Die Ethylacetat-Lösung wurde nacheinander mit Wasser und gesättigter wässriger Natriumchlorid-Lösung gewaschen, über wasserfreiem Magnesiumsulfat getrocknet und filtriert. Nach Entfernen des Lösungsmittels wurde das erhaltene Öl mit Petrolether verrieben. Nach Absaugen des resultierenden Feststoffs und Trocknen im Hochvakuum wurden 2.65 g (96% d. Th.) der Titelverbindung erhalten.

¹H-NMR (400 MHz, CDCl₃, δ/ppm): 8.0 (s, breit, 1H), 7.62 (d, 2H), 7.52 (d, 2H), 4.88 (s, breit, 2H), 1.60 (s, 6H).

LC/MS (Methode D, ESIpos): Rₜ = 1.34 min, m/z = 247 [M+H]⁺.

### Beispiel 2A

### 4-(2-Fluorpropan-2-yl)-N'-hydroxybenzolcarboximidamid

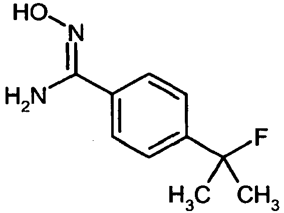

### Schritt 1: 4-(2-Fluorpropan-2-yl)benzolcarbonitril

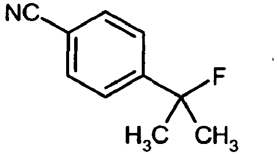

Zu einer Lösung von 1.00 g (6.20 mmol) 4-(2-Hydroxypropan-2-yl)benzolcarbonitril [erhalten aus 4-(Propan-2-yl)benzolcarbonitril gemäß J.L. Tucker et al., Synth. Comm. 2006, 36 (15), 2145-2155] in 20 ml Dichlormethan wurden 1.20 g (7.44 mmol) Diethylaminoschwefeltrifluorid (DAST) bei einer Temperatur von 0°C gegeben. Das Reaktionsgemisch wurde 2 h bei RT gerührt und danach mit Wasser verdünnt und mit Dichlormethan extrahiert. Die organische Phase wurde mit Wasser gewaschen, über wasserfreiem Magnesiumsulfat getrocknet und filtriert. Nach Entfernen des Lösungsmittels am Rotationsverdampfer wurde der Rückstand mittels MPLC gereinigt (Kieselgel, Laufmittel: Cyclohexan/Ethylacetat 95:5). Es wurden 675 mg (67% d. Th.) der Titelverbindung erhalten.

¹H-NMR (400 MHz, CDCl₃, δ/ppm): 7.57 (d, 2H), 7.48 (d, 2H), 1.72 (s, 3H), 1.68 (s, 3H).

LC/MS (Methode D, ESIpos): Rₜ = 2.12 min, m/z = 163 [M+H]⁺.

### Schritt 2: 4-(2-Fluorpropan-2-yl)-N'-hydroxybenzolcarboximidamid

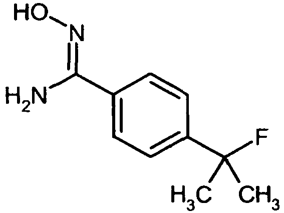

Nach dem unter Beispiel 1A / Schritt 5 beschriebenen Verfahren wurden aus 675 mg (4.14 mmol) der Verbindung aus Beispiel 2A / Schritt 1 756 mg (93% d. Th.) der Titelverbindung erhalten.

¹H-NMR (400 MHz, CDCl₃, δ/ppm): 7.62 (d, 2H), 7.41 (d, 2H), 4.89 (s, breit, 2H), 1.72 (s, 3H), 1.68 (s, 3H).

LC/MS (Methode D, ESIpos): Rₜ = 1.04 min, m/z = 197 [M+H]⁺.

### Beispiel 3A

### N'-Hydroxy-4-[(trifluormethyl)sulfonyl]benzolcarboximidamid

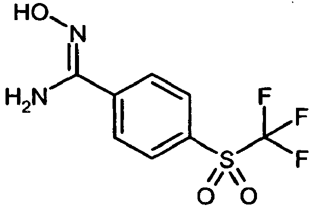

Nach dem unter Beispiel 1A / Schritt 5 beschriebenen Verfahren wurden aus 4.60 g (19.56 mmol) 4-[(Trifluormethyl)sulfonyl]benzolcarbonitril [W. Su, Tetrahedron. Lett. 1994, 35 (28), 4955-4958] 5.08 g (97% d. Th.) der Titelverbindung erhalten.

¹H-NMR (400 MHz, DMSO-d₆, δ/ppm): 10.26 (s, 1H), 8.13 (dd, 4H), 6.12 (s, 2H).

LC/MS (Methode D, ESIpos): Rₜ = 1.57 min, m/z = 269 [M+H]⁺.

### Beispiel 4A

### N'-Hydroxy-4-(3-methyloxetan-3-yl)benzolcarboximidamid

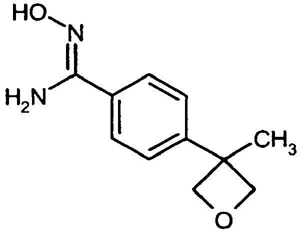

### Schritt 1: [4-(Dibenzylamino)phenyl]boronsäure

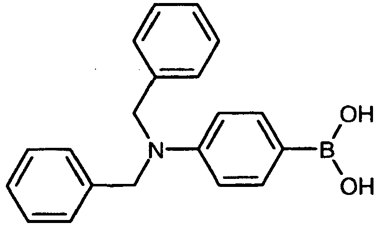

Unter inerten Bedingungen wurde eine Lösung von 6.0 g (17.03 mmol) *N,N*-Dibenzyl-4-bromanilin [T. Saitoh et al., J. Am. Chem. Soc. 2005, 127 (27), 9696-9697] in einem Gemisch aus 75 ml wasserfreiem Diethylether und 75 ml wasserfreiem THF vorgelegt. Bei -78°C wurde diese Lösung tropfenweise mit 13.9 ml (22.14 mmol) einer 1.6 M Lösung von n-Butyllithium in Hexan versetzt. Nach beendeter Zugabe wurde 60 min bei -78°C gerührt, bevor bei derselben Temperatur 6.3 ml (27.25 mol) Borsäuretriisopropylester zugetropft wurden. Nach weiteren 15 min bei -78°C ließ man das Reaktionsgemisch auf RT kommen. Nach 3 h Rühren bei RT wurden 18 ml 2 M Salzsäure hinzugefügt und das resultierende Gemisch 20 min intensiv bei RT gerührt. Nach Verdünnen mit ca. 200 ml Wasser wurde dreimal mit je ca. 200 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Der erhaltene ölige Rückstand wurde mit einem Gemisch aus 50 ml *tert*.-Butylmethylether und 50 ml Pentan verrieben. Nach Absaugen des resultierenden Feststoffs und Trocknen im Hochvakuum wurden 3.91 g (72% d. Th., 90% Reinheit) der Titelverbindung erhalten, welche ohne weitere Reinigung in der nächsten Stufe eingesetzt wurde.

¹H-NMR (400 MHz, DMSO-d₆, δ/ppm): 7.58 (d, 2H), 7.32-7.30 (m, 4H), 7.27-7.23 (m, 6H), 6.66 (d, 2H), 4.70 (s, 4H).

HPLC (Methode A): Rₜ = 4.35 min.

MS (ESIpos): m/z = 318 [M+H]⁺.

### Schritt 2: Ethyl-{3-[4-(dibenzylamino)phenyl]oxetan-3-yl}acetat

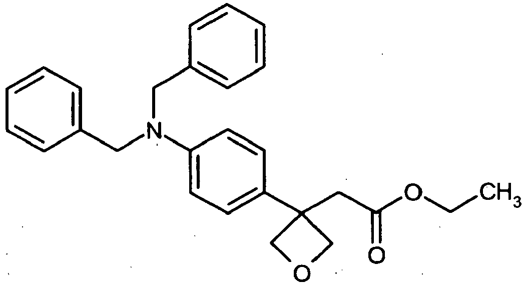

Eine Lösung von 304 mg (0.616 mmol) (1,5-Cyclooctadien)rhodium(I)chlorid-Dimer in 30 ml 1,4-Dioxan wurde mit 10.7 ml (16.0 mmol) einer 1.5 M Kalilauge versetzt. Nacheinander wurden dann Lösungen von 1.75 g (12.31 mmol) Ethyl-oxetan-3-ylidenacetat [G. Wuitschik et al., Angew. Chem. Int. Ed. Engl. 2006, 45 (46), 7736-7739] in 1 ml 1,4-Dioxan und 3.91 g (12.31 mmol) der Verbindung aus Beispiel 4A / Schritt 1 in 60 ml 1,4-Dioxan hinzugefügt. Das Reaktionsgemisch wurde 6 h bei RT gerührt. Anschließend wurde mit ca. 200 ml Wasser verdünnt und dreimal mit je ca. 200 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde mittels MPLC gereinigt (Kieselgel, Laufmittel: Cyclohexan/Ethylacetat 20:1 → 5:1). Es wurden 3.51 g (67% d. Th.) der Titelverbindung erhalten.

¹H-NMR (400 MHz, CDCl₃, δ/ppm): 7.33-7.30 (m, 4H), 7.27-7.23 (m, 6H), 6.97 (d, 2H), 6.69 (d, 2H), 4.94 (d, 2H), 4.81 (d, 2H), 4.62 (s, 4H), 4.00 (quart, 2H), 3.04 (s, 2H), 1.11 (t, 3H).

LC/MS (Methode E, ESIpos): Rₜ = 2.57 min, m/z = 416 [M+H]⁺.

### Schritt 3: 2-{3-[4-(Dibenzylamino)phenyl]oxetan-3-yl}ethanol

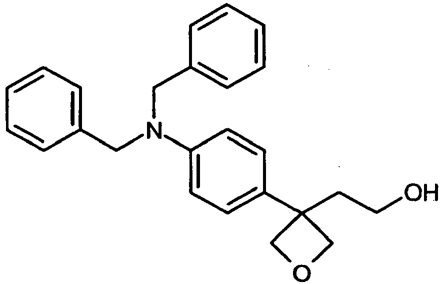

Unter inerten Bedingungen und bei einer Temperatur von 0°C wurde eine Lösung von 2.90 g (6.98 mmol) der Verbindung aus Beispiel 4A / Schritt 2 in 145 ml wasserfreiem THF tropfenweise mit 4.9 ml (4.88 mmol) einer 1 M Lösung von Lithiumaluminiumhydrid in THF versetzt. Nach beendetem Zutropfen wurde das Reaktionsgemisch 1.5 h bei 0°C gerührt. Anschließend wurden 2 g Kieselgur und 2 ml Wasser vorsichtig hinzugefügt. Das heterogene Gemisch wurde über ein Papierfilter abgesaugt. Das Filtrat wurde mit ca. 250 ml Wasser verdünnt und dreimal mit je ca. 250 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde mittels MPLC gereinigt (Kieselgel, Laufmittel: Cyclohexan/Ethylacetat 4:1). Es wurden 2.34 g (87% d. Th.) der Titelverbindung erhalten.

¹H-NMR (400 MHz, CDCl₃, δ/ppm): 7.36-7.31 (m, 4H), 7.27-7.22 (m, 6H), 6.88 (d, 2H), 6.71 (d, 2H), 4.93 (d, 2H), 4.71 (d, 2H), 4.63 (s, 4H), 3.55 (quart, 2H), 2.29 (t, 2H), 1.12 (t, 1H).

HPLC (Methode B): Rₜ = 3.98 min.

MS (DCI, NH₃): m/z = 374 [M+H]⁺.

LC/MS (Methode E, ESIpos): Rₜ = 2.15 min, m/z = 374 [M+H]⁺.

### Schritt 4: {3-[4-(Dibenzylamino)phenyl]oxetan-3-yl}acetaldehyd

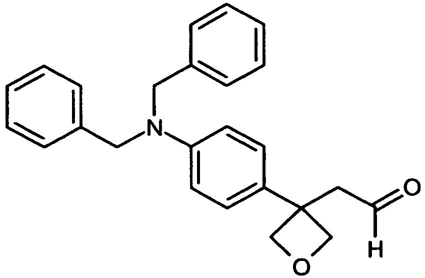

Unter inerten Bedingungen wurde eine Lösung von 496 µl (5.68 mmol) Oxalylchlorid in 5 ml wasserfreiem Dichlormethan bei -78°C tropfenweise mit 807 µl wasserfreiem DMSO versetzt. Nach 20 min wurde bei derselben Temperatur eine Lösung von 1.93 g (5.17 mmol) der Verbindung aus Beispiel 4A / Schritt 3 in 5 ml wasserfreiem Dichlormethan langsam zugetropft. Nach 60 min Rühren bei -78°C wurden 3.7 ml (26.87 mmol) wasserfreies Triethylamin zugetropft. Nach weiteren 10 min bei dieser Temperatur ließ man das Reaktionsgemisch auf RT erwärmen. Anschließend wurde das Gemisch in einen mit Kieselgel gefüllten Saugfilter gegeben, und es wurde zunächst mit Cyclohexan und dann mit Cyclohexan/Ethylacetat 7:1 → 1:1 eluiert. Die Produktfraktionen wurden vereinigt, zur Trockene eingedampft und der Rückstand in Ethylacetat aufgenommen. Es wurde nacheinander mit gesättigter Natriumhydrogencarbonat-Lösung, Wasser und gesättigter Kochsalz-Lösung gewaschen. Nach Trocknen über wasserfreiem Magnesiumsulfat wurde filtriert und das Lösungsmittel am Rotationsverdampfer entfernt. Es wurden 1.81 g (92% d. Th.) der Titelverbindung erhalten.

¹H-NMR (400 MHz, CDCl₃, δ/ppm): 9.69 (t, 1H), 7.34-7.31 (m, 4H), 7.28-7.23 (m, 6H), 6.97 (d, 2H), 6.70 (d, 2H), 5.00 (d, 2H), 4.72 (d, 2H), 4.63 (s, 4H), 3.18 (d, 2H).

HPLC (Methode B): Rₜ = 4.61 min.

MS (DCI, NH₃): m/z = 372 [M+H]⁺.

LC/MS (Methode F, ESIpos): Rₜ = 1.43 min, m/z = 372 [M+H]⁺.

### Schritt 5: N,N-Dibenzyl-4-(3-methyloxetan-3-yl)anilin

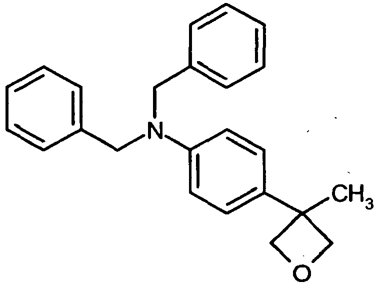

Unter inerten Bedingungen wurde eine Lösung von 1.81 g (4.87 mmol) der Verbindung aus Beispiel 4A / Schritt 4 und 13.57 g (14.62 mmol) Tris(triphenylphosphin)rhodium(I)chlorid in 240 ml Toluol eine Stunde unter Rückfluss erhitzt. Nach dem Abkühlen auf RT wurde von unlöslichen Bestandteilen abfiltriert. Das Lösungsmittel wurde am Rotationsverdampfer entfernt und der Rückstand mittels MPLC gereinigt (Kieselgel, Cyclohexan/Ethylacetat 20:1 → 5:1). Es wurden 1.36 g (73% d. Th., ca. 90% Reinheit) der Titelverbindung erhalten.

¹H-NMR (400 MHz, CDCl₃, δ/ppm): 7.35-7.31 (m, 4H), 7.27-7.24 (m, 6H), 7.07 (d, 2H), 6.72 (d, 2H), 4.90 (d, 2H), 4.64 (s, 4H), 4.55 (d, 2H), 1.96 (s, 3H).

LC/MS (Methode F, ESIpos): Rₜ = 1.55 min, m/z = 344 [M+H]⁺.

### Schritt 6: 4-(3-Methyloxetan-3-yl)anilin

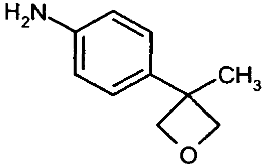

In einer Durchfluss-Hydrierapparatur ("H-Cube" der Firma ThalesNano, Budapest, Ungarn) wurde eine Lösung von 1.35 g (3.93 mmol) der Verbindung aus Beispiel 4A / Schritt 5 in 135 ml Ethanol hydriert (Bedingungen: 10% Pd/C-Katalysator, "full H₂"-Modus, 1 ml/min, 50°C). Nach Entfernen des Lösungsmittels am Rotationsverdampfer wurde das Rohprodukt mittels MPLC gereinigt (Kieselgel, Cyclohexan/Ethylacetat 4:1 → 2:1). Es wurden 386 mg (60% d. Th.) der Titelverbindung erhalten.

¹H-NMR (400 MHz, CDCl₃, δ/ppm): 7.03 (d, 2H), 6.69 (d, 2H), 4.92 (d, 2H), 4.58 (d, 2H), 3.63 (s, breit, 2H), 1.69 (s, 3H).

LC/MS (Methode D, ESIpos): Rₜ = 0.77 min, m/z = 164 [M+H]⁺.

### Schritt 7: 4-(3-Methyloxetan-3-yl)benzolcarbonitril

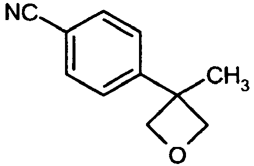

Eine Lösung von 375 mg (2.30 mmol) der Verbindung aus Beispiel 4A / Schritt 6 in 17 ml Wasser wurde bei 0°C zunächst mit 1.7 ml (20.7 mmol) konzentrierter Salzsäure und dann tropfenweise mit einer Lösung von 159 mg (2.30 mmol) Natriumnitrit in 5 ml Wasser versetzt. Es wurde 30 min bei 0°C gerührt, bevor 1.1 g (10.3 mmol) festes Natriumcarbonat portionsweise zugesetzt wurden. Die so erhaltene Lösung wurde bei 0°C zu einer Lösung von 257 mg (2.87 mmol) Kupfer(I)cyanid und 464 mg (7.12 mmol) Kaliumcyanid in 16 ml Toluol/Wasser (2:1) getropft. Das Reaktionsgemisch wurde 1 h bei 0°C gerührt. Anschließend ließ man das Gemisch auf RT erwärmen. Die organische Phase wurde danach abgetrennt und nacheinander mit Wasser und gesättigter Kochsalz-Lösung gewaschen. Nachdem das Lösungsmittel am Rotationsverdampfer abgetrennt worden war, wurde das Rohprodukt mittels MPLC gereinigt (Kieselgel, Cyclohexan/Ethylacetat 10:1 → 2:1). Es wurden 390 mg (83% d. Th., 84% Reinheit) der Titelverbindung erhalten.

¹H-NMR (400 MHz, CDCl₃, δ/ppm): 7.66 (d, 2H), 7.31 (d, 2H), 4.92 (d, 2H), 4.68 (d, 2H), 1.73 (s, 3H).

GC/MS (Methode L, EIpos): Rₜ = 5.45 min, m/z = 173 (M)⁺.

### Schritt 8: N'-Hydroxy-4-(3-methyloxetan-3-yl)benzolcarboximidamid

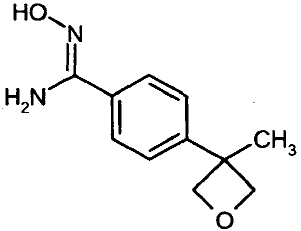

Nach dem unter Beispiel 1A / Schritt 5 beschriebenen Verfahren wurden aus 375 mg (1.83 mmol) der Verbindung aus Beispiel 4A / Schritt 7 297 mg (74% d. Th.) der Titelverbindung erhalten.

¹H-NMR (400 MHz, DMSO-d₆, δ/ppm): 9.59 (s, 1H), 7.64 (d, 2H), 7.23 (d, 2H), 5.79 (s, breit, 2H), 4.80 (d, 2H), 4.53 (d, 2H), 1.62 (s, 3H).

HPLC (Methode A): Rₜ = 2.74 min.

MS (DCI, NH₃): m/z = 207 [M+H]⁺.

### Beispiel 5A

### 4-(3-Fluoroxetan-3-yl)-N'-hydroxybenzolcarboximidamid

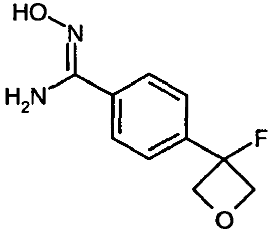

### Schritt 1: 4-(3-Hydroxyoxetan-3-yl)benzolcarbonitril

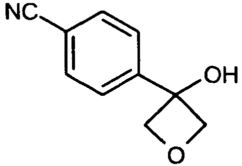

Unter inerten Bedingungen wurde bei -40°C eine Lösung von 5.0 g (21.8 mmol) 4-Iodbenzonitril in 100 ml wasserfreiem THF tropfenweise mit 11 ml (21.8 mmol) einer 2 M Lösung von Isopropylmagnesiumchlorid in Diethylether versetzt. Nachdem das Gemisch 1.5 h bei derselben Temperatur gerührt worden war, wurde es auf -78°C heruntergekühlt und mit Hilfe einer Kanüle zu einer ebenfalls auf -78°C gekühlten Lösung von 2.95 g (32.7 mmol, 80% in Dichlormethan) 3-Oxooxetan [G. Wuitschik et al., Angew. Chem. Int. Ed. Engl. 2006, 45 (46), 7736-7739] in 100 ml wasserfreiem THF langsam hinzugefügt. Nach beendeter Zugabe wurde das Reaktionsgemisch zunächst 10 min bei -78°C, dann 2 h bei 0°C und schließlich 30 min bei RT gerührt. Es wurde dann mit einigen ml gesättigter wässriger Ammoniumchlorid-Lösung versetzt. Anschließend wurde das Lösungsmittel am Rotationsverdampfer weitgehend entfernt. Der erhaltene Rückstand wurde mit 200 ml Wasser verdünnt und dreimal mit je ca. 200 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde durch Kristallisation aus Cyclohexan/Ethylacetat 10:1 gereinigt. Es wurden 2.42 g (63% d. Th.) der Titelverbindung erhalten.

¹H-NMR (400 MHz, DMSO-d₆, δ/ppm): 7.88 (d, 2H), 7.80 (d, 2H), 6.63 (s, 1H), 4.79 (d, 2H), 4.65 (d, 2H).

HPLC (Methode A): Rₜ = 3.09 min.

MS (DCI, NH₃): m/z = 193 [M+NH₄]⁺.

### Schritt 2: 4-(3-Fluoroxetan-3-yl)benzolcarbonitril

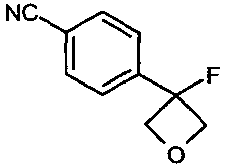

Unter inerten Bedingungen wurde bei -78°C eine Suspension von 600 mg (3.43 mmol) der Verbindung aus Beispiel 5A / Schritt 1 in 55 ml Dichlormethan tropfenweise mit einer Lösung von 662 mg (4.11 mmol) Diethylaminoschwefeltrifluorid (DAST) in 5 ml Dichlormethan versetzt. Nach 30 min bei -78°C wurde das Reaktionsgemisch mit Hilfe eines Eis/Wasser-Bades sehr schnell auf -20°C erwärmt. Nach ca. 30 Sekunden wurden 20 ml 1 M Natronlauge zugesetzt, und man ließ das Gemisch auf RT erwärmen. Nach Verdünnen mit 150 ml Wasser wurde dreimal mit je ca. 50 ml Diethylether extrahiert. Die vereinigten organischen Extrakte wurden über wasserfreiem Magnesiumsulfat getrocknet. Nach Filtration wurde das Lösungsmittel am Rotationsverdampfer entfernt. Das Rohprodukt wurde mittels MPLC gereinigt (Kieselgel, Cyclohexan/Ethylacetat 8:1). Es wurden 495 mg (82% d. Th.) der Titelverbindung erhalten.

¹H-NMR (400 MHz, CDCl₃, δ/ppm): 7.76 (d, 2H), 7.73 (d, 2H), 5.15 (dd, 2H), 4.81 (dd, 2H).

LC/MS (Methode D, ESIpos): Rₜ = 1.59 min, m/z = 178 [M+H]⁺.

### Schritt 3: 4-(3-Fluoroxetan-3-yl)-N'-hydroxybenzolcarboximidamid

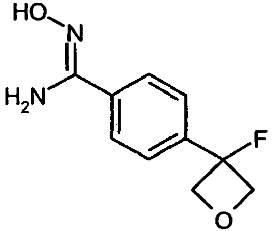

Nach dem unter Beispiel 1A / Schritt 5 beschriebenen Verfahren wurden aus 450 mg (2.54 mmol) der Verbindung aus Beispiel 5A / Schritt 2 470 mg (86% d. Th.) der Titelverbindung erhalten.

¹H-NMR (400 MHz, DMSO-d₆, δ/ppm): 9.71 (s, 1H), 7.77 (d, 2H), 7.54 (d, 2H), 5.87 (breites s, 2H), 4.97 (dd, 2H), 4.91 (dd, 2H).

HPLC (Methode A): Rₜ = 2.64 min.

MS (DCI, NH₃): m/z = 211 [M+H]⁺.

LC/MS (Methode D, ESIpos): Rₜ = 0.80 min, m/z = 211 [M+H]⁺.

### Beispiel 6A

### N'-Hydroxy-4-(3-methoxyoxetan-3-yl)benzolcarboximidamid

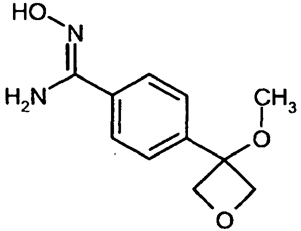

### Schritt 1: 4-(3-Methoxyoxetan-3-yl)benzolcarbonitril

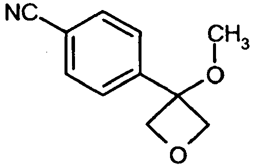

Eine Lösung von 600 mg (3.43 mmol) der Verbindung aus Beispiel 5A / Schritt 1 in 12.5 ml wasserfreiem DMF wurde bei 5°C mit 151 mg (3.77 mmol) einer 60%-igen Dispersion von Natrium-hydrid in Mineralöl versetzt. Das Gemisch wurde 1 h bei 5°C gerührt, bevor 256 µl (4.11 mmol) Methyliodid zugesetzt wurden. Man ließ das Reaktionsgemisch dann auf RT kommen. Nach 15 h Rühren wurden 150 ml Wasser zugesetzt, und es wurde zweimal mit je ca. 150 ml Diethylether extrahiert. Die vereinigten organischen Extrakte wurden über wasserfreiem Magnesiumsulfat getrocknet. Nach Filtration und Entfernen des Lösungsmittels am Rotationsverdampfer wurde der erhaltene Rückstand mittels MPLC gereinigt (Kieselgel, Cyclohexan/Ethylacetat 20:1 → 4:1). Es wurden 566 mg (87% d. Th.) der Titelverbindung erhalten.

¹H-NMR (400 MHz, DMSO-d₆, δ/ppm): 7.92 (d, 2H), 7.68 (d, 2H), 4.81 (d, 2H), 4.74 (d, 2H), 3.07 (s,3H).

HPLC (Methode A): Rₜ = 3.63 min.

MS (DCI, NH₃): m/z = 207 [M+NH₄]⁺.

LC/MS (Methode D, ESIpos): Rₜ = 1.50 min, m/z = 190 [M+H]⁺.

### Schritt 2: N'-Hydroxy-4-(3-methoxyoxetan-3-yl)benzolcarboximidamid

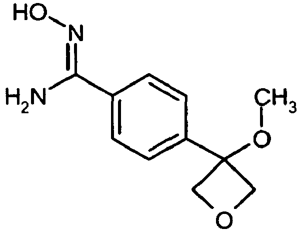

Nach dem unter Beispiel 1A / Schritt 5 beschriebenen Verfahren wurden aus 500 mg (2.64 mmol) der Verbindung aus Beispiel 6A / Schritt 1 520 mg (89% d. Th.) der Titelverbindung erhalten.

¹H-NMR (400 MHz, DMSO-d₆, δ/ppm): 9.67 (s, 1H), 7.73 (d, 2H), 7.43 (d, 2H), 5.83 (breites s, 2H), 4.77 (m, 4H), 3.03 (s, 3H).

HPLC (Methode A): Rₜ = 2.54 min.

MS (DCI, NH₃): m/z = 223 [M+H]⁺.

### Beispiel 7A

### 4-(4-Fluortetrahydro-2H-pyran-4-yl)-N'-hydroxybenzolcarboximidamid

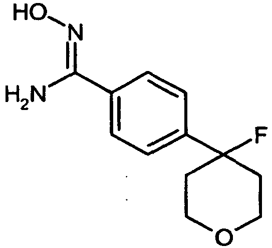

### Schritt 1: 4-(4-Hydroxytetrahydro-2H-pyran-4-yl)benzolcarbonitril

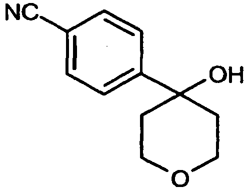

Nach dem unter Beispiel 5A / Schritt 1 beschriebenen Verfahren wurden 25.0 g (109 mmol) 4-Iodbenzonitril mit 16.4 g (164 mmol) Tetrahydro-4*H*-pyran-4-on zu 7.56 g (34% d. Th.) der Titelverbindung umgesetzt.

¹H-NMR (400 MHz, DMSO-d₆, δ/ppm): 7.80 (d, 2H), 7.70 (d, 2H), 5.30 (s, 1H), 3.81-3.70 (m, 4H), 2.02-1.94 (m, 2H), 1.51-1.48 (m, 2H).

HPLC (Methode A): Rₜ = 3.35 min.

MS (DCI, NH₃): m/z = 204 [M+H]⁺, 221 [M+NH₄]⁺.

### Schritt 2: 4-(4-Fluortetrahydro-2H-pyran-4-yl)benzolcarbonitril

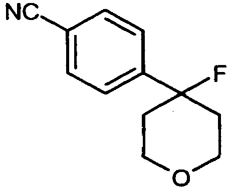

Nach dem unter Beispiel 5A / Schritt 2 beschriebenen Verfahren wurden 6.5 g (31.98 mmol) der Verbindung aus Beispiel 7A / Schritt 1 zu 3.73 g (57% d. Th.) der Titelverbindung umgesetzt.

¹H-NMR (400 MHz, CDCl₃, δ/ppm): 7.68 (d, 2H), 7.50 (d, 2H), 3.98-3.83 (m, 4H), 2.23-2.05 (m, 2H), 1.91-1.85 (m, 2H).

HPLC (Methode A): Rₜ = 4.04 min.

MS (DCI, NH₃): m/z = 223 [M+NH₄]⁺.

### Schritt 3: 4-(4-Fluortetrahydro-2H-pyran-4-yl)-N'-hydroxybenzolcarboximidamid

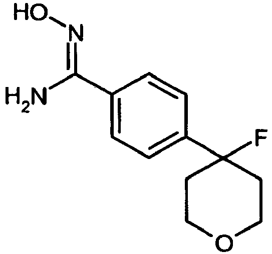

Nach dem unter Beispiel 1A / Schritt 5 beschriebenen Verfahren wurden aus 3.5 g (17.05 mmol) der Verbindung aus Beispiel 7A / Schritt 2 3.57 g (88% d. Th.) der Titelverbindung erhalten.

¹H-NMR (500 MHz, DMSO-d₆, δ/ppm): 9.64 (s, 1H), 7.70 (d, 2H), 7.44 (d, 2H), 5.81 (s, 2H), 3.88-3.83 (m, 2H), 3.73-3.67 (m, 2H), 2.23-2.06 (m, 2H), 1.87-1.81 (m, 2H).

HPLC (Methode A): Rₜ = 3.06 min.

MS (DCI, NH₃): m/z = 239 [M+H]⁺.

LC/MS (Methode F, ESIpos): Rₜ = 0.40 min, m/z = 239 [M+H]⁺.

### Beispiel 8A

### N'-Hydroxy-4-(4-methoxytetrahydro-2H-pyran-4-yl)benzolcarboximidamid

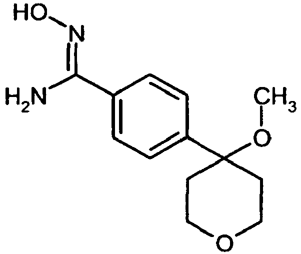

### Schritt 1: 4-(4-Methoxytetrahydro-2H-pyran-4-yl)benzolcarbonitril

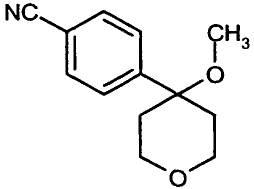

Nach dem unter Beispiel 6A / Schritt 1 beschriebenen Verfahren wurden aus 300 mg (1.48 mmol) der Verbindung aus Beispiel 7A / Schritt 1 und 111 µl (1.77 mmol) Methyliodid 238 mg (74% d. Th.) der Titelverbindung erhalten.

¹H-NMR (500 MHz, CDCl₃, δ/ppm): 7.68 (d, 2H), 7.51 (d, 2H), 3.89-3.82 (m, 4H), 2.99 (s, 3H), 2.03-1.98 (m, 2H), 1.94-1.91 (m, 2H).

HPLC (Methode A): Rₜ = 3.99 min.

MS (DCI, NH₃): m/z = 235 [M+NH₄]⁺.

GC/MS (Methode L, EIpos): Rₜ = 6.57 min, m/z = 217 (M)⁺.

### Schritt 2: N'-Hydroxy-4-(4-methoxytetrahydro-2H-pyran-4-yl)benzolcarboximidamid

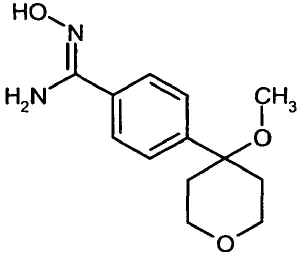

Nach dem unter Beispiel 1A / Schritt 5 beschriebenen Verfahren wurden aus 200 mg (0.921 mmol) der Verbindung aus Beispiel 8A / Schritt 1 229 mg (99% d. Th.) der Titelverbindung erhalten.

¹H-NMR (400 MHz, DMSO-d₆, δ/ppm): 9.63 (s, 1H), 7.68 (d, 2H), 7.39 (d, 2H), 5.80 (s, 2H), 3.71-3.67 (m, 4H), 2.88 (m, 2H), 1.93-1.89 (m, 4H).

HPLC (Methode B): Rₜ = 2.95 min.

MS (DCI, NH₃): m/z = 251 [M+H]⁺.

LC/MS (Methode D, ESIpos): Rₜ = 0.93 min, m/z = 251 [M+H]⁺.

Analog zu dem unter Beispiel 1A / Schritt 5 beschriebenen Verfahren wurden die in der folgenden Tabelle aufgeführten *N'*-Hydroxybenzolcarboximidamide aus den entsprechenden, kommerziell erhältlichen Benzonitrilen hergestellt. Die nicht kommerziell erhältlichen Benzonitrile wurden gemäß der folgenden Literaturvorschriften hergestellt: 4-Cyclohexylbenzolcarbonitril [E. Riguet et al., J. Organomet. Chem. 2001, 624 (1-2), 376-379], 4-(Piperidin-1-yl)benzolcarbonitril [A.-H. Kuthier et al., J. Org. Chem. 1987, 52 (9), 1710-1713], 4-(Pentafluor-λ⁶-sulfanyl)benzolcarbonitril [P.J. Crowley et al., Chimia 2004, 58 (3), 138-142].

| **Beispiel** | **Struktur** | **HPLC: Rₜ [min]** | **MS: m/z [M+H]⁺** | **LC/MS-Methode** |
|---|---|---|---|---|
| **9A** | 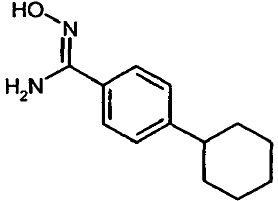 | 1.24 | 219 | H |
| | ¹H-NMR (400 MHz, DMSO-d₆, δ/ppm): 9.51 (s, 1H), 7.56 (d, 2H), 7.20 (d, 2H), 5.72 (s, breit, 2H), 2.52-2.48 (m, 1H), 1.81-1.74 (m, 4H), 1.73-1.67 (m, 1H), 1.45-1.31 (m, 4H), 1.28-1.19 (m, 1H). | | | |
| **10A** | 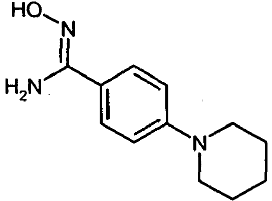 | 1.11 | 220 | D |
| | ¹H-NMR (400 MHz, CDCl₃, δ/ppm): 7.50 (d, 2H), 6.90 (d, 2H), 4.80 (s, breit, 2H), 3.23-3.20 (m, 4H), 1.71-1.65 (m, 4H), 1.63-1.57 (m, 2H). | | | |
| **11A** | 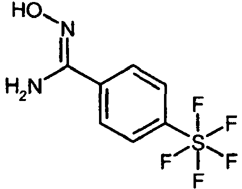 | 1.49 | 263 | D |
| | ¹H-NMR (400 MHz, DMSO-d₆, δ/ppm): 9.99 (s, 1H), 7.94-7.85 (m, 4H), 6.00 (s, 2H). | | | |
| **12A** | 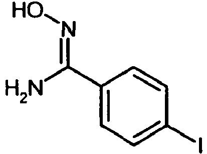 | 1.98 | 263 | G |
| | ¹H-NMR (400 MHz, DMSO-d₆, δ/ppm): 9.71 (s, 1H), 7.73 (d, 2H), 7.47 (d, 2H), 5.84 (s, breit, 2H). | | | |
| **13A** | 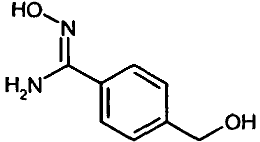 | 0.24 | 167 | D |
| | ¹H-NMR (400 MHz, DMSO-d₆, δ/ppm): 9.55 (s, 1H), 7.62 (d, 2H), 7.29 (d, 2H), 5.78 (s, 2H), 5.20 (t, 1H), 4.50 (d, 2H). | | | |
| **14A** | 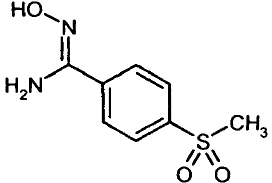 | 0.21 | 215 | F |
| | ¹H-NMR (400 MHz, DMSO-d₆, δ/ppm): 9.98 (s, 1H), 7.92 (s, 4H), 6.00 (s, breit, 2H), 3.23 (s, 3H). | | | |
| **15A** | 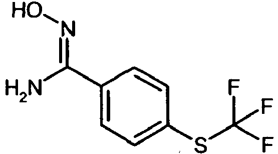 | 1.42 | 237 | D |
| | ¹H-NMR (400 MHz, DMSO-d₆, δ/ppm): 9.90 (s, 1H), 7.80 (d, 2H), 7.72 (d, 2H), 5.94 (s, 2H). | | | |
| **16A** | 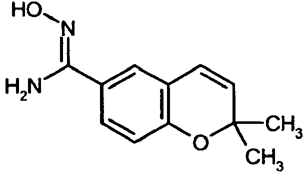 | 0.65 | 219 | F |
| | ¹H-NMR (400 MHz, CDCl₃, δ/ppm): 11.2 (sehr breit, 1H), 7.35 (dd, 1H), 7.26 (d, 1H), 6.78 (d, 1H), 6.31 (d, 1H), 5.63 (d, 1H), 4.82 (breit, 2H), 1.43 (s, 6H). | | | |
| **17A** | 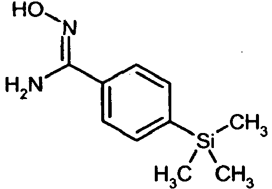 | 0.75 | 209 | I |
| | ¹H-NMR (400 MHz, CDCl₃, δ/ppm): 7.95 (s, breit, 1H), 7.60 (d, 2H), 7.55 (d, 2H), 4.86 (s, breit, 2H), 0.27 (s, 9H). | | | |
| **18A** | 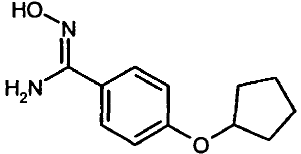 | 3.69 | 221 | A |
| | ¹H-NMR (400 MHz, DMSO-d₆, δ/ppm): 9.43 (s, 1H), 7.57 (d, 2H), 6.87 (d, 2H), 5.70 (s, breit, 2H), 4.84-4.81 (m, 1H), 1.97-1.88 (m, 2H), 1.73-1.66 (m, 4H), 1.62-1.53 (m, 2H). | | | |
| **19A** | 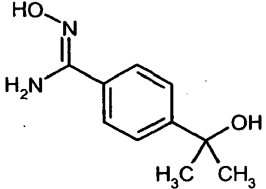 | 0.78 | 195 | D |
| | ¹H-NMR (400 MHz, DMSO-d₆, δ/ppm): 9.53 (s, 1H), 7.59 (d, 2H), 7.44 (d, 2H), 5.74 (s, breit, 2H), 5.02 (s, 1H), 1.41 (s, 6H). | | | |
| **20A** | 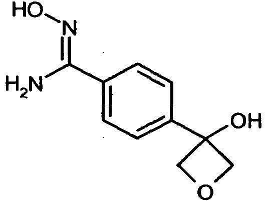 | 0.39 | 209 | D |
| | ¹H-NMR (400 MHz, DMSO-d₆, δ/ppm): 9.61 (s, 1H), 7.70 (d, 2H), 7.59 (d, 2H), 6.37 (s, 1H), 5.79 (s, breit, 2H), 4.76 (d, 2H), 4.68 (d, 2H). | | | |
| **21A** | 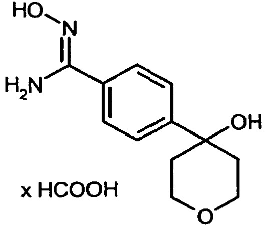 | 0.72 | 237 | D |
| | ¹H-NMR (400 MHz, DMSO-d₆, δ/ppm): 9.71 (breit, 1H), 9.59 (s, 1H), 7.62 (d, 2H), 7.48 (d, 2H), 7.17 (breit, 1H), 5.78 (s, breit, 2H), 5.06 (s, 1H), 3.78 (dd, 2H), 3.72-3.69 (m, 2H), 1.97 (dt, 2H), 1.52 (d, 2H). | | | |
| **22A** | 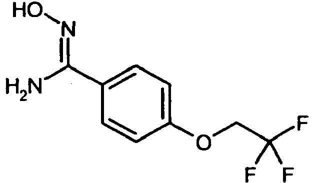 | 0.51 | 235 | I |
| | ¹H-NMR (400 MHz, DMSO-d₆, δ/ppm): 9.51 (s, 1H), 7.64 (d, 2H), 7.06 (d, 2H), 5.77 (s, breit, 2H), 4.79 (quart, 2H). | | | |
| **23A** | 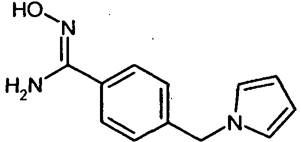 | 0.54 | 216 | I |
| | ¹H-NMR (400 MHz, CDCl₃, δ/ppm): 7.76 (breit, 1H), 7.58 (d, 2H), 7.13 (d, 2H), 6.68 (dd, 2H), 6.20 (dd, 2H), 5.09 (s, 2H), 4.84 (s, breit, 2H). | | | |

### Beispiel 24A

### N'-Hydroxy-4-(1-hydroxycyclobutyl)benzolcarboximidamid

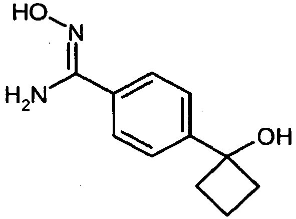

### Schritt 1: 4-(1-Hydroxycyclobutyl)benzolcarbonitril

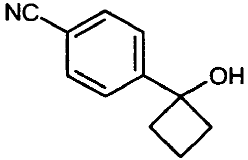

Analog zu dem unter Beispiel 5A / Schritt 1 beschriebenen Verfahren wurden aus 15.0 g (65.5 mmol) 4-Iodbenzonitril, 34.4 ml (68.8 mmol) Isopropylmagnesiumchlorid-Lösung (2 M in Diethylether) sowie 7.4 ml (98.2 mmol) Cyclobutanon 9.47 g (83% d. Th.) der Titelverbindung erhalten. Die Aufreinigung des Produktes erfolgte mittels MPLC (Kieselgel; Laufmittel: Cyclohexan/Ethylacetat 10:1 → 4:1).

¹H-NMR (400 MHz, CDCl₃, δ/ppm): 7.67 (d, 2H), 7.62 (d, 2H), 2.58-2.51 (m, 2H), 2.44-2.37 (m, 2H), 2.23-2.04 (m, 2H), 1.83-1.72 (m, 1H).

HPLC (Methode A): Rₜ = 3.47 min.

MS (DCI, NH₃): m/z = 191 [M+NH₄]⁺.

### Schritt 2: N'-Hydroxy-4-(1-hydroxycyclobutyl)benzolcarboximidamid

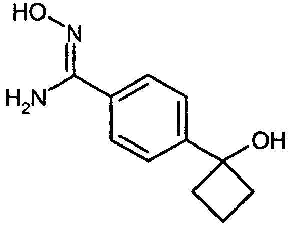

Analog zu dem unter Beispiel 1A / Schritt 5 beschriebenen Verfahren wurden ausgehend von 1.0 g (5.77 mmol) der Verbindung aus Beispiel 24A / Schritt 1 1.1 g der Titelverbindung (92% d. Th.) erhalten. Anders als unter Beispiel 1A / Schritt 5 beschrieben, wurde jedoch nach dem Entfernen des Lösungsmittels der Rückstand mit ca. 50 ml Wasser versetzt und dreimal mit je ca. 50 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt und der erhaltene Rückstand mittels MPLC gereinigt (Kieselgel; Laufmittel: Dichlormethan/Methanol 50:1 → 10:1).

¹H-NMR (400 MHz, DMSO-d₆, δ/ppm): 9.57 (s, 1H), 7.63 (d, 2H), 7.47 (d, 2H), 5.79 (s, breit, 2H), 5.50 (s, 1H), 2.42-2.33 (m, 2H), 2.30-2.22 (m, 2H), 1.97-1.60 (m, 1H), 1.70-1.59 (m, 1H).

HPLC (Methode A): Rₜ = 2.26 min.

MS (EIpos): m/z = 207 [M+H]⁺.

LC/MS (Methode I, ESIpos): Rₜ = 0.25 min, m/z = 207 [M+H]⁺.

### Beispiel 25A

### N'-Hydroxy-4-(1-methoxycyclobutyl)benzolcarboximidamid

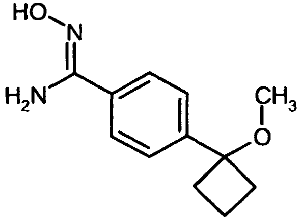

### Schritt 1: 4-(1-Methoxycyclobutyl)benzolcarbonitril

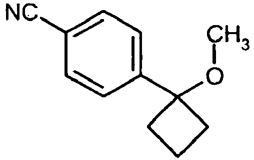

Analog zu dem unter Beispiel 6A / Schritt 1 beschriebenen Verfahren wurden aus 2.0 g (11.5 mmol) der Verbindung aus Beispiel 24A / Schritt 1, 508 mg (12.7 mmol) einer 60%-igen Dispersion von Natriumhydrid in Mineralöl sowie 863 µl (13.9 mmol) Methyliodid 1.27 g (59% d. Th.) der Titelverbindung erhalten. Die Aufreinigung des Produktes erfolgte mittels MPLC (Kieselgel; Laufmittel: Cyclohexan/Ethylacetat 20:1 → 4:1).

¹H-NMR (400 MHz, CDCl₃, δ/ppm): 7.68 (d, 2H), 7.54 (d, 2H), 2.95 (s, 3H), 2.46-2.32 (m, 4H), 2.03-1.93 (m, 1H), 1.76-1.63 (m, 1H).

MS (DCI, NH₃): m/z = 205 [M+NH₄]⁺.

### Schritt 2: N'-Hydroxy-4-(1-methoxycyclobutyl)benzolcarboximidamid

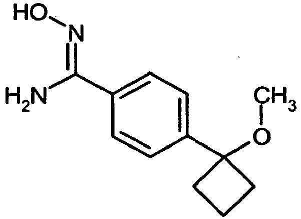

Analog zu dem unter Beispiel 1A / Schritt 5 beschriebenen Verfahren wurden ausgehend von 1.1 g (5.87 mmol) der Verbindung aus Beispiel 25A / Schritt 1 1.28 g der Titelverbindung (98% d. Th.) erhalten.

¹H-NMR (400 MHz, DMSO-d₆, δ/ppm): 9.62 (s, 1H), 7.68 (d, 2H), 7.40 (d, 2H), 5.80 (s, breit, 2H), 2.83 (s, 3H), 2.37-2.24 (m, 4H), 1.91-1.81 (m, 1H), 1.65-1.53 (m, 1H).

HPLC (Methode A): Rₜ = 3.02 min.

MS (DCI, NH₃): m/z = 221 [M+H]⁺.

### Beispiel 26A

### 4-(1-Fluorcyclobutyl)-N'-hydroxybenzolcarboximidamid

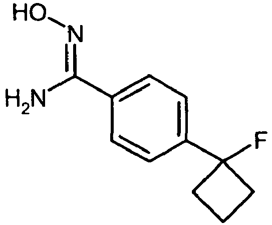

### Schritt 1: 4-(1-Fluorcyclobutyl)benzolcarbonitril

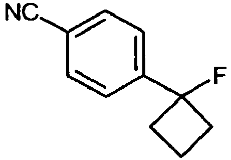

Analog zu dem unter Beispiel 5A / Schritt 2 beschriebenen Verfahren wurden aus 2.0 g (11.5 mmol) der Verbindung aus Beispiel 24A / Schritt 1 und 1.8 ml (13.9 mmol) Diethylamino-schwefeltrifluorid (DAST) 1.39 g (69% d. Th.) der Titelverbindung erhalten. Die Aufreinigung des Produktes erfolgte mittels MPLC (Kieselgel; Laufmittel: Cyclohexan/Ethylacetat 10:1 → 5:1).

¹H-NMR (400 MHz, CDCl₃, δ/ppm): 7.69 (d, 2H), 7.57 (d, 2H), 2.78-2.62 (m, 2H), 2.58-2.48 (m, 2H), 2.20-2.09 (m, 1H), 1.87-1.75 (m, 1H).

GC/MS (Methode L, EIpos): Rₜ = 4.71 min, m/z = 155 [M-HF]⁺.

### Schritt 2: 4-(1-Fluorcyclobutyl)-N'-hydroxybenzolcarboximidamid

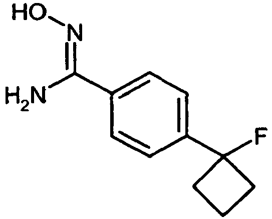

Analog zu dem unter Beispiel 1A / Schritt 5 beschriebenen Verfahren wurden ausgehend von 1.25 g (7.13 mmol) der Verbindung aus Beispiel 26A / Schritt 1 1.16 g der Titelverbindung (78% d. Th.) erhalten.

¹H-NMR (400 MHz, CDCl₃, δ/ppm): 7.67 (d, 2H), 7.50 (d, 2H), 4.87 (s, breit, 2H), 2.72-2.52 (m, 5H), 2.16-2.05 (m, 1H), 1.82-1.71 (m, 1H).

HPLC (Methode A): Rₜ = 3.17 min.

MS (DCI, NH₃): m/z = 209 [M+H]⁺.

### Beispiel 27A

### 2-Amino-2-[4-(trifluormethoxy)phenyl]ethanol

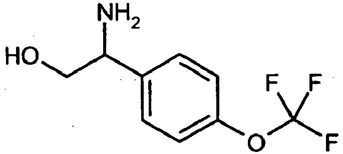

Eine Lösung von 3.0 g (12.8 mmol) racemischem 4-(Trifluormethoxy)phenylglycin in 20 ml THF wurde nacheinander mit 834 mg (38.3 mmol) Lithiumborhydrid und 1 ml (19.1 mmol) konzentrierter Schwefelsäure, gelöst in 1 ml THF, versetzt. Das Reaktionsgemisch wurde 24 h bei RT gerührt. Dann wurden 15 ml Methanol zugesetzt und die Mischung so lange gerührt, bis eine klare Lösung entstand. Zu dieser Lösung wurden anschließend 20 ml 4 M Natronlauge hinzugetropft. Dabei fiel ein Niederschlag aus, der abgesaugt und verworfen wurde. Das Filtrat wurde am Rotationsverdampfer von den organischen Lösungsmitteln befreit. Der Rückstand wurde dreimal mit je ca. 20 ml Toluol extrahiert. Die vereinigten organischen Extrakte wurden am Rotationsverdampfer eingeengt. Es wurden 2.25 g (80% d. Th.) der Titelverbindung erhalten.

¹H-NMR (400 MHz, DMSO-d₆, δ/ppm): 7.48 (d, 2H), 7.31 (d, 2H), 5.63 und 5.51 (jeweils breit, zus. 2H), 4.91 (breit, 1H), 3.71-3.67 (m, 1H), 3.66-3.59 (m, 2H).

MS (DCI, NH₃): m/z = 222 [M+H]⁺.

### Beispiel 28A

### 5-(5-Methyl-1H-pyrazol-3-yl)-3-[4-(trifluormethoxy)phenyl]-1,2,4-oxadiazol

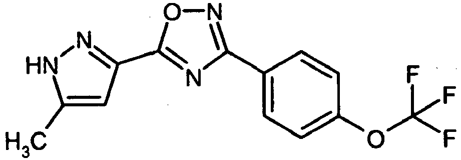

Eine Lösung von 15.3 g (0.121 mol) 5-Methyl-1*H*-pyrazol-3-carbonsäure in 600 ml wasserfreiem DMF wurde bei RT nacheinander mit 23.3 g (0.121 mol) EDC, 16.4 g (0.121 mol) HOBt und 26.7 g (0.121 mol) *N*'-Hydroxy-4-(trifluormethoxy)benzolcarboximidamid versetzt. Das Gemisch wurde zunächst 2 h bei RT und anschließend 5 h bei 140°C gerührt. Nach dem Abkühlen wurde mit 2 Litern Wasser verdünnt und dreimal mit je 1 Liter Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das erhaltene Rohprodukt wurde mittels Saugfiltration über eine mit Kieselgel gefüllte Filternutsche gereinigt (Eluent: Cyclohexan/Ethylacetat 5:1 → 1:1). Die Produktfraktionen wurden vereinigt und das Lösungsmittel am Rotationsverdampfer so weit entfernt, dass das Produkt gerade begann auszufallen. Die Fällung wurde bei RT vervollständigt. Durch Filtration und weiteres Einengen der Mutterlauge wurden zwei Fraktionen Feststoff erhalten, die vereinigt und im Hochvakuum getrocknet wurden. Insgesamt wurden so 19.7 g (52% d. Th.) der Titelverbindung erhalten.

¹H-NMR (400 MHz, CDCl₃, δ/ppm): 10.75 (breit, 1H), 8.24 (d, 2H), 7.34 (d, 2H), 6.81 (s, 1H), 2.46 (s, 3H).

HPLC (Methode A): Rₜ = 4.72 min.

MS (DCI, NH₃): m/z = 311 [M+H]⁺.

LC/MS (Methode F, ESIpos): Rₜ = 1.27 min, m/z = 311 [M+H]⁺.

Die in der folgenden Tabelle aufgeführten Verbindungen wurden nach dem in Beispiel 28A beschriebenen Verfahren aus 5-Methyl-1*H*-pyrazol-3-carbonsäure, 5-(Trifluormethyl)-1*H*-pyrazol-3-carbonsäure, 5-Nitro-1*H*-pyrazol-3-carbonsäure bzw. 2-Methyl-1*H*-imidazol-4-carbonsäure-Hydrat und den entsprechenden *N'*-Hydroxybenzolcarboximidamiden hergestellt. Je nach Größe des Ansatzes betrug die Reaktionszeit, während der zunächst bei RT gerührt wurde, 0.5 bis 4 h. Auf 140°C wurde nachfolgend für 1 bis 15 h erhitzt. Je nach Polarität des erhaltenen Produkts fiel dieses bereits bei der Zugabe von Wasser nach der beendeten Reaktion aus, es wurde dann gewaschen und im Hochvakuum getrocknet. Alternativ wurde, wie oben beschrieben, extraktiv aufgearbeitet und anschließend chromatographisch über Kieselgel gereinigt; für die Chromatographie wurden unterschiedliche Laufmittel verwendet. In manchen Fällen konnte auf die Chromatographie verzichtet und das Produkt direkt durch Ausrühren in Dichlormethan, Ethylacetat, Acetonitril oder *tert*.-Butyl-methylether gereinigt werden. Die Verbindung in Beispiel 41A wurde mittels präparativer HPLC (Methode N) gereinigt.

| **Beispiel** | **Struktur** | **HPLC: Rₜ [min]** | **MS: m/z [M+H]⁺** | **LC/MS-Methode** |
|---|---|---|---|---|
| **29A** | 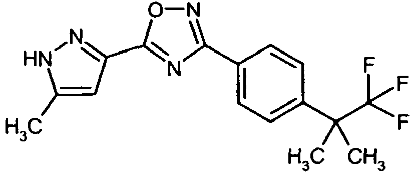 | 1.34 | 337 | F |
| | ¹H-NMR (400 MHz, DMSO-d₆, δ/ppm): 11.80 (s, breit, 1H), 8.17 (d, 2H), 7.63 (d, 2H), 6.83 (s, 1H), 2.46 (s, 3H), 1.63 (s, 6H). | | | |
| **30A** | 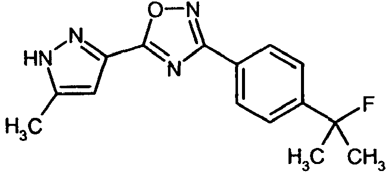 | 2.19 | 287 | D |
| | ¹H-NMR (400 MHz, DMSO-d₆, δ/ppm): 13.54 (s, breit, 1H), 8.08 (d, 2H), 7.62 (d, 2H), 6.81 (s, 1H), 2.33 (s, 3H), 1.72 (s, 3H), 1.68 (s, 3H). | | | |
| **31A** | 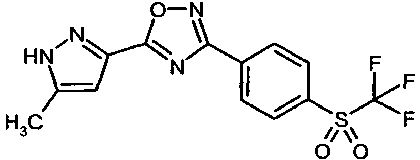 | 1.25 | 359 | F |
| | ¹H-NMR (400 MHz, DMSO-d₆, δ/ppm). 13.62 (s, breit, 1H), 8.49 (d, 2H), 8.38 (d, 2H), 6.83 (s, 1H), 2.34 (s, 3H). | | | |
| **32A** | 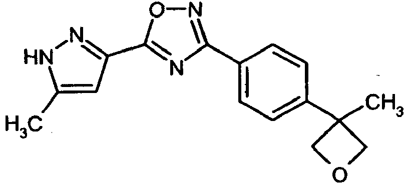 | 1.98 | 297 | C |
| | ¹H-NMR (400 MHz, CDCl₃, δ/ppm): 8.17 (d, 2H), 7.33 (d, 2H), 6.82 (s, 1H), 5.00 (d, 2H), 4.68 (d, 2H), 2.45 (s, 3H), 1.77 (s, 3H). | | | |
| **33A** | 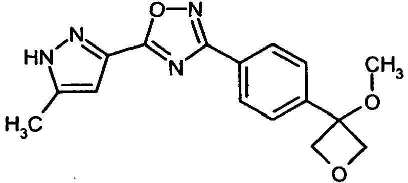 | 0.99 | 313 | F |
| | ¹H-NMR (400 MHz, DMSO-d₆, δ/ppm): 13.54 (s, breit, 1H), 8.14 (d, 2H), 7.69 (d, 2H), 6.80 (s, 1H), 4.82 (d, 2H), 4.78 (d, 2H), 3.08 (s, 3H), 2.37 (s, 3H). | | | |
| **34A** | 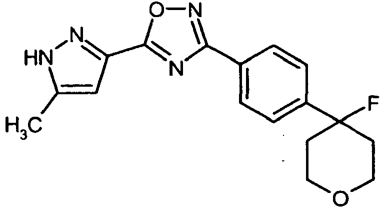 | 4.24 | 329 | C |
| | ¹H-NMR (400 MHz, CDCl₃, δ/ppm): 10.73 (breit, 1H), 8.20 (d, 2H), 7.52 (d, 2H), 6.81 (s, 1H), 4.00-3.88 (m, 4H), 2.45 (s, 3H), 2.30-2.11 (m, 2H), 1.98-1.91 (m, 2H). | | | |
| **35A** | 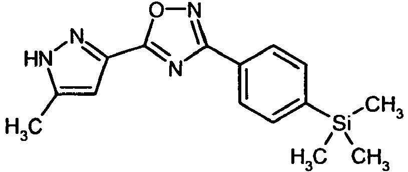 | 2.39 | 299 | E |
| | ¹H-NMR (400 MHz, CDCl₃, δ/ppm): 11.3 (s, breit, 1H), 8.12 (d, 2H), 7.63 (d, 2H), 6.81 (s, 1H), 2.43 (s, 3H), 0.31 (s, 9H). | | | |
| **36A** | 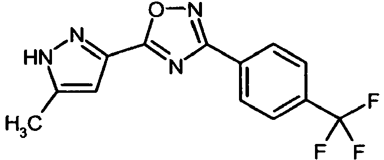 | 1.11 | 295 | I |
| | ¹H-NMR (400 MHz, CDCl₃, δ/ppm): 10.52 (breit, 1H), 8.32 (d, 2H), 7.77 (d, 2H), 6.82 (s, 1H), 2.63 (s, 3H). | | | |
| **37A** | 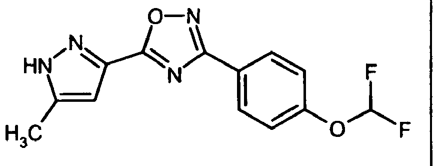 | 1.02 | 293 | I |
| | ¹H-NMR (400 MHz, CDCl₃, δ/ppm): 10.85 (breit, 1H), 8.20 (d, 2H), 7.23 (d, 2H), 6.81 (s, 1H), 6.60 (t, 1H), 2.46 (s, 3H). | | | |
| **38A** | 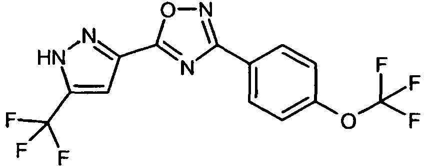 | 2.41 | 365 | E |
| | ¹H-NMR (400 MHz, CDCl₃, δ/ppm): 11.73 (breit, 1H), 8.19 (d, 2H), 7.38 (d, 2H), 7.37 (s, 1H). | | | |
| **39A** | 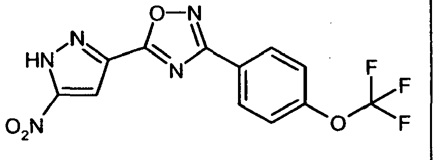 | 2.18 | 342 | E |
| | ¹H-NMR (500 MHz, DMSO-d₆, δ/ppm): 8.20 (d, 2H), 7.58 (d, 2H), 7.34 (s, 1H). | | | |
| **40A** | 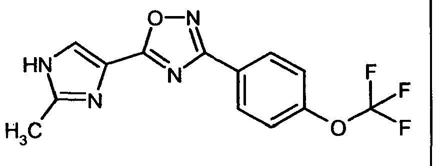 | 1.08 | 311 | F |
| | ¹H-NMR (400 MHz, CDCl₃, δ/ppm): 8.23 (d, 2H), 7.82 (d, 2H), 7.33 (s, 1H), 2.56 (s, 3H). | | | |
| **41A** | 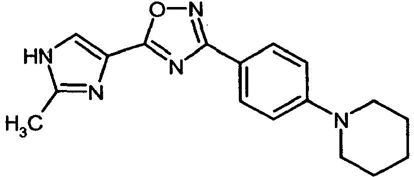 | 0.97 | 310 | F |
| | ¹H-NMR (400 MHz, CDCl₃, δ/ppm): 9.61 (breit, 1H), 8.02 (d, 2H), 7.79 (s, 1H), 6.96 (d, 2H), 3.31-3.27 (m, 4H), 2.54 (s, 3H), 1.73-1.61 (m, 6H). | | | |

### Beispiel 42A

### 3-{3-[4-(Trifluormethoxy)phenyl]-1,2,4-oxadiazol-5-yl}-1H-pyrazol-5-amin

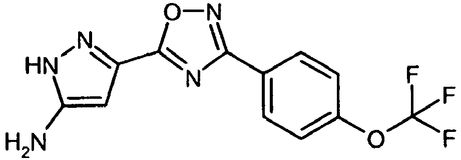

In einer Durchfluss-Hydrierapparatur ("H-Cube" der Firma ThalesNano, Budapest, Ungarn) wurde eine Lösung von 342 mg (1.0 mmol) der Verbindung aus Beispiel 39A in 43 ml Ethylacetat hydriert (Bedingungen: 10% Pd/C-Katalysator, 1 bar H₂, 25°C, 1 ml/min). Nach Entfernen des Lösungsmittels am Rotationsverdampfer wurde das Rohprodukt mittels MPLC gereinigt (Kieselgel, Cyclohexan/Ethylacetat 1:1). Es wurden 322 mg (93% d. Th.) der Titelverbindung erhalten.

¹H-NMR (400 MHz, DMSO-d₆, δ/ppm): 12.49 (s, 1H), 8.19 (d, 2H), 7.49 (d, 2H), 5.93 (s, 1H), 5.44 (s, 2H).

MS (DCI, NH₃): m/z = 312 [M+H]⁺.

LC/MS (Methode E, ESIpos): Rₜ = 1.76 min, m/z = 312 [M+H]⁺.

### Beispiel 43A

### 2-Chlor-4-(chlormethyl)pyridin

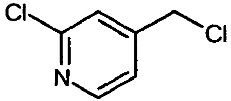

Man löste 1.00 g (6.97 mmol) (2-Chlorpyridin-4-yl)methanol in 40 ml Dichlormethan, gab langsam 10 ml Thionylchlorid bei RT hinzu und rührte das Gemisch über Nacht bei RT. Anschließend wurde das Gemisch am Rotationsverdampfer eingeengt und der Rückstand in einem Mischung aus Dichlormethan und wässriger Natriumhydrogencarbonat-Lösung verrührt. Man trennte die Phasen, trocknete die Dichlormethan-Phase über wasserfreiem Magnesiumsulfat, filtrierte und engte am Rotationsverdampfer ein. Es wurden 1.10 g (97% d. Th.) der Titelverbindung erhalten.

¹H-NMR (400 MHz, CDCl₃, δ/ppm): 8.49 (d, 1H), 7.38 (s, 1H), 7.27-7.22 (m, 1H), 4.52 (s, 2H).

LC/MS (Methode E, ESIpos): Rₜ = 1.43 min, m/z = 162 [M+H]⁺.

### Beispiel 44A

### 2-(Chlormethyl)-5-iodpyridin

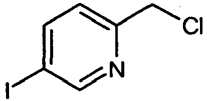

### Schritt 1: 2-(Hydroxymethyl)-5-iodpyridin

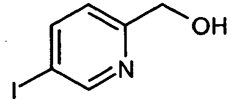

Unter inerten Bedingungen und einer Temperatur von -78°C wurde eine Lösung von 2.50 g (7.56 mmol) 2,5-Diiodpyridin in 90 ml Toluol tropfenweise mit 5.7 ml (9.07 mmol) einer 1.6 M Lösung von n-Butyllithium in Hexan versetzt. Es wurde 2.5 h bei -78°C gerührt und dann bei derselben Temperatur 756 µl wasserfreies DMF zugegeben. Nach weiteren 60 min bei -78°C ließ man das Reaktionsgemisch auf -10°C erwärmen, fügte 572 mg (15.11 mmol) festes Natriumborhydrid hinzu und setzte das Rühren für 30 min bei 0°C fort. Anschließend wurde mit 25 ml gesättigter wässriger Ammoniumchlorid-Lösung versetzt und das Gemisch auf RT erwärmt. Die organische Phase wurde abgetrennt und das Lösungsmittel am Rotationsverdampfer entfernt. Der Rückstand wurde mittels präparativer HPLC gereinigt. Es wurden 890 mg (50% d. Th.) der Titelverbindung (analytische Daten siehe unten) sowie 243 mg (14% d. Th.) des isomeren 5-(Hydroxymethyl)-2-iodpyridins erhalten [präparative HPLC-Bedingungen: Säule: Sunfire C18 OBD 5 µm, 19 mm x 150 mm; Temperatur: 40°C; Laufmittel: Wasser/Acetonitril/1%-ige wässrige TFA 76:5:19; Flussrate: 25 ml/min; 1.3 g Rohprodukt wurden in einem Gemisch aus 8 ml 1%-iger wässriger TFA und 4 ml Acetonitril gelöst; Injektionsvolumen: 1 ml].

¹H-NMR (400 MHz, CDCl₃, δ/ppm): 8.87 (d, 1H), 8.30 (dd, 1H), 7.38 (d, 1H), 5.43 (breit, 1H), 4.85 (s, 2H).

HPLC (Methode A): Rₜ = 0.87 min.

MS (DCI, NH₃): m/z = 236 [M+H]⁺.

LC/MS (Methode E, ESIpos): Rₜ = 0.85 min, m/z = 236 [M+H]⁺.

### Schritt 2: 2-(Chlormethyl)-5-iodpyridin

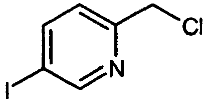

Eine Lösung von 765 mg (3.26 mmol) der Verbindung aus Beispiel 44A / Schritt 1 in 12 ml wasserfreiem Dichlormethan wurde bei 0°C tropfenweise mit 357 µl (4.88 mmol) Thionylchlorid versetzt. Man ließ das Reaktionsgemisch anschließend 15 h bei RT rühren. Dann wurde mit ca. 50 ml gesättigter wässriger Natriumhydrogencarbonat-Lösung versetzt und dreimal mit je ca. 50 ml Dichlormethan extrahiert. Die vereinigten organischen Extrakte wurden mit gesättigter Kochsalz-Lösung gewaschen und über wasserfreiem Magnesiumsulfat getrocknet. Nach dem Filtrieren wurde das Lösungsmittel am Rotationsverdampfer entfernt. Es wurden 541 mg (66% d. Th.) der Titelverbindung erhalten.

¹H-NMR (400 MHz, CDCl₃, δ/ppm): 8.79 (d, 1H), 8.03 (dd, 1H), 7.29 (d, 1H), 4.61 (s, 2H).

MS (ESIpos): m/z = 254/256 (³⁵Cl/³⁷Cl) [M+H]⁺.

LC/MS (Methode D, ESIpos): Rₜ = 1.87 min, m/z = 254/256 (³⁵Cl/³⁷Cl) [M+H]⁺.

### Beispiel 45A

### 5-(Chlormethyl)pyridin-2-carbonitril-Hydrochlorid

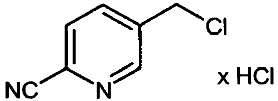

Eine Lösung von 250 mg (1.86 mmol) 5-(Hydroxymethyl)pyridin-2-carbonitril [A. Ashimori et al., Chem. Pharm. Bull. 1990, 38 (9), 2446-2458] in 5 ml wasserfreiem Dichlormethan wurde bei 0°C mit 272 µl (3.73 mmol) Thionylchlorid versetzt. Dann wurde das Reaktionsgemisch 6 h bei RT gerührt. Anschließend wurden alle flüchtigen Bestandteile am Rotationsverdampfer entfernt und der erhaltene Rückstand im Hochvakuum getrocknet. Es wurden 263 mg (75% d. Th.) der Titelverbindung erhalten.

¹H-NMR (400 MHz, CDCl₃, δ/ppm): 8.73 (d, 1H), 7.90 (dd, 1H), 7.72 (d, 1H), 4.63 (s, 2H).

MS (ESIpos): m/z= 153/155 (³⁵Cl/³⁷Cl) [M+H]⁺.

LC/MS (Methode F, ESIpos): Rₜ = 0.75 min, m/z = 153/155 (³⁵Cl/³⁷Cl) [M+H]⁺.

### Beispiel 46A

### (6-Cyanopyridin-3-yl)methylmethansulfonat

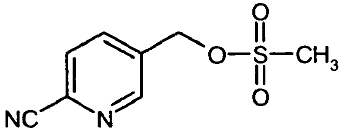

Eine Lösung von 2.8 g (20.87 mmol) 5-(Hydroxymethyl)pyridin-2-carbonitril [A. Ashimori et al., Chem. Pharm. Bull. 1990, 38 (9), 2446-2458] in 50 ml wasserfreiem Dichlormethan wurde bei 0°C nacheinander mit 3.51 ml (27.14 mmol) *N,N-*Diisopropylethylamin und 2.87 ml (25.05 mmol) Methansulfonsäurechlorid versetzt. Dann wurde das Reaktionsgemisch 1 h bei RT gerührt. Anschließend wurde mit 10 ml Wasser versetzt, die Phasen getrennt und die wässrige Phase zweimal mit je ca. 10 ml Dichlormethan extrahiert. Die vereinigten organischen Extrakte wurden mit gesättigter Kochsalz-Lösung gewaschen, über wasserfreiem Magnesiumsulfat getrocknet, filtriert und am Rotationsverdampfer vom Lösungsmittel befreit. Der erhaltene Rückstand wurde mittels MPLC in seine Komponenten aufgetrennt (Kieselgel, Cyclohexan/Ethylacetat 1:1). Es wurden 2.12 g (48% d. Th.) der Titelverbindung (analytische Daten siehe unten) sowie 1.51 g (47% d. Th.) der in Beispiel 45A beschriebenen Verbindung erhalten.

¹H-NMR (400 MHz, CDCl₃, δ/ppm): 8.76 (d, 1H), 7.93 (dd, 1H), 7.78 (d, 1H), 5.32 (s, 2H), 3.10 (s, 3H).

MS (DCI, NH₃): m/z = 213 [M+H]⁺, 230 [M+NH₄]⁺.

LC/MS (Methode F, ESIpos): Rₜ = 0.57 min, m/z = 213 [M+H]⁺.

### Beispiel 47A

### [3-(Brommethyl)phenoxy](tripropan-2-yl)silan

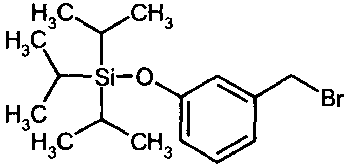

### Schritt 1: Ethyl-3-[(tripropan-2-ylsilyl)oxy]benzolcarboxylat

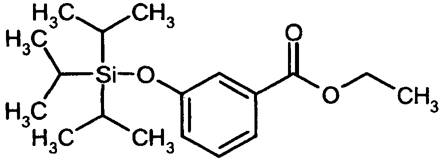

Eine Lösung von 5.0 g (30.09 mmol) 3-Hydroxybenzoesäureethylester und 2.41 g (35.35 mmol) Imidazol in 20 ml wasserfreiem DMF wurde bei 0°C tropfenweise mit 5.98 g (30.99 mmol) Tri-isopropylsilylchlorid versetzt. Nachdem das Reaktionsgemisch 15 h bei RT gerührt worden war, wurde mit ca. 100 ml Wasser versetzt und dreimal mit je ca. 100 ml Diethylether extrahiert. Die vereinigten organischen Extrakte wurden nacheinander mit Wasser und gesättigter Kochsalz-Lösung gewaschen. Nach Trocknen über wasserfreiem Magnesiumsulfat und Filtration wurde das Lösungsmittel am Rotationsverdampfer entfernt. Der erhaltene Rückstand wurde per Saugfiltration über Kieselgel mit Cyclohexan/Ethylacetat 10:1 → 1:1 als Laufmittel gereinigt. Es wurden 9.70 g (100% d. Th.) der Titelverbindung erhalten.

¹H-NMR (400 MHz, CDCl₃, δ/ppm): 7.62 (dd, 1H), 7.53 (m, 1H), 7.28 (dd, 1H), 7.06 (dd, 1H), 4.37 (quart, 2H), 1.39 (t, 3H), 1.28 (sept, 3H), 1.10 (d, 18H).

GC/MS (Methode L, EI): Rₜ = 6.62 min, m/z = 322 (M)⁺, 279 (M-C₃H₇)⁺.

### Schritt 2: {3-[(Tripropan-2-ylsilyl)oxy]phenyl}methanol

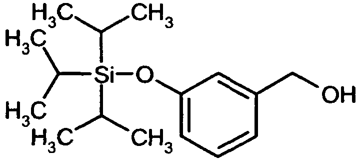

Unter inerten Bedingungen wurden 50 ml (49.61 mmol) einer 1 M Lösung von Lithiumaluminiumhydrid in THF mit 50 ml wasserfreiem Diethylether verdünnt und anschließend bei 0°C tropfenweise mit einer Lösung von 8.0 g (24.80 mmol) der Verbindung aus Beispiel 47A / Schritt 1 in 50 ml wasserfreiem Diethylether versetzt. Das Reaktionsgemisch wurde 1 h bei RT gerührt. Dann wurden zunächst einige ml Methanol zugesetzt, um überschüssiges Hydrid zu solvolysieren, und anschließend ca. 150 ml 0.1 M Salzsäure. Die organische Phase wurde zügig abgetrennt, und die wässrige Phase wurde zweimal mit je ca. 50 ml Diethylether extrahiert. Die vereinigten organischen Extrakte wurden nacheinander mit Wasser und gesättigter Kochsalz-Lösung gewaschen. Nach Trocknen über wasserfreiem Magnesiumsulfat und anschließender Filtration wurde das Lösungsmittel am Rotationsverdampfer entfernt. Der erhaltene Rückstand wurde per Saugfiltration über Kieselgel mit Cyclohexan/Ethylacetat 5:1 → 1:1 als Laufmittel gereinigt. Es wurden 6.69 g (96% d. Th.) der Titelverbindung erhalten.

¹H-NMR (400 MHz, CDCl₃, δ/ppm): 7.20 (dd, 1H), 6.93-6.90 (m, 2H), 6.80 (dd, 1H), 4.64 (d, 2H), 1.61 (t, 3H), 1.26 (sept, 3H), 1.09 (d, 18H).

GC/MS (Methode L, EI): Rₜ = 6.38 min, m/z = 280 (M)⁺, 237 (M-C₃H₇)⁺.

### Schritt 3: [3-(Brommethyl)phenoxy](tripropan-2-yl)silan

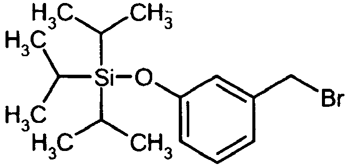

1.0 g (3.57 mmol) der Verbindung aus Beispiel 47A / Schritt 2 wurde in 20 ml wasserfreiem THF gelöst und mit 1.12 g (4.28 mmol) Triphenylphosphin versetzt. Nachdem dieses in Lösung gegangen war, wurden 1.42 g (4.28 mmol) Tetrabrommethan zugesetzt. Anschließend wurde 20 h bei RT gerührt. Dann wurde von dem ausgefallenen Niederschlag abfiltriert und das Filtrat am Rotationsverdampfer vom Lösungsmittel befreit. Das Rohprodukt wurde mittels MPLC gereinigt (Kieselgel, Cyclohexan/Ethylacetat 50:1). Es wurden 1.10 g (90% d. Th., ca. 90% Reinheit) der Titelverbindung erhalten, welche ohne weitere Aufreinigung verwendet wurde.

¹H-NMR (400 MHz, CDCl₃, δ/ppm): 7.18 (dd, 1H), 6.95 (dd, 1H), 6.91 (m, 1H), 6.80 (dd, 1H), 4.43 (s, 2H), 1.25 (sept, 3H), 1.10 (d, 18H).

HPLC (Methode B): Rₜ = 6.17 min.

GC/MS (Methode L, EI): Rₜ = 6.56 min, m/z = 342/344 (⁷⁹Br/⁸¹Br) (M)⁺.

### Beispiel 48A

### Ethyl-(4-{[(methylsulfonyl)oxy]methyl}phenyl)acetat

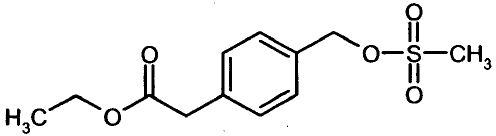

Eine Lösung von 1.1 g (5.66 mmol) [4-(Hydroxymethyl)phenyl]essigsäureethylester [G. Biagi *et al., Farmaco Ed. Sci.* 1988, *43 (7*/*8),* 597-612] und 1.03 ml (7.36 mmol) Triethylamin in 10 ml wasserfreiem THF wurde auf 0°C abgekühlt. Dann wurde tropfenweise mit einer Lösung von 526 µl (6.80 mmol) Methansulfonsäurechlorid in 5 ml wasserfreiem THF versetzt. Nach 15 min bei 0°C wurde auf RT erwärmt. Nach einer weiteren Stunde wurden ca. 60 ml Wasser hinzugefügt und zweimal mit je ca. 50 ml Ethylacetat extrahiert. Die vereinigten organischen Extrakte wurden mit gesättigter Kochsalz-Lösung gewaschen. Nach Trocknen über wasserfreiem Magnesiumsulfat und Filtration wurde das Lösungsmittel am Rotationsverdampfer entfernt. Das Rohprodukt wurde mittels MPLC aufgereinigt (Kieselgel, Cyclohexan/Ethylacetat 7:3). Es wurden 1.19 g (56% d. Th., ca. 73% Reinheit) der Titelverbindung erhalten, welche ohne weitere Reinigung verwendet wurde.

MS (DCI, NH₃): m/z = 290 [M⁺NH₄]⁺.

LC/MS (Methode C, ESIpos): Rₜ = 1.96 min, m/z = 177 (M-CH₃SO₂O)⁺.

### Beispiel 49A

### 1-[(6-Chlorpyridin-3-yl)methyl]-5-methyl-1H-pyrazol-3-carbonsäure

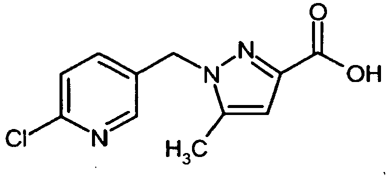

### Schritt 1: Ethyl-1-[(6-chlorpyridin-3-yl)methyl]-5-methyl-1H-pyrazol-3-carboxylat

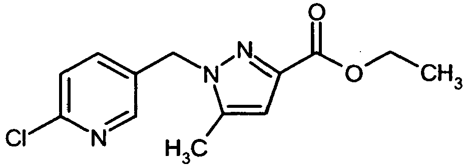

Zu einer Lösung von 10.0 g (64.9 mmol) Ethyl-3-methyl-*1H*-pyrazol-5-carboxylat und 13.66 g (84.3 mmol) 2-Chlor-5-(chlormethyl)pyridin in 162 ml wasserfreiem THF gab man 9.46 g (84.3 mmol) Kalium-*tert*.-butylat bei 0°C hinzu. Man ließ das Gemisch auf RT kommen und rührte weitere 18 h bei RT. Anschließend verdünnte man mit 200 ml Ethylacetat und 350 ml Wasser, durchmischte die Phasen und extrahierte die abgetrennte wässrige Phase noch zweimal mit jeweils 200 ml Ethylacetat. Man trocknete die vereinigten organischen Phasen über wasserfreiem Natriumsulfat, filtrierte und engte am Rotationsverdampfer ein. Der Rückstand wurde säulenchromatographisch an Kieselgel gereinigt (Laufmittel: Cyclohexan/Ethylacetat 4:1 → 2:1). Nach Trocknen im Vakuum erhielt man 12.4 g (65% d. Th.) der Titelverbindung in einer Reinheit von 95%.

¹H-NMR (400 MHz, DMSO-d₆, δ/ppm): 8.30 (d, 1H), 7.58 (dd, 1H), 7.52 (d, 1H), 6.60 (s, 1H), 5.45 (s, 2H), 4.24 (quart, 2H), 2.28 (s, 3H), 1.27 (t, 3H).

LC/MS (Methode C, ESIpos): Rₜ = 1.88 min, m/z = 280 [M+H]⁺.

### Schritt 2: 1-[(6-Chlorpyridin-3-yl)methyl]-5-methyl-1H-pyrazol-3-carbonsäure

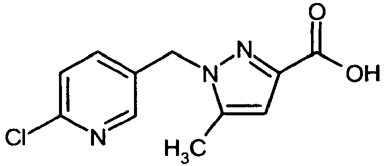

Zu einer Lösung von 11.85 g (42.36 mmol) der Verbindung aus Beispiel 49A / Schritt 1 in 100 ml THF gab man 3.39 g (84.7 mmol) Natriumhydroxid, gelöst in 100 ml Wasser, und rührte das Gemisch 5 h bei RT. Anschließend verdünnte man das Gemisch mit 150 ml Wasser und wusch einmal mit 100 ml Ethylacetat. Die wässrige Phase wurde mit 1 N Salzsäure auf einen pH-Wert von ca. 3 eingestellt und dreimal mit jeweils 150 ml Ethylacetat extrahiert. Letztere Ethylacetat-Phasen wurden vereinigt, über wasserfreiem Natriumsulfat getrocknet, filtriert und am Rotationsverdampfer eingeengt. Nach Trocknen des Rückstands im Vakuum wurden 9.72 g (91% d. Th.) der Titelverbindung erhalten.

¹H-NMR (400 MHz, DMSO-d₆, δ/ppm): 12.60 (s, breit, 1H), 8.31 (d, 1H), 7.60 (dd, 1H), 7.52 (d, 1H), 6.53 (s, 1H), 5.42 (s, 2H), 2.28 (s, 3H).

LC/MS (Methode F, ESIpos): Rₜ = 0.75 min, m/z = 252 [M+H]⁺.

### Beispiel 50A

### 1-[(6-Chlorpyridin-3-yl)methyl]-5-methyl-1H-pyrrol-3-carbonsäure

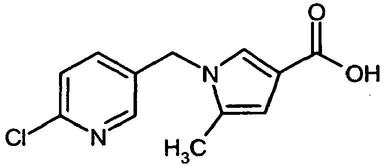

### Schritt 1: Methyl-2-(hydroxymethyliden)-4-oxopentanoat

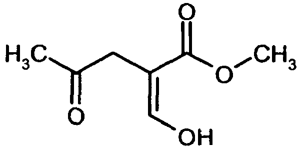

Unter inerten Bedingungen wurden 7.63 g (190.7 mmol) einer 60%-igen Suspension von Natrium-hydrid in Mineralöl mit Pentan entölt. Anschließend wurden 150 ml wasserfreier Diethylether und bei 0°C 138 µl (3.4 mmol) Methanol zugesetzt. Nach 10 min Rühren bei RT wurde erneut auf 0°C abgekühlt und ein Gemisch von 12.6 ml (204.3 mmol) Ameisensäuremethylester und 30.0 g (170.2 mmol) Methyl-4,4-dimethoxypentanoat [C. Meister et al., Liebigs Ann. Chem. 1983 (6), 913-921] langsam hinzugefügt. Das Reaktionsgemisch wurde 16 h bei RT gerührt. Dann wurden ca. 60 ml Eiswasser zugesetzt, und es wurde mit 100 ml Diethylether extrahiert. Der organische Extrakt wurde verworfen und die wässrige Phase mit 3 M Salzsäure auf einen pH-Wert von 2-3 gebracht. Es wurde viermal mit je ca. 50 ml tert.-Butyl-methylether extrahiert. Die vereinigten organischen Extrakte wurden über wasserfreiem Magnesiumsulfat getrocknet, filtriert und am Rotationsverdampfer vom Lösungsmittel befreit. Es wurden 4.2 g (13% d. Th., 85% Reinheit) der Titelverbindung erhalten, welche ohne weitere Reinigung in der nächsten Stufe eingesetzt wurde.

GC/MS (Methode L, EI): Rₜ = 3.33 min, m/z = 158 (M)⁺, 140 (M-H₂O)⁺.

### Schritt 2: Methyl-1-[(6-chlorpyridin-3-yl)methyl]-5-methyl-1H-pyrrol-3-carboxylat

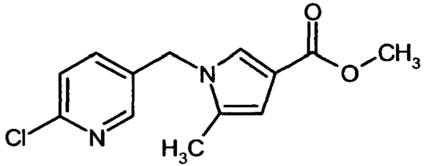

Eine Mischung von 4.20 g (22.73 mmol, 85% Reinheit) der Verbindung aus Beispiel 50A / Schritt 1 und 3.24 g (22.73 mmol) 5-(Aminomethyl)-2-chlorpyridin in 42 ml Methanol wurde drei Tage lang bei RT gerührt. Anschließend wurde das Lösungsmittel am Rotationsverdampfer entfernt und das Rohprodukt mittels MPLC gereinigt (Kieselgel, Cyclohexan/Ethylacetat 2:1). Es wurden 3.37 g (56% d. Th.) der Titelverbindung erhalten.

¹H-NMR (400 MHz, CDCl₃, δ/ppm): 8.19 (d, 1H), 7.30-7.20 (m, 3H), 6.38 (d, 1H), 5.03 (s, 2H), 3.79 (s, 3H), 2.12 (s, 3H).

HPLC (Methode A): Rₜ = 4.10 min.

MS (DCI, NH₃): m/z = 265 [M+H]⁺.

### Schritt 3: 1-[(6-Chlorpyridin-3-yl)methyl]-5-methyl-1H-pyrrol-3-carbonsäure

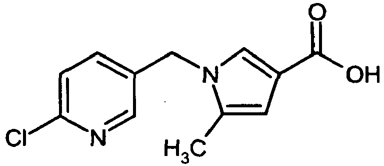

Eine Lösung von 1.93 g (7.29 mmol) der Verbindung aus Beispiel 50A / Schritt 2 in 38 ml Methanol wurde mit 14.5 ml (14.5 mmol) 1 M Natronlauge versetzt. Das Reaktionsgemisch wurde 15 h unter Rückfluss erhitzt. Nach dem Abkühlen auf RT wurde das Methanol am Rotationsverdampfer weitestgehend entfernt. Der Rückstand wurde zunächst mit 100 ml Wasser verdünnt und dann mit 2 M Salzsäure sauer gestellt. Der ausfallende Niederschlag wurde abfiltriert, mit Wasser nachgewaschen und im Hochvakuum getrocknet. Es wurden 1.41 g (76% d. Th.) der Titelverbindung erhalten.

¹H-NMR (400 MHz, DMSO-d₆, δ/ppm): 11.67 (s, 1H), 8.23 (s, 1H), 7.51 (d, 2H), 7.45 (d, 2H), 6.18 (d, 1H), 5.19 (s, 2H), 2.07 (s, 3H).

HPLC (Methode A): Rₜ = 3.59 min.

MS (ESIpos): m/z = 251 [M+H]⁺.

### Beispiel 51A

### 2-Chlor-5-[(2-methyl-4-{3-[4-(trifluormethoxy)phenyl]-1,2,4-oxadiazol-5-yl}-1H-pyrrol-1-yl)-methyl]pyridin

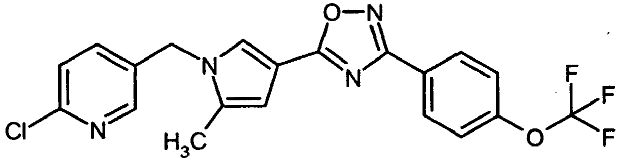

Unter inerten Bedingungen wurde bei 0°C eine Lösung von 400 mg (1.60 mmol) der Verbindung aus Beispiel 50A in 20 ml wasserfreiem Dichlormethan mit 418 µl (4.79 mmol) Oxalylchlorid versetzt. Das Reaktionsgemisch wurde 2 h bei RT gerührt. Anschließend wurden alle flüchtigen Bestandteile am Rotationsverdampfer entfernt und der so erhaltene Rückstand 20 min im Hochvakuum getrocknet. Der Rückstand wurde danach erneut in 4 ml Dichlormethan gelöst, und diese Lösung wurde bei 0°C zu einer Lösung von 527 mg (2.39 mmol) 4-(Trifluormethoxy)-*N'*-hydroxy-benzolcarboximidamid und 445 µl (3.19 mmol) Triethylamin in 16 ml Dichlormethan hinzugetropft. Nachdem das Reaktionsgemisch 16 h bei RT gerührt worden war, wurde wiederum alles Flüchtige am Rotationsverdampfer entfernt und der erhaltene Rückstand in 30 ml DMSO gelöst. Diese Lösung wurde dann in einer Mikrowelle 30 min lang auf 140°C erhitzt (CEM Discover, initiale Einstrahlleistung 250 W). Nach dem Abkühlen auf RT wurde das Reaktionsgemisch mittels präparativer HPLC (Methode N) aufgereinigt. Es wurden 196 mg (28% d. Th.) der Titelverbindung erhalten.

¹H-NMR (400 MHz, CDCl₃, δ/ppm): 8.24 (d, 1H), 8.17 (d, 2H), 7.47 (d, 1H), 7.32-7.27 (m, 4H), 6.60 (d, 1H), 5.10 (s, 2H), 2.20 (s, 3H).

LC/MS (Methode C, ESIpos): Rₜ = 3.01 min, m/z = 435 [M+H]⁺.

### Beispiel 52A

### 2-Chlor-5-[(3-(3-[4-(2-fluorpropan-2-yl)phenyl]-1,2,4-oxadiazol-5-yl)-5-methyl-1H-pyrazol-1-yl)-methyl]pyridin

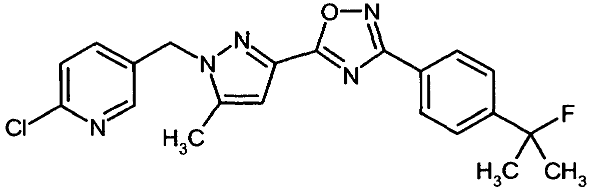

Eine Lösung von 667 mg (2.65 mmol) der Verbindung aus Beispiel 49A in 10 ml wasserfreiem DMF wurde bei RT mit 508 mg (2.65 mmol) EDC und 358 mg (2.65 mmol) HOBt versetzt. Nach 30 min wurden 520 mg (2.65 mmol) der Verbindung aus Beispiel 2A, gelöst in 5 ml DMF, hinzugefügt. Das Gemisch wurde zunächst 1 h bei RT und anschließend 1 h bei 140°C gerührt. Nach dem Abkühlen wurde der Großteil des Lösungsmittels am Rotationsverdampfer entfernt. Es wurden je 50 ml Wasser und Ethylacetat zugesetzt. Nach Phasentrennung wurde die organische Phase nacheinander mit je 50 ml 10%-iger wässriger Zitronensäure, gesättigter Natriumhydrogencarbonat-Lösung und gesättigter Kochsalz-Lösung gewaschen. Nach dem Trocknen über wasserfreiem Natriumsulfat wurde filtriert und das Lösungsmittel am Rotationsverdampfer entfernt. Das erhaltene Rohprodukt wurde mittels MPLC gereinigt (Kieselgel, Cyclohexan/Ethylacetat 2:1). Es wurden 418 mg (36% d. Th., 93% Reinheit) der Titelverbindung erhalten, welche ohne weitere Reinigung eingesetzt wurde.

¹H-NMR (400 MHz, DMSO-d₆, δ/ppm): 8.39 (d, 1H), 8.08 (d, 2H), 7.68 (dd, 1H), 7.62 (d, 2H), 7.52 (d, 1H), 6.93 (s, 1H), 5.56 (s, 2H), 2.39 (s, 3H), 1.72 (s, 3H), 1.86 (s, 3H).

LC/MS (Methode F, ESIpos): Rₜ = 1.43 min, m/z = 412 [M+H]⁺.

Die Verbindungen in der folgenden Tabelle wurden analog zu einem der unter Beispiel 51A und 52A beschriebenen Verfahren aus den entsprechenden Vorstufen hergestellt. Die Herstellung der meisten der eingesetzten *N*'-Hydroxycarboximidamide (Hydroxyamidine) wurde weiter oben beschrieben; einige wenige waren kommerziell erhältlich oder ihre Herstellung ist in der Literatur beschrieben.

| **Beispiel** | **Struktur** | **HPLC: Rₜ [min]** | **MS: m/z [M+H]⁺** | **LC/MS-Methode** |
|---|---|---|---|---|
| **53A** | 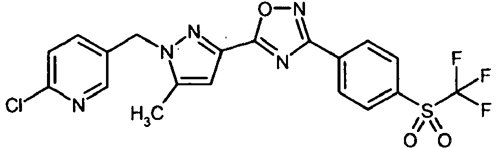 | 2.39 | 484 | E |
| | ¹H-NMR (400 MHz, DMSO-d₆, δ/ppm): 8.50 (d, 2H), 8.42-8.33 (m, 3H), 7.70 (dd, 1H), 7.53 (d, 2H), 6.98 (s, 1H), 5.56 (s, 2H), 2.39 (s, 3H). | | | |
| **54A** | 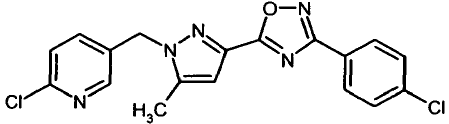 | 1.42 | 386 | F |
| | ¹H-NMR (400 MHz, CDCl₃, δ/ppm): 8.32 (d, 2H), 8.14 (d, 2H), 7.51 (dd, 1H), 7.48 (d, 2H), 7.31 (d, 1H), 6.82 (s, 1H), 5.43 (s, 2H), 2.32 (s, 3H). | | | |
| **55A** | 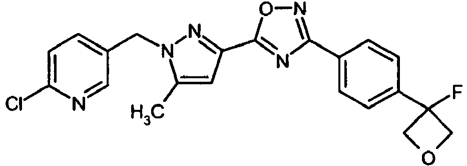 | 1.14 | 426 | I |
| | ¹H-NMR (400 MHz, CDCl₃, δ/ppm): 8.32 (d, 1H), 8.28 (d, 2H), 7.72 (d, 2H), 7.52 (dd, 1H), 7.33 (d, 1H), 6.84 (s, 1H), 5.45 (s, 2H), 5.05 (dd, 2H), 5.00 (dd, 2H), 2.33 (s, 3H). | | | |
| **56A** | 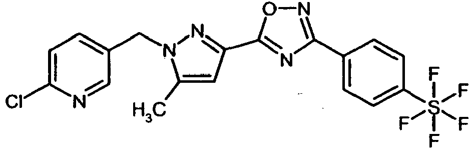 | 1.50 | 478 | F |
| | ¹H-NMR (400 MHz, CDCl₃, δ/ppm): 8.31 (m, 3H), 7.89 (d, 2H), 7.52 (dd, 1H), 7.32 (d, 2H), 6.84 (s, 1H), 5.44 (s, 2H), 2.32 (s, 3H). | | | |
| **57A** | 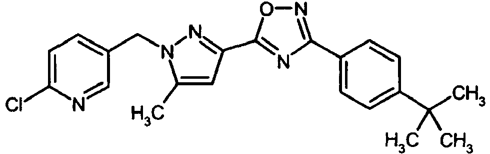 | 5.10 | 408 | A |
| | ¹H-NMR (400 MHz, CDCl₃, δ/ppm): 8.31 (d, 1H), 8.13 (d, 2H), 7.51 (d, 2H), 7.51 (dd, 1H), 7.32 (d, 1H), 6.83 (s, 1H), 5.44 (s, 2H), 2.32 (s, 3H), 1.36 (s, 9H). | | | |
| **58A** | 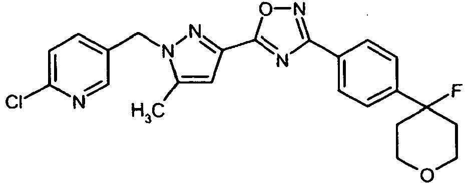 | 4.60 | 454 | A |
| | ¹H-NMR (400 MHz, CDCl₃, δ/ppm): 8.32 (d, 1H), 8.23 (d, 2H), 7.53 (d, 2H), 7.51 (dd, 1H), 7.32 (d, 1H), 6.83 (s, 1H), 5.44 (s, 2H), 4.00-3.86 (m, 4H), 2.33 (s, 3H), 2.29-2.12 (m, 2H), 1.98-1.92 (m, 2H). | | | |
| **59A** | 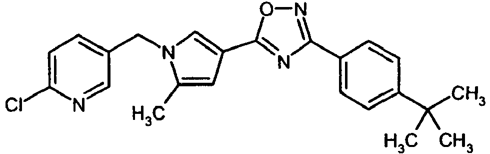 | 5.20 | 407 | A |
| | ¹H-NMR (400 MHz, CDCl₃, δ/ppm): 8.25 (d, 1H), 8.03 (d, 2H), 7.50 (d, 2H), 7.47 (d, 1H), 7.33 (d, 1H), 7.30 (dd, 1H), 6.60 (d, 1H), 5.10 (s, 2H), 2.20 (s, 3H), 1.37 (s, 9H). | | | |
| **60A** | 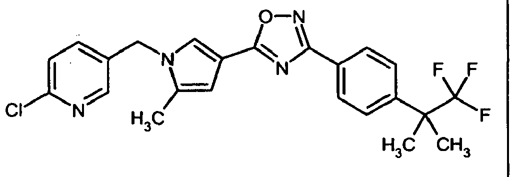 | 1.40 | 461 | I |
| | ¹H-NMR (400 MHz, CDCl₃, δ/ppm): 8.24 (d, 1H), 8.10 (d, 2H), 7.61 (d, 2H), 7.50 (d, 1H), 7.34-7.30 (m, 2H), 6.60 (d, 1H), 5.13 (s, 2H), 2.31 (s, 6H), 2.21 (s, 3H). | | | |
| **61A** | 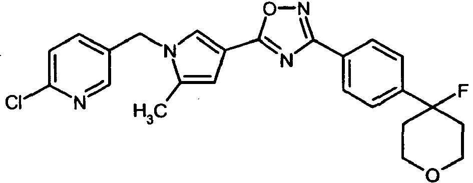 | 4.74 | 453 | A |
| | ¹H-NMR (400 MHz, CDCl₃, δ/ppm): 8.25 (d, 1H), 8.14 (d, 2H), 7.51 (d, 2H), 7.48 (d, 1H), 7.33 (d, 1H), 7.28 (dd, 1H), 6.60 (d, 1H), 5.11 (s, 2H), 4.00-3.87 (m, 4H), 2.29-2.11 (m, 2H), 2.21 (s, 3H), 1.98-1.91 (m, 2H). | | | |
| **62A** | 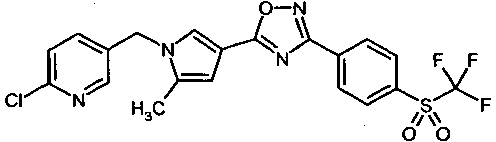 | 4.95 | 483 | A |
| | ¹H-NMR (400 MHz, CDCl₃, δ/ppm): 8.43 (d, 2H), 8.24 (d, 1H), 7.16 (d, 2H), 7.50 (d, 1H), 7.33 (d, 1H), 7.30 (dd, 1H), 6.61 (d, 1H), 5.12 (s, 2H), 2.22 (s, 3H). | | | |
| **63A** | 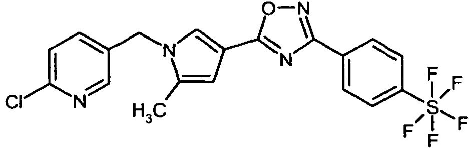 | 1.40 | 477 | I |
| | ¹H-NMR (400 MHz, CDCl₃, δ/ppm): 8.25-8.20 (m, 3H), 7.87 (d, 2H), 7.49 (d, 1H), 7.33 (d, 1H), 7.29 (dd, 1H), 6.60 (d, 1H), 5.12 (s, 2H), 2.21 (s, 3H). | | | |
| **64A** | 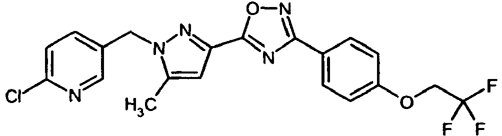 | 1.23 | 450 | I |
| | ¹H-NMR (400 MHz, CDCl₃, δ/ppm): 8.32 (d, 1 H), 8.18 (d, 2H), 7.51 (dd, 1H), 7.32 (d, 1H), 7.05 (d, 2H), 6.82 (s, 1H), 5.44 (s, 2H), 4.43 (quart, 2H), 2.33 (s, 3H). | | | |
| **65A** | 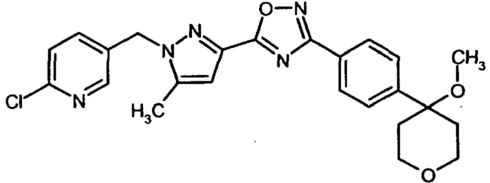 | 1.18 | 466 | I |
| | ¹H-NMR (400 MHz, CDCl₃, δ/ppm): 8.32 (d, 1H), 8.21 (d, 2H), 7.53 (d, 2H), 7.52 (dd, 1H), 7.32 (d, 1H), 6.84 (s, 1H), 5.44 (s, 2H), 3.93-3.83 (m, 4H), 3.01 (s, 3H), 2.33 (s, 3H), 2.11-1.98 (m, 4H). | | | |
| **66A** | 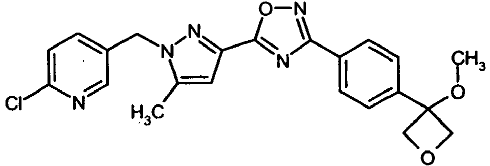 | 1.11 | 438 | I |
| | ¹H-NMR (400 MHz, CDCl₃, δ/ppm): 8.32 (d, 1H), 8.26 (d, 2H), 7.61 (d, 2H), 7.52 (dd, 1H), 7.32 (d, 1H), 6.84 (s, 1H), 5.45 (s, 2H), 4.97 (d, 2H), 4.85 (d, 2H), 3.19 (s, 3H), 2.33 (s, 3H). | | | |
| **67A** | 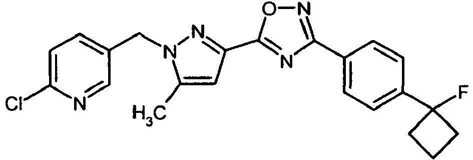 | 1.32 | 424 | I |
| | ¹H-NMR (400 MHz, CDCl₃, δ/ppm): 8.32 (d, 1H), 8.22 (d, 2H), 7.60 (d, 2H), 7.51 (dd, 1H), 7.32 (d, 1H), 6.84 (s, 1H), 5.44 (s, 2H), 2.77-2.55 (m, 4H), 2.33 (s, 3H), 2.20-2.08 (m, 1H), 1.87-1.75 (m, 1H). | | | |
| **68A** | 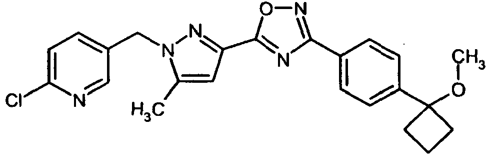 | 1.30 | 436 | I |
| | ¹H-NMR (400 MHz, CDCl₃, δ/ppm): 8.32 (d, 1H), 8.20 (d, 2H), 7.56 (d, 2H), 7.52 (dd, 1H), 7.32 (d, 1H), 6.84 (s, 1H), 5.44 (s, 2H), 2.97 (s, 3H), 2.44-2.41 (m, 4H), 2.33 (s, 3H), 2.03-1.93 (m, 1H), 1.78-1.67 (m, 1H). | | | |
| **69A** | 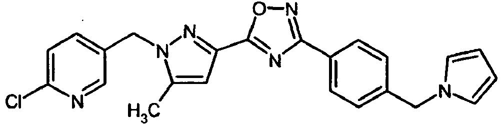 | 1.24 | 431 | I |
| | ¹H-NMR (400 MHz, CDCl₃, δ/ppm): 8.31 (d, 1H), 8.13 (d, 2H), 7.50 (dd, 1H), 7.31 (d, 1H), 7.22 (d, 2H), 6.82 (s, 1H), 6.72 (s, 2H), 6.22 (s, 2H), 5.42 (s, 2H), 5.13 (s, 2H), 2.31 (s, 3H). | | | |
| **70A** | 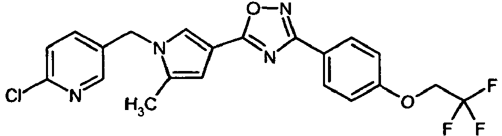 | 1.30 | 449 | I |
| | ¹H-NMR (400 MHz, CDCl₃, δ/ppm): 8.25 (d, 1H), 8.10 (d, 2H), 7.46 (d, 1H), 7.33 (d, 1H), 7.27 (dd, 1H), 7.04 (d, 2H), 6.59 (d, 1H), 5.10 (s, 2H), 5.03 (s, 2H), 4.42 (quart, 2H), 2.20 (s, 3H). | | | |
| **71A** | 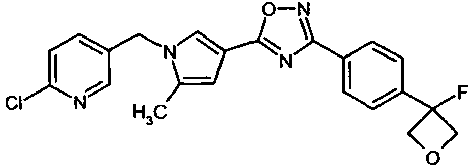 | 1.21 | 425 | I |
| | ¹H-NMR (400 MHz, CDCl₃, δ/ppm): 8.25 (d, 1H), 8.20 (d, 2H), 7.70 (d, 2H), 7.49 (d, 1H), 7.33 (d, 1H), 7.28 (dd, 1H), 6.61 (d, 1H), 5.11 (s, 2H), 5.05 (dd, 2H), 5.00 (dd, 2H), 2.21 (s, 3H). | | | |
| **72A** | 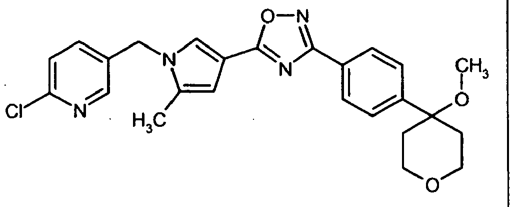 | 1.38 | 465 | F |
| | ¹H-NMR (400 MHz, CDCl₃, δ/ppm): 8.25 (d, 1H), 8.12 (d, 2H), 7.51 (d, 2H), 7.48 (d, 1H), 7.33 (d, 1H), 7.28 (dd, 1H), 6.60 (d, 1H), 5.11 (s, 2H), 3.94-3.81 (m, 4H), 3.01 (s, 3H), 2.20 (s, 3H), 2.11-1.97 (m, 4H). | | | |
| **73A** | 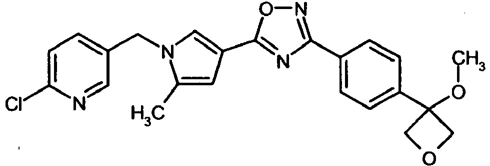 | 1.17 | 437 | I |
| | ¹H-NMR (400 MHz, CDCl₃, δ/ppm): 8.25 (d, 1H), 8.18 (d, 2H), 7.59 (d, 2H), 7.48 (d, 1H), 7.33 (d, 1H), 7.29 (dd, 1H), 6.61 (d, 1H), 5.11 (s, 2H), 4.96 (d, 2H), 4.85 (d, 2H), 3.17 (s, 3H), 2.21 (s, 3H). | | | |
| **74A** | 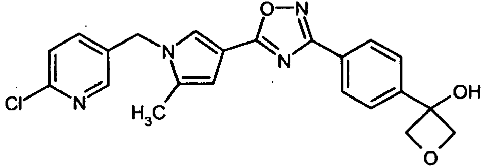 | 4.17 | 423 | A |
| | ¹H-NMR (400 MHz, CDCl₃, δ/ppm): 8.25 (d, 1H), 8.17 (d, 2H), 7.75 (d, 2H), 7.48 (d, 1H), 7.33 (d, 1H), 7.28 (dd, 1H), 6.61 (d, 1H), 5.11 (s, 2H), 4.97-4.94 (m, 4H), 2.78 (breit, 1H), 2.20 (s, 3H). | | | |

### Beispiel 75A

### 2-Brom-6-[(5-methyl-3-{3-[4-(trifluormethoxy)phenyl]-1,2,4-oxadiazol-5-yl}-1H-pyrazol-1-yl)-methyl]pyridin

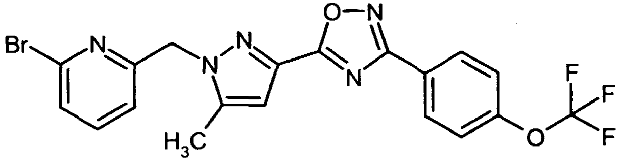

Eine Lösung von 1.83 g (5.90 mmol) der Verbindung aus Beispiel 28A und 2.04 g (7.67 mmol) (6-Brompyridin-2-yl)methylmethansulfonat [T. Kawano et al., Bull. Chem. Soc. Jpn. 2003, 76 (4), 709-720] in 50 ml wasserfreiem THF wurde bei 0°C mit 0.73 g (6.49 mmol) festem Kalium-tert.-butylat versetzt. Man ließ das Reaktionsgemisch danach auf RT kommen. Nach 1.5 h wurden ca. 100 ml Wasser zugesetzt, und es wurde dreimal mit je ca. 100 ml Ethylacetat extrahiert. Die vereinigten organischen Extrakte wurden über wasserfreiem Natriumsulfat getrocknet, und nach Filtration wurde das Lösungsmittel am Rotationsverdampfer entfernt. Der erhaltene Rückstand wurde mit 30 ml Dichlormethan verrührt. Nach Filtration und Trocknen des Filter-Rückstands wurde so eine erste Menge von 1.21 g (43% d. Th.) der Titelverbindung erhalten. Die Mutterlauge wurde am Rotationsverdampfer vom Lösungsmittel befreit und der Rückstand mittels MPLC gereinigt (Kieselgel, Cyclohexan/Ethylacetat 4:1 → 1:1). Auf diese Weise wurden weitere 0.42 g (16% d. Th.) der Titelverbindung erhalten.

¹H-NMR (400 MHz, DMSO-d₆, δ/ppm): 8.20 (d, 2H), 7.78 (t, 1H), 7.63-7.58 (m, 3H), 7.18 (d, 1H), 6.96 (s, 1H), 5.60 (s, 2H), 2.39 (s, 3H).

LC/MS (Methode F, ESIpos): Rₜ = 1.53 min, m/z = 480/482 (⁷⁹Br/⁸¹Br) [M+H]⁺.

### Beispiel 76A

### 5-Iod-2-[(5-methyl-3-{3-[4-(trifluormethoxy)phenyl]-1,2,4-oxadiazol-5-yl}-1H-pyrazol-1-yl)-methyl]pyridin

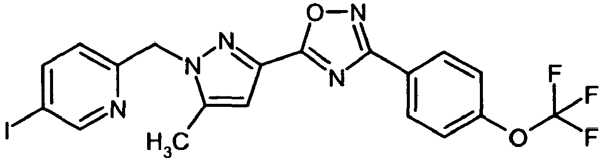

Eine Lösung von 504 mg (1.62 mmol) der Verbindung aus Beispiel 28A und 535 mg (2.11 mmol) der Verbindung aus Beispiel 44A in 20 ml wasserfreiem THF wurde bei 0°C mit 219 mg (1.95 mmol) festem Kalium-*tert*.-butylat versetzt. Man ließ das Reaktionsgemisch danach auf RT kommen. Nach 15 h wurden ca. 100 ml Wasser zugesetzt, und es wurde dreimal mit je ca. 100 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt. Die Titelverbindung wurde mittels präparativer HPLC (Methode N) isoliert. Es wurden 657 mg (77% d. Th.) erhalten.

¹H-NMR (400 MHz, CDCl₃, δ/ppm): 8.79 (d, 1H), 8.24 (d, 2H), 7.97 (dd, 1H), 7.33 (d, 2H), 6.86 (d, 1H), 6.83 (s, 1H), 5.50 (s, 2H), 2.36 (s, 3H).

HPLC (Methode B): Rₜ = 5.25 min.

MS (ESIpos): m/z = 528 [M+H]⁺.

Die Verbindungen in der folgenden Tabelle wurden analog zu den in Beispiel 75A und 76A beschriebenen Verfahren aus den korrespondierenden Edukten hergestellt. In Abhängigkeit von der Polarität der Verbindungen wurden sie entweder durch Ausrühren aus Dichlormethan, Ethylacetat, Acetonitril oder Diethylether, mittels präparativer HPLC oder mittels MPLC über Kieselgel mit Cyclohexan/Ethylacetat-Gemischen als Laufmittel isoliert. Die als Edukte verwendeten Arylmethylchloride, -bromide oder -methansulfonate waren entweder kommerziell erhältlich oder sie wurden wie weiter oben beschrieben hergestellt oder ihre Herstellung ist in der Literatur beschrieben: (6-Chlorpyridin-3-yl)methylmethansulfonat [K.C. Iee et al., J. Org. Chem. 1999, 64 (23), 8576-8581].

| **Beispiel** | **Struktur** | **HPLC: Rₜ [min]** | **MS: m/z [M+H]⁺** | **LC/MS-Methode** |
|---|---|---|---|---|
| **77A** | 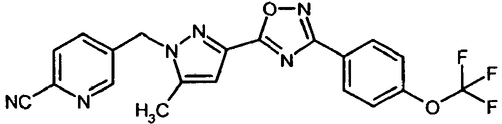 | 2.70 | 427 | C |
| | ¹H-NMR (400 MHz, CDCl₃, δ/ppm): 8.62 (d, 1H), 8.24 (d, 2H), 7.70 (d, 1H), 7.62 (dd, 1H), 7.34 (d, 2H), 6.88 (s, 1H), 5.52 (s, 2H), 2.34 (s, 3H). | | | |
| **78A** | 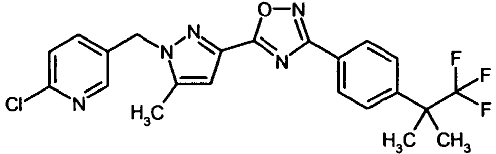 | 2.94 | 462 | C |
| | ¹H-NMR (400 MHz, CDCl₃, δ/ppm): 8.33 (d, 1H), 8.18 (d, 2H), 7.63 (d, 2H), 7.52 (dd, 1H), 7.32 (d, 1H), 6.84 (s, 1H), 5.45 (s, 2H), 2.32 (s, 3H), 1.63 (s, 6H). | | | |
| **79A** | 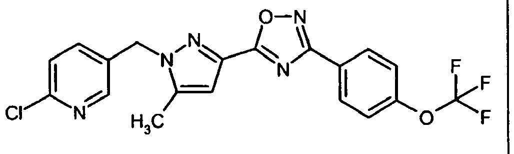 | 2.83 | 436 | C |
| | ¹H-NMR (400 MHz, CDCl₃, δ/ppm): 8.31 (d, 1H), 8.25 (d, 2H), 7.51 (dd, 1H), 7.36-7.30 (m, 3H), 6.82 (s, 1H), 5.43 (s, 2H), 2.32 (s, 3H). | | | |
| **80A** | 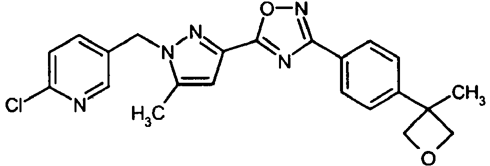 | 1.26 | 422 | F |
| | ¹H-NMR (400 MHz, CDCl₃, δ/ppm): 8.32 (d, 1H), 8.20 (d, 2H), 7.51 (dd, 1H), 7.33 (d, 2H), 7.31 (d, 1H), 6.83 (s, 1H), 5.44 (s, 2H), 5.00 (d, 2H), 4.58 (d, 2H), 2.33 (s, 3H), 1.77 (s, 3H). | | | |
| **81A** | 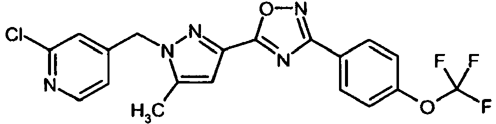 | 1.47 | 436 | F |
| | ¹H-NMR (400 MHz, CDCl₃, δ/ppm): 8.37 (d, 1H), 8.28-8.22 (m, 2H), 7.34 (d, 2H), 7.05 (s, 1H), 6.97 (d, 1H), 6.88 (s, 1H), 5.43 (s, 2H), 2.32 (s, 3H). | | | |
| **82A** | 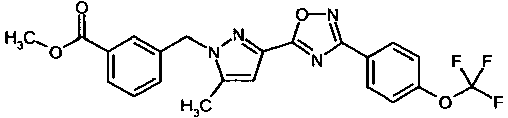 | 1.55 | 459 | F |
| | ¹H-NMR (400 MHz, CDCl₃, δ/ppm): 8.25 (d, 2H), 7.99 (d, 1H), 7.89 (s, 1H), 7.42 (dd, 1H), 7.33 (d, 2H und d, 1H), 6.82 (s, 1H), 5.50 (s, 2H), 3.90 (s, 3H), 2.29 (s, 3H). | | | |
| **83A** | 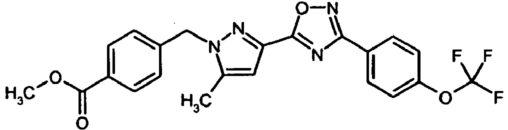 | 1.54 | 459 | F |
| | ¹H-NMR (400 MHz, CDCl₃, δ/ppm): 8.25 (d, 2H), 8.01 (d, 2H), 7.33 (d, 2H), 7.21 (d, 2H), 6.83 (s, 1H), 5.52 (s, 2H), 3.91 (s, 3H), 2.27 (s, 3H). | | | |
| **84A** | 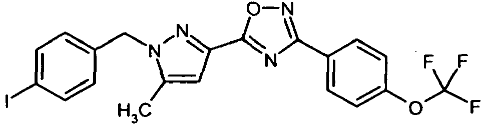 | 2.85 | 527 | E |
| | ¹H-NMR (400 MHz, CDCl₃, δ/ppm): 8.25 (d, 2H), 7.67 (d, 2H), 7.32 (d, 2H), 6.91 (d, 2H), 6.81 (s, 1H), 5.39 (s, 2H), 2.27 (s, 3H). | | | |
| **85A** | 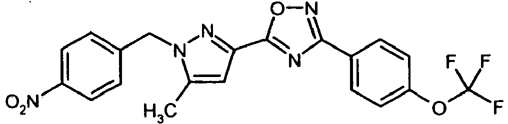 | 5.20 | 446 | B |
| | ¹H-NMR (400 MHz, CDCl₃, δ/ppm): 8.18 (d, 2H), 8.14 (d, 2H), 7.27 (d, 2H), 7.24 (d, 2H), 6.80 (s, 1H), 5.48 (s, 2H), 2.23 (s, 3H). | | | |
| **86A** | 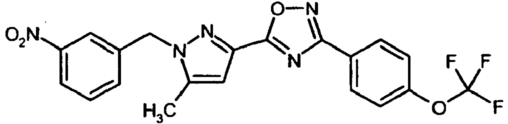 | 1.52 | 446 | F |
| | ¹H-NMR (400 MHz, CDCl₃, δ/ppm): 8.25 (d, 2H), 8.19 (d, 1H), 8.04 (s, 1H), 7.54 (dd, 1H), 7.49 (d, 1H), 7.33 (d, 2H), 6.84 (s, 1H), 5.55 (s, 2H), 2.33 (s, 3H). | | | |
| **87A** | 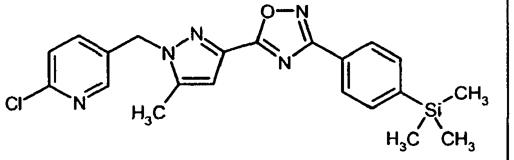 | 1.45 | 424 | I |
| | ¹H-NMR (400 MHz, DMSO-d₆, δ/ppm): 8.32 (dd, 1H), 8.16 (d, 2H), 7.63 (d, 2H), 7.52-7.49 (dd, 1H), 7.31 (d, 1H), 6.82 (s, 1H), 5.42 (s, 2H), 2.32 (s, 3H), 0.31 (s, 9H). | | | |
| **88A** | 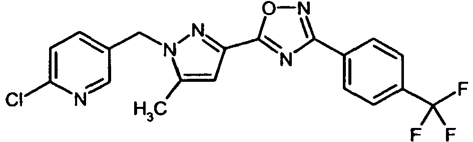 | 1.30 | 420 | I |
| | ¹H-NMR (400 MHz, CDCl₃, δ/ppm): 8.34-8.30 (m, 3H), 7.77 (d, 2H), 7.52 (dd, 1H), 7.31 (d, 1H), 6.84 (s, 1H), 5.44 (s, 2H), 2.32 (s, 3H). | | | |
| **89A** | 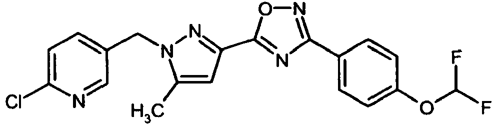 | 1.21 | 418 | I |
| | ¹H-NMR (400 MHz, CDCl₃, δ/ppm): 8.32 (d, 1H), 8.21 (d, 2H), 7.51 (dd, 1H), 7.31 (d, 1H), 7.22 (d, 2H), 6.82 (s, 1H), 6.60 (t, 1H), 5.43 (s, 2H), 2.32 (s, 3H). | | | |
| **90A** | 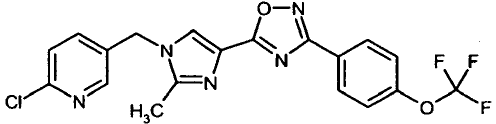 | 1.29 | 436 | F |
| | ¹H-NMR (400 MHz, CDCl₃, δ/ppm): 8.33 (s, 1H), 8.22 (d, 2H), 7.71 (s, 1H), 7.39 (s, 2H), 7.32 (d, 2H), 5.18 (s, 2H), 2.50 (s, 3H). | | | |

### Beispiel 91A

### 5-[(5-Methyl-3-{3-[4-(trifluormethoxy)phenyl]-1,2,4-oxadiazol-5-yl}-1H-pyrazol-1-yl)methyl]-pyridin-2-carbaldehyd

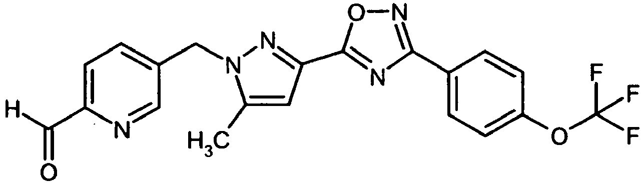

Unter inerten Bedingungen und bei -78°C wurde eine Lösung von 980 mg (2.30 mmol) der Verbindung aus Beispiel 77A in 30 ml wasserfreiem THF mit 3.5 ml (3.5 mmol) einer 1 M Lösung von Diisobutylalumiumhydrid (DIBAL-H) in Heptan versetzt. Nachdem das Reaktionsgemisch 3 h bei -78°C gerührt worden war, wurden 22 ml 1 M Salzsäure zugesetzt. Unter Rühren ließ man das Gemisch auf RT erwärmen. Es wurde dann mit Ethylacetat extrahiert. Der organische Extrakt wurde nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Das Rohprodukt wurde mittels MPLC gereinigt (Kieselgel, Cyclohexan/Ethylacetat 1:1). Es wurden 300 mg (30% d. Th.) der Titelverbindung erhalten.

¹H-NMR (400 MHz, CDCl₃, δ/ppm): 10.07 (s, 1H), 8.67 (d, 1H), 8.25 (d, 2H), 7.95 (d, 1H), 7.67 (dd, 1H), 7.34 (d, 2H), 6.87 (s, 1H), 5.57 (s, 2H), 2.35 (s, 3H).

MS (DCI, NH₃): m/z = 430 [M+H]⁺.

LC/MS (Methode C, ESIpos): Rₜ = 2.66 min, m/z = 430 [M+H]⁺.

### Beispiel 92A

### 5-[(5-Methyl-3-{3-[4-(trifluormethoxy)phenyl]-1,2,4-oxadiazol-5-yl}-1H-pyrazol-1-yl)methyl]-pyridin-2-carbonsäure

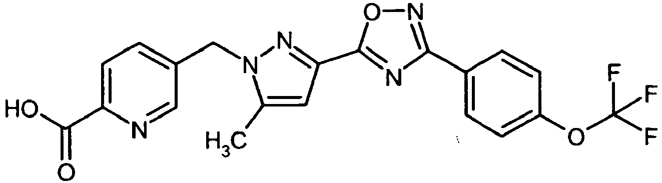

Eine Lösung von 500 mg (1.17 mmol) der Verbindung aus Beispiel 77A in 5 ml Ethanol wurde mit 5 ml einer 30%-igen Kaliumhydroxid-Lösung in Wasser versetzt und 1 h zum Rückfluss erhitzt. Nach dem Abkühlen auf RT wurde mit ca. 20 ml Wasser versetzt und mit konzentrierter Salzsäure das Produkt ausgefällt. Dieses wurde abfiltriert, mit Wasser neutral gewaschen und im Hochvakuum getrocknet. Es wurden 448 mg (86% d. Th.) der Titelverbindung erhalten.

¹H-NMR (400 MHz, CDCl₃, δ/ppm): 8.52 (d, 1H), 8.24 (d, 2H), 8.22 (d, 1H), 7.75 (dd, 1H), 7.33 (d, 2H), 6.88 (s, 1H), 5.57 (s, 2H), 2.36 (s, 3H).

MS (DCI, NH₃): m/z = 446 [M+H]⁺.

LC/MS (Methode F, ESIpos): Rₜ = 1.22 min, m/z = 446 [M+H]⁺.

### Beispiel 93A

### 3-[(5-Methyl-3-{3-[4-(trifluormethoxy)phenyl]-1,2,4-oxadiazol-5-yl}-1H-pyrazol-1-yl)methyl]-benzolcarbonsäure

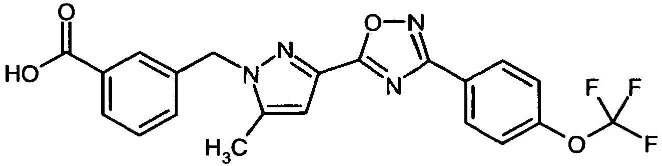

Eine Suspension von 8.13 g (17.7 mmol) der Verbindung aus Beispiel 82A in 120 ml Methanol wurde mit 89 ml (88.7 mmol) einer 1 M Natronlauge versetzt und 1 h zum Rückfluss erhitzt. Anschließend wurde das Methanol am Rotationsverdampfer weitestgehend entfernt. Die zurückgebliebene wässrige Lösung wurde unter Rühren mit 100 ml 1 M Salzsäure angesäuert. Dabei fiel das Produkt aus, welches abgesaugt, mit Wasser gewaschen und im Hochvakuum getrocknet wurde. Es wurden 7.51 g (95% d. Th.) der Titelverbindung erhalten.

¹H-NMR (400 MHz, DMSO-d₆, δ/ppm): 13.07 (s, breit, 1H), 8.20 (d, 2H), 7.40 (d, 1H), 7.78 (s, 1H), 7.59 (d, 2H), 7.51 (dd, 1H), 7.46 (d, 1H), 6.97 (s, 1H), 5.60 (s, 2H), 2.34 (s, 3H).

LC/MS (Methode C, ESIpos): Rₜ = 2.68 min, m/z = 445 [M+H]⁺.

Analog zu dem in Beispiel 93A beschriebenen Verfahren wurde die Verbindung in der folgenden Tabelle durch Verseifen des korrespondierenden Esters erhalten:

| **Beispiel** | **Struktur** | **HPLC: Rₜ [min]** | **MS: m/z [M+H]⁺** | **LC/MS-Methode** |
|---|---|---|---|---|
| **94A** | 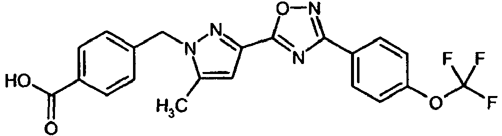 | 2.56 | 445 | D |
| | ¹H-NMR (400 MHz, DMSO-d₆, δ/ppm): 13.00 (breit, 1H), 8.20 (d, 2H), 7.94 (d, 2H), 7.59 (d, 2H), 7.29 (d, 2H), 6.96 (s, 1H), 5.60 (s, 2H), 2.33 (s, 3H). | | | |

### Beispiel 95A

### 3-[(5-Methyl-3-{3-[4-(trifluormethoxy)phenyl]-1,2,4-oxadiazol-5-yl}-1H-pyrazol-1-yl)methyl]-phenol

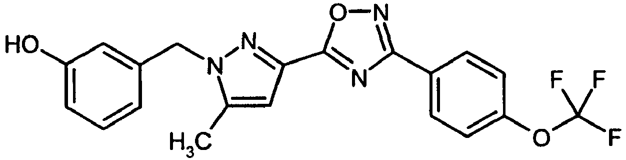

Eine Lösung von 500 mg (1.61 mmol) der Verbindung aus Beispiel 28A und 719 mg (2.10 mmol) der Verbindung aus Beispiel 47A in 10 ml wasserfreiem THF wurde bei 0°C mit 199 mg (1.77 mmol) festem Kalium-*tert*.-butylat versetzt. Man ließ das Reaktionsgemisch danach auf RT kommen. Nach 15 h wurden ca. 100 ml Wasser zugesetzt, und es wurde dreimal mit je ca. 100 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt. Der so erhaltene Rückstand wurde erneut in 20 ml THF gelöst und bei 0°C mit 3.2 ml (3.2 mmol) einer 1 M Lösung von Tetra-n-butylammoniumfluorid in THF versetzt. Nachdem das Gemisch 1 h bei RT gerührt worden war, wurde der Ansatz mit einigen ml Methanol verdünnt und direkt mittels präparativer HPLC (Methode N) in seine Komponenten aufgetrennt. Es wurden 218 mg (32% d. Th.) der Titelverbindung erhalten.

¹H-NMR (400 MHz, CDCl₃, δ/ppm): 8.11 (d, 2H), 7.29 (d, 2H), 7.20 (t, 1H), 6.80 (d, 1H), 6.79 (s, 1H), 6.73 (d, 1H), 6.62 (s, 1H), 6.50 (s, 1H), 5.33 (s, 2H), 2.06 (s, 3H).

HPLC (Methode A): Rₜ = 4.81 min.

MS (DCI, NH₃): m/z = 417 [M+H]⁺.

LC/MS (Methode E, ESIpos): Rₜ = 2.34 min, m/z = 417 [M+H]⁺.

Analog zu dem unter Beispiel 95A beschriebenen Verfahren wurde aus den korrespondierenden Edukten die Verbindung in der folgenden Tabelle erhalten:

| **Beispiel** | **Struktur** | **HPLC: Rₜ [min]** | **MS: m/z [M+H]⁺** | **LC/MS-Methode** |
|---|---|---|---|---|
| **96A** | 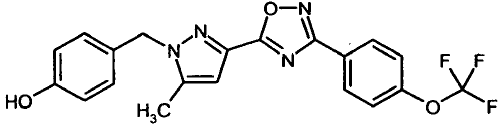 | 2.55 | 417 | D |
| | ¹H-NMR (400 MHz, CDCl₃, δ/ppm): 8.24 (d, 2H), 7.32 (d, 2H), 7.07 (d, 2H), 6.80 (s, 1H), 6.79 (d, 2H), 5.37 (s, 2H), 5.31 (s, breit, 1H), 2.28 (s, 3H). | | | |

### Beispiel 97A

### 4-[(5-Methyl-3-(3-[4-(trifluormethoxy)phenyl]-1,2,4-oxadiazol-5-y1)-1H-pyrazol-1-yl)methyl]-anilin

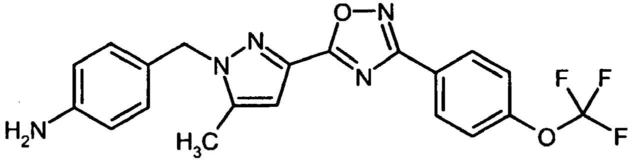

In einer Durchfluss-Hydrierapparatur ("H-Cube" der Firma ThalesNano, Budapest, Ungarn) wurde eine Lösung von 400 mg (0.898 mmol) der Verbindung aus Beispiel 85A in einem Gemisch aus 25 ml Ethanol und 25 ml Ethylacetat hydriert (Bedingungen: 10% Pd/C-Katalysator, "full H₂"-Modus, 1 ml/min, 25°C). Nach Entfernen des Lösungsmittels wurde der Rückstand in einigen ml Ethanol aufgenommen und vom Ungelösten abfiltriert. Bei diesem Ungelösten handelte es sich um Eduktmaterial, das daraufhin noch einmal, wie oben beschrieben, hydriert wurde. Das aus beiden Hydrierungen erhaltene Rohprodukt wurde vereinigt und mittels präparativer HPLC (Methode N) aufgereinigt. Es wurden 229 mg (62% d. Th.) der Titelverbindung erhalten.

¹H-NMR (400 MHz, CDCl₃, δ/ppm): 8.25 (d, 2H), 7.33 (d, 2H), 7.01 (d, 2H), 6.87 (s, 1H), 6.63 (d, 2H), 5.33 (s, 2H), 3.69 (breit, 2H), 2.27 (s, 3H).

LC/MS (Methode C, ESIpos): Rₜ = 2.57 min, m/z = 416 [M+H]⁺.

Die Verbindung in der folgenden Tabelle wurde analog zu dem unter Beispiel 97A beschriebenen Verfahren aus der entsprechenden Nitroverbindung durch Hydrierung hergestellt:

| **Beispiel** | **Struktur** | **HPLC: Rₜ [min]** | **MS: m/z [M+H]⁺** | **LC/MS-Methode** |
|---|---|---|---|---|
| **98A** | 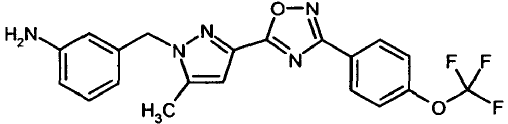 | 2.63 | 416 | D |
| | ¹H-NMR (400 MHz, CDCl₃, δ/ppm): 8.26 (d, 2H), 7.33 (d, 2H), 7.10 (dd, 1H), 6.80 (s, 1H), 6.60 (dd, 1H), 6.55 (dd, 1H), 6.44 (dd, 1H), 5.36 (s, 2H), 3.67 (s, breit, 2H), 2.27 (s, 3H). | | | |

### Beispiel 99A

### tert.-Butyl- {4-[(5-methyl-3-{3-[4-(trifluormethoxy)phenyl]-1,2,4-oxadiazol-5-yl}-1H-pyrazol-1-yl)methyl]phenyl} carbamat

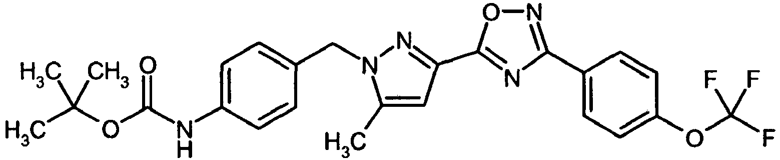

Eine Lösung von 200 mg (0.481 mmol) der Verbindung aus Beispiel 97A in 10 ml wasserfreiem THF wurde mit 134 µl (0.963 mmol) Triethylamin und 3 mg (0.024 mmol) DMAP versetzt. Es wurde auf 0°C abgekühlt und 132 mg (0.602 mmol) Di-*tert*.-butyldicarbonat zugefügt. Das Reaktionsgemisch wurde 1 h bei 0°C und dann weitere 16 h bei RT gerührt. Danach wurde mit 5 ml Methanol verdünnt und das Produkt in zwei Portionen mittels präparativer HPLC (Methode N) isoliert. Es wurden 74 mg (30% d. Th.) der Titelverbindung erhalten.

¹H-NMR (400 MHz, CDCl₃, δ/ppm): 8.26 (d, 2H), 7.33 (2 d, zus. 4H), 7.12 (d, 2H), 6.79 (s, 1H), 6.49 (s, breit, 1H), 5.39 (s, 2H), 2.26 (s, 3H), 1.50 (s, 9H).

LC/MS (Methode E, ESIpos): Rₜ = 2.74 min, m/z = 516 [M+H]⁺.

### Beispiel 100A

### 2-Brom-5-[(5-methyl-3-{3-[4-(trifluormethoxy)phenyl]-1,2,4-oxadiazol-5-yl}-1H-pyrazol-1-yl)- - methyl]pyridin

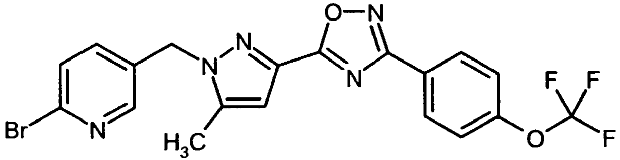

Man erhitzte ein Gemisch von 1.95 g (4.47 mmol) der Verbindung aus Beispiel 79A und 1.37 g (8.95 mmol) Brom(trimethyl)silan in 0.5 ml Propionitril für 70 min unter Rühren in einem Mikrowellengerät auf 120°C (CEM Discover, initiale Einstrahlleistung 250 W). Dabei war in den ersten 10 min ein relativ starker Druck- und Temperaturanstieg zu beobachten. Nach Abkühlen auf RT gab man weitere 350 mg (2.29 mmol) Brom(trimethyl)silan hinzu und erhitzte das Gemisch für weitere 60 min in der Mikrowelle auf 120°C. Dabei war erneut in den ersten 10 min ein relativ starker Druck- und Temperaturanstieg zu beobachten. Nach Abkühlen auf RT verdünnte man das Gemisch mit 100 ml Wasser und 100 ml Ethylacetat und trennte die Phasen. Die organische Phase wurde einmal mit 100 ml Wasser gewaschen, über Natriumsulfat getrocknet, filtriert und am Rotationsverdampfer eingeengt. Der Rückstand wurde säulenchromatographisch an Kieselgel gereinigt (Laufmittel:: Cyclohexan/Ethylacetat 3:2). Es wurden 1.45 g (65% d. Th.) der Titelverbindung mit einer Reinheit von 86% laut LC-MS erhalten. Als Verunreinigung waren ca. 10% des Eduktes (Verbindung aus Beispiel 79A) enthalten.

¹H-NMR (400 MHz, CDCl₃, δ/ppm): 8.31 (d, 1H), 8.23 (d, 2H), 7.47 (d, 1H), 7.40 (dd, 1H), 7.33 (d, 2H), 6.82 (s, 1H), 5.41 (s, 2H), 2.32 (s, 3H).

LC/MS (Methode E, ESIpos): Rₜ = 2.54 min, m/z = 480 [M+H]⁺.

### Beispiel 101A

### 2-Iod-5-[(5-methyl-3-{3-[4-(trifluormethoxy)phenyl]-1,2,4-oxadiazol-5-yl}-1H-pyrazol-1-yl)-methyl]pyridin

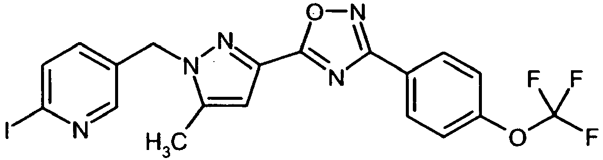

In einem Mikrowellen-Reaktionsgefäß wurden bei RT zu einer Lösung von 100 mg (0.229 mmol) der Verbindung aus Beispiel 79A in 0.5 ml Propionitril 103 mg (0.688 mmol) Natriumiodid und 27 mg (0.252 mmol) Chlor(trimethyl)silan gegeben, woraufhin das Reaktionsgemisch rasch eine feste Konsistenz annahm. Dieses Gemisch wurde anschließend in einem Mikrowellengerät 1 h auf 120°C erhitzt (CEM Discover, initiale Einstrahlleistung 250 W). Nach dem Abkühlen auf RT wurde das Reaktionsgemisch mit 2 ml Acetonitril und 1 ml Wasser verdünnt. Es bildeten sich zwei Flüssigkeitsphasen, welche voneinander getrennt wurden. Die organische Phase wurde ohne weitere Behandlung direkt mittels präparativer HPLC (Methode O) aufgereinigt. Man erhielt 61 mg (50% d. Th.) der Titelverbindung.

¹H-NMR (400 MHz, CDCl₃, δ/ppm): 8.29 (d, 1H), 8.24 (d, 2H), 7.71 (d, 1H), 7.32 (d, 2H), 7.18 (dd, 1H), 6.82 (s, 1H), 5.39 (s, 2H), 2.31 (s, 3H).

LC/MS (Methode F, ESIpos): Rₜ = 1.52 min, m/z = 528 [M+H]⁺.

### Beispiel 102A

### 4-[(5-Methyl-3-{3-[4-(trifluormethoxy)phenyl]-1,2,4-oxadiazol-5-yl}-1H-pyrazol-1-yl)methyl]-pyridin-2-carbonitril

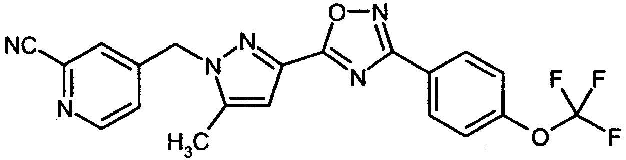

Man legte 200 mg (0.459 mmol) der Verbindung aus Beispiel 81A in 3.4 ml Dimethylacetamid vor, gab bei RT nacheinander 31 mg (0.266 mmol) Zinkcyanid, 6.7 mg (0.020 mmol) Palladium(II)trifluoracetat, 16 mg (0.040 mmol) racemisches 2-(Di-*tert*.-butylphosphino)-1,1'-binaph-thyl sowie 6 mg (0.092 mmol) Zink-Pulver (97.5%, 325 mesh) hinzu und rührte über Nacht bei 90°C. Nach Abkühlen auf RT gab man weitere 6.7 mg (0.020 mmol) Palladium(II)trifluoracetat hinzu und rührte weitere 24 h bei 90°C. Nach Abkühlen auf RT gab man erneut 6.7 mg (0.020 mmol) Palladium(II)trifluoracetat, 16 mg (0.040 mmol) racemisches 2-(Di-tert.-butylphosphino)-1,1'-binaphthyl sowie 6 mg (0.092 mmol) Zink-Pulver (97.5%, 325 mesh) hinzu und rührte abermals über Nacht bei 90°C. Nach Abkühlen auf RT wurden dann die festen Bestandteile abfiltriert und das verbleibende Gemisch mittels präparativer HPLC (Methode O) gereinigt. Die vereinigten produkthaltigen Fraktionen wurden bis auf ein kleines Restvolumen am Rotationsverdampfer eingeengt und anschließend mit Natriumhydrogencarbonat versetzt, worauf ein Feststoff ausfiel. Dieser wurde abfiltriert und im Vakuum getrocknet. Man erhielt so 21 mg (11% d. Th.) der Titelverbindung.

¹H-NMR (400 MHz, CDCl₃, δ/ppm): 8.71 (d, 1H), 8.24 (d, 2H), 7.41 (s, 1H), 7.34 (d, 2H), 7.24 (s, 1H), 6.90 (s, 1H), 5.51 (s, 2H), 2.32 (s, 3H).

LC/MS (Methode D, ESIpos): Rₜ = 2.52 min, m/z = 427 [M+H]⁺.

### Beispiel 103A

### 1-(2-Fluorethyl)piperazin-Dihydrochlorid

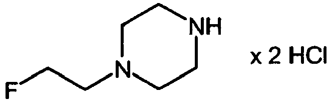

### Schritt 1: tert.-Butyl-4-(2-fluorethyl)piperazin-1-carboxylat

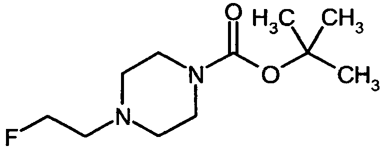

Ein Gemisch aus 1.00 g (5.37 mmol) *tert.*-Butylpiperazin-1-carboxylat, 937 µl (8.05 mmol) 1-Brom-2-fluorethan und 1.86 g (13.4 mmol) Kaliumcarbonat in 15 ml Acetonitril wurde 16 h lang auf 60°C erhitzt. Nach dem Abkühlen auf RT wurde vom Ungelösten abfiltriert und das Filtrat am Rotationsverdampfer vom Lösungsmittel befreit. Der erhaltene Rückstand wurde mittels MPLC gereinigt (Kieselgel; Laufmittel: Cyclohexan/Ethylacetat 1:2). Es wurden 1.12 g (89% d. Th.) der Titelverbindung erhalten.

¹H-NMR (400 MHz, CDCl₃, δ/ppm): 4.58 (td, 2H), 3.46 (t, 4H), 2.71 (td, 2H), 2.48 (t, 4H), 1.46 (s, 9H).

GC/MS (Methode L, EI): Rₜ = 4.74 min, m/z = 232 [M]⁺.

### Schritt 2: 1-(2-Fluorethyl)piperazin-Dihydrochlorid

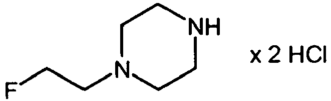

1.10 g (4.72 mmol) der Verbindung aus Beispiel 103A / Schritt 1 wurden mit 30 ml einer 4 M Lösung von Chlorwasserstoff in 1,4-Dioxan versetzt und 16 h bei RT gerührt. Anschließend wurden alle flüchtigen Bestandteile am Rotationsverdampfer entfernt. Der erhaltene Rückstand wurde mit Diethylether verrührt, abgesaugt und mit Diethylether nachgewaschen. Nach Trocknen im Hochvakuum wurden 938 mg (97% d. Th.) der Titelverbindung erhalten.

MS (DCI, NH₃): m/z = 133 [M+H]⁺.

### Beispiel 104A

### 1-(2,2-Difluorethyl)piperazin-Dihydrochlorid

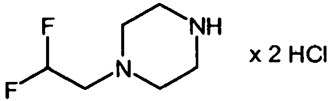

### Schritt 1: tert.-Butyl-4-(2,2-difluorethyl)piperazin-1-carboxylat

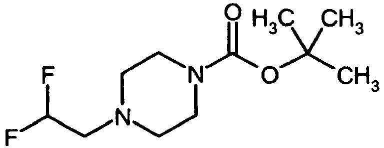

Eine Lösung von 408 µl (6.44 mmol) 2,2-Difluorethanol in 10 ml wasserfreiem Dichlormethan wurde bei 0°C mit 1.35 ml (9.66 mmol) wasserfreiem Triethylamin und 1.27 ml (7.52 mmol) Trifluormethansulfonsäureanhydrid versetzt. Nach 30 min Rühren bei 0°C wurde mit einer Lösung von 1.0 g (5.37 mmol) *tert.*-Butylpiperazin-1-carboxylat in 10 ml wasserfreiem Dichlormethan versetzt. Anschließend ließ man das Reaktionsgemisch auf RT erwärmen. Nach 16 h wurde mit ca. 20 ml Wasser versetzt und die Phasen getrennt. Die organische Phase wurde mit Wasser gewaschen und über wasserfreiem Magnesiumsulfat getrocknet. Nach Filtration wurde das Lösungsmittel am Rotationsverdampfer entfernt und der Rückstand mittels MPLC gereinigt (Kieselgel; Laufmittel: Cyclohexan/Ethylacetat 1:2). Es wurden 538 mg (45% d. Th.) der Titelverbindung erhalten.

¹H-NMR (400 MHz, CDCl₃, δ/ppm): 5.88 (tt, 1H), 3.43 (t, 4H), 2.75 (dt, 2H), 2.53 (t, 4H), 1.45 (s, 9H).

GC/MS (Methode L, EI): Rₜ = 4.41 min, m/z = 250 [M]⁺.

### Schritt 2: 1-(2,2-Difluorethyl)piperazin-Dihydrochlorid

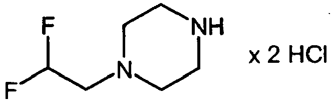

Analog zu dem unter Beispiel 103A / Schritt 2 beschriebenen Verfahren wurden ausgehend von 314 mg (1.26 mmol) der Verbindung aus Beispiel 104A / Schritt 1 257 mg (92% d. Th.) der Titelverbindung erhalten.

MS (DCI, NH₃): m/z = 151 [M+H]⁺.

### Beispiel 105A

### 5-Methyl-1-{3-[(4-methylpiperazin-1-yl)carbonyl]benzyl}-1H-pyrazol-3 -carbonsäure

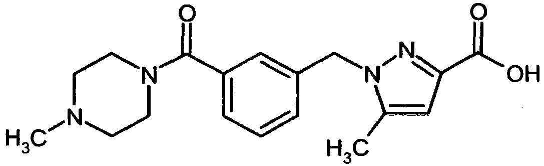

### Schritt 1: Ethyl-1-[3-(tert.-butoxycarbonyl)benzyl]-5-methyl-1H-pyrazol-3-carboxylat

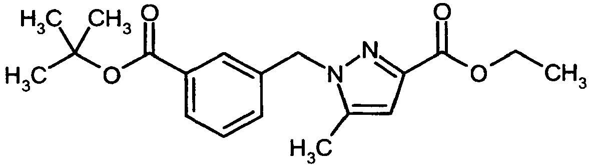

Eine Lösung von 2.38 g (15.4 mmol) Ethyl-5-methyl-1*H*-pyrazol-3-carboxylat und 4.60 g (17.0 mmol) *tert.*-Butyl-3-(brommethyl)benzolcarboxylat in 50 ml wasserfreiem THF wurde bei 0°C mit 1.90 g (17.0 mmol) festem Kalium-*tert.*-butylat versetzt. Das Reaktionsgemisch wurde 16 h bei RT gerührt. Dann wurde mit ca. 250 ml Wasser versetzt und dreimal mit je ca. 150 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Der erhaltene Rückstand wurde mittels MPLC gereinigt (Kieselgel; Laufmittel: Cyclohexan/Ethylacetat 10:1 → 2:1). Es wurden 4.45 g (84% d. Th.) der Titelverbindung erhalten.

¹H-NMR (400 MHz, CDCl₃, δ/ppm): 7.90 (d, 1H), 7.77 (s, 1H), 7.36 (t, 1H), 7.19 (d, 1H), 6.63 (s, 1H), 5.42 (s, 2H), 4.42 (quart, 2H), 2.19 (s, 3H), 1.58 (s, 9H), 1.40 (t, 3H).

MS (DCI, NH₃): m/z = 345 [M+H]⁺.

### Schritt 2: 3-{[3-(Ethoxycarbonyl)-5-methyl-1H-pyrazol-1-yl]methyl}benzolcarbonsäure

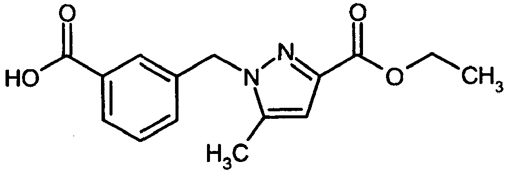

Eine Lösung von 4.47 g (13.0 mmol) der Verbindung aus Beispiel 105A / Schritt 1 in 50 ml Dichlormethan wurde mit 10 ml Trifluoressigsäure versetzt. Nachdem das Reaktionsgemisch 6 h bei RT gerührt worden war, wurden alle flüchtigen Bestandteile am Rotationsverdampfer entfernt. Der erhaltene Rückstand wurde mit Diethylether verrührt und abgesaugt. Nach Trocknen im Hochvakuum wurden 3.19 g (85% d. Th.) der Titelverbindung erhalten.

¹H-NMR (400 MHz, DMSO-d₆, δ/ppm): 13.05 (s, breit, 1H), 7.87 (d, 1H), 7.69 (s, 1H), 7.49 (t, 1H), 7.37 (d, 1H), 6.60 (s, 1H), 5.49 (s, 2H), 4.25 (quart, 2H), 2.24 (s, 3H), 1.27 (t, 3H).

HPLC (Methode A): Rₜ = 3.71 min.

MS (ESIpos): m/z = 289 [M+H]⁺.

LC/MS (Methode F, ESIpos): Rₜ = 0.94 min, m/z = 289 [M+H]⁺.

### Schritt 3: Ethyl-5-methyl-1-{3-[(4-methylpiperazin-1-yl)carbonyl]benzyl}-1H-pyrazol-3-carboxylat

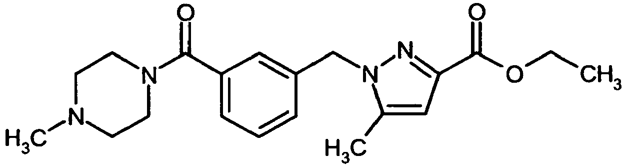

Unter inerten Bedingungen wurden 3.15 g (10.9 mmol) der Verbindung aus Beispiel 105A / Schritt 2 in 100 ml wasserfreiem Dichlormethan gelöst und mit 4.8 ml (54.6 mmol) Oxalylchlorid und einem Tropfen DMF versetzt. Nachdem das Gemisch ca. 2.5 h bei RT gerührt worden war, wurde es am Rotationsverdampfer zur Trockene eingeengt. Der erhaltene Rückstand wurde ca. 1 h im Hochvakuum getrocknet und dann in 40 ml wasserfreiem THF gelöst. Diese Lösung wurde zu einer Lösung von 2.19 g (21.9 mmol) 1-Methylpiperazin und 5.7 ml (32.8 mmol) *N,N-*Diisopropylethylamin in 60 ml wasserfreiem THF hinzugetropft. Nach 16 h Rühren bei RT wurde das Reaktionsgemisch mit ca. 400 ml Wasser verdünnt und dreimal mit je ca. 100 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Lösungsmittel am Rotationsverdampfer entfernt. Es wurden 4.04 g (99% d. Th.) der Titelverbindung erhalten.

¹H-NMR (400 MHz, CDCl₃, δ/ppm): 7.36 (t, 1H), 7.31 (d, 1H), 7.13 (d, 1H), 7.09 (s, 1H), 6.62 (s, 1H), 5.40 (s, 2H), 4.40 (quart, 2H), 3.77 (breit, 2H), 3.36 (breit, 2H), 2.46 (breit, 2H), 2.31 (s, 3H), 2.30 (breit, 2H), 2.20 (s, 3H), 1.40 (t, 3H).

LC/MS (Methode I, ESIpos): Rₜ = 0.61 min, m/z = 371 [M+H]⁺.

### Schritt 4: 5-Methyl-1-{3-[(4-methylpiperazin-1-yl)carbonyl]benzyl}-1H-pyrazol-3-carbonsäure

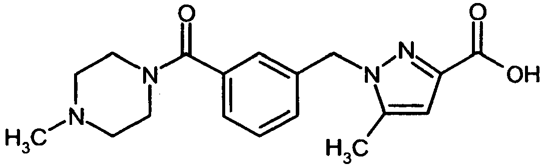

Eine Lösung von 4.0 g (10.8 mmol) der Verbindung aus Beispiel 105A / Schritt 3 in 70 ml Ethanol wurde mit 21.6 ml (21.6 mmol) 1 M Natronlauge versetzt und 2 h auf 70°C erwärmt. Anschließend wurde das Ethanol am Rotationsverdampfer weitestgehend entfernt. Die zurückgebliebene wässrige Lösung wurde bei 0°C unter Rühren mit 3 M Salzsäure versetzt, bis ein pH-Wert von etwa 4 erreicht war. Dabei fiel ein Feststoff aus, der durch Saugfiltration entfernt wurde. Das Filtrat wurde am Rotationsverdampfer zur Trockene eingedampft und der feste Rückstand dann über Nacht mit Dichlormethan verrührt. Nach Filtration wurde das Filtrat am Rotationsverdampfer vom Lösungsmittel befreit. Es wurden 2.35 g (63% d. Th.) der Titelverbindung erhalten.

¹H-NMR (400 MHz, DMSO-d₆, δ/ppm): 7.43 (t, 1H), 7.30 (d, 1H), 7.21 (d, 1H), 7.07 (s, 1H), 6.51 (s, 1H), 5.76 (s, 1H), 5.41 (s, 2H), 3.57 (breit, 2H), 3.24 (breit, 2H), 2.34 (breit, 2H), 2.23 (s, 3H), 2.20 (breit, 2H), 2.18 (s, 3H).

LC/MS (Methode I, ESIpos): Rₜ = 0.33 min, m/z = 343 [M+H]⁺.

### Beispiel 106A

### 5-Methyl-1-{3-[(4-methylpiperazin-1-yl)carbonyl]benzyl}-1H-pyrrol-3-carbonsäure

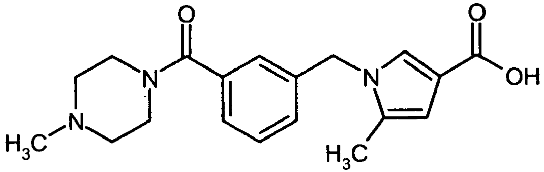

### Schritt 1: 3-[(4-Methylpiperazin-1-yl)carbonyl]benzolcarbonitril

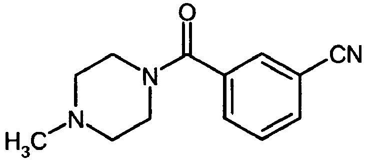

Eine Lösung von 4.57 g (45.6 mmol) 1-Methylpiperazin und 8.5 ml (60.8 mmol) Triethylamin in 100 ml Dichlormethan wurde bei 0°C tropfenweise mit einer Lösung von 3-Cyanobenzoesäurechlorid in 100 ml Dichlormethan versetzt. Anschließend wurde 6 h bei RT gerührt. Dann wurden 200 ml Wasser zugefügt, die Phasen getrennt und die organische Phase mit Wasser gewaschen. Nach dem Trocknen über wasserfreiem Magnesiumsulfat wurde filtriert und das Lösungsmittel am Rotationsverdampfer entfernt. Es wurden 6.9 g (99% d. Th.) der Titelverbindung erhalten.

¹H-NMR (400 MHz, CDCl₃, δ/ppm): 7.72 (d, 1H), 7.70 (s, 1H), 7.65 (d, 1H), 7.55 (t, 1H), 3.80 (breit, 2H), 3.40 (breit, 2H), 2.50 (breit, 2H), 2.37 (breit, 2H), 2.34 (s, 3H).

LC/MS (Methode I, ESIpos): Rₜ = 0.21 min, m/z = 230 [M+H]⁺.

GC/MS (Methode L, ESIpos): Rₜ = 7.36 min, m/z = 229 [M]⁺.

### Schritt 2: [3-(Aminomethyl)phenyl](4-methylpiperazin-1-yl)methanon

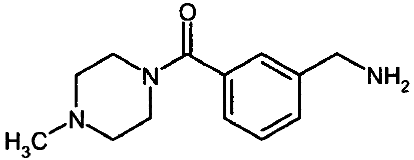

Eine Lösung von 1.0 g (4.36 mmol) der Verbindung aus Beispiel 106A / Schritt 1 in 100 ml Ethanol wurde in einer Durchflusshydrierapparatur hydriert ("H-Cube" der Firma ThalesNano, Budapest, Ungarn; Raney-Nickel-Katalysator, "full H₂"-Modus, 0.5 ml/min, 50°C). Nach dem Abdampfen des Lösungsmittels wurden 1.0 g (99% d. Th.) der Titelverbindung erhalten.

LC/MS (Methode D, ESIpos): Rₜ = 0.19 min, m/z = 234 [M+H]⁺.

### Schritt 3: Methyl-5-methyl-1-{3-[(4-methylpiperazin-1-yl)carbonyl]benzyl}-1H-pyrrol-3-carboxylat

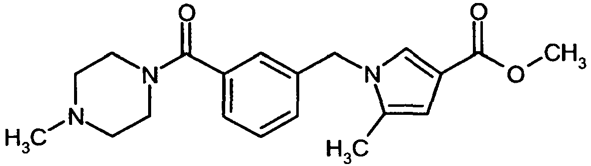

1.0 g (4.41 mmol) der Verbindung aus Beispiel 106A / Schritt 2 wurden in 10 ml Methanol gelöst und mit 698 mg (4.41 mmol) der Verbindung aus Beispiel 50A / Schritt 1 versetzt. Nachdem das Reaktionsgemisch 1 h bei RT gerührt worden war, wurde das Lösungsmittel am Rotationsverdampfer entfernt und der Rückstand mittels MPLC gereinigt (Kieselgel; Laufmittel: Dichlormethan/Methanol 10:1). Es wurden 1.07 g (68% d. Th.) der Titelverbindung erhalten.

¹H-NMR (400 MHz, CDCl₃, δ/ppm): 7.37 (t, 1H), 7.32 (d, 1H), 7.27 (d, 1H), 7.04 (d, 1H), 7.03 (d, 1H), 5.05 (s, 2H), 3.79 (s, 3H), 3.77 (breit, 2H), 3.36 (breit, 2H), 2.48 (breit, 2H), 2.31 (s, 3H), 2.11 (s, 3H), 1.95 (breit, 2H).

LC/MS (Methode I, ESIpos): Rₜ = 0.63 min, m/z = 356 [M+H]⁺.

### Schritt 4: 5-Methyl-1-{3-[(4-methylpiperazin-1-yl)carbonyl]benzyl}-1H-pyrrol-3-carbonsäure

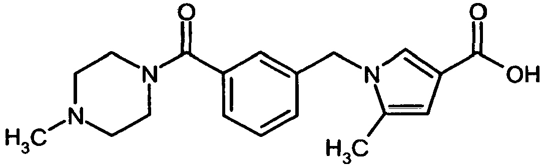

0.90 g (2.53 mmol) der Verbindung aus Beispiel 106A / Schritt 3 wurden in 17.5 ml Methanol gelöst und mit 5 ml (5.0 mmol) 1 M Natronlauge versetzt. Das Gemisch wurde portionsweise in einem Mikrowellenofen bei 80°C jeweils 30 min lang zur Reaktion gebracht (CEM Discover, initiale Einstrahlleistung 250 W). Anschließend wurde das Reaktionsgemisch durch Zusatz von 6 M Salzsäure auf einen pH-Wert von ca. 4-5 eingestellt und dann portionsweise mittels präparativer HPLC (Methode N) aufgereinigt. Es wurden 344 mg (39% d. Th.) der Titelverbindung erhalten.

LC/MS (Methode I, ESIpos): Rₜ = 0.53 min, m/z = 342 [M+H]⁺.

### Beispiel 107A

### N'-Hydroxy-4-(tetrahydro-2H-pyran-4-yl)benzolcarboximidamid

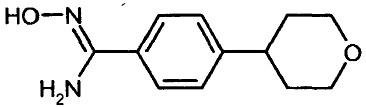

### Schritt 1: 4-(Tetrahydro-2H-pyran-4-yl)benzonitril

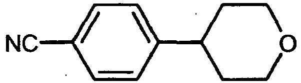

Eine Lösung von 2.91 g (19.8 mmol) 4-Cyanophenylboronsäure [M. Nishimura et al., Tetrahedron 2002, 58 (29), 5779-5788] in 20 ml Isopropanol wurde mit 186 mg (0.594 mmol) Nickel(II)iodid, 90 mg (0.594 mmol) *trans*-2-Aminocyclohexanol-Hydrochlorid und 3.63 g (19.8 mmol) Natriumhexamethyldisilazid versetzt. Die so erhaltene Suspension wurde 5 min bei RT unter einer Argonatmosphäre gerührt. Dann wurden 2.1 g (9.90 mmol) 4-Iodtetrahydropyran [Heuberger et al., J. Chem. Soc. 1952, 910] zugefügt. Nachdem das Reaktionsgemisch 15 h bei einer Temperatur von 75°C gerührt worden war, wurde es auf RT abgekühlt und mit Dichlormethan durch Filtration über ca. 50 g Kieselgel von anorganischen Salzen weitgehend befreit. Das Rohprodukt wurde durch MPLC (Kieselgel, Laufmittel: Dichlormethan) gereinigt. Es wurden so 986 mg (53% d. Th.) der Titelverbindung erhalten.

¹H-NMR (400 MHz, CDCl₃, δ/ppm): 7.60 (d, 2H), 7.32 (d, 2H), 4.12-4.07 (m, 2H), 3.56-3.50 (m, 2H), 2.87-2.79 (m, 1H), 1.86-1.73 (m, 4H).

GC/MS (Methode L, EIpos): Rₜ = 5.97 min, m/z = 187 [M]⁺.

### Schritt 2: N'-Hydroxy-4-(tetrahydro-2H-pyran-4-yl)benzolcarboximidamid

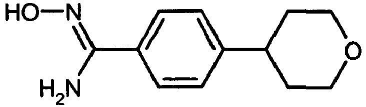

Analog zu dem unter Beispiel 1A / Schritt 5 beschriebenen Verfahren wurden 480 mg (2.56 mmol) der Verbindung aus Beispiel 107A / Schritt 1 zu 525 mg (93% d. Th.) der Titelverbindung umgesetzt.

¹H-NMR (400 MHz, CDCl₃, δ/ppm): 7.58 (d, 2H), 7.26 (d, 2H), 6.79 (breit, 1H), 4.82 (s, breit, 2H), 4.11-4.05 (m, 2H), 3.57-3.50 (m, 2H), 2.83-2.74 (m, 1H), 1.87-1.73 (m, 4H).

LC/MS (Methode D, ESIpos): Rₜ = 0.92 min, m/z = 221 [M+H]⁺.

### Beispiel 108A

### N'-Hydroxy-3-methyl-4-(tetrahydro-2H-pyran-4-yl)benzolcarboximidamid

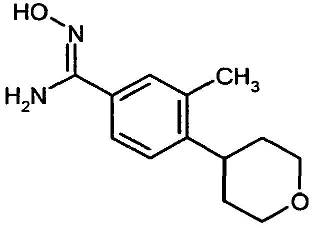

### Schritt 1: 3-Methyl-4-(tetrahydro-2H-pyran-4-yl)benzonitril

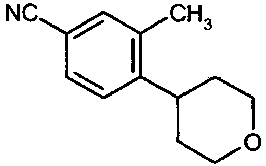

Analog zu dem unter Beispiel 107A / Schritt 1 beschriebenen Verfahren wurden aus 4.17 g (25.9 mmol) 4-Cyano-2-methylphenylboronsäure [D. Stones et al., Chem. Eur. J. 2004, 10 (1), 92-100] und 2.75 g (13.0 mmol) 4-Iodtetrahydropyran [Heuberger et al., J. Chem. Soc. 1952, 910] 481 mg (18% d. Th.) der Titelverbindung erhalten.

¹H-NMR (400 MHz, CDCl₃, δ/ppm): 7.49 (dd, 1H), 7.43 (d, 1H), 7.31 (d, 1H), 4.12-4.09 (m, 2H), 3.59-3.52 (m, 2H), 3.05-2.97 (m, 1H), 2.39 (s, 3H), 1.86-1.75 (m, 2H), 1.69-1.64 (m, 2H).

GC/MS (Methode L, EIpos): Rₜ = 6.31 min, m/z = 201 [M]⁺.

### Schritt 2: N'-Hydroxy-3-methyl-4-(tetrahydro-2H-pyran-4-yl)benzolcarboximidamid

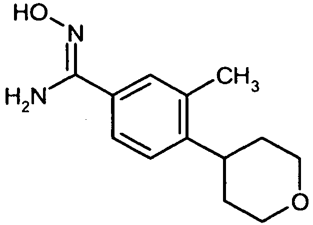

Analog zu dem unter Beispiel 1A / Schritt 5 beschriebenen Verfahren wurden aus 500 mg (2.48 mmol) der Verbindung aus Beispiel 108A / Schritt 1 492 mg (84% d. Th.) der Titelverbindung erhalten.

¹H-NMR (400 MHz, DMSO-d₆, δ/ppm): 9.49 (s, 1H), 7.45 (d, 1H), 7.44 (s, 1H), 7.21 (d, 1H), 5.69 (s, breit, 2H), 3.97-3.93 (m, 2H), 3.50-3.43 (m, 2H), 3.00-2.92 (m, 1H), 2.33 (s, 3H), 1.72-1.57 (m, 4H).

LC/MS (Methode I, ESIpos): Rₜ = 0.49 min, m/z = 235 [M+H]⁺.

### Beispiel 109A

### Ethyl-1-[4-(N'-hydroxycarbamimidoyl)phenyl]cyclobutancarboxylat

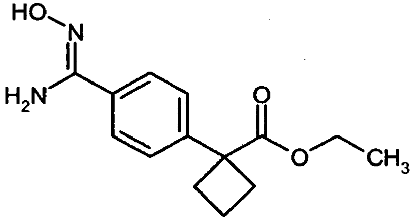

### Schritt 1: Ethyl-1-(4-bromphenyl)cyclobutancarboxylat

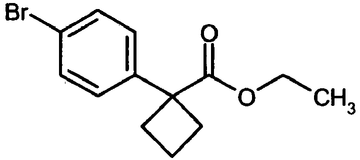

Eine Lösung von 10.0 g (41.1 mmol) 4-Bromphenylessigsäureethylester in 250 ml wasserfreiem THF wurde bei 0°C mit 45 ml (45.2 mmol) einer 1 M Lösung von Lithiumhexamethyldisilazid in THF versetzt. Nach 15 min wurden 5.4 ml (53.5 mmol) 1,3-Dibrompropan hinzugefügt. Man ließ das Reaktionsgemisch auf RT erwärmen und 1 h bei dieser Temperatur nachrühren. Dann wurde erneut auf 0°C gekühlt und mit weiteren 45 ml (45.2 mmol) Lithiumhexamethyldisilazid-Lösung (1 M in THF) versetzt. Danach wurde wieder auf RT erwärmt. Nach 1 h wurde die Reaktion durch Zugabe von ca. 10 ml gesättigter wässriger Ammoniumchlorid-Lösung beendet. Das THF wurde am Rotationsverdampfer weitgehend entfernt. Es wurde mit Wasser verdünnt und mit Ethylacetat extrahiert. Der organische Extrakt wurde nacheinander mit Wasser und gesättigter Kochsalz-Lösung gewaschen. Nach Trocknen über wasserfreiem Magnesiumsulfat wurde filtriert und das Filtrat am Rotationsverdampfer vom Lösungsmittel befreit. Das so erhaltene Rohprodukt wurde mittels Saugfiltration über ca. 300 g Kieselgel mit Cyclohexan/Ethylacetat 3:1 als Laufmittel grob gereinigt. Es wurden 7.1 g (44% d. Th., 73% Reinheit) der Titelverbindung erhalten, welche in dieser Form weiter umgesetzt wurde.

¹H-NMR (400 MHz, CDCl₃, δ/ppm): 7.44 (d, 2H), 7.17 (d, 2H), 4.10 (quart, 2H), 2.85-2.79 (m, 2H), 2.49-2.41 (m, 2H), 2.10-1.98 (m, 1H), 1.91-1.81 (m, 1H), 1.18 (t, 3H).

MS (DCI, NH₃): m/z = 300/302 [M+NH₄]⁺.

LC/MS (Methode D, ESIpos): Rₜ = 2.70 min, m/z = 283/285 [M+H]⁺.

### Schritt 2: Ethyl-1-(4-cyanophenyl)cyclobutancarboxylat

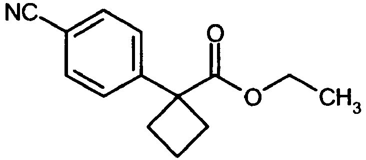

Eine Mischung aus 160 mg (0.565 mmol) der Verbindung aus Beispiel 109A / Schritt 1, 76 mg (0.644 mmol) Zinkcyanid, 26 mg (0.028 mmol) Tris(dibenzylidenaceton)dipalladium und 23 mg (0.057 mmol) Dicyclohexyl-(2',6'-dimethoxybiphen-2-yl)phosphan in 6 ml DMF/Wasser (99:1) wurde unter sauerstofffreien Bedingungen 1 h auf 120°C erhitzt. Nach dem Abkühlen auf RT wurde mit ca. 30 ml Wasser verdünnt und dreimal mit je ca. 20 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt. Der erhaltene Rückstand wurde zunächst mittels MPLC (Kieselgel, Laufmittel: Cyclohexan/Ethylacetat 10:1) vorgereinigt. Das Produkt wurde dann mittels präparativer HPLC (Methode N) in reiner Form isoliert. Es wurden 110 mg (85% d. Th.) der Titelverbindung erhalten.

¹H-NMR (400 MHz, CDCl₃, δ/ppm): 7.62 (d, 2H), 7.39 (d, 2H), 4.10 (quart, 2H), 2.90-2.83 (m, 2H), 2.52-2.44 (m, 2H), 2.15-2.03 (m, 1H), 1.93-1.83 (m, 1H), 1.17 (t, 3H).

LC/MS (Methode D, ESIpos): Rₜ = 2.32 min, m/z = 230 [M+H]⁺.

### Schritt 3: Ethyl-1-[4-(N'-hydroxycarbamimidoyl)phenyl]cyclobutancarboxylat

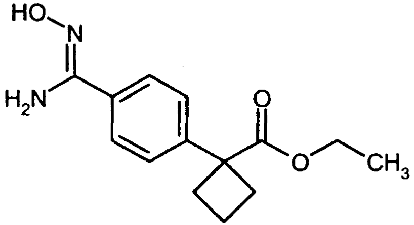

Analog zu dem unter Beispiel 1A / Schritt 5 beschriebenen Verfahren wurden aus 105 mg (0.458 mmol) der Verbindung aus Beispiel 109A / Schritt 2 122 mg (91% d. Th., 90% Reinheit) der Titelverbindung erhalten.

¹H-NMR (400 MHz, CDCl₃, δ/ppm): 7.59 (d, 2H), 7.33 (d, 2H), 4.84 (breit, 2H), 4.10 (quart, 2H), 2.88-2.80 (m, 2H), 2.53-2.46 (m, 2H), 2.10-1.99 (m, 1H), 1.92-1.82 (m, 1H), 1.17 (t, 3H).

LGMS (Methode I, ESIpos): Rₜ = 0.67 min, m/z = 263 [M+H]⁺.

### Beispiel 110A

### N'-Hydroxy-4-[1-(methoxymethyl)cyclobutyl]benzolcarboximidamid

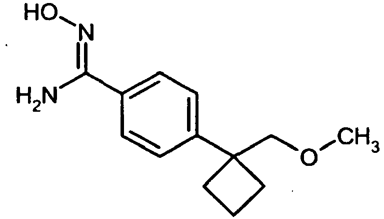

### Schritt 1: [1-(4-Bromphenyl)cyclobutyl]methanol

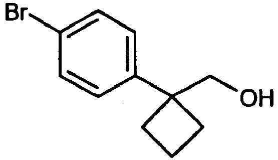

7.20 g (25.4 mmol) der Verbindung aus Beispiel 109A / Schritt 1 wurden in 150 ml wasserfreiem THF gelöst und bei 0°C tropfenweise mit 25 ml (25 mmol) einer 1 M Lösung von Lithiumaluminiumhydrid in THF versetzt. Nach beendeter Zugabe wurde das Eis/Wasser-Bad entfernt und das Rühren bei RT fortgesetzt. Nach 1 h wurde die Reaktion durch - anfangs vorsichtiges - Hinzufügen von ca. 450 ml gesättigter wässriger Ammoniumchlorid-Lösung beendet. Es wurde dann mit Ethylacetat extrahiert. Nach Trocknen des organischen Extrakts über wasserfreiem Magnesiumsulfat und anschließender Filtration wurde das Lösungsmittel am Rotationsverdampfer entfernt. Es wurden 6.04 g (88% d. Th., 90% Reinheit) der Titelverbindung erhalten.

¹H-NMR (400 MHz, CDCl₃, δ/ppm): 7.43 (d, 2H), 7.02 (d, 2H), 3.72 (d, 2H), 2.33-2.20 (m, 4H), 2.13-2.01 (m, 1H), 1.93-1.83 (m, 1H).

MS (DCI, NH₃): m/z = 258/260 [M+NH₄]⁺.

GC/MS (Methode L, ESIpos): Rₜ = 5.77 min, m/z = 240/242 [M]⁺.

### Schritt 2: 1-Brom-4-[1-(methoxymethyl)cyclobutyl]benzol

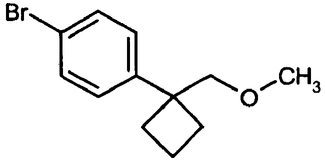

Eine Lösung von 7.0 g (29.0 mmol) der Verbindung aus Beispiel 110A / Schritt 1 in 120 ml wasserfreiem DMF wurde bei ca. 5°C mit 1.28 g (31.9 mmol) einer 60%-igen Suspension von Natriumhydrid in Mineralöl versetzt. Nachdem 1 h bei dieser Temperatur gerührt worden war, wurden 2.2 ml (34.8 mmol) Methyliodid zugefügt. Man ließ das Reaktionsgemisch auf RT erwärmen und setzte das Rühren 15 h lang fort. Anschließend wurde das Reaktionsgemisch am Rotationsverdampfer auf ein Volumen von ca. 20 ml eingeengt. Es wurden ca. 500 ml Wasser zugefügt und das Gemisch dreimal mit je ca. 200 ml Diethylether extrahiert. Die vereinigten organischen Extrakte wurden mit gesättigter Kochsalz-Lösung gewaschen und über wasserfreiem Magnesiumsulfat getrocknet. Nach Filtration und Entfernen des Lösungsmittels am Rotationsverdampfer wurde das erhaltene Rohprodukt mittels Saugfiltration über ca. 200 g Kieselgel mit Cyclohexan/Ethylacetat 50:1 als Laufmittel gereinigt. Es wurden 4.92 g (66% d. Th.) der Titelverbindung erhalten.

¹H-NMR (400 MHz, CDCl₃, δ/ppm): 7.41 (d, 2H), 7.04 (d, 2H), 3.48 (s, 2H), 3.27 (s, 3H), 2.32-2.22 (m, 4H), 2.12-2.00 (m, 1H), 1.90-1.80 (m, 1H).

MS (DCI, NH₃): m/z = 272/274 [M+NH₄]⁺.

GC/MS (Methode L, ESIpos): Rₜ = 5.25 min, m/z = 254/256 [M]⁺.

### Schritt 3: 4-[1-(Methoxymethyl)cyclobutyl]benzonitril

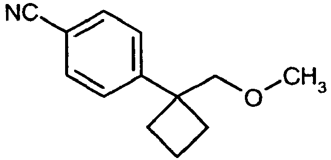

Analog zu dem unter Beispiel 109A / Schritt 2 beschriebenen Verfahren wurden aus 4.80 g (18.8 mmol) der Verbindung aus Beispiel 110A / Schritt 2 1.82 g (48% d. Th.) der Titelverbindung erhalten.

¹H-NMR (400 MHz, CDCl₃, δ/ppm): 7.58 (d, 2H), 7.24 (d, 2H), 3.52 (s, 2H), 3.26 (s, 3H), 2.34-2.24 (m, 4H), 2.16-2.03 (m, 1H), 1.92-1.83 (m, 1H).

LC/MS (Methode F, ESIpos): Rₜ = 1.22 min, m/z = 202 [M+H]⁺.

### Schritt 4: N'-Hydroxy-4-[1-(methoxymethyl)cyclobutyl]benzolcarboximidamid

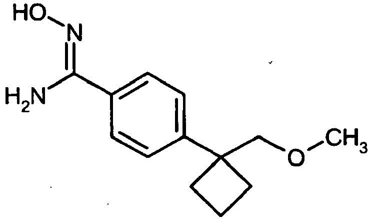

Analog zu dem unter Beispiel 1A / Schritt 5 beschriebenen Verfahren wurden aus 1.82 g (9.04 mmol) der Verbindung aus Beispiel 110A / Schritt 3 2.04 g (96% d. Th.) der Titelverbindung erhalten.

¹H-NMR (400 MHz, CDCl₃, δ/ppm): 7.55 (d, 2H), 7.20 (d, 2H), 7.10 (breit, 1H), 4.83 (breit, 2H), 3.51 (s, 2H), 3.27 (s, 3H), 2.36-2.25 (m, 4H), 2.12-2.01 (m, 1H), 1.90-1.81 (m, 1H).

LC/MS (Methode I, ESIpos): Rₜ = 0.61 min, m/z = 235 [M+H]⁺.

### Beispiel 111A

### N'-Hydroxy-4-(1,1,1-trifluor-2-methoxypropan-2-yl)benzolcarboximidamid (Racemat)

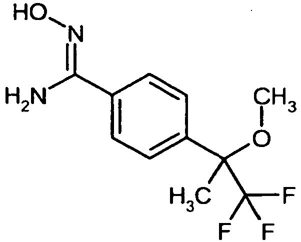

### Schritt 1: 4-(1,1,1-Trifluor-2-hydroxypropan-2-yl)benzonitril (Racemat)

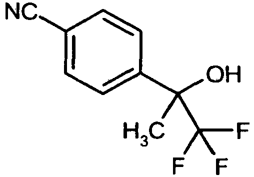

5.0 g (21.8 mmol) 4-Iodbenzonitril wurden in 100 ml wasserfreiem THF gelöst und auf -40°C abgekühlt. Es wurde tropfenweise mit 11.5 ml (22.9 mmol) einer 2 M Lösung von Isopropylmagnesiumchlorid in Diethylether versetzt, so dass die Temperatur des Reaktionsgemisches im Bereich zwischen -30°C und -40°C blieb. Nach beendeter Zugabe wurde noch weitere 1.5 h in diesem Temperaturintervall gerührt, bevor auf -78°C abgekühlt wurde. Es wurden dann tropfenweise 11.2 g (100 mmol) 1,1,1-Trifluoraceton zugesetzt. Man ließ das Reaktionsgemisch im Verlaufe mehrerer Stunden auf RT kommen und rührte noch ca. 5 h bei RT nach. Anschließend wurde vorsichtig mit ca. 5 ml Wasser versetzt. Dann wurde das Lösungsmittel zum größten Teil am Rotationsverdampfer entfernt, bis ein Restvolumen von ca. 50 ml übrig blieb. Dieser Rückstand wurde mit ca. 100 ml 0.5 M Salzsäure versetzt und dreimal mit je ca. 100 ml Ethylacetat extrahiert. Die vereinigten organischen Extrakte wurden nacheinander mit Wasser und gesättigter Kochsalz-Lösung gewaschen und über wasserfreiem Magnesiumsulfat getrocknet. Nach Filtration wurde das Lösungsmittel am Rotationsverdampfer entfernt und der erhaltene Rückstand mittels MPLC gereinigt (ca. 200 g Kieselgel, Laufmittel: Cyclohexan/Ethylacetat 20:1 → 5:1). Es wurden 3.63 g (73% d. Th., 95% Reinheit) der Titelverbindung erhalten.

LC/MS (Methode I, ESIneg): Rₜ = 0.89 min, m/z = 214 [M-H]⁻, 260 [M-H+HCO₂H]⁻.

### Schritt 2: 4-(1,1,1-Trifluor-2-methoxypropan-2-yl)benzonitril (Racemat)

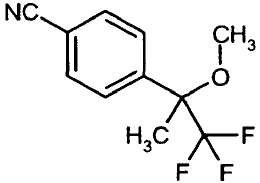

Analog zu dem unter Beispiel 110A / Schritt 2 beschriebenen Verfahren wurden aus 1.6 g (7.44 mmol) der Verbindung aus Beispiel 111A / Schritt 1 und 555 µl (8.92 mmol) Methyliodid 1.01 g (59% d. Th.) der Titelverbindung erhalten. Die chromatographische Reinigung erfolgte mit Cyclohexan/Ethylacetat 100:0 → 20:1 als Laufmittel.

¹H-NMR (400 MHz, CDCl₃, δ/ppm): 7.71 (d, 2H), 7.63 (d, 2H), 3.27 (s, 3H), 1.80 (s, 3H).

HPLC (Methode A): Rₜ = 4.01 min.

GC/MS (Methode L, EIpos): Rₜ = 4.13 min, m/z = 214 [M-CH₃]⁺, 160 [M-CF₃]⁺.

### Schritt 3: N'-Hydroxy-4-(1,1,1-trifluor-2-methoxypropan-2-yl)benzolcarboximidamid (Racemat)

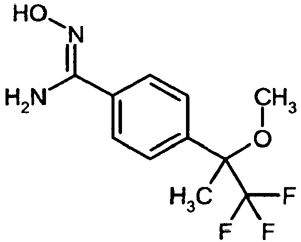

Analog zu dem unter Beispiel 1A / Schritt 5 beschriebenen Verfahren wurden aus 990 mg (4.32 mmol) der Verbindung aus Beispiel 111A / Schritt 2 1.07 g (89% d. Th., 94% Reinheit) der Titelverbindung erhalten.

LC/MS (Methode D, ESIpos): Rₜ = 1.23 min, m/z = 263 [M+H]⁺.

### Beispiel 112A

### 3-Fluor-N'-hydroxy-4-(tetrahydro-2H-pyran-4-yl)benzolcarboximidamid

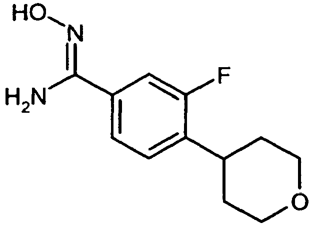

### Schritt 1: (4-Cyano-2-fluorphenyl)boronsäure

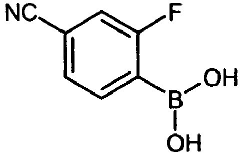

Eine Lösung von 10.0 g (40.5 mmol) 3-Fluor-4-iodbenzonitril in einem Gemisch aus 120 ml wasserfreiem THF und 120 ml wasserfreiem Diethylether wurde bei -78°C tropfenweise mit 24.3 ml (48.6 mmol) einer 2 M Lösung von Isopropylmagnesiumchlorid in Diethylether versetzt. Nach beendeter Zugabe wurde noch weitere 75 min bei -78°C gerührt. Dann wurden 15 ml (64.8 mmol) Triisopropylborat hinzugetropft. Anschließend wurde noch 15 min bei -78°C gerührt, bevor das Kältebad entfernt wurde und man das Reaktionsgemisch sich auf RT erwärmen ließ. Nach 3 h bei RT wurde mit 80 ml 2 M Salzsäure versetzt und die Mischung für 20 min intensiv bei RT gerührt. Danach wurde mit ca. 400 ml Wasser verdünnt. Die Phasen wurden getrennt, und die wässrige Phase wurde dreimal mit je ca. 150 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Filtrat bis zur Trockene am Rotationsverdampfer eingeengt. Es wurden 3.68 g (55% d. Th.) der Titelverbindung erhalten, die ohne weitere Aufreinigung für Folgereaktionen eingesetzt wurde.

LC/MS (Methode F, ESIneg): Rₜ = 0.53 min, m/z = 164 [M-H]⁻.

### Schritt 2: 4-Brom-3,6-dihydro-2H-pyran

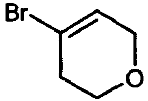

Eine Lösung von 8.52 g (27.5 mmol) Triphenylphosphit in 78 ml wasserfreiem Dichlormethan wurde bei -60°C tropfenweise mit 4.79 g (30.0 mmol) Brom versetzt. Nach Zugabe von 4.5 ml (32.5 mmol) Triethylamin wurde eine Lösung von 2.5 g (25.0 mmol) Tetrahydro-4*H*-pyran-on in

2 ml Dichlormethan zugetropft. Man ließ das Reaktionsgemisch langsam (über ca. 5 h) auf RT erwärmen und setzte das Rühren für weitere ca. 10 h bei RT fort. Anschließend wurden alle flüchtigen Bestandteile am Rotationsverdampfer entfernt und der erhaltene Rückstand durch Saugfiltration über ca. 100 g Kieselgel mit Dichlormethan als Laufmittel grob chromatographiert. Nach neuerlichem Abdampfen des Lösungsmittels wurde das Produkt mittels Kugelrohr-Destillation isoliert (Druck: 8 mbar; Temperatur: bis 120°C). Es wurden 2.51 g (62% d. Th.) der Titelverbindung erhalten.

¹H-NMR (400 MHz, CDCl₃, δ/ppm): 6.07 (m, 1H), 4.13 (m, 2H), 3.83 (m, 2H), 2.55-2.50 (m, 2H).

GC/MS (Methode L, ESIpos): Rₜ = 2.32 min, m/z = 162/164 [M]⁺.

### Schritt 3: 4-(3,6-Dihydro-2H-pyran-4-yl)-3-fluorbenzonitril

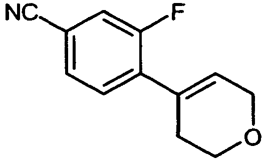

Ein Gemisch aus 300 mg (1.84 mmol) der Verbindung aus Beispiel 112A / Schritt 2, 334 mg (2.02 mmol) der Verbindung aus Beispiel 112A / Schritt 1, 8 mg (0.037 mmol) Palladium(II)acetat, 1.17 g (5.52 mmol) Kaliumphosphat sowie 44 mg (0.092 mmol) 2-Dicyclohexylphosphin-2',4',6'-triisopropylbiphenyl (XPhos) in 4 ml wasserfreiem THF wurde entgast und unter Argon in einem Mikrowellenofen (CEM Discover, initiale Einstrahlleistung 250 W) 1 h lang bei 80°C gerührt. Nach dem Abkühlen auf RT wurden alle flüchtigen Bestandteile am Rotationsverdampfer entfernt. Aus dem Rückstand wurde das Produkt mittels MPLC isoliert (ca. 50 g Kieselgel, Laufmittel: Cyclohexan/Dichlormethan 100:0 → 50:50 → 5:95). Es wurden 160 mg (43% d. Th.) der Titelverbindung erhalten.

¹H-NMR (400 MHz, CDCl₃, δ/ppm): 7.42 (d, 1H), 7.37 (d, 1H), 7.36 (d, 1H), 6.18 (m, 1H), 4.33 (m, 2H), 3.92 (t, 2H), 2.52-2.48 (m, 2H).

GC/MS (Methode L, ESIpos): Rₜ = 5.79 min, m/z = 203 [M]⁺.

### Schritt 4: 3-Fluor-4-(tetrahydro-2H-pyran-4-yl)benzonitril

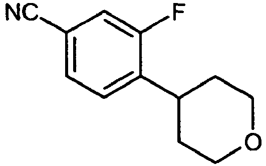

330 mg (1.62 mmol) der Verbindung aus Beispiel 112A / Schritt 3 wurden in einem Gemisch aus 22 ml Ethylacetat und 22 ml Ethanol gelöst. Es wurde in einer Durchfluss-Hydrierapparatur hydriert ("H-Cube" der Fa. Thales Nano, Budapest, Ungarn; Bedingungen: Kartusche mit 5% Palladium auf Kohle, 10 bar Wasserstoffdruck, Temperatur 20°C, Fluss 1 ml/min). Insgesamt wurde die Lösung viermal durch die Apparatur geleitet, bis die Reaktion vollständig war. Anschließend wurde das Lösungsmittel am Rotationsverdampfer entfernt. Es wurden 211 mg (63% d. Th.) der Titelverbindung erhalten.

¹H-NMR (400 MHz, CDCl₃, δ/ppm): 7.43 (d, 1H), 7.37 (d, 1H), 7.32 (d, 1H), 4.11-4.07 (m, 2H), 3.59-3.53 (m, 2H), 3.21-3.13 (m, 1H), 1.88-1.72 (m, 4H).

GC/MS (Methode L, EIpos): Rₜ = 5.59 min, m/z = 205 [M]⁺.

### Schritt 5: 3-Fluor-N'-hydroxy-4-(tetrahydro-2H-pyran-4-yl)benzolcarboximidamid

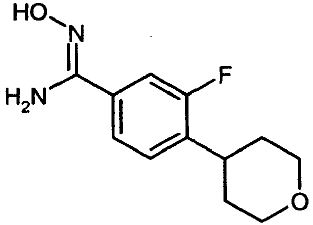

Analog zu dem unter Beispiel 1A / Schritt 5 beschriebenen Verfahren wurden aus 175 mg (0.853 mmol) der Verbindung aus Beispiel 112A / Schritt 4 172 mg (85% d. Th.) der Titelverbindung erhalten.

¹H-NMR (400 MHz, CDCl₃, δ/ppm): 7.40-7.24 (m, 3H), 7.08 (breit, 1H), 4.81 (breit, 2H), 4.10-4.06 (m, 2H), 3.59-3.52 (m, 2H), 3.17-3.10 (m, 1H), 1.89-1.71 (m, 4H).

LC/MS (Methode F, ESIpos): Rₜ = 0.46 min, m/z = 239 [M+H]⁺.

### Beispiel 113A

### 4-[1-(2-Fluorethyl)cyclobutyl]-N'-hydroxybenzolcarboximidamid

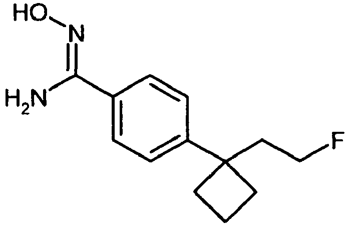

### Schritt 1: Ethyl-{1-[4-(dibenzylamino)phenyl]cyclobutyl}acetat

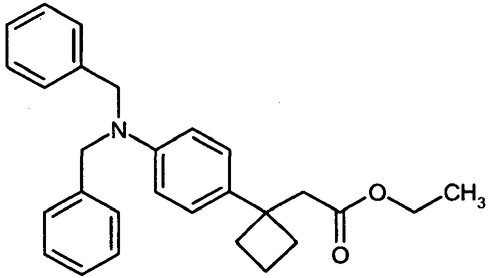

440 mg (0.892 mmol) Bis[(1,5-cyclooctadien)rhodium(I)chlorid] wurden in 20 ml 1,4-Dioxan vorgelegt und mit 15.5 ml (23.2 mmol) 1.5 M Kalilauge versetzt. Dann wurde eine Lösung von 2.5 g (17.8 mmol) Cyclobutylidenessigsäureethylester [M. Afzal et al., J. Chem. Soc. Perkin Trans. 2, 1999 (5), 937-946] in 1 ml 1,4-Dioxan zugesetzt. Anschließend wurde eine Lösung von 5.66 g (17.8 mmol) der Verbindung aus Beispiel 4A / Schritt 1 in 100 ml 1,4-Dioxan hinzugegeben. Nachdem das Reaktionsgemisch 16 h bei RT gerührt worden war, wurde am Rotationsverdampfer bis zur Trockene eingeengt. Der erhaltene Rückstand wurde in wenig Dichlormethan gelöst und mittels Saugfiltration über ca. 100 g Kieselgel mit Dichlormethan als Laufmittel vorgereinigt. Das Produkt wurde mittels MPLC (ca. 300 g Kieselgel, Laufmittel: Cyclohexan/Dichlormethan 100:0 → 50:50) in reiner Form isoliert. Es wurden 4.02 g (54% d. Th.) der Titelverbindung erhalten.

¹H-NMR (400 MHz, CDCl₃, δ/ppm): 7.32-7.30 (m, 4H), 7.26-7.21 (m, 6H), 6.97 (d, 2H), 6.67 (d, 2H), 4.61 (s, 4H), 3.93 (quart, 2H), 2.70 (s, 2H), 2.43-2.28 (m, 4H), 2.07-1.96 (m, 1H), 1.88-1.78 (m, 1H).

HPLC (Methode A): Rₜ = 4.74 min.

MS (DCI, NH₃): m/z = 414 [M+H]⁺.

### Schritt 2: 2-{1-[4-(Dibenzylamino)phenyl]cyclobutyl}ethanol

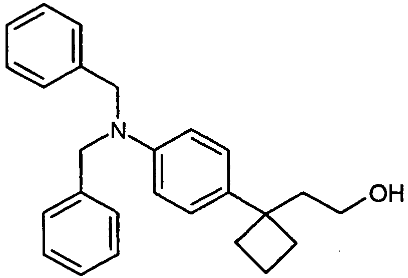

Eine Lösung von 15.0 g (36.3 mmol) der Verbindung aus Beispiel 113A / Schritt 1 in 500 ml wasserfreiem THF wurde bei 0°C tropfenweise mit 36.3 ml (36.3 mmol) einer 1 M Lösung von Lithiumaluminiumhydrid in THF versetzt. Nach beendeter Zugabe ließ man das Reaktionsgemisch auf RT erwärmen und setzte das Rühren für 2 h fort. Dann wurde die Reaktion vorsichtig bei 0°C durch Zugabe von 20 g Kieselgur und 20 ml Wasser beendet. Das Gemisch wurde über ein Papierfilter abgesaugt, und es wurde mit *tert*.-Butylmethylether nachgewaschen. Das Filtrat wurde am Rotationsverdampfer weitestgehend vom Lösungsmittel befreit. Der Rückstand wurde in ca. 400 ml Ethylacetat aufgenommen und je einmal mit Wasser und gesättigter Kochsalz-Lösung gewaschen. Nach Trocknen über wasserfreiem Magnesiumsulfat wurde filtriert und erneut das Lösungsmittel im Vakuum abgedampft. Das Rohprodukt wurde mittels Saugfiltration über ca. 250 g Kieselgel mit Cyclohexan/Ethylacetat 10:1 → 3:1 als Laufmittel gereinigt. Es wurden 11.6 g (85% d. Th.) der Titelverbindung erhalten.

¹H-NMR (400 MHz, CDCl₃, δ/ppm): 7.33-7.30 (m, 4H), 7.26-7.22 (m, 6H), 6.93 (d, 2H), 6.69 (d, 2H), 4.61 (s, 4H), 3.49-3.43 (m, 2H), 2.35-2.28 (m, 2H), 2.12-2.00 (m, 5H), 1.85-1.78 (m, 1H).

HPLC (Methode A): Rₜ = 4.81 min.

MS (DCI, NH₃): m/z = 372 [M+H]⁺.

### Schritt 3: N,N-Dibenzyl-4-[1-(2-fluorethyl)cyclobutyl]anilin

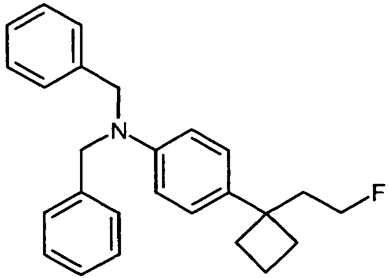

Eine Lösung von 3.0 g (8.07 mmol) der Verbindung aus Beispiel 113A / Schritt 2 in 150 ml wasserfreiem Dichlormethan wurde bei einer Temperatur von -78°C tropfenweise mit 1.3 ml (9.69 mmol) Diethylaminoschwefeltrifluorid (DAST) versetzt. Nach 30 min bei -78°C wurde das Reaktionsgemisch für ca. 30 Sekunden auf etwa -20°C erwärmt und das Reaktionsgefäß dann wieder in das -78°C-Kältebad getaucht. Nach Zusatz von 20 ml 1 M Natronlauge wurde die Mischung auf RT erwärmt. Es wurde mit 75 ml Wasser verdünnt und dreimal mit je ca. 75 ml Dichlormethan extrahiert. Die vereinigten organischen Extrakte wurden mit Wasser gewaschen und anschließend über wasserfreiem Magnesiumsulfat getrocknet. Nach Filtration wurde das Lösungsmittel am Rotationsverdampfer entfernt. Das Produkt wurde mittels MPLC isoliert (ca. 300 g Kieselgel, Laufmittel: Cyclohexan/Ethylacetat 100:0 → 20:1). Es wurden 1.48 g (49% d. Th.) der Titelverbindung erhalten.

¹H-NMR (400 MHz, CDCl₃, δ/ppm): 7.41-7.37 (m, 4H), 7.34-7.30 (m, 6H), 7.00 (d, 2H), 6.75 (d, 2H), 4.69 (s, 4H), 4.30 (td, 2H), 2.44-2.37 (m, 2H), 2.26-2.07 (m, 5H), 1.93-1.84 (m, 1H).

HPLC (Methode A): Rₜ = 5.31 min.

MS (DCI, NH₃): m/z = 374 [M+H]⁺.

### Schritt 4: 4-[1-(2-Fluorethyl)cyclobutyl]anilin

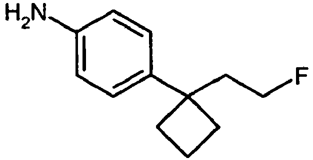

Analog zu dem unter Beispiel 4A / Schritt 6 beschriebenen Verfahren wurden aus 1.43 g (3.83 mmol) der Verbindung aus Beispiel 113A / Schritt 3 460 mg (62% d. Th.) der Titelverbindung erhalten. Als Lösungsmittel wurden in diesem Fall 180 ml eines Gemisches aus Ethanol und Ethylacetat (3:1) verwendet. Das nach dem Abdampfen des Lösungsmittels erhaltene Rohprodukt wurde ohne weitere chromatographische Aufreinigung in der Folgereaktion eingesetzt.

¹H-NMR (400 MHz, CDCl₃, δ/ppm): 6.93 (d, 2H), 6.64 (d, 2H), 4.23 (td, 2H), 3.60 (breit, 2H), 2.38-2.31 (m, 2H), 2.20-2.01 (m, 5H), 1.88-1.78 (m, 1H).

LC/MS (Methode I, ESIpos): Rₜ = 0.77 min, m/z = 194 [M+H]⁺.

### Schritt 5: 4-[1-(2-Fluorethyl)cyclobutyl]benzonitril

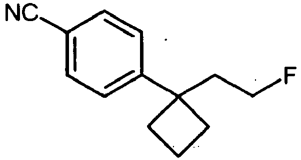

Analog zu dem unter Beispiel 4A / Schritt 7 beschriebenen Verfahren wurden aus 440 mg (2.28 mmol) der Verbindung aus Beispiel 113A / Schritt 4 259 mg (56% d. Th.) der Titelverbindung erhalten.

¹H-NMR (400 MHz, CDCl₃, δ/ppm): 7.61 (d, 2H), 7.24 (d, 2H), 4.23 (td, 2H), 2.44-2.36 (m, 2H), 2.31-2.10 (m, 5H), 1.91-1.82 (m, 1H).

GC/MS (Methode L, EIpos): Rₜ = 5.63 min, m/z = 203 [M]⁺.

### Schritt 6: 4-[1-(2-Fluorethyl)cyclobutyl]-N'-hydroxybenzolcarboximidamid

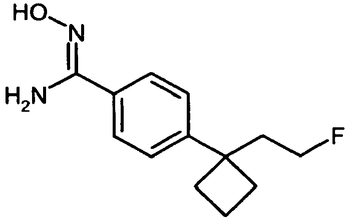

Analog zu dem unter Beispiel 1A / Schritt 5 beschriebenen Verfahren wurde aus 250 mg (1.23 mmol) der Verbindung aus Beispiel 113A / Schritt 5 102 mg (35% d. Th.) der Titelverbindung erhalten. Das Rohprodukt wurde mittels präparativer HPLC (Methode N) gereinigt.

LC/MS (Methode D, ESIpos): Rₜ = 1.37 min, m/z = 237 [M+H]⁺.

### Beispiel 114A

### N'-Hydroxy-4-(1,1,1-trifluor-2-hydroxypropan-2-yl)benzolcarboximidamid (Racemat)

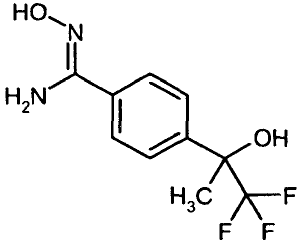

Analog zu dem unter Beispiel 1A / Schritt 5 beschriebenen Verfahren wurden 1.0 g (4.65 mmol) der Verbindung aus Beispiel 111A / Schritt 1 zu 1.12 g (83% d. Th., 85% Reinheit) der Titelverbindung umgesetzt.

LC/MS (Methode F, ESIpos): Rₜ = 0.36 min, m/z = 249 [M+H]⁺.

### Beispiel 115A

### {1-[4-(N'-Hydroxycarbamimidoyl)phenyl]cyclobutyl}methylacetat

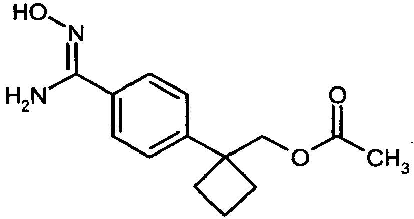

### Schritt 1: [1-(4-Bromphenyl)cyclobutyl]methylacetat

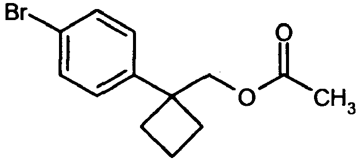

Eine Lösung von 402 mg (1.67 mmol) der Verbindung aus Beispiel 110A / Schritt 1 in 6 ml Pyridin wurde bei 0°C mit 236 µl (2.50 mmol) Essigsäureanhydrid versetzt. Nachdem das Reaktionsgemisch 16 h bei RT gerührt worden war, wurden alle flüchtigen Bestandteile am Rotationsverdampfer entfernt. Aus dem erhaltenen Rückstand wurde das Produkt mittels MPLC isoliert (Kieselgel, Laufmittel: Cyclohexan/Ethylacetat 10:1). Es wurden 450 mg (91% d. Th., 95% Reinheit) der Titelverbindung erhalten.

¹H-NMR (400 MHz, CDCl₃, δ/ppm): 7.42 (d, 2H), 7.02 (d, 2H), 4.21 (s, 2H), 2.38-2.30 (m, 2H), 2.29-2.21 (m, 2H), 2.16-2.03 (m, 1H), 1.98 (s, 3H), 1.93-1.83 (m, 1H).

MS (DCI, NH₃): m/z = 300/302 [M+NH₄]⁺.

### Schritt 2: [1-(4-Cyanophenyl)cyclobutyl]methylacetat

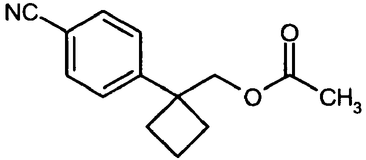

Analog zu dem unter Beispiel 109A / Schritt 2 beschriebenen Verfahren wurden 440 mg (1.55 mmol) der Verbindung aus Beispiel 115A / Schritt 1 zu 314 mg (84% d. Th., 95% Reinheit) der Titelverbindung umgesetzt. Die Aufreinigung des Rohprodukts erfolgte direkt mittels präparativer HPLC (Methode N).

¹H-NMR (400 MHz, CDCl₃, δ/ppm): 7.60 (d, 2H), 7.25 (d, 2H), 4.26 (s, 2H), 2.42-2.26 (m, 4H), 2.20-2.09 (m, 1H), 1.98 (s, 3H), 1.98-1.87 (m, 1H).

MS (DCI, NH₃): m/z = 247 [M+NH₄]⁺.

### Schritt 3: {1-[4-(N'-Hydroxycarbamimidoyl)phenyl]cyclobutyl}methylacetat

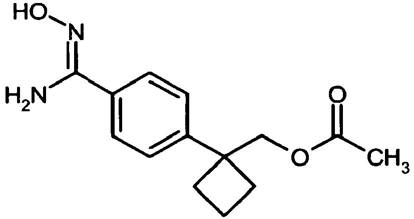

Analog zu dem unter Beispiel 1A / Schritt 5 beschriebenen Verfahren wurden 260 mg (1.13 mmol) der Verbindung aus Beispiel 115A / Schritt 2 zu 312 mg (94% d. Th., 90% Reinheit) der Titelverbindung umgesetzt.

¹H-NMR (400 MHz, EDCl₃, δ/ppm): 7.57 (d, 2H), 7.19 (d, 2H), 4.84 (breit, 2H), 4.24 (s, 2H), 2.44-2.34 (m, 2H), 2.32-2.24 (m, 2H), 2.17-2.07 (m, 1H), 1.99 (s, 3H), 1.94-1.86 (m, 1H).

MS (DCI, NH₃): m/z = 263 [M+NH₄]⁺.

LC/MS (Methode F, ESIpos): Rₜ = 0.74 min, m/z = 263 [M+H]⁺.

### Beispiel 116A

### N'-Hydroxy-4-[1-(2-hydroxyethyl)cyclobutyl]benzolcarboximidamid

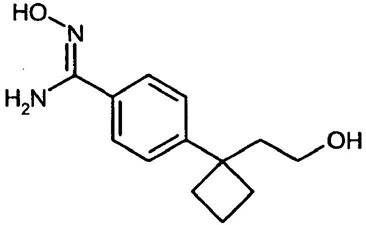

### Schritt 1: 2-{1-[4-(Dibenzylamino)phenyl]cyclobutyl}ethylacetat

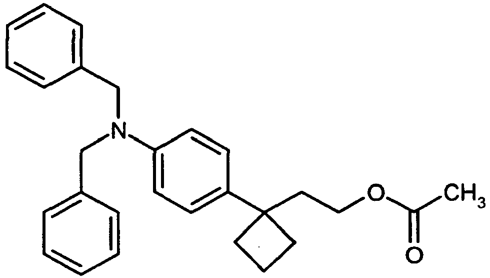

Analog zu dem unter Beispiel 115A / Schritt 1 beschriebenen Verfahren wurden aus 3.50 g (9.42 mmol) der Verbindung aus Beispiel 113A / Schritt 2 3.64 g (87% d. Th.) der Titelverbindung erhalten. Die abschließende Saugfiltration über Kieselgel erfolgte mit Cyclohexan/Ethylacetat 20:1 als Laufmittel.

¹H-NMR (400 MHz, CDCl₃, δ/ppm): 7.35-7.30 (m, 4H), 7.28-7.22 (m, 6H), 6.91 (d, 2H), 6.67 (d, 2H), 4.62 (s, 4H), 3.86-3.82 (m, 2H), 2.37-2.29 (m, 2H), 2.15-2.00 (m, 5H), 1.94 (s, 3H), 1.86-1.77 (m, 1H).

HPLC (Methode A): Rₜ = 5.22 min.

MS (DCI, NH₃): m/z = 414 [M+H]⁺.

LC/MS (Methode I, ESIpos): Rₜ = 1.51 min, m/z = 414 [M+H]⁺.

### Schritt 2: 2-[1-(4-Aminophenyl)cyclobutyl]ethylacetat

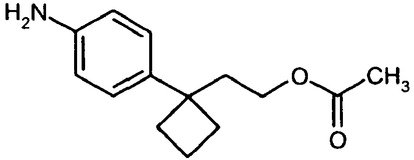

Analog zu dem unter Beispiel 4A / Schritt 6 beschriebenen Verfahren wurden aus 3.50 g (8.46 mmol) der Verbindung aus Beispiel 116A / Schritt 1 1.79 g (85% d. Th., 94% Reinheit) der Titelverbindung erhalten. Als Lösungsmittel wurden in diesem Fall 300 ml eines Gemisches aus Ethanol und Ethylacetat (3:1) verwendet. Das nach dem Abdampfen des Lösungsmittels erhaltene Rohprodukt wurde ohne weitere chromatographische Aufreinigung in der Folgereaktion eingesetzt.

LC/MS (Methode D, ESIpos): Rₜ = 1.46 min, m/z = 467 [2M+H]⁺, 234 [M+H]⁺.

### Schritt 3: 2-[1-(4-Cyanophenyl)cyclobutyl]ethylacetat

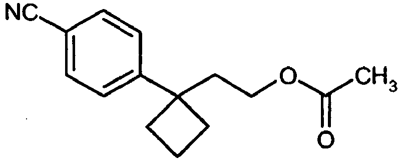

Analog zu dem unter Beispiel 4A / Schritt 7 beschriebenen Verfahren wurden aus 500 mg (2.14 mmol) der Verbindung aus Beispiel 116A / Schritt 2 152 mg (29% d. Th.) der Titelverbindung hergestellt.

¹H-NMR (400 MHz, CDCl₃, δ/ppm): 7.60 (d, 2H), 7.23 (d, 2H), 3.83 (t, 2H), 2.42-2.33 (m, 2H), 2.27-2.21 (m, 2H), 2.17 (t, 2H), 2.15-2.08 (m, 1H), 1.93-1.82 (m, 1H), 1.89 (s, 3H).

MS (DCI, NH₃): m/z = 461 [M+NH₄]⁺.

### Schritt 4: N'-Hydroxy-4-[1-(2-hydroxyethyl)cyclobutyl]benzolcarboximidamid

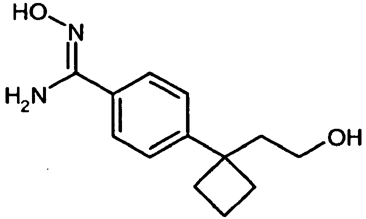

Analog zu dem in Beispiel 1A / Schritt 5 beschriebenen Verfahren wurden aus 150 mg (0.617 mmol) der Verbindung aus Beispiel 116A / Schritt 3 164 mg eines Gemisches der Titelverbindung mit dem korrespondierenden Acetat (2-{1-[4-(*N'*-Hydroxycarbamimidoyl)phenyl]cyclobutyl}ethylacetat) erhalten. Dieses Gemisch wurde nicht aufgetrennt, sondern als solches in Folgereaktionen eingesetzt.

LC/MS (Methode I, ESIpos): Titelverbindung: Rₜ = 0.52 min, m/z = 234 [M+H]⁺; korrespondierendes Acetat: Rₜ = 0.70 min, m/z = 277 [M+H]⁺.

### Beispiel 117A

### 2-{1-[4-(N'-Hydroxycarbamimidoyl)phenyl]cyclobutyl}-N,N-dimethylacetamid

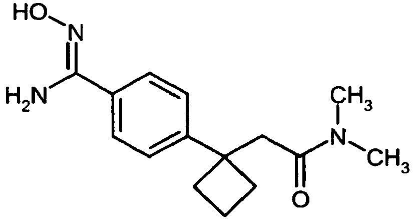

### Schritt 1: {1-[4-(Dibenzylamino)phenyl]cyclobutyl}essigsäure

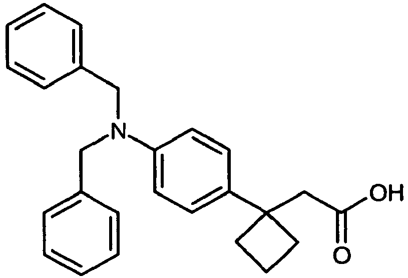

Eine Lösung von 6.0 g (14.5 mmol) der Verbindung aus Beispiel 113A / Schritt 1 in 90 ml Ethanol wurde mit 43.5 ml (43.5 mmol) 1 M Natronlauge versetzt und 3 h unter Rückfluss erhitzt. Nach dem Abkühlen auf RT wurde mit 1 M Salzsäure neutralisiert und das Ethanol am Rotationsverdampfer weitestgehend abgezogen. Die erhaltene wässrige Lösung wurde dreimal mit je ca. 100 ml Ethylacetat extrahiert. Die vereinigten organischen Extrakte wurden über wasserfreiem Magnesiumsulfat getrocknet. Nach Filtration wurde das Lösungsmittel am Rotationsverdampfer entfernt. Das Rohprodukt wurde mittels Saugfiltration über ca. 200 g Kieselgel mit Cyclohexan/ Ethylacetat 4:1 als Laufmittel grob gereinigt. Es wurden so 5.35 g (86% d. Th., 90% Reinheit) der Titelverbindung erhalten.

¹H-NMR (400 MHz, CDCl₃, δ/ppm): 10.97 (sehr breit, 1H), 7.33-7.28 (m, 4H), 7.25-7.22 (m, 6H), 6.99 (d, 2H), 6.66 (d, 2H), 4.60 (s, 4H), 2.73 (s, 2H), 2.43-2.27 (m, 4H), 2.04-1.96 (m, 1H), 1.88-1.78 (m, 1H).

HPLC (Methode A): Rₜ = 4.76 min.

MS (DCI, NH₃): m/z = 386 [M+H]⁺.

LC/MS (Methode I, ESIpos): Rₜ = 1.35 min, m/z = 386 [M+H]⁺.

### Schritt 2: 2-{1-[4-(Dibenzylamino)phenyl]cyclobutyl}-N,N-dimethylacetamid

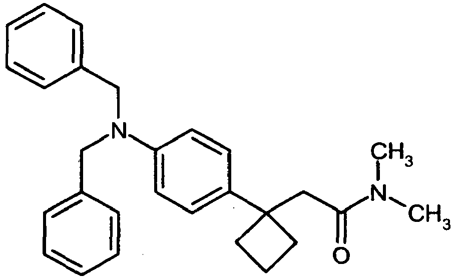

Unter inerten Bedingungen wurden 2.65 g (6.87 mmol) der Verbindung aus Beispiel 117A / Schritt 1 in 70 ml wasserfreiem Dichlormethan gelöst und mit 3 ml (34.4 mmol) Oxalylchlorid sowie einem Tropfen DMF versetzt. Nachdem das Gemisch 2 h bei RT gerührt worden war, wurde es am Rotationsverdampfer bis zur Trockene eingeengt. Der erhaltene Rückstand wurde ca. 1 h im Hochvakuum getrocknet und dann in 30 ml wasserfreiem THF gelöst. Diese Lösung wurde zu einem Gemisch von 10.3 ml (20.6 mmol) einer 2 M Lösung von Dimethylamin in THF, die zuvor mit 30 ml THF verdünnt worden war, und 3.6 ml (20.6 mmol) *N,N*-Diisopropylethylamin hinzugetropft. Nach 16 h Rühren bei RT wurde das Reaktionsgemisch am Rotationsverdampfer von allem Flüchtigen befreit. Der erhaltene Rückstand wurde in 300 ml Ethylacetat aufgenommen und nacheinander mit je ca. 100 ml gesättigter Natriumhydrogencarbonat-Lösung, Wasser und gesättigter Kochsalz-Lösung gewaschen. Nach Trocknen über wasserfreiem Magnesiumsulfat wurde filtriert und das Filtrat am Rotationsverdampfer eingeengt. Es wurden 1.93 g (68% d. Th.) der Titelverbindung erhalten.

¹H-NMR (400 MHz, CDCl₃, δ/ppm): 7.33-7.29 (m, 4H), 7.26-7.22 (m, 6H), 6.94 (d, 2H), 6.65 (d, 2H), 4.61 (s, 4H), 2.69 (s, 2H), 2.67 (s, 3H), 2.47-2.31 (m, 4H), 2.25 (s, 3H), 2.12-2.01 (m, 1H), 1.87-1.77 (m, 1H).

LC/MS (Methode F, ESIpos): Rₜ = 1.53 min, m/z = 413 [M+H]⁺.

### Schritt 3: 2-[1-(4-Aminophenyl)cyclobutyl]-N,N-dimethylacetamid

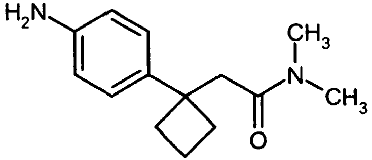

Analog zu dem unter Beispiel 4A / Schritt 6 beschriebenen Verfahren wurden aus 1.93 g (4.68 mmol) der Verbindung aus Beispiel 117A / Schritt 2 1.1 g (99% d. Th., 98% Reinheit) der Titelverbindung erhalten. Als Lösungsmittel wurden in diesem Fall 250 ml eines Gemisches aus Ethanol und Ethylacetat (3:1) verwendet. Das nach dem Abdampfen des Lösungsmittels erhaltene Produkt wurde ohne weitere chromatographische Aufreinigung in der Folgereaktion eingesetzt.

¹H-NMR (400 MHz, CDCl₃, δ/ppm): 6.96 (d, 2H), 6.62 (d, 2H), 3.58 (breit, 2H), 2.74 (s, 3H), 2.72 (s, 2H), 2.50-2.43 (m, 2H), 2.39-2.32 (m, 2H), 2.33 (s, 3H), 2.17-2.03 (m, 1H), 1.87-1.78 (m, 1H).

LC/MS (Methode F, ESIpos): Rₜ = 0.55 min, m/z = 233 [M+H]⁺.

### Schritt 4: 2-[1-(4-Cyanophenyl)cyclobutyl]-N,N-dimethylacetamid

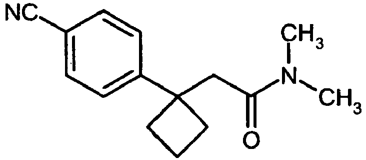

Analog zu dem unter Beispiel 4A / Schritt 7 beschriebenen Verfahren wurden aus 1.0 g (4.30 mmol) der Verbindung aus Beispiel 117A / Schritt 3 776 mg (74% d. Th.) der Titelverbindung erhalten. Das Rohprodukt wurde mittels präparativer HPLC (Methode N) gereinigt.

¹H-NMR (400 MHz, CDCl₃, δ/ppm): 7.57 (d, 2H), 7.33 (d, 2H), 2.86 (s, 2H), 2.76 (s, 3H), 2.57 (s, 3H), 2.51-2.39 (m, 4H), 2.19-2.07 (m, 1H), 1.92-1.82 (m, 1H).

MS (DCI, NH₃): m/z = 260 [M+NH₄]⁺, 243 [M+H]⁺.

GC/MS (Methode L, EIpos): Rₜ = 7.39 min, m/z = 242 [M]⁺.

### Schritt 5: 2-{1-[4-(N'-Hydroxycarbamimidoyl)phenyl]cyclobutyl}-N,N-dimethylacetamid

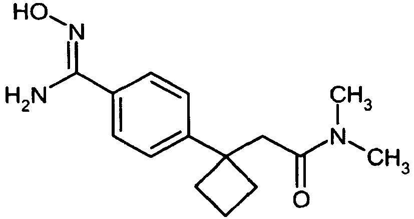

Analog zu dem unter Beispiel 1A / Schritt 5 beschriebenen Verfahren wurden aus 730 mg (3.01 mmol) der Verbindung aus Beispiel 117A / Schritt 4 669 mg (73% d. Th., 91% Reinheit) der Titelverbindung erhalten. Das abschließende Verrühren des Produktes erfolgte hier nicht in Petrolether, sondern in Ethanol.

¹H-NMR (400 MHz, DMSO-d₆, δ/ppm): 9.53 (s, 1H), 7.56 (d, 2H), 7.17 (d, 2H), 5.73 (s, 2H), 2.82 (s, 2H), 2.62 (s, 3H), 2.53 (s, 3H), 2.41-2.23 (m, 2H), 2.32-2.25 (m, 2H), 2.10-1.98 (m, 1H), 1.80-1.70 (m, 1H).

HPLC (Methode A): Rₜ = 3.27 min.

MS (DCI, NH₃): m/z = 551 [2M+H]⁺, 276 [M+H]⁺.

### Beispiel 118A

### 4-[(Diisopropylamino)methyl]-N'-hydroxybenzolcarboximidamid

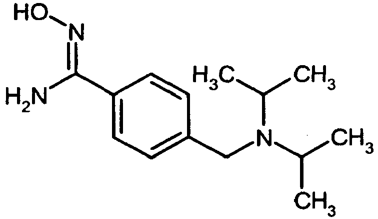

### Schnitt 1: 4-[(Diisopropylamino)methyl]benzonitril

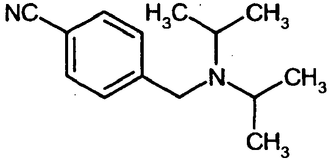

Man erhitzte ein Gemisch aus 4.0 g (20.4 mmol) 4-(Brommethyl)benzonitril und 6.19 g (61.2 mmol) Diisopropylamin in 40 ml Toluol in zwei Portionen in einem Mikrowellengerät (CEM Discover, initiale Einstrahlleistung 250 W) für jeweils 3 h auf 150°C. Nach dem Abkühlen auf RT filtrierte man vom gebildeten Feststoff ab, engte das Filtrat ein und erhielt so 4.52 g (92% d. Th., 90% Reinheit) der Titelverbindung.

LC/MS (Methode F, ESIpos): Rₜ = 0.30 min, m/z = 217 [M+H]⁺.

### Schritt 2: 4-[(Diisopropylamino)methyl]-N'-hydroxybenzolcarboximidamid

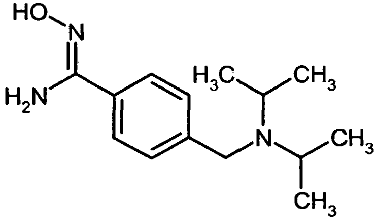

Analog zu dem unter Beispiel 1A / Schritt 5 beschriebenen Verfahren wurden aus 6.80 g (28.3 mmol, 90% Reinheit) der Verbindung aus Beispiel 118A / Schritt 1 4.93 g (70% d. Th.) der Titelverbindung erhalten.

¹H-NMR (400 MHz, CDCl₃, δ/ppm): 7.52 (d, 2H), 7.41 (d, 2H), 4.84 (s, breit, 2H), 3.64 (s, 2H), 3.05-2.95 (m, 2H), 1.01 (d, 12H).

LC/MS (Methode I, ESIpos): Rₜ = 0.18 min, m/z = 250 [M+H]⁺.

### Beispiel 119A

### N'-Hydroxy-4-[N-methyl-S-(trifluormethyl)sulfonimidoyl]benzolcarboximidamid (Racemat)

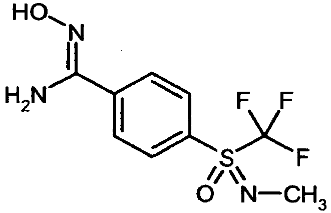

### Schritt 1: 4-[S-(Trifluormethyl)sulfonimidoyl]benzonitril (Racemat)

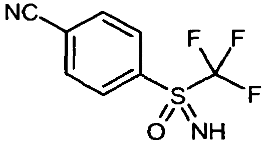

150 mg (0.66 mmol) 1-Fluor-4-[*S*-(trifluormethyl)sulfonimidoyl]benzol [N.V. Kondratenko, Zhurnal Organicheskoi Khimii 1986, 22 (8), 1716-1721; ibid. 1984, 20 (10), 2250-2252] wurden in 20 ml DMSO gelöst und mit 115 mg (0.83 mmol) Kaliumcarbonat, 140 mg (0.84 mmol) Kaliumiodid sowie 130 mg (2.0 mmol) Kaliumcyanid versetzt. Man erhitzte das Gemisch über Nacht unter Rühren auf 110°C. Nach dem Abkühlen auf RT wurde das Gemisch mit ca. 10 ml Wasser versetzt und mit Ethylacetat extrahiert. Nach Einengen der organischen Phase wurde der Rückstand mittels Flash-Chromatographie an Kieselgel gereinigt. Man erhielt 50 mg (33% d. Th.) der Titelverbindung.

### Schritt 2: 4-[N-Methyl-S-(trifluonnethyl)sulfonimidoyl]benzonitril (Racemat)

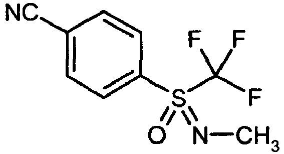

400 mg (1.60 mmol) der Verbindung aus Beispiel 119A / Schritt 1 wurden in 8 ml THF unter Argon gelöst und mit 224 mg (2.0 mmol) Kalium-*tert.*-butylat versetzt. Man rührte das Gemisch zunächst 1 h bei RT, gab dann 283 mg (2.0 mmol) Iodmethan hinzu und rührte das Gemisch weiter über Nacht bei RT. Anschließend wurde der Ansatz mit Wasser versetzt und mit Ethylacetat extrahiert. Der organische Extrakt wurde mit gesättigter wässriger Natriumchlorid-Lösung gewaschen, über Magnesiumsulfat getrocknet und eingeengt. Der Rückstand wurde mittels Flash-Chromatographie an Kieselgel gereinigt. Man erhielt 298 mg (70% d. Th.) der Titelverbindung.

¹H-NMR (400 MHz, CDCl₃, δ/ppm): 8.22 (d, 2H), 7.90 (d, 2H), 3.10 (s, 3H).

LC/MS (Methode D, ESIpos): Rₜ = 2.17 min, m/z = 249 [M+H]⁺.

### Schritt 3: N'-Hydroxy-4-[N-methyl-S-(trifluormethyl)sulfonimidoyl]benzolcarboximidamid (Racemat)

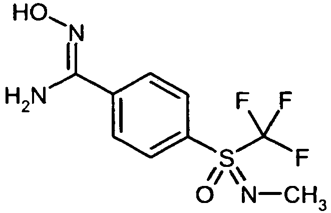

1.00 g (4.03 mmol) der Verbindung aus Beispiel 119A / Schritt 2 wurden in 20 ml Ethanol vorgelegt. Man gab 616 mg (8.86 mmol) Hydroxylamin-Hydrochlorid sowie 1.2 ml (8.86 mmol) Triethylamin hinzu und erhitzte das Gemisch 1 h unter Rückfluss. Anschließend engte man ein und nahm den Rückstand in einem Gemisch aus Ethylacetat und Wasser auf. Die Phasen wurden getrennt, und die wässrige Phase wurde einmal mit Ethylacetat extrahiert. Man wusch die vereinigten Ethylacetat-Phasen einmal mit gesättigter wässriger Natriumchlorid-Lösung, trocknete über Magnesiumsulfat, filtrierte und engte ein. Der Rückstand wurde mittels Säulenchromatographie (Kieselgel, Laufmittel: Cyclohexan/Ethylacetat 7:3) gereinigt. Die vereinigten Produktfraktionen wurden eingeengt und der Rückstand mit Pentan verrührt. Der resultierende Feststoff wurde abfiltriert und im Vakuum getrocknet. Man erhielt 775 mg (66% d. Th.) der Titelverbindung.

¹H-NMR (400 MHz, CDCl₃, δ/ppm): 8.12 (d, 2H), 8.04 (s, breit, 1H), 7.87 (d, 2H), 4.93 (s, 2H), 3.10 (s, 3H).

LC/MS (Methode I, ESIpos): Rₜ = 0.76 min, m/z = 282 [M+H]⁺.

### Beispiel 120A

### 3-Chlor-N'-hydroxy-4-(trifluormethoxy)benzolcarboximidamid

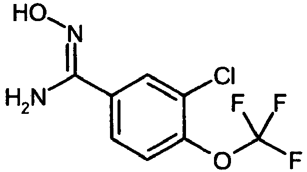

Analog zu dem unter Beispiel 1A / Schritt 5 beschriebenen Verfahren wurden aus 1.00 g (4.51 mmol) 3-Chlor-4-(trifluormethoxy)benzonitril 842 mg (73% d. Th.) der Titelverbindung erhalten.

¹H-NMR (400 MHz, CDCl₃, δ/ppm): 7.77 (d, 1H), 7.58-7.55 (dd, 1H), 7.37-7.33 (m, 1H), 4.82 (s, breit, 1H).

LC/MS (Methode D, ESIpos): Rₜ = 1.64 min, m/z = 255/257 [M+H]⁺.

### Beispiel 121A

### N'-Hydroxy-4-[1-(trifluormethyl)cyclopropyl]benzolcarboximidamid

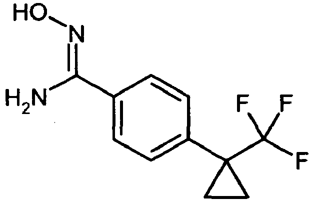

### Schritt 1: 1-Brom-4-[1-(trifluormethyl)cyclopropyl]benzol

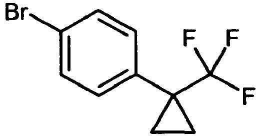

Zunächst wurde aktiviertes Zinkbromid auf Montmorillonit wie folgt dargestellt: Man legte 1.40 g (6.22 mmol) Zinkbromid in 56 ml Methanol vor, versetzte mit 5.64 g Montmorillonit K10 und rührte das Gemisch 1 h bei RT. Nach Entfernen des Methanols wurde das verbleibende Pulver 1 h bei 200°C Bad-Temperatur im Sandbad erhitzt und dann unter Argon erkalten gelassen.

Die Titelverbindung wurde anschließend wie folgt dargestellt: 10.0 g (53.7 mmol) 1-Phenyl-1-(trifluormethyl)cyclopropan wurden in 50 ml Pentan vorgelegt. Man fügte 6.1 g (5.37 mmol) des oben erhaltenen aktivierten Zinkbromids auf Montmorillonit hinzu und tropfte anschließend langsam unter Rühren in der Dunkelheit 27.7 ml (537 mmol) Brom hinzu. Das Gemisch wurde dann über Nacht bei RT in der Dunkelheit weiter gerührt. Man tropfte danach langsam 150 ml einer gesättigten wässrigen Natriumsulfit-Lösung unter Eiskühlung hinzu und rührte weitere ca. 30 min bei RT bis zur Entfärbung des Gemisches. Der Feststoff wurde abfiltriert und zweimal mit Pentan nachgewaschen. Nach Trennung der Filtrat-Phasen wurde die wässrige Phase zweimal mit je 200 ml Pentan extrahiert. Die vereinigten organischen Phasen wurden über Natriumsulfat getrocknet, filtriert und schonend eingeengt (signifikante Flüchtigkeit der Zielverbindung). Man erhielt auf diese Weise 17.1 g (> 100% d. Th.) der Titelverbindung, welche laut ¹H-NMR noch Pentan enthielt.

¹H-NMR (400 MHz, CDCl₃, δ/ppm): 7.47 (d, 2H), 7.32 (s, 2H), 1.39-1.30 (m, 2H), 1.04-0.95 (m, 2H).

GC/MS (Methode L, ESIpos): Rₜ = 3.45 min, m/z = 264/266 [M+H]⁺.

### Schritt 2: 4-[1-(Trifluormethyl)cyclopropyl]benzonitril

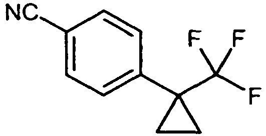

Man legte 6.00 g (22.6 mmol) der Verbindung aus Beispiel 121A / Schritt 1 in 30 ml DMF unter Argon vor, gab 1.86 g (15.8 mmol) Zinkcyanid sowie 1.57 g (1.36 mmol) Tetrakis(triphenylphosphin)palladium(0) hinzu und rührte das Gemisch über Nacht bei 80°C. Nach Abkühlen auf RT gab man weitere 4.0 g (34.1 mmol) Zinkcyanid sowie 3.0 g (2.56 mmol) Tetrakis(triphenylphosphin)-palladium(0) hinzu und erhitzte erneut unter Rühren für 5 h auf 120°C. Nach Abkühlen auf RT wurde der vorhandene Feststoff abfiltriert und einmal mit DMF gewaschen. Das mit der Waschlösung vereinigte Filtrat wurde eingeengt. Der Rückstand wurde in 200 ml Ethylacetat aufgenommen und die erhaltene Lösung zweimal mit 2 M wässriger Ammoniak-Lösung und einmal mit gesättigter wässriger Natriumchlorid-Lösung gewaschen. Nach Trocknen über Natriumsulfat, Filtrieren und Einengen wurde der erhaltene Rückstand durch Flash-Chromatographie (Kieselgel, Laufmittel: Cyclohexan/Ethylacetat 40:1) gereinigt. Nach kurzem Trocknen im Vakuum wurden 3.46 g (72% d. Th.) der Titelverbindung erhalten.

¹H-NMR (400 MHz, CDCl₃, δ/ppm): 7.66 (d, 2H), 7.58 (d, 2H), 1.47-1.41 (m, 2H), 1.09-1.03 (m, 2H).

GC/MS (Methode L, ESIpos): Rₜ = 3.81 min, m/z = 212 [M+H]⁺.

### Schritt 3: N'-Hydroxy-4-[1-(trifluormethyl)cyclopropyl]benzolcarboximidamid

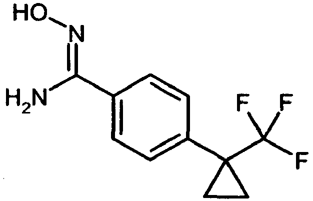

Analog zu dem unter Beispiel 1A / Schritt 5 beschriebenen Verfahren wurden aus 3.40 g (16.1 mmol) der Verbindung aus Beispiel 121A / Schritt 2 3.82 g der Titelverbindung (98% d. Th.) erhalten.

¹H-NMR (400 MHz, CDCl₃, δ/ppm): 7.62 (d, 2H), 7.50 (d, 2H), 4.88 (s, breit, 2H), 1.42-1.36 (m, 2H), 1.06-1.00 (m, 2H).

LC/MS (Methode F, ESIpos): Rₜ = 0.81 min, m/z = 245 [M+H]⁺.

### Beispiel 122A

### 3-Fluor-N'-hydroxy-4-(trifluormethoxy)benzolcarboximidamid

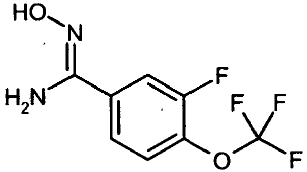

Analog zu dem unter Beispiel 1A / Schritt 5 beschriebenen Verfahren wurden aus 5.0 g (23.9 mmol, 98% Reinheit) 3-Fluor-4-(trifluormethoxy)benzonitril 5.7 g (99% d. Th., 98% Reinheit) der Titelverbindung erhalten.

¹H-NMR (400 MHz, CDCl₃, δ/ppm): 7.53-7.49 (dd, 1H), 7.45-7.41 (m, 1H), 7.37-7.31 (t, 1H), 4.87 (s, breit, 2H).

LC/MS (Methode I, ESIpos): Rₜ = 0.74 min, m/z = 239 [M+H]⁺.

### Beispiel 123A

### 4-[4-(Fluormethyl)tetrahydro-2H-pyran-4-yl]-N'-hydroxybenzolcarboximidamid

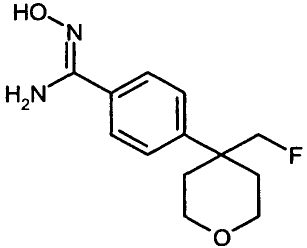

### Schritt 1: Ethyl-4-(4-bromphenyl)tetrahydro-2H-pyran-4-carboxylat

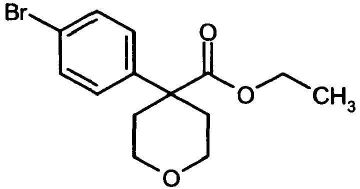

6.0 g (24.7 mmol) Ethyl-4-bromphenylacetat wurden in 120 ml abs. DMF unter Argon gelöst, unter Eisbadkühlung mit 1.48 g (37.0 mmol, 60%-ig) Natriumhydrid versetzt und 30 min gerührt. Anschließend gab man unter stetiger Eisbadkühlung 5.72 g (24.7 mmol) Bis(2-bromethyl)ether hinzu und rührte das Gemisch 1 h bei ca. 0°C. Nach erneuter Zugabe von 1.48 g 60%-igem Natriumhydrid wurde nochmals 1 h unter Eisbadkühlung gerührt. Anschließend wurde mit gesättigter wässriger Ammoniumchlorid-Lösung versetzt und mit Ethylacetat extrahiert. Die organische Phase wurde mit Wasser und mit gesättigter wässriger Natriumchlorid-Lösung gewaschen, über Magnesiumsulfat getrocknet, filtriert und am Rotationsverdampfer eingeengt. Der Rückstand wurde säulenchromatographisch an Kieselgel gereinigt (Laufmittel: Cyclohexan/Ethylacetat 10:1). Es wurden 2.62 g (33% d. Th.) der Titelverbindung erhalten.

¹H-NMR (400 MHz, CDCl₃, δ/ppm): 7.47 (d, 2H), 7.25 (d, 2H), 4.14 (q, 2H), 3.93 (dt, 2H), 3.56 (td, 2H), 2.59 (dd, 2H), 1.93 (m, 2H), 1.19 (t, 3H).

MS (DCI, NH₃): m/z = 329 [M+NH₄]⁺.

LC/MS (Methode F, ESIpos): Rₜ = 1.33 min, keine Ionisierung.

### Schritt 2: [4-(4-Bromphenyl)tetrahydro-2H-pyran-4-yl]methanol

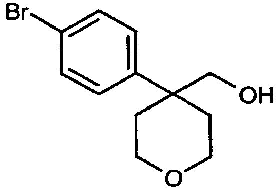

1.14 g (3.64 mmol) der Verbindung aus Beispiel 123A / Schritt 1 wurden in 18 ml THF gelöst, bei 0°C mit 3.64 ml (3.64 mmol) einer 1 M Lösung von Lithiumaluminiumhydrid in THF versetzt und 1 h unter Eisbadkühlung gerührt. Anschließend wurde gesättigte wässrige Ammoniumchlorid-Lösung zugetropft und das Gemisch dann mit Ethylacetat verdünnt. Die organische Phase wurde nacheinander mit 1 N Natronlauge, Wasser und gesättigter wässriger Natriumchlorid-Lösung gewaschen, über Magnesiumsulfat getrocknet, filtriert und am Rotationsverdampfer eingeengt. Nach Trocknen des Rückstands im Vakuum wurden 780 mg (79% d. Th.) der Titelverbindung erhalten.

¹H-NMR (400 MHz, CDCl₃, δ/ppm): 7.51 (d, 2H), 7.22 (d, 2H), 3.79 (m, 2H), 3.59 (d, 2H), 3.54 (t, 2H), 2.09 (d, 2H), 1.91 (m, 2H).

GC/MS (Methode L): Rₜ = 7.09 min, m/z = 252 [M-H₂O]⁺.

### Schritt 3: 4-(4-Bromphenyl)-4-(fluormethyl)tetrahydro-2H-pyran

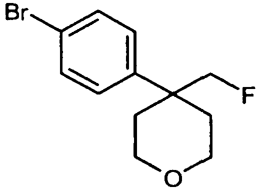

Zu einer Lösung von 400 mg (1.47 mmol) der Verbindung aus Beispiel 123A / Schritt 2 in 4.0 ml Dichlormethan wurden bei -78°C 0.63 ml einer 50%-igen Lösung von Bis(2-methoxyethyl)amino-schwefeltrifluorid in THF getropft und die Mischung dann über Nacht bei RT gerührt. Der Ansatz wurde danach mit 1 N Natronlauge versetzt, mit Dichlormethan verdünnt und mit Wasser sowie gesättigter wässriger Natriumchlorid-Lösung gewaschen. Die organische Phase wurde über Magnesiumsulfat getrocknet, filtriert und am Rotationsverdampfer vom Lösungsmittel befreit. Der erhaltene Rückstand wurde mittels präparativer HPLC gereinigt (Methode P). Es wurden 131 mg (33% d. Th.) der Titelverbindung erhalten.

¹H-NMR (400 MHz, DMSO-d₆, δ/ppm): 7.50 (d, 2H), 7.18 (d, 2H), 3.69 (m, 2H), 3.49 (td, 2H), 2.92 (d, 2H), 1.79-1.62 (m, 2H), 1.54 (t, 2H).

GC/MS (Methode L): Rₜ = 6.03 min, m/z = 272 [M]⁺.

### Schritt 4: 4-[4-(Fluormethyl)tetrahydro-2H-pyran-4-yl]benzonitril

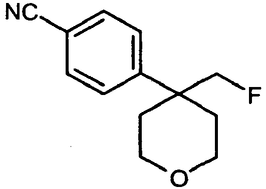

Man legte 360 mg (1.32 mmol) der in Beispiel 123A / Schritt 3 erhaltenen Verbindung in 2.1 ml entgastem DMF unter Argon vor, gab 93 mg (0.79 mmol) Zinkcyanid sowie 91 mg (0.08 mmol) Tetrakis(triphenylphosphin)palladium(0) hinzu und rührte 1 h in der Mikrowelle bei 110°C. Nach Abkühlen auf RT wurde vom Feststoff abfiltriert und das Filtrat direkt mittels präparativer HPLC gereinigt (Methode P). Es wurden 195 mg (67% d. Th.) der Titelverbindung erhalten.

¹H-NMR (400 MHz, DMSO-d₆, δ/ppm): 7.78 (d, 2H), 7.43 (d, 2H), 3.71 (m, 2H), 3.48 (td, 2H), 3.05 (d, 2H), 1.81-1.65 (m, 2H), 1.54 (t, 2H).

GC/MS (Methode L): Rₜ = 6.32 min, m/z = 199 [M-HF]⁺.

### Schritt 5: 4-[4-(Fluormethyl)tetrahydro-2H-pyran-4-yl]-N'-hydroxybenzolcarboximidamid

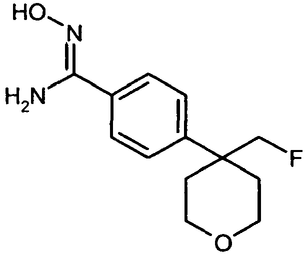

Ein Gemisch aus 410 mg (1.84 mmol) der Verbindung aus Beispiel 123A / Schritt 4, 286 mg (4.11 mmol) Hydroxylamin-Hydrochlorid und 0.57 ml (4.11 mmol) Triethylamin in 9.1 ml Ethanol wurde 2 h bei 80°C gerührt. Nach dem Abkühlen auf RT wurde das Lösungsmittel am Rotationsverdampfer nahezu vollständig entfernt. Der Rückstand wurde mit etwas Wasser unter UltraschallBestrahlung aufgeschlämmt. Der weiße Festkörper wurde abfiltriert, mit etwas kaltem Wasser gewaschen und dann mit Pentan verrührt. Der Feststoff wurde erneut abfiltriert und im Vakuum getrocknet. Man erhielt so 320 mg (65% d. Th.) der Titelverbindung.

LC/MS (Methode I): Rₜ = 0.47 min, m/z = 253 [M+H]⁺.

### Beispiel 124A

### 5-(5-Methyl-1H-pyrazol-3-yl)-3-[4-(tetrahydro-2H-pyran-4-yl)phenyl]-1,2,4-oxadiazol

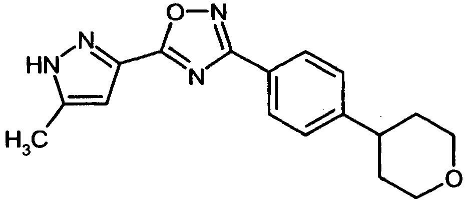

Analog zu dem unter Beispiel 28A beschriebenen Verfahren wurden aus 469 mg (3.72 mmol) 5-Methyl-1*H*-pyrazol-3-carbonsäure und 820 mg (3.72 mmol) der Verbindung aus Beispiel 107A 450 mg der Titelverbindung nach Ausrühren des Rohprodukts in Acetonitril und weitere 97 mg der Titelverbindung nach Aufreinigung der Mutterlauge durch präparative HPLC (Methode N) erhalten (Ausbeute insgesamt 47% d. Th.).

¹H-NMR (400 MHz, DMSO-d₆, δ/ppm): 13.52 (s, 1H), 8.01 (d, 2H), 7.49 (d, 2H), 6.79 (s, 1H), 3.99-3.95 (m, 2H), 3.49-3.42 (m, 2H), 2.92-2.84 (m, 1H), 2.34 (s, 3H), 1.77-1.65 (m, 4H).

LC/MS (Methode I, ESIpos): Rₜ = 0.98 min, m/z = 311 [M+H]⁺.

### Beispiel 125A

### 3-(4-tert.-Butylphenyl)-5-(5-methyl-1H-pyrazol-3-yl)-1,2,4-oxadiazol

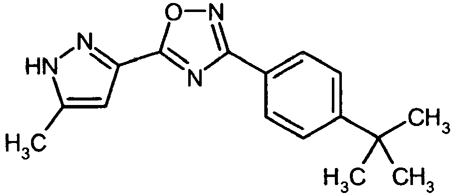

Analog zu dem unter Beispiel 28A beschriebenen Verfahren wurden 2.50 g (19.8 mmol) 5-Methyl-1*H*-pyrazol-3-carbonsäure und 4.19 g (21.8 mmol) 4-*tert*.-Butyl-*N*'-hydroxybenzolcarboximidamid zu 2.60 g (46% d. Th.) der Titelverbindung umgesetzt. Das Reaktionsgemisch wurde hier zunächst 1 h bei RT und anschließend 30 min bei 140°C gerührt.

¹H-NMR (400 MHz, CDCl₃, δ/ppm): 11.08 (breit, 1H), 8.10 (d, 2H), 7.51 (d, 2H), 6.81 (s, 1H), 2.45 (s, 3H), 1.37 (s, 9H).

LC/MS (Methode I, ESIpos): Rₜ = 1.21 min, m/z = 283 [M+H]⁺, 565 [2M+H]⁺.

### Beispiel 126A

### 3-{4-[1-(Methoxymethyl)cyclobutyl]phenyl}-5-(5-methyl-1H-pyrazol-3-yl)-1,2,4-oxadiazol

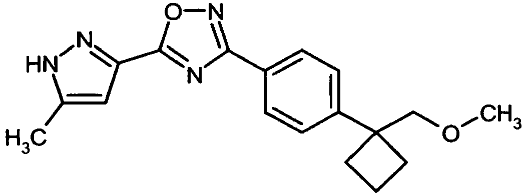

Analog zu dem unter Beispiel 28A beschriebenen Verfahren wurden 1.08 g (8.52 mmol) 5-Methyl-1*H*-pyrazol-3-carbonsäure und 2.0 g (8.52 mmol) der Verbindung aus Beispiel 110A zu 1.87 g (46% d. Th.) der Titelverbindung umgesetzt. Das Reaktionsgemisch wurde hier zunächst 1 h bei RT und anschließend 30 min bei 140°C gerührt.

¹H-NMR (400 MHz, CDCl₃, δ/ppm): 11.57 (breit, 1H), 8.10 (d, 2H), 7.30 (d, 2H), 6.81 (s, 1H), 3.57 (s, 2H), 3.29 (s, 3H), 2.45 (s, 3H), 2.41-2.28 (m, 4H), 2.15-2.03 (m, 1H), 1.93-1.84 (m, 1H).

LC/MS (Methode F, ESIpos): Rₜ = 1.28 min, m/z = 325 [M+H]⁺.

### Beispiel 127A

### 5-(5-Methyl-1H-pyrazol-3-yl)-3-[3-methyl-4-(tetrahydro-2H-pyran-4-yl)phenyl]-1,2,4-oxadiazol

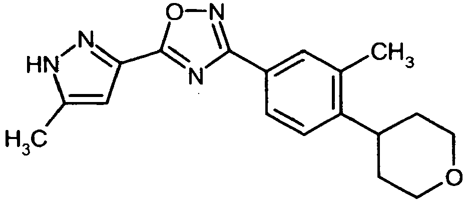

Analog zu dem unter Beispiel 28A beschriebenen Verfahren wurden 180 mg (1.43 mmol) 5-Methyl-1*H*-pyrazol-3-carbonsäure und 335 mg (1.43 mmol) der Verbindung aus Beispiel 108A zu 189 mg (39% d. Th.) der Titelverbindung umgesetzt. Das Reaktionsgemisch wurde hier zunächst 16 h bei RT und anschließend 30 min bei 140°C gerührt. Die Reinigung des Produktes erfolgte mittels präparativer HPLC (Methode N).

¹H-NMR (400 MHz, CDCl₃, δ/ppm): 10.63 (breit, 1H), 8.00 (d, 1H), 7.99 (s, 1H), 7.36 (d, 1H), 6.80 (s, 1H), 4.13-4.10 (m, 2H), 3.61-3.54 (m, 2H), 3.07-3.00 (m, 1H), 2.45 (s, 3H), 2.43 (s, 3H), 1.92-1.80 (m, 2H), 1.73-1.68 (m, 2H).

LC/MS (Methode I, ESIpos): Rₜ = 1.02 min, m/z = 325 [M+H]⁺.

### Beispiel 128A

### 3-[3-Chlor-4-(trifluormethoxy)phenyl]-5-(5-methyl-1H-pyrazol-3-yl)-1,2,4-oxadiazol

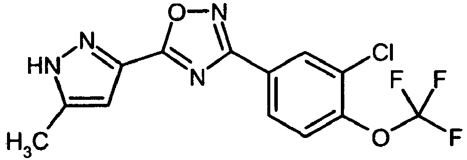

Analog zu dem unter Beispiel 28A beschriebenen Verfahren wurden 631 mg (5.00 mmol) 5-Methyl-1*H*-pyrazol-3-carbonsäure und 1.27 g (5.00 mmol) der Verbindung aus Beispiel 120A zu 1.08 g (60% d. Th., 95% Reinheit) der Titelverbindung umgesetzt. Das Reaktionsgemisch wurde hier zunächst ca. 30 min bei RT und anschließend ca. 1 h bei 150°C gerührt. Das Produkt wurde erhalten, indem man nach beendeter Reaktion den nach Zugabe von Wasser ausgefallenen Feststoff abfiltrierte, mit Wasser wusch und im Vakuum trocknete.

LC/MS (Methode I, ESIpos): Rₜ = 1.20 min, m/z = 345/347 [M+H]⁺.

### Beispiel 129A

### 5-(5-Methyl-1H-pyrazol-3-yl)-3-{4-[1-(trifluormethyl)cyclopropyl]phenyl}-1,2,4-oxadiazol

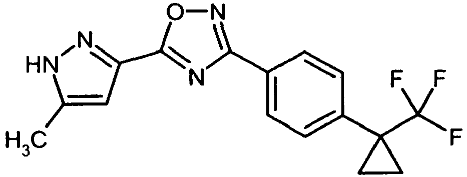

Analog zu dem unter Beispiel 28A beschriebenen Verfahren wurden 1.19 g (9.42 mmol) 5-Methyl-1*H*-pyrazol-3-carbonsäure und 2.30 g (9.42 mmol) der Verbindung aus Beispiel 121A zu 1.05 g (62% d. Th.) der Titelverbindung umgesetzt. Das Rohprodukt wurde mittels präparativer HPLC (Methode O) aufgereinigt.

¹H-NMR (400 MHz, CDCl₃, δ/ppm): 11.0-10.5 (s, breit, 1H), 8.16 (d, 2H), 7.60 (d, 2H), 6.82 (s, 1H), 1.43-1.39 (m, 2H), 1.12-1.08 (m, 2H).

LC/MS (Methode I): Rₜ = 1.17 min, m/z = 335 [M+H]⁺.

### Beispiel 130A

### 5-(5-Methyl-1H-pyrazol-3-yl)-3-{4-[N-methyl-S-(trifluormethyl)sulfonimidoyl]phenyl}-1,2,4-oxadiazol (Racemat)

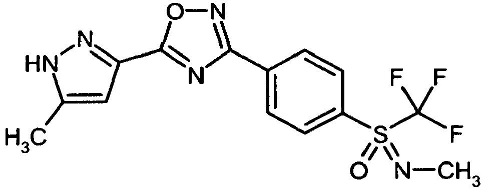

Analog zu dem unter Beispiel 28A beschriebenen Verfahren wurden 89 mg (0.709 mmol) 5-Methyl-1*H*-pyrazol-3-carbonsäure und 206 mg (0.709 mmol, 97% Reinheit) der Verbindung aus Beispiel 119A zu 103 mg (39% d. Th.) der Titelverbindung umgesetzt. Das Rohprodukt wurde mittels präparativer HPLC (Methode O) aufgereinigt.

¹H-NMR (400 MHz, CDCl₃, δ/ppm): 10.59 (s, breit, 1H), 8.42 (d, 2H), 8.22 (d, 2H), 6.83 (s, 1H), 3.12 (d, 3H), 2.47 (s, 3H).

LC/MS (Methode I, ESIpos): Rₜ = 1.10 min, m/z = 372 [M+H]⁺.

### Beispiel 131A

### 3-[3-Fluor-4-(trifluormethoxy)phenyl]-5-(5-methyl-1H-pyrazol-3-yl)-1,2,4-oxadiazol

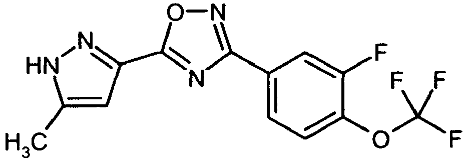

Analog zu dem unter Beispiel 28A beschriebenen Verfahren wurden 2.0 g (15.9 mmol) 5-Methyl-1*H*-pyrazol-3-carbonsäure und 3.78 g (15.9 mmol) der Verbindung aus Beispiel 122A zu 3.15 g (56% d. Th., 92% Reinheit) der Titelverbindung umgesetzt. Das Produkt wurde in diesem Fall nicht durch chromatographische Reinigung, sondern durch Waschen des Rohprodukts mit Wasser und Pentan und anschließendes Trocknen im Vakuum erhalten.

¹H-NMR (400 MHz, CDCl₃, δ/ppm): 12.0-9.5 (s, breit, 1H), 8.10-7.97 (m, 2H), 7.46-7.41 (t, 1H), 6.81 (s, 1H), 2.47 (s, 3H).

LC/MS (Methode I, ESIpos): Rₜ = 1.16 min, m/z = 329 [M+H]⁺.

### Beispiel 132A

### N-Isopropyl-N-{4-[5-(5-methyl-1H-pyrazol-3-yl)-1,2,4-oxadiazol-3-yl]benzyl}propan-2-amin

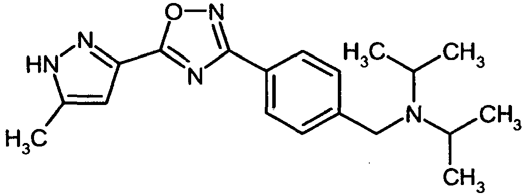

Analog zu dem unter Beispiel 28A beschriebenen Verfahren wurden 2.00 g (15.9 mmol) 5-Methyl-1*H*-pyrazol-3-carbonsäure und 3.95 g (15.9 mmol) der Verbindung aus Beispiel 118A zu 1.49 g (26% d. Th., 93% Reinheit) der Titelverbindung umgesetzt.

¹H-NMR (400 MHz, CDCl₃, δ/ppm): 11.50 (s, breit, 1H), 8.08 (d, 2H), 7.51 (d, 2H), 6.81 (s, 1H), 3.70 (s, 2H), 3.10-2.98 (m, 2H), 2.42 (s, 3H), 1.02 (d, 12H).

LC/MS (Methode F, ESIpos): Rₜ = 0.73 min, m/z = 340 [M+H]⁺.

### Beispiel 133A

### 2-Brom-4-(brommethyl)pyridin

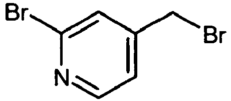

Analog zu dem unter Beispiel 47A / Schritt 3 beschriebenen Verfahren wurden aus 1.50 g (7.66 mmol) 2-Brom-4-(hydroxymethyl)pyridin 1.83 g (95% d. Th.) der Titelverbindung hergestellt.

¹H-NMR (400 MHz, CDCl₃, δ/ppm): 8.36 (d, 1H), 7.52 (s, 1H), 7.27 (d, 1H), 4.32 (s, 2H).

HPLC (Methode A): Rₜ = 3.47 min.

MS (DCI, NH₃): m/z = 250/252/254 [M+H]⁺.

### Beispiel 134A

### 3-(Brommethyl)benzolsulfonylchlorid

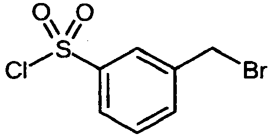

Zu einer Lösung von 5.0 g (26.2 mmol) m-Toluolsulfonsäurechlorid in 50 ml Acetonitril gab man 5.13 g (28.8 mmol) *N*-Bromsuccinimid sowie 43 mg (0.26 mmol) 2,2'-Azobis-2-methylpropannitril und erhitzte über Nacht unter Rückfluss. Der Ansatz wurde dann am Rotationsverdampfer eingeengt und der Rückstand direkt mittels MPLC gereinigt (Kieselgel, Laufmittel: Cyclohexan/ Ethylacetat 10:1). Es wurden 4.58 g (65% d. Th.) der Titelverbindung erhalten.

GC/MS (Methode L): Rₜ = 6.14 min, m/z = 189 [M-Br]⁺.

### Beispiel 135A

### 1-[4-(Chlormethyl)pyridin-2-yl]-4-cyclopropylpiperazin

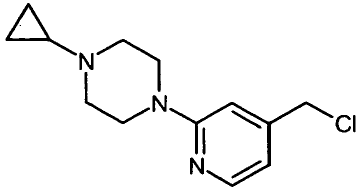

### Schritt 1: [2-(Piperazin-1-yl)pyridin-4-yl]methanol

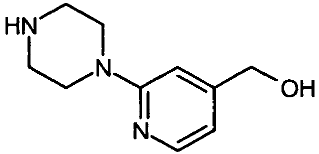

10.0 g (69.6 mmol) (2-Chlorpyridin-4-yl)methanol wurden unter Argon mit 120 g (1.39 mol) Piperazin versetzt. Man erhitzte das Gemisch über Nacht unter Rühren auf 150°C. Nach Abkühlen auf RT wurde der Teil des überschüssigen Piperazins, welcher sich im oberen Teil des Reaktionsgefäßes abgeschieden hatte, entfernt und der harzige Kolbeninhalt in 700 ml Dichlormethan aufgenommen und 30 min bei RT gerührt. Man filtrierte den vorhandenen Feststoff ab, wusch mit Dichlormethan nach, verwarf den Feststoff und engte das Filtrat ein. Der Rückstand wurde im Vakuum getrocknet. Man erhielt 13.3 g (ca. 99% d. Th.) der Titelverbindung, welche laut ¹H-NMR noch Piperazin enthielt.

¹H-NMR (400 MHz, CDCl₃, δ/ppm): 8.14 (d, 1H), 6.67 (s, 1H), 6.58 (d, 1H), 4.64 (s, 2H), 3.55-3.45 (m, 4H), 3.01-2.94 (m, 4H).

LC/MS (Methode D, ESIpos): Rₜ = 0.19 min, m/z = 194 [M+H]⁺.

### Schritt 2: [2-(4-Cyclopropylpiperazin-1-yl)pyridin-4-yl]methanol

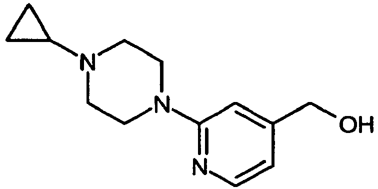

13.1 g (67.9 mmol) der Verbindung aus Beispiel 135A / Schritt 1 wurden in einem Gemisch aus 535 ml Methanol und 39 ml (679 mmol) Essigsäure gelöst. Man gab 9.2 g Molekularsieb (3Å) und 82 ml (407 mmol) [(1-Ethoxycyclopropyl)oxy](trimethyl)silan hinzu. Nach 10 min Rühren bei RT fügte man 12.8 g (203 mmol) Natriumcyanoborhydrid hinzu und erhitzte das Gemisch 2 h unter Rühren zum Rückfluss. Nach Abkühlen auf RT filtrierte man den vorhandenen Feststoff ab und wusch zweimal mit jeweils 20 ml Methanol nach. Das Filtrat wurde eingeengt und der Rückstand in 550 ml Dichlormethan aufgenommen. Man wusch zweimal mit je 500 ml gesättigter wässriger Natriumhydrogencarbonat-Lösung und einmal mit 500 ml gesättigter wässriger Natriumchlorid-Lösung, trocknete über Magnesiumsulfat, filtrierte und engte ein. Der Rückstand wurde mittels Säulenchromatographie (Kieselgel, Laufmittel: Dichlormethan/Methanol 95:5) gereinigt. Nach Trocknen im Vakuum wurden 9.59 g (61% d. Th.) der Titelverbindung erhalten.

¹H-NMR (400 MHz, CDCl₃, δ/ppm): 8.13 (d, 1H), 6.67 (s, 1H), 6.57 (d, 1H), 4.63 (s, 2H), 3.58-3.46 (m, 4H), 2.77-2.66 (m, 4H), 1.70-1.60 (m, 1H), 0.55-0.41 (m, 4H).

LC/MS (Methode I, ESIpos): Rₜ = 0.17 min, m/z = 234 [M+H]⁺.

### Schritt 3: 1-[4-(Chlormethyl)pyridin-2-yl]-4-cyclopropylpiperazin

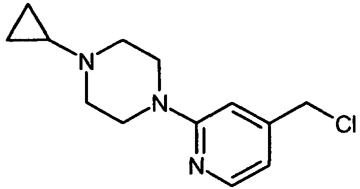

9.59 g (41.1 mmol) der Verbindung aus Beispiel 135A / Schritt 2 wurden in 60 ml Dichlormethan vorgelegt. Man gab 15 ml (205 mmol) Thionylchlorid bei RT langsam hinzu und rührte zunächst 10 min bei RT, dann 4.5 h unter Rückfluss. Nach Abkühlen auf RT wurde das Gemisch mit 40 ml Wasser versetzt, mit 460 ml gesättigter wässriger Natriumhydrogencarbonat-Lösung basisch gestellt und dreimal mit jeweils 500 ml Dichlormethan extrahiert. Die vereinigten Dichlormethan-Phasen wurden über Magnesiumsulfat getrocknet, filtriert und eingeengt. Der Rückstand wurde mittels Säulenchromatographie (Kieselgel, Laufmittel: Cyclohexan/Ethylacetat 7:3) gereinigt. Nach Trocknen im Vakuum wurden 5.47 g (53% d. Th.) der Titelverbindung erhalten.

¹H-NMR (400 MHz, CDCl₃, δ/ppm): 8.16 (d, 1H), 6.68-6.56 (m, 2H), 4.45 (s, 2H), 3.61-3.45 (m, 4H), 2.79-2.67 (m, 4H), 1.69-1.62 (m, 1H), 0.58-0.35 (m, 4H).

LC/MS (Methode I, ESIpos): Rₜ = 0.43 min, m/z = 252/254 [M+H]⁺.

### Beispiel 136A

### 1-{[3-(Brommethyl)phenyl]sulfonyl}-4-hydroxypiperidin

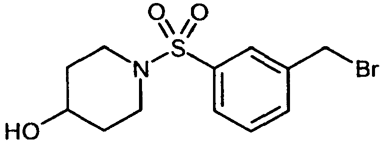

50 mg (0.5 mmol) 4-Hydroxypiperidin wurden in 2 ml THF vorgelegt und unter Eisbadkühlung mit 133 mg (0.5 mmol) der Verbindung aus Beispiel 134A und anschließend mit 69 µl (0.5 mmol) Triethylamin versetzt. Nach zweistündigem Rühren bei RT wurde der Ansatz mit 5 ml Ethylacetat verdünnt, mit Wasser und gesättigter Natriumchlorid-Lösung gewaschen, über Magnesiumsulfat getrocknet, filtriert und am Rotationsverdampfer vom Lösungsmittel befreit. Der erhaltene Rückstand (ca. 70% Reinheit nach HPLC) wurde ohne weitere Aufreinigung in Folgereaktionen eingesetzt.

### Beispiel 137A

### 1-{[3-(Brommethyl)phenyl]sulfonyl} -4-methylpiperazin

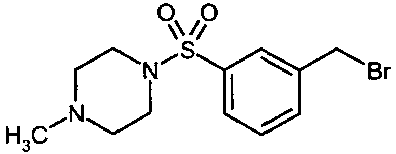

0.11 ml (1.0 mmol) 1-Methylpiperazin wurden in 2 ml THF vorgelegt und unter Eisbadkühlung mit 269 mg (1.0 mmol) der Verbindung aus Beispiel 134A und anschließend mit 139 µl (1.0 mmol) Triethylamin versetzt. Nach 30 Minuten Rühren bei RT wurde vom Niederschlag abfiltriert und das Filtrat als Lösung der Titelverbindung in THF direkt weiter umgesetzt.

### Beispiel 138A

### 1-(2,2,2-Trifluorethyl)piperazin-Dihydrochlorid

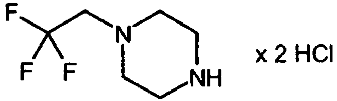

### Schritt 1: tert.-Butyl-4-(2,2,2-trifluorethyl)piperazin-1-carboxylat

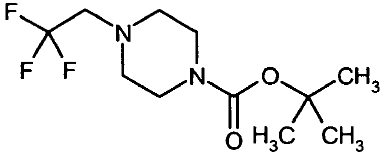

Analog zu dem unter Beispiel 104A / Schritt 1 beschriebenen Verfahren wurden aus 1.0 g (5.37 mmol) *tert*.-Butylpiperazin-1-carboxylat, 391 µl (5.37 mmol) 2,2,2-Trifluorethanol, 1 ml (6.44 mmol) Trifluormethansulfonsäureanhydrid und 1.2 ml (8.05 mmol) Triethylamin 805 mg (56% d. Th.) der Titelverbindung erhalten.

¹H-NMR (400 MHz, CDCl₃, δ/ppm): 3.44 (dd, 4H), 2.98 (quart, 2H), 2.61 (dd, 4H), 1.47 (s, 9H).

MS (DCI, NH₃): m/z = 269 [M+H]⁺.

GC/MS (Methode L, EIpos): Rₜ = 3.87 min, m/z = 212 [M-C₄H₉+H]⁺.

### Schritt 2: 1-(2,2,2-Trifluorethyl)piperazin-Dihydrochlorid

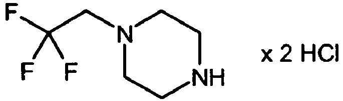

Analog zu dem unter Beispiel 103A / Schritt 2 beschriebenen Verfahren wurden ausgehend von 790 mg (2.94 mmol) der Verbindung aus Beispiel 138A / Schritt 1 241 mg (93% d. Th.) der Titelverbindung erhalten.

¹H-NMR (400 MHz, DMSO-d₆, δ/ppm): 9.14 (breit, 2H), 3.30 (quart, 2H), 3.08-3.03 (m, 4H), 2.87-2.83 (m, 4H).

MS (DCI, NH₃): m/z = 169 [M+H]⁺.

### Beispiel 139A

### 2-Brom-4-[(5-methyl-3-{3-[4-(trifluormethoxy)phenyl]-1,2,4-oxadiazol-5-yl}-1H-pyrazol-1-yl)-methyl]pyridin

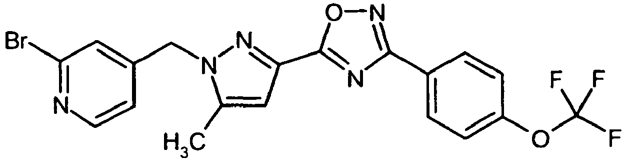

Analog zu dem unter Beispiel 76A beschriebenen Verfahren wurden 1.05 g (4.19 mmol) der Verbindung aus Beispiel 133A mit 1.0 g (3.22 mmol) der Verbindung aus Beispiel 28A zu 0.71 g (45% d. Th.) der Titelverbindung umgesetzt. Das Produkt wurde mittels MPLC gereinigt (Kieselgel, Laufmittel: Cyclohexan/Ethylacetat 4:1).

¹H-NMR (400 MHz, CDCl₃, δ/ppm): 8.35 (d, 1H), 8.24 (d, 2H), 7.33 (d, 2H), 7.22 (d, 1H), 6.99 (dd, 1H), 6.89 (s, 1H), 5.42 (s, 2H), 2.31 (s, 3H).

LC/MS (Methode I, ESIpos): Rₜ = 1.32 min, m/z = 480/482 [M+H]⁺.

### Beispiel 140A

### 2-Chlor-4-[(5-methyl-3-{3-[4-(tetrahydro-2H-pyran-4-yl)phenyl]-1,2,4-oxadiazol-5-yl}-1H-pyrazol-1-yl)methyl]pyridin

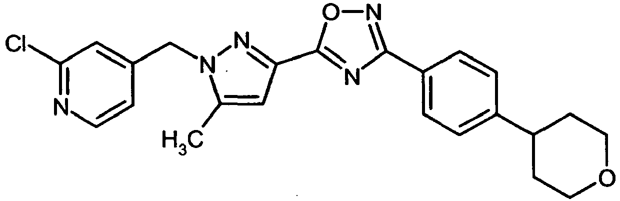

Eine Lösung von 204 mg (1.26 mmol) der Verbindung aus Beispiel 43A und 300 mg (0.967 mmol) der Verbindung aus Beispiel 124A in 10 ml wasserfreiem THF wurde bei 0°C mit 119 mg (1.06 mmol) festem Kalium-*tert*.-butylat versetzt. Dann wurde das Reaktionsgemisch zunächst 15 h bei RT und anschließend 4.5 h beim Siedepunkt des Lösungsmittels gerührt. Nach dem Abkühlen auf RT wurde mit ca. 1 ml Wasser und soviel Methanol versetzt, dass eine klare Lösung entstand. Diese wurde direkt mittels präparativer HPLC (Methode N) in ihre Komponenten aufgetrennt. Nach Entfernen des Lösungsmittels am Rotationsverdampfer wurden aus den vereinigten Produktfraktionen 220 mg (52% d. Th.) der Titelverbindung erhalten.

¹H-NMR (400 MHz, CDCl₃, δ/ppm): 8.37 (d, 1H), 8.14 (d, 2H), 7.35 (d, 2H), 7.05 (d, 1H), 6.96 (dd, 1H), 6.88 (s, 1H), 5.43 (s, 2H), 4.12-4.08 (m, 2H), 3.58-3.52 (m, 2H), 2.88-2.79 (m, 1H), 2.31 (s, 3H), 1.92-1.79 (m, 4H).

LC/MS (Methode I, ESIpos): Rₜ = 1.16 min, m/z = 436 [M+H]⁺.

### Beispiel 141A

### 4-({3-[3-(4-tert.-Butylphenyl)-1,2,4-oxadiazol-5-yl]-5-methyl-1H-pyrazol-1-yl}methyl)-2-chlor-pyridin

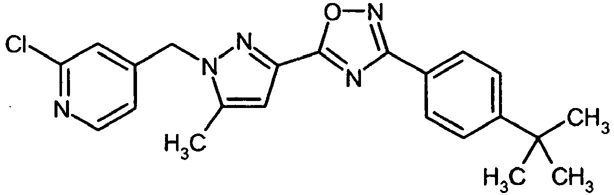

Eine Lösung von 1.15 g (7.08 mmol) der Verbindung aus Beispiel 43A und 1.00 g (3.54 mmol) der Verbindung aus Beispiel 125A in 30 ml wasserfreiem THF wurde bei 0°C mit 596 mg (5.31 mmol) festem Kalium-*tert*.-butylat versetzt. Dann wurde das Reaktionsgemisch zunächst 15 h bei RT und anschließend 4 h beim Siedepunkt des Lösungsmittels gerührt. Nach dem Abkühlen auf RT wurde mit ca. 120 ml Wasser versetzt und dreimal mit je ca. 60 ml Ethylacetat extrahiert. Die vereinigten organischen Extrakte wurden mit gesättigter Kochsalz-Lösung gewaschen und über wasserfreiem Magnesiumsulfat getrocknet. Nach Filtration und Eindampfen wurde das erhaltene Rohprodukt durch MPLC gereinigt (Kieselgel, Laufmittel: Cyclohexan/Ethylacetat 4:1 → 1:2). Es wurden 578 mg (40% d. Th.) der Titelverbindung erhalten.

¹H-NMR (400 MHz, CDCl₃, δ/ppm): 8.37 (d, 1H), 8.12 (d, 2H), 7.51 (d, 2H), 7.06 (s, 1H), 6.97 (d, 1H), 6.88 (s, 1H), 5.43 (s, 2H), 2.31 (s, 3H), 1.37 (s, 9H).

LC/MS (Methode F, ESIpos): Rₜ = 1.55 min, m/z = 408/410 [M+H]⁺.

### Beispiel 142A

### 2-Chlor-4-{[3-(3-{4-[1-(methoxymethyl)cyclobutyl]phenyl}-1,2,4-oxadiazol-5-yl)-5-methyl-1H-pyrazol-1-yl]methyl}pyridin

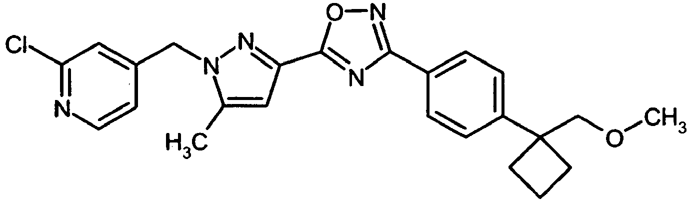

Eine Lösung von 749 mg (4.62 mmol) der Verbindung aus Beispiel 43A und 750 mg (2.31 mmol) der Verbindung aus Beispiel 126A in 22.5 ml wasserfreiem THF wurde bei 0°C mit 519 mg (4.62 mmol) festem Kalium-*tert*.-butylat versetzt. Dann wurde das Reaktionsgemisch 5 h beim Siedepunkt des Lösungsmittels gerührt. Nach dem Abkühlen auf RT wurde mit ca. 3 Tropfen Wasser versetzt, und alle flüchtigen Bestandteile wurden am Rotationsverdampfer entfernt. Der erhaltene Rückstand wurde mittels MPLC aufgereinigt (ca. 100 g Kieselgel, Laufmittel: Cyclohexan/Ethyl-acetat 2:1). Zur weiteren Reinigung wurden die vereinigten, eingeengten Produktfraktionen anschließend mit 1 ml Ethanol verrührt und der Feststoff abgesaugt. Es wurden 447 mg (43% d. Th.) der Titelverbindung erhalten.

¹H-NMR (400 MHz, CDCl₃, δ/ppm): 8.37 (d, 1H), 8.13 (d, 2H), 7.30 (d, 2H), 7.06 (s, 1H), 6.97 (d, 1H), 6.88 (s, 1H), 5.43 (s, 2H), 3.57 (s, 2H), 3.28 (s, 3H), 2.41-2.28 (m, 4H), 2.30 (s, 3H), 2.16-2.04 (m, 1H), 1.93-1.83 (m, 1H).

LC/MS (Methode I, ESIpos): Rₜ = 1.34 min, m/z = 450/452 [M+H]⁺.

### Beispiel 143A

### 2-Chlor-4-[(5-methyl-3-{3-[3-methyl-4-(tetrahydro-2H-pyran-4-yl)phenyl]-1,2,4-oxadiazol-5-yl}-1H-pyrazol-1-yl)methyl]pyridin

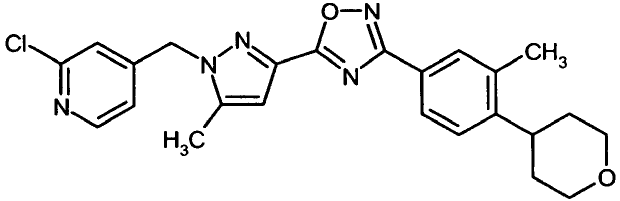

Eine Lösung von 187 mg (0.576 mmol) der Verbindung aus Beispiel 127A und 121 mg (0.749 mmol) der Verbindung aus Beispiel 43A in 6 ml wasserfreiem THF wurde bei 0°C mit 71 mg (0.634 mmol) festem Kalium-*tert*.-butylat versetzt. Anschließend ließ man das Reaktionsgemisch 16 h bei RT rühren. Da der Umsatz nicht vollständig war, wurde noch weitere 8 h unter Rückfluss erhitzt. Nach dem Abkühlen auf RT wurden 1 ml Wasser und soviel Methanol zugesetzt, dass eine klare Lösung entstand. Diese Lösung wurde dann direkt mittels präparativer HPLC (Methode N) in ihre Komponenten aufgetrennt. Die Produktfraktionen wurden vereinigt und am Rotationsverdampfer vom Lösungsmittel befreit. Es wurden 150 mg (58% d. Th.) der Titelverbindung erhalten.

¹H-NMR (400 MHz, CDCl₃, δ/ppm): 8.37 (d, 1H), 8.02 (d, 1H), 8.01 (dd, 1H), 7.34 (d, 1H), 7.05 (d, 1H), 6.96 (dd, 1H), 6.88 (s, 1H), 5.43 (s, 2H), 4.13-4.09 (m, 2H), 3.61-3.53 (m, 2H), 3.07-2.99 (m, 1H), 2.43 (s, 3H), 2.31 (s, 3H), 1.92-1.81 (m, 2H), 1.74-1.69 (m, 2H).

LC/MS (Methode F, ESIpos): Rₜ = 1.34 min, m/z = 448 [M+H]⁺.

### Beispiel 144A

### 2-Chlor-4-[(3-{3-[3-chlor-4-(trifluormethoxy)phenyl]-1,2,4-oxadiazol-5-yl}-5-methyl-1H-pyrazol-1-yl)methyl]pyridin

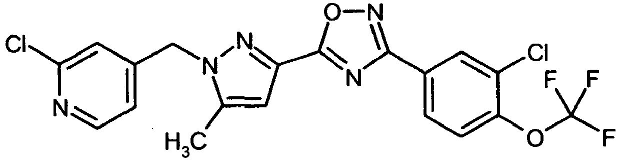

Analog zu dem unter Beispiel 75A beschriebenen Verfahren wurden 500 mg (1.38 mmol, Reinheit 95%) der Verbindung aus Beispiel 128A und 290 mg (1.79 mmol) der Verbindung aus Beispiel 43A zu 386 mg (57% d. Th., Reinheit 96%) der Titelverbindung umgesetzt, wobei die Reaktionskomponenten in diesem Fall 14 h unter Rückfluss miteinander gerührt wurden.

¹H-NMR (400 MHz, CDCl₃, δ/ppm): 8.40-8.37 (m, 2H), 8.12 (d, 1H), 7.44 (d, 1H), 7.05 (s, 1H), 6.96 (d, 1H), 6.89 (s, 1H), 5.45 (s, 2H), 2.31 (s, 3H).

LC/MS (Methode I, ESIpos): Rₜ = 1.36 min, m/z = 469 [M+H]⁺.

### Beispiel 145A

### 2-Chlor-4-{[5-methyl-3-(3-{4-[1-(trifluormethyl)cyclopropyl]phenyl}-1,2,4-oxadiazol-5-yl)-1H-pyrazol-1-yl]methyl} pyridin

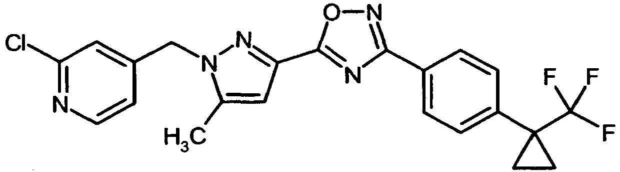

Ein Gemisch aus 450 mg (1.35 mmol) der Verbindung aus Beispiel 129A, 284 mg (1.75 mmol) 2-Chlor-4-(chlormethyl)pyridin und 166 mg (1.48 mmol) Kalium-*tert*.-butylat in 12 ml THF wurde über Nacht unter Rühren zum Rückfluss erhitzt. Nach Abkühlen auf RT wurde das Gemisch mit Ethylacetat und Wasser versetzt. Man trennte die Phasen und extrahierte die wässrige Phase zweimal mit Ethylacetat. Die vereinigten Ethylacetat-Phasen wurden einmal mit gesättigter Natrium-chlorid-Lösung gewaschen, über Magnesiumsulfat getrocknet, filtriert und eingeengt. Der Rückstand wurde mittels Säulenchromatographie gereinigt (Kieselgel, Laufmittel: Cyclohexan/Ethylacetat 7:3). Nach dem Trocknen im Vakuum erhielt man 352 mg (57% d. Th.) der Titelverbindung.

¹H-NMR (400 MHz, CDCl₃, δ/ppm): 8.37 (d, 1H), 8.19 (d, 2H), 7.60 (d, 2H), 7.05 (s, 1H), 6.96 (d, 1H), 6.88 (s, 1H), 5.44 (s, 2H), 2.30 (s, 3H), 1.48-1.33 (m, 2H), 1.09 (s, breit, 2H).

LC/MS (Methode F, ESIpos): Rₜ = 1.48 min, m/z = 460/462 [M+H]⁺.

### Beispiel 146A

### tert.-Butyl-4-[(5-methyl-3-{3-[4-(trifluormethoxy)phenyl]-1,2,4-oxadiazol-5-yl}-1H-pyrazol-1-yl)-methyl]-3',6'-dihydro-2,4'-bipyridin-1'(2'H)-carboxylat

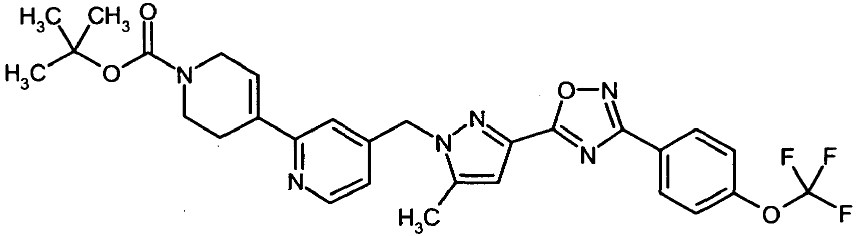

Eine Lösung von 480 mg (1.00 mmol) der Verbindung aus Beispiel 139A in 7.5 ml DME wurde mit 464 mg (1.50 mmol) *tert*.-Butyl-4-(4,4,5,5-tetramethyl-1,3,2-dioxaborolan-2-yl)-3,6-dihydropyridin-1(2*H*)-carboxylat [P.R. Eastwood, Tetrahedron Lett. 2000, 41 (19), 3705-3708], 70 mg (0.10 mmol) Bis(triphenylphosphin)palladium(II)dichlorid sowie 1.5 ml (3.0 mmol) 2 M wässrige Natriumcarbonat-Lösung versetzt. Nachdem das Reaktionsgemisch 13 h unter Rückfluss erhitzt worden war, ließ man auf RT erkalten und verdünnte mit ca. 50 ml Wasser. Es wurde dreimal mit je ca. 20 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt und der erhaltene Rückstand mittels Saugfiltration über Kieselgel gereinigt (Laufmittel: Dichlormethan/Ethylacetat 100:1 → 1:1). Es wurden 318 mg (54% d. Th.) der Titelverbindung erhalten.

¹H-NMR (400 MHz, CDCl₃, δ/ppm): 8.52 (d, 1H), 8.25 (d, 2H), 7.33 (d, 2H), 7.09 (s, 1H), 6.88 (d, 1H), 6.87 (s, 1H), 6.58-6.55 (m, 1H), 5.45 (s, 2H), 4.13-4.10 (m, 2H), 3.63-3.60 (m, 1H), 2.61-2.56 (m, 2H), 2.29 (s, 3H), 1.47 (s, 9H).

LC/MS (Methode D, ESIpos): Rₜ = 2.88 min, m/z = 583 [M+H]⁺.

### Beispiel 147A

### 3-[(5-Methyl-3-{3-[4-(tetrahydro-2H-pyran-4-yl)phenyl]-1,2,4-oxadiazol-5-yl}-1H-pyrazol-1-yl)-methyl]benzoesäure

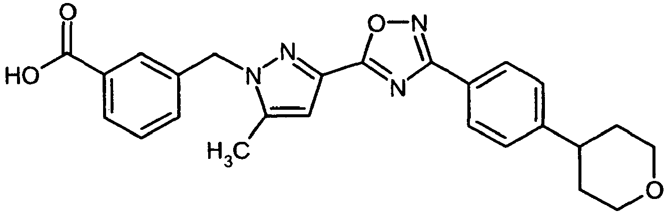

### Schritt 1: Methyl-3-[(5-methyl-3-{3-[4-(tetrahydro-2H-pyran-4-yl)phenyl]-1,2,4-oxadiazol-5-yl}-1H-pyrazol-1-yl)methyl]benzoat

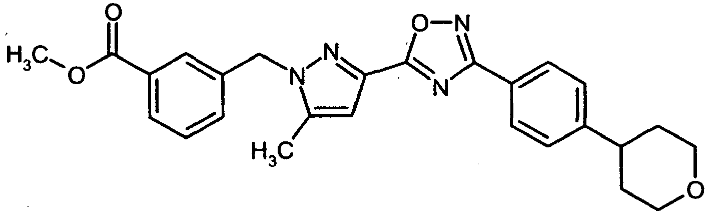

Analog zu dem unter Beispiel 76A beschriebenen Verfahren wurden 168 mg (0.733 mmol) 3-(Brommethyl)benzoesäuremethylester und 175 mg (0.564 mmol) der Verbindung aus Beispiel 124A zu 208 mg (80% d. Th.) der Titelverbindung umgesetzt.

¹H-NMR (400 MHz, CDCl₃, δ/ppm): 8.15 (d, 2H), 7.98 (d, 1H), 7.39 (s, 1H), 7.42 (t, 2H), 7.35 (d, 2H), 7.34 (d, 1H), 6.82 (s, 1H), 5.50 (s, 2H), 4.12-4.08 (m, 2H), 3.91 (s, 3H), 3.55 (dt, 2H), 2.87-2.80 (m, 1H), 2.29 (s, 3H), 1.92-1.77 (m, 4H).

LC/MS (Methode I, ESIpos): Rₜ = 1.24 min, m/z = 459 [M+H]⁺.

### Schritt 2: 3-[(5-Methyl-3-{3-[4-(tetrahydro-2H-pyran-4-yl)phenyl]-1,2,4-oxadiazol-5-yl}-1H-pyrazol-1-yl)methyl]benzoesäure

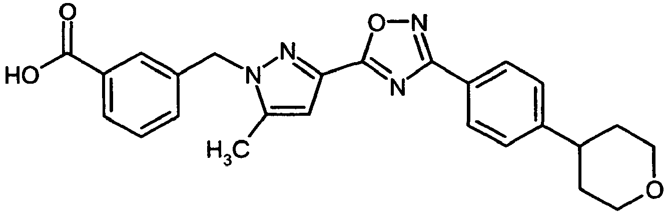

Analog zu dem unter Beispiel 93A beschriebenen Verfahren wurden aus 100 mg (0.218 mmol) der Verbindung aus Beispiel 147A / Schritt 1 85 mg (87% d. Th.) der Titelverbindung erhalten.

¹H-NMR (400 MHz, DMSO-d₆, δ/ppm): 13.07 (breit, 1H), 8.01 (d, 2H), 7.89 (d, 1H), 7.78 (s, 1H), 7.53-7.44 (m, 4H), 6.93 (s, 1H), 5.59 (s, 2H), 3.99-3.94 (m, 2H), 3.47 (dt, 2H), 2.92-2.83 (m, 1H), 2.34 (s, 3H), 1.77-1.66 (m, 4H).

LC/MS (Methode F, ESIpos): Rₜ = 1.22 min, m/z = 445 [M+H]⁺.

### Beispiel 148A

### N'-Hydroxy-5-methyl-1-{3-[(4-methylpiperazin-1-yl)carbonyl]benzyl}-1H-pyrazol-3-carboximid-amid

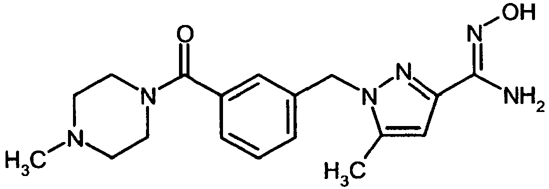

### Schritt 1: 5-Methyl-1-{3-[(4-methylpiperazin-1-yl)carbonyl]benzyl}-1H-pyrazol-3-carboxamid

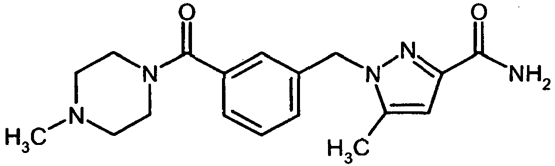

Zu einem Gemisch aus 27.0 g (78.9 mmol) der Verbindung aus Beispiel 105A und einigen Tropfen DMF in 100 ml Dichlormethan tropfte man langsam 34.4 ml (0.394 mol) Oxalylchlorid bei RT hinzu, rührte 1 h bei RT und engte dann ein. Der Rückstand wurde portionsweise in 300 ml einer auf 0°C gekühlten 33%-igen wässrigen Ammoniak-Lösung eingetragen. Man rührte 1 h bei RT, filtrierte dann den entstandenen Feststoff ab und erhielt nach Waschen mit Wasser und Trocknen im Vakuum eine erste Charge der Titelverbindung. Die Mutterlauge wurde zur Trockene eingeengt, der Rückstand mit Toluol versetzt und das Gemisch erneut zur Trockene eingeengt. Nach zweimaligem Wiederholen dieser Prozedur wurde der Rückstand mit Methanol verrührt, der Feststoff abfiltriert, mit Methanol gewaschen und im Vakuum getrocknet. Die so erhaltene zweite Charge der Titelverbindung wurde mit der ersten vereinigt. Man erhielt insgesamt 33.0 g (94% d. Th., Reinheit 77%) der Titelverbindung.

LC/MS (Methode D, ESIpos): Rₜ = 0.88 und 0.93 min, jeweils m/z = 342 [M+H]⁺.

### Schritt 2: 5-Methyl-1-{3-[(4-methylpiperazin-1-yl)carbonyl]benzyl}-1H-pyrazol-3-carbonitril

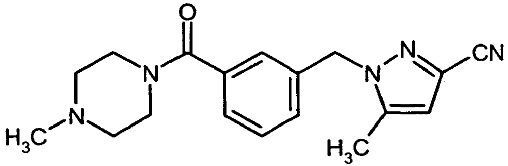

21.0 g (30.7 mmol, Reinheit 50%) der Verbindung aus Beispiel 148A / Schritt 1 wurden mit 10.9 ml (76.9 mmol) Trifluoressigsäureanhydrid versetzt und 1 h bei RT gerührt. Man versetzte anschließend mit gesättigter wässriger Natriumhydrogencarbonat-Lösung und extrahierte dreimal mit Ethylacetat. Die vereinigten Ethylacetat-Phasen wurden über Magnesiumsulfat getrocknet, filtriert und eingeengt. Der Rückstand wurde mittels Flash-Chromatographie gereinigt (Kieselgel, Laufmittel: Dichlormethan/Methanol 95:5). Man erhielt nach Einengen und Trocknen der Produktfraktionen 2.50 g (25% d. Th.) der Titelverbindung.

LC/MS (Methode D, ESIpos): Rₜ = 1.07 min, m/z = 324 [M+H]⁺.

### Schritt 3: N'-Hydroxy-5-methyl-1-{3-[(4-methylpiperazin-1-yl)carbonyl]benzyl}-1H-pyrazol-3-carboximidamid

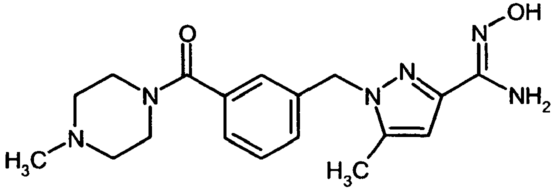

8.00 g (24.7 mmol) der Verbindung aus Beispiel 148A / Schritt 2 wurden in 320 ml Ethanol gelöst, mit 3.78 g (54.4 mmol) Hydroxylamin-Hydrochlorid sowie 7.6 ml (54.4 mmol) Triethylamin versetzt und 5 h unter Rühren zum Rückfluss erhitzt. Nach Abkühlen auf RT wurde das Gemisch eingeengt. Der Rückstand wurde mit einem Gemisch aus Dichlormethan und Methanol (8:1) verrührt und der entstandene Feststoff abgesaugt. Nach Trocknen wurde so eine erste Charge der Titelverbindung erhalten. Die Mutterlauge wurde eingeengt und der Rückstand mittels Flash-Chromatographie gereinigt (Kieselgel, Laufmittel: Dichlormethan/Methanol 8:2). Man erhielt nach Einengen und Trocknen der Produktfraktionen eine zweite Charge der Titelverbindung. Zusammen wurden 6.70 g (68% d. Th., Reinheit 90%) der Titelverbindung erhalten.

LC/MS (Methode I, ESIpos): Rₜ = 0.81 min, m/z = 357 [M+H]⁺.

### Ausführungsbeispiele:

### Beispiel 1

### (4-Methylpiperazin-1-yl){5-[(5-methyl-3-{3-[4-(trifluormethoxy)phenyl]-1,2,4-oxadiazol-5-yl}-1H-pyrazol-1-yl)methyl]pyridin-2-yl}methanon

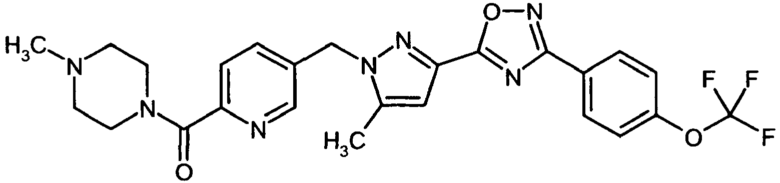

Unter inerten Bedingungen wurden 85 mg (0.191 mmol) der Verbindung aus Beispiel 92A in 3 ml wasserfreiem Dichlormethan gelöst und mit 83 µl (0.954 mmol) Oxalylchlorid und einem kleinen Tropfen DMF versetzt. Nachdem das Gemisch 1 h bei RT gerührt worden war, wurde es am Rotationsverdampfer zur Trockene eingeengt. Der erhaltene Rückstand wurde ca. 1 h im Hochvakuum getrocknet und dann in 2 ml wasserfreiem THF gelöst. Diese Lösung wurde zu einer Lösung von 29 mg (0.286 mmol) 1-Methylpiperazin und 66 µl (0.382 mmol) *N*,*N*-Diisopropylethylamin in 1 ml wasserfreiem THF hinzugetropft. Nach 16 h Rühren bei RT wurde das Reaktionsgemisch mit 3 ml Wasser versetzt und mittels präparativer HPLC (Methode N) in seine Komponenten aufgetrennt. Die Produktfraktionen wurden vereinigt und am Rotationsverdampfer vom Lösungsmittel befreit. Der Rückstand wurde in einigen ml Methanol wieder aufgelöst und über eine Hydrogencarbonat-Kartusche gegeben (Polymerlabs, Stratospheres SPE, PL-HCO₃ MP SPE, Kapazität 0.9 mmol). Nach neuerlichem Abdampfen des Lösungsmittels wurden so 93 mg (93% d. Th.) der Titelverbindung erhalten.

¹H-NMR (400 MHz, CDCl₃, δ/ppm): 8.45 (d, 1H), 8.24 (d, 2H), 7.64 (d, 1H), 7.60 (dd, 1H), 7.33 (d, 2H), 6.85 (s, 1H), 5.50 (s, 2H), 3.82 (dd, 2H), 3.60 (dd, 2H), 2.51 (dd, 2H), 2.40 (dd, 2H), 2.34 (s, 3H), 2.32 (s, 3H).

HPLC (Methode B): Rₜ = 4.32 min.

MS (ESIpos): m/z = 528 [M+H]⁺.

### Beispiel 2

### 1-Methyl-4-[({5-[(5-methyl-3-{3-[4-(trifluormethoxy)phenyl]-1,2,4-oxadiazol-5-yl}-1H-pyrazol-1-yl)methyl]pyridin-2-yl}amino)methyl]piperidin-4-ol

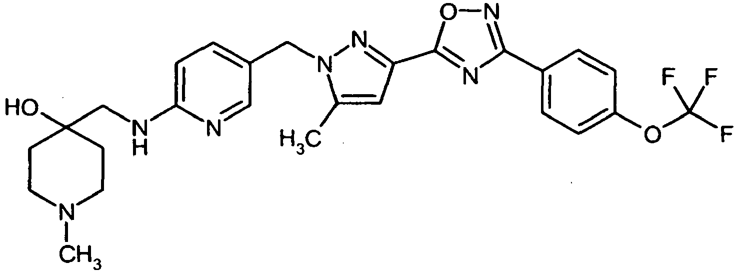

Ein Gemisch von 100 mg (0.229 mmol) der Verbindung aus Beispiel 79A und 249 mg (1.15 mmol) 4-(Aminomethyl)-1-methylpiperidin-4-ol wurde unter Rühren 3 h lang in einer Mikrowelle (CEM Discover, initiale Einstrahlleistung 250 W) auf 180°C erhitzt. Nach Abkühlen auf RT wurde vorhandene Flüssigkeit vom Feststoff abdekantiert. Der Feststoff wurde anschließend in einem Gemisch aus 2 ml Acetonitril und 1 ml Wasser gelöst und mittels präparativer HPLC (Methode O) gereinigt. Man engte die vereinigten produkthaltigen Fraktionen am Rotationsverdampfer bis auf ein Restvolumen ein, versetzte mit gesättigter wässriger Natriumhydrogencarbonat-Lösung und filtrierte den gebildeten Feststoff ab. Nach Trocknen im Vakuum wurden 15 mg (12% d. Th.) der Titelverbindung erhalten.

¹H-NMR (400 MHz, CDCl₃, δ/ppm): 8.26 (d, 2H), 7.92 (d, 1H), 7.37-7.32 (m, 3H), 6.77 (s, 1H), 6.43 (d, 1H), 5.36 (s, breit, 1H), 5.28 (s, 2H), 4.84 (t, 1H), 3.40 (d, 2H), 2.66-2.60 (m, 2H), 2.45-2.36 (m, 2H), 2.31 (s, 6H), 1.75-1.58 (m, 4H).

LC/MS (Methode F, ESIpos): Rₜ = 1.01 min, m/z = 544 [M+H]⁺.

### Beispiel 3

### (3R)-1-{5-[(5-Methyl-3-{3-[4-(trifluormethoxy)phenyl]-1,2,4-oxadiazol-5-yl}-1H-pyrazol-1-yl)-methyl]pyridin-2-yl pyrrolidin-3 -amin

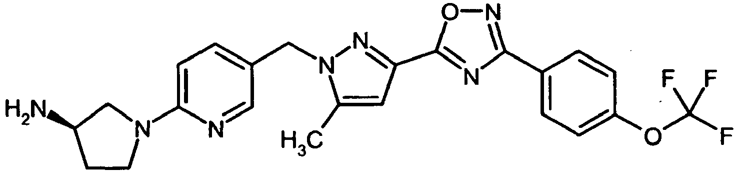

Man erhitzte ein Gemisch von 150 mg (0.344 mmol) der Verbindung aus Beispiel 79A und 641 mg (3.44 mmol) *tert*.-Butyl-(3*R*)-pyrrolidin-3-ylcarbamat in 1 ml Ethylenglykoldimethylether unter Rühren 3 h lang in einem Mikrowellengerät (CEM Discover, initiale Einstrahlleistung 250 W) auf 180°C. Nach Abkühlen auf RT wurde das Gemisch mit 3 ml Acetonitril und 1 ml Wasser verdünnt und anschließend mittels präparativer HPLC (Methode O) gereinigt. Man vereinigte die produkthaltigen Fraktionen, stellte mit Natriumhydrogencarbonat einen basischen pH-Wert ein und entfernte am Rotationsverdampfer einen Teil des Flüssigkeitsvolumens. Anschließend extrahierte man dreimal mit jeweils 30 ml Ethylacetat, trocknete die vereinigten Ethylacetat-Phasen über Natriumsulfat, filtrierte und entfernte das Lösungsmittel am Rotationsverdampfer. Der Rückstand wurde erneut mittels präparativer HPLC gereinigt (Abwandlung von Methode O: statt 0.1 % wässriger TFA wurde 0.1 % wässrige Ammoniak-Lösung verwendet). Es wurden 49 mg (29% d. Th.) der Titelverbindung erhalten.

¹H-NMR (400 MHz, DMSO-d₆, δ/ppm): 8.20 (d, 2H), 8.07 (d, 1H), 7.60 (d, 2H), 7.40 (dd, 1H), 6.88 (s, 1H), 6.38 (d, 1H), 5.32 (s, 2H), 3.56-3.42 (m, 3H), 3.38-3.28 (m, 1H), 3.02 (dd, 1H), 2.38 (s, 3H), 2.06-1.96 (m, 1H), 1.81-1.65 (m, 3H).

LC/MS (Methode D, ESIpos): Rₜ = 1.69 min, m/z = 486 [M+H]⁺.

Die Verbindungen in der folgenden Tabelle wurden analog zu einem der unter Beispiel 2 und 3 beschriebenen Verfahren aus der Verbindung aus Beispiel 79A und der entsprechenden AminKomponente hergestellt:

| **Beispiel** | **Struktur** | **HPLC: Rₜ [min]** | **MS: m/z [M+H]⁺** | **LC/MS-Methode** |
|---|---|---|---|---|
| **4** | 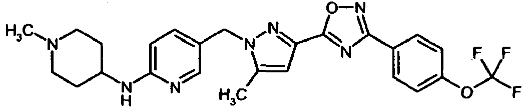 | 1.51 | 514 | C |
| | ¹H-NMR (400 MHz, DMSO-d₆, δ/ppm): 8.20 (d, 2H), 7.97 (d, 1H), 7.60 (d, 2H), 7.31-7.27 (dd, 1H), 6.88 (s, 1H), 6.52 (d, 1H), 6.42 (d, 1H), 5.27 (s, 2H), 3.68-3.57 (m, 1H), 2.73-2.66 (d, 2H), 2.39 (s, 3H), 2.13 (s, 3H), 2.00-1.92 (t, 2H), 1.87-1.79 (m, 2H), 1.42-1.33 (m, 2H). | | | |
| **5** | 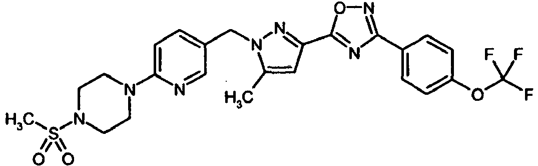 | 2.23 | 564 | E |
| | ¹H-NMR (400 MHz, CDCl₃, δ/ppm): 8.25 (d, 2H), 8.12 (s, 1H), 7.44 (d, 1H), 7.33 (d, 2H), 6.79 (s, 1H), 6.63 (d, 1H), 5.32 (s, 2H), 3.71-3.66 (m, 4H), 3.32-3.28 (m, 4H), 2.80 (s, 3H), 2.32 (s, 3H). | | | |
| **6** | 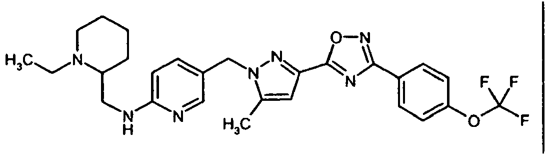 | 0.98 | 542 | K |
| **7** | 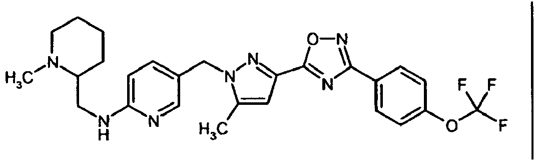 | 0.82 | 528 | K |
| **8** | 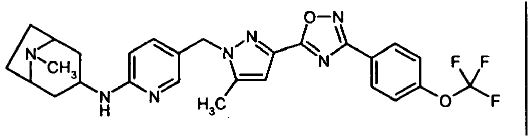 | 0.86 | 540 | K |
| **9** | 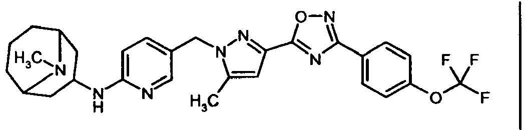 | 0.89 | 554 | K |
| **10** | 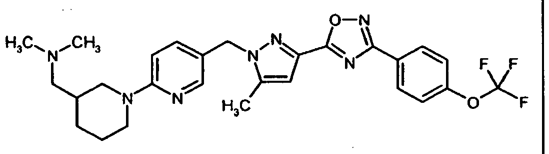 | 0.94 | 542 | K |
| **11** | 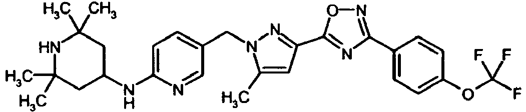 | 0.89 | 556 | K |
| **12** | 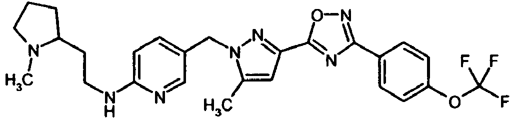 | 0.82 | 529 | K |
| **13** | 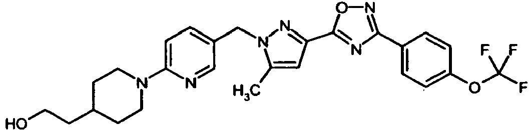 | 1.02 | 529 | K |
| **14** | 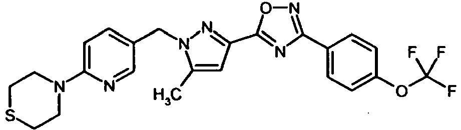 | 1.26 | 503 | K |

### Beispiel 15

### 1-Methyl-4-{4-[(5-methyl-3-{3-[4-(trifluormethoxy)phenyl]-1,2,4-oxadiazol-5-yl}-1H-pyrazol-1-yl)methyl]pyridin-2-yl}piperazin

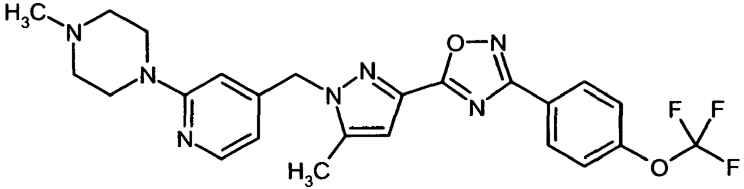

Ein Gemisch aus 150 mg (0.344 mmol) der Verbindung aus Beispiel 81A und 690 mg (6.88 mmol) 1-Methylpiperazin in 1 ml Ethylenglykoldimethylether wurde unter Rühren 3 h lang in einem Mikrowellengerät (CEM Discover, initiale Einstrahlleistung 250 W) auf 180°C erhitzt. Nach dem Abkühlen auf RT wurde das Gemisch direkt mittels präparativer HPLC (Methode O) gereinigt. Die vereinigten produkthaltigen Fraktionen wurden am Rotationsverdampfer bis auf ein kleines Rest-Flüssigkeitsvolumen aufkonzentriert, mit Natriumhydrogencarbonat auf einen leicht basischen pH-Wert eingestellt und anschließend dreimal mit jeweils 30 ml Dichlormethan extrahiert. Die vereinigten Dichlormethan-Phasen wurden über Magnesiumsulfat getrocknet, filtriert und das Lösungsmittel am Rotationsverdampfer entfernt. Nach Kristallisation aus Hexan wurden 114 mg (66% d. Th.) der Titelverbindung erhalten.

¹H-NMR (400 MHz, CDCl₃, δ/ppm): 8.27 (d, 2H), 8.12 (d, 1H), 7.33 (d, 2H), 6.84 (s, 1H), 6.36-6.32 (m, 2H), 5.36 (s, 2H), 3.55-3.48 (m, 4H), 2.55-2.45 (m, 4H), 2.31 (s, 3H), 2.29 (s, 3H).

LC/MS (Methode F, ESIpos): Rₜ = 0.97 min, m/z = 500 [M+H]⁺.

### Beispiel 16

### N,N-Dimethyl-1-{4-[(5-methyl-3-{3-[4-(trifluormethoxy)phenyl]-1,2,4-oxadiazol-5-yl}-1H-pyrazol-1-yl)methyl]pyridin-2-yl}piperidin-4-amin

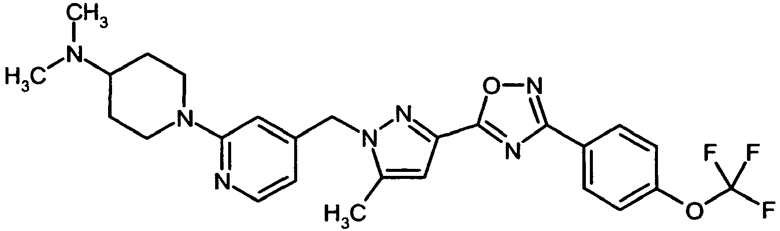

200 mg (0.459 mmol) der Verbindung aus Beispiel 81A wurden unter Argon bei RT mit 294 mg (2.29 mmol) *N,N*-Dimethylpiperidin-4-amin versetzt. Das Gemisch wurde anschließend über Nacht bei 150°C Badtemperatur gerührt. Nach dem Abkühlen auf RT wurde das Gemisch in Acetonitril aufgenommen und direkt mittels präparativer HPLC (Methode O) gereinigt. Die vereinigten produkthaltigen Fraktionen wurden mit gesättigter Natriumhydrogencarbonat-Lösung basisch gestellt und am Rotationsverdampfer bis auf ein geringes Restvolumen an Lösungsmittel eingeengt. Der dabei gebildete Feststoff wurde abfiltriert, zweimal mit Wasser und zweimal mit Pentan gewaschen und im Vakuum getrocknet. Man erhielt 137 mg (57% d. Th.) der Titelverbindung.

¹H-NMR (400 MHz, CDCl₃, δ/ppm): 8.26 (d, 2H), 8.12 (d, 1H), 7.32 (d, 2H), 6.82 (s, 1H), 6.32 (d, 1H), 6.31 (s, 1H), 5.35 (s, 2H), 3.51 (t, 4H), 2.76-2.67 (m, 1H), 2.62 (t, 4H), 2.30 (s, 3H), 1.08 (d, 6H).

LC/MS (Methode F, ESIpos): Rₜ = 1.12 min, m/z = 528 [M+H]⁺.

Die Verbindungen in der folgenden Tabelle wurden analog zu einem der unter Beispiel 15 und 16 beschriebenen Verfahren aus der Verbindung aus Beispiel 81A und der entsprechenden AminKomponente hergestellt:

| **Beispiel** | **Struktur** | **HPLC: Rₜ [min]** | **MS: m/z [M+H]⁺** | **LC/MS-Methode** |
|---|---|---|---|---|
| **17** | 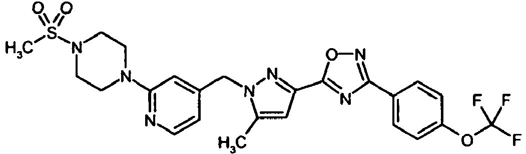 | 2.14 | 564 | J |
| **18** | 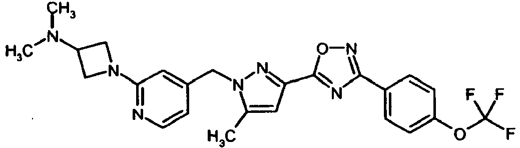 | 0.95 | 500 | I |
| | ¹H-NMR (400 MHz, CDCl₃, δ/ppm): 8.25 (d, 2H), 8.18 (d, 1H), 7.32 (d, 2H), 6.82 (s, 1H), 6.32 (d, 1H), 5.95 (s, 1H), 5.32 (s, 2H), 4.06-4.02 (m, 2H), 3.83-3.80 (m, 2H), 3.25 (m, 1H), 2.26 (s, 3H), 2.22 (s, 6H). | | | |
| **19** | 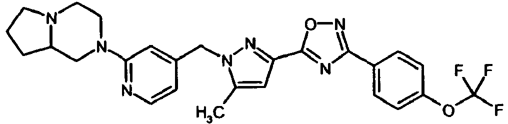 | 1.67 | 526 | J |
| **20** | 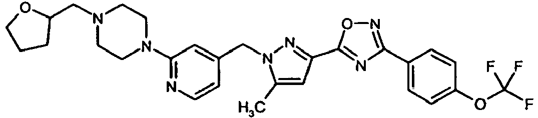 | 1.71 | 570 | J |
| **21** | 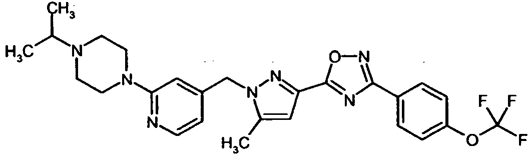 | 0.96 | 528 | I |
| | ¹H-NMR (400 MHz, CDCl₃, δ/ppm): 8.25 (d, 2H), 8.12 (d, 1H), 7.33 (d, 2H), 6.83 (s, 1H), 6.34-6.31 (m, 2H), 5.35 (s, 2H), 3.51 (t, 4H), 2.73-2.66 (m, 1H), 2.61 (t, 4H), 2.28 (s, 3H), 1.06 (d, 6H). | | | |
| **22** | 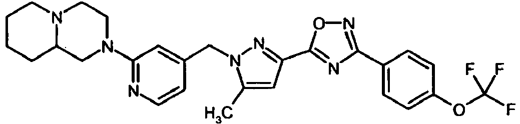 | 1.71 | 540 | J |
| **23** | 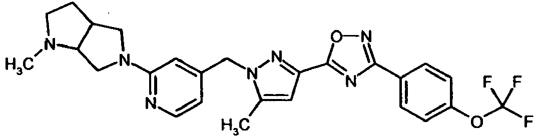 | 0.94 | 526 | I |
| | ¹H-NMR (400 MHz, CDCl₃, δ/ppm): 8.25 (d, 2H), 8.09 (d, 1H), 7.32 (d, 2H), 6.82 (s, 1H), 6.31 (d, 1H), 6.10 (s, 1H), 5.33 (s, 2H), 3.89-3.82 (m, 1H), 3.55 (t, 1H), 3.44-3.33 (m, 3H), 3.33-3.20 (m, breit, 1H), 3.07-2.98 (m, breit, 1H), 2.55 (s, 3H), 2.29 (s, 3H), 2.29-2.19 (m, breit, 1H), 1.92-1.81 (m, breit, 2H). | | | |
| **24** | 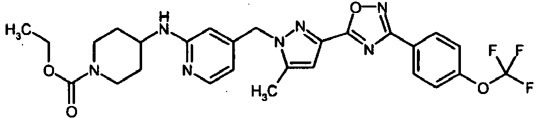 | 1.77 | 572 | J |
| **25** | 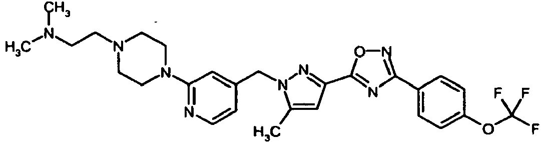 | 0.90 | 557 | F |
| | ¹H-NMR (400 MHz, CDCl₃, δ/ppm): 8.26 (d, 2H), 8.12 (d, 1H), 7.33 (d, 2H), 6.83 (s, 1H), 6.35-6.30 (m, 2H), 5.36 (s, 2H), 3.55-3.49 (m, 4H), 2.60-2.55 (m, 4H), 2.55-2.42 (m, 4H), 2.29 (s, 3H), 2.26 (s, 3H). | | | |
| **26** | 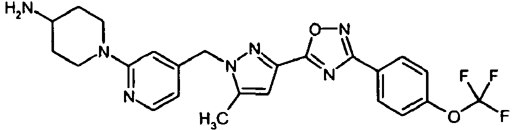 | 0.93 | 500 | I |
| | ¹H-NMR (400 MHz, CDCl₃, δ/ppm): 8.23 (d, 2H), 8.11 (d, 1H), 7.32 (d, 2H), 6.82 (s, 1H), 6.32 (s, 1H), 6.31-6.29 (m, 1H), 5.32 (s, 2H), 4.17 (m, 2H), 2.92-2.84 (m, 3H), 2.27 (s, 3H), 1.90-1.82 (m, 1H), 1.75-1.65 (m, 1H), 1.38-1.28 (m, 2H). | | | |
| **27** | 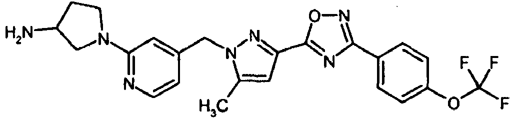 | 1.68 | 486 | D |
| | ¹H-NMR (400 MHz, CDCl₃, δ/ppm): 8.25 (d, 2H), 8.19 (d, 1H), 7.32 (d, 2H), 6.82 (s, 1H), 6.28 (d, 1H), 6.00 (s, 1H), 5.34 (s, 2H), 3.75-3.68 (m, 1H), 3.65-3.52 (m, 2H), 3.48-3.38 (m, 1H), 3.18 (dd, 1H), 2.29 (s, 3H), 2.23-2.15 (m, 1H), 1.83-1.68 (m, 1H). | | | |
| **28** | 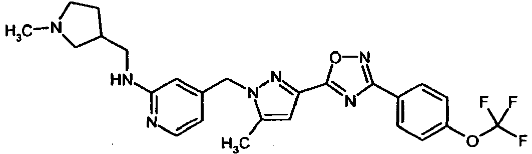 | 1.41 | 513 | J |
| **29** | 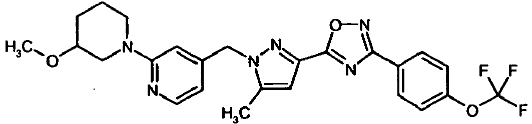 | 1.88 | 515 | J |
| **30** | 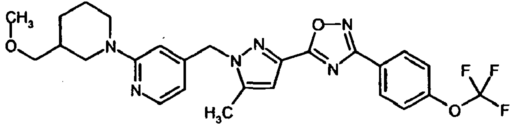 | 1.90 | 529 | J |
| **31** | 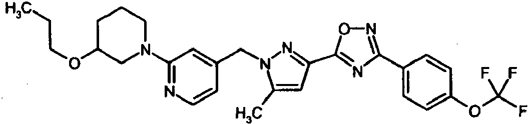 | 2.04 | 543 | J |

### Beispiel 32

### (4-Methylpiperazin-1-yl){3-[(5-methyl-3-{3-[4-(trifluormethoxy)phenyl]-1,2,4-oxadiazol-5-yl}-1H-pyrazol-1-yl)methyl]phenyl}methanon

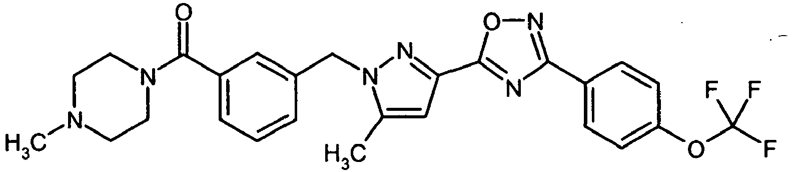

Unter inerten Bedingungen wurden 80 mg (0.180 mmol) der Verbindung aus Beispiel 93A in 2 ml wasserfreiem Dichlormethan gelöst und mit 79 µl (0.90 mmol) Oxalylchlorid und einem kleinen Tropfen DMF versetzt. Nachdem das Gemisch 1 h bei RT gerührt worden war, wurde es am Rotationsverdampfer zur Trockene eingeengt. Der erhaltene Rückstand wurde ca. 1 h im Hochvakuum getrocknet und dann in 1 ml wasserfreiem THF gelöst. Diese Lösung wurde zu einer Lösung von 36 mg (0.360 mmol) 1-Methylpiperazin und 94 µl (0.540 mmol) *N*,*N*-Diisopropylethylamin in 1 ml wasserfreiem THF hinzugetropft. Nach 16 h Rühren bei RT wurde das Reaktionsgemisch mit 3 ml Wasser versetzt und mittels präparativer HPLC (Methode N) in seine Komponenten aufgetrennt. Die Produktfraktionen wurden vereinigt und am Rotationsverdampfer vom Lösungsmittel befreit. Der Rückstand wurde in einigen ml Methanol wieder aufgelöst und über eine Hydrogencarbonat-Kartusche gegeben (Polymerlabs, Stratospheres SPE, PL-HCO₃ MP SPE, Kapazität 0.9 mmol). Nach neuerlichem Abdampfen des Lösungsmittels wurden 78 mg (82% d. Th.) der Titelverbindung erhalten.

¹H-NMR (400 MHz, CDCl₃, δ/ppm): 8.25 (d, 2H), 7.41-7.33 (m, 4H), 7.20 (d, 1H), 7.17 (s, 1H), 6.83 (s, 1H), 5.48 (s, 2H), 3.76 (breit, 2H), 3.37 (breit, 2H), 2.44 (breit, 2H), 2.30 (s, 3H), 2.29 (breit, 2H), 2.26 (s, 3H).

HPLC (Methode B): Rₜ = 4.45 min.

MS (DCI, NH₃): m/z = 527 [M+H]⁺.

LC/MS (Methode C, ESIpos): Rₜ = 1.71 min, m/z = 527 [M+H]⁺.

Die Verbindung in der folgenden Tabelle wurde analog zu dem unter Beispiel 32 beschriebenen Verfahren aus der Verbindung aus Beispiel 93A und dem entsprechenden Amin hergestellt. Diese Amine waren entweder kommerziell erhältlich, oder ihre Herstellung wurde weiter oben oder in der Literatur beschrieben: 1-Cyclopropylpiperazin [F. Zaragoza et al., J. Med. Chem. 2004, 47 (11), 2833-2838], 1-(2,2,2-Trifluorethyl)piperazin [H.-L. Wang et al., J. Med. Chem. 2007, 50 (15), 3528-3539]. Wurden die Amine in Form ihrer Hydrochloride bzw. Dihydrochloride eingesetzt, so wurde die Menge der verwendeten Base (*N*,*N*-Diisopropylethylamin) jeweils um ein entsprechendes Äquivalent erhöht.

| **Beispiel** | **Struktur** | **HPLC: Rₜ [min]** | **MS: m/z [M+H]⁺** | **LC/MS-Methode** |
|---|---|---|---|---|
| **33** | 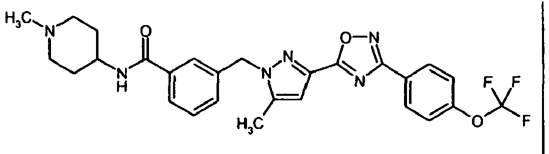 | 1.75 | 541 | C |
| | ¹H-NMR (400 MHz, CDCl₃, δ/ppm): 8.25 (d, 2H), 7.65 (s, 1H), 7.63 (d, 1H), 7.40 (t, 1H), 7.33 (d, 2H), 7.24 (d, 1H), 6.82 (s, 1H), 5.94 (d, breit, 1H), 5.50 (s, 2H), 4.01-3.92 (m, 1H), 2.83-2.77 (m, 2H), 2.29 (s, 3H), 2.28 (s, 3H), 2.17-2.10 (m, 2H), 2.05-2.00 (m, 2H), 1.60-1.50 (m, 2H). | | | |
| **34** | 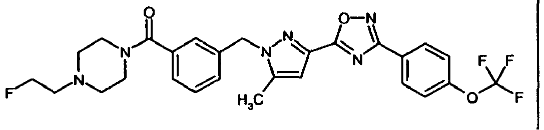 | 0.97 | 559 | I |
| | ¹H-NMR (400 MHz, CDCl₃, δ/ppm): 8.25 (d, 2H), 7.41-7.33 (m, 4H), 7.21 (dd, 1H), 7.16 (d, 1H), 6.83 (s, 1H), 5.48 (s, 2H), 4.53 (td, 2H), 3.78 (breit, 2H), 3.38 (breit, 2H), 2.67 (td, 2H), 2.58 (breit, 2H), 2.43 (breit, 2H), 2.31 (s, 3H). | | | |
| **35** | 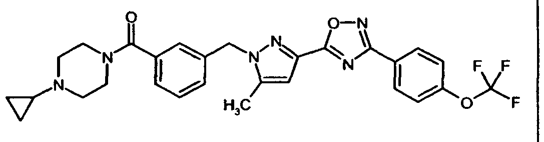 | 1.03 | 553 | I |
| | ¹H-NMR (400 MHz, CDCl₃, δ/ppm): 8.25 (d, 2H), 7.42-7.32 (m, 4H), 7.20 (dd, 1H), 7.15 (d, 1H), 6.83 (s, 1H), 5.48 (s, 2H), 3.70 (breit, 2H), 3.29 (breit, 2H), 2.65 (breit, 2H), 2.47 (breit, 2H), 2.31 (s, 3H), 1.60-1.54 (m, 1H), 0.44-0.34 (m, 4H). | | | |
| **36** | 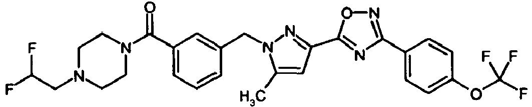 | 1.23 | 577 | I |
| | ¹H-NMR (400 MHz, CDCl₃, δ/ppm): 8.25 (d, 2H), 7.42-7.33 (m, 4H), 7.21 (dd, 1H), 7.16 (d, 1H), 6.83 (s, 1H), 5.84 (tt, 1H), 5.48 (s, 2H), 3.75 (breit, 2H), 3.37 (breit, 2H), 2.72 (dt, 2H), 2.62 (breit, 2H), 2.48 (breit, 2H), 2.31 (s, 3H). | | | |
| **37** | 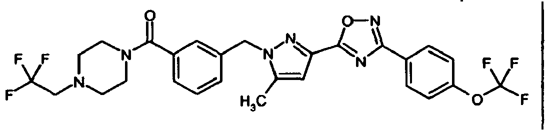 | 1.33 | 595 | I |
| | ¹H-NMR (400 MHz, CDCl₃, δ/ppm): 8.24 (d, 2H), 7.42-7.33 (m, 4H), 7.21 (dd, 1H), 7.16 (d, 1H), 6.83 (s, 1H), 5.48 (s, 2H), 3.76 (breit, 2H), 3.37 (breit, 2H), 2.96 (quart, 2H), 2.72 (breit, 2H), 2.55 (breit, 2H), 2.31 (s, 3H). | | | |

### Beispiel 38

### {3-[(5-Methyl-3-{3-[4-(trifluormethoxy)phenyl]-1,2,4-oxadiazol-5-yl}-1H-pyrazol-1-yl)methyl]-phenyl} (piperazin-1-yl)methanon

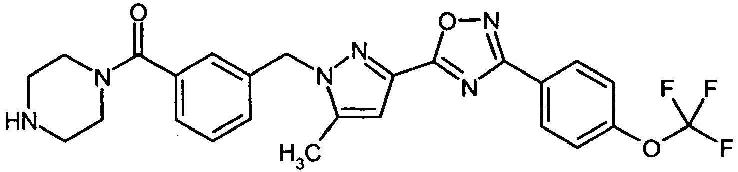

### Schritt 1: tert.-Butyl-4-({3-[(5-methyl-3-{3-[4-(trifluormethoxy)phenyl]-1,2,4-oxadiazol-5-yl}-1H-pyrazol-1-yl)methyl]phenyl}carbonyl)piperazin-1-carboxylat

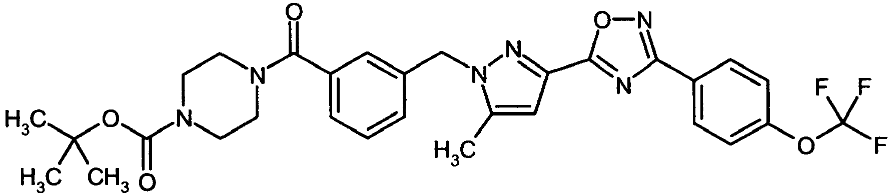

Analog zu dem unter Beispiel 32 beschriebenen Verfahren wurden aus 80 mg (0.180 mmol) der Verbindung aus Beispiel 93A und 67 mg (0.360 mmol) *tert*.-Butyl-piperazin-1-carboxylat 110 mg (96% d. Th.) der Titelverbindung erhalten. Abweichend von dem unter Beispiel 32 beschriebenen Verfahren wurde hier auf die Perkolation über eine Natriumhydrogencarbonat-Kartusche (nach der HPLC-Reinigung) verzichtet.

¹H-NMR (400 MHz, CDCl₃, δ/ppm): 8.25 (d, 2H), 7.40 (t, 1H), 7.33 (2 d, zus. 3H), 7.22 (d, 1H), 7.20 (s, 1H), 6.83 (s, 1H), 5.48 (s, 2H), 3.70 (breit, 2H), 3.49 (breit, 2H), 3.35 (breit, 4H), 2.31 (s, 3H), 1.13 (s, 9H).

HPLC (Methode B): Rₜ = 5.22 min.

MS (DCI, NH₃): m/z = 613 [M+H]⁺.

LC/MS (Methode C, ESIpos): Rₜ = 2.98 min, m/z = 613 [M+H]⁺.

### Schritt 2: {3-[(5-Methyl-3-{3-[4-(trifluormethoxy)phenyl]-1,2,4-oxadiazol-5-yl}-1H-pyrazol-1-yl)methyl]phenyl}(piperazin-1-yl)methanon

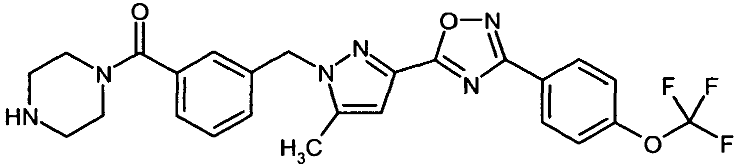

70 mg (0.114 mmol) der Verbindung aus Beispiel 38 / Schritt 1 wurden bei RT mit 5 ml einer 4 M Lösung von Chlorwasserstoff in 1,4-Dioxan versetzt und 15 h bei RT gerührt. Anschließend wurde durch Zugabe von 10 ml Diethylether die Fällung des Produktes vervollständigt. Der Feststoff wurde abgesaugt, mit wenig kaltem Diethylether gewaschen und im Hochvakuum getrocknet. Dann wurde der Feststoff in wenigen ml Methanol gelöst und über eine Hydrogencarbonat-Kartusche gegeben (Polymerlabs, Stratospheres SPE, PL-HCO₃ MP SPE, Kapazität 0.9 mmol), um die Base freizusetzen. Nach erneutem Abdampfen des Lösungsmittels und Trocknen im Hochvakuum wurden 40 mg (68% d. Th.) der Titelverbindung erhalten.

¹H-NMR (400 MHz, CDCl₃, δ/ppm): 8.24 (d, 2H), 7.41-7.33 (m, 4H), 7.20 (d, 1H), 7.19 (s, 1H), 6.83 (s, 1H), 5.48 (s, 2H), 3.73 (breit, 2H), 3.33 (breit, 2H), 2.92 (breit, 2H), 2.77 (breit, 2H), 2.30 (s, 3H).

HPLC (Methode B): Rₜ = 4.40 min.

LC/MS (Methode C, ESIpos): Rₜ = 1.68 min, m/z = 513 [M+H]⁺.

Die Verbindungen in der folgenden Tabelle wurden analog zu dem unter Beispiel 38 beschriebenen, zweistufigen Verfahren aus der Verbindung aus Beispiel 93A und den entsprechenden mono-*tert*.-Butoxycarbonyl-geschützten Diamin-Komponenten hergestellt:

| **Beispiel** | **Struktur** | **HPLC: Rₜ [min]** | **MS: m/z [M+H]⁺** | **LC/MS-Methode** |
|---|---|---|---|---|
| **39** | 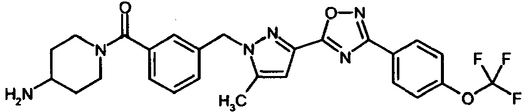 | 1.07 | 527 | F |
| | ¹H-NMR (400 MHz, CDCl₃, δ/ppm): 8.24 (d, 2H), 7.40-7.32 (m, 4H), 7.19 (d, 1H), 7.18 (s, 1H), 6.82 (s, 1H), 5.47 (s, 2H), 4.60-4.50 (m, 1H), 3.68-3.60 (m, 1H), 3.03-2.85 (m, 3H), 2.30 (s, 3H), 1.91 (breit, 1H), 1.73 (breit, 1H), 1.35 (breit, 1H), 1.20 (breit, 1H). | | | |
| **40** | 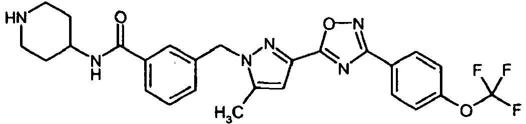 | 1.73 | 527 | C |
| | ¹H-NMR (400 MHz, CDCl₃, δ/ppm): 8.25 (d, 2H), 7.67 (s, 1H), 7.65 (d, 1H), 7.41 (t, 1H), 7.33 (d, 2H), 7.27 (d, 1H), 6.83 (s, 1H), 6.01 (d, breit, 1H), 5.49 (s, 2H), 4.10-4.01 (m, 1H), 3.22-3.06 (m, 2H), 2.28-2.70 (m, 2H), 2.30 (s, 3H), 2.05-1.99 (m, 2H), 1.46-1.37 (m, 2H). | | | |

### Beispiel 41

### 4-Methylpiperazin-1-yl)(3-{[5-methyl-3-(3-{4-[(trifluormethyl)sulfonyl]phenyl}-1,2,4-oxadiazol-5-yl)-1H-pyrazol-1-yl]methyl}phenyl)methanon

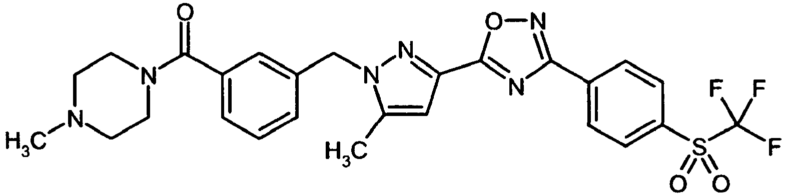

Unter inerten Bedingungen wurde bei 0°C eine Lösung von 100 mg (0.292 mmol) der Verbindung aus Beispiel 105A in 3 ml wasserfreiem Dichlormethan mit 76 µl (0.876 mmol) Oxalylchlorid versetzt. Das Reaktionsgemisch wurde 2 h bei RT gerührt. Anschließend wurden alle flüchtigen Bestandteile am Rotationsverdampfer entfernt und der so erhaltene Rückstand 20 min im Hochvakuum getrocknet. Der Rückstand wurde danach erneut in 2 ml wasserfreiem Dichlormethan gelöst, und diese Lösung wurde bei 0°C zu einer Lösung von 94 mg (0.350 mmol) der Verbindung aus Beispiel 3A und 81 µl (0.584 mmol) Triethylamin in 1 ml Dichlormethan getropft. Nachdem das Reaktionsgemisch 16 h bei RT gerührt worden war, wurde wiederum alles Flüchtige am Rotationsverdampfer entfernt und der Rückstand in 4 ml DMSO gelöst. Diese Lösung wurde dann in einem Mikrowellenofen 30 min lang auf 120°C erhitzt (CEM Discover, initiale Einstrahlleistung 250 W). Nach dem Abkühlen auf RT wurde das Reaktionsgemisch direkt mittels präparativer HPLC (Methode N) aufgereinigt. Die Produktfraktion wurde am Rotationsverdampfer zur Trockene eingedampft. Der Rückstand wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol). Nach neuerlichem Abdampfen des Lösungsmittels wurden so 47 mg (28% d. Th.) der Titelverbindung erhalten.

¹H-NMR (400 MHz, CDCl₃, δ/ppm): 8.52 (d, 2H), 8.18 (d, 2H), 7.40 (t, 1H), 7.34 (d, 1H), 7.21 (d, 1H), 7.18 (s, 1H), 6.85 (s, 1H), 5.49 (s, 2H), 3.76 (breit, 2H), 3.37 (breit, 2H), 2.45 (breit, 2H), 2.31 (s, 3H), 2.29 (breit, 2H), 2.27 (s, 3H).

HPLC (Methode A): Rₜ = 4.28 min.

MS (DCI, NH₃): m/z = 575 [M+H]⁺.

Analog zu dem unter Beispiel 41 beschriebenen Verfahren wurden die Verbindungen in der folgenden Tabelle aus der Verbindung aus Beispiel 105A oder der Verbindung aus Beispiel 106A und den entsprechenden *N*'-Hydroxycarboximidamiden (Hydroxyamidinen) hergestellt:

| **Beispiel** | **Struktur** | **HPLC: Rₜ [min]** | **MS: m/z [M+H]⁺** | **LC/MS-Methode** |
|---|---|---|---|---|
| **42** | 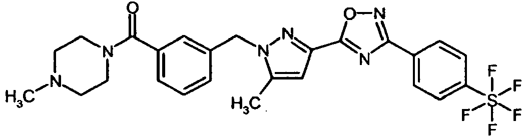 | 4.39 | 569 | A |
| | ¹H-NMR (400 MHz, CDCl₃, δ/ppm): 8.31 (d, 2H), 7.89 (d, 2H), 7.39 (t, 1H), 7.34 (d, 1H), 7.20 (d, 1H), 7.17 (s, 1H), 6.85 (s, 1H), 5.48 (s, 2H), 3.76 (breit, 2H), 3.37 (breit, 2H), 2.45 (breit, 2H), 2.31 (s, 3H), 2.28 (breit, 2H), 2.26 (s, 3H). | | | |
| **43** | 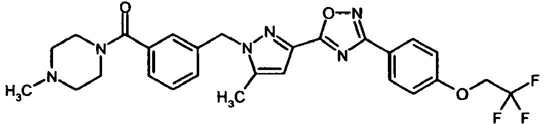 | 0.93 | 541 | I |
| | ¹H-NMR (400 MHz, CDCl₃, δ/ppm): 8.18 (d, 2H), 7.39 (t, 1H), 7.34 (d, 1H), 7.20 (d, 1H), 7.16 (s, 1H), 7.05 (d, 2H), 6.82 (s, 1H), 5.47 (s, 2H), 4.42 (quart, 2H), 3.76 (breit, 2H), 3.36 (breit, 2H), 2.45 (breit, 2H), 2.30 (s, 3H), 2.27 (breit, 2H), 2.26 (s, 3H). | | | |
| **44** | 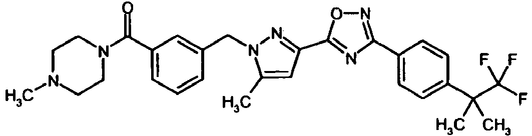 | 4.42 | 553 | A |
| | ¹H-NMR (400 MHz, CDCl₃, δ/ppm): 8.19 (d, 2H), 7.62 (d, 2H), 7.39 (t, 1H), 7.34 (d, 1H), 7.20 (d, 1H), 7.16 (s, 1H), 6.83 (s, 1H), 5.48 (s, 2H), 3.76 (breit, 2H), 3.36 (breit, 2H), 2.44 (breit, 2H), 2.30 (s, 3H), 2.29 (breit, 2H), 2.25 (s, 3H), 1.63 (s, 6H). | | | |
| **45** | 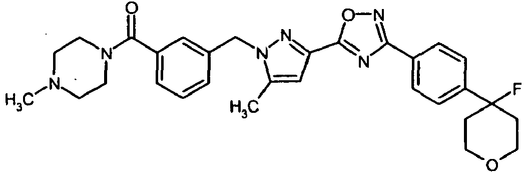 | 0.98 | 545 | F |
| | ¹H-NMR (400 MHz, CDCl₃, δ/ppm): 8.22 (d, 2H), 7.52 (d, 2H), 7.39 (t, 1H), 7.34 (d, 1H), 7.20 (d, 1H), 7.16 (s, 1H), 6.84 (s, 1H), 5.48 (s, 2H), 4.00-3.87 (m, 4H), 3.75 (breit, 2H), 3.36 (breit, 2H), 2.45 (breit, 2H), 2.30 (s, 3H), 2.28 (breit, 2H), 2.27-2.12 (m, 2H), 2.26 (s, 3H), 1.98-1.92 (m, 2H). | | | |
| **46** | 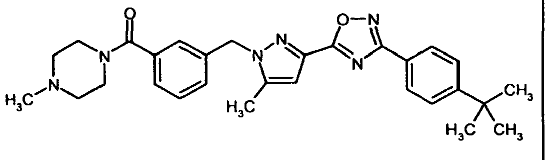 | 1.01 | 499 | I |
| | ¹H-NMR (400 MHz, CDCl₃, δ/ppm): 8.12 (d, 2H), 7.51 (d, 2H), 7.39 (t, 1H), 7.34 (d, 1H), 7.20 (d, 1H), 7.15 (s, 1H), 6.83 (s, 1H), 5.48 (s, 2H), 3.76 (breit, 2H), 3.36 (breit, 2H), 2.44 (breit, 2H), 2.30 (s, 3H), 2.28 (breit, 2H), 2.25 (s, 3H), 1.36 (s, 9H). | | | |
| **47** | 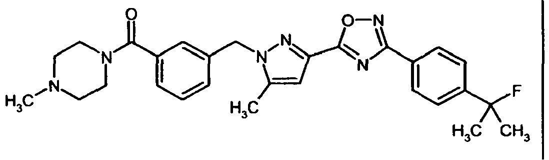 | 1.03 | 503 | F |
| | ¹H-NMR (400 MHz, CDCl₃, δ/ppm): 8.11 (d, 2H), 7.42 (d, 1H), 7.32-7.26 (m, 2H), 7.12 (d, 1H), 7.06 (s, 1H), 6.75 (s, 1H), 5.40 (s, 2H), 3.70 (breit, 2H), 3.30 (breit, 2H), 2.43-2.35 (breit, 2H), 2.30-2.18 (breit, 2H), 2.22 (s, 3H), 2.20 (s, 3H), 1.66 (s, 3H), 1.62 (s, 3H). | | | |
| **48** | 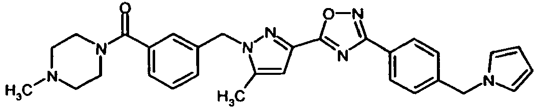 | 0.90 | 522 | I |
| | ¹H-NMR (400 MHz, CDCl₃, δ/ppm): 8.12 (d, 2H), 7.45-7.08 (m, 6H), 6.80 (s, breit, 1H), 6.71 (s, breit, 2H), 6.20 (s, breit, 2H), 5.47 (s, breit, 2H), 5.12 (s, breit, 2H), 3.75 (s, breit, 2H), 3.35 (s, breit, 2H), 2.42 (s, breit, 2H), 2.28 (s, 3H), 2.25 (s, breit, 2H), 2.25 (s, 3H). | | | |
| **49** | 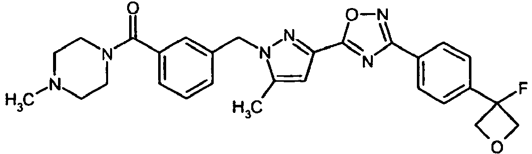 | 3.96 | 517 | A |
| | ¹H-NMR (400 MHz, CDCl₃, δ/ppm): 8.28 (d, 2H), 7.71 (d, 2H), 7.39 (t, 1H), 7.34 (d, 1H), 7.20 (d, 1H), 7.16 (s, 1H), 6.84 (s, 1H), 5.48 (s, 2H), 5.15 (dd, 2H), 4.90 (dd, 2H), 3.76 (breit, 2H), 3.36 (breit, 2H), 2.44 (breit, 2H), 2.30 (s, 3H), 2.29 (breit, 2H), 2.26 (s, 3H). | | | |
| **50** | 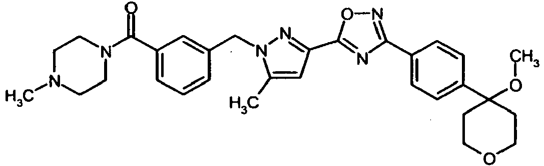 | 0.85 | 557 | I |
| | ¹H-NMR (400 MHz, CDCl₃, δ/ppm): 8.20 (d, 2H), 7.53 (d, 2H), 7.39 (t, 1H), 7.34 (d, 1H), 7.21 (d, 1H), 7.16 (s, 1H), 6.83 (s, 1H), 5.48 (s, 2H), 3.93-3.82 (m, 4H), 3.76 (breit, 2H), 3.37 (breit, 2H), 3.01 (s, 3H), 2.45 (breit, 2H), 2.30 (s, 3H), 2.28 (breit, 2H), 2.26 (s, 3H), 2.11-1.98 (m, 4H). | | | |
| **51** | 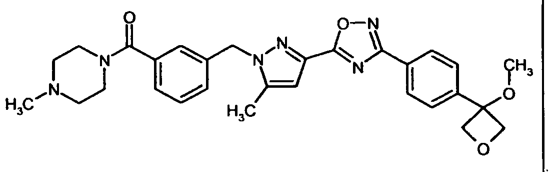 | 0.80 | 529 | I |
| | ¹H-NMR (400 MHz, DMSO-d₆, δ/ppm): 8.14 (d, 2H), 7.68 (d, 2H), 7.46 (t, 1H), 7.33 (d, 1H), 7.31 (d, 1H), 7.15 (s, 1H), 6.95 (s, 1H), 5.56 (s, 2H), 4.83 (d, 2H), 4.79 (d, 2H), 3.56 (breit, 2H), 3.25 (breit, 2H), 3.09 (s, 3H), 2.35 (s, 3H), 2.31 (breit, 2H), 2.18 (breit, 2H), 2.12 (s, 3H). | | | |
| **52** | 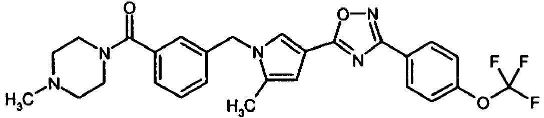 | 1.01 | 526 | I |
| | ¹H-NMR (400 MHz, CDCl₃, δ/ppm): 8.16 (d, 2H), 7.49 (d, 1H), 7.40 (t, 1H), 7.35-7.28 (m, 3H), 7.10 (d, 1H), 7.09 (s, 1H), 6.58 (d, 1H), 5.13 (s, 2H), 3.76 (breit, 2H), 3.37 (breit, 2H), 2.45 (breit, 2H), 2.28 (breit, 2H), 2.27 (s, 3H), 2.19 (s, 3H). | | | |

### Beispiel 53

### 1-Cyclopropyl-4-{5-[(5-methyl-3-{3-[4-(1,1,1-trifluor-2-methylpropan-2-yl)phenyl]-1,2,4-oxadiazol-5-yl}-1H-pyrazol-1-yl)methyl]pyridin-2-yl}piperazin

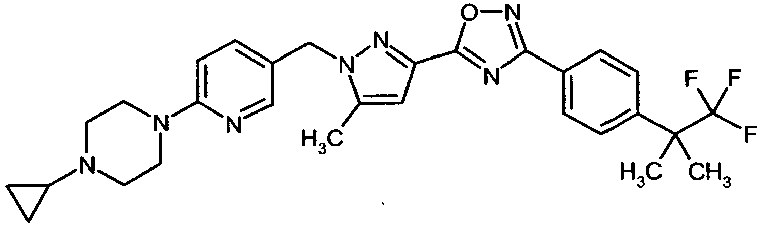

Eine Dispersion von 431 mg (2.17 mmol) 1-Cyclopropylpiperazin-Dihydrochlorid und 729 mg (8.68 mmol) Natriumhydrogencarbonat in ca. 50 ml Methanol wurde 2 h bei RT heftig gerührt. Dann wurde vom Ungelösten abfiltriert und das Filtrat zur Trockene eingedampft. Die Hälfte des so erhaltenen 1-Cyclopropylpiperazins wurde in 0.5 ml Ethylenglykoldimethylether gelöst, die andere Hälfte in 0.5 ml *N*,*N*-Dimethylacetamid. Die beiden Lösungen wurden jeweils mit 50 mg (0.108 mmol) der Verbindung aus Beispiel 78A versetzt und anschließend separat für 36 h auf 150°C erhitzt. Nach dieser Zeit war der Umsatz für beide Ansätze etwa gleich (LC/MS-Kontrolle). Die Reaktionsgemische wurden deshalb vereinigt, mit ca. 2 ml Acetonitril verdünnt und direkt mittels präparativer HPLC (Methode N) in ihre Komponenten aufgetrennt. Es wurden 31 mg (26% d. Th.) der Titelverbindung erhalten.

¹H-NMR (400 MHz, CDCl₃, δ/ppm): 8.19 (d, 2H), 8.10 (d, 1H), 7.61 (d, 2H), 7.41 (dd, 1H), 6.77 (s, 1H), 6.60 (d, 1H), 5.30 (s, 2H), 3.52-3.49 (m, 5H), 2.71-2.68 (m, 4H), 2.31 (s, 3H), 1.62 (s, 6H), 0.49-0.44 (m, 4H).

LC/MS (Methode F, ESIpos): Rₜ = 1.22 min, m/z = 552 [M+H]⁺.

### Beispiel 54

### 1-{5-[(5-Methyl-3-{3-[4-(trimethylsilyl)phenyl]-1,2,4-oxadiazol-5-yl}-1H-pyrazol-1-yl)methyl]-pyridin-2-yl}piperazin

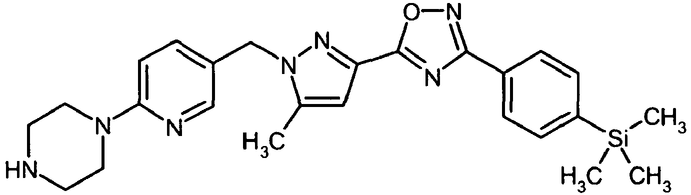

Ein Gemisch aus 200 mg (0.472 mmol) der Verbindung aus Beispiel 87A und 813 mg (9.43 mmol) Piperazin wurde 16 h unter Argon bei einer Temperatur von 150°C gerührt. Nach dem Abkühlen wurde mit ca. 50 ml Wasser versetzt und dreimal mit je ca. 50 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Filtrat am Rotationsverdampfer vom Lösungsmittel befreit. Das Produkt wurde mittels präparativer HPLC (Methode N) isoliert. Es wurden 133 mg (59% d. Th.) der Titelverbindung erhalten.

¹H-NMR (400 MHz, CDCl₃, δ/ppm): 8.17 (d, 2H), 8.11 (d, 1H), 7.63 (d, 2H), 7.41 (dd, 1H), 6.78 (s, 1H), 6.60 (d, 1H), 5.31 (s, 2H), 3.52-3.49 (m, 4H), 2.99-2.95 (m, 4H), 2.32 (s, 3H), 0.32 (s, 9H).

LC/MS (Methode F, ESIpos): Rₜ = 1.23 min, m/z = 474 [M+H]⁺.

### Beispiel 55

### 1-Cyclopropyl-4-{5-[(5-methyl-3-{3-[4-(trimethylsilyl)phenyl]-1,2,4-oxadiazol-5-yl}-1H-pyrazol-1-yl)methyl]pyridin-2-yl}piperazin

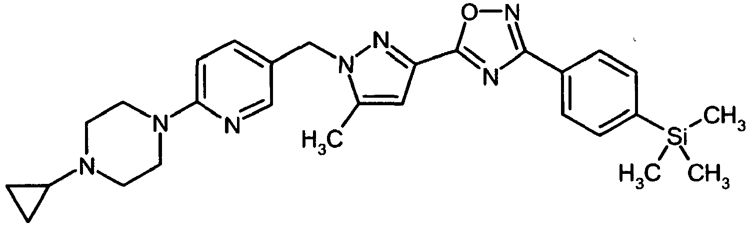

Eine Lösung von 100 mg (0.211 mmol) der Verbindung aus Beispiel 54 in 2 ml Methanol wurde nacheinander mit 121 µl (2.11 mmol) Eisessig, 30 mg getrocknetem, pulverisiertem Molekularsieb (3Å) und 255 µl (1.27 mmol) 1-Ethoxy-1-(trimethylsilyl)oxycyclopropan versetzt. Nach 10 min Rühren bei RT wurden 40 mg (0.633 mmol) festes Natriumcyanoborhydrid zugesetzt und das Gemisch 2 h unter Rückfluss erhitzt. Nach dem Abkühlen auf RT wurde zunächst vom Feststoff abgesaugt, mit Methanol nachgewaschen und vom Filtrat alle flüchtigen Bestandteile am Rotationsverdampfer entfernt. Der erhaltene Rückstand wurde mit ca. 50 ml halbgesättigter Natriumhydrogencarbonat-Lösung versetzt und dreimal mit je ca. 50 ml Ethylacetat extrahiert. Die vereinigten organischen Extrakte wurden nacheinander mit Wasser und gesättigter Kochsalz-Lösung gewaschen. Nach Trocknen über wasserfreiem Natriumsulfat wurde filtriert und das Filtrat am Rotationsverdampfer vom Lösungsmittel befreit. Das Produkt wurde mittels MPLC (Kieselgel, Laufmittel: Cyclohexan/Ethylacetat 1:1 → 1:3) isoliert. Es wurden 57 mg (53% d. Th.) der Titelverbindung erhalten.

¹H-NMR (400 MHz, CDCl₃, δ/ppm): 8.17 (d, 2H), 8.11 (d, 1H), 7.63 (d, 2H), 7.40 (dd, 1H), 6.78 (s, 1H), 6.60 (d, 1H), 5.30 (s, 2H), 3.52-3.49 (m, 4H), 2.71-2.68 (m, 4H), 2.31 (s, 3H), 1.66-1.61 (m, 1H), 0.49-0.43 (m, 4H), 0.30 (s, 9H).

LC/MS (Methode I, ESIpos): Rₜ = 1.08 min, m/z = 514 [M+H]⁺.

### Beispiel 56

### 5-[(5-Methyl-3-{3-[4-(trifluormethoxy)phenyl]-1,2,4-oxadiazol-5-yl}-1H-pyrazol-1-yl)methyl]-2-(pyrrolidin-1-yl)pyridin

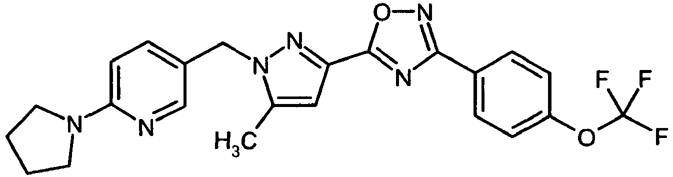

Ein Gemisch aus 200 mg (0.459 mmol) der Verbindung aus Beispiel 79A und 2 ml (23.9 mmol) Pyrrolidin wurde in einem Mikrowellenofen (CEM Discover, initiale Einstrahlleistung 250 W) 3 h bei 160°C gerührt. Anschließend wurde das überschüssige Pyrrolidin am Rotationsverdampfer entfernt. Das so erhaltene Rohprodukt wurde mittels MPLC gereinigt (Kieselgel, Laufmittel: Cyclohexan/Ethylacetat 100:0 → 2:1). Es wurden 53 mg (24% d. Th.) der Titelverbindung erhalten.

¹H-NMR (400 MHz, CDCl₃, δ/ppm): 8.25 (d, 2H), 8.09 (d, 1H), 7.38 (dd, 1H), 7.33 (d, 2H), 6.76 (s, 1H), 6.31 (d, 1H), 5.30 (s, 2H), 3.45-3.40 (m, 4H), 2.32 (s, 3H), 2.02-1.97 (m, 4H).

LC/MS (Methode I, ESIpos): Rₜ = 1.00 min, m/z = 471 [M+H]⁺.

### Beispiel 57

### 1-Cyclopropyl-4-{5-[(5-methyl-3-{3-[4-(trifluormethoxy)phenyl]-1,2,4-oxadiazol-5-yl}-1H-pyrazol-1-yl)methyl]pyridin-2-yl}piperazin

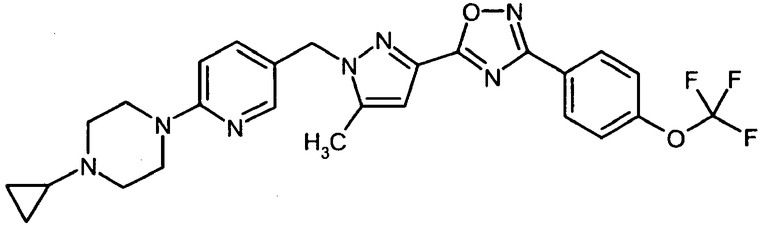

Analog zu dem unter Beispiel 53 beschriebenen Verfahren wurde aus 457 mg (2.29 mmol) 1-Cyclopropylpiperazin-Dihydrochlorid die freie Base hergestellt, die dann zusammen mit 100 mg (0.229 mmol) der Verbindung aus Beispiel 79A 4 Tage lang auf 150°C erhitzt wurde. Nach dem Abkühlen auf RT wurde die erstarrte Schmelze in ca. 4 ml Acetonitril gelöst und mittels präparativer HPLC (Methode N) in ihre Komponenten aufgetrennt. Die Produktfraktionen wurden vereinigt und am Rotationsverdampfer zur Trockene eingeengt. Das erhaltene Produkt wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Es wurden so 42 mg (35% d. Th.) der Titelverbindung erhalten.

¹H-NMR (400 MHz, CDCl₃, δ/ppm): 8.25 (d, 2H), 8.10 (d, 1H), 7.41 (dd, 1H), 7.33 (d, 2H), 6.77 (s, 1H), 6.60 (d, 1H), 5.30 (s, 2H), 3.52-3.49 (m, 4H), 2.71-2.68 (m, 4H), 2.31 (s, 3H), 1.66-1.60 (m, 1H), 0.49-0.44 (m, 4H).

HPLC (Methode A): Rₜ = 4.12 min.

LC/MS (Methode I, ESIpos): Rₜ = 1.03 min, m/z = 526 [M+H]⁺.

### Beispiel 58

### 1-{5-[(5-Methyl-3-{3-(4-(trifluormethyl)phenyl]-1,2,4-oxadiazol-5-yl}-1H-pyrazol-1-yl)methyl]-pyridin-2-yl}piperazin

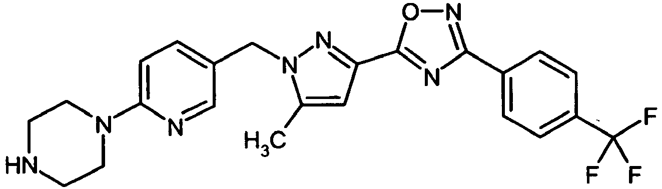

Analog zu dem unter Beispiel 54 beschriebenen Verfahren wurden aus 200 mg (0.476 mmol) der Verbindung aus Beispiel 88A und 821 mg (9.53 mmol) Piperazin 149 mg (67% d. Th.) der Titelverbindung erhalten. Das Produkt wurde mittels MPLC isoliert (Kieselgel, Laufmittel: Dichlormethan/Methanol 20:1 → 5:1).

¹H-NMR (400 MHz, CDCl₃, δ/ppm): 8.33 (d, 2H), 8.11 (d, 1H), 7.76 (d, 2H), 7.41 (dd, 1H), 6.79 (s, 1H), 6.60 (d, 1H), 5.31 (s, 2H), 3.53-3.49 (m, 4H), 2.99-2.96 (m, 4H), 2.32 (s, 3H).

LC/MS (Methode F, ESIpos): Rₜ = 1.09 min, m/z = 470 [M+H]⁺.

### Beispiel 59

### 1-Cyclopropyl-4-{5-[(5-methyl-3-{3-[4-(trifluormethyl)phenyl]-1,2,4-oxadiazol-5-yl}-1H-pyrazol-1-yl)methyl]pyridin-2-yl}piperazin

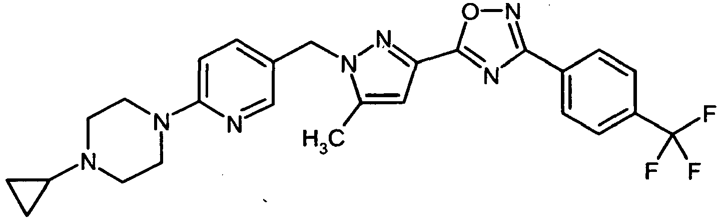

Analog zu dem unter Beispiel 55 beschriebenen Verfahren wurden aus 100 mg (0.213 mmol) der Verbindung aus Beispiel 58 und 257 µl (1.28 mmol) 1-Ethoxy-1-(trimethylsilyl)oxycyclopropan 43 mg (39% d. Th.) der Titelverbindung erhalten.

¹H-NMR (400 MHz, CDCl₃, δ/ppm): 8.33 (d, 2H), 8.11 (d, 1H), 7.76 (d, 2H), 7.41 (dd, 1H), 6.78 (s, 1H), 6.60 (d, 1H), 5.30 (s, 2H), 3.52-3.49 (m, 4H), 2.72-2.68 (m, 4H), 2.33 (s, 3H), 1.67-1.60 (m, 1H), 0.49-0.43 (m, 4H).

LC/MS (Methode I, ESIpos): Rₜ = 0.96 min, m/z = 510 [M+H]⁺.

### Beispiel 60

### 5-[(5-Methyl-3-{3-[4-(trifluormethoxy)phenyl]-1,2,4-oxadiazol-5-yl}-1H-pyrazol-1-yl)methyl]-2-(piperidin-1-yl)pyridin

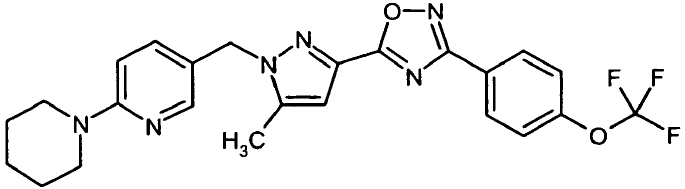

Analog zu dem unter Beispiel 56 beschriebenen Verfahren wurden aus 200 mg (0.459 mmol) der Verbindung aus Beispiel 79A und 2.3 ml (22.9 mmol) Piperidin 154 mg (69% d. Th.) der Titelverbindung erhalten.

¹H-NMR (400 MHz, CDCl₃, δ/ppm): 8.25 (d, 2H), 8.09 (d, 1H), 7.39 (dd, 1H), 7.33 (d, 2H), 6.77 (s, 1H), 6.60 (d, 1H), 5.30 (s, 2H), 3.53-3.50 (m, 4H), 2.32 (s, 3H), 1.64-1.60 (m, 6H).

HPLC (Methode A): Rₜ = 4.39 min.

MS (DCI, NH₃): m/z = 485 [M+H]⁺.

LC/MS (Methode F, ESIpos): Rₜ = 1.33 min, m/z = 485 [M+H]⁺.

### Beispiel 61

### 2-(Azetidin-1-yl)-5-[(5-methyl-3-{3-[4-(trifluormethoxy)phenyl]-1,2,4-oxadiazol-5-yl}-1H-pyrazol-1-yl)methyl]pyridin

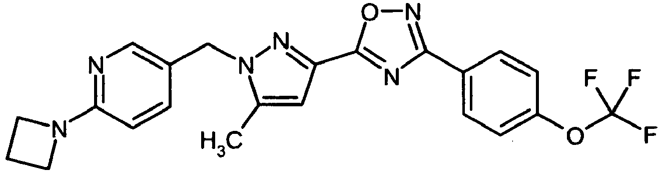

Analog zu dem unter Beispiel 16 beschriebenen Verfahren wurden 200 mg (0.495 mmol) der Verbindung aus Beispiel 79A und 310 µl (4.59 mmol) Azetidin zu 81 mg (39% d. Th.) der Titelverbindung umgesetzt.

¹H-NMR (400 MHz, CDCl₃, δ/ppm): 8.25 (d, 2H), 8.07 (d, 1H), 7.41-7.29 (m, 3H), 6.77 (s, 1H), 6.22 (d, 1H), 5.30 (s, 2H), 4.03 (t, 4H), 2.42-2.37 (m, 2H), 2.31 (s, 3H).

LC/MS (Methode D, ESIpos): Rₜ = 2.00 min, m/z = 457 [M+H]⁺.

### Beispiel 62

### tert.-Butyl-4-{4-[(5-methyl-3-{3-[4-(trifluormethoxy)phenyl]-1,2,4-oxadiazol-5-yl}-1H-pyrazol-1-yl)methyl]pyridin-2-yl}piperidin-1-carboxylat

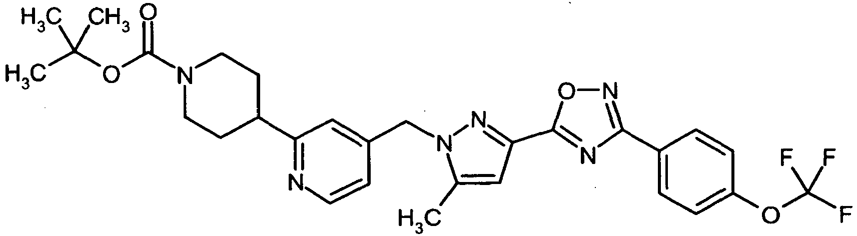

Eine Lösung von 301 mg (0.517 mmol) der Verbindung aus Beispiel 146A in 30 ml Methanol wurde in einer Durchfluss-Hydrierapparatur hydriert ["H-Cube" der Fa. Thales Nano, Budapest, Ungarn; Bedingungen: Pd-Kartusche (10% auf Kohle), 10 bar H₂, 25°C, Fluss 1 ml/min]. Da die Umsetzung beim ersten Durchlauf nicht vollständig war, wurde das Reaktionsgemisch ein zweites Mal über die Kartusche gegeben. Nach dem Abdampfen des Lösungsmittels am Rotationsverdampfer wurde das Rohprodukt mittels präparativer HPLC (Methode N) gereinigt. Es wurden 165 mg (53% d. Th., 97% Reinheit) der Titelverbindung erhalten.

¹H-NMR (400 MHz, CDCl₃, δ/ppm): 8.50 (d, 1H), 8.25 (d, 2H), 7.33 (d, 2H), 6.87-6.83 (m, 3H), 5.43 (s, 2H), 4.28-4.17 (m, 2H), 2.85-2.75 (m, 3H), 2.29 (s, 3H), 1.88-1.82 (m, 2H), 1.72-1.62 (m, 2H), 1.45 (s, 9H).

LC/MS (Methode I, ESIpos): Rₜ = 1.34 min, m/z = 585 [M+H]⁺.

### Beispiel 63

### 4-[(5-Methyl-3-{3-[4-(trifluormethoxy)phenyl]-1,2,4-oxadiazol-5-yl)-1H-pyrazol-1-yl)methyl]-2-(piperidin-4-yl)pyridin-Hydrochlorid

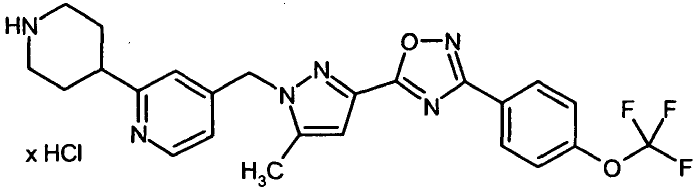

Eine Lösung von 150 mg (0.257 mmol) der Verbindung aus Beispiel 62 in 1 ml Dioxan wurde mit 641 µl (2.57 mmol) einer 4 M Lösung von Chlorwasserstoff in Dioxan versetzt. Nachdem das Reaktionsgemisch 2 h bei RT gerührt worden war, wurde es am Rotationsverdampfer zur Trockene eingeengt. Der erhaltene Rückstand wurde mit ca. 5 ml Pentan/Dioxan (10:1) verrieben. Nach Trocknen im Hochvakuum wurden 143 mg (97% d. Th., 91 % Reinheit) der Titelverbindung erhalten.

¹H-NMR (400 MHz, DMSO-d₆, δ/ppm): 8.96 (s, breit, 1H), 8.70 (s, breit, 1H), 8.57 (d, 1H), 8.20 (d, 2H), 7.61 (d, 2H), 7.18 (d, 1H), 7.11 (dd, 1H), 7.01 (s, 1H), 5.65 (s, 2H), 3.38-3.32 (m, 2H), 3.14-3.06 (m, 1H), 3.03-2.93 (m, 2H), 2.32 (s, 3H), 2.03-1.86 (m, 4H).

LC/MS (Methode F, ESIpos): Rₜ = 1.06 min, m/z = 485 [M+H]⁺.

### Beispiel 64

### 1-{4-[(5-Methyl-3-{3-[4-(trifluormethoxy)phenyl]-1,2,4-oxadiazol-5-yl}-1H-pyrazol-1-yl)methyl]-pyridin-2-yl}piperazin

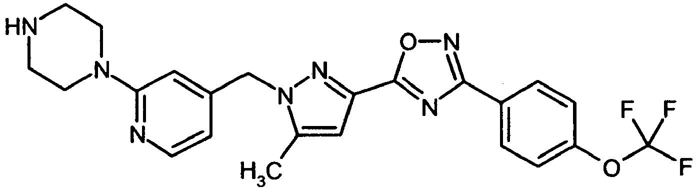

91.5 g (0.210 mol) der Verbindung aus Beispiel 81A und 362 g (4.20 mol) Piperazin wurden ohne Zusatz von Lösungsmittel 16 h lang auf 150°C erhitzt. Nachdem die Schmelze auf RT abgekühlt war, wurden 6 L Wasser und 4 L Ethylacetat zugesetzt und das Gemisch intensiv gerührt. Nach Abtrennen der organischen Phase wurde diese nacheinander mit je ca. 2.5 L Wasser und gesättigter Kochsalz-Lösung gewaschen. Nach Trocknen über wasserfreiem Magnesiumsulfat wurde filtriert und das Filtrat anschließend am Rotationsverdampfer vom Lösungsmittel befreit. Der erhaltene Rückstand wurde über ca. 3 kg Kieselgel (0.04-0.06 mm) chromatographiert (Laufmittel: Dichlormethan/Methanol 9:1, 12 L → 8:2, 12 L → 7:3, 16 L → 6:4, 8 L). Die Produktfraktionen wurden vereinigt und am Rotationsverdampfer eingeengt. Es wurden 67.1 g (66% d. Th.) der Titelverbindung erhalten.

¹H-NMR (400 MHz, CDCl₃, δ/ppm): 8.25 (d, 2H), 8.13 (d, 1H), 7.33 (d, 2H), 6.84 (s, 1H), 6.35 (d, 1H), 6.32 (s, 1H), 5.35 (s, 2H), 3.48-3.45 (m, 4H), 2.97-2.94 (m, 4H), 2.30 (s, 3H).

LC/MS (Methode D, ESIpos): Rₜ = 1.89 min, m/z = 486 [M+H]⁺.

### Beispiel 65

### 1-Cyclopropyl-4-{4-[(5-methyl-3-{3-[4-(trifluormethoxy)phenyl]-1,2,4-oxadiazol-5-yl}-1H-pyrazol-1-yl)methyl]pyridin-2-yl} piperazin

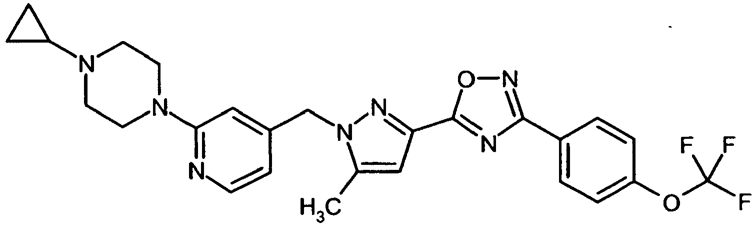

Eine Lösung von 56.0 g (0.115 mol) der Verbindung aus Beispiel 64 in 1.13 L Methanol wurde nacheinander mit 66 ml (1.15 mol) Eisessig, 13.9 g getrocknetem, pulverisiertem Molekularsieb (3Å) und 139 ml (0.692 mol) 1-Ethoxy-1-(trimethylsilyl)oxycyclopropan versetzt. Nach 10 min Rühren bei RT wurden 21.7 g (0.346 mol) festes Natriumcyanoborhydrid zugesetzt. Anschließend wurde das Gemisch 1 h unter Rückfluss erhitzt. Nach dem Abkühlen auf RT wurde vom Ungelösten abgesaugt und das Filtrat am Rotationsverdampfer eingeengt. Der erhaltene Rückstand wurde in 1 L Ethylacetat aufgenommen und zweimal mit je ca. 750 ml gesättigter Natriumhydrogencarbonat-Lösung und anschließend mit ca. 750 ml gesättigter Kochsalz-Lösung gewaschen. Nach Trocknen über wasserfreiem Natriumsulfat wurde filtriert und das Filtrat am Rotationsverdampfer vom Lösungsmittel befreit. Der Rückstand (53 g) wurde aus einem siedenden Gemisch aus 293 ml Ethanol und 59 ml Wasser umkristallisiert. Nachdem die Kristallisation vollständig war (nach ca. 20 h bei RT), wurde abgesaugt. Der Feststoff wurde mit 36 ml Ethanol/Wasser (5:1) gewaschen und anschließend im Hochvakuum getrocknet. Es wurden so 26.4 g der Titelverbindung als erste Charge erhalten. Die Mutterlauge der Kristallisation wurde am Rotationsverdampfer eingeengt. Mittels präparativer HPLC (Methode N) wurden weitere 20.3 g des Produkts in Form des Formiat-Salzes erhalten. Zur Freisetzung der Base wurde eine Suspension dieses Formiats in 1 L Ethylacetat nacheinander mit je ca. 200 ml gesättigter Natriumhydrogencarbonat-Lösung, Wasser und gesättigter Kochsalz-Lösung gewaschen. Nach Trocknen über wasserfreiem Natriumsulfat wurde filtriert und das Filtrat am Rotationsverdampfer vom Lösungsmittel befreit. Der Rückstand (13 g) wurde aus einem siedenden Gemisch aus 80 ml Ethanol und 16 ml Wasser umkristallisiert. Nachdem die Kristallisation vollständig war (nach ca. 4 h bei RT), wurde abgesaugt und der Feststoff im Hochvakuum getrocknet. Auf diese Weise wurden weitere 11.2 g der Titelverbindung erhalten (Ausbeute insgesamt 37.6 g, 62% d. Th.).

Schmelzpunkt: 140°C ¹H-NMR (400 MHz, CDCl₃, δ/ppm): 8.26 (d, 2H), 8.13 (d, 1H), 7.33 (d, 2H), 6.83 (s, 1H), 6.33 (d, 1H), 6.32 (s, 1H), 5.35 (s, 2H), 3.47 (dd, 4H), 2.69 (dd, 4H), 2.30 (s, 3H), 1.65-1.60 (m, 1H), 0.48-0.42 (m, 4H).

LC/MS (Methode D, ESIpos): Rₜ = 1.91 min, m/z = 526 [M+H]⁺.

### Beispiel 66

### 1-Cyclopropyl-4-{4-[(5-methyl-3-{3-[4-(trifluormethoxy)phenyl]-1,2,4-oxadiazol-5-yl}-1H-pyrazol-1-yl)methyl]pyridin-2-yl}piperazin-Hydrochlorid

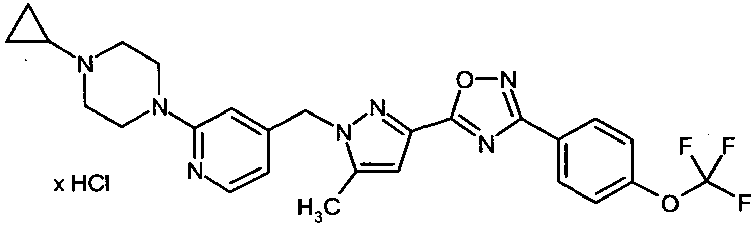

Eine Lösung von 362 mg (0.690 mmol) der Verbindung aus Beispiel 65 in 50 ml THF wurde bei RT mit 690 µl 1M Salzsäure versetzt und 1h bei RT gerührt. Anschließend wurde am Rotationsverdampfer bis zur vollständigen Trockene eingeengt. Der erhaltene Rückstand wurde aus einem siedenden Gemisch von 11.5 ml Isopropanol und 5 ml Ethanol umkristallisiert. Nach Trocknen im Hochvakuum wurden 330 mg (85% d. Th.) der Titelverbindung erhalten, welche als Solvat mit einem Äquivalent Isopropanol im Kristall vorlag.

Schmelzpunkt: 206-210°C

¹H-NMR (400 MHz, DMSO-d₆, δ/ppm): 10.40 (breit, 1H), 8.20 (d, 2H), 8.12 (d, 1H), 7.60 (d, 2H), 6.97 (s, 1H), 6.83 (d, 1H), 6.40 (s, 1H), 5.47 (s, 2H), 4.41-4.31 (m, 2H), 3.77 (sept, 1H), 3.60-3.53 (breit, 2H), 3.30-3.20 (breit, 4H), 2.90 (breit, 1H), 2.36 (s, 3H), 1.12-1.08 (breit, 2H), 1.03 (d, 6H), 0.84-0.79 (m, 2H).

LC/MS (Methode I, ESIpos): Rₜ = 0.97 min, m/z = 526 [M+H]⁺.

### Beispiel 67

### 1-Cyclopropyl-4-{4-[(5-methyl-3-{3-[4-(trifluormethoxy)phenyl]-1,2,4-oxadiazol-5-yl}-1H-pyrazol-1-yl)methyl]pyridin-2-yl}piperazin-Methansulfonat

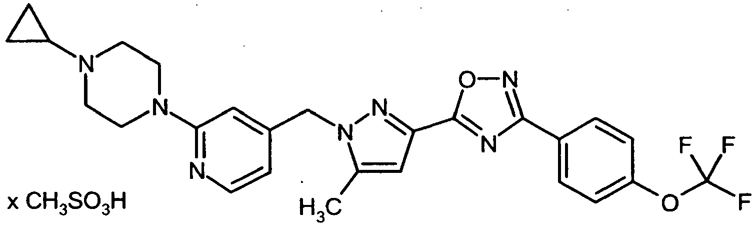

Eine Lösung von 500 mg (0.952 mmol) der Verbindung aus Beispiel 65 in 30 ml THF wurde bei RT mit 91.5 mg (0.952 mmol) Methansulfonsäure versetzt und 1 h bei RT gerührt. Anschließend wurde am Rotationsverdampfer bis zur vollständigen Trockene eingeengt. Nach Trocknen des Rückstands im Hochvakuum wurden 500 mg (85% d. Th.) der Titelverbindung erhalten.

¹H-NMR (400 MHz, DMSO-d₆, δ/ppm): 9.12 (breit, 1H), 8.20 (d, 2H), 8.13 (d, 1H), 7.60 (d, 2H), 6.98 (s, 1H), 6.83 (s, 1H), 6.41 (d, 1H), 5.47 (s, 2H), 4.44-4.36 (m, 2H), 3.63-3.56 (m, 2H), 3.32-3.23 (m, 2H), 3.14-3.03 (m, 2H), 2.96 (breit, 1H), 2.37 (s, 3H), 2.30 (s, 3H), 1.00-0.97 (breit, 2H), 0.89-0.82 (m, 2H).

### Beispiel 68

### 1-Cyclopropyl-4-{4-[(5-methyl-3-{3-[4-(trifluormethoxy)phenyl]-1,2,4-oxadiazol-5-yl}-1H-pyrazol-1-yl)methyl]pyridin-2-yl}piperazin-4-Sulfamoylbenzoat

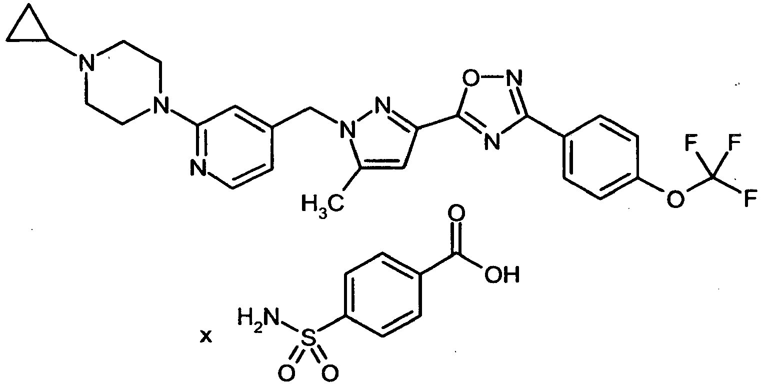

Eine Lösung von 522 mg (0.994 mmol) der Verbindung aus Beispiel 65 in 20 ml THF wurde bei RT mit 206 mg (0.994 mmol) 4-Sulfamoylbenzoesäure versetzt und 1 h bei RT gerührt. Anschließend wurde am Rotationsverdampfer bis zur vollständigen Trockene eingeengt. Nach Trocknen des Rückstands im Hochvakuum wurden 632 mg (88% d. Th.) der Titelverbindung erhalten.

¹H-NMR (400 MHz, DMSO-d₆, δ/ppm): 13.37 (breit, 1H), 8.20 (d, 2H), 8.11 (d, 2H), 8.04 (d, 1H), 7.93 (d, 2H), 7.59 (d, 2H), 7.53 (s, 2H), 6.96 (s, 1H), 6.68 (s, 1H), 6.27 (d, 1H), 5.43 (s, 2H), 3.44-3.40 (m, 4H), 2.62-2.58 (m, 4H), 2.33 (s, 3H), 1.67-1.61 (m, 1H), 0.47-0.41 (m, 2H), 0.37-0.33 (m, 2H).

### Beispiel 69

### 1-(2,2-Difluorethyl)-4-{4-[(5-methyl-3-{3-[4-(trifluormethoxy)phenyl]-1,2,4-oxadiazol-5-yl}-1H-pyrazol-1-yl)methyl]pyridin-2-yl}piperazin

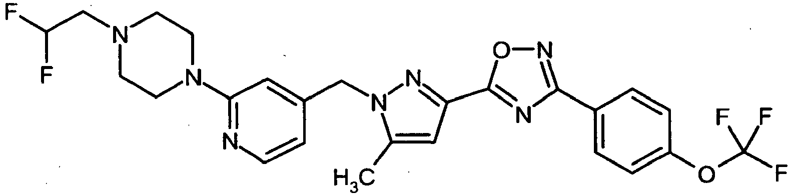

65 mg (0.149 mmol) der Verbindung aus Beispiel 81A und 166 mg (0.746 mmol) der Verbindung aus Beispiel 104A wurden zusammen mit 260 µl (1.49 mmol) *N,N*-Diisopropylethylamin in einem Mikrowellenofen (CEM Discover, initiale Einstrahlleistung 250 W) 3 h lang bei 160°C gerührt. Nach dem Abkühlen auf RT wurde mit ca. 3 ml Methanol verdünnt und das Reaktionsgemisch direkt mittels präparativer HPLC (Methode N) in seine Komponenten aufgetrennt. Die Produktfraktionen wurden vereinigt und am Rotationsverdampfer vom Lösungsmittel befreit. Anschließend wurde erneut in ca. 5 ml Methanol gelöst und die Lösung über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Es wurden 65 mg (78% d. Th.) der Titelverbindung erhalten.

¹H-NMR (400 MHz, CDCl₃, δ/ppm): 8.25 (d, 2H), 8.12 (d, 1H), 7.33 (d, 2H), 6.83 (s, 1H), 6.37 (d, 1H), 6.31 (s, 1H), 5.90 (tt, 1H), 5.34 (s, 2H), 3.50 (dd, 4H), 2.77 (dt, 2H), 2.66 (dd, 4H), 2.29 (s, 3H).

LC/MS (Methode I, ESIpos): Rₜ = 1.19 min, m/z = 550 [M+H]⁺.

### Beispiel 70

### 1-{4-[(5-Methyl-3-{3-[4-(tetrahydro-2H-pyran-4-yl)phenyl]-1,2,4-oxadiazol-5-yl}-1H-pyrazol-1-yl)methyl]pyridin-2-yl}piperazin-Formiat

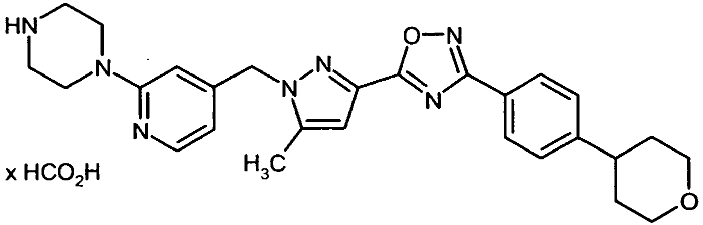

Ein Gemisch aus 220 mg (0.505 mmol) der Verbindung aus Beispiel 140A und 869 mg (10.1 mmol) Piperazin wurde ohne Zusatz von Lösungsmittel 16 h lang bei 160°C gerührt. Nachdem die Schmelze auf RT erkaltet war, wurde mit ca. 50 ml Wasser versetzt und dreimal mit je ca. 20 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt und der Rückstand mittels präparativer HPLC gereinigt (Methode N). Es wurden 178 mg (66% d. Th.) der Titelverbindung erhalten.

¹H-NMR (400 MHz, CDCl₃, δ/ppm): 8.33 (s, 1H), 8.15 (2d, 2H+1H), 7.35 (d, 2H), 6.83 (s, 1H), 6.43 (d, 1H), 6.34 (s, 1H), 5.37 (s, 2H), 4.13-4.08 (m, 2H), 3.70-3.67 (m, 4H), 3.58-3.52 (m, 2H), 3.15-3.11 (m, 4H), 2.88-2.80 (m, 1H), 2.29 (s, 3H), 1.92-1.78 (m, 4H).

LC/MS (Methode I, ESIpos): Rₜ = 0.83 min, m/z = 486 [M+H]⁺.

### Beispiel 71

### 1-Cyclopropyl-4-{4-[(5-methyl-3-{3-[4-(tetrahydro-2H-pyran-4-yl)phenyl]-1,2,4-oxadiazol-5-yl}-1H-pyrazol-1-yl)methyl]pyridin-2-yl}piperazin

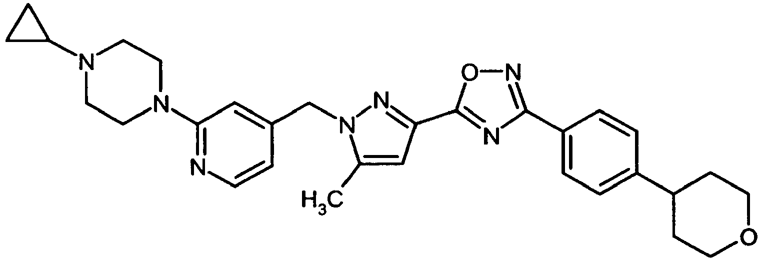

Eine Lösung von 175 mg (0.360 mmol) der Verbindung aus Beispiel 70 in 5 ml Methanol wurde nacheinander mit 206 µl (3.60 mmol) Eisessig, 51 mg getrocknetem, pulverisiertem Molekularsieb (3Å) und 435 µl (2.17 mmol) 1-Ethoxy-1-(trimethylsilyl)oxycyclopropan versetzt. Nach 10 min Rühren bei RT wurden 68 mg (1.08 mmol) festes Natriumcyanoborhydrid zugesetzt. Anschließend wurde das Gemisch 2 h unter Rückfluss erhitzt. Nach dem Abkühlen auf RT wurde vom Ungelösten abgesaugt und das Filtrat am Rotationsverdampfer eingeengt. Das Produkt wurde aus dem Rückstand mittels MPLC (Kieselgel, Laufmittel:: Dichlormethan/Methanol 30:1) isoliert. Durch Verreiben mit Pentan wurde das erhaltene zähe Öl in einen Feststoff überführt, der mit Acetonitril/ Methanol (10:1) verrührt wurde. Nach Trocknen im Hochvakuum wurden 30 mg (15% d. Th., 95% Reinheit) der Titelverbindung erhalten.

¹H-NMR (400 MHz, CDCl₃, δ/ppm): 8.15 (d, 2H), 8.12 (d, 1H), 7.35 (d, 2H), 6.83 (s, 1H), 6.33 (d, 1H), 6.32 (s, 1H), 5.35 (s, 2H), 4.13-4.08 (m, 2H), 3.59-3.52 (m, 2H), 3.49-3.44 (m, 4H), 2.88-2.79 (m, 1H), 2.71-2.66 (m, 4H), 2.28 (s, 3H), 1.92-1.78 (m, 4H), 1.67-1.58 (m, 1H), 0.49-0.42 (m, 4H).

LC/MS (Methode F, ESIpos): Rₜ = 0.83 min, m/z = 526 [M+H]⁺.

### Beispiel 72

### 1-{4-[(5-Methyl-3-{3-[4-(trifluormethoxy)phenyl]-1,2,4-oxadiazol-5-yl}-1H-pyrazol-1-yl)methyl]-pyridin-2-yl}-4-(2,2,2-trifluorethyl)piperazin

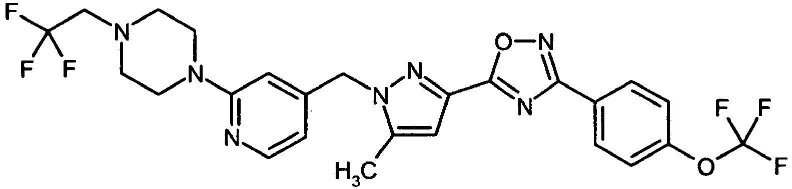

100 mg (0.229 mmol) der Verbindung aus Beispiel 81A und 277 mg (1.15 mmol) der Verbindung aus Beispiel 138A wurden zusammen mit 400 µl (2.30 mmol) *N,N*-Diisopropylethylamin in einem Mikrowellenofen (CEM Discover, initiale Einstrahlleistung 250 W) 3 h lang bei 160°C gerührt. Nach dem Abkühlen auf RT wurde mit ca. 3 ml Methanol verdünnt und das Reaktionsgemisch direkt mittels präparativer HPLC (Methode N) in seine Komponenten aufgetrennt. Die Produktfraktionen wurden vereinigt und am Rotationsverdampfer vom Lösungsmittel befreit. Anschließend wurde erneut in ca. 5 ml Methanol gelöst und die Lösung über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Es wurden 64 mg (49% d. Th.) der Titelverbindung erhalten.

¹H-NMR (400 MHz, CDCl₃, δ/ppm): 8.24 (d, 2H), 8.12 (d, 1H), 7.33 (d, 2H), 6.83 (s, 1H), 6.37 (d, 1H), 6.31 (s, 1H), 5.35 (s, 2H), 3.51 (dd, 4H), 3.00 (quart, 2H), 2.73 (dd, 4H), 2.29 (s, 3H).

LC/MS (Methode I, ESIpos): Rₜ = 1.31 min, m/z = 568 [M+H]⁺.

### Beispiel 73

### 1-Cyclobutyl-4-{4-[(5-methyl-3-{3-[4-(trifluormethoxy)phenyl]-1,2,4-oxadiazol-5-yl}-1H-pyrazol-1-yl)methyl]pyridin-2-yl piperazin

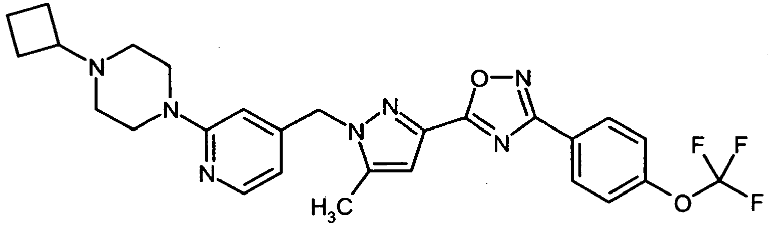

200 mg (0.412 mmol) der Verbindung aus Beispiel 64 und 37 µl (0.494 mmol) Cyclobutanon wurden in 5 ml wasserfreiem Ethanol gelöst und 1 h bei RT gerührt. Dann wurden 47 mg (1.24 mmol) festes Natriumborhydrid zugefügt. Nachdem das Reaktionsgemisch 16 h bei RT gerührt worden war, wurden 25 ml Wasser zugesetzt, und es wurde zweimal mit je ca. 20 ml Dichlormethan extrahiert. Die vereinigten organischen Extrakte wurden über wasserfreiem Magnesiumsulfat getrocknet. Nach Filtration wurde das Lösungsmittel am Rotationsverdampfer entfernt und das Rohprodukt mittels präparativer HPLC (Methode N) gereinigt. Das erhaltene Produkt wurde erneut in ca. 5 ml Methanol gelöst und die Lösung über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Es wurden 74 mg (31% d. Th., 95% Reinheit) der Titelverbindung erhalten.

¹H-NMR (400 MHz, CDCl₃, δ/ppm): 8.25 (d, 2H), 8.12 (d, 1H), 7.33 (d, 2H), 6.83 (s, 1H), 6.33 (d, 1H), 6.31 (s, 1H), 5.35 (s, 2H), 3.50 (dd, 4H), 2.73 (quint, 1H), 2.39 (dd, 4H), 2.29 (s, 3H), 2.07-2.00 (m, 2H), 1.94-1.85 (m, 2H), 1.77-1.65 (m, 2H).

LC/MS (Methode I, ESIpos): Rₜ = 0.93 min, m/z = 540 [M+H]⁺.

### Beispiel 74

### 1-[4-({3-[3-(4-tert.-Butylphenyl)-1,2,4-oxadiazol-5-yl]-5-methyl-1H-pyrazol-1-yl}methyl)pyridin-2-yl]piperazin

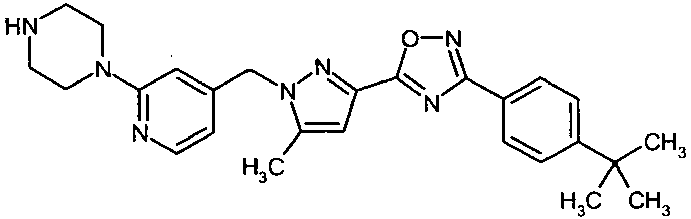

Eine Lösung von 400 mg (0.981 mmol) der Verbindung aus Beispiel 141A und 1.69 g (19.6 mmol) Piperazin in 12 ml Ethanol wurde in einem Mikrowellengerät (Biotage Initiator 2.5) automatisch gesteuert auf 140°C und dann manuell innerhalb von 3 min auf 190°C erhitzt. Nach 1h bei 190°C ließ man das Reaktionsgemisch auf RT abkühlen. Es wurde mit 100 ml Wasser versetzt und dreimal mit je ca. 50 ml Ethylacetat extrahiert. Die vereinigten organischen Extrakte wurden mit gesättigter Kochsalz-Lösung gewaschen und über wasserfreiem Magnesiumsulfat getrocknet. Nach Filtration und Eindampfen wurde ein Rohprodukt erhalten, das durch MPLC (ca. 30 g Kieselgel, Laufmittel: Dichlormethan/Methanol 10:1) gereinigt wurde. Es wurden 339 mg (76% d. Th.) der Titelverbindung erhalten.

¹H-NMR (400 MHz, CDCl₃, δ/ppm): 8.12 (2d, 2H+1H), 7.51 (d, 2H), 6.83 (s, 1H), 6.37 (d, 1H), 6.32 (s, 1H), 5.36 (s, 2H), 3.50-3.47 (m, 4H), 2.98-2.95 (m, 4H), 2.29 (s, 3H), 1.37 (s, 9H).

LC/MS (Methode I, ESIpos): Rₜ = 0.96 min, m/z = 458 [M+H]⁺.

### Beispiel 75

### 1-[4-({3-[3-(4-tert.-Butylphenyl)-1,2,4-oxadiazol-5-yl]-5-methyl-1H-pyrazol-1-yl}methyl)pyridin-2-yl]-4-cyclopropylpiperazin

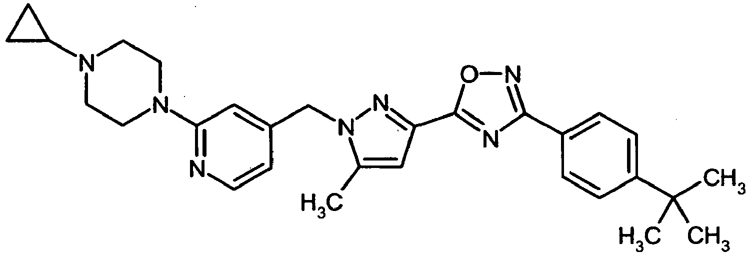

Analog zu dem unter Beispiel 55 beschriebenen Verfahren wurden 135 mg (0.295 mmol) der Verbindung aus Beispiel 74 zu 60 mg (40% d. Th., 98% Reinheit) der Titelverbindung umgesetzt.

¹H-NMR (400 MHz, CDCl₃, δ/ppm): 8.13 (d, 2H), 8.12 (d, 1H), 7.51 (d, 2H), 6.83 (s, 1H), 6.34 (d, 1H), 6.32 (s, 1H), 5.34 (s, 2H), 3.48-3.45 (m, 4H), 2.70-2.67 (m, 4H), 2.29 (s, 3H), 1.65-1.60 (m, 1H), 1.37 (s, 9H), 0.48-0.43 (m, 4H).

LC/MS (Methode D, ESIpos): Rₜ = 2.04 min, m/z = 498 [M+H]⁺.

### Beispiel 76

### 1-(4-{[3-(3-{4-[1-(Methoxymethyl)cyclobutyl]phenyl}-1,2,4-oxadiazol-5-yl)-5-methyl-1H-pyrazol-1-yl]methyl}pyridin-2-yl)piperazin-Formiat

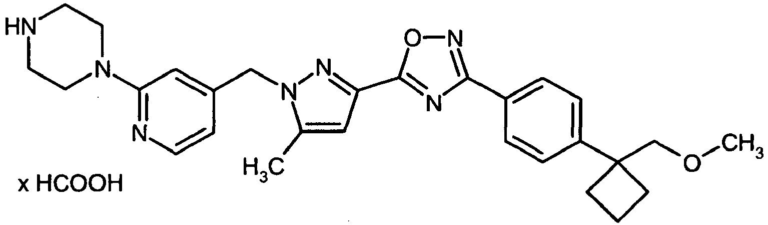

Analog zu dem unter Beispiel 74 beschriebenen Verfahren wurden 766 mg (8.89 mmol) Piperazin und 200 mg (0.444 mmol) der Verbindung aus Beispiel 142A zu 198 mg (82% d. Th.) der Titelverbindung umgesetzt. Das Rohprodukt wurde mittels präparativer HPLC (Methode N) gereinigt.

¹H-NMR (400 MHz, CDCl₃, δ/ppm): 8.39 (s, 1H), 8.13 (d, 1H), 8.12 (d, 2H), 7.30 (d, 2H), 6.83 (s, 1H), 6.44 (d, 1H), 6.33 (s, 1H), 5.37 (s, 2H), 3.71-3.69 (m, 4H), 3.55 (s, 2H), 3.29 (s, 3H), 3.15-3.12 (m, 4H), 2.42-2.29 (m, 4H), 2.29 (s, 3H), 2.16-2.03 (m, 1H), 1.93-1.83 (m, 1H).

LC/MS (Methode D, ESIpos): Rₜ = 1.86 min, m/z = 500 [M+H]⁺.

### Beispiel 77

### 1-Cyclopropyl-4-(4-{[3-(3-{4-[1-(methoxymethyl)cyclobutyl]phenyl}-1,2,4-oxadiazol-5-yl)-5-methyl-1H-pyrazol-1-yl]methyl}pyridin-2-yl)piperazin

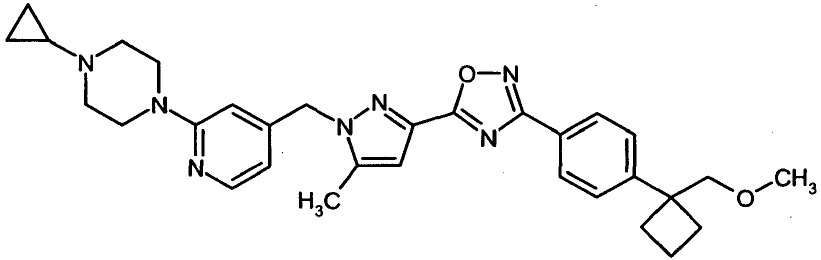

Analog zu dem unter Beispiel 55 beschriebenen Verfahren wurden 100 mg (0.183 mmol) der Verbindung aus Beispiel 76 zu 65 mg (65% d. Th., 98% Reinheit) der Titelverbindung umgesetzt.

¹H-NMR (400 MHz, CDCl₃, δ/ppm): 8.13 (d, 2H), 8.12 (d, 1H), 7.30 (d, 2H), 6.83 (s, 1H), 6.34 (d, 1H), 6.32 (s, 1H), 5.35 (s, 2H), 3.56 (s, 2H), 3.48-3.45 (m, 4H), 3.29 (s, 3H), 2.70-2.67 (m, 4H), 2.44-2.29 (m, 4H), 2.29 (s, 3H), 2.16-2.03 (m, 1H), 1.93-1.83 (m, 1H), 1.65-1.60 (m, 1H), 0.49-0.43 (m, 4H).

LC/MS (Methode F, ESIpos): Rₜ = 1.15 min, m/z = 540 [M+H]⁺.

### Beispiel 78

### 1-[4-({3-[3-(4-tert. -Butylphenyl)-1,2,4-oxadiazol-5-yl]-5-methyl-1H-pyrazol-1-yl}methyl)pyridin-2-yl]-4-(2,2,2-trifluorethyl)piperazin

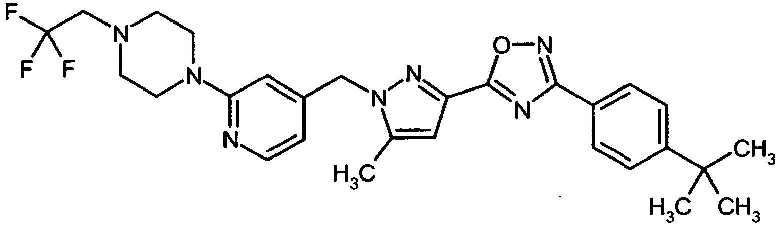

Eine Lösung von 32 µl (0.437 mmol) 2,2,2-Trifluorethanol in 2 ml wasserfreiem Dichlormethan wurde bei 0°C zunächst mit 76 µl (0.546 mmol) Triethylamin und 74 µl (0.437 mmol) Trifluormethansulfonsäureanhydrid versetzt. Nach 2 h Rühren bei 0°C wurde eine Lösung von 100 mg (0.219 mmol) der Verbindung aus Beispiel 74 in 1 ml Dichlormethan hinzugefügt. Das Rühren wurde bei RT fortgesetzt. Nach 15 h wurde mit ca. 20 ml Wasser versetzt und mit Dichlormethan extrahiert. Der organische Extrakt wurde mit Wasser gewaschen und über wasserfreiem Magnesiumsulfat getrocknet. Nach Filtration wurde das Lösungsmittel am Rotationsverdampfer entfernt und das Produkt mittels präparativer HPLC (Methode N) isoliert. Es wurden 34 mg (28% d. Th., 98% Reinheit) der Titelverbindung erhalten.

¹H-NMR (400 MHz, CDCl₃, δ/ppm): 8.13 (2d, 2H+1H), 7.52 (d, 2H), 6.83 (s, 1H), 6.38 (d, 1H), 6.31 (s, 1H), 5.35 (s, 2H), 3.53-3.50 (m, 4H), 3.00 (quart, 2H), 2.76-2.73 (m, 4H), 2.28 (s, 3H), 1.37 (s, 9H).

LC/MS (Methode Q, ESIpos): Rₜ = 2.70 min, m/z = 540 [M+H]⁺.

### Beispiel 79

### 1-{4-[(5-Methyl-3-{3-[3-methyl-4-(tetrahydro-2H-pyran-4-yl)phenyl]-1,2,4-oxadiazol-5-yl}-1H-pyrazol-1-yl)methyl]pyridin-2-yl}piperazin

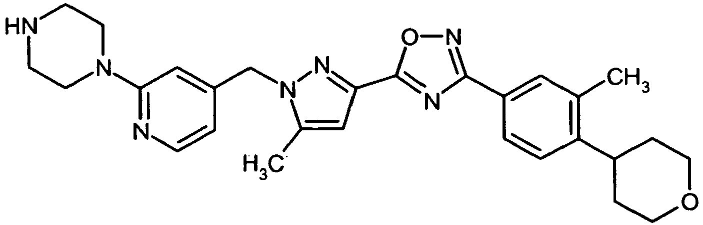

Analog zu dem unter Beispiel 54 beschriebenen Verfahren wurden aus 145 mg (0.322 mmol) der Verbindung aus Beispiel 143A und 555 mg (6.45 mmol) Piperazin 160 mg (95% d. Th., 95% Reinheit) der Titelverbindung erhalten. Das Rohprodukt wurde in diesem Falle nicht mittels präparativer HPLC, sondern durch Verreiben mit Pentan gereinigt.

¹H-NMR (400 MHz, CDCl₃, δ/ppm): 8.12 (d, 1H), 8.02 (s, 1H), 8.01 (d, 1H), 7.34 (d, 1H), 6.83 (s, 1H), 6.36 (d, 1H), 6.32 (s, 1H), 5.35 (s, 2H), 4.13-4.10 (m, 2H), 3.67-3.54 (m, 4H), 3.64 (dd, 4H), 3.07-3.00 (m, 1H), 2.94 (dd, 4H), 2.42 (s, 3H), 2.28 (s, 3H), 1.91-1.81 (m, 2H).

LC/MS (Methode D, ESIpos): Rₜ = 1.72 min, m/z = 500 [M+H]⁺.

### Beispiel 80

### 1-{4-[(3-{3-[3-Chlor-4-(trifluormethoxy)phenyl]-1,2,4-oxadiazol-5-yl}-5-methyl-1H-pyrazol-1-yl)-methyl]pyridin-2-yl}piperazin

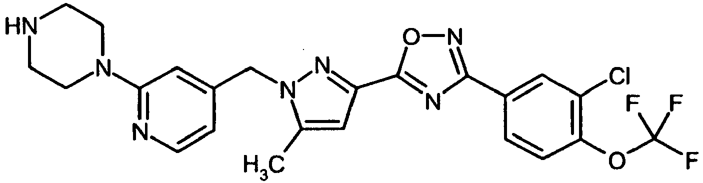

Analog zu dem unter Beispiel 16 beschriebenen Verfahren wurden 340 mg (0.723 mmol) der Verbindung aus Beispiel 144A und 1.24 g (14.5 mmol) Piperazin zu 114 mg (30% d. Th.) der Titelverbindung umgesetzt.

¹H-NMR (400 MHz, DMSO-d₆, δ/ppm): 8.04 (d, 1H), 8.01-7.93 (m, 2H), 7.30 (d, 1H), 6.93 (s, 1H), 6.61 (s, 1H), 6.26 (d, 1H), 5.42 (s, 2H), 4.10 (s, breit, 1H), 3.35-3.25 (t, 4H), 2.80-2.70 (t, 4H), 2.32 (s, 3H).

LC/MS (Methode D, ESIpos): Rₜ = 1.27 min, m/z = 520/522 [M+H]⁺.

### Beispiel 81

### 1-Cyclopropyl-4-{4-[(5-methyl-3-{3-[3-methyl-4-(tetrahydro-2H-pyran-4-yl)phenyl]-1,2,4-oxa-diazol-5-yl}-1H-pyrazol-1-yl)methyl]pyridin-2-yl}piperazin

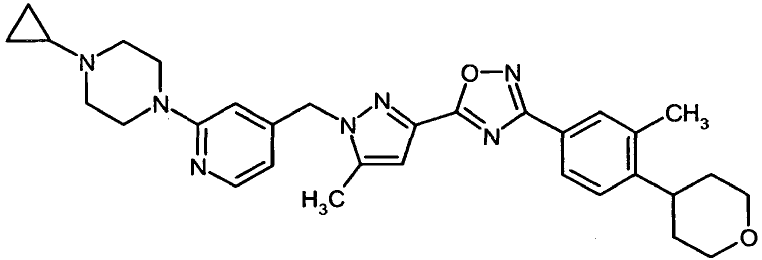

Analog zu dem unter Beispiel 71 beschriebenen Verfahren wurden aus 150 mg (0.30 mmol) der Verbindung aus Beispiel 79 und 362 µl (1.80 mmol) 1-Ethoxy-1-(trimethylsilyl)oxycyclopropan 23 mg (13% d. Th., 93% Reinheit) der Titelverbindung erhalten. Die Reinigung des Rohprodukts erfolgte mittels präparativer HPLC (Methode N).

¹H-NMR (400 MHz, CDCl₃, δ/ppm): 8.12 (d, 1H), 8.02 (s, 1H), 8.01 (d, 1H), 7.34 (d, 1H), 6.83 (s, 1H), 6.33 (d, 1H), 6.32 (s, 1H), 5.34 (s, 2H), 4.15-4.09 (m, 2H), 3.60-3.53 (m, 2H), 3.50-3.44 (m, 4H), 3.08-2.99 (m, 1H), 2.70-2.67 (m, 4H), 2.42 (s, 3H), 2.28 (s, 3H), 1.92-1.80 (m, 2H), 1.75-1.69 (m, 2H), 1.65-1.59 (m, 1H), 0.50-0.42 (m, 4H).

LC/MS (Methode I, ESIpos): Rₜ = 0.93 min, m/z = 540 [M+H]⁺.

### Beispiel 82

### N-{4-[5-(1-{[2-(4-Cyclopropylpiperazin-1-yl)pyridin-4-yl]methyl}-5-methyl-1H-pyrazol-3-yl)-1,2,4-oxadiazol-3-yl]benzyl}-N-isopropylpropan-2-amin

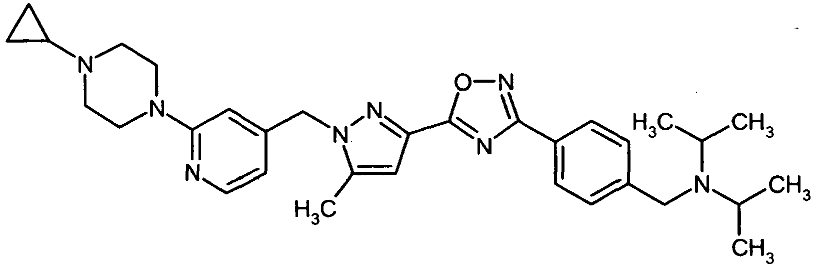

365 mg (1.00 mmol, Reinheit 93%) der Verbindung aus Beispiel 132A wurden mit 277 mg (1.10 mmol) der Verbindung aus Beispiel 135A in 10 ml THF vorgelegt. Man kühlte auf 0°C ab, gab 146 mg (1.30 mmol) Kalium-*tert*.-butylat hinzu und rührte das Gemisch zunächst 1 h bei RT und dann 24 h unter Rückfluss. Nach Abkühlen auf RT verdünnte man das Gemisch mit Ethylacetat, wusch einmal mit Wasser und extrahierte die wässrige Phase einmal mit Ethylacetat. Die vereinigten organischen Phasen wurden einmal mit gesättigter wässriger Natriumchlorid-Lösung gewaschen, über Magnesiumsulfat getrocknet, filtriert und eingeengt. Der Rückstand wurde mittels präparativer HPLC (Methode O) gereinigt. Die vereinigten Produktfraktionen wurden bis auf ein Restvolumen an Wasser eingeengt, mit gesättigter wässriger Natriumhydrogencarbonat-Lösung versetzt und zweimal mit Ethylacetat extrahiert. Die vereinigten Ethylacetat-Phasen wurden über Magnesiumsulfat getrocknet, filtriert und eingeengt. Der Rückstand wurde abschließend im Vakuum getrocknet. Es wurden 259 mg (47% d. Th.) der Titelverbindung erhalten.

¹H-NMR (400 MHz, CDCl₃, δ/ppm): 8.13-8.10 (m, 3H), 7.50 (d, 2H), 6.82 (s, 1H), 6.36-6.30 (m, 2H), 5.35 (s, 2H), 3.70 (s, 2H), 3.45 (s, breit, 4H), 3.10-3.00 (m, 2H), 2.69 (s, breit, 4H), 3.30 (s, 3H), 1.67-1.60 (m, 1H), 1.05 (d, 12H), 0.50-0.45 (m, 4H).

LC/MS (Methode D, ESIpos): Rₜ = 1.19 min, m/z = 555 [M+H]⁺.

### Beispiel 83

### 4-{4-[(5-Methyl-3-{3-[4-(trifluormethoxy)phenyl]-1,2,4-oxadiazol-5-yl}-1H-pyrazol-1-yl)methyl]-pyridin-2-yl morpholin

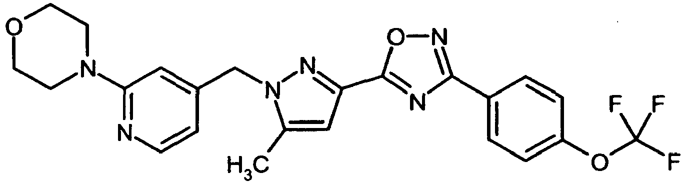

Analog zu dem unter Beispiel 56 beschriebenen Verfahren wurden aus 200 mg (0.459 mmol) der Verbindung aus Beispiel 81A und 2.1 ml (23.9 mmol) Morpholin 156 mg (69% d. Th., 98% Reinheit) der Titelverbindung erhalten. Das Rohprodukt wurde mittels präparativer HPLC (Methode N) gereinigt.

¹H-NMR (400 MHz, CDCl₃, δ/ppm): 8.26 (d, 2H), 8.14 (d, 1H), 7.33 (d, 2H), 6.83 (s, 1H), 6.40 (d, 1H), 6.31 (s, 1H), 5.37 (s, 2H), 3.78 (dd, 4H), 3.46 (dd, 4H), 2.30 (s, 3H).

LC/MS (Methode D, ESIpos): Rₜ = 2.25 min, m/z = 587 [M+H]⁺.

### Beispiel 84

### 1-{4-[(5-Methyl-3-{3-[4-(trifluormethoxy)phenyl]-1,2,4-oxadiazol-5-yl}-1H-pyrazol-1-yl)methyl]-pyridin-2-yl}piperidin-4-ol

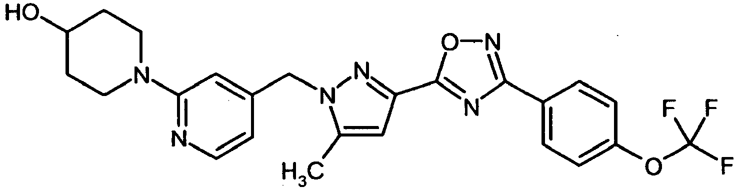

Analog zu dem unter Beispiel 56 beschriebenen Verfahren wurden aus 200 mg (0.459 mmol) der Verbindung aus Beispiel 81A und 464 mg (4.59 mmol) 4-Hydroxypiperidin 33 mg (14% d. Th.) der Titelverbindung erhalten. Das Rohprodukt wurde mittels präparativer HPLC (Methode N) gereinigt.

¹H-NMR (400 MHz, CDCl₃, δ/ppm): 8.25 (d, 2H), 8.12 (d, 1H), 7.33 (d, 2H), 6.83 (s, 1H), 6.36 (s, 1H), 6.32 (d, 1H), 5.34 (s, 2H), 4.02-3.96 (m, 2H), 3.94-3.88 (m, 1H), 3.17-3.10 (m, 2H), 2.29 (s, 3H), 1.98-1.91 (m, 2H), 1.59-1.51 (m, 2H).

LC/MS (Methode D, ESIpos): Rₜ = 2.25 min, m/z = 501 [M+H]⁺.

### Beispiel 85

### 1-{4-[(5-Methyl-3-{3-[4-(trifluormethoxy)phenyl]-1,2,4-oxadiazol-5-yl}-1H-pyrazol-1-yl)methyl]-pyridin-2-yl}piperidin-4-carbonitril

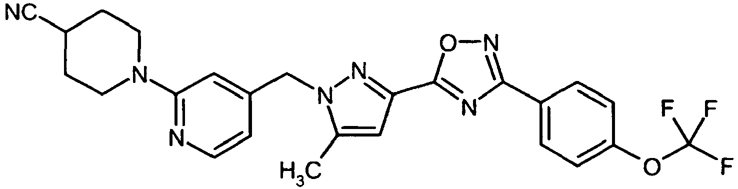

Ein Gemisch aus 120 mg (0.275 mmol) der Verbindung aus Beispiel 81A und 606 mg (5.51 mmol) 4-Cyanopiperidin wurde in einem Mikrowellenofen (CEM Discover, initiale Einstrahlleistung 250 W) 3 h lang bei 160°C gerührt. Anschließend wurde mit ca. 4 ml Methanol versetzt und das Reaktionsgemisch direkt mittels präparativer HPLC (Methode N) gereinigt. Die Produktfraktionen wurden vereinigt und am Rotationsverdampfer vom Lösungsmittel befreit. Der Rückstand wurde mit ca. 5 ml Cyclohexan/Ethylacetat (20:1) verrieben. Nach Trocknen im Hochvakuum wurden 103 mg (73% d. Th.) der Titelverbindung erhalten.

¹H-NMR (400 MHz, CDCl₃, δ/ppm): 8.25 (d, 2H), 8.13 (d, 1H), 7.33 (d, 2H), 6.84 (s, 1H), 6.38 (d, 1H), 6.34 (s, 1H), 5.35 (s, 2H), 3.80-3.73 (m, 2H), 3.48-3.41 (m, 2H), 2.88-2.82 (m, 1H), 2.29 (s, 3H), 2.00-1.93 (m, 2H), 1.92-1.83 (m, 2H).

LC/MS (Methode F, ESIpos): Rₜ = 1.28 min, m/z = 510 [M+H]⁺.

### Beispiel 86

### 1-Methyl-4-{4-[(5-methyl-3-{3-[4-(trifluormethoxy)phenyl]-1,2,4-oxadiazol-5-yl}-1H-pyrazol-1-yl)methyl]pyridin-2-yl}piperazin-2-on

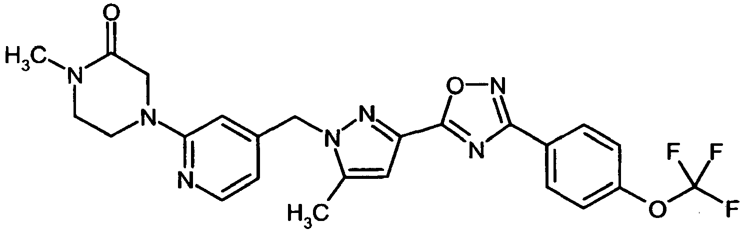

Analog zu dem unter Beispiel 56 beschriebenen Verfahren wurden aus 200 mg (0.459 mmol) der Verbindung aus Beispiel 81A und 524 mg (4.59 mmol) 1-Methylpiperazin-2-on [H. R. Buerki et al., Eur. J. Med. Chem. 1978 (13), 479-485] 154 mg (65% d. Th.) der Titelverbindung erhalten. Das Rohprodukt wurde mittels präparativer HPLC (Methode N) gereinigt.

¹H-NMR (400 MHz, CDCl₃, δ/ppm): 8.25 (d, 2H), 8.13 (d, 1H), 7.33 (d, 2H), 6.84 (s, 1H), 6.42 (d, 1H), 6.23 (s, 1H), 5.37 (s, 2H), 4.02 (s, 2H), 3.90 (dd, 2H), 3.43 (dd, 2H), 3.03 (s, 3H), 2.29 (s, 3H).

LC/MS (Methode F, ESIpos): Rₜ = 1.24 min, m/z = 514 [M+H]⁺.

### Beispiel 87

### 1-(4-{4-[(5-Methyl-3-{3-[4-(trifluormethoxy)phenyl]-1,2,4-oxadiazol-5-yl}-1H-pyrazol-1-yl)-methyl]pyridin-2-yl}piperazin-1-yl)ethanon

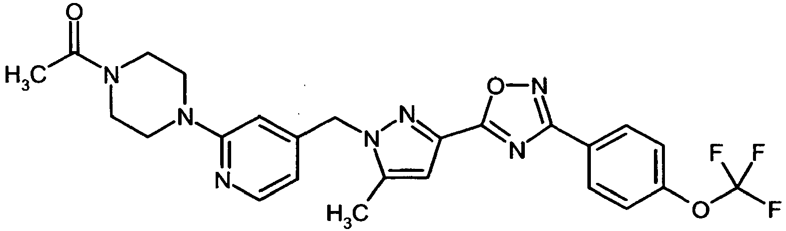

Eine Lösung von 300 mg (0.618 mmol) der Verbindung aus Beispiel 64 in 50 ml wasserfreiem Dichlormethan wurde bei 0°C mit 128 µl (0.927 mmol) Triethylamin und 44 µl (0.618 mmol) Acetylchlorid versetzt. Anschließend wurde das Reaktionsgemisch 16 h bei RT gerührt. Dann wurde mit 50 ml gesättigter wässriger Natriumhydrogencarbonat-Lösung versetzt. Nach Ausschütteln wurde die abgetrennte organische Phase mit Wasser gewaschen und anschließend über wasserfreiem Magnesiumsulfat getrocknet. Nach Filtration wurde das Lösungsmittel am Rotationsverdampfer entfernt. Das Rohprodukt wurde mittels präparativer HPLC (Methode N) gereinigt. Das erhaltene Produkt wurde mit 3 ml Ethanol verrührt. Nach Filtration und Trocknen im Hochvakuum wurden 234 mg (72% d. Th.) der Titelverbindung erhalten.

¹H-NMR (400 MHz, CDCl₃, δ/ppm): 8.25 (d, 2H), 8.14 (d, 1H), 7.33 (d, 2H), 6.85 (s, 1H), 6.40 (d, 1H), 6.33 (s, 1H), 5.37 (s, 2H), 3.73-3.70 (m, 2H), 2.60-2.53 (m, 4H), 3.49-3.46 (m, 2H), 2.30 (s, 3H), 2.13 (s, 3H).

LC/MS (Methode Q, ESIpos): Rₜ = 2.14 min, m/z = 528 [M+H]⁺.

### Beispiel 88

### 2-(4-Methylpiperidin-1-yl)-4-[(5-methyl-3-{3-[4-(trifluormethoxy)phenyl]-1,2,4-oxadiazol-5-yl}-1H-pyrazol-1-yl)methyl]pyridin

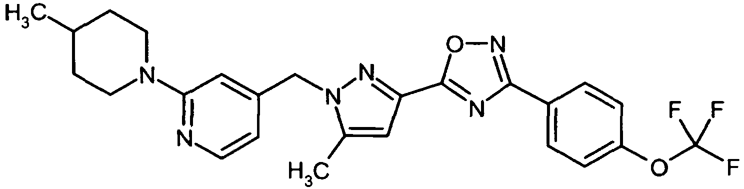

Analog zu dem unter Beispiel 56 beschriebenen Verfahren wurden aus 200 mg (0.459 mmol) der Verbindung aus Beispiel 81A und 2.8 ml (23.9 mmol) 4-Methylpiperidin 136 mg (59% d. Th.) der Titelverbindung erhalten. Das Rohprodukt wurde mittels präparativer HPLC (Methode N) gereinigt.

¹H-NMR (400 MHz, CDCl₃, δ/ppm): 8.25 (d, 2H), 8.10 (d, 1H), 7.33 (d, 2H), 6.83 (s, 1H), 6.33 (s, 1H), 6.28 (d, 1H), 5.33 (s, 2H), 4.22-4.15 (m, 2H), 2.82-2.74 (m, 2H), 2.29 (s, 3H), 1.72-1.67 (m, 2H), 1.63-1.53 (m, 1H), 1.22-1.13 (m, 2H), 0.95 (d, 3H).

LC/MS (Methode D, ESIpos): Rₜ = 2.27 min, m/z = 499 [M+H]⁺.

### Beispiel 89

### 1-(Cyclopropylmethyl)-4-{4-[(5-methyl-3-{3-[4-(trifluormethoxy)phenyl]-1,2,4-oxadiazol-5-yl}-1H-pyrazol-1-yl)methyl]pyridin-2-yl}piperazin

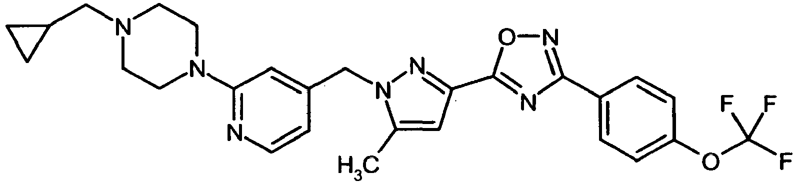

Analog zu dem unter Beispiel 73 beschriebenen Verfahren wurden aus 200 mg (0.412 mmol) der Verbindung aus Beispiel 64 und 37 µl (0.494 mmol) Cyclopropancarbaldehyd 52 mg (23% d. Th.) der Titelverbindung erhalten.

¹H-NMR (400 MHz, CDCl₃, δ/ppm): 8.25 (d, 2H), 8.12 (d, 1H), 7.33 (d, 2H), 6.83 (s, 1H), 6.33 (d, 1H), 6.32 (s, 1H), 5.35 (s, 2H), 3.52 (dd, 4H), 2.60 (dd, 4H), 2.29 (s, 3H), 2.28 (d, 2H), 0.93-0.83 (m, 1 H), 0.54-0.51 (m, 2H), 0.13-0.10 (m, 2H).

LC/MS (Methode I, ESIpos): Rₜ = 0.99 min, m/z = 540 [M+H]⁺.

### Beispiel 90

### 1-(4-{[5-Methyl-3-(3-{4-[1-(trifluormethyl)cyclopropyl]phenyl}-1,2,4-oxadiazol-5-yl)-1H-pyrazol-1-yl]methyl}pyridin-2-yl)piperazin

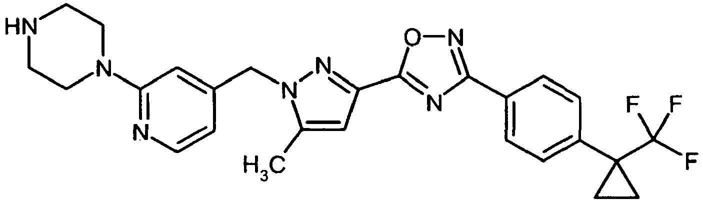

Ein Gemisch aus 175 mg (0.381 mmol) der Verbindung aus Beispiel 145A und 656 mg (7.61 mmol) Piperazin wurde über Nacht bei 150°C Badtemperatur unter Argon gerührt. Nach Abkühlen auf RT versetzte man mit Ethylacetat und Wasser und trennte die Phasen. Man extrahierte die wässrige Phase dreimal mit Ethylacetat, engte die vereinigten Ethylacetat-Phasen ein, trocknete den Rückstand im Vakuum und erhielt so 195 mg (98% d. Th., Reinheit 97%) der Titelverbindung.

LC/MS (Methode F, ESIpos): Rₜ = 1.10 min, m/z = 510 [M+H]⁺.

### Beispiel 91

### 1-Cyclopropyl-4-(4-{[5-methyl-3-(3-{4-[1-(trifluormethyl)cyclopropyl]phenyl}-1,2,4-oxadiazol-5-yl)-1H-pyrazol-1-yl]methyl}pyridin-2-yl)piperazin

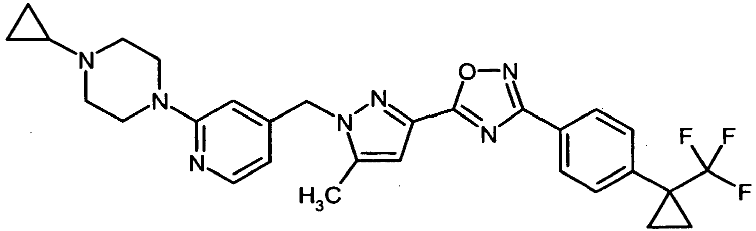

Man legte unter Argon 195 mg (0.371 mmol, Reinheit 97%) der Verbindung aus Beispiel 90 in einem Gemisch aus 55 ml Methanol und 213 µl (3.71 mmol) Essigsäure bei RT vor und gab 50 mg Molekularsieb (3Å) sowie 448 µl (2.23 mmol) [(1-Ethoxycyclopropyl)oxy](trimethyl)silan hinzu. Nach 10 min Rühren bei RT gab man 70 mg (1.11 mmol) Natriumcyanoborhydrid hinzu und erhitzte das Gemisch 2 h unter Rückfluss. Nach Abkühlen auf RT filtrierte man den vorhandenen Feststoff ab, wusch einmal mit Methanol nach und engte das Filtrat ein. Der Rückstand wurde mittels präparativer HPLC (Methode O) gereinigt. Die vereinigten Produktfraktionen wurden bis auf ein Restvolumen an wässriger Phase eingeengt. Man versetzte mit gesättigter wässriger Natriumhydrogencarbonat-Lösung und extrahierte zweimal mit Ethylacetat. Die vereinigten Ethylacetat-Phasen wurden über Magnesiumsulfat getrocknet, filtriert und eingeengt. Der Rückstand wurde aus Diethylether umkristallisiert. Nach Trocknen im Vakuum erhielt man 86 mg (42% d. Th.) der Titelverbindung.

¹H-NMR (400 MHz, CDCl₃, δ/ppm): 8.18 (d, 2H), 8.12 (d, 1H), 7.59 (d, 2H), 6.84 (s, 1H), 6.34 (s, 1H), 6.33 (s, 1H), 5.38 (s, 2H), 3.50-3.42 (m, 4H), 2.72-2.66 (m, 4H), 2.28 (s, 3H), 1.68-1.58 (m, 1H), 1.47-1.36 (m, 2H), 1.09 (s, 2H), 0.50-0.40 (m, 4H).

LC/MS (Methode F, ESIpos): Rₜ = 1.17 min, m/z = 550 [M+H]⁺.

### Beispiel 92

### 1-Cyclopropyl-4-(4-{[5-methyl-3-(3-{4-[N-methyl-S-(trifluormethyl)sulfonimidoyl)phenyl}-1,2,4-oxadiazol-5-yl)-1H-pyrazol-1-yl]methyl}pyridin-2-yl)piperazin

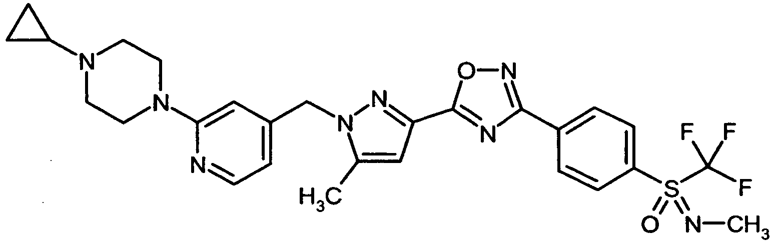

80 mg (0.215 mmol) der Verbindung aus Beispiel 130A wurden mit 54 mg (0.215 mmol) der Verbindung aus Beispiel 135A in 1 ml THF vorgelegt. Man kühlte auf 0°C ab, gab 31 mg (0.280 mmol) Kalium-*tert*.-butylat hinzu und rührte das Gemisch zunächst 1h bei RT und dann 24 h unter Rückfluss. Man engte danach ein und reinigte den Rückstand durch zweimalige präparative HPLC (Methode O). Die vereinigten Produktfraktionen wurden bis auf ein Restvolumen an Wasser eingeengt und mit gesättigter wässriger Natriumhydrogencarbonat-Lösung versetzt. Der entstandene Feststoff wurde abfiltriert, zweimal mit Wasser gewaschen und im Vakuum getrocknet. Man erhielt so 28 mg (22% d. Th.) der Titelverbindung.

¹H-NMR (400 MHz, CDCl₃, δ/ppm): 8.44 (s, breit, 2H), 8.30-8.00 (m, 3H), 6.86 (s, breit, 1H), 6.34 (s, breit, 2H), 5.36 (s, breit, 2H), 3.50 (s, breit, 4H), 3.12 (s, breit, 3H), 2.70 (s, breit, 4H), 2.30 (s, breit, 3H), 1.60 (s, breit, 1H), 0.50 (s, breit, 4H).

LC/MS (Methode I, ESIpos): Rₜ = 0.97 min, m/z = 587 [M+H]⁺.

### Beispiel 93

### 1-Cyclopropyl-4-{4-[(3-{3-[3-fluor-4-(trifluormethoxy)phenyl]-1,2,4-oxadiazol-5-yl}-5-methyl-1H-pyrazol-1-yl)methyl]pyridin-2-yl}piperazin

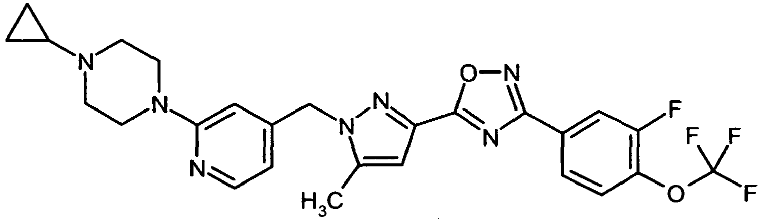

Zu einem Gemisch aus 357 mg (1.0 mmol, Reinheit 92%) der Verbindung aus Beispiel 131A und 277 mg (1.10 mmol) der Verbindung aus Beispiel 135A in 10 ml THF gab man 146 mg (1.30 mmol) Kalium-*tert*.-butylat hinzu und erhitzte über Nacht unter Rühren zum Rückfluss. Nach Abkühlen auf RT wurde mit Ethylacetat verdünnt und einmal mit Wasser gewaschen. Die wässrige Phase wurde einmal mit Ethylacetat extrahiert. Die vereinigten organischen Phasen wurden einmal mit gesättigter Natriumchlorid-Lösung gewaschen, über Magnesiumsulfat getrocknet, filtriert und eingeengt. Der Rückstand wurde mittels präparativer HPLC (Methode O) gereinigt. Die vereinigten Produktfraktionen wurden bis auf ein Restvolumen an wässriger Phase eingeengt, mit gesättigter wässriger Natriumhydrogencarbonat-Lösung versetzt und zweimal mit Ethylacetat extrahiert. Die vereinigten Ethylacetat-Phasen wurden über Magnesiumsulfat getrocknet, filtriert und eingeengt. Nach Trocknen des Rückstands im Vakuum wurden 269 mg (49% d. Th.) der Titelverbindung erhalten.

¹H-NMR (400 MHz, CDCl₃, δ/ppm): 8.11 (d, 1H), 8.10-8.01 (m, 2H), 7.46-7.41 (t, 1H), 6.83 (s, 1H), 6.32 (s, 2H), 5.35 (s, 2H), 3.49-3.44 (m, 4H), 2.71-2.66 (m, 4H), 2.29 (s, 3H), 1.68-1.60 (m, 1H), 0.50-0.40 (m, 4H).

LC/MS (Methode D, ESIpos): Rₜ = 1.97 min, m/z = 544 [M+H]⁺.

### Beispiel 94

### 4-[(5-Methyl-3-{3-[4-(trifluormethoxy)phenyl]-1,2,4-oxadiazol-5-yl}-1H-pyrazol-1-yl)methyl]-2-(pyrrolidin-1-yl)pyridin

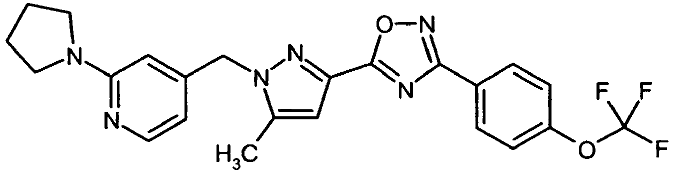

Analog zu dem unter Beispiel 56 beschriebenen Verfahren wurden aus 200 mg (0.459 mmol) der Verbindung aus Beispiel 81A und 770 µl (9.18 mmol) Pyrrolidin 63 mg (28% d. Th.) der Titelverbindung erhalten. Zur Aufarbeitung wurde das Reaktionsgemisch nach beendeter Reaktion zunächst am Rotationsverdampfer bis zur Trockene eingeengt und der Rückstand anschließend mit Acetonitril verrührt. Der dabei erhaltene Feststoff wurde abgetrennt. Das Produkt wurde aus dem Filtrat durch präparative HPLC (Methode N) isoliert.

¹H-NMR (400 MHz, CDCl₃, δ/ppm). 8.26 (d, 2H), 8.10 (d, 1H), 7.34 (d, 2H), 6.83 (s, 1H), 6.27 (d, 1H), 6.02 (s, 1H), 5.35 (s, 2H), 3.41-3.36 (m, 4H), 2.28 (s, 3H), 1.99-1.97 (m, 4H).

LC/MS (Methode I, ESIpos): Pₜ = 0.99 min, m/z = 471 [M+H]⁺.

### Beispiel 95

### 2-(Azetidin-1-yl)-4-[(5-methyl-3-{3-[4-(trifluormethoxy)phenyl]-1,2,4-oxadiazo1-5-yl}-1H-pyrazol-1-yl)methyl]pyridin

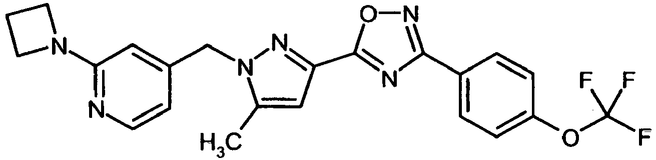

Analog zu dem unter Beispiel 16 beschriebenen Verfahren wurden 150 mg (0.344 mmol) der Verbindung aus Beispiel 81A und 232 µl (3.44 mmol) Azetidin zu 66 mg (42% d. Th.) der Titelverbindung umgesetzt.

¹H-NMR (400 MHz, CDCl₃, δ/ppm): 8.25 (d, 2H), 8.09 (d, 1H), 7.34 (d, 2H), 6.83 (s, 1H), 6.31 (d, 1H), 5.93 (s, 1H), 5.34 (s, 2H), 4.03-3.98 (m, 4H), 2.42-2.31 (m, 2H), 2.28 (s, 3H).

LC/MS (Methode I, ESIpos): Rₜ = 0.96 min, m/z = 457 [M+H]⁺.

### Beispiel 96

### 4-[(5-Methyl-3-{3-[4-(pyrrolidin-1-yl)phenyl]-1,2,4-oxadiazol-5-yl]-1H-pyrazol-1-yl)methyl]-2-(pyrrolidin-1-yl)pyridin

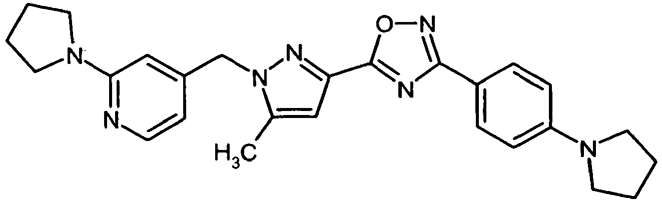

Analog zu dem unter Beispiel 56 beschriebenen Verfahren wurden aus 200 mg (0.459 mmol) der Verbindung aus Beispiel 81A und 770 µl (9.18 mmol) Pyrrolidin 89 mg (40% d. Th.) der Titelverbindung erhalten. Zur Aufarbeitung wurde das Reaktionsgemisch nach beendeter Reaktion zunächst am Rotationsverdampfer bis zur Trockene eingeengt und der Rückstand anschließend mit Acetonitril verrührt. Das Produkt blieb dabei ungelöst zurück, wurde abgetrennt und im Hochvakuum getrocknet.

¹H-NMR (400 MHz, CDCl₃, δ/ppm): 8.09 (d, 1H), 8.04 (d, 2H), 6.80 (s, 1H), 6.60 (d, 2H), 6.27 (d, 1H), 6.00 (s, 1H), 5.34 (s, 2H), 3.40-3.34 (m, 8H), 2.27 (s, 3H), 2.05-2.01 (m, 4H), 1.99-1.95 (m, 4H).

LC/MS (Methode I, ESIpos): Rₜ = 0.99 min, m/z = 456 [M+H]⁺.

### Beispiel 97

### 2-(4-tert.-Butylpiperidin-1-yl)-4-[(5-methyl-3-{3-[4-(trifluormethoxy)phenyl]-1,2,4-oxadiazol-5-yl}-1H-pyrazol-1-yl)methyl]pyridin

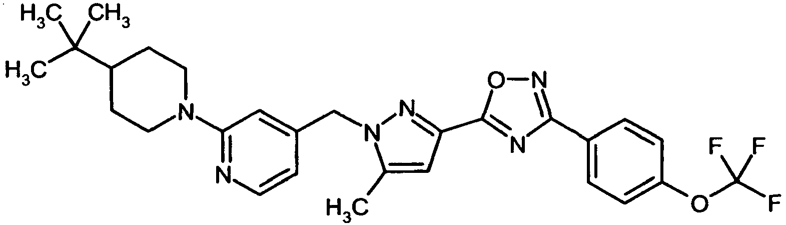

Analog zu dem unter Beispiel 56 beschriebenen Verfahren wurden aus 120 mg (0.275 mmol) der Verbindung aus Beispiel 81A und 778 mg (5.51 mmol) 4-*tert*.-Butylpiperidin 99 mg (65% d. Th., 98% Reinheit) der Titelverbindung erhalten. Das Produkt wurde mittels präparativer HPLC (Methode N) isoliert und abschließend mit Pentan/Diethylether (20:1) verrührt.

¹H-NMR (400 MHz, CDCl₃, δ/ppm): 8.25 (d, 2H), 8.11 (d, 1H), 7.33 (d, 2H), 6.83 (s, 1H), 6.33 (s, 1H), 6.28 (d, 1H), 5.33 (s, 2H), 4.31-4.26 (m, 2H), 2.72-2.65 (m, 2H), 2.28 (s, 3H), 1.77-1.72 (m, 2H), 1.31-1.14 (m, 3H), 0.86 (s, 9H).

LC/MS (Methode I, ESIpos): Rₜ = 1.33 min, m/z = 541 [M+H]⁺.

### Beispiel 98

### 1-{4-[(5-Methyl-3-{3-[4-(trifluormethoxy)phenyl]-1,2,4-oxadiazol-5-yl}-1H-pyrazol-1-yl)methyl]-pyridin-2-yl}-4-(2,2,2-trifluorethyl)-1,4-diazepan

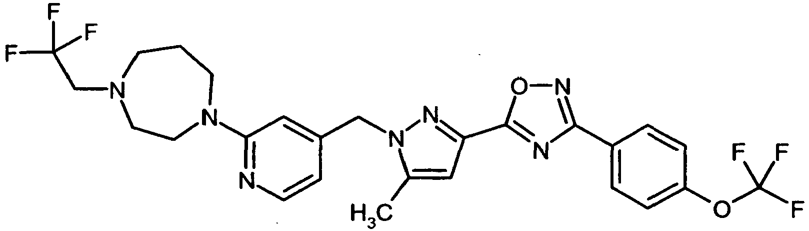

100 mg (0.23 mmol) der Verbindung aus Beispiel 81A und 209 mg (1.15 mmol) 1-(2,2,2-Trifluorethyl)-1,4-diazepan wurden 3 h lang in der Mikrowelle auf 160°C erhitzt. Nach dem Abkühlen auf RT wurde das Reaktionsgemisch direkt durch präparative HPLC (Methode P) gereinigt. Man engte die vereinigten Produktfraktionen am Rotationsverdampfer ein. Nach Trocknen des Rückstands im Vakuum wurden 92 mg (65% d. Th.) der Titelverbindung erhalten.

¹H-NMR (400 MHz, DMSO-d₆, δ/ppm): 8.20 (d, 2H), 8.00 (d, 1H), 7.59 (d, 2H), 6.95 (s, 1H), 6.44 (s, 1H), 6.20 (d, 1H), 5.43 (s, 2H), 3.67 (t, 2H), 3.57 (t, 2H), 3.27 (m, 2H), 2.92 (d, 2H), 2.75 (d, 2H), 2.33 (s, 3H), 1.80 (m, 2H).

LC/MS (Methode D, ESIpos): Rₜ = 2.26 min, m/z = 581 [M+H]⁺.

### Beispiel 99

### 2-(1-Cyclopropylpiperidin-4-yl)-4-[(5-methyl-3-{3-[4-(trifluormethoxy)phenyl]-1,2,4-oxadiazol-5-yl}-1H-pyrazol-1-yl)methyl]pyridin

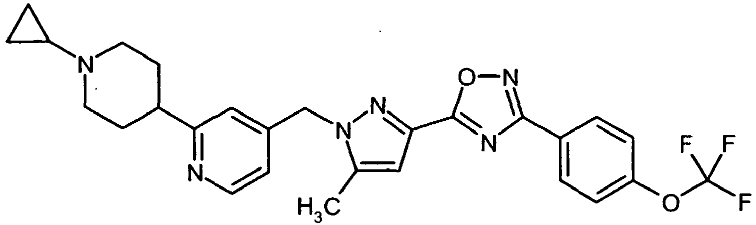

130 mg (0.250 mmol) der Verbindung aus Beispiel 63 wurden in ca. 10 ml Methanol gelöst und durch Perkolation über eine Hydrogencarbonat-Kartusche (Polymerlabs, Stratospheres SPE, PL-HCO₃ MP SPE, Kapazität 0.9 mmol) in die freie Base überführt. Nach dem Abdampfen des Lösungsmittels wurde erneut in 3.5 ml Methanol aufgenommen und mit 143 µl (2.49 mmol) Eisessig, 301 µl (1.50 mmol) 1-Ethoxy-1-(trimethylsilyloxy)cyclopropan sowie 40 mg getrocknetem, pulverisiertem Molekularsieb (3Å) versetzt. Nach 10 min Rühren bei RT wurden 47 mg (0.749 mmol) festes Natriumcyanoborhydrid hinzugefügt. Anschließend wurde das Reaktionsgemisch 4 h unter Rückfluss erhitzt. Nach dem Abkühlen auf RT wurde mit ca. 10 ml Dichlormethan verdünnt und vom Ungelösten abfiltriert. Das Filtrat wurde am Rotationsverdampfer bis zur Trockene eingeengt und der Rückstand anschließend in ca. 4 ml Methanol wieder in Lösung gebracht. Eine Vorreinigung des Produktes erfolgte mittels präparativer HPLC (Methode N). Die Produktfraktionen wurden vereinigt und vom Lösungsmittel befreit. Der erhaltene Rückstand wurde durch Perkolation über eine Hydrogencarbonat-Kartusche (Polymerlabs, Stratospheres SPE, PL-HCO₃ MP SPE, Kapazität 0.9 mmol) von aus der präparativen HPLC stammender Ameisensäure befreit. Eine abschließende Feinreinigung erfolgte mittels Chromatographie über Kieselgel (Laufmittel: Cyclohexan/Ethylacetat 10:1 → 1:1). Es wurden so 74 mg (54% d. Th., 96% Reinheit) der Titelverbindung erhalten.

¹H-NMR (400 MHz, CDCl₃, δ/ppm): 8.48 (d, 1H), 8.25 (d, 2H), 7.33 (d, 2H), 6.89 (s, 1H), 6.85 (s, 1H), 6.83 (d, 1H), 5.41 (s, 2H), 3.17-3.11 (m, 2H), 2.76-2.68 (m, 1H), 2.33-2.27 (m, 2H), 2.28 (s, 3H), 1.92-1.87 (m, 2H), 1.74-1.63 (m, 2H), 1.62-1.58 (m, 1H), 0.48-0.39 (m, 4H).

LC/MS (Methode I, ESIpos): Rₜ = 0.97 min, m/z = 525 [M+H]⁺.

### Beispiel 100

### {3-[(5-Methyl-3-{3-[4-(trifluormethoxy)phenyl]-1,2,4-oxadiazol-5-yl}-1H-pyrazol-1-yl)methyl]-phenyl}(1,2-oxazolidin-2-yl)methanon

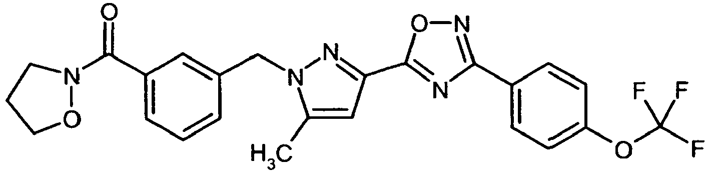

Analog zu dem unter Beispiel 32 beschriebenen Verfahren wurden aus 100 mg (0.225 mmol) der Verbindung aus Beispiel 93A und 49 mg (0.450 mmol) 1,2-Oxazolidin-Hydrochlorid 68 mg (60% d. Th.) der Titelverbindung erhalten. Abweichend von der genannten Vorschrift wurde hier ein weiteres Äquivalent *N,N-*Diisopropylethylamin als Base eingesetzt. Auf die abschließende Perkolation über eine Hydrogencarbonat-Kartusche wurde verzichtet.

¹H-NMR (400 MHz, CDCl₃, δ/ppm): 8.25 (d, 2H), 7.73 (d, 1H), 7.62 (s, 1H), 7.39 (t, 1H), 7.33 (d, 2H), 7.25 (d, 1H), 6.81 (s, 1H), 5.49 (s, 2H), 3.95 (t, 2H), 3.88 (t, 2H), 2.33 (quint, 2H), 2.29 (s, 3H).

HPLC (Methode A): Rₜ = 4.58 min.

MS (DCI, NH₃): m/z = 500 [M+H]⁺, 517 [M+NH₄]⁺.

LC/MS (Methode F, ESIpos): Rₜ = 1.39 min, m/z = 500 [M+H]⁺.

### Beispiel 101

### (4-Hydroxypiperidin-1-yl){3-[(5-methyl-3-{3-[4-(trifluormethoxy)phenyl]-1,2,4-oxadiazol-5-yl}-1H-pyrazol-1-yl)methyl]phenyl}methanon

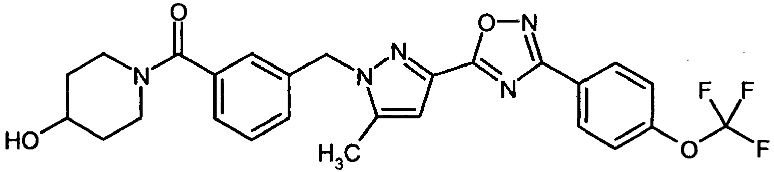

Analog zu dem unter Beispiel 32 beschriebenen Verfahren wurden aus 100 mg (0.225 mmol) der Verbindung aus Beispiel 93A und 46 mg (0.450 mmol) 4-Hydroxypiperidin 117 mg (98% d. Th.) der Titelverbindung erhalten. Auf die abschließende Perkolation über eine Hydrogencarbonat-Kartusche wurde hier verzichtet.

¹H-NMR (400 MHz, CDCl₃, δ/ppm): 8.25 (d, 2H), 7.39 (t, 1H), 7.33 (2d, 2H+1H), 7.20 (d, 1H), 7.19 (s, 1H), 6.82 (s, 1H), 5.48 (s, 2H), 4.15 (breit, 1H), 3.95 (breit, 1H), 3.59 (breit, 1H), 3.37 (breit, 1H), 3.14 (breit, 1H), 2.30 (s, 3H), 1.95 (breit, 1H), 1.79 (breit, 1H), 1.59 (breit, 2H), 1.45 (breit, 1H).

HPLC (Methode A): Rₜ = 4.41 min.

MS (DCI, NH₃): m/z = 528 [M+H]⁺, 545 [M+NH₄]⁺.

LC/MS (Methode I, ESIpos): Rₜ = 1.12 min, m/z = 528 [M+H]⁺.

### Beispiel 102

### {3-[(5-Methyl-3-{3-[4-(trifluormethoxy)phenyl]-1,2,4-oxadiazol-5-yl}-1H-pyrazol-1-yl)methyl]-phenyl}(morpholin-4-yl)methanon

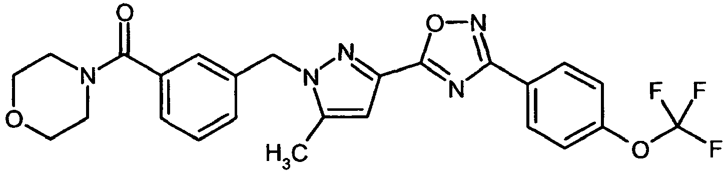

Analog zu dem unter Beispiel 32 beschriebenen Verfahren wurden aus 100 mg (0.225 mmol) der Verbindung aus Beispiel 93A und 40 µl (0.450 mmol) Morpholin 76 mg (66% d. Th.) der Titelverbindung erhalten. Auf die Reinigung durch präparative HPLC und die abschließende Perkolation über eine Hydrogencarbonat-Kartusche wurde in diesem Falle verzichtet; das Produkt fiel bei der Zugabe von Wasser zum Reaktionsgemisch aus, wurde abgesaugt und im Hochvakuum getrocknet.

¹H-NMR (400 MHz, CDCl₃, δ/ppm): 8.24 (d, 2H), 7.40 (t, 1H), 7.33 (2d, 2H+1H), 7.21 (d, 1H), 7.20 (s, 1H), 6.83 (s, 1H), 5.48 (s, 2H), 3.74 (breit, 4H), 3.60 (breit, 2H), 3.39 (breit, 2H), 2.31 (s, 3H).

HPLC (Methode A): Rₜ = 4.54 min.

MS (DCI, NH₃): m/z = 514 [M+H]⁺, 531 [M+NH₄]⁺.

LC/MS (Methode I, ESIpos): Rₜ = 1.19 min, m/z = 514 [M+H]⁺.

### Beispiel 103

### {3-[(5-Methyl-3-{3-[4-(trifluormethoxy)phenyl]-1,2,4-oxadiazol-5-yl}-1H-pyrazol-1-yl)methyl]-phenyl}(pyrrolidin-1-yl)methanon

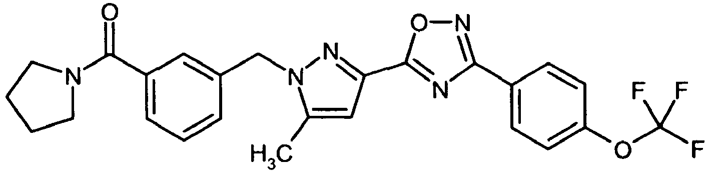

Analog zu dem unter Beispiel 32 beschriebenen Verfahren wurden aus 100 mg (0.225 mmol) der Verbindung aus Beispiel 93A und 38 µl (0.450 mmol) Pyrrolidin 72 mg (64% d. Th.) der Titelverbindung erhalten. Auf die abschließende Perkolation über eine Hydrogencarbonat-Kartusche wurde hier verzichtet.

¹H-NMR (400 MHz, CDCl₃, δ/ppm): 8.25 (d, 2H), 7.46 (d, 1H), 7.38 (t, 1H), 7.33 (d, 2H), 7.31 (s, 1H), 7.19 (d, 1H), 6.82 (s, 1H), 5.47 (s, 2H), 3.61 (t, 2H), 3.35 (t, 2H), 2.29 (s, 3H), 1.94 (quint, 2H), 1.85 (quint, 2H).

HPLC (Methode A): Rₜ = 4.68 min.

MS (ESIpos): m/z = 498 [M+H]⁺, 995 [2M+H]⁺.

LC/MS (Methode F, ESIpos): Rₜ = 1.43 min, m/z = 498 [M+H]⁺.

### Beispiel 104

### Azetidin-1-yl{3-[(5-methyl-3-{3-[4-(trifluormethoxy)phenyl]-1,2,4-oxadiazol-5-yl}-1H-pyrazol-1-yl)methyl]phenyl}methanon

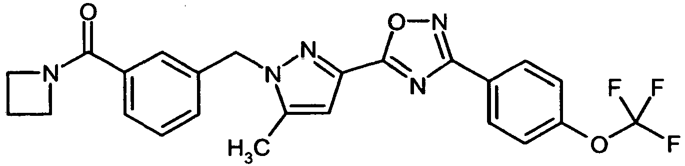

Analog zu dem unter Beispiel 32 beschriebenen Verfahren wurden aus 100 mg (0.225 mmol) der Verbindung aus Beispiel 93A und 79 µl (0.450 mmol) Azetidin 84 mg (78% d. Th.) der Titelverbindung erhalten. Auf die abschließende Perkolation über eine Hydrogencarbonat-Kartusche wurde hier verzichtet.

¹H-NMR (400 MHz, CDCl₃, δ/ppm): 8.25 (d, 2H), 7.55 (d, 1H), 7.45 (s, 1H), 7.39 (t, 1H), 7.32 (s, 2H), 7.24 (d, 1H), 6.82 (s, 1H), 5.48 (s, 2H), 4.24-4.18 (m, 4H), 2.32 (quint, 2H), 2.29 (s, 3H).

HPLC (Methode A): Rₜ = 4.62 min.

MS (DCI, NH₃): m/z = 484 [M+H]⁺, 501 [M+NH₄]⁺.

LC/MS (Methode F, ESIpos): R, = 1.39 min, m/z = 484 [M+H]⁺.

### Beispiel 105

### (3-Fluorazetidin-1-yl){3-[(5-methyl-3-{3-[4-(trifluormethoxy)phenyl]-1,2,4-oxadiazol-5-yl}-1H-pyrazol-1-yl)methyl]phenyl} methanon

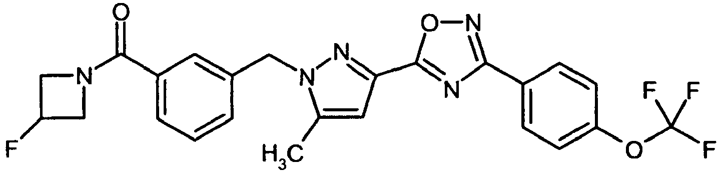

Analog zu dem unter Beispiel 32 beschriebenen Verfahren wurden aus 100 mg (0.225 mmol) der Verbindung aus Beispiel 93A und 50 mg (0.450 mmol) 3-Fluorazetidin-Hydrochlorid [B. Hulin et al., Bioorg. Med. Chem. Lett. 2005, 15 (21), 4770-4773] 95 mg (84% d. Th.) der Titelverbindung erhalten. Abweichend von der genannten Vorschrift wurde hier ein weiteres Äquivalent *N,N-*Diisopropylethylamin als Base eingesetzt. Auf die abschließende Perkolation über eine Hydrogencarbonat-Kartusche wurde verzichtet.

¹H-NMR (400 MHz, CDCl₃, δ/ppm): 8.24 (d, 2H), 7.74 (d, 1H), 7.47 (s, 1H), 7.41 (t, 1H), 7.34 (d, 2H), 7.29 (d, 1H), 6.83 (s, 1H), 5.48 (s, 2H), 5.41-5.22 (m, 1H), 4.50-4.39 (m, 2H), 4.38-4.24 (m, 2H), 2.30 (s, 3H).

HPLC (Methode A): Rₜ = 4.59 min.

MS (ESIpos): m/z = 502 [M+H]⁺, 1003 [2M+H]⁺.

LC/MS (Methode F, ESIpos): Rₜ = 1.40 min, m/z = 502 [M+H]⁺.

### Beispiel 106

### (3-Methoxyazetidin-1-yl){3-[(5-methyl-3-{3-[4-(trifluormethoxy)phenyl]-1,2,4-oxadiazol-5-yl}-1H-pyrazol-1-yl)methyl]phenyl}methanon

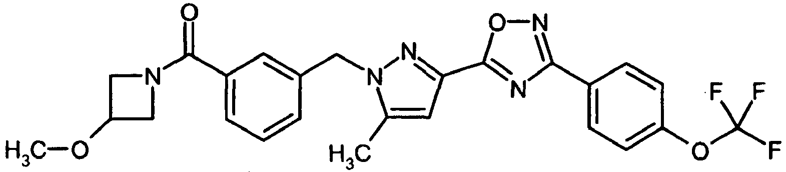

Analog zu dem unter Beispiel 32 beschriebenen Verfahren wurden aus 100 mg (0.225 mmol) der Verbindung aus Beispiel 93A und 56 mg (0.450 mmol) 3-Methoxyazetidin-Hydrochlorid [L. Provins et al., Bioorg. Med. Chem. Lett. 2007, 17 (11), 3077-3080] 72 mg (60% d. Th., 96% Reinheit) der Titelverbindung erhalten. Abweichend von der genannten Vorschrift wurde hier ein weiteres Äquivalent *N,N*-Diisopropylethylamin als Base eingesetzt. Auf die abschließende Perkolation über eine Hydrogencarbonat-Kartusche wurde verzichtet.

¹H-NMR (400 MHz, CDCl₃, δ/ppm): 8.25 (d, 2H), 7.55 (d, 1H), 7.44 (s, 1H), 7.39 (t, 1H), 7.33 (d, 2H), 7.25 (d, 1H), 6.83 (s, 1H), 5.48 (s, 2H), 4.37-4.29 (m, 2H), 4.22-4.17 (m, 1H), 4.10-4.01 (m, 2H), 3.26 (s, 3H), 2.30 (s, 3H).

HPLC (Methode A): Rₜ = 4.58 min.

MS (ESIpos): m/z = 514 [M+H]⁺, 1027 [2M+H]⁺.

LC/MS (Methode F, ESIpos): Fₜ = 1.38 min, m/z = 514 [M+H]⁺.

### Beispiel 107

### (3-Methylazetidin-1-yl){3-[(5-methyl-3-{3-[4-(trifluormethoxy)phenyl]-1,2,4-oxadiazol-5-yl}-1H-pyrazol-1-yl)methyl]phenyl}methanon

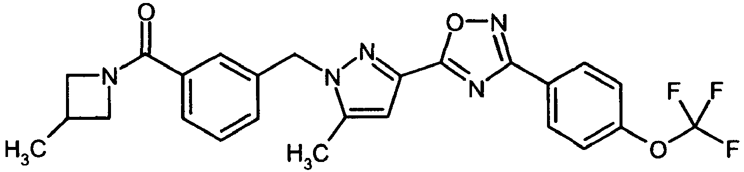

Analog zu dem unter Beispiel 32 beschriebenen Verfahren wurden aus 100 mg (0.225 mmol) der Verbindung aus Beispiel 93A und 48 mg (0.450 mmol) 3-Methylazetidin-Hydrochlorid [L. Provins *et al., Bioorg. Med. Chem. Lett.* 2007, *17 (11)*, 3077-3080] 72 mg (60% d. Th., 96% Reinheit) der Titelverbindung erhalten. Abweichend von der genannten Vorschrift wurde hier ein weiteres Äquivalent N,N-Diisopropylethylamin als Base eingesetzt. Auf die abschließende Perkolation über eine Hydrogencarbonat-Kartusche wurde verzichtet.

¹H-NMR (400 MHz, CDCl₃, δ/ppm): 8.25 (d, 2H), 7.55 (d, 1H), 7.45 (s, 1H), 7.38 (t, 1H), 7.33 (d, 2H), 7.23 (d, 1H), 6.82 (s, 1H), 5.48 (s, 2H), 4.32-4.27 (m, 2H), 3.79-3.71 (m, 2H), 2.79-2.69 (m, 1H), 2.29 (s, 3H), 1.23 (d, 3H).

LC/MS (Methode F, ESIpos): Rₜ = 1.45 min, m/z = 498 [M+H]⁺.

### Beispiel 108

### Ethyl-1-{4-[5-(5-methyl-1-{3-[(4-methylpiperazin-1-yl)carbonyl]benzyl}-1H-pyrazol-3-yl)-1,2,4-oxadiazol-3-yl]phenyl}cyclobutancarboxylat

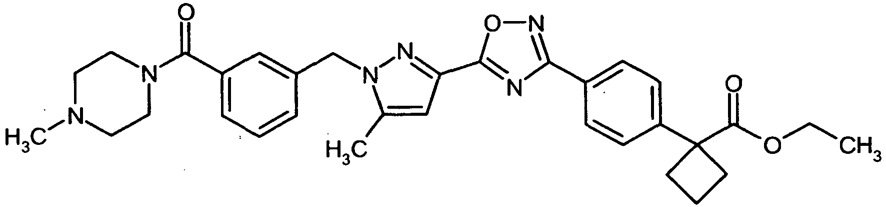

Eine Lösung von 155 mg (0.409 mmol) des Hydrochlorids der Verbindung aus Beispiel 105A in 2 ml wasserfreiem DMF wurde mit 86 mg (0.450 mmol) EDC und 69 mg (0.450 mmol) HOBt versetzt und 30 min bei RT gerührt. Dann wurde eine Lösung von 118 mg (0.450 mmol) der Verbindung aus Beispiel 109A in 2 ml wasserfreiem DMF hinzugefügt und das Rühren bei RT 15 h lang fortgesetzt. Nach dieser Zeit wurde der Reaktionsansatz in ein auf 140°C vorgeheiztes Ölbad getaucht und 1 h darin belassen. Nach dem Abkühlen auf RT wurde das Reaktionsgemisch direkt mittels präparativer HPLC (Methode N) in seine Komponenten aufgetrennt. Die Produktfraktionen wurden vereinigt und am Rotationsverdampfer bis zur Trockene eingeengt. Der erhaltene Rückstand wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Nach Einengen und Trocknen wurden 58 mg (24% d. Th., 95% Reinheit) der Titelverbindung erhalten.

¹H-NMR (400 MHz, CDCl₃, δ/ppm): 8.15 (d, 2H), 7.42 (d, 2H), 7.38 (t, 1H), 7.33 (d, 1H), 7.19 (d, 1H), 7.15 (s, 1H), 6.83 (s, 1H), 5.47 (s, 2H), 4.12 (quart, 2H), 3.76 (breit, 2H), 3.37 (breit, 2H), 2.91-2.83 (m, 2H), 2.58-2.50 (m, 2H), 2.43 (breit, 2H), 2.30 (s, 3H), 2.28 (breit, 2H), 2.24 (s, 3H), 2.13-2.02 (m, 1H), 1.94-1.85 (m, 1H), 1.17 (t, 3H).

LC/MS (Methode F, ESIpos): Rₜ = 1.13 min, m/z = 569 [M+H]⁺.

### Beispiel 109

### (4-Methylpiperazin-1-yl)(3-{[5-methyl-3-(3-{4-[(trifluormethyl)sulfanyl]phenyl}-1,2,4-oxadiazol-5-yl)-1H-pyrazol-1-yl]methyl}phenyl)methanon

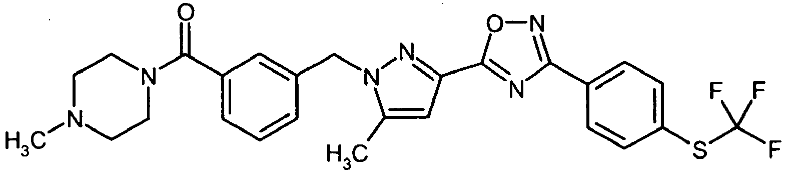

Eine Lösung von 160 mg (0.422 mmol) des Hydrochlorids der Verbindung aus Beispiel 105A in 2 ml wasserfreiem DMF wurde mit 89 mg (0.465 mmol) EDC, 71 mg (0.465 mmol) HOBt sowie 59 µl (0.422 mmol) Triethylamin versetzt und 30 min bei RT gerührt. Dann wurde eine Lösung von 120 mg (0.507 mmol) der Verbindung aus Beispiel 15A in 2 ml wasserfreiem DMF hinzugefügt und das Rühren bei RT 1 h lang fortgesetzt. Nach dieser Zeit wurde der Reaktionsansatz in ein auf 140°C vorgeheiztes Ölbad getaucht und 1 h darin belassen. Nach dem Abkühlen auf RT wurde das Reaktionsgemisch direkt mittels präparativer HPLC (Methode N) in seine Komponenten aufgetrennt. Die Produktfraktionen wurden vereinigt und am Rotationsverdampfer auf etwa die Hälfte des ursprünglichen Volumens eingeengt. Dann wurde durch Zusatz von festem Natriumhydrogencarbonat ein pH-Wert von ca. 8-9 eingestellt. Es wurde dreimal mit je ca. 20 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat und Filtration wurde das Lösungsmittel am Rotationsverdampfer entfernt. Es wurden 48 mg (21% d. Th., 95% Reinheit) der Titelverbindung erhalten.

¹H-NMR (400 MHz, DMSO-d₆, δ/ppm): 8.21 (d, 2H), 7.93 (d, 2H), 7.45 (t, 1H), 7.32 (d, 1H), 7.30 (d, 1H), 7.15 (s, 1H), 6.96 (s, 1H), 5.55 (s, 2H), 3.55 (breit, 2H), 3.24 (breit, 2H), 2.34 (s, 3H), 2.32 (breit, 2H), 2.18 (breit, 2H), 2.11 (s, 3H).

LC/MS (Methode D, ESIpos): Rₜ = 2.00 min, m/z = 543 [M+H]⁺.

### Beispiel 110

### (3-{[3-(3-{4-[1-(Methoxymethyl)cyclobutyl]phenyl}-1,2,4-oxadiazol-5-yl)-5-methyl-1H-pyrazol-1-yl]methyl}phenyl)(4-methylpiperazin-1-yl)methanon

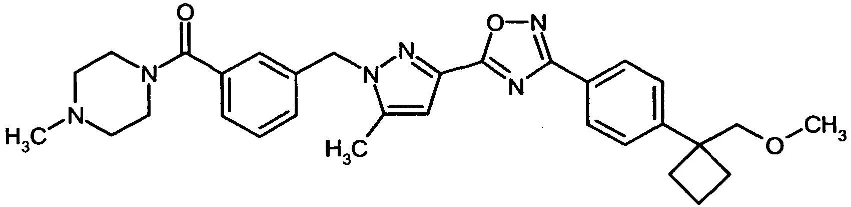

Eine Lösung von 180 mg (0.475 mmol) des Hydrochlorids der Verbindung aus Beispiel 105A in 3 ml wasserfreiem DMF wurde mit 100 mg (0.523 mmol) EDC und 80 mg (0.523 mmol) HOBt versetzt und 30 min bei RT gerührt. Dann wurde eine Lösung von 122 mg (0.523 mmol) der Verbindung aus Beispiel 110A in 2 ml wasserfreiem DMF hinzugefügt und das Rühren bei RT 15 h lang fortgesetzt. Nach dieser Zeit wurde der Reaktionsansatz in ein auf 140°C vorgeheiztes Ölbad getaucht und 30 min darin belassen. Nach dem Abkühlen auf RT wurde das Reaktionsgemisch direkt mittels präparativer HPLC (Methode N) in seine Komponenten aufgetrennt. Die Produktfraktionen wurden vereinigt und am Rotationsverdampfer bis zur Trockene eingeengt. Der erhaltene Rückstand wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Nach Einengen und Trocknen wurden 67 mg (25% d. Th., 95% Reinheit) der Titelverbindung erhalten.

¹H-NMR (400 MHz, CDCl₃, δ/ppm): 8.13 (d, 2H), 7.38 (t, 1H), 7.33 (d, 1H), 7.29 (d, 2H), 7.19 (d, 1H), 7.14 (s, 1H), 6.82 (s, 1H), 5.47 (s, 2H), 3.75 (breit, 2H), 3.54 (s, 2H), 3.36 (breit, 2H), 3.28 (s, 3H), 2.50-2.25 (m, 8H), 2.29 (s, 3H), 2.24 (s, 3H), 2.15-2.03 (m, 1H), 1.93-1.83 (m, 1H).

LC/MS (Methode I, ESIpos): Rₜ = 1.01 min, m/z = 541 [M+H]⁺.

### Beispiel 111

### {3-[(3-{3-[4-(1-Fluorcyclobutyl)phenyl]-1,2,4-oxadiazol-5-yl}-5-methyl-1H-pyrazol-1-yl)methyl]-phenyl}(4-methylpiperazin-1-yl)methanon

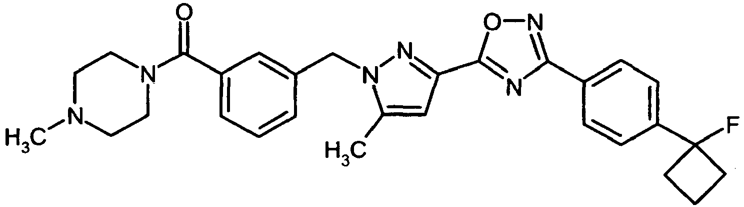

Analog zu dem unter Beispiel 41 beschriebenen Verfahren wurden aus 100 mg (0.292 mmol) der Verbindung aus Beispiel 105A und 73 mg (0.350 mmol) der Verbindung aus Beispiel 26A 57 mg (38% d. Th.) der Titelverbindung erhalten.

¹H-NMR (400 MHz, CDCl₃, δ/ppm): 8.23 (d, 2H), 7.59 (d, 2H), 7.39 (t, 1H), 7.34 (d, 1H), 7.20 (d, 1H), 7.16 (s, 1H), 6.84 (s, 1H), 5.48 (s, 2H), 3.76 (breit, 2H), 3.36 (breit, 2H), 2.77-2.55 (m, 4H), 2.44 (breit, 2H), 2.30 (s, 3H), 2.28 (breit, 2H), 2.25 (s, 3H), 2.19-2.07 (m, 1H), 1.87-1.75 (m, 1H).

HPLC (Methode A): Rₜ = 4.24 min.

MS (DCI, NH₃): m/z = 515 [M+H]⁺.

### Beispiel 112

### (4-Methylpiperazin-1-yl) {3-[(5-methyl-3-{3-[4-(1,1,1-trifluor-2-methoxypropan-2-yl)phenyl]-1,2,4-oxadiazol-5-yl}-1H-pyrazol-1-yl)methyl]phenyl}methanon (Racemat)

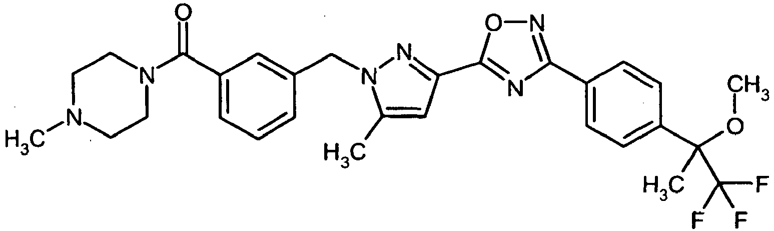

Analog zu dem unter Beispiel 110 beschriebenen Verfahren wurden 500 mg (1.32 mmol) des Hydrochlorids der Verbindung aus Beispiel 105A und 381 mg (1.45 mmol) der Verbindung aus Beispiel 111A zu 190 mg (24% d. Th., 95% Reinheit) der Titelverbindung umgesetzt.

¹H-NMR (400 MHz, CDCl₃, δ/ppm): 8.23 (d, 2H), 7.64 (d, 2H), 7.39 (t, 1H), 7.33 (d, 1H), 7.20 (d, 1H), 7.16 (s, 1H), 6.83 (s, 1H), 5.48 (s, 2H), 3.75 (breit, 2H), 3.36 (breit, 2H), 3.27 (s, 3H), 2.44 (breit, 2H), 2.30 (s, 3H), 2.29 (breit, 2H), 2.26 (s, 3H), 1.82 (s, 3H).

LC/MS (Methode I, ESIpos): Rₜ = 0.95 min, m/z = 569 [M+H]⁺.

### Beispiel 113

### (4-Methylpiperazin-1-yl){3-[(5-methyl-3-{3-[4-(1,1,1-trifluor-2-methoxypropan-2-yl)phenyl]-1,2,4-oxadiazol-5-yl}-1H-pyrazol-1-yl)methyl]phenyl}methanon (Enantiomer 1)

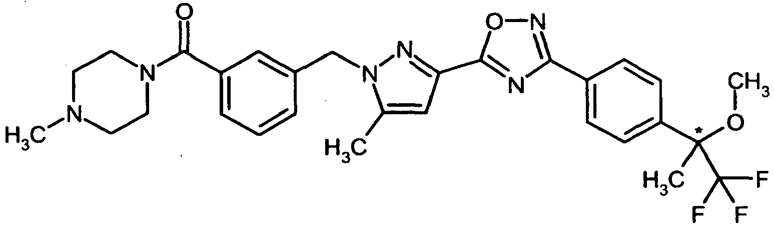

160 mg (0.281 mmol) der racemischen Verbindung aus Beispiel 112 wurden in einem Gemisch aus 4 ml Isopropanol und 11 ml Isohexan gelöst und durch Chromatographie an chiraler Phase in die Enantiomere aufgetrennt [Säulenmaterial: Daicel Chiralpak AD-H, 5 µm, 250 mm x 20 mm; Injektionsvolumen: 0.3 ml; Fluss: 15 ml/min; Temperatur: 40°C; UV-Detektion: 220 nm; Laufmittel: 50% Isohexan, 49.8% Isopropanol, 0.2% Diethylamin]. Es wurden 72 mg (90% d. Th., ee >98.5%) der Titelverbindung (Enantiomer 1) und 76 mg (95% d. Th., ee >99.0%) des anderen Enantiomers (Beispiel 114) erhalten.

Analytische HPLC [Daicel Chiracel AD-H, 5 µm, 250 mm x 4.6 mm; Laufmittel: 40% Isohexan, 59.8% Isopropanol, 0.2% Diethylamin; Fluss: 1 ml/min; Temperatur: 40°C]: Rₜ = 5.27 min.

### Beispiel 114

### (4-Methylpiperazin-1-yl) {3-[(5-methyl-3-{3-[4-(1,1,1-trifluor-2-methoxypropan-2-yl)phenyl]-1,2,4-oxadiazol-5-yl}-1H-pyrazol-1-yl)methyl]phenyl}methanon (Enantiomer 2)

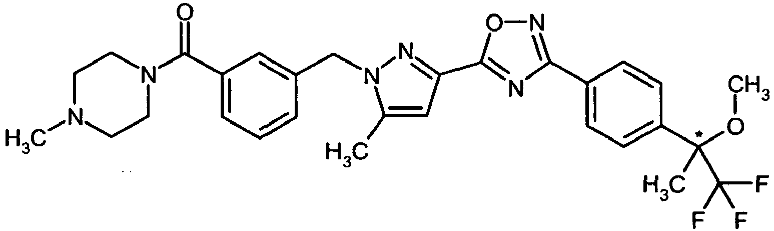

160 mg (0.281 mmol) der racemischen Verbindung aus Beispiel 112 wurden in einem Gemisch aus 4 ml Isopropanol und 11 ml Isohexan gelöst und durch Chromatographie an chiraler Phase in die Enantiomere aufgetrennt [Säulenmaterial: Daicel Chiralpak AD-H, 5 µm, 250 mm x 20 mm; Injektionsvolumen: 0.3 ml; Fluss: 15 ml/min; Temperatur: 40°C; UV-Detektion: 220 nm; Laufmittel: 50% Isohexan, 49.8% Isopropanol, 0.2% Diethylamin]. Es wurden 76 mg (95% d. Th., ee >99.0%) der Titelverbindung (Enantiomer 2) und 72 mg (90% d. Th., ee >98.5%) des anderen Enantiomers (Beispiel 113) erhalten.

Analytische HPLC [Daicel Chiracel AD-H, 5 µm, 250 mm x 4.6 mm; Laufmittel: 40% Isohexan, 59.8% Isopropanol, 0.2% Diethylamin; Fluss: 1 ml/min; Temperatur: 40°C]: Rₜ = 5.68 min.

### Beispiel 115

### {3-[(3-{3-[3-Fluor-4-(tetrahydro-2H-pyran-4-yl)phenyl]-1,2,4-oxadiazol-5-yl}-5-methyl-1H-pyrazol-1-yl)methyl]phenyl} (4-methylpiperazin-1-yl)methanon

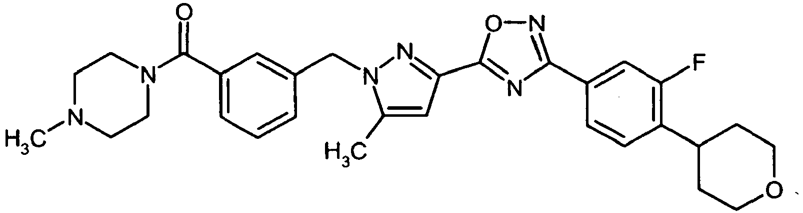

Analog zu dem unter Beispiel 110 beschriebenen Verfahren wurden 230 mg (0.607 mmol) des Hydrochlorids der Verbindung aus Beispiel 105A und 159 mg (0.668 mmol) der Verbindung aus Beispiel 112A zu 43 mg (13% d. Th., 98% Reinheit) der Titelverbindung umgesetzt.

¹H-NMR (400 MHz, CDCl₃, δ/ppm): 7.96 (d, 1H), 7.87 (d, 1H), 7.41-7.33 (m, 3H), 7.20 (d, 1H), 7.16 (s, 1H), 6.82 (s, 1H), 5.47 (s, 2H), 4.12-4.08 (m, 2H), 3.76 (breit, 2H), 3.61-3.54 (m, 2H), 3.36 (breit, 2H), 3.22-3.14 (m, 1H), 2.43 (breit, 2H), 2.30 (s, 3H), 2.28 (breit, 2H), 2.24 (s, 3H), 1.93-1.76 (m, 4H).

LC/MS (Methode I, ESIpos): Rₜ = 0.89 min, m/z = 545 [M+H]⁺.

### Beispiel 116

### [3-({3-[3-(4-Chlorphenyl)-1,2,4-oxadiazol-5-yl]-5-methyl-1H-pyrazol-1-yl}methyl)phenyl]-(4-methylpiperazin-1-yl)methanon

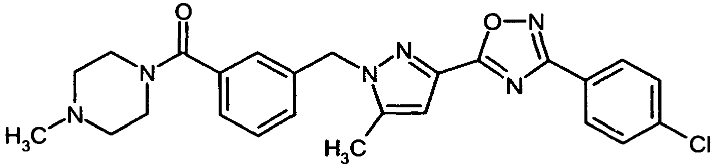

Analog zu dem unter Beispiel 109 beschriebenen Verfahren wurden aus 160 mg (0.422 mmol) des Hydrochlorids der Verbindung aus Beispiel 105A und 86 mg (0.507 mmol) 4-Chlor-*N*-hydroxy-benzamidin 40 mg (20% d. Th., 98% Reinheit) der Titelverbindung hergestellt. Nach der Reinigung durch präparative HPLC wurde das Produkt in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure zu entfernen.

¹H-NMR (400 MHz, DMSO-d₆, δ/ppm): 8.07 (d, 2H), 7.67 (d, 2H), 7.46 (t, 1H), 7.32 (d, 1H), 7.30 (d, 1H), 7.14 (s, 1H), 6.93 (s, 1H), 5.54 (s, 2H), 3.56 (breit, 2H), 3.23 (breit, 2H), 2.34 (s, 3H), 2.30 (breit, 2H), 2.18 (breit, 2H), 2.11 (s, 3H).

LC/MS (Methode D, ESIpos): R, = 1.82 min, m/z = 477 [M+H]⁺.

### Beispiel 117

### (4-Methylpiperazin-1-yl) {3-[(5-methyl-3-{3-[4-(tetrahydro-2H-pyran-4-yl)phenyl]-1,2,4-oxadiazol-5-yl}-1H-pyrazol-1-yl)methyl]phenyl} methanon

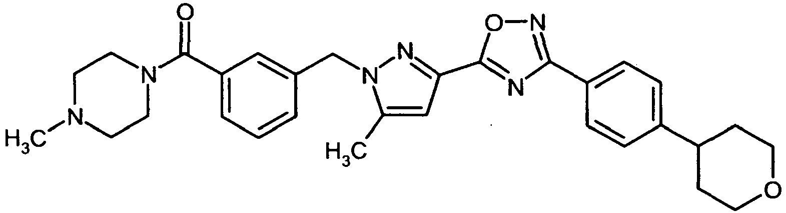

Analog zu dem unter Beispiel 110 beschriebenen Verfahren wurden aus 125 mg (0.330 mmol) des Hydrochlorids der Verbindung aus Beispiel 105A und 80 mg (0.363 mmol) der Verbindung aus Beispiel 113A 27 mg (15% d. Th., 96% Reinheit) der Titelverbindung erhalten.

¹H-NMR (400 MHz, CDCl₃, δ/ppm): 8.04 (d, 2H), 7.40-7.30 (m, 4H), 7.20 (d, 1H), 7.15 (s, 1H), 6.82 (s, 1H), 5.47 (s, 2H), 4.12-4.08 (m, 2H), 3.75 (breit, 2H), 3.58-3.52 (m, 2H), 3.36 (breit, 2H), 2.88-2.80 (m, 1H), 2.43 (breit, 2H), 2.30 (s, 3H), 2.28 (breit, 2H), 2.25 (s, 3H), 1.91-1.77 (m, 4H).

LC/MS (Methode F, ESIpos): Rₜ = 0.97 min, m/z = 527 [M+H]⁺.

### Beispiel 118

### (3-{[3-(3-{4-[1-(2-Fluorethyl)cyclobutyl]phenyl}-1,2,4-oxadiazol-5-yl)-5-methyl-1H-pyrazol-1-yl]methyl}phenyl)(4-methylpiperazin-1-yl)methanon

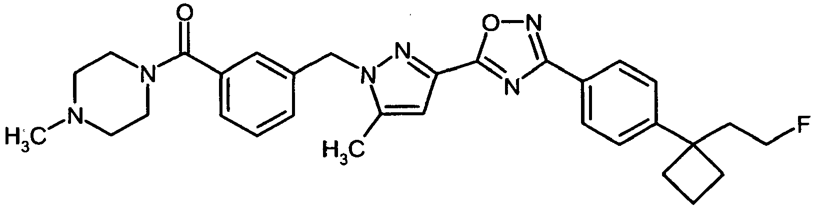

Analog zu dem unter Beispiel 110 beschriebenen Verfahren wurden aus 140 mg (0.370 mmol) des Hydrochlorids der Verbindung aus Beispiel 105A und 96 mg (0.406 mmol) der Verbindung aus Beispiel 113A 36 mg (17% d. Th., 95% Reinheit) der Titelverbindung erhalten.

¹H-NMR (400 MHz, CDCl₃, δ/ppm): 8.14 (d, 2H), 7.41-7.33 (m, 2H), 7.30-7.23 (m, 2H), 7.20 (d, 1H), 7.16 (s, 1H), 6.83 (s, 1H), 5.47 (s, 2H), 4.31 (td, 2H), 3.75 (breit, 2H), 3.37 (breit, 2H), 2.50-2.40 (m, 4H), 2.33-2.09 (m, 7H), 2.30 (s, 3H), 2.25 (s, 3H), 1.93-1.84 (m, 1H).

LC/MS (Methode I, ESIpos): Rₜ = 1.01 min, m/z = 543 [M+H]⁺.

### Beispiel 119

### (4-Methylpiperazin-1-yl) {3-[(5-methyl-3-{3-[3-(trifluormethoxy)phenyl]-1,2,4-oxadiazol-5-yl}-1H-pyrazol-1-yl)methyl]phenyl}methanon-Hydrochlorid

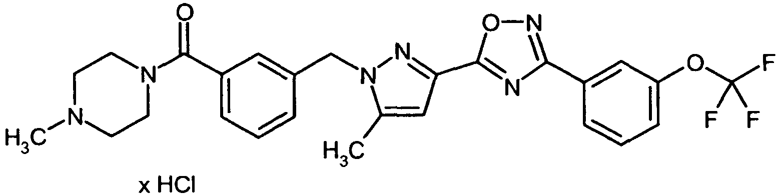

Eine Lösung von 160 mg (0.422 mmol) des Hydrochlorids der Verbindung aus Beispiel 105A in 2 ml wasserfreiem DMF wurde nacheinander mit 89 mg (0.465 mmol) EDC, 71 mg (0.465 mmol) HOBt und 59 µl (0.422 mmol) Triethylamin versetzt. Nach 30 min Rühren bei RT wurde eine Lösung von 112 mg (0.507 mmol) 3-Trifluormethoxy-*N*-hydroxybenzamidin in 2 ml wasserfreiem DMF hinzugefügt. Das Reaktionsgemisch wurde zunächst 1 h bei RT und dann 1 h bei 140°C gerührt. Nach dem Abkühlen auf RT wurde das Reaktionsgemisch direkt über präparative HPLC (Methode N) in seine Komponenten aufgetrennt. Die Produktfraktionen wurden vereinigt und zur Trockene eingedampft. Der erhaltene Rückstand wurde in ca. 3 ml Methanol gelöst und durch Perkolation über eine Hydrogencarbonat-Kartusche (Polymerlabs, Stratospheres SPE, PL-HCO₃ MP SPE, Kapazität 0.9 mmol) von anhaftender Ameisensäure befreit. Anschließend wurde das Produkt nochmals über eine Saugfiltration (Kieselgel, Laufmittel: Dichlormethan/Methanol 20:1) gereinigt. Nach Eindampfen der Produktfraktion wurde der Rückstand in ca. 2 ml Dichlormethan gelöst und mit ca. 5 ml einer 4 M Lösung von Chlorwasserstoff in Dioxan versetzt. Nach dem Eindampfen bis zur Trockene wurde erneut in Dichlormethan gelöst und abermals mit einer 4 M Lösung von Chlorwasserstoff in Dioxan versetzt. Nach neuerlichem Eindampfen und Trocknen im Hochvakuum wurden 61 mg (26% d. Th., 90% Reinheit) der Titelverbindung erhalten.

¹H-NMR (400 MHz, DMSO-d₆, δ/ppm): 10.38 (breit, 1H), 8.11 (d, 1H), 7.95 (s, 1H), 7.77 (t, 1H), 7.65 (d, 1H), 7.49 (t, 1H), 7.41 (d, 1H), 7.33 (d, 1H), 7.30 (s, 1H), 5.56 (s, 2H), 3.73-3.64 (m, 4H), 3.50-3.44 (m, 2H), 3.41-3.28 (m, 2H), 3.12-3.01 (m, 2H), 2.78 (s, 3H), 2.37 (s, 3H).

LC/MS (Methode D, ESIpos): Rₜ = 1.92 min, m/z = 527 [M+H]⁺.

### Beispiel 120

### {3-[(5-Methyl-3-{3-[4-(tetrahydro-2H-pyran-4-yl)phenyl]-1,2,4-oxadiazol-5-yl}-1H-pyrazol-1-yl)-methyl]phenyl}(morpholin-4-yl)methanon

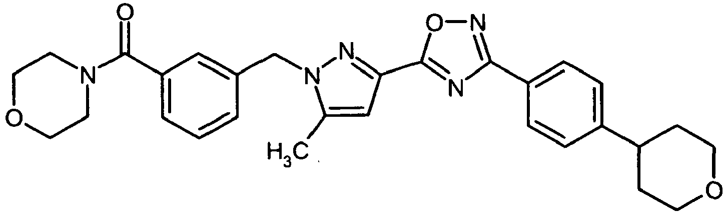

Analog zu dem unter Beispiel 32 beschriebenen Verfahren wurden aus 80 mg (0.180 mmol) der Verbindung aus Beispiel 147A und 31 µl (0.360 mmol) Morpholin 81 mg (88% d. Th.) der Titelverbindung erhalten. Auf die abschließende Perkolation über eine Hydrogencarbonat-Kartusche wurde hier verzichtet.

¹H-NMR (400 MHz, CDCl₃, δ/ppm): 8.14 (d, 2H), 7.41-7.32 (m, 4H), 7.21 (d, 1H), 7.20 (s, 1H), 6.83 (s, 1H), 5.47 (s, 2H), 4.12-4.08 (m, 2H), 3.80-3.70 (breit, 4H), 3.61 (breit, 2H), 3.54 (dt, 2H), 3.40 (breit, 2H), 2.87-2.79 (m, 1H), 2.30 (s, 3H), 1.92-1.78 (m, 4H).

LC/MS (Methode F, ESIpos): Rₜ = 1.20 min, m/z = 514 [M+H]⁺.

### Beispiel 121

### (4-Methylpiperazin-1-yl){3-[(5-methyl-3-{3-[4-(1,1,1-trifluor-2-hydroxypropan-2-yl)phenyl]-1,2,4-oxadiazol-5-yl}-1H-pyrazol-1-yl)methyl]phenyl}methanon (Racemat)

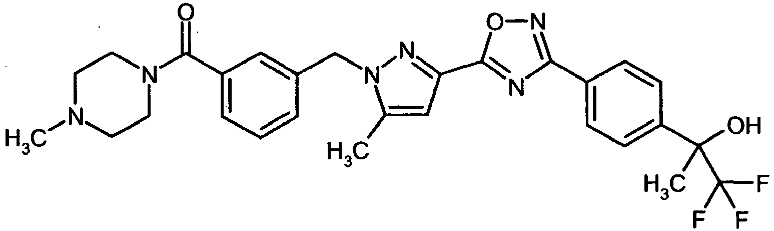

Analog zu dem unter Beispiel 110 beschriebenen Verfahren wurden 500 mg (1.32 mmol) des Hydrochlorids der Verbindung aus Beispiel 105A und 360 mg (1.45 mmol) der Verbindung aus Beispiel 114A zu 72 mg (10% d. Th., 97% Reinheit) der Titelverbindung umgesetzt. Abweichend von der genannten Vorschrift wurde das Reaktionsgemisch wie folgt aufgearbeitet: Zunächst wurde das Lösungsmittel DMF am Rotationsverdampfer weitestgehend entfernt. Der Rückstand wurde mit ca. 50 ml Wasser versetzt und dreimal mit je ca. 50 ml Ethylacetat extrahiert. Die vereinigten organischen Extrakte wurden mit gesättigter Kochsalz-Lösung gewaschen und über wasserfreiem Magnesiumsulfat getrocknet. Nach Filtration und Abdampfen des Lösungsmittels wurde das Rohprodukt mittels MPLC vorgereinigt (ca. 100 g Kieselgel, Laufmittel: Cyclohexan/Ethylacetat 1:1). Die Produktfraktionen wurden vereinigt, vom Lösungsmittel befreit und dann mittels präparativer HPLC, wie beschrieben, nachgereinigt.

¹H-NMR (400 MHz, CDCl₃, δ/ppm): 8.23 (d, 2H), 7.72 (d, 2H), 7.39 (t, 1H), 7.34 (d, 1H), 7.20 (d, 1H), 7.16 (s, 1H), 6.83 (s, 1H), 5.48 (s, 2H), 3.76 (breit, 2H), 3.37 (breit, 2H), 2.78 (breit, 1H), 2.43 (breit, 2H), 2.31 (s, 3H), 2.29 (breit, 2H), 2.24 (s, 3H), 1.82 (s, 3H).

LC/MS (Methode I, ESIpos): Rₜ = 0.85 min, m/z = 555 [M+H]⁺.

### Beispiel 122

### (4-Methylpiperazin-1-yl){3-[(5-methyl-3-{3-[4-(1,1,1-trifluor-2-hydroxypropan-2-yl)phenyl]-1,2,4-oxadiazol-5-yl}-1H-pyrazol-1-yl)methyl]phenyl}methanon (Enantiomer 1)

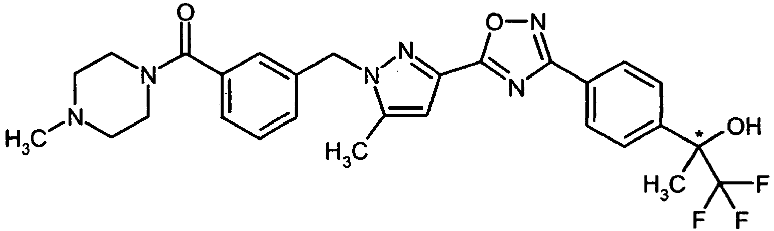

62 mg (0.108 mmol) der racemischen Verbindung aus Beispiel 121 wurden in 1 ml Ethanol gelöst und durch Chromatographie an chiraler Phase in die Enantiomere aufgetrennt [Säulenmaterial: Daicel Chiralpak AD-H, 5 µm, 250 mm x 20 mm; Injektionsvolumen: 0.5 ml; Fluss: 15 ml/min; Temperatur: 40°C; UV-Detektion: 220 nm; Laufmittel: 50% Isohexan, 49.8% Ethanol, 0.2% Diethylamin]. Es wurden 24 mg (80% d. Th., ee >99.5%) der Titelverbindung (Enantiomer 1) und 27 mg (90% d. Th., ee >99.5%) des anderen Enantiomers (Beispiel 123) erhalten.

Analytische HPLC [Daicel Chiralpak AD-H, 5 µm, 250 mm x 4.6 mm; Laufmittel: 40% Isohexan, 59.8% Ethanol, 0.2% Diethylamin; Fluss: 1 ml/min; Temperatur: 40°C]: Rₜ = 7.20 min.

### Beispiel 123

### (4-Methylpiperazin-1-yl){3-[(5-methyl-3-{3-[4-(1,1,1-trifluor-2-hydroxypropan-2-yl)phenyl]-1,2,4-oxadiazol-5-yl}-1H-pyrazol-1-yl)methyl]phenyl}methanon (Enantiomer 2)

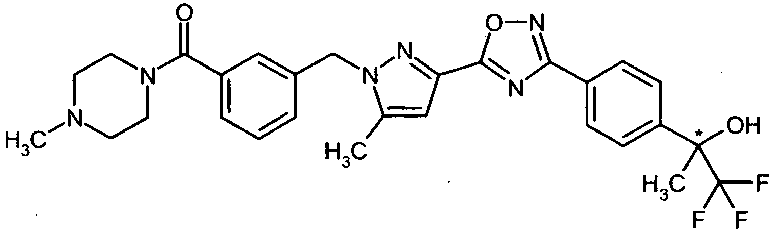

62 mg (0.108 mmol) der racemischen Verbindung aus Beispiel 121 wurden in 1 ml Ethanol gelöst und durch Chromatographie an chiraler Phase in die Enantiomere aufgetrennt [Säulenmaterial: Daicel Chiralpak AD-H, 5 µm, 250 mm x 20 mm; Injektionsvolumen: 0.5 ml; Fluss: 15 ml/min; Temperatur: 40°C; UV-Detektion: 220 nm; Laufmittel:: 50% Isohexan, 49.8% Ethanol, 0.2% Diethylamin]. Es wurden 27 mg (90% d. Th., ee >99.5%) der Titelverbindung (Enantiomer 2) und 24 mg (80% d. Th., ee >99.5%) des anderen Enantiomers (Beispiel 122) erhalten.

Analytische HPLC [Daicel Chiralpak AD-H, 5 µm, 250 mm x 4.6 mm; Laufmittel: 40% Isohexan, 59.8% Ethanol, 0.2% Diethylamin; Fluss: 1 ml/min; Temperatur: 40°C]: Rₜ = 9.26 min.

### Beispiel 124

### {3-[(3-{3-[4-(1-Methoxycyclobutyl)phenyl]-1,2,4-oxadiazol-5-yl}-5-methyl-1H-pyrazol-1-yl)-methyl]phenyl} (4-methylpiperazin-1-yl)methanon

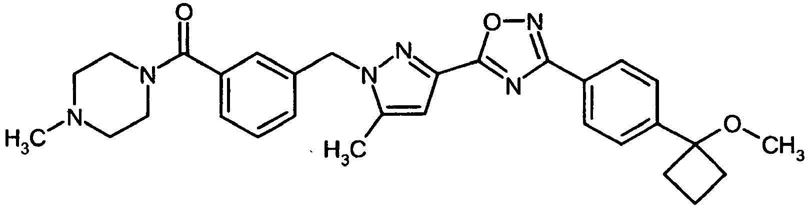

Analog zu dem unter Beispiel 41 beschriebenen Verfahren wurden aus 100 mg (0.292 mmol) der Verbindung aus Beispiel 105A und 77 mg (0.350 mmol) der Verbindung aus Beispiel 25A 66 mg (43% d. Th.) der Titelverbindung erhalten.

¹H-NMR (400 MHz, CDCl₃, δ/ppm): 8.21 (d, 2H), 7.56 (d, 2H), 7.39 (t, 1H), 7.33 (d, 1H), 7.20 (d, 1H), 7.17 (s, 1H), 6.83 (s, 1H), 5.48 (s, 2H), 3.76 (breit, 2H), 3.37 (breit, 2H), 2.97 (s, 3H), 2.47-2.38 (m, 6H), 2.30 (s, 3H), 2.27 (breit, 2H), 2.26 (s, 3H), 2.02-1.93 (m, 1H), 1.78-1.67 (m, 1H).

HPLC (Methode A): Rₜ = 4.14 min.

MS (DCI, NH₃): m/z = 527 [M+H]⁺.

### Beispiel 125

### [3-({5-Methyl-3-[3-(4-methylphenyl)-1,2,4-oxadiazol-5-yl]-1H-pyrazol-1-yl}methyl)phenyl]-(4-methylpiperazin-1-yl)methanon

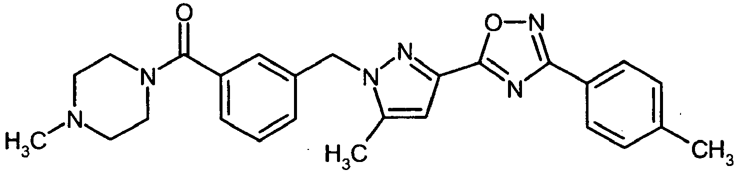

Analog zu dem unter Beispiel 119 beschriebenen Verfahren wurden aus 160 mg (0.422 mmol) des Hydrochlorids der Verbindung aus Beispiel 105A und 76 mg (0.507 mmol) 4-Methyl-*N*-hydroxybenzamidin 48 mg (25% d. Th., 97% Reinheit) der Titelverbindung erhalten. Abweichend von der genannten Vorschrift wurde hier nach der Perkolation über eine Hydrogencarbonat-Kartusche auf eine anschließende Kieselgel-Chromatographie sowie die Überführung in das korrespondierende Hydrochlorid verzichtet.

¹H-NMR (400 MHz, DMSO-d₆, δ/ppm): 7.96 (d, 2H), 7.46 (t, 1H), 7.40 (d, 2H), 7.32 (d, 1H), 7.30 (d, 1H), 7.13 (s, 1H), 6.93 (s, 1H), 5.54 (s, 2H), 3.56 (breit, 2H), 3.23 (breit, 2H), 2.40 (s, 3H), 2.34 (s, 3H), 2.30 (breit, 2H), 2.18 (breit, 2H), 2.11 (s, 3H).

LC/MS (Methode I, ESIpos): Rₜ = 0.87 min, m/z = 457 [M+H]⁺.

### Beispiel 126

### [3-({3-[3-(4-Fluorphenyl)-1,2,4-oxadiazol-5-yl]-5-methyl-1H-pyrazol-1-yl}methyl)phenyl]-(4-methylpiperazin-1-yl)methanon

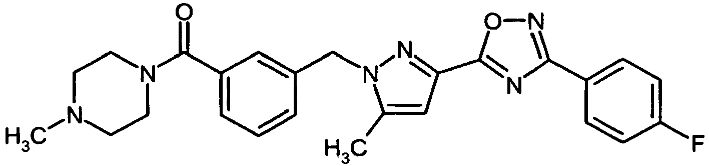

Analog zu dem unter Beispiel 119 beschriebenen Verfahren wurden aus 160 mg (0.422 mmol) des Hydrochlorids der Verbindung aus Beispiel 105A und 78 mg (0.507 mmol) 4-Fluor-*N*-hydroxybenzamidin 45 mg (23% d. Th., 98% Reinheit) der Titelverbindung erhalten. Abweichend von der genannten Vorschrift wurde hier nach der Perkolation über eine Hydrogencarbonat-Kartusche auf eine anschließende Kieselgel-Chromatographie sowie die Überführung in das korrespondierende Hydrochlorid verzichtet.

¹H-NMR (400 MHz, DMSO-d₆, δ/ppm): 8.12 (dd, 2H), 7.47-7.41 (m, 3H), 7.32 (d, 1H), 7.30 (d, 1H), 7.15 (s, 1H), 6.94 (s, 1H), 5.55 (s, 2H), 3.55 (breit, 2H), 3.26 (breit, 2H), 2.34 (s, 3H), 2.29 (breit, 2H), 2.21 (breit, 2H), 2.12 (s, 3H).

LC/MS (Methode I, ESIpos): Rₜ = 0.85 min, m/z = 461 [M+H]⁺.

### Beispiel 127

### (1-{4-[5-(5-Methyl-1-{3-[(4-methylpiperazin-1-yl)carbonyl]benzyl}-1H-pyrazol-3-yl)-1,2,4-oxa-diazol-3-yl]phenyl}cyclobutyl)methylacetat-Formiat

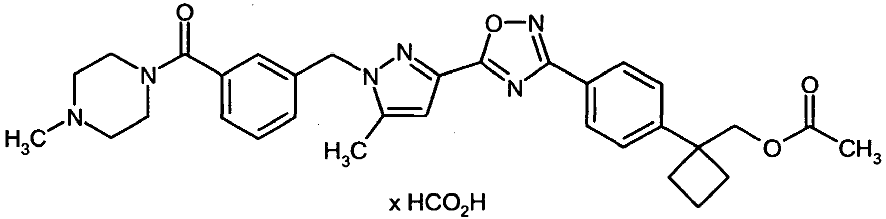

Analog zu dem unter Beispiel 110 beschriebenen Verfahren wurden aus 300 mg (0.792 mmol) des Hydrochlorids der Verbindung aus Beispiel 105A und 287 mg (0.871 mmol) der Verbindung aus Beispiel 115A 131 mg (26% d. Th., 95% Reinheit) der Titelverbindung erhalten. Eine abschließende Perkolation über eine Hydrogencarbonat-Kartusche wurde in diesem Falle nicht durchgeführt.

¹H-NMR (400 MHz, CDCl₃, δ/ppm): 8.18 (s, 1H), 8.13 (d, 2H), 7.40 (t, 1H), 7.34 (d, 1H), 7.28 (d, 2H), 7.23 (d, 1H), 7.18 (s, 1H), 6.83 (s, 1H), 5.97 (s, 2H), 4.28 (s, 2H), 3.86 (breit, 2H), 3.51 (breit, 2H), 2.69 (breit, 2H), 2.47-2.38 (m, 2H), 2.39 (s, 3H), 2.33-2.27 (m, 2H), 2.30 (s, 3H), 2.22-2.08 (m, 1H), 1.99 (s, 3H), 1.97-1.88 (m, 1H).

LC/MS (Methode D, ESIpos): Rₜ = 1.90 min, m/z = 569 [M+H]⁺.

### Beispiel 128

### (3-{[3-(3-{4-[1-(2-Hydroxyethyl)cyclobutyl]phenyl}-1,2,4-oxadiazol-5-yl)-5-methyl-1H-pyrazol-1-yl]methyl}phenyl)(4-methylpiperazin-1-yl)methanon

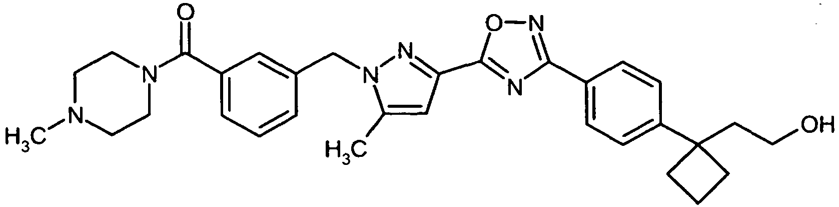

Eine Lösung von 200 mg (0.528 mmol) des Hydrochlorids der Verbindung aus Beispiel 105A in 5 ml wasserfreiem DMF wurde mit 111 mg (0.581 mmol) EDC, 89 mg (0.581 mmol) HOBt sowie 110 µl (0.792 mmol) Triethylamin versetzt und 30 min bei RT gerührt. Dann wurde eine Lösung von 160 mg (0.581 mmol) der Verbindung aus Beispiel 116A in 2 ml wasserfreiem DMF hinzugefügt und das Rühren bei RT 1 h lang fortgesetzt. Nach dieser Zeit wurde der Reaktionsansatz in ein auf 140°C vorgeheiztes Ölbad getaucht und 1 h darin belassen. Nach dem Abkühlen auf RT wurde das Lösungsmittel am Rotationsverdampfer weitestgehend abgezogen. Der erhaltene Rückstand wurde mit 50 ml Wasser versetzt und dreimal mit je ca. 50 ml Ethylacetat extrahiert. Die vereinigten organischen Extrakte wurden mit gesättigter Kochsalz-Lösung gewaschen und über wasserfreiem Magnesiumsulfat getrocknet. Nach Filtration wurde das Lösungsmittel am Rotationsverdampfer entfernt und der Rückstand mittels präparativer HPLC (Methode N) in seine Komponenten aufgetrennt. Es wurden leicht verunreinigte Fraktionen der Titelverbindung (13 mg) und des korrespondierenden Acetats (27 mg) erhalten. Die letztgenannte Fraktion wurde in 1 ml Ethanol gelöst und mit 100 µl 1 M Natronlauge versetzt. Nach 1 h Rühren bei RT wurde durch Zusatz von 90 µl 1 M Salzsäure neutralisiert und am Rotationsverdampfer bis zur Trockene eingeengt. Der Rückstand wurde mit den oben erhaltenen 13 mg der Produktfraktion vereinigt und anschließend mittels MPLC (Kieselgel, Laufmittel: Cyclohexan/Ethylacetat 20:1 → 1:1) nochmals gereinigt. Es wurden so 26 mg (9% d. Th., 93% Reinheit) der Titelverbindung erhalten.

¹H-NMR (400 MHz, CDCl₃, δ/ppm): 8.13 (d, 2H), 7.39 (t, 1H), 7.33 (d, 1H), 7.25 (d, 2H), 7.20 (d, 1H), 7.15 (s, 1H), 6.83 (s, 1H), 5.47 (s, 2H), 3.75 (breit, 2H), 3.47 (t, 2H), 3.36 (breit, 2H), 2.47-2.39 (m, 4H), 2.29 (s, 3H), 2.28 (breit, 2H), 2.25 (s, 3H), 2.24 (breit, 2H), 2.19-2.10 (m, 1H), 2.14 (t, 2H), 1.92-1.83 (m, 1H).

LC/MS (Methode I, ESIpos): Rₜ = 0.86 min, m/z = 541 [M+H]⁺.

### Beispiel 129

### (3- {[3-(3-{4-[1-(Hydroxymethyl)cyclobutyl]phenyl}-1,2,4-oxadiazol-5-yl)-5-methyl-1H-pyrazol-1-yl]methyl}phenyl)(4-methylpiperazin-1-yl)methanon

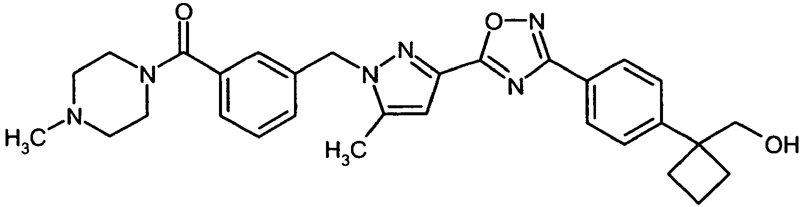

Eine Lösung von 125 mg (0.203 mmol) der Verbindung aus Beispiel 127 in 5 ml Ethanol wurde mit 0.5 ml 1 M Natronlauge versetzt und 30 min bei RT gerührt. Anschließend wurde das Reaktionsgemisch direkt mittels präparativer HPLC (Methode N) in seine Komponenten aufgetrennt. Die Produktfraktionen wurden vereinigt und am Rotationsverdampfer bis zur Trockene eingeengt. Der Rückstand wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Nach Einengen und Trocknen wurden 93 mg (85% d. Th.) der Titelverbindung erhalten.

¹H-NMR (400 MHz, CDCl₃, δ/ppm): 8.16 (d, 2H), 7.39 (t, 1H), 7.33 (d, 1H), 7.27 (d, 2H), 7.20 (d, 1H), 7.15 (s, 1H), 6.83 (s, 1H), 5.48 (s, 2H), 3.80 (d, 2H), 3.77 (breit, 2H), 3.35 (breit, 2H), 2.49-2.33 (m, 4H), 2.29 (s, 3H), 2.28 (breit, 4H), 2.25 (s, 3H), 2.17-2.06 (m, 1H), 1.97-1.88 (m, 1H), 1.29 (t, 1H).

LC/MS (Methode I, ESIpos): Rₜ = 0.84 min, m/z = 527 [M+H]⁺.

### Beispiel 130

### {3-[(5-Methyl-3-{3-[3-methyl-4-(tetrahydro-2H-pyran-4-yl)phenyl]-1,2,4-oxadiazol-5-yl}-1H-pyrazol-1-yl)methyl]phenyl} (4-methylpiperazin-1-yl)methanon

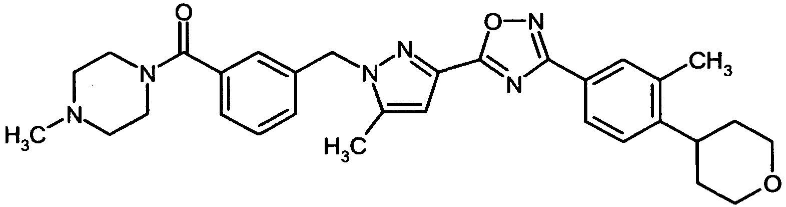

Analog zu dem unter Beispiel 110 beschriebenen Verfahren wurden 220 mg (0.581 mmol) des Hydrochlorids der Verbindung aus Beispiel 105A und 150 mg (0.639 mmol) der Verbindung aus Beispiel 108A zu 57 mg (17% d. Th.) der Titelverbindung umgesetzt.

¹H-NMR (400 MHz, CDCl₃, δ/ppm): 8.02-8.00 (m, 2H), 7.41-7.33 (m, 3H), 7.20 (d, 1H), 7.15 (s, 1H), 6.83 (s, 1H), 5.47 (s, 2H), 4.13-4.10 (m, 2H), 3.77 (breit, 2H), 3.60-3.54 (m, 2H), 3.37 (breit, 2H), 3.07-3.00 (m, 1H), 2.45 (breit, 2H), 2.43 (s, 3H), 2.30 (s, 3H), 2.29 (breit, 2H), 2.27 (s, 3H), 1.92-1.81 (m, 2H), 1.73-1.69 (m, 2H).

LC/MS (Methode I, ESIpos): Rₜ = 0.89 min, m/z = 541 [M+H]⁺.

### Beispiel 131

### N,N-Dimethyl-2-(1-{4-[5-(5-methyl-1-{3-[(4-methylpiperazin-1-yl)carbonyl]benzyl}-1H-pyrazol-3-yl)-1,2,4-oxadiazol-3-yl]phenyl}cyclobutyl)acetamid

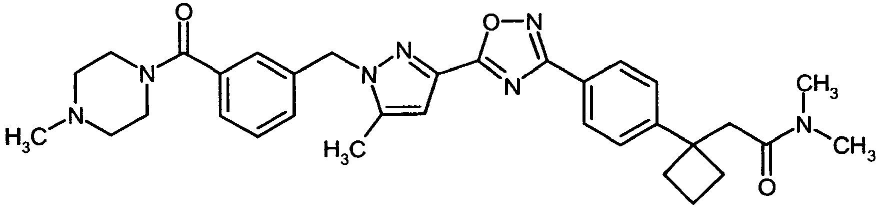

Analog zu dem unter Beispiel 121 beschriebenen Verfahren wurden 300 mg (0.792 mmol) des Hydrochlorids der Verbindung aus Beispiel 105A und 300 mg (0.871 mmol) der Verbindung aus Beispiel 117A zu 68 mg (15% d. Th.) der Titelverbindung umgesetzt. Nach Perkolation über eine Hydrogencarbonat-Kartusche zur Entfernung anhaftender Ameisensäure aus der HPLC-Reinigung wurde das Produkt abschließend durch Verrühren mit Ethanol gereinigt.

¹H-NMR (400 MHz, CDCl₃, δ/ppm): 8.11 (d, 2H), 7.38 (t, 1H), 7.33 (d, 1H), 7.31 (d, 2H), 7.20 (d, 1H), 7.15 (s, 1H), 6.82 (s, 1H), 5.47 (s, 2H), 3.76 (breit, 2H), 3.36 (breit, 2H), 2.83 (s, 2H), 2.73 (s, 3H), 2.60-2.52 (m, 2H), 2.51-2.40 (m, 4H), 2.35 (s, 3H), 2.30 (s, 3H), 2.28 (breit, 2H), 2.25 (s, 3H), 2.22-2.10 (m, 1H), 1.93-1.83 (m, 1H).

LC/MS (Methode I, ESIpos): Rₜ = 0.85 min, m/z = 582 [M+H]⁺.

### Beispiel 132

### {3-{[3-(3-{4-[(Diisopropylamino)methyl]phenyl}-1,2,4-oxadiazol-5-yl)-5-methyl-1H-pyrazol-1-yl]methyl} phenyl)(4-methylpiperazin-1-yl)methanon

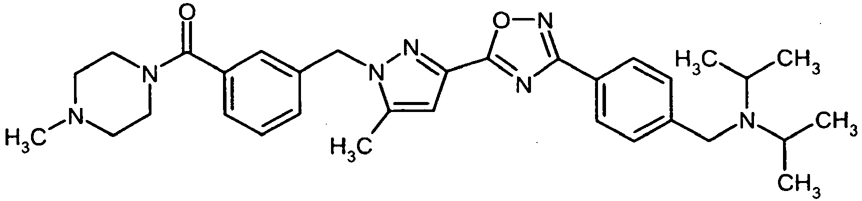

Analog zu dem unter Beispiel 41 beschriebenen Verfahren wurden 100 mg (0.292 mmol) der Verbindung aus Beispiel 105A und 73 mg (0.292 mmol) der Verbindung aus Beispiel 118A zu 45 mg (28% d. Th.) der Titelverbindung umgesetzt. Vor der Aufreinigung des Reaktionsgemisches durch präparative HPLC (Methode O) wurde das im Gemisch enthaltene DMSO über eine Gefriertrocknung entfernt.

¹H-NMR (400 MHz, CDCl₃, δ/ppm): 8.03 (d, 2H), 7.50-7.40 (m, 2H), 7.33-7.23 (m, 2H), 7.12 (d, 1H), 7.08 (s, 1H), 6.75 (s, 1H), 5.40 (s, 2H), 3.75-3.60 (m, breit, 4H), 3.35-3.25 (m, breit, 2H), 3.06-2.93 (m, breit, 2H), 2.42-2.30 (m, breit, 2H), 2.30-2.18 (m, breit, 2H), 2.22 (s, 3H), 2.20 (s, 3H), 1.08-0.89 (m, 12H).

LC/MS (Methode I, ESIpos): Rₜ = 0.62 min, m/z = 556 [M+H]⁺.

### Beispiel 133

### (4-Methylpiperazin-1-yl){3-[(5-methyl-3-{3-[4-(trifluormethyl)phenyl]-1,2,4-oxadiazol-5-yl}-1H-pyrazol-1-yl)methyl]phenyl}methanon

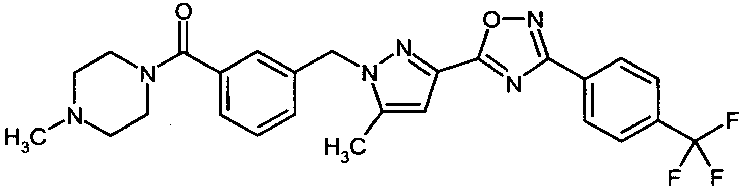

Analog zu dem unter Beispiel 41 beschriebenen Verfahren wurden 100 mg (0.292 mmol) der Verbindung aus Beispiel 105A und 60 mg (0.292 mmol) *N*'-Hydroxy-4-(trifluormethyl)benzolcarbox-imidamid zu 56 mg (38% d. Th.) der Titelverbindung umgesetzt. Vor der Aufreinigung des Reaktionsgemisches durch präparative HPLC (Methode O) wurde das im Gemisch enthaltene DMSO über eine Gefriertrocknung entfernt.

¹H-NMR (400 MHz, CDCl₃, δ/ppm): 8.32 (d, 2H), 7.77 (d, 2H), 7.41-7.33 (m, 2H), 7.22-7.16 (m, 2H), 6.83 (s, 1H), 5.49 (s, 2H), 3.77 (s, breit, 2H), 3.37 (s, breit, 2H), 2.43 (s, breit, 2H), 2.31 (s, 3H), 2.33-2.23 (m, 2H), 2.28 (s, 3H).

LC/MS (Methode I, ESIpos): Rₜ = 0.93 min, m/z = 511 [M+H]⁺.

### Beispiel 134

### (4-Methylpiperazin-1-yl){3-[(5-methyl-3-{3-[4-(trimethylsilyl)phenyl]-1,2,4-oxadiazol-5-yl}-1H-pyrazol-1-yl)methyl]phenyl}methanon

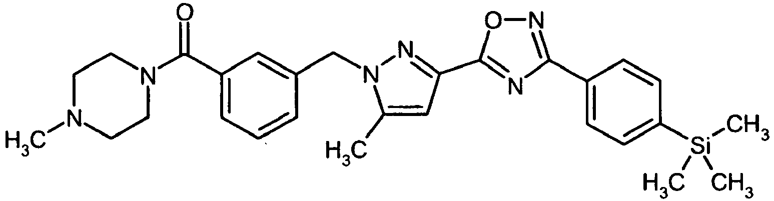

Man legte 100 mg (0.292 mmol) der Verbindung aus Beispiel 105A in 3 ml Dichlormethan bei 0°C vor, gab einen Tropfen DMF hinzu und tropfte anschließend 76 µl (0.876 mmol) Oxalylchlorid hinzu. Man rührte 1 h bei RT, engte danach ein und trocknete den Rückstand im Vakuum. Der Rückstand wurde dann in 2 ml Dichlormethan gelöst und bei 0°C zu einem Gemisch aus 68 mg (0.292 mmol, Reinheit 90%) der Verbindung aus Beispiel 17A und 81 µl (0.584 mmol) Triethylamin in 1 ml Dichlormethan gegeben. Man rührte 1 h bei RT, engte wiederum ein und trocknete den Rückstand im Vakuum. Der Rückstand wurde anschließend in 3 ml DMSO gelöst und 30 min lang in einem Mikrowellengerät (CEM Discover, initiale Einstrahlleistung 250 W) auf 120°C erhitzt. Nach dem Abkühlen auf RT wurde das Reaktionsgemisch direkt mittels präparativer HPLC (Methode O) gereinigt. Die Produktfraktionen wurden vereinigt und bis auf ein Restvolumen an Wasser eingeengt. Man versetzte mit gesättigter wässriger Natriumhydrogencarbonat-Lösung und extrahierte zweimal mit Ethylacetat. Die vereinigten Ethylacetat-Phasen wurden über Magnesiumsulfat getrocknet, filtriert und eingeengt. Nach Trocknen des Rückstands im Vakuum wurden 76 mg (50% d. Th.) der Titelverbindung erhalten.

¹H-NMR (400 MHz, CDCl₃, δ/ppm): 8.18 (d, 2H), 7.65 (d, 2H), 7.41-7.32 (m, 2H), 7.21 (d, 1H), 7.17 (s, 1H), 6.82 (s, 1H), 5.49 (s, 2H), 3.78 (s, breit, 2H), 3.38 (s, breit, 2H), 2.45 (s, breit, 2H), 2.31 (s, 3H), 2.33-2.23 (m, 2H), 2.28 (s, 3H), 0.31 (s, 9H).

LC/MS (Methode I, ESIpos): Rₜ = 1.03 min, m/z = 515 [M+H]⁺.

### Beispiel 135

### (3-{[5-Methyl-3-(3-{4-[N-methyl-S-(trifluormethyl)sulfonimidoyl]phenyl}-1,2,4-oxadiazol-5-yl)-1H-pyrazol-1-yl]methyl}phenyl)(4-methylpiperazin-1-yl)methanon (Racemat)

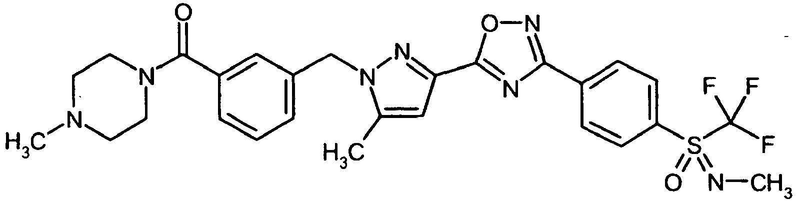

Man legte 162 mg (0.427 mmol) der Verbindung aus Beispiel 105A in 4 ml Dichlormethan vor, gab 0.74 ml (8.53 mmol) Oxalylchlorid hinzu und rührte 1 h bei RT. Das Gemisch wurde danach eingeengt und der Rückstand im Vakuum getrocknet. Anschließend löste man den Rückstand in 2 ml Dichlormethan, gab eine Lösung von 120 mg (0.427 mmol) der Verbindung aus Beispiel 119A und 0.18 ml (1.28 mmol) Triethylamin in 1 ml Dichlormethan hinzu und rührte das Gemisch 1 h bei RT. Das Gemisch wurde dann eingeengt und der Rückstand im Vakuum getrocknet. Der Rückstand wurde anschließend in 3 ml DMSO gelöst und 1.5 h unter Rühren auf 120°C erhitzt. Nach dem Abkühlen auf RT wurde das Reaktionsgemisch direkt mittels präparativer HPLC (Methode O) gereinigt. Die vereinigten Produktfraktionen wurden bis auf ein kleines Restvolumen an wässriger Phase eingeengt, mit gesättigter wässriger Natriumhydrogencarbonat-Lösung versetzt und dreimal mit Ethylacetat extrahiert. Die vereinigten Ethylacetat-Phasen wurden über Natriumsulfat getrocknet, filtriert und eingeengt. Nach Trocknen des Rückstands wurden 48 mg (19% d. Th.) der Titelverbindung erhalten.

¹H-NMR (400 MHz, CDCl₃, δ/ppm): 8.44 (d, 2H), 8.21 (d, 2H), 7.44-7.30 (m, 2H), 7.23-7.13 (m, 2H), 6.85 (s, 1H), 5.48 (s, 2H), 3.76 (s, breit, 2H), 3.37 (s, breit, 2H), 3.12 (s, 3H), 2.50-2.36 (m, 2H), 2.35-2.25 (m, breit, 2H), 2.30 (s, 3H), 2.27 (s, 3H).

LC/MS (Methode I, ESIpos): Rₜ = 0.94 min, m/z = 588 [M+H]⁺.

### Beispiel 136

### {3-[(3-{3-[3-Chlor-4-(trifluormethoxy)phenyl]-1,2,4-oxadiazol-5-yl}-5-methyl-1H-pyrazol-1-yl)-methyl]phenyl}(4-methylpiperazin-1-yl)methanon

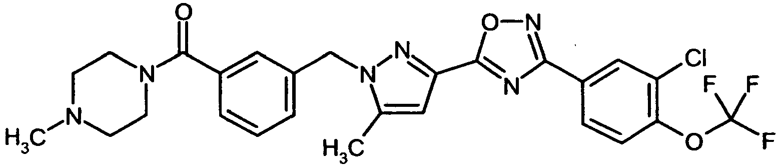

Analog zu dem unter Beispiel 41 beschriebenen Verfahren wurden 200 mg (0.528 mmol) der Verbindung aus Beispiel 105A und 134 mg (0.528 mmol) der Verbindung aus Beispiel 120A zu 11.2 mg (4% d. Th.) der Titelverbindung umgesetzt.

¹H-NMR (400 MHz, CDCl₃, δ/ppm): 8.37 (d, 1H), 8.19-8.05 (m, 1H), 7.49-7.30 (m, 3H), 7.23-7.11 (m, 2H), 6.83 (s, 1H), 5.48 (s, 2H), 3.80 (s, breit, 2H), 3.42 (s, breit, 2H), 2.60-2.30 (m, breit, 4H), 2.32 (s, 3H), 2.30 (s, 3H).

LC/MS (Methode I, ESIpos): Rₜ = 1.01 min, m/z = 561/563 [M+H]⁺.

### Beispiel 137

### (4-Methylpiperazin-1-yl)(3-{[5-methyl-3-(3-{4-[1-(trifluormethyl)cyclopropyl]phenyl}-1,2,4-oxa-diazol-5-yl)-1H-pyrazol-1-yl]methyl}phenyl)methanon

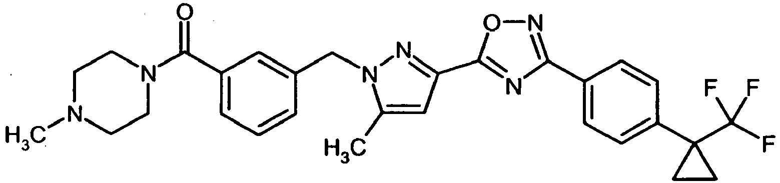

Man legte 450 mg (1.19 mmol) der Verbindung aus Beispiel 105A in 12 ml Dichlormethan bei 0°C vor, gab einen Tropfen DMF hinzu und tropfte dann 311 µl (3.56 mmol) Oxalylchlorid hinzu. Man rührte das Gemisch 1 h bei RT, engte danach ein und trocknete den Rückstand im Vakuum. Anschließend nahm man den Rückstand in 8 ml Dichlormethan auf und gab diese Lösung bei 0°C zu einem Gemisch von 290 mg (1.19 mmol) der Verbindung aus Beispiel 121A und 331 µl (2.38 mmol) Triethylamin in 4 ml Dichlormethan. Man rührte 1 h bei RT, engte dann ein und trocknete den Rückstand im Vakuum. Anschließend löste man den Rückstand in 12 ml DMSO und erhitzte das Gemisch 30 min lang in einem Mikrowellengerät (CEM Discover, initiale Einstrahlleistung 250 W) auf 120°C. Nach dem Abkühlen wurde das Reaktionsgemisch direkt mittels präparativer HPLC (Methode O) gereinigt. Die vereinigten Produktfraktionen wurden bis auf Restvolumen an wässriger Phase eingeengt. Man versetzte mit gesättigter wässriger Natriumhydrogencarbonat-Lösung und extrahierte zweimal mit Ethylacetat. Die vereinigten Ethylacetat-Phasen wurden über Magnesiumsulfat getrocknet, filtriert und eingeengt. Nach Trocknen des Rückstands im Vakuum wurden 109 mg (17% d. Th.) der Titelverbindung erhalten.

¹H-NMR (400 MHz, CDCl₃, δ/ppm): 8.10 (d, 2H), 7.52 (d, 2H), 7.38-7.23 (m, 2H), 7.18-7.05 (m, 2H), 6.76 (s, 1H), 5.40 (s, 2H), 3.70 (s, breit, 2H), 3.30 (s, breit, 2H), 2.49-2.07 (m, 4H), 2.22 (s, 3H), 2.19 (s, 3H), 1.34 (s, breit, 2H), 1.02 (s, breit, 2H).

LC/MS (Methode I, ESIpos): Rₜ = 0.97 min, m/z = 551 [M+H]⁺.

### Beispiel 138

### [3-({3-[5-(4-Cyclopropylphenyl)-1,2,4-oxadiazol-3-yl]-5-methyl-1H-pyrazol-1-yl}methyl)phenyl]-(4-methylpiperazin-1-yl)methanon

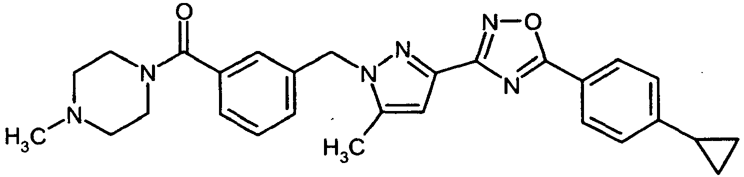

Man legte 68 mg (0.421 mmol) 4-Cyclopropylbenzoesäure in 2 ml Dichlormethan vor, versetzte mit einem Tropfen DMF und kühlte auf 0°C ab. Anschließend fügte man bei dieser Temperatur 160 mg (1.26 mmol) Oxalylchlorid hinzu und rührte danach für 20 min bei 40°C. Man gab dann 5 ml Dichlormethan hinzu, engte ein und trocknete den Rückstand im Vakuum. Der Rückstand wurde in 3 ml Dichlormethan aufgenommen und bei RT zu einem Gemisch aus 150 mg (0.421 mmol) der Verbindung aus Beispiel 148A und 140 µl Triethylamin in 5 ml Dichlormethan gegeben. Man rührte 3 h bei RT, engte wieder ein und trocknete den Rückstand im Vakuum. Der Rückstand wurde anschließend in 2 ml trockenem DMSO gelöst und 1 h lang in einem Mikrowellengerät (CEM Discover, initiale Einstrahlleistung 250 W) auf 140°C erhitzt. Nach dem Abkühlen auf RT wurde mit 60 ml Wasser versetzt und dreimal mit je 30 ml Ethylacetat extrahiert. Die vereinigten Ethylacetat-Phasen wurden einmal mit gesättigter Natriumchlorid-Lösung gewaschen, über Magnesiumsulfat getrocknet, filtriert und eingeengt. Der Rückstand wurde in Acetonitril aufgenommen und mittels präparativer HPLC (Methode O) gereinigt. Die vereinigten Produktfraktionen wurden bis auf ein Restvolumen an wässriger Phase eingeengt, mit gesättigter wässriger Natriumhydrogencarbonat-Lösung versetzt und dreimal mit je ca. 30 ml Dichlormethan extrahiert. Die vereinigten Dichlormethan-Phasen wurden einmal mit Wasser gewaschen, über Magnesiumsulfat getrocknet, filtriert und eingeengt. Nach Trocknen des Rückstands im Vakuum wurden 65 mg (32% d. Th.) der Titelverbindung erhalten.

¹H-NMR (400 MHz, CDCl₃, δ/ppm): 8.13 (d, 2H), 7.44-7.30 (m, 2H), 7.23-7.15 (m, 3H), 7.12 (s, 1H), 6.74 (s, 1H), 5.47 (s, 2H), 3.82-3.58 (m, 4H), 3.45-3.28 (s, breit, 2H), 2.54-2.34 (m, 2H), 2.28 (s, 3H), 2.22 (s, 3H), 2.04-1.90 (m, 1H), 1.14-1.01 (m, 2H), 0.85-0.74 (m, 2H).

LC/MS (Methode I, ESIpos): Rₜ = 0.91 min, m/z = 483 [M+H]⁺.

### Beispiel 139

### (3-{[3-(3-{4-[4-(Fluormethyl)tetrahydro-2H-pyran-4-yl]phenyl}-1,2,4-oxadiazol-5-yl)-5-methyl-1H-pyrazol-1-yl]methyl}phenyl)(4-methylpiperazin-1-yl)methanon

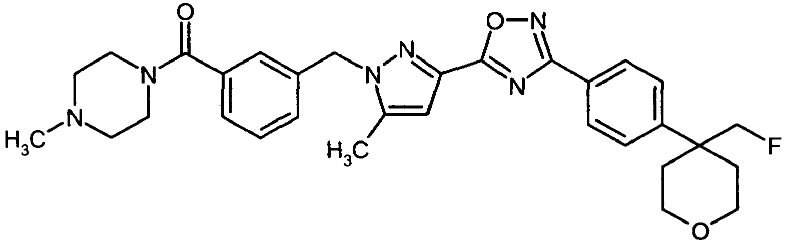

Zu 100 mg (0.26 mmol) des Hydrochlorids der Verbindung aus Beispiel 105A in 2.6 ml DMF gab man nacheinander 74 µl (0.53 mmol) Triethylamin, 51 mg (0.26 mmol) EDC und 40 mg (0.26 mmol) HOBt. Nach 10-minütigem Rühren bei RT fügte man 66 mg (0.26 mmol) der Verbindung aus Beispiel 123A hinzu, rührte zunächst weitere 10 min bei RT und erhitzte anschließend für 30 min auf 140°C. Nach dem Abkühlen wurde das Reaktionsgemisch direkt mittels präparativer HPLC gereinigt (Methode P). Es wurden 7.0 mg (5% d. Th.) der Titelverbindung erhalten.

¹H-NMR (400 MHz, DMSO-d₆, δ/ppm): 8.01 (d, 2H), 7.44 (m, 3H), 7.32 (t, 2H), 7.15 (s, 1H), 6.94 (s, 1H), 5.55 (s, 2H), 3.72 (d, breit, 2H), 3.62-3.45 (m, breit, 4H), 3.24 (m, breit, 2H), 3.05 (d, 2H), 2.34 (s, 3H), 2.37-2.12 (m, breit, 4H), 2.12 (s, 3H), 1.84-1.65 (m, 2H), 1.59 (t, 2H).

LC/MS (Methode D, ESIpos): Rₜ = 1.75 min, m/z = 559 [M+H]⁺.

### Beispiel 140

### 1-({3-[(5-Methyl-3-{3-[4-(1,1,1-trifluor-2-methylpropan-2-yl)phenyl]-1,2,4-oxadiazol-5-yl}-1H-pyrazol-1-yl)methyl]phenyl}sulfonyl)piperidin-4-ol

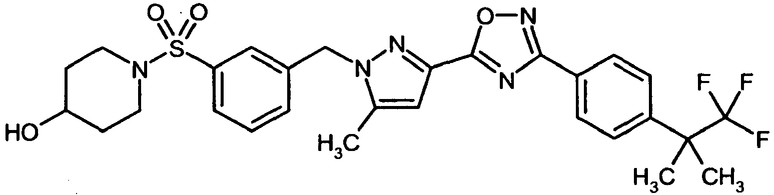

Zu einer Lösung von 95 mg (0.28 mmol) der Verbindung aus Beispiel 29A und 35 mg (0.31 mmol) Kalium-*tert*.-butylat in 3 ml THF gab man unter Eisbadkühlung eine Lösung von 162 mg (0.34 mmol) der Verbindung aus Beispiel 136A in 1 ml THF und rührte das Gemisch anschließend über Nacht bei RT. Es wurde dann mit Ethylacetat verdünnt und mit Magnesiumsulfat versetzt. Nach Filtration wurde das Filtrat am Rotationsverdampfer vom Lösungsmittel befreit und der erhaltene Rückstand mittels präparativer HPLC gereinigt (Methode P). Es wurden 77 mg (38% d. Th.) der Titelverbindung erhalten.

¹H-NMR (400 MHz, DMSO-d₆, δ/ppm): 8.09 (d, 2H), 7.77 (d, 2H), 7.70-7.60 (m, 3H), 7.49 (d, 1H), 6.96 (s, 1H), 5.66 (s, 2H), 4.65 (d, 1H), 3.50 (m, 1H), 3.11 (m, 2H), 2.69 (m, 2H), 2.35 (s, 3H), 1.69 (m, 2H), 1.61 (s, 6H), 1.40 (m, 2H).

LC/MS (Methode I): Rₜ = 1.24 min, m/z = 590 [M+H]⁺.

### Beispiel 141

### 1-Methyl-4-({3-[(5-methyl-3-{3-[4-(1,1,1-trifluor-2-methylpropan-2-yl)phenyl]-1,2,4-oxadiazol-5-yl}-1H-pyrazol-1-yl)methyl]phenyl}sulfonyl)piperazin

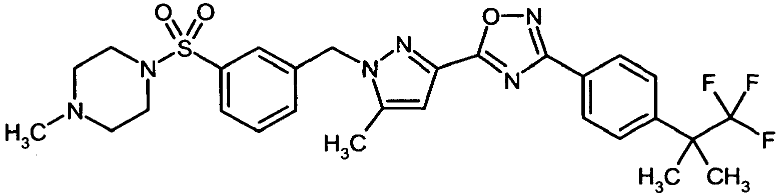

Analog zu dem unter Beispiel 140 beschriebenen Verfahren wurden aus 140 mg (0.42 mmol) der Verbindung aus Beispiel 29A und 1.0 ml (ca. 0.5 mmol) der in Beispiel 137A erhaltenen Intermediat-Lösung in THF 126 mg (43% d. Th.) der Titelverbindung erhalten.

¹H-NMR (400 MHz, DMSO-d₆, δ/ppm): 8.09 (d, 2H), 7.77 (d, 2H), 7.69-7.64 (m, 2H), 7.57 (s, 1H), 7.53 (d, 1H), 6.97 (s, 1H), 5.66 (s, 2H), 2.84 (s, 4H), 2.35 (s, 3H), 2.29 (s, 4H), 2.07 (s, 3H), 1.61 (s, 6H).

LC/MS (Methode I): Rₜ = 1.02 min, m/z = 589 [M+H]⁺.

### Beispiel 142

### 1-Methyl-4-({3-[(5-methyl-3-{3-[4-(trifluormethoxy)phenyl]-1,2,4-oxadiazol-5-yl}-1H-pyrazol-1-yl)methyl]phenyl}sulfonyl)piperazin

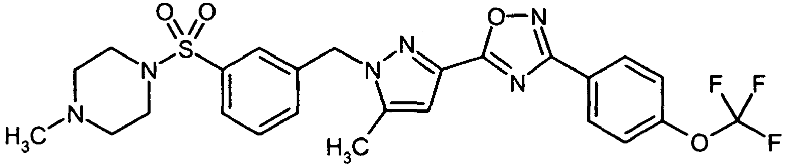

Analog zu dem unter Beispiel 140 beschriebenen Verfahren wurden aus 129 mg (0.42 mmol) der Verbindung aus Beispiel 28A und 1.0 ml (ca. 0.5 mmol) der in Beispiel 137A erhaltenen Intermediat-Lösung in THF 75 mg (27% d. Th.) der Titelverbindung erhalten.

¹H-NMR (400 MHz, DMSO-d₆, δ/ppm): 8.20 (d, 2H), 7.69-7.64 (m, 2H), 7.61 (s, 1H), 7.58 (d, 2H), 7.53 (d, 1H), 6.96 (s, 1H), 5.66 (s, 2H), 2.84 (s, 4H), 2.36 (s, 3H), 2.29 (s, 4H), 2.07 (s, 3H).

LC/MS (Methode I): Rₜ = 0.98 min, m/z = 563 [M+H]⁺.

### Beispiel 143

### [3-({4-[3-(4-tert-Butylphenyl)-1,2,4-oxadiazol-5-yl]-2-methyl-1H-pyrrol-1-yl} methyl)phenyl]-(4-methylpiperazin-1-yl)methanon

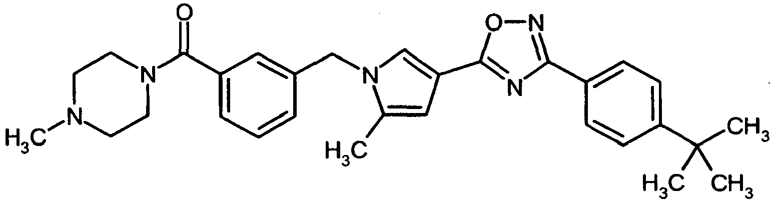

Eine Suspension von 100 mg (0.281 mmol) der Verbindung aus Beispiel 106A / Schritt 3 und 60 mg (0.309 mmol) 4-*tert*.-Butyl-*N*'-hydroxybenzamidin in 3 ml Ethanol wurde mit 100 mg (0.309 mmol) einer 21%-igen Lösung von Natriumethanolat in Ethanol versetzt. Das Gemisch wurde in einem Mikrowellen-Ofen (CEM Discover, initiale Einstrahlleistung 250 W) 30 min lang auf 160°C erhitzt. Nach dem Abkühlen auf RT wurde der Reaktionsansatz direkt mittels präparativer HPLC (Methode N) in seine Komponenten aufgetrennt. Die Produktfraktionen wurden vereinigt und am Rotationsverdampfer bis zur Trockene eingeengt. Der erhaltene Rückstand wurde in ca. 5 ml Methanol gelöst und über eine Hydrogencarbonat-Kartusche gegeben (Polymerlabs, Stratospheres SPE, PL-HCO₃ MP SPE, Kapazität 0.9 mmol), um anhaftende Ameisensäure aus der HPLC-Reinigung zu entfernen. Nach Einengen und Trocknen wurden 6.4 mg (5% d. Th.) der Titelverbindung erhalten.

¹H-NMR (400 MHz, CDCl₃, δ/ppm): 8.03 (d, 2H), 7.49 (d, 2H), 7.48 (s, 1H), 7.39 (t, 1H), 7.33 (d, . 1H), 7.09 (d, 1H), 7.08 (s, 1H), 6.58 (s, 1H), 5.11 (s, 2H), 3.76 (breit, 2H), 3.37 (breit, 2H), 2.44 (breit, 2H), 2.28 (breit, 2H), 2.26 (s, 3H), 2.19 (s, 3H), 1.35 (s, 9H).

LC/MS (Methode I, ESIpos): Rₜ = 1.06 min, m/z = 498 [M+H]⁺.

### Beispiel 144

### (4-Methylpiperazin-1-yl){3-[(2-methyl-4-{3-[4-(1,1,1-trifluor-2-methylpropan-2-yl)phenyl]-1,2,4-oxadiazol-5-yl}-1H-pyrrol-1-yl)methyl]phenyl}methanon

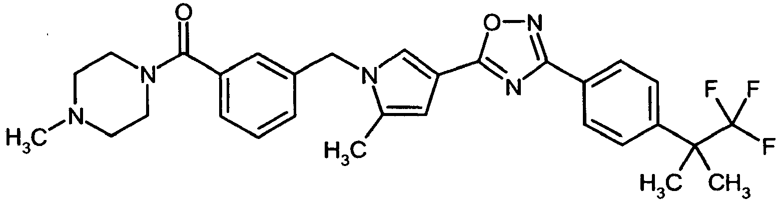

Analog zu dem unter Beispiel 41 beschriebenen Verfahren wurden 100 mg (0.293 mmol) der Verbindung aus Beispiel 106A und 79 mg (0.322 mmol) der Verbindung aus Beispiel 1A zu 21 mg (13% d. Th.) der Titelverbindung umgesetzt.

¹H-NMR (400 MHz, CDCl₃, δ/ppm): 8.10 (d, 2H), 7.60 (d, 2H), 7.48 (s, 1H), 7.39 (t, 1H), 7.33 (d, 1H), 7.10 (d, 1H), 7.09 (s, 1H), 6.59 (s, 1H), 5.12 (s, 2H), 3.77 (breit, 2H), 3.37 (breit, 2H), 2.44 (breit, 2H), 2.28 (breit, 2H), 2.26 (s, 3H), 2.19 (s, 3H), 1.62 (s, 6H).

LC/MS (Methode F, ESIpos): Rₜ = 1.21 min, m/z = 552 [M+H]⁺.

### Beispiel 145

### (4-Methylpiperazin-1-yl){3-[(2-methyl-4-{3-[4-(trimethylsilyl)phenyl]-1,2,4-oxadiazol-5-yl}-1H-pyrrol-1-yl)methyl]phenyl}methanon

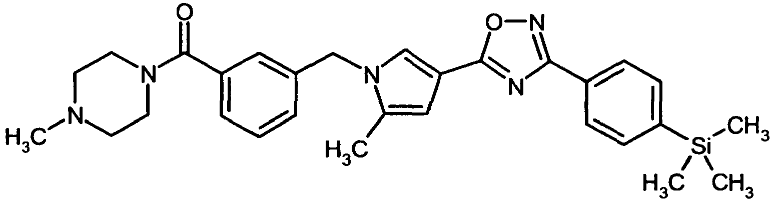

Analog zu dem unter Beispiel 41 beschriebenen Verfahren wurden 100 mg (0.293 mmol) der Verbindung aus Beispiel 106A und 67 mg (0.322 mmol) der Verbindung aus Beispiel 17A zu 11 mg (7.4% d. Th.) der Titelverbindung umgesetzt.

¹H-NMR (400 MHz, CDCl₃, δ/ppm): 8.08 (d, 2H), 7.62 (d, 2H), 7.49 (s, 1H), 7.39 (t, 1H), 7.33 (d, 1H), 7.10 (d, 1H), 7.09 (s, 1H), 6.59 (s, 1H), 5.12 (s, 2H), 3.76 (breit, 2H), 3.37 (breit, 2H), 2.43 (breit, 2H), 2.29 (breit, 2H), 2.25 (s, 3H), 2.19 (s, 3H), 0.30 (s, 9H).

LC/MS (Methode F, ESIpos): Rₜ = 1.27 min, m/z = 514 [M+H]⁺.

### Beispiel 146

### {3-[(4-{3-[4-(4-Fluortetrahydro-2H-pyran-4-yl)phenyl]-1,2,4-oxadiazol-5-yl}-2-methyl-1H-pyrrol-1-yl)methyl]phenyl}(4-methylpiperazin-1-yl)methanon

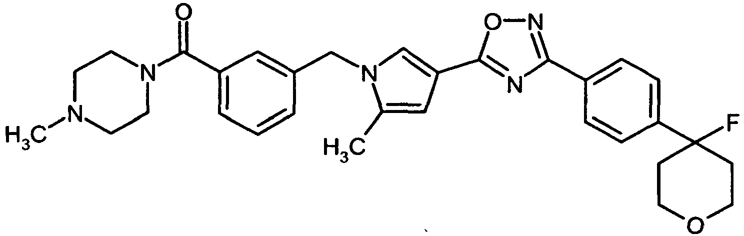

Analog zu dem unter Beispiel 41 beschriebenen Verfahren wurden 120 mg (0.351 mmol) der Verbindung aus Beispiel 106A und 92 mg (0.387 mmol) der Verbindung aus Beispiel 7A zu 20 mg (10% d. Th.) der Titelverbindung umgesetzt.

¹H-NMR (400 MHz, CDCl₃, δ/ppm): 8.14 (d, 2H), 7.50 (d, 2H), 7.49 (s, 1H), 7.39 (t, 1H), 7.33 (d, 1H), 7.10 (d, 1H), 7.09 (s, 1H), 6.59 (s, 1H), 5.12 (s, 2H), 3.99-3.86 (m, 4H), 3.76 (breit, 2H), 3.37 (breit, 2H), 2.44 (breit, 2H), 2.29 (breit, 2H), 2.26 (s, 3H), 2.23-2.10 (m, 2H), 2.18 (s, 3H), 1.97-1.91(m,2H).

LC/MS (Methode I, ESIpos): Rₜ = 0.90 min, m/z = 544 [M+H]⁺.

### Beispiel 147

### (4-Methylpiperazin-1-yl){3-[(2-methyl-4-{3-[4-(trifluormethyl)phenyl]-1,2,4-oxadiazol-5-yl}-1H-pyrrol-1-yl)methyl]phenyl} methanon

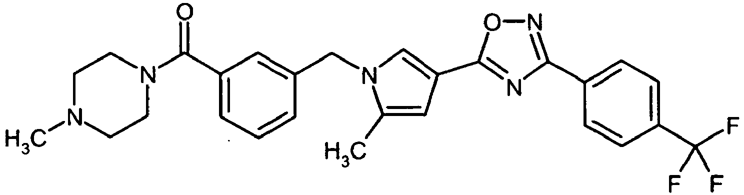

Analog zu dem unter Beispiel 41 beschriebenen Verfahren wurden 120 mg (0.351 mmol) der Verbindung aus Beispiel 106A und 79 mg (0.387 mmol) 4-Trifluormethyl-*N*'-hydroxybenzamidin zu 15 mg (8.4% d. Th.) der Titelverbindung umgesetzt.

¹H-NMR (400 MHz, CDCl₃, δ/ppm): 8.25 (d, 2H), 7.74 (d, 2H), 7.49 (s, 1H), 7.40 (t, 1H), 7.33 (d, 1H), 7.11-7.08 (m, 2H), 6.59 (s, 1H), 5.13 (s, 2H), 3.77 (breit, 2H), 3.37 (breit, 2H), 2.43 (breit, 2H), 2.29 (breit, 2H), 2.27 (s, 3H), 2.20 (s, 3H).

LC/MS (Methode I, ESIpos): Rₜ = 0.99 min, m/z = 510 [M+H]⁺.

### Beispiel 148

### 1-Methyl-4-{3-[(5-methyl-3-{3-[4-(trifluormethoxy)phenyl]-1,2,4-oxadiazol-5-yl}-1H-pyrazol-1-yl)methyl]phenoxy}piperidin

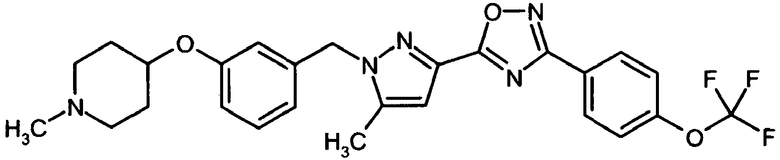

Eine Lösung von 69 mg (0.264 mmol) Triphenylphosphin und 52 µl (0.264 mmol) Diisopropylazodicarboxylat (DIAD) in 3 ml wasserfreiem THF wurde zunächst mit 30 mg (0.264 mmol) 4-Hydroxy-1-methylpiperidin und nach 5 min mit 100 mg (0.240 mmol) der Verbindung aus Beispiel 95A versetzt. Nachdem das Reaktionsgemisch 16 h bei RT gerührt worden war, wurde nochmals die gleiche Menge an DIAD hinzugefügt. Nach weiteren 5 Tagen bei RT wurden weitere 30 mg (0.264 mmol) 4-Hydroxy-1-methylpiperidin zugesetzt. Nach erneuten 16 h bei RT wurde das Reaktionsgemisch mit 1 ml Wasser und ca. 3 ml DMF versetzt. Diese Lösung wurde direkt mittels präparativer HPLC (Methode N) in ihre Komponenten aufgetrennt. Die Produktfraktionen wurden vereinigt und am Rotationsverdampfer vom Lösungsmittel befreit. Das so erhaltene Produkt wurde mittels MPLC (Kieselgel, Laufmittel: Dichlormethan/Methanol 10:1) nochmals nachgereinigt. Es wurden 32 mg (26% d. Th.) der Titelverbindung erhalten.

¹H-NMR (400 MHz, CDCl₃, δ/ppm): 8.25 (d, 2H), 7.33 (d, 2H), 7.22 (t, 1H), 6.82 (d, 1H), 6.81 (s, 1H), 6.72 (d, 1H), 6.71 (s, 1H), 5.40 (s, 2H), 4.31-4.24 (m, 1H), 2.69 (breit, 2H), 2.30 (s, 3H), 2.29 (breit, 2H), 2.28 (s, 3H), 1.98 (breit, 2H), 1.81 (breit, 2H).

HPLC (Methode A): Rₜ = 4.41 min.

LC/MS (Methode I, ESIpos): Rₜ = 0.99 min, m/z = 514 [M+H]⁺.

### Beispiel 149

### 2-[4-(Methoxymethyl)piperidin-1-yl]-4-[(5-methyl-3-{3-[4-(trifluormethoxy)phenyl]-1,2,4-oxa-diazol-5-yl}-1H-pyrazol-1-yl)methyl]pyridin

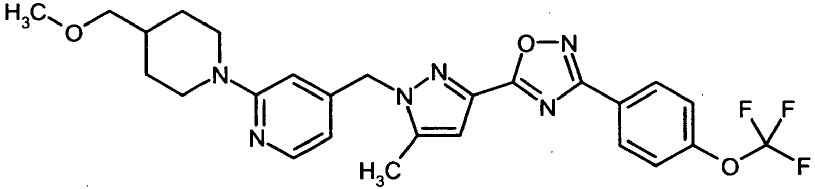

445 mg (3.44 mmol) 4-(Methoxymethyl)piperidin und 100 mg (0.229 mmol) der Verbindung aus Beispiel 81A wurden ohne Zusatz eines Lösungsmittels in einem Mikrowellenofen (Biotage Initiator 2.5, automatische Steuerung der Einstrahlleistung) 3 h lang auf 160°C erhitzt. Nach dem Abkühlen auf RT wurde das Reaktionsgemisch in ca. 2 ml Methanol aufgenommen. Diese Lösung wurde direkt mittels präparativer HPLC (Methode N) in ihre Komponenten aufgetrennt. Die Produktfraktionen wurden vereinigt, vom Lösungsmittel befreit und der Rückstand mit Pentan verrührt. Es wurden 77 mg (60% d. Th., 94% Reinheit) der Titelverbindung erhalten.

¹H-NMR (400 MHz, CDCl₃, δ/ppm): 8.25 (d, 2H), 8.11 (d, 1H), 7.33 (d, 2H), 6.83 (s, 1H), 6.35 (s, 1H), 6.29 (d, 1H), 5.33 (s, 2H), 4.26-4.20 (m, 2H), 3.33 (s, 3H), 3.23 (d, 2H), 2.83-2.77 (m, 2H), 2.29 (s, 3H), 1.88-1.77 (m, 3H), 1.29-1.18 (m, 2H).

LC/MS (Methode I, ESIpos): Rₜ = 1.09 min, m/z = 529 [M+H]⁺.

### Beispiel 150

### 2-(4-Methoxypiperidin-1-yl)-4-[(5-methyl-3-{3-[4-(trifluormethoxy)phenyl]-1,2,4-oxadiazol-5-yl}-1H-pyrazol-1-yl)methyl]pyridin

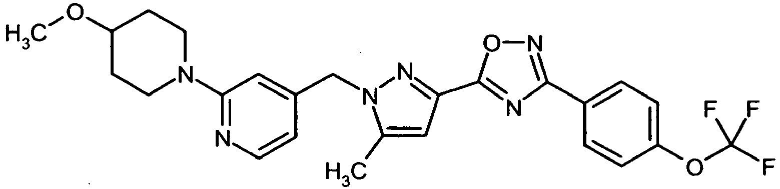

1.06 g (9.18 mmol) 4-Methoxypiperidin und 200 mg (0.229 mmol) der Verbindung aus Beispiel 81A wurden ohne Zusatz eines Lösungsmittels in einem Mikrowellenofen (Biotage Initiator 2.5, automatische Steuerung der Einstrahlleistung) 3 h lang auf 160°C erhitzt. Nach dem Abkühlen auf RT wurde das Reaktionsgemisch mit ca. 50 ml Wasser versetzt und dreimal mit je ca. 50 ml Ethylacetat extrahiert. Die vereinigten organischen Extrakte wurden nacheinander mit Wasser und gesättigter Kochsalz-Lösung gewaschen. Nach Trocknen über wasserfreiem Magnesiumsulfat wurde filtriert und das Filtrat am Rotationsverdampfer vom Lösungsmittel befreit. Das Rohprodukt wurde mittels MPLC gereinigt (ca. 50 g Kieselgel, Laufmittel: Cyclohexan/Ethylacetat 3:1 → 1:1). Es wurden 167 mg (70% d. Th.) der Titelverbindung erhalten.

¹H-NMR (400 MHz, CDCl₃, δ/ppm): 8.26 (d, 2H), 8.11 (d, 1H), 7.33 (d, 2H), 6.83 (s, 1H), 6.36 (s, 1H), 6.31 (d, 1H), 5.33 (s, 2H), 3.93-3.87 (m, 2H), 3.44-3.38 (m, 1H), 3.37 (s, 3H), 3.21-3.14 (m, 2H), 2.29 (s, 3H), 1.97-1.90 (m, 2H), 1.63-1.54 (m, 2H).

LC/MS (Methode I, ESIpos): Rₜ = 1.10 min, m/z = 515 [M+H]⁺.

### Beispiel 151

### 6-{4-[(5-Methyl-3-{3-[4-(trifluormethoxy)phenyl]-1,2,4-oxadiazol-5-yl}-1H-pyrazol-1-yl)methyl]-pyridin-2-yl} -2-oxa-6-azaspiro[3.3]heptan

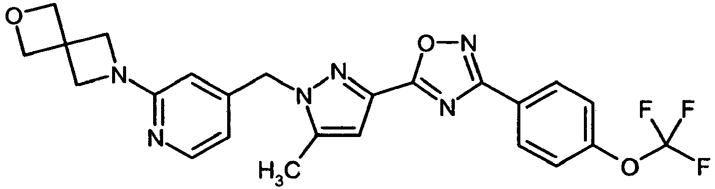

662 mg (2.30 mmol) 2-Oxa-6-azaspiro[3.3]heptan-Hemioxalat [M. Roger-Evans et al., Angew. Chem. Intl. Ed. Engl. 2008, 47 (24), 4512-4515], 100 mg (0.229 mmol) der Verbindung aus Beispiel 81A und 0.8 ml (4.59 mmol) *N*,*N*-Diisopropylethylamin wurden in 2.5 ml Methanol gelöst und in einem Mikrowellenofen (Biotage Initiator 2.5, automatische Steuerung der Einstrahlleistung) zunächst automatisch gesteuert auf 140°C erhitzt. Nachdem diese Temperatur erreicht war, wurde die Temperatur manuell gesteuert auf 160°C erhöht. Nach 15 h bei 160°C ließ man auf RT abkühlen. Das Reaktionsgemisch wurde mit weiteren ca. 3 ml Methanol verdünnt und direkt über präparative HPLC (Methode N) in seine Komponenten aufgetrennt. Die Produktfraktionen wurden vereinigt und am Rotationsverdampfer vom Lösungsmittel befreit. Der Rückstand wurde anschließend zur weiteren Reinigung über eine Chromabond-Kartusche chromatographiert (1.5 g Kieselgel, Laufmittel: Cyclohexan/Ethylacetat 1:1 → 1:5). Es wurden so 22 mg (19% d. Th.) der Titelverbindung erhalten.

¹H-NMR (400 MHz, CDCl₃, δ/ppm): 8.25 (d, 2H), 8.09 (d, 1H), 7.34 (d, 2H), 6.83 (s, 1H), 6.37 (d, 1H), 5.95 (s, 1H), 5.33 (s, 2H), 4.81 (s, 4H), 4.13 (s, 4H), 2.28 (s, 3H).

LC/MS (Methode D, ESIpos): Rₜ = 1.89 min, m/z = 499 [M+H]⁺.

### Beispiel 152

### 2-(1-{4-[(5-Methyl-3-{3-[4-(trifluormethoxy)phenyl]-1,2,4-oxadiazol-5-yl}-1H-pyrazol-1-yl)-methyl]pyridin-2-yl}piperidin-4-yl)propan-2-ol

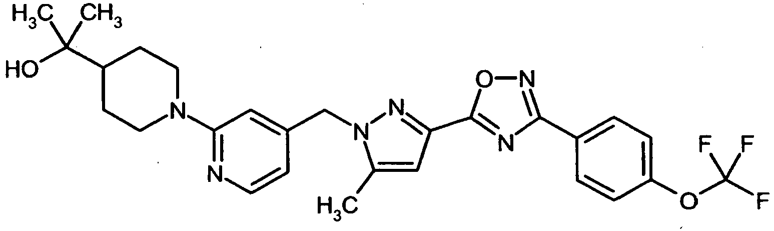

Analog zu dem unter Beispiel 149 beschriebenen Verfahren wurden 493 mg (3.44 mmol) 2-(Piperidin-4-yl)propan-2-ol und 100 mg (0.229 mmol) der Verbindung aus Beispiel 81A zu 40 mg (32% d. Th.) der Titelverbindung umgesetzt. Das nach der Reinigung über präparative HPLC erhaltene Produkt wurde abschließend mit Ethanol (anstelle von Pentan) verrührt.

¹H-NMR (400 MHz, CDCl₃, δ/ppm): 8.25 (d, 2H), 8.11 (d, 1H), 7.33 (d, 2H), 6.83 (s, 1H), 6.35 (s, 1H), 6.30 (d, 1H), 5.33 (s, 2H), 4.36-4.30 (m, 2H), 2.77-2.70 (m, 2H), 2.29 (s, 3H), 1.87-1.80 (m, 2H), 1.53-1.47 (m, 1H), 1.38-1.28 (m, 2H), 1.23 (s, breit, 1H), 1.18 (s, 6H).

LC/MS (Methode I, ESIpos): Rₜ = 1.03 min, m/z = 543 [M+H]⁺.

### B. Bewertung der pharmakologischen Wirksamkeit

Die pharmakologische Aktivität der erfindungsgemäßen Verbindungen kann durch *in vitro-* und in vivo-Untersuchungen, wie sie dem Fachmann bekannt sind, nachgewiesen werden. Die Nützlichkeit der erfindungsgemäßen Substanzen kann beispielhaft illustriert werden durch *in vitro-*(Tumor-)Zellversuche und *in vivo*-Tumormodelle, wie sie weiter unten aufgeführt sind. Der Zusammenhang zwischen einer Hemmung der HIF-Transkriptionsaktivität und der Hemmung von Tumorwachstum ist durch zahlreiche in der Literatur beschriebene Untersuchungen belegt (vgl. z.B. Warburg, 1956; Semenza, 2007).

### B-1. HIF-Luciferase-Assay:

HCT 116-Zellen wurden mit einem Plasmid stabil transfiziert, das einen Luciferase-Reporter unter der Kontrolle einer HIF-responsiven Sequenz enthielt. Diese Zellen wurden in Mikrotiterplatten ausgesät [20.000 Zellen/Kavität in RPMI 1640-Medium mit 10% fötalem Kälberserum (FKS) und 100 µg/ml Hygromycin]. Es wurde über Nacht unter Standardbedingungen inkubiert (5% CO₂, 21% O₂, 37°C, befeuchtet). Am anderen Morgen wurden die Zellen mit unterschiedlichen Konzentrationen der Testsubstanzen (0-10 µmol/L) in einer Hypoxiekammer (1% O₂) inkubiert. Nach 24 h wurde Bright Glo-Reagenz (Fa. Promega, Wisconsin, USA) entsprechend den Vorschriften des Herstellers zugefügt, und nach 5 min wurde die Lumineszenz gemessen. Zellen, die unter Normoxie inkubiert wurden, dienten als Hintergrundkontrollen.

In der folgenden Tabelle sind für repräsentative Ausführungsbeispiele die IC₅₀-Werte aus diesem Assay aufgeführt:

| **Beispiel Nr.** | **IC₅₀ [nmol/L]** |
|---|---|
| 16 | 4 |
| 18 | 5 |
| 21 | 10 |
| 35 | 2 |
| 41 | 6 |
| 45 | 10 |
| 52 | 3 |
| 65 | 0.6 |
| 71 | 1 |
| 72 | 1 |
| 75 | 1 |
| 77 | 1 |
| 78 | 0.5 |
| 85 | 2 |
| 86 | 4 |
| 91 | 0.6 |
| 93 | 0.8 |
| 100 | 2.5 |
| 119 | 20 |
| 137 | 3 |
| 140 | 4 |
| 150 | 3 |

### B-2. Suppression von HIF-Target-Genen in vitro:

Humane Bronchialkarzinom-Zellen (H460 und A549) wurden unter normoxischen Bedingungen sowie unter 1% Sauerstoffpartialdruck (siehe HIF-Luciferase-Assay) für 16 h mit variablen Konzentrationen der Testsubstanzen inkubiert (1 nM bis 10 µM). Aus den Zellen wurde die Gesamt-RNA isoliert, in cDNA umgeschrieben und in der Echtzeit-PCR die mRNA-Expression von HIF-Target-Genen analysiert. Bereits unter normoxischen Bedingungen, vor allem aber unter hypoxischen Bedingungen, erniedrigen aktive Testsubstanzen die mRNA-Expression der HIF-Target-Gene verglichen mit unbehandelten Zellen.

### B-3. Humane Xenograft- und syngene Tumormodelle:

Humane Tumor-Xenograftmodelle in immundefizienten Mäusen und syngene Tumor-Mausmodelle wurden zur Substanzbewertung herangezogen. Dazu wurden Tumorzellen *in vitro* kultiviert und subkutan implantiert, oder es wurden Tumor-Xenotransplantatstückchen subkutan weitertransplantiert. Die Behandlung der Tiere erfolgte durch orale, subkutane oder intraperitoneale Therapie nach der Etablierung des Tumors. Die Wirksamkeit von Testsubstanzen wurde in Monotherapie und in Kombinationstherapie mit anderen pharmakologischen Wirksubstanzen analysiert. Außerdem wurde die tumorinhibitorische Potenz von Testsubstanzen an Tumoren fortgeschrittener Größe (ca. 100 mm²) charakterisiert. Der Gesundheitszustand der Tiere wurde täglich überprüft, und die Behandlungen erfolgten entsprechend den Tierschutzbestimmungen. Die Tumorfläche wurde mit Schublehren gemessen (Länge L, Breite B = kleinere Ausdehnung). Das Tumorvolumen wurde nach der Formel (L x B²)/2 berechnet. Die Hemmung des Tumorwachstums wurde am Ende des Versuches als T/C-Verhältnis der Tumorflächen bzw. Tumorgewichte und als TGI-Wert (tumor growth inhibition, berechnet nach der Formel [1-(T/C)] x 100) bestimmt (T = Tumorgröße der behandelten Gruppe; C = Tumorgröße der unbehandelten Kontrollgruppe).

Der Einfluss von Testsubstanzen auf die Tumor-Gefäßarchitektur und den Blutfluss innerhalb des Tumors wurde mit Hilfe von Mikro-Computertomographie- und Mikro-Ultraschall-Untersuchungen anhand von behandelten und unbehandelten tumortragenden Mäusen identifiziert.

### B-4. Bestimmung_pharmakokinetischer Kenngrößen nach intravenöser und peroraler Gabe:

Die zu untersuchende Substanz wurde Tieren (z.B. Mäusen oder Ratten) intravenös als Lösung appliziert (z.B. in entsprechendem Plasma mit geringem DMSO-Zusatz oder in einem PEG/ Ethanol/Wasser-Gemisch), die perorale Applikation erfolgte als Lösung (z.B. in Solutol/Ethanol/ Wasser- oder PEG/Ethanol/Wasser-Gemischen) oder als Suspension (z.B. in Tylose) jeweils über eine Schlundsonde. Nach Substanzgabe wurde den Tieren zu festgelegten Zeitpunkten Blut entnommen. Dieses wurde heparinisiert, anschließend wurde daraus durch Zentrifugation Plasma gewonnen. Die Substanz wurde im Plasma über LC-MS/MS analytisch quantifiziert. Aus den so ermittelten Plasmakonzentration-Zeit-Verläufen wurden unter Verwendung eines internen Standards und mit Hilfe eines validierten Rechenprogramms die pharmakokinetischen Kenngrößen berechnet, wie AUC (Fläche unter der Konzentration-Zeit-Kurve), Cₘₐₓ (maximale Plasmakonzentration), T_{1/2} (Halbwertszeit), Vss (Verteilungsvolumen) und CL (Clearance) sowie die absolute und die relative Bioverfügbarkeit (i.v./p.o.-Vergleich bzw. Vergleich von Suspension zu Lösung nach p.o.-Gabe).

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### D. Literaturangaben

### • Globocan 2002 Report

IARC International Agency for Research on Cancer: Globocan 2002, http://www-dep.iarc.fr/globocan/downloads.htm

• American Cancer Society, Cancer Facts and Figures 2005American Cancer Society: Cancer Facts and Figures 2007, http://www.cancer.org/docroot/STT/content/STT_1x_Cancer_Facts_Figures_2007.asp

### • Gibbs JB, 2000

Gibbs JB: Mechanism-based target identification and drug discovery in cancer research, Science 2000, 287 (5460), 1969-1973.

### • Semenza und Wang, 1992

Semenza GL, Wang GL: A nuclear factor induced by hypoxia via de novo protein synthesis binds to the human erythropoietin gene enhancer at a site required for transcriptional activation, Mol. Cell. Biol. 1992, 12 (12), 5447-5454.

### • Wang und Semenza, 1995

Wang GL, Semenza GL: Purification and characterization of hypoxia-inducible factor 1, J. Biol. Chem. 1995, 270 (3), 1230-1237.

### • Wang, Jiang et al., 1995

Wang GL, Jiang BH, Rue EA, Semenza GL: Hypoxia-inducible factor 1 is a basic-helix-loophelix-PAS heterodimer regulated by cellular O2 tension, PNAS 1995, 92 (12), 5510-5514.

### • Jiang, Rue et al., 1996

Jiang BH, Rue E, Wang GL, Roe R, Semenza GL: Dimerization, DNA binding, and transactivation properties of hypoxia-inducible factor 1, J. Biol. Chem. 1996, 277 (30), 17771-17778.

### • Makino, Cao et al., 2001

Makino Y, Cao R, Svensson K, Bertilsson G, Asman M, Tanaka H, Cao Y, Poellinger L: Nature 2001, 414 (6863), 550-554.

### • Jiang, Semenza et al., 1996

Jiang BH, Semenza GL, Bauer C, Marti HH: Hypoxia-inducible factor 1 levels vary exponentially over a physiologically relevant range of O2 tension, Am. J. Physiol. 1996, 271, 1172-1180.

### • Maxwell, Wiesener et al., 1999

Maxwell PH, Wiesener MS, Chang GW, Clifford SC, Vaux EC, Cockman ME, Wykoff CC, Ratcliffe PJ: The tumour suppressor protein VHL targets hypoxia-inducible factors for oxygendependent proteolysis, Nature 1999, 399 (6733), 271-275.

### • Hirota und Semenza, 2006

Hirota K, Semenza GL: Regulation of angiogenesis by hypoxia-inducible factor 1, Crit. Rev. Oncol. Hematol. 2006, 59 (1), 15-26.

### • Chen, Zhao et al., 2003

Chen J, Zhao S, Nakada K, Kuge Y, Tamaki N, Okada F, Wang J, Shindo M, Higashino F, Takeda K, Asaka M, Katoh H, Sugiyama T, Hosokawa M, Kobayashi M: Dominant-negative hypoxia-inducible factor-1 alpha reduces tumorigenicity of pancreatic cancer cells through the suppression of glucose metabolism, Am. J. Pathol. 2003, 162 (4), 1283-1291.

### • Stoeltzing, McCarty et al., 2004

Stoeltzing O, McCarty MF, Wey JS, Fan F, Liu W, Belcheva A, Bucana CD, Semenza GL, Ellis LM: Role of hypoxia-inducible factor-1alpha in gastric cancer cell growth, angiogenesis, and vessel maturation, J. Natl. Cancer Inst. 2004, 96 (12), 946-956.

### • Li, Lin et al., 2005

Li L, Lin X, Staver M, Shoemaker A, Semizarov D, Fesik SW, Shen Y: Evaluating hypoxia-inducible factor-1 alpha as a cancer therapeutic target via inducible RNA interference in vivo, Cancer Res. 2005, 65 (16), 7249-7258.

### • Mizukami, Jo et al., 2005

Mizukami Y, Jo WS, Duerr EM, Gala M, Li J, Zhang X, Zimmer MA, Iliopoulos O, Zukerberg LR, Kohgo Y, Lynch MP, Rueda BR, Chung DC: Induction of interleukin-8 preserves the angiogenic response in HIF-1alpha-deficient colon cancer cells, Nat. Med. 2005, 11 (9), 992-997.

### • Li, Shi et al., 2006

Li J, Shi M, Cao Y, Yuan W, Pang T, Li B, Sun Z, Chen L, Zhao RC: Knockdown of hypoxia-inducible factor-1 alpha in breast carcinoma MCF-7 cells results in reduced tumor growth and increased sensitivity to methotrexate, Biochem. Biophys. Res. Commun. 2006, 342, 1341-1351.

### • Semenza, 2007

Semenza GL: Drug Discov. Today 2007, 12 (19-20), 853-859.

### • Weidemann und Johnson, 2008

Weidemann A, Johnson RS: Cell Death and Differentiation 2008, 15, 621-627.

### • Aiello et al., 1994

Aiello et al.: New Engl. J. Med. 1994, 331, 1480.

### • Peer et al., 1995

Peer et al.: Lab. Invest. 1995, 72, 638.

### • Lopez et al., 1996

Lopez et al.: Invest. Ophthalmol. Vis. Sci. 1996, 37, 855.

### • Warburg, 1956

Warburg O: Science 1956, 123 (3191), 309-314.

## Patentansprüche

1. Verbindung der Formel (I) 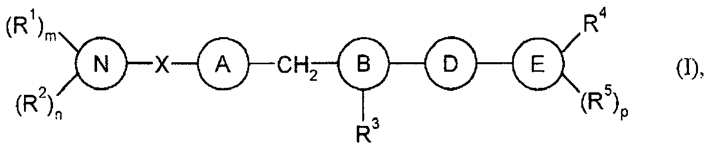 in welcher
der Ring 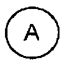 für einen Phenyl- oder Pyridyl-Ring steht,
der Ring 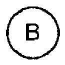 mit dem Substituenten R³ für einen Heteroaryl-Ring der Formel 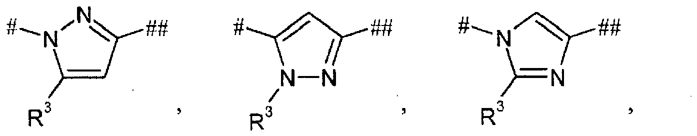 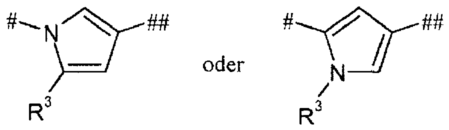 steht, worin
# die Verknüpfungsstelle mit der angrenzenden CH₂-Gruppe
und
## die Verknüpfungsstelle mit dem Ring 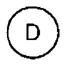 bezeichnen,
der Ring 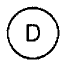 für einen Heteroaryl-Ring der Formel 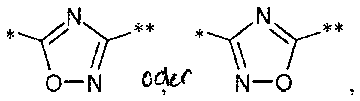 steht, worin
* die Verknüpfungsstelle mit dem Ring 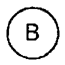 und
** die Verknüpfungsstelle mit dem Ring 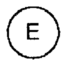 bezeichnen,
der Ring 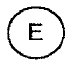 für einen Phenyl- oder Pyridyl-Ring steht,
der Ring 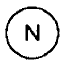 für einen gesättigten 4- bis 10-gliedrigen Aza-Heterocyclus steht, der mindestens ein N-Atom als Ringglied enthält und darüber hinaus ein oder zwei weitere Hetero-Ringglieder aus der Reihe N, O, S und/oder S(O)₂ enthalten kann,
X für eine Bindung oder für ◆-(CH₂)_{q}-N(R⁶)-◆◆, ◆-N(R⁶)-(CH₂)_{q}-◆◆, -O-, -S-, -C(=O)-, -S(=O)₂-, ◆-C(=O)-N(R⁶)-◆◆ oder ◆-N(R⁶)-C(=O)-◆◆ steht, worin
◆ die Verknüpfungsstelle mit dem Ring 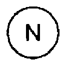 und
◆◆ die Verknüpfungsstelle mit dem Ring 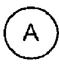 bezeichnen,
q die Zahl 0, 1 oder 2 bedeutet und
R⁶ Wasserstoff, (C₁-C₆)-Alkyl oder (C₃-C₆)-Cycloalkyl bedeutet, wobei (C₁-C₆)-Alkyl und (C₃-C₆)-Cycloalkyl jeweils mit Hydroxy oder (C₁-C₄)-Alkoxy substituiert sein können,
R¹ für einen an ein Kohlenstoffatom des Ringes Ⓝ gebundenen Substituenten ausgewählt aus der Reihe Fluor, Cyano, (C₁-C₆)-Alkyl, Hydroxy, (C₁-C₆)-Alkoxy, Oxo, Amino, Mono-(C₁-C₆)-alkylamino, Di-(C₁-C₆)-alkylamino und (C₃-C₆)-Cycloalkyl steht,
wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
m für die Zahl 0, 1, 2, 3 oder 4 steht, wobei im Fall, dass der Substituent R¹ mehrfach auftritt, seine Bedeutungen gleich oder verschieden sein können,
R² für einen an ein Stickstoffatom des Ringes 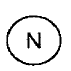 gebundenen Substituenten ausgewählt aus der Reihe (C₁-C₆)-Alkyl, (C₁-C₆)-Alkylcarbonyl, (C₁-C₆)-Alkoxy-carbonyl, (C₁-C₆)-Alkylsulfonyl und (C₃-C₆)-Cycloalkyl steht, wobei die Alkyl-Gruppe in (C₁-C₆)-Alkyl, (C₁-C₆)-Alkylcarbonyl, (C₁-C₆)-Alkoxycarbonyl und (C₁-C₆)-Alkylsulfonyl ihrerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkyl-amino, (C₃-C₆)-Cycloalkyl und 4- bis 6-gliedriges Heterocyclyl substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
n für die Zahl 0 oder 1 oder auch, sofern der Aza-Heterocyclus Ⓝ weitere N-Atome als Ringglieder enthält, die Zahl 2 steht,
wobei im Fall, dass der Substituent R² zweifach auftritt, seine Bedeutungen gleich oder verschieden sein können,
R³ für Methyl, Ethyl oder Trifluormethyl steht,
R⁴ für Wasserstoff oder einen Substituenten ausgewählt aus der Reihe Halogen, Cyano, Pentafluorthio, (C₁-C₆)-Alkyl, Tri-(C₁-C₄)-alkylsilyl, -OR⁷, -NR⁷R⁸, -N(R⁷)-C(=O)-R⁸, -N(R⁷)-C(=O)-OR⁸, -N(R⁷)-S(=O)₂-R⁸, -C(=O)-OR⁷, -C(=O)-NR⁷R⁸, -SR⁷, -S(=O)-R⁷, -S(=O)₂-R⁷, -S(=O)₂-NR⁷R⁸, -S(=O)(=NH)-R⁷, -S(=O)(=NCH₃)-R⁷, (C₃-C₆)-Cycloalkyl, 4- bis 6-gliedriges Heterocyclyl und 5- oder 6-gliedriges Heteroaryl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -N(R⁷)-C(=O)-OR⁸, -C(=O)-OR⁷, -C(=O)-NR⁷R⁸, (C₃-C₆)-Cyclo-alkyl, 4- bis 6-gliedriges Heterocyclyl und 5- oder 6-gliedriges Heteroaryl substituiert sein kann
und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo, Amino, Mono-(C₁-C₄)-alkylamino, Di-(C₁-C₄)-alkylamino, (C₁-C₄)-Alkylcarbonylamino, (C₁-C₄)-Alkoxy-carbonylamino, (C₁-C₄)-Alkylcarbonyl und (C₁-C₄)-Alkoxycarbonyl
sowie die genannten Heteroaryl-Gruppen ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy substituiert sein können, wobei die hierin genannten (C₁-C₄)-Alkyl-Substituenten ihrerseits mit Hydroxy, (C₁-C₄)-Alkoxy, Trifluormethoxy, (C₁-C₄)-Alkylcarbonyloxy, Aminocarbonyl, Mono-(C₁-C₄)-alkylaminocarbonyl oder Di-(C₁-C₄)-alkyl-aminocarbonyl oder bis zu dreifach mit Fluor substituiert sein können,
und worin
R⁷ und R⁸ unabhängig voneinander bei jedem einzelnen Auftreten Wasserstoff, (C₁-C₆)-Alkyl, (C₃-C₆)-Cycloalkyl oder 4- bis 6-gliedriges Heterocyclyl bedeuten,
wobei (C₁-C₆)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, Amino, Mono-(C₁-C₄)-alkylamino, Di-(C₁-C₄)-alkylamino, (C₁-C₄)-Alkoxycarbonyl, (C₃-C₆)-Cyclo-alkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, (C₁-C₄)-Alkylcarbonyl und (C₁-C₄)-Alkoxycarbonyl substituiert sein können,
oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkyl-amino, (C₁-C₄)-Alkylcarbonyl und (C₁-C₄)-Alkoxycarbonyl substituiert sein kann,
R⁵ für einen Substituenten ausgewählt aus der Reihe Fluor, Chlor, Cyano, Methyl, Trifluormethyl und Hydroxy steht
und
p für die Zahl 0, 1 oder 2 steht, wobei im Fall, dass der Substituent R⁵ zweifach auftritt, seine Bedeutungen gleich oder verschieden sein können,
sowie ihre Salze, Solvate und Solvate der Salze.

2. Verbindung der Formel (I) nach Anspruch 1, in welcher der Ring 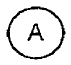 für einen Phenyl- oder Pyridyl-Ring steht und die angrenzenden Gruppen X und CH₂ in 1,3- oder 1,4-Relation zueinander an Ring-Kohlenstoffatome von 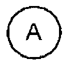 gebunden sind und der Ring 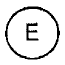 mit den Substituenten R⁴ und R⁵ für einen Phenyl-Ring der Formel 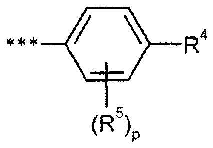 steht, worin
*** die Verknüpfungsstelle mit dem Ring 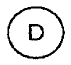 bezeichnet,
sowie ihre Salze, Solvate und Solvate der Salze.

3. Verbindung der Formel (I) nach Anspruch 1 oder 2, in welcher der Ring 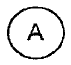 für einen Pyridyl-Ring steht und die angrenzenden Gruppen X und CH₂ in 1,3- oder 1,4-Relation zueinander an Ring-Kohlenstoffatome dieses Pyridyl-Rings gebunden sind und der Ring 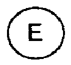 mit den Substituenten R⁴ und R⁵ für einen Phenyl-Ring der Formel 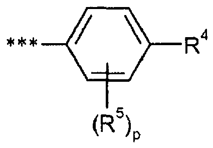 steht, worin
*** die Verknüpftingsstelle mit dem Ring 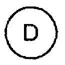 bezeichnet,
sowie ihre Salze, Solvate und Solvate der Salze.

4. Verbindung der Formel (I) nach Anspruch 1 oder 2, in welcher der Ring 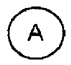 für einen Phenyl-Ring steht und die angrenzenden Gruppen X und CH₂ in 1,3- oder 1,4-Relation zueinander an diesen Phenyl-Ring gebunden sind,
der Ring 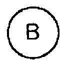 mit dem Substituenten R³ für einen Heteroaryl-Ring der Formel 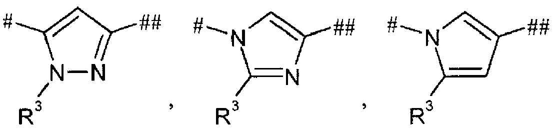 oder 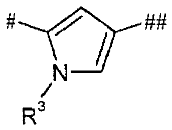 steht, worin
# die Verknüpfungsstelle mit der angrenzenden CH₂-Gruppe und
## die Verknüpfungsstelle mit dem Ring 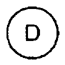 bezeichnen,
und
der Ring 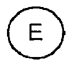 mit den Substituenten R⁴ und R⁵ für einen Phenyl-Ring der Formel 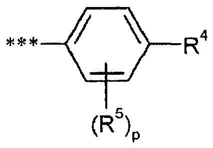 steht, worin
*** die Verknüpfungsstelle mit dem Ring 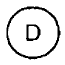 bezeichnet, sowie ihre Salze, Solvate und Solvate der Salze.

5. Verbindung der Formel (I) nach Anspruch 1 oder 2, in welcher der Ring 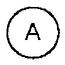 für einen Phenyl-Ring steht und die angrenzenden Gruppen X und CH₂ in 1,3- oder 1,4-Relation zueinander an diesen Phenyl-Ring gebunden sind, der Ring 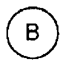 mit dem Substituenten R³ für einen Heteroaryl-Ring der Formel 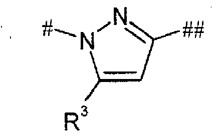 steht, worin
# die Verknüpfungsstelle mit der angrenzenden CH₂-Gruppe
und
## die Verknüpfungsstelle mit dem Ring 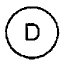 bezeichnen,
der Ring 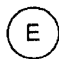 mit den Substituenten R⁴ und R⁵ für einen Phenyl-Ring der Formel 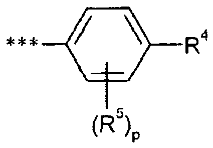 steht, worin
*** die Verknüpfungsstelle mit dem Ring 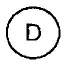 bezeichnet,
R¹ für einen an ein Kohlenstoffatom des Ringes 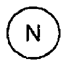 gebundenen Substituenten ausgewählt aus der Reihe Cyano, (C₁-C₆)-Alkyl, Oxo und (C₃-C₆)-Cycloalkyl steht, wobei (C₁-C₆)-Alkyl seinerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino und Di-(C₁-C₄)-alkylamino substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
R² für einen an ein Stickstoffatom des Ringes 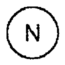 gebundenen Substituenten ausgewählt aus der Reihe (C₁-C₆)-Alkyl, (C₁-C₆)-Alkylcarbonyl, (C₁-C₆)-Alkoxy-carbonyl, (C₁-C₆)-Alkylsulfonyl und (C₃-C₆)-Cycloalkyl steht,
wobei die Alkyl-Gruppe in (C₁-C₆)-Alkyl, (C₁-C₆)-Alkylcarbonyl, (C₁-C₆)-Alkoxy-carbonyl und (C₁-C₆)-Alkylsulfonyl ihrerseits bis zu dreifach mit Fluor sowie bis zu zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkyl-amino, (C₃-C₆)-Cycloalkyl und 4- bis 6-gliedriges Heterocyclyl substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylan-iino und Di-(C₁-C₄)-alkylamino substituiert sein kann,
m für die Zahl 0, 1, 2, 3 oder 4 steht, wobei im Fall, dass der Substituent R¹ mehrfach auftritt, seine Bedeutungen gleich oder verschieden sein können,
und
n für die Zahl 0 oder 1 oder auch, sofern der Aza-Heterocyclus 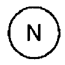 weitere N-Atome als Ringglieder enthält, die Zahl 2 steht, wobei im Fall, dass der Substituent R² zweifach auftritt, seine Bedeutungen gleich oder verschieden sein können,
wobei die Summe aus m und n ungleich der Zahl 0 ist,
sowie ihre Salze, Solvate und Solvate der Salze.

6. Verbindung der Formel (I) nach Anspruch 1, 2 oder 3, in welcher der Ring 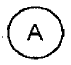 für einen Pyridyl-Ring steht und die angrenzenden Gruppen X und CH₂ in 1,3- oder 1,4-Relation zueinander an Ring-Kohlenstoffatome dieses Pyridyl-Rings gebunden sind,
der Ring 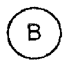 mit dem Substituenten R³ für einen Heteroaryl-Ring der Formel 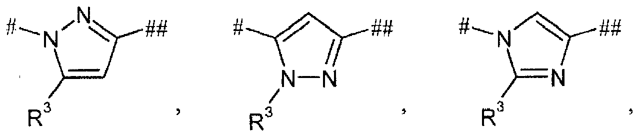 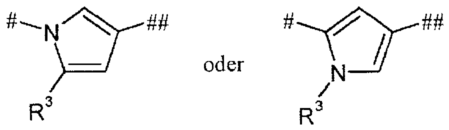 steht, worin
# die Verknüpfungsstelle mit der angrenzenden CH₂-Gruppe
und
## die Verknüpfungsstelle mit dem Ring 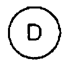 bezeichnen,
der Ring 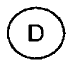 für einen Heteroaryl-Ring der Formel 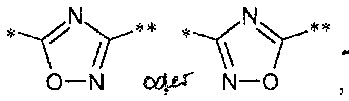 steht, worin
* die Verknüpfungsstelle mit dem Ring 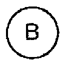 und
** die Verknüpfungsstelle mit dem Ring 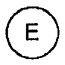 bezeichnen,
der Ring 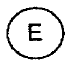 mit den Substituenten R⁴ und R⁵ für einen Phenyl-Ring der Formel 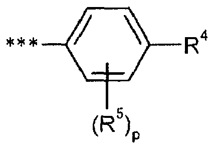 steht, worin
*** die Verktaüpfizrigsstelle mit dem Ring 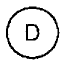 bezeichnet,
der Ring 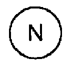 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH₂)q-N(R⁶)-◆◆, -O-, -S-, -C(=O)-, -S(=O)₂- oder ◆-N(R⁶)-C(=O)-◆◆ steht, worin
◆ die Verknüpfungsstelle mit dem Ring 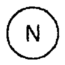 und
◆◆ die Verknüpfungsstelle mit dem Ring 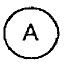 bezeichnen,
q die Zahl 0, 1 oder 2 bedeutet und
R⁶ Wasserstoff, (C₁-C₄)-Alkyl oder (C₃-C₆)-Cycloalkyl bedeutet,
R¹ für einen an ein Kohlenstoffatom des Ringes 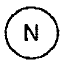 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
m für die Zahl 0, 1 oder 2 steht, wobei im Fall, dass der Substituent R¹ zweifach auftritt, seine Bedeutungen gleich oder verschieden sein können,
R² für einen an ein Stickstoffatom des Ringes 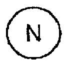 gebundenen Substituenten ausgewählt aus der Reihe (C₁-C₄)-Alkyl, (C₁-C₄)-Alkylcarbonyl, (C₁-C₄)-Alkoxy-carbonyl, (C₁-C₄)-Alkylsulfonyl und (C₃-C₆)-Cycloalkyl steht, wobei die Alkyl-Gruppe in (C₁-C₄)-Alkyl, (C₁-C₄)-Alkylcarbonyl, (C₁-C₄)-Alkoxy-carbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylainino, Di-(C₁-C₄)-alkylamino, (C₃-C₆)-Cycloalkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
n für die Zahl 0 oder 1 steht,
R³ für Methyl, Ethyl oder Trifluormethyl steht,
R^{a} für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, -S(=O)(=NH)-R⁷, -S(=O)(=NCH₃)-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann und wobei
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy substituiert sein können, wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können, und worin
R⁷ und R⁸ unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten, wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für einen Substituenten ausgewählt aus der Reihe Fluor, Chlor und Methyl steht
und
p für die Zahl 0 oder 1 steht,
sowie ihre Salze, Solvate und Solvate der Salze.

7. Verbindung der Formel (I) nach Anspruch 1, 2 oder 4, in welcher der Ring 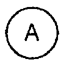 für einen Phenyl-Ring steht und die angrenzenden Gruppen X und CH₂ in 1,3- oder 1,4-Relation zueinander an diesen Phenyl-Ring gebunden sind, der Ring 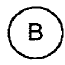 mit dem Substituenten R³ für einen Heteroaryl-Ring der Formel 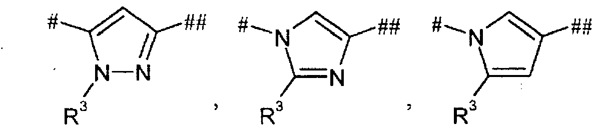 oder 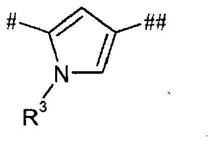 steht, worin
# die Verknüpfungsstelle mit der angrenzenden CH₂-Gruppe und
## die Verknüpfungsstelle mit dem Ring 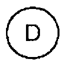 bezeichnen, der Ring 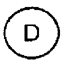 für einen Heteroaryl-Ring der Formel 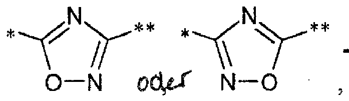 steht, worin
* die Verknüpfungsstelle mit dem Ring 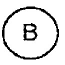 und
** die Verknüpfungsstelle mit dem Ring 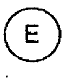 bezeichnen,
der Ring 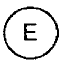 mit den Substituenten R⁴ und R⁵ für einen Phenyl-Ring der Formel 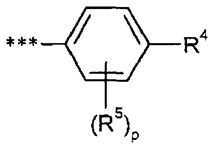 steht, worin
*** die Verknüpfungsstelle mit dem Ring 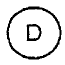 bezeichnet,
der Ring 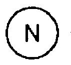 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH₂)_{q}-N(R₆)-◆◆, -O-, -S-, -C(=O)-, -S(=O)₂- oder ◆-N(R⁶)-C(=O)-◆◆ steht, worin
◆ die Verknüpfungsstelle mit dem Ring 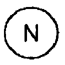 und
◆◆ die Verknüpfungsstelle mit dem Ring 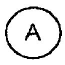 bezeichnen,
q die Zahl 0, 1 oder 2 bedeutet und
R⁶ Wasserstoff, (C₁-C₄)-Alkyl oder (C₃-C₆)-Cycloalkyl bedeutet,
R¹ für einen an ein Kohlenstoffatom des Ringes 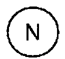 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino und (C₃-C₆)-Cycloalkyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkyl]-amino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
m für die Zahl 0, 1 oder 2 steht, wobei im Fall, dass der Substituent R¹ zweifach auftritt, seine Bedeutungen gleich oder verschieden sein können,
R² für einen an ein Stickstoffatom des Ringes 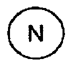 gebundenen Substituenten ausgewählt aus der Reihe (C₁-C₄)-Alkyl, (C₁-C₄)-Alkylcarbonyl, (C₁-C₄)-Alkoxy-carbonyl, (C₁-C₄)-Alkylsulfonyl und (C₃-C₆)-Cycloalkyl steht,
wobei die Alkyl-Gruppe in (C₁-C₄)-Alkyl, (C₁-C₄)-Alkylcarbonyl, (C₁-C₄)-Alkoxy-carbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₆)-Cycloalkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
n für die Zahl 0 oder 1 steht,
R³ für Methyl, Ethyl oder Trifluormethyl steht,
R⁴ für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, -S(=O)(=NH)-R⁷, -S(=O)(=NCH₃)-R⁷, (C₃-C₅)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann und wobei die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie
die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy substituiert sein können,
wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können,
und worin
R⁷ und R⁸ unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten,
wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Trifluormethoxy, Oxo und (C₁-C₄)-Alkylcarbonyl substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für einen Substituenten ausgewählt aus der Reihe Fluor, Chlor und Methyl steht und
p für die Zahl 0 oder 1 steht,
sowie ihre Salze, Solvate und Solvate der Salze.

8. Verbindung der Formel (1) nach Anspruch 1, 2 oder 5, in welcher der Ring 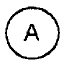 für einen Phenyl-Ring steht und die angrenzenden Gruppen X und CH₂ in 1,3- oder 1,4-Relation zueinander an diesen Phenyl-Ring gebunden sind,
der Ring 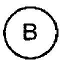 mit dem Substituenten R³ für einen Heteroaryl-Ring der Formel 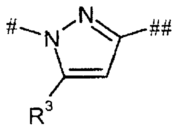 steht, worin
# die Verknüpfungsstelle mit der angrenzenden CH₂-Gruppe und
## die Verknüpfungsstelle mit dem Ring 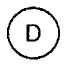 bezeichnen,
der Ring 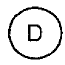 für einen Heteroaryl-Ring der Formel 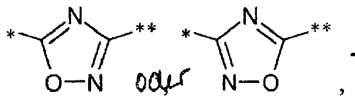 steht, worin
* die Verknüpfungsstelle mit dem Ring 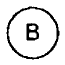 und
** die Verknüpfungsstelle mit dem Ring 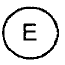 bezeichnen,
der Ring 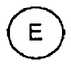 mit den Substituenten R⁴ und R⁵ für einen Phenyl-Ring der Formel 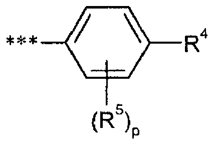 steht, worin
*** die Verknüpfungsstelle mit dem Ring 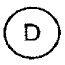 bezeichnet,
der Ring 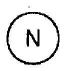 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH²)q-N(R⁶)-◆◆, -O-, -S-, -C(=O)-, -S(=O)₂- oder ◆-N(R⁶)-C(=O)-◆◆ steht, worin
◆ die Verknüpfungsstelle mit dem Ring 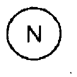 und
◆◆ die Verknüpfungsstelle mit dem Ring 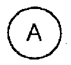 bezeichnen,
q die Zahl 0, 1 oder 2 bedeutet und
R⁶ Wasserstoff, (C₁-C₄)-Alkyl oder (C₃-C₆)-Cycloalkyl bedeutet,
R¹ für einen an ein Kohlenstoffatom des Ringes 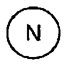 gebundenen Substituenten ausgewählt aus der Reihe Cyano, (C₁-C₄)-Alkyl, Oxo und (C₃-C₆)-Cycloalkyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
R² für einen an ein Stickstoffatom des Ringes 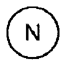 gebundenen Substituenten ausgewählt aus der Reihe (C₁-C₄)-Alkyl, (C₁-C₄)-Alkylcarbonyl, (C₁-C₄)-Alkoxy-carbonyl, (C₁-C₄)-Alkylsulfonyl und (C₃-C₆)-Cycloalkyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylaniino, Di-(C₁-C₄)-alkylamino, (C₃-C₆)-Cycloalkyl und 4- bis 6-gliedriges Heterocyclyl sowie bis zu dreifach mit Fluor substituiert sein kann
und
(C₃-C₆)-Cycloalkyl seinerseits bis zu zweifach, gleich oder verschieden, mit einem Rest ausgewählt aus der Reihe Fluor, (C₁-C₄)-Alkyl, Hydroxy, (C₁-C₄)-Alkoxy, Amino, Mono-(C₁-C₄)-alkylamino und Di-(C₁-C₄)-alkylamino substituiert sein kann,
m für die Zahl 0, 1 oder 2 steht, wobei im Fall, dass der Substituent R¹ zweifach auftritt, seine Bedeutungen gleich oder verschieden sein können,
n für die Zahl 0 oder 1 steht,
wobei die Summe aus m und n gleich der Zahl 1, 2 oder 3 ist,
R³ für Methyl, Ethyl oder Trifluormethyl steht,
R⁴ für einen Substituenten ausgewählt aus der Reihe Fluor, Chlor, Cyano, Pentafluorthio, (C₁-C₆)-Alkyl, Tri-(C₁-C₄)-alkylsilyl, -OR⁷, -NR⁷R⁸, -SR⁷, -S(=O)-R⁷, -S(=O)₂-R⁷, -S(=O)(=NH)-R⁷, -S(=O)(=NCH₃)-R⁷, (C₃-C₆)-Cycloalkyl und 4- bis 6-gliedriges Heterocyclyl steht,
wobei (C₁-C₆)-Alkyl seinerseits bis zu dreifach mit Fluor sowie bis zu zweifach, gleich oder verschieden, mit einem Rest ausgewählt aus der Reihe -OR⁷, -NR⁷R⁸, -N(R⁷)-C(=O)-R⁸, -C(=O)-NR⁷R⁸, (C₃-C₆)-Cycloalkyl, 4- bis 6-gliedriges Heterocyclyl und 5- oder 6-gliedriges Heteroaryl substituiert sein kann
und wobei die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Hydroxy, (C₁-C₄)-Alkoxy, Trifluormethoxy, Oxo und (C₁-C₄)-Alkylcarbonyl
sowie die genannte Heteroaryl-Gruppe ihrerseits bis zu zweifach, gleich oder verschieden, mit einem Rest ausgewählt aus der Reihe Fluor, Chlor, Cyano, (C₁-C₄)-Alkyl, (C₁-C₄)-Alkoxy und Trifluormethoxy substituiert sein können, wobei die hierin genannten (C₁-C₄)-Alkyl-Substituenten ihrerseits mit Hydroxy, Methoxy, Trifluormethoxy, Ethoxy, Acetoxy, Aminocarbonyl, Methylaminocarbonyl oder Dimethylaminocarbonyl oder bis zu dreifach mit Fluor substituiert sein können,
und worin
R⁷ und R⁸ unabhängig voneinander bei jedem einzelnen Auftreten Wasserstoff, (C₁-C₄)-Alkyl, (C₃-C₆)-Cycloalkyl oder 4- bis 6-gliedriges Heterocyclyl bedeuten, wobei (C₁-C₄)-Alkyl bis zu dreifach mit Fluor sowie bis zu zweifach, gleich oder verschieden, mit einem Rest ausgewählt aus der Reihe Hydroxy, (C₁-C₄)-Alkoxy, Trifluormethoxy, (C₃-C₆)-Cycloalkyl und 4- bis 6-gliedriges Heterocyclyl substituiert sein kann
und
die genannten Cycloalkyl- und Heterocyclyl-Gruppen bis zu zweifach, gleich oder verschieden, mit einem Rest ausgewählt aus der Reihe Fluor, (C₁-C₄)-Alky), Trifluormethyl, Hydroxy, (C₁-C₄)-Alkoxy, Trifluonnethoxy, Oxo und (C₁-C₄)-Alkylcarbonyl substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für einen Substituenten ausgewählt aus der Reihe Fluor, Chlor und Methyl steht und
p für die Zahl 0 oder 1 steht,
sowie ihre Salze, Solvate und Solvate der Salze.

9. Verbindung der Formel (I) nach Anspruch 1, 2, 3 oder 6, in welcher der Ring Ⓐ für einen Pyridyl-Ring der Formel 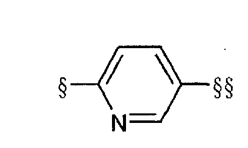 steht, worin 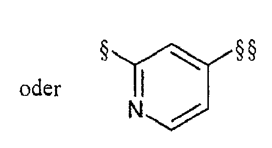
§ die Verknüpfungsstelle mit der angrenzenden Gruppe X und
§§ die Verknüpfungsstelle mit der angrenzenden CH₂-Gruppe bezeichnen,
der Ring 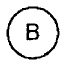 mit dem Substituenten R³ für einen Heteroaryl-Ring der Formel 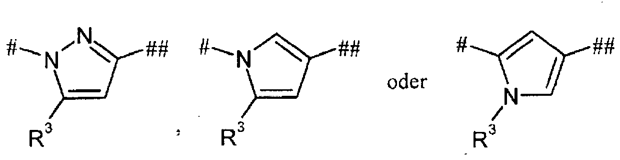 steht, worin
# die Verknüpfungsstelle mit der angrenzenden CH₂-Gruppe
und
## die Verknüpfungsstelle mit dem Ring 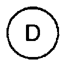 bezeichnen,
der Ring 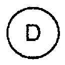 für einen Heteroaryl-Ring der Formel 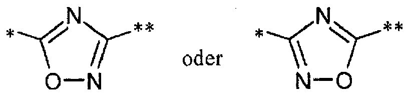 steht, worin
* die Verknüpfungsstelle mit dem Ring 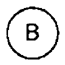 und
** die Verknüpfungsstelle mit dem Ring 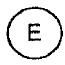 bezeichnen, der Ring 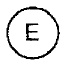 mit den Substituenten R⁴ und R⁵ für einen Phenyl-Ring der Formel 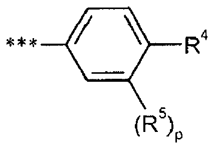 steht, worin
*** die Verknüpfungsstelle mit dem Ring 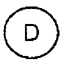 bezeichnet, der Ring 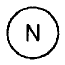 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 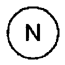 und
◆◆ die Verknüpfungsstelle mit dem Ring 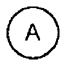 bezeichnen,
q die Zahl 0 oder 1 bedeutet und
R⁶ Wasserstoff, Methyl, Ethyl, Isopropyl, Cyclopropyl oder Cyclobutyl bedeutet,
R¹ für einen an ein Kohlenstoffatom des Ringes 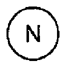 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkyla_{M}ino, Di-(C₁-C₄)-alkylamino, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-aikylamino und Di-(C₁-C₄)-alkyl-amino sowie bis zu dreifach mit Fluor substituiert sein kann,
m für die Zahl 0 oder 1 steht,
R² für einen an ein Stickstoffatom des Ringes 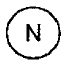 gebundenen Substituenten ausgewählt aus der Reihe (C₁-C₄)-Alkyl, (C₁-C₄)-Alkylcarbonyl, (C₁-C₄)-Alkoxy-carbonyl, (C₁-C₄)-Alkylsulfonyl, Cyclopropyl und Cyclobutyl steht, wobei die Alkyl-Gruppe in (C₁-C₄)-Alkyl, (C₁-C₄)-Alkylcarbonyl, (C₁-C₄)-Alkoxy-carbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cycloalkyl und 4- oder 5-gliedriges Heterocyclyl sowie bis zu dreifach mit Fluor substituiert sein kann,
n für die Zahl 0 oder 1 steht,
R³ für Methyl steht,
R⁴ für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cycloalkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der genannte (C₁-C₄)-Alkyl-Substituent seinerseits mit Methoxy, Trifluormethoxy oder Ethoxy substituiert sein kann, und worin
R⁷ und R⁸ unabhängig voneinander bei jedem einzelnen Auftreten Wasserstoff, (C₁-C₄)-Alkyl oder (C₃-C₆)-Cycloalkyl bedeuten, wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann und die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für Fluor steht und
p für die Zahl 0 oder 1 steht,
sowie ihre Salze, Solvate und Solvate der Salze.

10. Verbindung der Formel (I) nach Anspruch 1, 2, 4 oder 7, in welcher der Ring 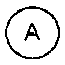 für einen Phenyl-Ring steht und die angrenzenden Gruppen X und CH₂ in 1,3- oder 1,4-Relation zueinander an diesen Phenyl-Ring gebunden sind, der Ring 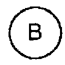 mit dem Substituenten R³ für einen Heteroaryl-Ring der Formel 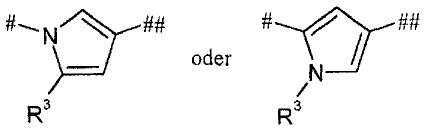 steht, worin
# die Verknüpfungsstelle mit der angrenzenden CH₂-Gruppe und
## die Verknüpfungsstelle mit dem Ring 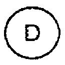 bezeichnen, der Ring 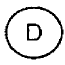 für einen Heteroaryl-Ring der Formel 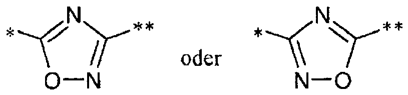 steht, worin
* die Verknüpfungsstelle mit dem Ring 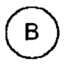 und
** die Verknüpfungsstelle mit dem Ring 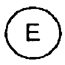 bezeichnen, der Ring 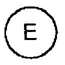 mit den Substituenten R⁴ und R⁵ für einen Phenyl-Ring der Formel 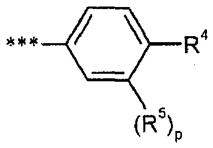 steht, worin
*** die Verknüpfungsstelle mit dem Ring 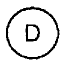 bezeichnet,
der Ring 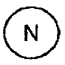 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 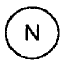 und
◆◆ die Verknüpfungsstelle mit dem Ring 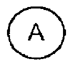 bezeichnen,
q die Zahl 0 oder 1 bedeutet und
R⁶ Wasserstoff, Methyl, Ethyl, Isopropyl, Cyclopropyl oder Cyclobutyl bedeutet,
R¹ für einen an ein Kohlenstoffatom des Ringes 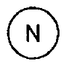 gebundenen Substituenten ausgewählt aus der Reihe Fluor, Cyano, (C₁-C₄)-Alkyl, Hydroxy, (C₁-C₄)-Alkoxy, Oxo, Amino, Mono-(C₁-C₄)-alkylamino, Di-(C₁-C₄)-alkylamino, Cyclopropyl und Cyclobutyl steht, wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkylamino sowie bis zu dreifach mit Fluor substituiert sein kann,
m für die Zahl 0 oder 1 steht,
R² für einen an ein Stickstoffatom des Ringes 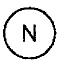 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht, wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cycloalkyl und 4- oder 5-gliedriges Heterocyclyl sowie bis zu dreifach mit Fluor substituiert sein kann,
n für die Zahl 0 oder 1 steht,
R³ für Methyl steht,
R⁴ für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cycloalkyl und 4- bis 6-gliedriges Heterocyclyl steht, wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann und die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können, wobei der genannte (C₁-C₄)-Alkyl-Substituent seinerseits mit Methoxy, Trifluormethoxy oder Ethoxy substituiert sein kann, und worin
R⁷ und R⁸ unabhängig voneinander bei jedem einzelnen Auftreten Wasserstoff, (C₁-C₄)-Alkyl oder (C₃-C₆)-Cycloalkyl bedeuten, wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann und die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für Fluor steht und
p für die Zahl 0 oder 1 steht,
sowie ihre Salze, Solvate und Solvate der Salze.

11. Verbindung der Formel (1) nach Anspruch 1, 2, 5 oder 8, in welcher der Ring 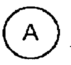 für einen Phenyl-Ring steht und die angrenzenden Gruppen X und CH₂ in 1,3- oder 1,4-Relation zueinander an diesen Phenyl-Ring gebunden sind, der Ring 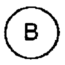 mit dem Substituenten R³ für einen Heteroaryl-Ring der Formel 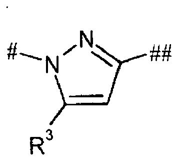 steht, worin
# die Verknüpfungsstelle mit der angrenzenden CH₂-Gruppe und
## die Verknüpfungsstelle mit dem Ring 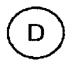 bezeichnen,
der Ring 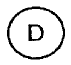 für einen Heteroaryl-Ring der Formel 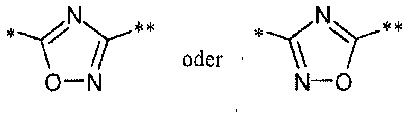 steht, worin
* die Verknüpfungsstelle mit dem Ring 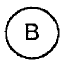 und
** die Verknüpfungsstelle mit dem Ring 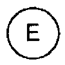 bezeichnen,
der Ring 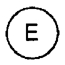 mit den Substituenten R⁴ und R⁵ für einen Phenyl-Ring der Formel 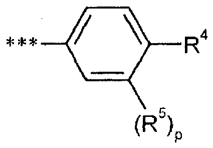 steht, worin
*** die Verknüpfungsstelle mit dem Ring 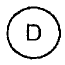 bezeichnet,
der Ring 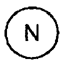 für einen gesättigten 4- bis 10-gliedrigen Aza-Heterocyclus steht, der mindestens ein N-Atom als Ringglied enthält und darüber hinaus ein weiteres Hetero-Ringglied aus der Reihe N, O, S oder S(O)₂ enthalten kann,
X für eine Bindung oder für ◆-(CH₂)_{q}-N(R⁶)-◆◆, -C(=O)- oder ◆-N(R⁶)-C(=O)-◆◆ steht, worin
◆ die Verknüpfungsstelle mit dem Ring 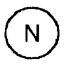 und
◆◆ die Verknüpfungsstelle mit dem Ring 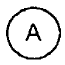 bezeichnen,
q die Zahl 0 oder 1 bedeutet und
R⁶ Wasserstoff, Methyl, Ethyl, Isopropyl, Cyclopropyl oder Cyclobutyl bedeutet,
R¹ für einen an ein Kohlenstoffatom des Ringes 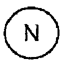 gebundenen Substituenten ausgewählt aus der Reihe Cyano, (C₁-C₄)-Alkyl, Oxo, Cyclopropyl und Cyclobutyl steht,
wobei (C₁-C₄)-Alkyl seinerseits mit einem Rest ausgewählt aus der Reihe Hydroxy, (C₁-C₄)-Alkoxy, Amino, Mono-(C₁-C₄)-alkylamino und Di-(C₁-C₄)-alkyl-amino sowie bis zu dreifach mit Fluor substituiert sein kann,
R² für einen an ein Stickstoffatom des Ringes 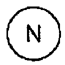 gebundenen Substituenten ausgewählt aus der Reihe (C₁-C₄)-Alkyl, (C₁-C₄)-Alkylcarbonyl, (C₁-C₄)-Alkoxycarbonyl, (C₁-C₄)-Alkylsulfonyl, Cyclopropyl und Cyclobutyl steht,
wobei die Alkyl-Gruppe in (C₁-C₄)-Alkyl, (C₁-C₄)-Alkylcarbonyl, (C₁-C₄)-Alkoxycarbonyl und (C₁-C₄)-Alkylsulfonyl ihrerseits mit einem Rest ausgewählt aus der Reihe Hydroxy, (C₁-C₄)-Alkoxy, Amino, Mono-(C₁-C₄)-alkylamino, Di-(C₁-C₄)-alkylamino, (C₃-C₅)-Cycloakyl und 4- oder 5-gliedriges Heterocyclyl sowie bis zu dreifach mit Fluor substituiert sein kann,
m für die Zahl 0 oder 1 steht,
n für die Zahl 0 oder 1 steht,
wobei die Summe aus m und n gleich der Zahl 1 oder 2 ist,
R³ für Methyl steht,
R⁴ für einen Substituenten ausgewählt aus der Reihe Chlor, Pentafluorthio, (C₁-C₆)-Alkyl, Trimethylsilyl, -OR⁷, -SR⁷, -S(=O)-R⁷, -S(=O)₂-R⁷, -S(=O)(=NCH₃)-CF₃, (C₃-C₆)-Cycloalkyl und 4- bis 6-gliedriges Heterocyclyl steht,
wobei (C₁-C₆)-Alkyl seinerseits mit einem Rest ausgewählt aus der Reihe -OR⁷, -NR⁷R⁸, -C(=O)-NR⁷R⁸, (C₃-C₆)-Cycloalkyl und 4- bis 6-gliedriges Heterocyclyl sowie bis zu dreifach mit Fluor substituiert sein kann
und
die genannten Cycloalkyl- und Heterocyclyl-Gruppen ihrerseits bis zu zweifach, gleich oder verschieden, mit einem Rest ausgewählt aus der Reihe Fluor, (C₁-C₄)-Alkyl, Trifluormethyl, (C₁-C₄)-Alkoxy, Trifluormethoxy und Oxo substituiert sein können,
wobei der genannte (C₁-C₄)-Alkyl-Substituent seinerseits mit Methoxy, Trifluormethoxy oder Ethoxy substituiert sein kann, und worin
R⁷ und R⁸ unabhängig voneinander bei jedem einzelnen Auftreten Wasserstoff, (C₁-C₄)-Alkyl oder (C₃-C₆)-Cycloalkyl bedeuten,
wobei (C₁-C₄)-Alkyl mit einem Rest ausgewählt aus der Reihe Hydroxy, (C₁-C₄)-Alkoxy, Trifluormethoxy und (C₃-C₆)-Cycloalkyl sowie bis zu dreifach mit Fluor substituiert sein kann
und
die genannten Cycloalkyl-Gruppen bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy und Trifluormethoxy substituiert sein können, oder
R⁷ und R⁸ im Fall, dass beide an ein Stickstoffatom gebunden sind, zusammen mit diesem Stickstoffatom einen 4- bis 6-gliedrigen Heterocyclus bilden, der ein weiteres Ring-Heteroatom aus der Reihe N, O, S oder S(O)₂ enthalten kann und der bis zu zweifach, gleich oder verschieden, mit einem Rest ausgewählt aus der Reihe Fluor, (C₁-C₄)-Alkyl, Trifluormethyl, Hydroxy, (C₁-C₄)-Alkoxy, Oxo und (C₁-C₄)-Alkylcarbonyl substituiert sein kann,
R⁵ für Fluor steht und
p für die Zahl 0 oder 1 steht,
sowie ihre Salze, Solvate und Solvate der Salze.

12. Verbindung, wie in einem der Ansprüche 1 bis 11 definiert, zur Behandlung und/oder Prävention von Krankheiten.

13. Verbindung, wie in einem der Ansprüche 1 bis 11 definiert, zur Verwendung in einem Verfahren zur Behandlung und/oder Prävention von Krebs- oder Tumorerkrankungen.

14. Verbindung, wie in einem der Ansprüche 1 bis 11 definiert, zur Verwendung in einem Verfahren zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fibrotischen Erkrankungen von Niere und Lunge, Psoriasis, diabetischer Retinopathie, Makuladegeneration, rheumatischer Arthritis und der Chugwash-Polyzythämie.

15. Verwendung einer Verbindung, wie in einem der Ansprüche 1 bis 11 definiert, zur Herstellung eines Arzneimittels zur Behandlung und/oder Prävention von Krebs- oder Tumorerkrankungen.

16. Verwendung einer Verbindung, wie in einem der Ansprüche 1 bis 11 definiert, zur Herstellung eines Arzneimittels zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fibrotischen Erkrankungen von Niere und Lunge, Psoriasis, diabetischer Retinopathie, Makuladegeneration, rheumatischer Arthritis und der Chugwash-Polyzythämie.

17. Arzneimittel enthaltend eine Verbindung, wie in einem der Ansprüche 1 bis 11 definiert, in Kombination mit einem oder mehreren inerten, nicht-toxischen, pharmazeutisch geeigneten Hilfsstoffen.

18. Arzneimittel enthaltend eine Verbindung, wie in einem der Ansprüche 1 bis 11 definiert, in Kombination mit einem oder mehreren weiteren Wirkstoffen.

19. Arzneimittel nach Anspruch 17 oder 18 zur Behandlung und/oder Prävention von Krebs- oder Tumorerkrankungen.

20. Arzneimittel nach Anspruch 17 oder 18 zur Behandlung und/oder Prävention von ischämischen Herz-Kreislauf-Erkrankungen, Herzinsuffizienz, Herzinfarkt, Arrhythmie, Schlaganfall, pulmonaler Hypertonie, fbrotischen Erkrankungen von Niere und Lunge, Psoriasis, diabetischer Retinopathie, Makuladegeneration, rheumatischer Arthritis und der Chugwash-Polyzythämie.

21. Verfahren zur Herstellung von Verbindungen der Formel (I-D) 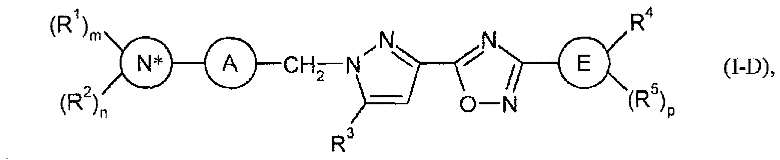 in welcher die Ringe A und E sowie R¹, R², R³, R⁴, R⁵, m, n und p jeweils die in den Ansprüchen 1 bis 11 angegebenen Bedeutungen haben
und
der Ring N* für einen über ein Ring-Stickstoffatom an den Ring A gebundenen Ring N, welcher wie in den Ansprüchen 1 bis 11 definiert ist, steht,
**dadurch gekennzeichnet, dass** man ein *N'*-Hydroxyamidin der Formel (IX) 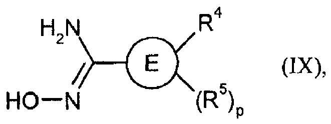 in welcher der Ring E sowie R⁴, R⁵ und p die oben angegebenen Bedeutungen haben,
zunächst entweder
[A] mit einer Pyrazolcarbonsäure der Formel (XXVII) 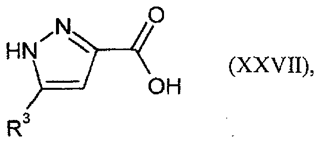 in welcher R³ die oben angegebene Bedeutung hat,
zu einem 1,2,4-Oxadiazol-Derivat der Formel (XXVIII) 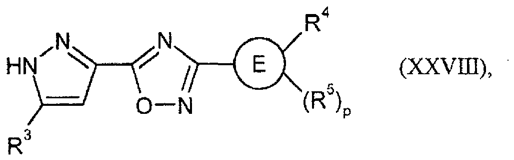 in welcher der Ring E sowie R³, R⁴, R⁵ und p die oben angegebenen Bedeutungen haben,
kondensiert und dieses dann in Gegenwart einer Base mit einer Verbindung der Formel (III) 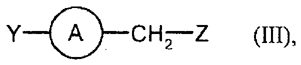 in welcher der Ring A die oben angegebene Bedeutung hat,
Y für Chlor, Brom oder Iod steht
und
Z für Chlor, Brom, Iod, Mesylat, Triflat oder Tosylat steht,
zu einer Verbindung der Formel (XXIX) 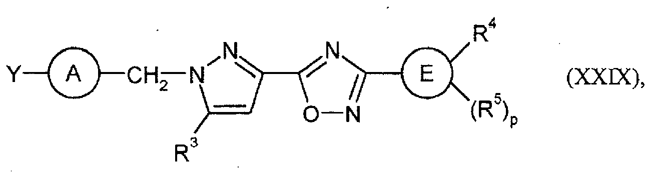 in welcher die Ringe A und E sowie R³, R⁴, R⁵, p und Y die oben angegebenen Bedeutungen haben,
alkyliert oder
[B] mit einer Pyrazolcarbonsäure der Formel (XXX) 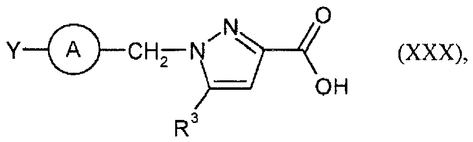 in welcher der Ring A sowie R³ die oben angegebenen Bedeutungen haben und
Y für Chlor, Brom oder Iod steht,
zu der Verbindung der Formel (XXIX) 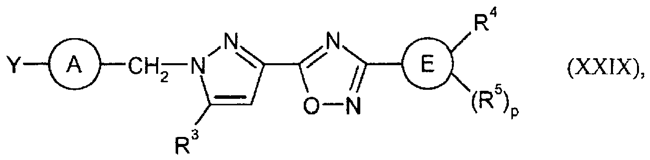 in welcher die Ringe A und E sowie R³, R⁴, R⁵, p und Y die oben angegebenen Bedeutungen haben,
kondensiert
und anschließend die so erhaltene Verbindung der Formel (XXIX) gegebenenfalls in Gegenwart eines Palladium-Katalysators und/oder einer Base mit einer Verbindung der Formel (XXXI) 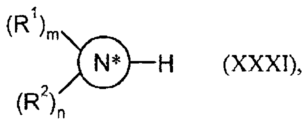 in welcher der Ring N* sowie R¹, R², m und n die oben angegebenen Bedeutungen haben und das angezeigte Wasserstoffatom an ein Stickstoffatom des Ringes N* gebunden ist,
umsetzt.

## Claims

1. Compound of the formula (I) 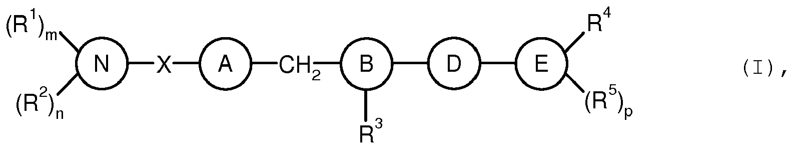 in which
the ring 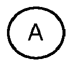 represents a phenyl or pyridyl ring,
the ring 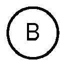 with the substituent R³ represents a
heteroaryl ring of the formula 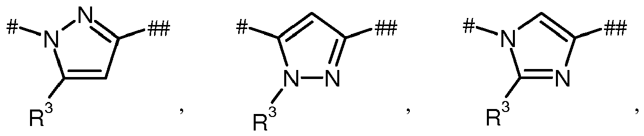 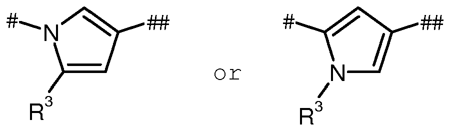 wherein
# designates the linkage point with the adjacent CH₂ group
and
## designates the linkage point with the ring 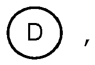 the ring 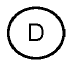 represents a heteroaryl ring of the formula 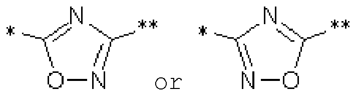 wherein
* designates the linkage point with the ring 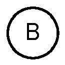 or and
** designates the linkage point with the ring 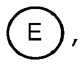 the ring 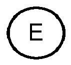 represents a phenyl or pyridyl ring, the ring 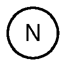 represents a saturated 4- to 10-membered aza-heterocycle, which contains at least one N atom as a ring member and in addition can contain one or two further hetero ring members from the series N, 0, S and/or S(O)₂,
and their salts, solvates and solvates of the salts.
X represents a bond or ◆-(CH₂)_{q}-N(R⁶)-◆◆, ◆-N(R⁶)-(CH₂)_{q}-◆◆, -O-, -S-, -C(=O)-, -S(=O)₂-, ◆-C(=O)-N(R⁶)-◆◆ or ◆-N(R⁶)-C(=O)-◆◆, wherein
◆ designates the linkage point with the ring 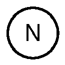 and
◆◆ designates the linkage point with the ring 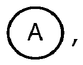
q denotes the number 0, 1 or 2
and
R⁶ denotes hydrogen, (C₁-C₆)-alkyl or (C₃-C₆)-cycloalkyl, wherein (C₁-C₆)-alkyl and (C₃-C₆)-cycloalkyl can each be substituted by hydroxyl or (C₁-C₄) -alkoxy,
R¹ represents a substituent bonded to a carbon atom of the ring 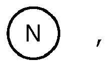 chosen from the series fluorine, cyano, (C₁-C₆)-alkyl, hydroxyl, (C₁-C₆)-alkoxy, oxo, amino, mono-(C₁-C₆)-alkylamino, di-(C₁-C₆)-alkylamino and (C₃-C₆)-cycloalkyl, wherein (C₁-C₆)-alkyl in its turn can be substituted up to three times by fluorine and up to two times in an identical or different manner by a radical chosen from the series hydroxyl, (C₁-C₄)-alkoxy, amino, mono-(C₁-C₄)-alkylamino and di-(C₁-C₄)-alkylamino
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
m represents the number 0, 1, 2, 3 or 4, wherein in the case where the substituent R¹ occurs several times, its meanings can be identical or different,
R² represents a substituent bonded to a nitrogen atom of the ring 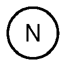 , chosen from the series (C₁-C₆)-alkyl, (C₁-C₆)-alkylcarbonyl, (C₁-C₆)-alkoxycarbonyl, (C₁-C₆)-alkylsulfonyl and (C₃-C₆)-cycloalkyl, wherein the alkyl group in (C₁-C₆)-alkyl, (C₁-C₆)-alkylcarbonyl, (C₁-C₆)-alkoxycarbonyl and (C₁-C₆)-alkylsulfonyl in its turn can be substituted up to three times by fluorine and up to two times in an identical or different manner by a radical chosen from the series hydroxyl, (C₁-C₄)-alkoxy, amino, mono- (C₁-C₄) -alkylamino, di- (C₁-C₄) -alkylamino (C₃-C₆)-cycloalkyl and 4- to 6-membered heterocyclyl
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
n represents the number 0 or 1 or also, if the azaheterocycle 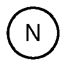 contains further N atoms as ring members, the number 2, wherein in the case where the substituent R² occurs twice, its meanings can be identical or different,
R³ represents methyl, ethyl or trifluoromethyl, R⁴ represents hydrogen or a substituent chosen from the series halogen, cyano, pentafluorothio, (C₁-C₆)-alkyl, tri-(C₁-C₄)-alkylsilyl, -OR⁷, -NR⁷R⁸, -N(R⁷)-C(=O)-R⁸, -N(R⁷)-C(=O)-OR⁸, -N(R⁷)-S(=O)₂-R⁸, -C(=O)-OR⁷, -C(=O)-NR⁷R⁸, -SR⁷, -S(=O)-R⁷, -S(=O)₂-R⁷, -S(=O)₂-NR⁷R⁸, -S(=O)(=NH)-R⁷, -S(=O)(=NCH₃)-R⁷, (C₃-C₆)-cycloalkyl, 4- to 6-membered heterocyclyl and 5- or 6-membered heteroaryl,
wherein (C₁-C₆)-alkyl in its turn can be substituted up to three times by fluorine and up to two times in an identical or different manner by a radical chosen from the series -OR⁷, -NR⁷R⁸, -N(R⁷)-C(=O)-R⁸, -N(R⁷)-C(=O)-OR⁸, -C(=O)-OR⁷, -C(=O)-NR⁷R⁸, (C₃-C₆) -cycloalkyl, 4- to 6-membered heterocyclyl and 5- or 6-membered heteroaryl
and wherein
the cycloalkyl and heterocyclyl groups mentioned in their turn can be substituted up to two times in an identical or different manner by a radical chosen from the series fluorine, (C₁-C₄)-alkyl, hydroxyl, (C₁-C₄)-alkoxy, trifluoromethoxy, oxo, amino, mono- (C₁-C₄) -alkylamino, di- (C₁-C₄) -alkyl-amino, (C₁-C₄)-alkylcarbonylamino, (C₁-C₄)-alkoxycarbonylamino, (C₁-C₄)-alkylcarbonyl and (C₁-C₄)-alkoxycarbonyl
and
the heteroaryl groups mentioned in their turn can be substituted up to two times in an identical or different manner by a radical chosen from the series fluorine, chlorine, cyano, (C₁-C₄)-alkyl, (C₁-C₄)-alkoxy and trifluoromethoxy
wherein the (C₁-C₄)-alkyl substituents mentioned herein in their turn can be substituted by hydroxyl, (C₁-C₄)-alkoxy, trifluoromethoxy, (C₁-C₄)-alkylcarbonyloxy, aminocarbonyl, mono-(C₁-C₄)-alkylaminocarbonyl or di-(C₁-C₄)-alkylaminocarbonyl or up to three times by fluorine,
and wherein
R⁷ and R⁸ independently of each other for each individual occurrence denote hydrogen, (C₁-C₆)-alkyl, (C₃-C₆)-cycloalkyl or 4- to 6-membered heterocyclyl,
wherein (C₁-C₆)-alkyl can be substituted up to three times by fluorine and up to two times in an identical or different manner by a radical chosen from the series hydroxyl, (C₁-C₄)-alkoxy, trifluoromethoxy, amino, mono-(C₁-C₄)-alkylamino, di-(C₁-C₄)-alkylamino, (C₁-C₄)-alkoxycarbonyl, (C₃-C₆)-cycloalkyl and 4- to 6-membered heterocyclyl
and
the cycloalkyl and heterocyclyl groups mentioned can be substituted up to two times in an identical or different manner by a radical chosen from the series fluorine, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkyl-amino, (C₁-C₄)-alkylcarbonyl and (C₁-C₄)-alkoxycarbonyl, or
R⁷ and R⁸ in the case where both are bonded to a nitrogen atom form a 4- to 6-membered heterocycle together with this nitrogen atom, which can contain a further ring hetero atom from the series N, O, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino, (C₁-C₄)-alkylcarbonyl and (C₁-C₄)-alkoxycarbonyl,
R⁵ represents a substituent chosen from the series fluorine, chlorine, cyano, methyl, trifluoromethyl and hydroxyl and
p represents the number 0, 1 or 2, wherein in the case where the substituent R⁵ occurs twice, its meanings can be identical or different,

2. Compound of the formula (I) according to claim 1, in which
the ring 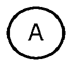 represents a phenyl or pyridyl ring and the adjacent groups X and CH₂ are bonded to ring
carbon atoms 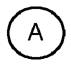 in 1, 3 or 1, 4 relation to one
another and
the ring 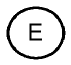 with the substituents R⁴ and R⁵ represents a phenyl ring of the formula 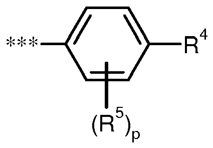 wherein
*** designates the linkage point with the ring 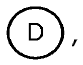
and their salts, solvates and solvates of the salts.

3. Compound of the formula (I) according to claim 1 or 2, in which
the ring 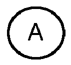 represents a pyridyl ring and the adjacent groups X and CH₂ are bonded to ring carbon atoms of this pyridyl ring in 1,3 or 1,4 relation to one another
and
the ring 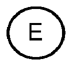 with the substituents R⁴ and R⁵ represents a phenyl ring of the formula 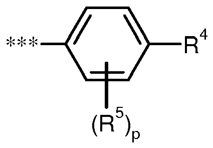 wherein
*** designates the linkage point with the ring 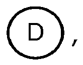
and their salts, solvates and solvates of the salts.

4. Compound of the formula (I) according to claim 1 or 2, in which
the ring 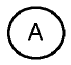 represents a phenyl ring and the adjacent groups X and CH₂ are bonded to this phenyl ring in 1,3 or 1,4 relation to one another,
the ring 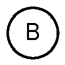 with the substituent R³ represents a
heteroaryl ring of the formula 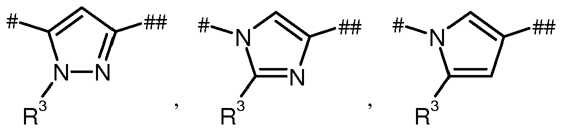 or 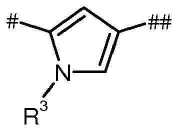 wherein
# designates the linkage point with the adjacent CH₂ group
and
## designates the linkage point with the ring 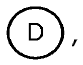
and
the ring 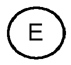 with the substituents R⁴ and R⁵ represents a phenyl ring of the formula 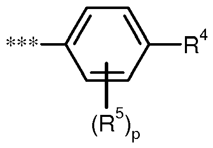 wherein
*** designates the linkage point with the ring 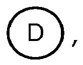 and their salts, solvates and solvates of the salts.

5. Compound of the formula (I) according to claim 1 or 2, in which
the ring 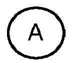 represents a phenyl ring and the adjacent groups X and CH₂ are bonded to this phenyl ring in 1,3 or 1,4 relation to one another,
the ring 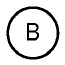 with the substituent R³ represents a heteroaryl ring of the formula 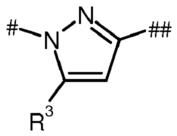 wherein
# designates the linkage point with the adjacent CH₂ group
and
## designates the linkage point with the ring 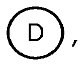
the ring 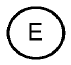 with the substituents R⁴ and R⁵ represents a phenyl ring of the formula 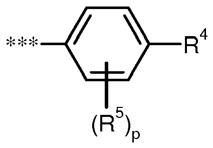 wherein
*** designates the linkage point with the ring 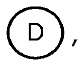
R¹ represents a substituent bonded to a carbon atom of the ring 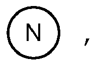 chosen from the series cyano, (C₁-C₆)-alkyl, oxo and (C₃-C₆)-cycloalkyl,
wherein (C₁-C₆)-alkyl in its turn can be substituted up to three times by fluorine and up to two times in an identical or different manner by a radical chosen from the series hydroxyl, (C₁-C₄)-alkoxy, amino, mono-(C₁-C₄)-alkylamino and di-(C₁-C₄)-alkylamino
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
R² represents a substituent bonded to a nitrogen atom of the ring 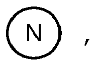 chosen from the series (C₁-C₆)-alkyl, (C₁-C₆)-alkylcarbonyl, (C₁-C₆)-alkoxycarbonyl, (C₁-C₆)-alkylsulfonyl and (C₃-C₆)-cycloalkyl,
wherein the alkyl group in (C₁-C₆)-alkyl, (C₁-C₆)-alkylcarbonyl, (C₁-C₆)-alkoxycarbonyl and (C₁-C₆)-alkylsulfonyl in its turn can be substituted up to three times by fluorine and up to two times in an identical or different manner by a radical chosen from the series hydroxyl, (C₁-C₄)-alkoxy, amino, mono-(C₁-C₄)-alkylamino, di- (C₁-C₄) -alkylamino (C₃-C₆)-cycloalkyl and 4- to 6-membered heterocyclyl
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
m represents the number 0, 1, 2, 3 or 4, wherein in the case where the substituent R¹ occurs several times, its meanings can be identical or different, and
n represents the number 0 or 1 or also, if the azaheterocycle 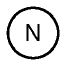 contains further N atoms as ring members, the number 2,
wherein in the case where the substituent R² occurs twice, its meanings can be identical or different,
wherein the sum of m and n does not equal the number 0, and their salts, solvates and solvates of the salts.

6. Compound of the formula (I) according to claim 1, 2 or 3, in which
the ring 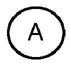 represents a pyridyl ring and the adjacent groups X and CH₂ are bonded to ring carbon atoms of this pyridyl ring in 1,3 or 1,4 relation to one another,
the ring 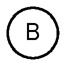 with the substituent R³ represents a heteroaryl ring of the formula 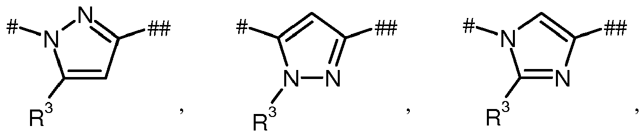 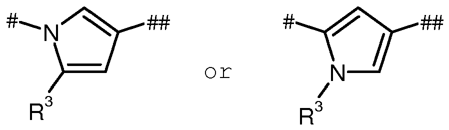 wherein
# designates the linkage point with the adjacent CH₂ group
and
## designates the linkage point with the ring 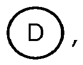 the ring* 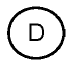 represents a heteroaryl ring of the formula 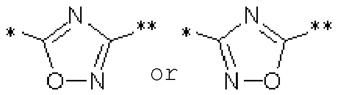 wherein
* designates the linkage point with the ring 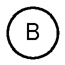 and
** designates the linkage point with the ring 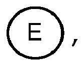 the ring 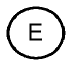 with the substituents R⁴ and R⁵ represents a phenyl ring of the formula 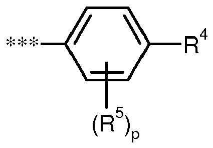 wherein
*** designates the linkage point with the ring 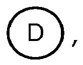 the ring 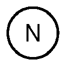 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -O-, -S-, - C(=O)-, -S(=O)₂- or ◆-N(R⁶)-C(=O)-◆◆, wherein
◆ designates the linkage point with the ring 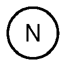 and
◆◆ designates the linkage point with the ring 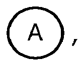
q denotes the number 0, 1 or 2 and
R⁶ denotes hydrogen, (C₁-C₄) -alkyl or (C₃-C₆)-cycloalkyl,
R¹ represents a substituent bonded to a carbon atom of the ring 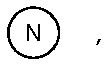 chosen from the series fluorine, cyano, (C₁-C₄)-alkyl, hydroxyl, (C₁-C₄)-alkoxy, oxo, amino, mono-(C₁-C₄)-alkylamino, di-(C₁-C₄)-alkylamino and (C₃-C₆)-cycloalkyl, wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine and (C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino, m represents the number 0, 1 or 2, wherein in the case where the substituent R¹ occurs twice, its meanings can be identical or different,
R² represents a substituent bonded to a nitrogen atom of the ring 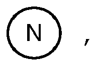 chosen from the series (C₁-C₄)-alkyl, (C₁-C₄)-alkylcarbonyl, (C₁-C₄)-alkoxycarbonyl, (C₁-C₄)-alkylsulfonyl and (C₃-C₆)-cycloalkyl, wherein the alkyl group in (C₁-C₄)-alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino (C₃-C₆)-cycloalkyl and 4- to 6-membered heterocyclyl and up to three times by fluorine
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
n represents the number 0 or 1,
R³ represents methyl, ethyl or trifluoromethyl,
R⁴ represents a substituent chosen from the series fluorine, chlorine, cyano, pentafluorothio, (C₁-C₆)-alkyl, tri-(C₁-C₄)-alkylsilyl, -OR⁷, -NR⁷R⁸, -SR⁷, -S(=O)-R⁷, -S(=O)₂-R⁷, -S(=O)(=NH)-R⁷, - S (=0) (=NCH₃)-R⁷, (C₃-C₆)-cycloalkyl and 4- to 6-membered heterocyclyl,
wherein (C₁-C₆)-alkyl in its turn can be substituted up to three times by fluorine and up to two times in an identical or different manner by a radical chosen from the series -OR⁷, -NR⁷R⁸, -N(R⁷)-C(=O)-R⁸, -C(=O)-NR⁷R⁸, (C₃-C₆)-cycloalkyl, 4- to 6-membered heterocyclyl and 5- or 6-membered heteroaryl
and wherein
the cycloalkyl and heterocyclyl groups mentioned in their turn can be substituted up to two times in an identical or different manner by a radical chosen from the series fluorine, (C₁-C₄)-alkyl, hydroxyl, (C₁-C₄)-alkoxy, trifluoromethoxy, oxo and (C₁-C₄)-alkylcarbonyl
and
the heteroaryl group mentioned in its turn can be substituted up to two times in an identical or different manner by a radical chosen from the series fluorine, chlorine, cyano, (C₁-C₄)-alkyl, (C₁-C₄)-alkoxy and trifluoromethoxy
wherein the (C₁-C₄)-alkyl substituents mentioned herein in their turn can be substituted by hydroxyl, methoxy, trifluoromethoxy, ethoxy, acetoxy, aminocarbonyl, methylaminocarbonyl or dimethylaminocarbonyl or up to three times by fluorine,
and wherein
R⁷ and R⁸ independently of each other for each individual occurrence denote hydrogen, (C₁-C₄)-alkyl, (C₃-C₆)-cycloalkyl or 4- to 6-membered heterocyclyl, wherein (C₁-C₄)-alkyl can be substituted up to three times by fluorine and up to two times in an identical or different manner by a radical chosen from the series hydroxyl, (C₁-C₄)-alkoxy, trifluoromethoxy, (C₃-C₆)-cycloalkyl and 4- to 6-membered heterocyclyl
and
the cycloalkyl and heterocyclyl groups mentioned can be substituted up to two times in an identical or different manner by a radical chosen from the series fluorine, (C₁-C₄)-alkyl, trifluoromethyl, hydroxyl, (C₁-C₄)-alkoxy, trifluoromethoxy, oxo and (C₁-C₄)-alkylcarbonyl or
R⁷ and R⁸ in the case where both are bonded to a nitrogen atom form a 4- to 6-membered heterocycle together with this nitrogen atom, which can contain a further ring hetero atom from the series N, O, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl, R⁵ represents a substituent chosen from the series fluorine, chlorine and methyl
and
p represents the number 0 or 1,
and their salts, solvates and solvates of the salts.

7. Compound of the formula (I) according to claim 1, 2 or 4, in which
the ring Ⓐ represents a phenyl ring and the adjacent groups X and CH₂ are bonded to this phenyl ring in 1,3 or 1,4 relation to one another, the ring Ⓑ with the substituent R³ represents a heteroaryl ring of the formula 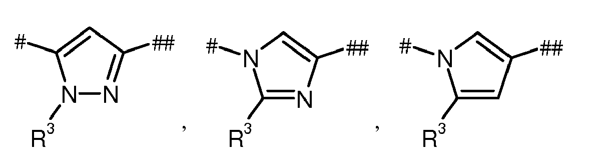 or 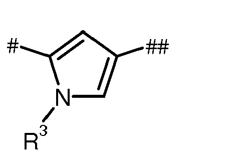 wherein
# designates the linkage point with the adjacent CH₂ group
and
## designates the linkage point with the ring 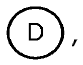
the ring 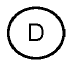 represents a heteroaryl ring of the formula 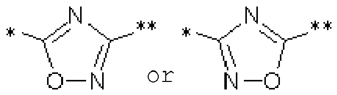 wherein
* designates the linkage point with the ring 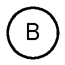
and
** designates the linkage point with the ring 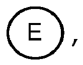
the ring 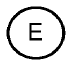 with the substituents R⁴ and R⁵ represents a phenyl ring of the formula 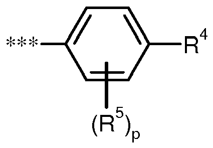 wherein
*** designates the linkage point with the ring 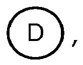
the ring 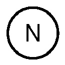 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -O-, -S-, - C(=O)-, -S(=O)₂- or ◆-N(R⁶)-C(=O)-◆◆, wherein
◆ designates the linkage point with the ring 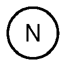
and
◆◆ designates the linkage point with the ring 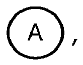
q denotes the number 0, 1 or 2
and
R⁶ denotes hydrogen, (C₁-C₄)-alkyl or (C₃-C₆)-cycloalkyl,
R¹ represents a substituent bonded to a carbon atom of the ring 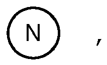 chosen from the series fluorine, cyano, (C₁-C₄)-alkyl, hydroxyl, (C₁-C₄)-alkoxy, oxo, amino, mono-(C₁-C₄)-alkylamino, di-(C₁-C₄)-alkylamino and (C₃-C₆)-cycloalkyl, wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
m represents the number 0, 1 or 2, wherein in the case where the substituent R¹ occurs twice, its meanings can be identical or different, R² represents a substituent bonded to a nitrogen atom of the ring 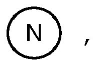 chosen from the series (C₁-C₄)-alkyl, (C₁-C₄)-alkylcarbonyl, (C₁-C₄)-alkoxycarbonyl, (C₁-C₄)-alkylsulfonyl and (C₃-C₆)-cycloalkyl, wherein the alkyl group in (C₁-C₄) -alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino (C₃-C₆)-cycloalkyl and 4- to 6-membered heterocyclyl and up to three times by fluorine
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
n represents the number 0 or 1,
R³ represents methyl, ethyl or trifluoromethyl,
R⁴ represents a substituent chosen from the series fluorine, chlorine, cyano, pentafluorothio, (C₁-C₆)-alkyl, tri-(C₁-C₄)-alkylsilyl, -OR⁷, -NR⁷R⁸, -SR⁷, -S(=O)-R⁷, -S(=O)₂-R⁷, -S(=O)(=NH)-R⁷, - S (=0) (=NCH₃)-R⁷, (C₃-C₆)-cycloalkyl and 4- to 6-membered heterocyclyl,
wherein (C₁-C₆)-alkyl in its turn can be substituted up to three times by fluorine and up to two times in an identical or different manner by a radical chosen from the series -OR⁷, -NR⁷R⁸, -N(R⁷)-C(=O)-R⁸, -C(=O)-NR⁷R⁸, (C₃-C₆)-cycloalkyl, 4- to 6-membered heterocyclyl and 5- or 6-membered heteroaryl
and wherein
the cycloalkyl and heterocyclyl groups mentioned in their turn can be substituted up to two times in an identical or different manner by a radical chosen from the series fluorine, (C₁-C₄)-alkyl, hydroxyl, (C₁-C₄)-alkoxy, trifluoromethoxy, oxo and (C₁-C₄)-alkylcarbonyl
and
the heteroaryl group mentioned in its turn can be substituted up to two times in an identical or different manner by a radical chosen from the series fluorine, chlorine, cyano, (C₁-C₄)-alkyl, (C₁-C₄)-alkoxy and trifluoromethoxy
wherein the (C₁-C₄)-alkyl substituents mentioned herein in their turn can be substituted by hydroxyl, methoxy, trifluoromethoxy, ethoxy, acetoxy, aminocarbonyl, methylaminocarbonyl or dimethylaminocarbonyl or up to three times by fluorine, and wherein
R⁷ and R⁸ independently of each other for each individual occurrence denote hydrogen, (C₁-C₄)-alkyl, (C₃-C₆)-cycloalkyl or 4- to 6-membered heterocyclyl,
wherein (C₁-C₄)-alkyl can be substituted up to three times by fluorine and up to two times in an identical or different manner by a radical chosen from the series hydroxyl, (C₁-C₄) -alkoxy, trifluoromethoxy, (C₃-C₆)-cycloalkyl and 4- to 6-membered heterocyclyl
and
the cycloalkyl and heterocyclyl groups mentioned can be substituted up to two times in an identical or different manner by a radical chosen from the series fluorine, (C₁-C₄)-alkyl, trifluoromethyl, hydroxyl, (C₁-C₄)-alkoxy, trifluoromethoxy, oxo and (C₁-C₄)-alkylcarbonyl or
R⁷ and R⁸ in the case where both are bonded to a nitrogen atom form a 4- to 6-membered heterocycle together with this nitrogen atom, which can contain a further ring hetero atom from the series N, O, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl,
R⁵ represents a substituent chosen from the series fluorine, chlorine and methyl and
p represents the number 0 or 1,
and their salts, solvates and solvates of the salts.

8. Compound of the formula (I) according to claim 1, 2 or 5, in which
the ring 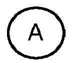 represents a phenyl ring and the adjacent groups X and CH₂ are bonded to this phenyl ring in 1,3 or 1,4 relation to one another,
the ring 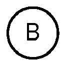 with the substituent R³ represents a heteroaryl ring of the formula 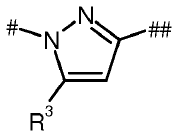 wherein
# designates the linkage point with the adjacent CH₂ group
and
## designates the linkage point with the ring 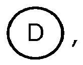
the ring 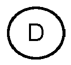 represents a heteroaryl ring of the formula 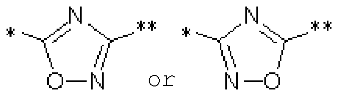 wherein
* designates the linkage point with the ring 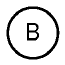
and
** designates the linkage point with the ring 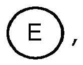
the ring 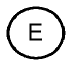 with the substituents R⁴ and R⁵ represents a phenyl ring of the formula 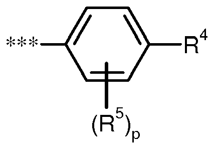 wherein
*** designates the linkage point with the ring 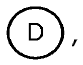
the ring 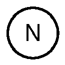 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -O-, -S-, - C(=O)-, -S(=O)₂- or ◆-N(R⁶)-C(=O)-◆◆, wherein
◆ designates the linkage point with the ring 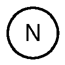
and
◆◆ designates the linkage point with the ring 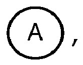
q denotes the number 0, 1 or 2
and
R⁶ denotes hydrogen, (C₁-C₄)-alkyl or (C₃-C₆)-cycloalkyl,
R¹ represents a substituent bonded to a carbon atom of the ring 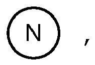 chosen from the series cyano, (C₁-C₄)-alkyl, oxo and (C₃-C₆)-cycloalkyl,
wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine
and
(C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
R² represents a substituent bonded to a nitrogen atom of the ring 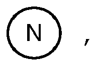 chosen from the series (C₁-C₄)-alkyl, (C₁-C₄)-alkylcarbonyl, (C₁-C₄)-alkoxycarbonyl, (C₁-C₄)-alkylsulfonyl and (C₃-C₆)-cycloalkyl,
wherein the alkyl group in (C₁-C₄)-alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino (C₃-C₆)-cycloalkyl and 4- to 6-membered heterocyclyl and up to three times by fluorine
and (C₃-C₆)-cycloalkyl in its turn can be substituted up to two times in an identical or different manner by a radical chosen from the series fluorine, (C₁-C₄)-alkyl, hydroxyl, (C₁-C₄)-alkoxy, amino, mono-(C₁-C₄)-alkylamino and di-(C₁-C₄)-alkylamino,
m represents the number 0, 1 or 2, wherein in the case where the substituent R¹ occurs twice, its meanings can be identical or different,
n represents the number 0 or 1,
wherein the sum of m and n equals the number 1, 2 or 3,
R³ represents methyl, ethyl or trifluoromethyl,
R⁴ represents a substituent chosen from the series fluorine, chlorine, cyano, pentafluorothio, (C₁-C₆)-alkyl, tri-(C₁-C₄)-alkylsilyl, -OR⁷, -NR⁷R⁸, -SR⁷, -S(=O)-R⁷, -S(=O)₂-R⁷, -S (=0) (=NH)-R⁷, - S (=0) (=NCH₃)-R⁷, (C₃-C₆)-cycloalkyl and 4- to 6-membered heterocyclyl,
wherein (C₁-C₆)-alkyl in its turn can be substituted up to three times by fluorine and up to two times in an identical or different manner by a radical chosen from the series -OR⁷, -NR⁷R⁸, -N(R⁷)-C(=O)-R⁸, -C(=O)-NR⁷R⁸, (C₃-C₆)-cycloalkyl, 4- to 6-membered heterocyclyl and 5- or 6-membered heteroaryl
and wherein
the cycloalkyl and heterocyclyl groups mentioned in their turn can be substituted up to two times in an identical or different manner by a radical chosen from the series fluorine, (C₁-C₄)-alkyl, hydroxyl, (C₁-C₄)-alkoxy, trifluoromethoxy, oxo and (C₁-C₄)-alkylcarbonyl and the heteroaryl group mentioned in its turn can be substituted up to two times in an identical or different manner by a radical chosen from the series fluorine, chlorine, cyano, (C₁-C₄)-alkyl, (C₁-C₄)-alkoxy and trifluoromethoxy wherein the (C₁-C₄)-alkyl substituents mentioned herein in their turn can be substituted by hydroxyl, methoxy, trifluoromethoxy, ethoxy, acetoxy, aminocarbonyl, methylaminocarbonyl or dimethylaminocarbonyl or up to three times by fluorine, and wherein
R⁷ and R⁸ independently of each other for each individual occurrence denote hydrogen, (C₁-C₄)-alkyl, (C₃-C₆)-cycloalkyl or 4- to 6-membered heterocyclyl,
wherein (C₁-C₄)-alkyl can be substituted up to three times by fluorine and up to two times in an identical or different manner by a radical chosen from the series hydroxyl, (C₁-C₄)-alkoxy, trifluoromethoxy, (C₃-C₆)-cycloalkyl and 4- to 6-membered heterocyclyl
and
the cycloalkyl and heterocyclyl groups mentioned can be substituted up to two times in an identical or different manner by a radical chosen from the series fluorine, (C₁-C₄)-alkyl, trifluoromethyl, hydroxyl, (C₁-C₄)-alkoxy, trifluoromethoxy, oxo and (C₁-C₄)-alkylcarbonyl
or
R⁷ and R⁸ in the case where both are bonded to a nitrogen atom form a 4- to 6-membered heterocycle together with this nitrogen atom, which can contain a further ring hetero atom from the series N, 0, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl,
R⁵ represents a substituent chosen from the series fluorine, chlorine and methyl and
p represents the number 0 or 1,
and their salts, solvates and solvates of the salts.

9. Compound of the formula (I) according to claim 1, 2, 3 or 6, in which
the ring 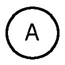 represents a pyridyl ring of the formula 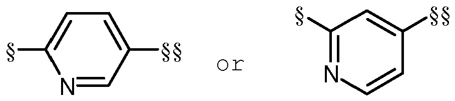 wherein
§ designates the linkage point with the adjacent group X
and
§§ designates the linkage point with the adjacent CH₂ group,
the ring 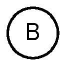 with the substituent R³ represents a heteroaryl ring of the formula 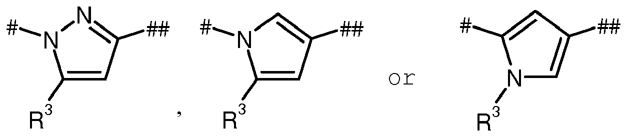 wherein
# designates the linkage point with the adjacent CH₂ group
and
## designates the linkage point with the ring 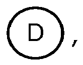
the ring 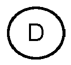 represents a heteroaryl ring of the formula 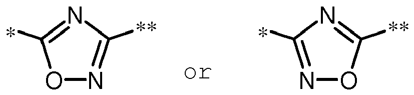 wherein
* designates the linkage point with the ring 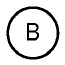
and
** designates the linkage point with the ring 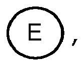
the ring 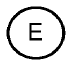 with the substituents R⁴ and R⁵ represents a phenyl ring of the formula 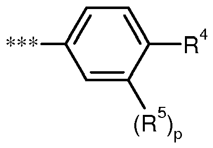 wherein
*** designates the linkage point with the ring 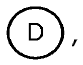
the ring 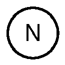 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -C(=O) - or ◆-N(R⁶)-C(=O)-◆◆, wherei
◆ designates the linkage point with the ring 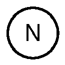
and
◆◆ designates the linkage point with the ring 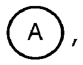
q denotes the number 0 or 1
and
R⁶ denotes hydrogen, methyl, ethyl, isopropyl, cyclopropyl or cyclobutyl,
R¹ represents a substituent bonded to a carbon atom of the ring 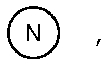 chosen from the series fluorine, cyano, (C₁-C₄)-alkyl, hydroxyl, (C₁-C₄)-alkoxy, oxo, amino, mono-(C₁-C₄)-alkylamino, di-(C₁-C₄)-alkylamino, cyclopropyl and cyclobutyl, wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine,
m represents the number 0 or 1,
R² represents a substituent bonded to a nitrogen atom of the ring 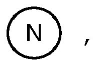 chosen from the series (C₁-C₄)-alkyl, (C₁-C₄)-alkylcarbonyl, (C₁-C₄)-alkoxycarbonyl, (C₁-C₄)-alkylsulfonyl, cyclopropyl and cyclobutyl, wherein the alkyl group in (C₁-C₄)-alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino, (C₃-C₅)-cycloalkyl and 4- or 5-membered heterocyclyl and up to three times by fluorine
n represents the number 0 or 1,
R³ represents methyl,
R⁴ represents a substituent chosen from the series chlorine, pentafluorothio, (C₁-C₆)-alkyl, trimethylsilyl, -OR⁷, -SR⁷, -S(=O)-R⁷, -S(=O)₂-R⁷, -S(=O) (=NCH₃)-CF₃, (C₃-C₆)-cycloalkyl and 4- to 6-membered heterocyclyl, wherein (C₁-C₆)-alkyl in its turn can be substituted by a radical chosen from the series - OR⁷, -NR⁷R⁸, -C(=O)-NR⁷R⁸, (C₃-C₆)-cycloalkyl and 4-to 6-membered heterocyclyl and up to three time by fluorine
and
the cycloalkyl and heterocyclyl groups mentioned in their turn can be substituted up to two times in an identical or different manner by a radical chosen from the series fluorine, (C₁-C₄)-alkyl, trifluoromethyl, (C₁-C₄)-alkoxy, trifluoromethoxy and oxo, wherein the (C₁-C₄)-alkyl substituent in its turn can be substituted by methoxy, trifluoromethoxy or ethoxy,
and wherein
R⁷ and R⁸ independently of each other for each individual occurrence denote hydrogen, (C₁-C₄)-alkyl or (C₃-C₆)-cycloalkyl, wherein (C₁-C₄)-alkyl can be substituted by a radical chosen from the series hydroxyl, (C₁-C₄)-alkoxy, trifluoromethoxy and (C₃-C₆)-cycloalkyl and up to three times by fluorine
and
the cycloalkyl groups mentioned can be substituted up to two times in an identical or different manner by a radical chosen from the series fluorine, (C₁-C₄)-alkyl, trifluoromethyl, hydroxyl, (C₁-C₄)-alkoxy and trifluoromethoxy,
or
R⁷ and R⁸ in the case where both are bonded to a nitrogen atom form a 4- to 6-membered heterocycle together with this nitrogen atom, which can contain a further ring hetero atom from the series N, 0, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl,
R⁵ represents fluorine, and
p represents the number 0 or 1, and their salts, solvates and solvates of the salts.

10. Compound of the formula (I) according to claim 1, 2, 4 or 7, in which
the ring 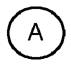 represents a phenyl ring and the adjacent groups X and CH₂ are bonded to this phenyl ring in 1,3 or 1,4 relation to one another,
the ring 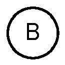 with the substituent R³ represents a heteroaryl ring of the formula 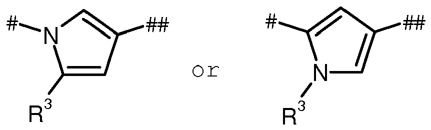 wherein
# designates the linkage point with the adjacent CH₂ group
and
## designates the linkage point with the ring 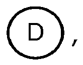
the ring 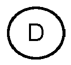 represents a heteroaryl ring of the formula 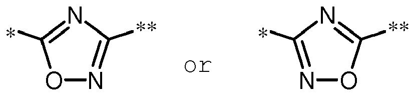 wherein
* designates the linkage point with the ring 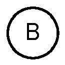
and
** designates the linkage point with the ring 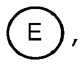
the ring 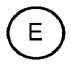 with the substituents R⁴ and R⁵ represents a phenyl ring of the formula 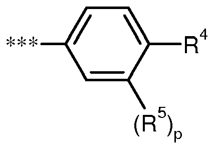 wherein
*** designates the linkage point with the ring 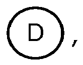
the ring 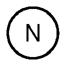 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -C(=O)- or ◆-N(R⁶)-C(=O)-◆◆, wherein
◆ designates the linkage point with the ring 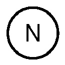
and
◆◆ designates the linkage point with the ring 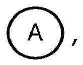
q denotes the number 0 or 1
and
R⁶ denotes hydrogen, methyl, ethyl, isopropyl, cyclopropyl or cyclobutyl,
R¹ represents a substituent bonded to a carbon atom of the ring 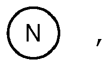 chosen from the series fluorine, cyano, (C₁-C₄)-alkyl, hydroxyl, (C₁-C₄)-alkoxy, oxo, amino, mono-(C₁-C₄)-alkylamino, di-(C₁-C₄)-alkylamino, cyclopropyl and cyclobutyl, wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine,
m represents the number 0 or 1,
R² represents a substituent bonded to a nitrogen atom of the ring 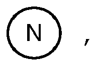 chosen from the series (C₁-C₄)-alkyl, (C₁-C₄)-alkylcarbonyl, (C₁-C₄)-alkoxycarbonyl, (C₁-C₄)-alkylsulfonyl, cyclopropyl and cyclobutyl,
wherein the alkyl group in (C₁-C₄)-alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino (C₃-C₅)-cycloalkyl and 4- or 5-membered heterocyclyl and up to three times by fluorine,
n represents the number 0 or 1,
R³ represents methyl,
R⁴ represents a substituent chosen from the series chlorine, pentafluorothio, (C₁-C₆)-alkyl, trimethylsilyl, -OR⁷, -SR⁷, -S(=O)-R⁷, -S(=O)₂-R⁷, -S(=O)(=NCH₃) -CF₃, (C₃-C₆)-cycloalkyl and 4- to 6-membered heterocyclyl,
wherein (C₁-C₆)-alkyl in its turn can be substituted by a radical chosen from the series - OR⁷, -NR⁷R⁸, -C(=O)-NR⁷R⁸, (C₃-C₆)-cycloalkyl and 4-to 6-membered heterocyclyl and up to three time by fluorine
and
the cycloalkyl and heterocyclyl groups mentioned in their turn can be substituted up to two times in an identical or different manner by a radical chosen from the series fluorine, (C₁-C₄)-alkyl, trifluoromethyl, (C₁-C₄)-alkoxy, trifluoromethoxy and oxo, wherein the (C₁-C₄)-alkyl substituent mentioned in its turn can be substituted by methoxy, trifluoromethoxy or ethoxy,
and wherein
R⁷ and R⁸ independently of each other for each individual occurrence denote hydrogen, (C₁-C₄)-alkyl or (C₃-C₆)-cycloalkyl, wherein (C₁-C₄)-alkyl can be substituted by a radical chosen from the series hydroxyl, (C₁-C₄)-alkoxy, trifluoromethoxy and (C₃-C₆)-cycloalkyl and up to three times by fluorine
and
the cycloalkyl groups mentioned can be substituted up to two times in an identical or different manner by a radical chosen from the series fluorine, (C₁-C₄)-alkyl, trifluoromethyl, hydroxyl, (C₁-C₄)-alkoxy and trifluoromethoxy, or
R⁷ and R⁸ in the case where both are bonded to a nitrogen atom form a 4- to 6-membered heterocycle together with this nitrogen atom, which can contain a further ring hetero atom from the series N, O, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl,
R⁵ represents fluorine, and
p represents the number 0 or 1,
and their salts, solvates and solvates of the salts.

11. Compound of the formula (I) according to claim 1, 2, 5 or 8, in which
the ring 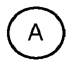 represents a phenyl ring and the adjacent groups X and CH₂ are bonded to this phenyl ring in 1,3 or 1,4 relation to one another,
the ring 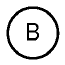 with the substituent R³ represents a heteroaryl ring of the formula 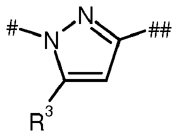 wherein
# designates the linkage point with the adjacent CH₂ group
and
## designates the linkage point with the ring 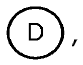
the ring 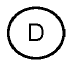 represents a heteroaryl ring of the formula 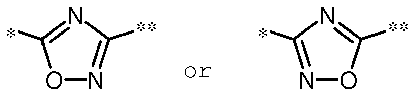 wherein
* designates the linkage point with the ring 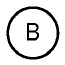
and
** designates the linkage point with the ring 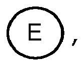
the ring 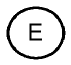 with the substituents R⁴ and R⁵ represents a phenyl ring of the formula 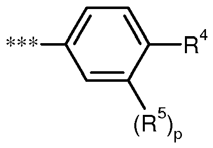 wherein
*** designates the linkage point with the ring 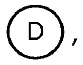
the ring 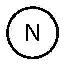 represents a saturated 4- to 10-membered aza-heterocycle, which contains at least one N atom as a ring member and in addition can contain a further hetero ring member from the series N, 0, S or S(O)₂,
X represents a bond or ◆-(CH₂)_{q}-N(R⁶)-◆◆, -C(=O)- or ◆-N(R⁶)-C(=O)-◆◆, wherein ◆ designates the linkage point with the ring 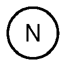
and
◆◆ designates the linkage point with the ring 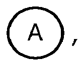
q denotes the number 0 or 1
and
R⁶ denotes hydrogen, methyl, ethyl, isopropyl, cyclopropyl or cyclobutyl,
R¹ represents a substituent bonded to a carbon atom of the ring 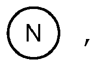 chosen from the series cyano, (C₁-C₄)-alkyl, oxo, cyclopropyl and cyclobutyl, wherein (C₁-C₄)-alkyl in its turn can be substituted by a radical chosen from the series hydroxyl, (C₁-C₄)-alkoxy, amino, mono-(C₁-C₄)-alkylamino and di-(C₁-C₄)-alkylamino and up to three times by fluorine,
R² represents a substituent bonded to a nitrogen atom of the ring 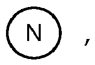 chosen from the series (C₁-C₄)-alkyl, (C₁-C₄)-alkylcarbonyl, (C₁-C₄)-alkoxycarbonyl, (C₁-C₄)-alkylsulfonyl, cyclopropyl and cyclobutyl, wherein the alkyl group in (C₁-C₄)-alkyl, (C₁-C₄)-alkylcarbonyl, (C₁-C₄)-alkoxycarbonyl and (C₁-C₄)-alkylsulfonyl in its turn can be substituted by a radical chosen from the series hydroxyl, (C₁-C₄)-alkoxy, amino, mono-(C₁-C₄)-alkylamino, di-(C₁-C₄)-alkylamino, (C₃-C₅)-cycloalkyl and 4- or 5-membered heterocyclyl and up to three times by fluorine,
m represents the number 0 or 1,
n represents the number 0 or 1,
wherein the sum of m and n equals the number 1 or 2,
R³ represents methyl,
R⁴ represents a substituent chosen from the series chlorine, pentafluorothio, (C₁-C₆)-alkyl, trimethylsilyl, -OR⁷, -SR⁷, -S(=O)-R⁷, -S(=O)₂-R⁷, -S(=O)(=NCH₃)-CF₃, (C₃-C₆)-cycloalkyl and 4- to 6-membered heterocyclyl, wherein (C₁-C₆)-alkyl in its turn can be substituted by a radical chosen from the series - OR⁷, -NR⁷R⁸, -C (=O)-NR⁷R⁸, (C₃-C₆)-cycloalkyl and 4-to 6-membered heterocyclyl and up to three time by fluorine
and
the cycloalkyl and heterocyclyl groups mentioned in their turn can be substituted up to two times in an identical or different manner by a radical chosen from the series fluorine, (C₁-C₄)-alkyl, trifluoromethyl, (C₁-C₄)-alkoxy, trifluoromethoxy and oxo, wherein the (C₁-C₄)-alkyl substituent mentioned in its turn can be substituted by methoxy, trifluoromethoxy or ethoxy, and wherein
R⁷ and R⁸ independently of each other for each individual occurrence denote hydrogen, (C₁-C₄)-alkyl or (C₃-C₆)-cycloalkyl,
wherein (C₁-C₄)-alkyl can be substituted by a radical chosen from the series hydroxyl, (C₁-C₄)-alkoxy, trifluoromethoxy and (C₃-C₆)-cycloalkyl and up to three times by fluorine
and
the cycloalkyl groups mentioned can be substituted up to two times in an identical or different manner by a radical chosen from the series fluorine, (C₁-C₄)-alkyl, trifluoromethyl, hydroxyl, (C₁-C₄)-alkoxy and trifluoromethoxy, or
R⁷ and R⁸ in the case where both are bonded to a nitrogen atom form a 4- to 6-membered heterocycle together with this nitrogen atom, which can contain a further ring hetero atom from the series N, 0, S or S(O)₂ and which can be substituted up to two times in an identical or different manner by a radical chosen from the series fluorine, (C₁-C₄)-alkyl, trifluoromethyl, hydroxyl, (C₁-C₄)-alkoxy, oxo and (C₁-C₄)-alkylcarbonyl,
R⁵ represents fluorine, and
p represents the number 0 or 1,
and their salts, solvates and solvates of the salts.

12. Compound as defined in one of claims 1 to 11, for treatment and/or prevention of diseases.

13. Compound as defined in one of claims 1 to 11, for use in a method for treatment and/or prevention of cancer diseases or tumour diseases.

14. Compound as defined in one of claims 1 to 11, for use in a method for treatment and/or prevention of ischaemic cardiovascular diseases, cardiac insufficiency, cardiac infarction, arrhythmia, stroke, pulmonary hypertension, fibrotic diseases of the kidney and lung, psoriasis, diabetic retinopathy, macular degeneration, rheumatic arthritis and Chugwash polycythaemia.

15. Use of a compound as defined in one of claims 1 to 11, for the preparation of a medicament for treatment and/or prevention of cancer diseases or tumour diseases.

16. Use of a compound as defined in one of claims 1 to 11, for the preparation of a medicament for treatment and/or prevention of ischaemic cardiovascular diseases, cardiac insufficiency, cardiac infarction, arrhythmia, stroke, pulmonary hypertension, fibrotic diseases of the kidney and lung, psoriasis, diabetic retinopathy, macular degeneration, rheumatic arthritis and Chugwash polycythaemia.

17. Medicament containing a compound as defined in one of claims 1 to 11 in combination with one or more inert, non-toxic, pharmaceutically suitable auxiliary substances.

18. Medicament containing a compound as defined in one of claims 1 to 11 in combination with one or more further active compounds.

19. Medicament according to claim 17 or 18, for treatment and/or prevention of cancer diseases or tumour diseases.

20. Medicament according to claim 17 or 18, for treatment and/or prevention of ischaemic cardiovascular diseases, cardiac insufficiency, cardiac infarction, arrhythmia, stroke, pulmonary hypertension, fibrotic diseases of the kidney and lung, psoriasis, diabetic retinopathy, macular degeneration, rheumatic arthritis and Chugwash polycythaemia.

21. Process for the preparation of compounds of the formula (I-D) 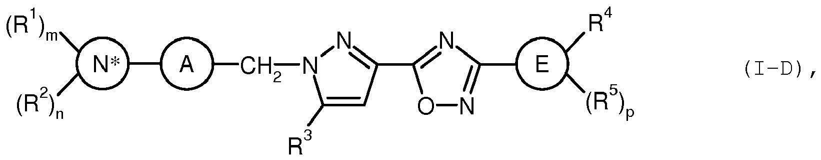 in which the rings A and E and R¹, R², R³, R⁴, R⁵, m, n and p each have the meanings given in claims 1 to 11,
and
the ring N* represents a ring N which is bonded to the ring A via a ring nitrogen atom and is as defined in claims 1 to 11,
**characterized in that** an *N'*-hydroxyamidine of the formula (IX) 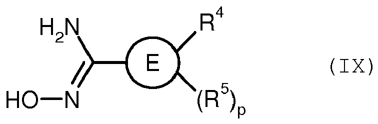 in which the ring E and R⁴, R⁵ and p have the meanings given above,
first can either be
[A] subjected to a condensation reaction with a pyrazolecarboxylic acid of the formula (XXVII) 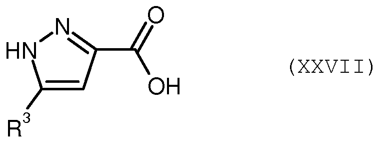 in which R³ has the meaning given above,
to give a 1,2,4-oxadiazole derivative of the formula (XXVIII) 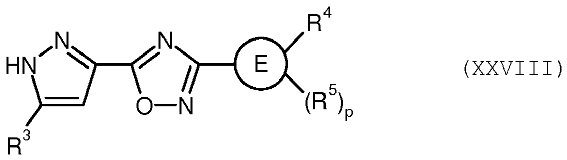 in which the ring E and R³, R⁴, R⁵ and p have the meanings given above,
and this is then alkylated in the presence of a base with a compound of the formula (III) 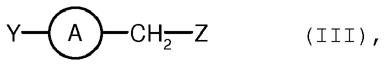 in which the ring A has the meaning given above, 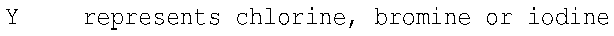 and 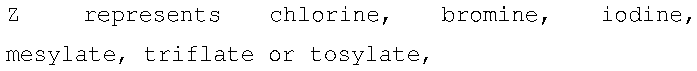 to give a compound of the formula (XXIX) 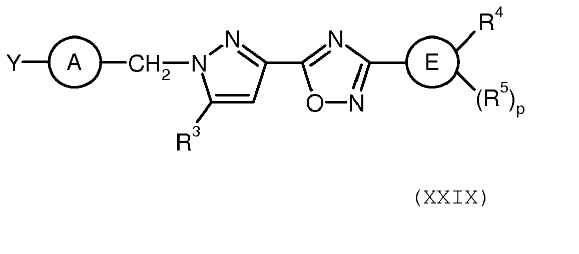 in which the rings A and E and R³, R⁴, R⁵, p and Y have the meanings given above,
or
[B] subjected to a condensation reaction with a
pyrazolecarboxylic acid of the formula (XXX) 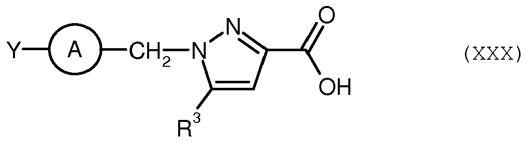 in which the ring A and R³ have the meanings given above,
and
Y represents chlorine, bromine or iodine,
to give the compound of the formula (XXIX) 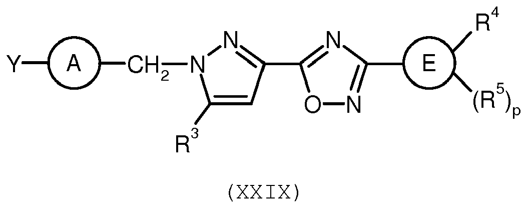 in which the rings A and E and R³, R⁴, R⁵, p and Y have the abovementioned meanings,
and the compound of the formula (XXIX) obtained in this way in then reacted, optionally in the presence of a palladium catalyst and/or a base, with a compound of the formula (XXXI) 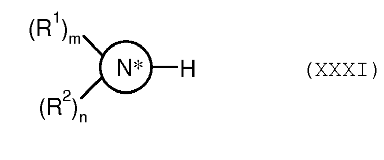 in which the ring N* and R¹, R², m and n have the meanings given above and the hydrogen atom shown is bonded to a nitrogen atom of the ring N*.

## Revendications

1. Composé de formule (I) 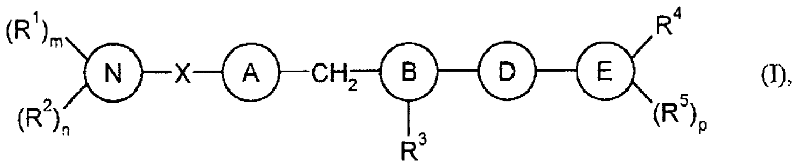 dans laquelle
le cycle 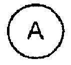 représente un cycle phényle ou pyridyle,
le cycle 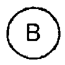 avec le substituant R³ représente un cycle
hétéroaryle de formule 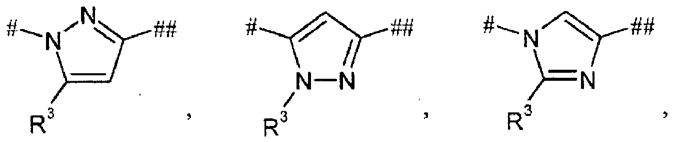 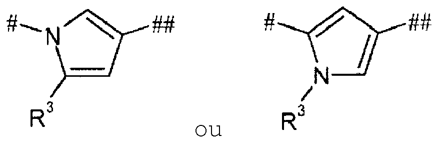 où
# désigne le point de liaison au groupe CH₂ contigu
et
# désigne le point de liaison au cycle 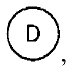 le cycle 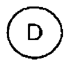 représente un cycle hétéroaryle de formule 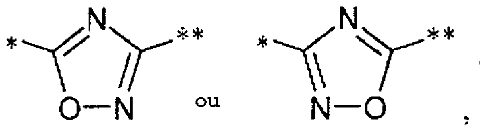 où
* désigne le point de liaison au cycle 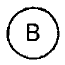 et
** désigne le point de liaison au cycle 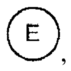
le cycle 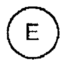 représente un cycle phényle ou pyridyle,
le cycle 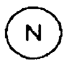 représente un aza-hétérocycle saturé à 4 à 10 chaînons, qui contient au moins un atome d'azote en tant que chaînon formant le cycle et peut en outre contenir un ou deux autres hétérochaînons dans le cycle, choisis dans la série N, 0, S et/ou S(O)₂,
X représente une liaison ou représente ◆-(CH₂)_{q}N(R⁶)-◆◆, ◆-N(R⁶)-(CH₂)_{q}-◆◆, -O-, -S-, -C(=O)-, -S(=O)₂-, ◆-C(=O)-N(R⁶)-◆◆ ou ◆-N(R⁶)-C(=O)-◆◆, où
◆ désigne le point de liaison au cycle 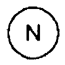
et
◆◆ désigne le point de liaison au cycle 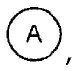
q représente le nombre 0, 1 ou 2 et
R⁶ représente un atome d'hydrogène, un groupe alkyle en C₁-C₆ ou cycloalkyle en C₃-C₆, les groupes alkyle en C₁-C₆ et cycloalkyle en C₃-C₆ pouvant être substitués chacun par hydroxy ou alcoxy en C₁-C₄,
R¹ représente un substituant lié à un atome de carbone du cycle 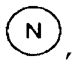 choisi dans la série fluoro, cyano, alkyle en C₁-C₆, hydroxy, alcoxy en C₁-C₆, oxo, amino, monoalkyl(C₁-C₆)amino, dialkyl(C₁-C₆)-amino et cycloalkyle en C₃-C₆,
le groupe alkyle en C₁-C₆ pouvant pour sa part être substitué jusqu'à trois fois par le fluor ainsi que jusqu'à deux fois par un radical, qui peut être le même ou différent, choisi dans la série hydroxy, alcoxy en C₁-C₄, amino, monoalkyl (C₁-C₄) amino et dialkyl (C₁-C₄) amino
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
m représente le nombre 0, 1, 2, 3 ou 4, dans le cas où le substituant R¹ apparaît plusieurs fois, ses significations pouvant être identiques ou différentes,
R² représente un substituant lié à un atome d'azote du cycle 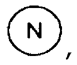 choisi dans la série alkyle en C₁-C₆, alkyl(C₁-C₆)carbonyle, alcoxy(C₁-C₆)-carbonyle, alkyl(C₁-C₆)sulfonyle et cycloalkyle en C₃-C₆,
le groupe alkyle dans les radicaux alkyle en C₁-C₆, alkyl(C₁-C₆)carbonyle, alcoxy(C₁-C₆)carbonyle et alkyl(C₁-C₆)sulfonyle pouvant pour sa part être substitué jusqu'à trois fois par le fluor ainsi que jusqu'à deux fois par un radical, qui peut être le même ou différent, choisi dans la série hydroxy, alcoxy en C₁-C₄, amino, monoalkyl(C₁-C₄)amino, dialkyl(C₁-C₄)amino, cycloalkyle en C₃-C₆ et hétérocyclyle à 4 à 6 chaînons
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
n représente le nombre 0 ou 1 ou bien, si l'azahétérocycle 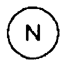 contient d'autres atomes d'azote en tant que chaînons formant le cycle, le nombre 2, dans le cas où le substituant R² apparaît deux fois, ses significations pouvant être identiques ou différentes,
R³ représente le groupe méthyle, éthyle ou trifluorométhyle,
R⁴ représente un atome d'hydrogène ou un substituant choisi dans la série halogéno, cyano, pentafluorothio, alkyle en C₁-C₆, trialkyl(C₁-C₄)-silyle, -OR⁷, -NR⁷R⁸, -N(R⁷)-C(=O)-R⁸, -N(R⁷)-C(=O)-OR⁸, -N(R⁷)-S(=O)₂-R⁸, -C(=O)-OR⁷, -C(=O)-NR⁷R⁸, -SR⁷, -S(=O)-R⁷, -S(=O)₂-R⁷, S(=O)₂-NR⁷R⁸, -S(=O)(=NH)-R⁷, -S(=O)(=NCH₃)-R⁷, cycloalkyle en C₃-C₆, hétérocyclyle à 4 à 6 chaînons et hétéroaryle à 5 ou 6 chaînons,
le groupe alkyle en C₁-C₆ pouvant pour sa part être substitué jusqu'à trois fois par le fluor ainsi que jusqu'à deux fois par un radical, qui peut être le même ou différent, choisi dans la série -OR⁷, -NR⁷R⁸, -N(R⁷)-C(=O)-R⁸, -N(R⁷)-C(=O)-OR⁸, -C(=O)-OR⁷, -C(=O)-NR⁷R⁸, cycloalkyle en C₃-C₆, hétérocyclyle à 4 à 6 chaînons et hétéroaryle à 5 ou 6 chaînons
et
lesdits groupes cycloalkyle et hétérocyclyle pouvant pour leur part être substitués jusqu'à trois fois par des radicaux, identiques ou différents, choisis dans la série fluoro, alkyle en C₁-C₄, hydroxy, alcoxy en C₁-C₄, trifluorométhoxy, oxo, amino, monoalkyl(C₁-C₄)-amino, dialkyl(C₁-C₄)amino, alkyl(C₁-C₄)carbonyl-amino, alcoxy(C₁-C₄)carbonylamino, alkyl(C₁-C₄)-carbonyle et alcoxy(C₁-C₄)carbonyle
ainsi que
lesdits groupes hétéroaryle pouvant pour leur part être substitués jusqu'à deux fois par un radical, qui peut être le même ou différent, choisi dans la série fluoro, chloro, cyano, alkyle en C₁-C₄, alcoxy en C₁-C₄ et trifluorométhoxy, les substituants alkyle en C₁-C₄ mentionnés ici pouvant pour leur part être substitués par hydroxy, alcoxy en C₁-C₄, trifluorométhoxy, alkyl(C₁-C₄)carbonyloxy, aminocarbonyle, monoalkyl(C₁-C₄)aminocarbonyle ou dialkyl(C₁-C₄) aminocarbonyle ou jusqu'à trois fois par le fluor,
et où
R⁷ et R⁸ représentent indépendamment l'un de l'autre dans chaque occurrence individuelle un atome d'hydrogène, un groupe alkyle en C₁-C₆, cycloalkyle en C₃-C₆ ou hétérocyclyle à 4 à 6 chaînons,
le groupe alkyle en C₁-C₆ pouvant être substitué jusqu'à trois fois par le fluor ainsi que jusqu'à deux fois par un radical, qui peut être le même ou différent, choisi dans la série hydroxy, alcoxy en C₁-C₄, trifluorométhoxy, amino, monoalkyl (C₁-C₄)-amino, dialkyl(C₁-C₄)amino, alcoxy(C₁-C₄)-carbonyle, cycloalkyle en C₃-C₆ et hétérocyclyle à 4 à 6 chaînons
et
lesdits groupes cycloalkyle et hétérocyclyle pouvant être substitués jusqu'à deux fois par des radicaux, identiques ou différents, choisis dans la série fluoro, alkyle en C₁-C₄, trifluorométhyle, hydroxy, alcoxy en C₁-C₄, trifluorométhoxy, oxo, amino, monoalkyl(C₁-C₄) amino, dialkyl(C₁-C₄)amino, alkyl(C₁-C₄)carbonyle et alcoxy(C₁-C₄)-carbonyle, ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou Su, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo, amino, monoalkyl(C₁-C₄)amino, dialkyl(C₁-C₄)amino, alkyl(C₁-C₄)carbonyle et alcoxy(C₁-C₄)carbonyle, R⁵ représente un substituant choisi dans la série fluoro, chloro, cyano, méthyle, trifluorométhyle et hydroxy et
p représente le nombre 0, 1 ou 2, dans le cas ou le substituant R⁵ apparaît deux fois, ses significations peuvent être identiques ou différentes,
ainsi que ses sels, produits de solvatation et produits de solvatation des sels.

2. Composé de formule (I) selon la revendication 1, dans lequel
le cycle 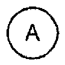 représente un cycle phényle ou pyridyle et les groupes adjacents X et CH₂ sont liés aux atomes
de carbone formant le cycle de 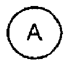 en relation 1,3 ou 1,4 l'un par rapport à l'autre et
le cycle 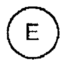 avec les substituants R⁴ et R⁵ représente un cycle phényle de formule 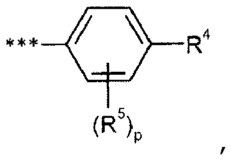 dans laquelle
*** désigne le point de liaison au cycle 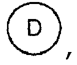
ainsi que ses sels, produits de solvatation et produits de solvatation des sels.

3. Composé de formule (I) selon la revendication 1 ou 2, dans lequel
le cycle 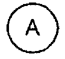 représente un cycle pyridyle et les groupes adjacents X et CH₂ sont liés aux atomes de carbone formant le cycle de ce cycle pyridyle en relation 1,3 ou 1,4 l'un par rapport à l'autre
et
le cycle 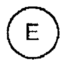 avec les substituants R⁴ et R⁵ représente un cycle phényle de formule 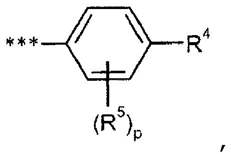 dans laquelle
*** désigne le point de liaison au cycle 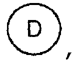
ainsi que ses sels, produits de solvatation et produits de solvatation des sels.

4. Composé de formule (I) selon la revendication 1 ou 2, dans lequel
le cycle 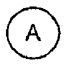 représente un cycle phényle et les groupes adjacents X et CH₂ sont liés à ce cycle phényle en relation 1,3 ou 1,4 l'un par rapport à l'autre,
le cycle 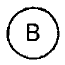 avec le substituant R³ représente un cycle
hétéroaryle de formule 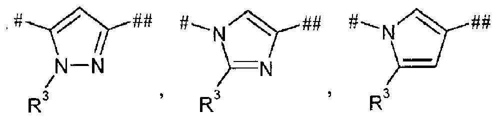 ou 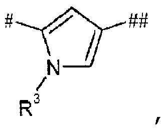 où
# désigne le point de liaison au groupe CH₂ contigu
et
## désigne le point de liaison au cycle 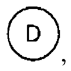
et
le cycle 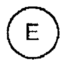 avec les substituants R⁴ et R⁵ représente un cycle phényle de formule 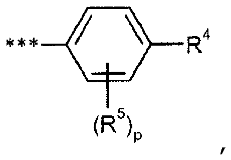 dans laquelle
*** désigne le point de liaison au cycle 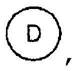 ainsi que ses sels, produits de solvatation et produits de solvatation des sels.

5. Composé de formule (I) selon la revendication 1 ou 2, dans lequel
le cycle 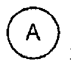 représente un cycle phényle et les groupes adjacents X et CH₂ sont liés à ce cycle phényle en relation 1,3 ou 1,4 l'un par rapport à l'autre,
le cycle 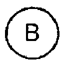 avec le substituant R³ représente un cycle
hétéroaryle de formule 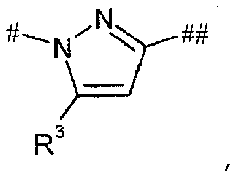 où
# désigne le point de liaison au groupe CH₂ contigu
et
## désigne le point de liaison au cycle 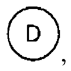
le cycle 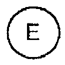 avec les substituants R⁴ et R⁵ représente un cycle phényle de formule 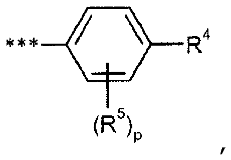 dans laquelle
*** désigne le point de liaison au cycle 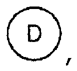
R¹ représente un substituant lié à un atome de carbone du cycle 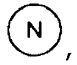 choisi dans la série cyano, alkyle en C₁-C₆, oxo et cycloalkyle en C₃-C₆, le groupe alkyle en C₁-C₆ pouvant pour sa part être substitué jusqu'à trois fois par le fluor ainsi que jusqu'à deux fois par un radical, qui peut être le même ou différent, choisi dans la série hydroxy, alcoxy en C₁-C₄, amino, monoalkyl(C₁-C₄)amino et dialkyl(C₁-C₄)amino
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
R² représente un substituant lié à un atome d'azotedu cycle 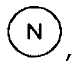 choisi dans la série alkyle en C₁-C₆, alkyl(C₁-C₆)carbonyle, alcoxy(C₁-C₆)-carbonyle, alkyl(C₁-C₆)sulfonyle et cycloalkyle en C₃-C₆, le groupe alkyle dans les radicaux alkyle en C₁-C₆, alkyl(C₁-C₆)carbonyle, alcoxy(C₁-C₆)carbonyle
et
alkyl(C₁-C₆)sulfonyle pouvant pour sa part être substitué jusqu'à trois fois par le fluor ainsi que jusqu'à deux fois par un radical, qui peut être le même ou différent, choisi dans la série hydroxy, alcoxy en C₁-C₄, amino,
monoalkyl(C₁-C₄)amino, dialky(C₁-C₄)amino, cycloalkyle en C₃-C₆ et hétérocyclyle à 4 à 6 chaînons
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
m représente le nombre 0, 1, 2, 3 ou 4, dans le cas où le substituant R¹ apparaît plusieurs fois, ses significations pouvant être identiques ou différentes, et
n représente le nombre 0 ou 1 ou bien, si l'azahétérocycle 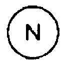 contient d'autres atomes d'azote
en tant que chaînons formant le cycle, le nombre 2,
dans le cas où le substituant R² apparaît deux fois, ses significations pouvant être identiques ou différentes,
la somme de m et n étant différente du nombre 0,
ainsi que ses sels, produits de solvatation et produits de solvatation des sels.

6. Composé de formule (I) selon la revendication 1, 2 ou 3, dans lequel
le cycle 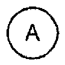 représente un cycle pyridyle et les groupes adjacents X et CH₂ sont liés à des atomes de carbone formant le cycle de ce cycle pyridyle en relation 1,3 ou 1,4 l'un par rapport à l'autre,
le cycle 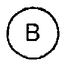 avec le substituant R³ représente un cycle
hétéroaryle de formule 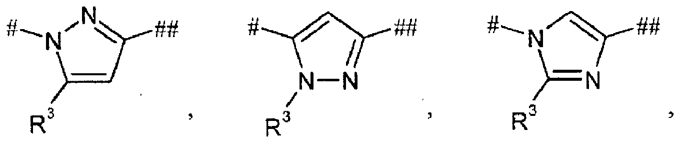 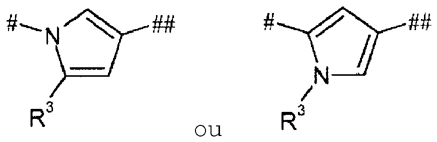 où
# désigne le point de liaison au groupe CH₂ contigu
et
## désigne le point de liaison au cycle 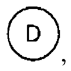
le cycle 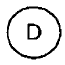 représente un cycle hétéroaryle de formule 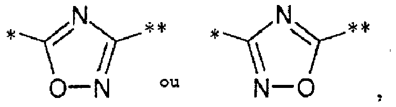 où
* désigne le point de liaison au cycle 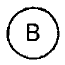
et
** désigne le point de liaison au cycle 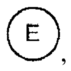
le cycle 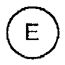 avec les substituants R⁴ et R⁵ représente un cycle phényle de formule 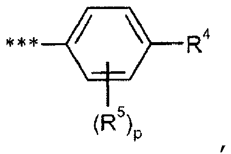 dans laquelle
*** désigne le point de liaison au cycle 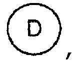
le cycle 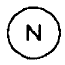 représente un aza-hétérocycle saturé à 4 à 10 chaînons, qui contient au moins un atome d'azote en tant que chaînon formant le cycle et peut en outre contenir un autre hétérochaînon dans le cycle, choisi dans la série N, O, S ou S(O)₂,
X représente une liaison ou représente ◆-(CH₂)_{q}N(R⁶)-◆◆, -O-, -S-, -C(=O)-, -S(=O)₂- ou ◆-N(R⁶)-C(=O)-◆◆, où
◆ désigne le point de liaison au cycle 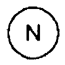
et
◆◆ désigne le point de liaison au cycle 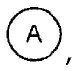
q représente le nombre 0, 1 ou 2 et
R⁶ représente un atome d'hydrogène, un groupe alkyle en C₁-C₄ ou cycloalkyle en C₃-C₆,
R¹ représente un substituant lié à un atome de carbone du cycle 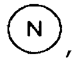 choisi dans la série fluoro, cyano, alkyle en C₁-C₄, hydroxy, alcoxy en C₁-C₄, oxo, amino, monoalkyl(C₁-C₄)amino, dialkyl(C₁-C₄)-amino et cycloalkyle en C₃-C₆, le groupe alkyle en C₁-C₄ pouvant pour sa part être substitué par un radical choisi dans la série hydroxy, alcoxy en C₁-C₄, amino, monoalkyl(C₁-C₄)amino et dialkyl(C₁-C₄)amino ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
m représente le nombre 0, 1 ou 2, dans le cas où le substituant R¹ apparaît deux fois, ses significations pouvant être identiques ou différentes,
R² représente un substituant lié à un atome d'azote du cycle 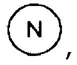 choisi dans la série alkyle en C₁-C₄, alkyl(C₁-C₄)carbonyle, alcoxy(C₁-C₄)-carbonyle, alkyl(C₁-C₄)sulfonyle et cycloalkyle en C₃-C₆,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl (C₁-C₄) amino, dialkyl (C₁-C₄) amino, cycloalkyle en C₃-C₆ et hétérocyclyle à 4 à 6 chaînons ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl(C₁-C₄)amino,
n représente le nombre 0 ou 1
R³ représente le groupe méthyle, éthyle ou trifluorométhyle,
R⁴ représente un substituant choisi dans la série fluoro, chloro, cyano, pentafluorothio, alkyle en C₁-C₆, trialkyl (C₁-C₄) silyle, -OR⁷, -NR⁷R⁸, -SR⁷, -S(=O)-R⁷, -S(=O)₂-R⁷, -S(=O) (=NH)-R⁷, -S(=O)(=NCH₃)-R⁷, cycloalkyle en C₃-C₆ et hétérocyclyle à 4 à 6 chaînons
, le groupe alkyle en C₁-C₆ pouvant pour sa part être substitué jusqu'à trois fois par le fluor ainsi que jusqu'à deux fois par un radical, qui peut être le même ou différent, choisi dans la série -OR⁷, -NR⁷R⁸, -N(R⁷)-C(=O)-R⁸, -C(=O)-NR⁷R⁸, cycloalkyle en C₃-C₆, hétérocyclyle à 4 à 6 chaînons et hétéroaryle à 5 ou 6 chaînons
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, hydroxy, alcoxy en C₁-C₄, trifluorométhoxy, oxo et alkyl(C₁-C₄)carbonyle
ainsi que
ledit groupe hétéroaryle pouvant pour sa part être substitué jusqu'à deux fois par un radical, qui peut être le même ou différent, choisi dans la série fluoro, chloro, cyano, alkyle en C₁-C₄, alcoxy en C₁-C₄ et trifluorométhoxy, les substituants alkyle en C₁-C₄ mentionnés ici pouvant pour leur part être substitués par hydroxy, méthoxy, trifluorométhoxy, éthoxy, acétoxy, aminocarbonyle, méthylamino-carbonyle ou diméthylaminocarbonyle ou jusqu'à trois fois par le fluor,
et où
R⁷ et R⁸ représentent indépendamment l'un de l'autre dans chaque occurrence individuelle un atome d'hydrogène, un groupe alkyle en C₁-C₄, cycloalkyle en C₃-C₆ ou hétérocyclyle à 4 à 6 chaînons,
le groupe alkyle en Cₗ-C₄ pouvant être substitué jusqu'à trois fois par le fluor ainsi que jusqu'à deux fois par un radical, qui peut être le même ou différent, choisi dans la série hydroxy, alcoxy en C₁-C₄, trifluorométhoxy, cycloalkyle en C₃-C₆ et hétérocyclyle à 4 à 6 chaînons
et
lesdits groupes cycloalkyle et hétérocyclyle pouvant être substitués jusqu'à deux fois par des radicaux, identiques ou différents, choisis dans la série fluoro, alkyle en C₁-C₄, trifluorométhyle, hydroxy, alcoxy en C₁-C₄, trifluorométhoxy, oxo et alkyl(C₁-C₄)-carbonyle, ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un substituant choisi dans la série fluoro, chloro et méthyle et
p représente le nombre 0 ou 1,
ainsi que ses sels, produits de solvatation et produits de solvatation des sels.

7. Composé de formule (I) selon la revendication 1, 2 ou 4, dans lequel
le cycle 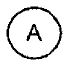 représente un cycle phényle et les groupes adjacents X et CH₂ sont liés à ce cycle phényle en relation 1,3 ou 1,4 l'un par rapport à l'autre,
le cycle 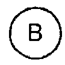 avec le substituant R³ représente un cycle hétéroaryle de formule 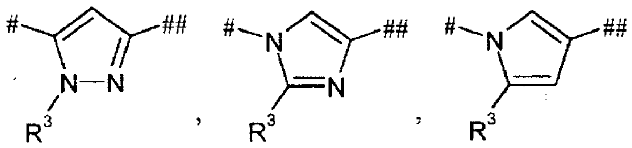 ou 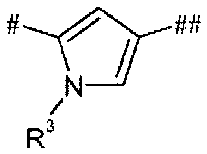 où
# désigne le point de liaison au groupe CH₂ contigu et
## désigne le point de liaison au cycle 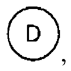
le cycle 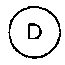 représente un cycle hétéroaryle de formule 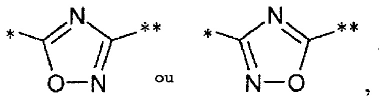 où
* désigne le point de liaison au cycle 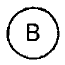
et
** désigne le point de liaison au cycle 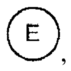
le cycle 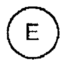 avec les substituants R⁴ et R⁵ représente un cycle phényle 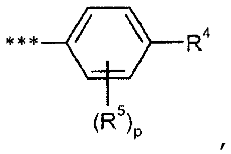 de formule dans laquelle
*** désigne le point de liaison au cycle 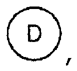
le cycle 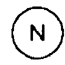 représente un aza-hétérocycle saturé à 4 à 10 chaînons, qui contient au moins un atome d'azote en tant que chaînon formant le cycle et peut en outre contenir un autre hétérochaînon dans le cycle, choisi dans la série N, 0, S ou Su,
X représente une liaison ou représente ◆-(CH₂)_{q}N(R⁶)-◆◆, -O-, -S-, -C(=O)-, -S(=O)₂- ou ◆-N(R⁶)-C(=O)-◆◆, où
◆ désigne le point de liaison au cycle 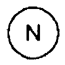 et
◆◆ désigne le point de liaison au cycle 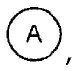
q représente le nombre 0, 1 ou 2
et
R⁶ représente un atome d'hydrogène, un groupe alkyle en C₁-C₄ ou cycloalkyle en C₃-C₆,
R¹ représente un substituant lié à un atome de carbone du cycle 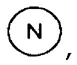 choisi dans la série fluoro, cyano, alkyle en C₁-C₄, hydroxy, alcoxy en C₁-C₄, oxo, amino, monoalkyl(C₁-C₄)amino, dialkyl(C₁-C₄)-amino et cycloalkyle en C₃-C₆, le groupe alkyle en C₁-C₄ pouvant pour sa part être substitué par un radical choisi dans la série hydroxy, alcoxy en C₁-C₄, amino, monoalkyl(C₁-C₄)-amino et dialkyl(C₁-C₄)amino ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl (C₁-C₄) amino,
m représente le nombre 0, 1 ou 2, dans le cas où le substituant R¹ apparaît deux fois, ses significations pouvant être identiques ou différentes,
R² représente un substituant lié à un atome d'azote du cycle 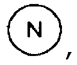 choisi dans la série alkyle en C₁-C₄, alkyl (C₁-C₄) carbonyle, alcoxy (C₁-C₄)-carbonyle, alkyl(C₁-C₄)sulfonyle et cycloalkyle en C₃-C₆,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl (C₁-C₄) amino, dialkyl (C₁-C₄) amino, cycloalkyle en C₃-C₆ et hétérocyclyle à 4 à 6 chaînons ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl (C₁-C₄) amino,
n représente le nombre 0 ou 1
R³ représente le groupe méthyle, éthyle ou trifluorométhyle,
R⁴ représente un substituant choisi dans la série fluoro, chloro, cyano, pentafluorothio, alkyle en C₁-C₆, trialkyl (C₁-C₄) silyle, -OR⁷, -NR⁷R⁸, -SR⁷, -S(=O)-R⁷, -S(=O)₂-R⁷, -S(=O)(=NH)-R⁷, -S (=0) (=NCH₃)-R⁷, cycloalkyle en C₃-C₆ et hétérocyclyle à 4 à 6 chaînons,
le groupe alkyle en C₁-C₆ pouvant pour sa part être substitué jusqu'à trois fois par le fluor ainsi que jusqu'à deux fois par un radical, qui peut être le même ou différent, choisi dans la série -OR⁷, -NR⁷R⁸, -N(R⁷)-C(=O)-R⁸, -C(=O)-NR⁷R⁸, cycloalkyle en C₃-C₆, hétérocyclyle à 4 à 6 chaînons et hétéroaryle à 5 ou 6 chaînons
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, hydroxy, alcoxy en C₁-C₄, trifluorométhoxy, oxo et alkyl(C₁-C₄)carbonyle
ainsi que
ledit groupe hétéroaryle pouvant pour sa part être substitué jusqu'à deux fois par un radical, qui peut être le même ou différent, choisi dans la série fluoro, chloro, cyano, alkyle en C₁-C₄, alcoxy en C₁-C₄ et trifluorométhoxy, les substituants alkyle en C₁-C₄ mentionnés ici pouvant pour leur part être substitués par hydroxy, méthoxy, trifluorométhoxy, éthoxy, acétoxy, aminocarbonyle, méthylamino-carbonyle ou diméthylaminocarbonyle ou jusqu'à trois fois par le fluor,
et où
R⁷ et R⁸ représentent indépendamment l'un de l'autre dans chaque occurrence individuelle un atome d'hydrogène, un groupe alkyle en C₁-C₄, cycloalkyle en C₃-C₆ ou hétérocyclyle à 4 à 6 chaînons,
le groupe alkyle en C₁-C₄ pouvant être substitué jusqu'à trois fois par le fluor ainsi que jusqu'à deux fois par un radical, qui peut être le même ou différent, choisi dans la série hydroxy, alcoxy en C₁-C₄, trifluorométhoxy, cycloalkyle en C₃-C₆ et hétérocyclyle à 4 à 6 chaînons
et
lesdits groupes cycloalkyle et hétérocyclyle pouvant être substitués jusqu'à deux fois par des radicaux, identiques ou différents, choisis dans la série fluoro, alkyle en C₁-C₄, trifluorométhyle, hydroxy, alcoxy en C₁-C₄, trifluorométhoxy, oxo et alkyl (C₁-C₄)-carbonyle,
ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un substituant choisi dans la série fluoro, chloro et méthyle et
p représente le nombre 0 ou 1,
ainsi que ses sels, produits de solvatation et produits de solvatation des sels.

8. Composé de formule (I) selon la revendication 1, 2 ou 5, dans lequel
le cycle 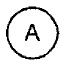 représente un cycle phényle et les groupes adjacents X et CH₂ sont liés à ce cycle phényle en relation 1,3 ou 1,4 l'un par rapport à l'autre,
le cycle 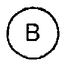 avec le substituant R³ représente un cycle hétéroaryle de formule 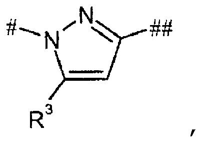 dans laquelle
# désigne le point de liaison au groupe CH₂ contigu
et
## désigne le point de liaison au cycle 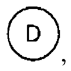
le cycle 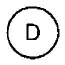 représente un cycle hétéroaryle de formule 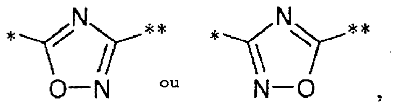 où
* désigne le point de liaison au cycle 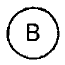
et
** désigne le point de liaison au cycle 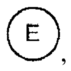
le cycle 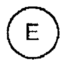 avec les substituants R⁴ et R⁵ représente un cycle phényle de formule 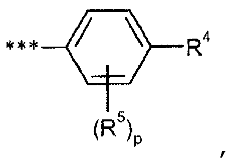 dans laquelle
*** désigne le point de liaison au cycle 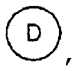
le cycle 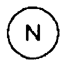 représente un aza-hétérocycle saturé à 4 à 10 chaînons, qui contient au moins un atome d'azote en tant que chaînon formant le cycle et peut en outre contenir un autre hétérochaînon dans
le cycle, choisi dans la série N, O, S ou S(O)₂,
X représente une liaison ou représente ◆-(CH₂)_{q}N(R⁶)-◆◆, -O-, -S-, -C(=O)-, -S(=O)₂- ou ◆-N(R⁶)-C(=O) -◆◆, où
◆ désigne le point de liaison au cycle 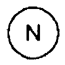
et
◆◆ désigne le point de liaison au cycle 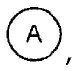
q représente le nombre 0, 1 ou 2
et
R⁶ représente un atome d'hydrogène, un groupe alkyle en C₁-C₄ ou cycloalkyle en C₃-C₆,
R¹ représente un substituant lié à un atome de carbone du cycle 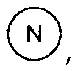 choisi dans la série cyano, alkyle en C₁-C₄, oxo, et cycloalkyle en C₃-C₆, le groupe alkyle en C₁-C₄ pouvant pour sa part être substitué par un radical choisi dans la série hydroxy, alcoxy en C₁-C₄, amino, monoalkyl(C₁-C₄)-amino et dialkyl(C₁-C₄)amino ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl (C₁-C₄) amino,
R² représente un substituant lié à un atome d'azote du cycle 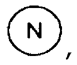 choisi dans la série alkyle en C₁-C₄, alkyl (C₁-C₄) carbonyle, alcoxy (C₁-C₄)-carbonyle, alkyl(C₁-C₄)sulfonyle et cycloalkyle en C₃-C₆,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl (C₁-C₄) amino, dialkyl (C₁-C₄) amino, cycloalkyle en C₃-C₆ et hétérocyclyle à 4 à 6 chaînons ainsi que jusqu'à trois fois par le fluor
et
le groupe cycloalkyle en C₃-C₆ pouvant pour sa part être substitué jusqu'à deux fois par un radical, qui peut être le même ou différent, choisi dans la série fluoro, alkyle en C₁-C₄, hydroxy, alcoxy en C₁-C₄, amino, monoalkyl(C₁-C₄)amino et dialkyl (C₁-C₄) amino,
m représente le nombre 0, 1 ou 2, dans le cas où le substituant R¹ apparaît deux fois, ses significations pouvant être identiques ou différentes,
n représente le nombre 0 ou 1
la somme de m et n étant égale au nombre 1, 2 ou 3,
R³ représente le groupe méthyle, éthyle ou trifluorométhyle,
R⁴ représente un substituant choisi dans la série fluoro, chloro, cyano, pentafluorothio, alkyle en C₁-C₆, trialkyl (C₁-C₄) silyle, -OR⁷, -NR⁷R⁸, -SR⁷, -S(=O)-R⁷, -S(=O)₂-R⁷, -S(=O) (=NH)-R⁷, -S(=O) (=NCH₃)-R⁷, cycloalkyle en C₃-C₆ et hétérocyclyle à 4 à 6 chaînons, le groupe alkyle en C₁-C₆ pouvant pour sa part être substitué jusqu'à trois fois par le fluor ainsi que jusqu'à deux fois par un radical, qui peut être le même ou différent, choisi dans la série -OR⁷, -NR⁷R⁸, -N(R⁷)-C(=O)-R⁸, -C(=O)-NR⁷R⁸, cycloalkyle en C₃-C₆, hétérocyclyle à 4 à 6 chaînons et hétéroaryle à 5 ou 6 chaînons
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, hydroxy, alcoxy en C₁-C₄, trifluorométhoxy, oxo et alkyl(C₁-C₄)carbonyle
ainsi que
ledit groupe hétéroaryle pouvant pour sa part être substitué jusqu'à deux fois par un radical, qui peut être le même ou différent, choisi dans la série fluoro, chloro, cyano, alkyle en C₁-C₄, alcoxy en C₁-C₄ et trifluorométhoxy, les substituants alkyle en C₁-C₄ mentionnés ici pouvant pour leur part être substitués par hydroxy, méthoxy, trifluorométhoxy, éthoxy, acétoxy, aminocarbonyle, méthylamino-carbonyle ou diméthylaminocarbonyle ou jusqu'à trois fois par le fluor, et où
R⁷ et R⁸ représentent indépendamment l'un de l'autre dans chaque occurrence individuelle un atome d'hydrogène, un groupe alkyle en C₁-C₄, cycloalkyle en C₃-C₆ ou hétérocyclyle à 4 à 6 chaînons, le groupe alkyle en C₁-C₄ pouvant être substitué jusqu'à trois fois par le fluor ainsi que jusqu'à deux fois par un radical, qui peut être le même ou différent, choisi dans la série hydroxy, alcoxy en C₁-C₄, trifluorométhoxy, cycloalkyle en C₃-C₆ et hétérocyclyle à 4 à 6 chaînons
et
lesdits groupes cycloalkyle et hétérocyclyle pouvant être substitués jusqu'à deux fois par des radicaux, identiques ou différents, choisis dans la série fluoro, alkyle en C₁-C₄, trifluorométhyle, hydroxy, alcoxy en C₁-C₄, trifluorométhoxy, oxo et alkyl (C₁-C₄) - carbonyle,
ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un substituant choisi dans la série fluoro, chloro et méthyle et
p représente le nombre 0 ou 1,
ainsi que ses sels, produits de solvatation et produits de solvatation des sels.

9. Composé de formule (I) selon la revendication 1, 2, 3 ou 6, dans lequel
le cycle 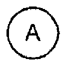 représente un cycle pyridyle de formule 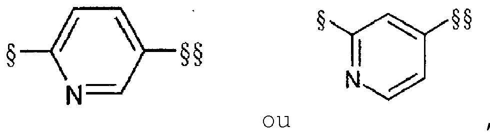 , formules dans lesquelles
§ désigne le point de liaison au groupe X contigu
et
§§ désigne le point de liaison au groupe CH₂ contigu,
le cycle 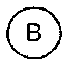 avec le substituant R³ représente un cycle hétéroaryle de formule 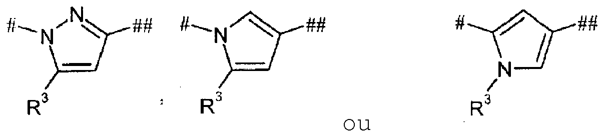 formules dans lesquelles
# désigne le point de liaison au groupe CH₂ contigu
et
## désigne le point de liaison au cycle 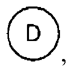
le cycle 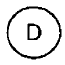 représente un cycle hétéroaryle de formule 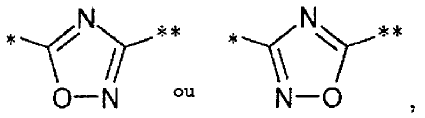 où
* désigne le point de liaison au cycle 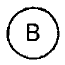
et
** désigne le point de liaison au cycle 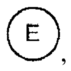
le cycle 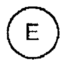 avec les substituants R⁴ et R⁵ représente un cycle phényle de formule 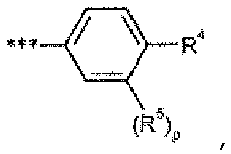 dans laquelle
*** désigne le point de liaison au cycle 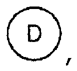
le cycle 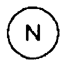 représente un aza-hétérocycle saturé à 4 à 10 chaînons, qui contient au moins un atome d'azote en tant que chaînon formant le cycle et peut en outre contenir un autre hétérochaînon dans
le cycle, choisi dans la série N, O, S ou S(O)₂,
X représente une liaison ou représente ◆-(CH₂)_{q}N(R⁶)-◆◆, -C(=O) - ou ◆-N(R⁶)-C(=O)-◆◆, où
◆ désigne le point de liaison au cycle 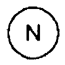
et
◆◆ désigne le point de liaison au cycle 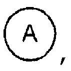
q représente le nombre 0 ou 1 et
R⁶ représente un atome d'hydrogène, le groupe méthyle, éthyle, isopropyle, cyclopropyle ou cyclobutyle,
R¹ représente un substituant lié à un atome de carbone du cycle 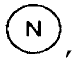 choisi dans la série fluoro, cyano, alkyle en C₁-C₄, hydroxy, alcoxy en C₁-C₄, oxo, amino, monoalkyl(C₁-C₄)amino, dialkyl(C₁-C₄)-amino, cyclopropyle et cyclobutyle, le groupe alkyle en C₁-C₄ pouvant pour sa part être substitué par un radical choisi dans la série hydroxy, alcoxy en C₁-C₄, amino, monoalkyl(C₁-C₄)-amino et dialkyl(C₁-C₄)amino ainsi que jusqu'à trois fois par le fluor
m représente le nombre 0 ou 1,
R² représente un substituant lié à un atome d'azote du cycle 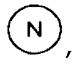 choisi dans la série alkyle en C₁-C₄, alkyl (C₁-C₄) carbonyle, alcoxy (C₁-C₄)-carbonyle, alkyl(C₁-C₄)sulfonyle, cyclopropyle et cyclobutyle,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl (C₁-C₄) amino, dialkyl (C₁-C₄) amino, cycloalkyle en C₃-C₅ et hétérocyclyle à 4 ou 5 chaînons ainsi que jusqu'à trois fois par le fluor,
n représente le nombre 0 ou 1
R³ représente le groupe méthyle,
R⁴ représente un substituant choisi dans la série chloro, pentafluorothio, alkyle en C₁-C₆, triméthylsilyle, -OR⁷, -SR⁷, -S(=O)-R⁷, -S(=O)₂-R⁷, -S(=O)(=NCH₃)-CF₃, cycloalkyle en C₃-C₆ et hétérocyclyle à 4 à 6 chaînons, le groupe alkyle en C₁-C₆ pouvant pour sa part être substitué par un radical choisi dans la série -OR⁷, -NR⁷R⁸, -C(=O)-NR⁷R⁸, cycloalkyle en C₃-C₆ et hétérocyclyle à 4 à 6 chaînons, ainsi que jusqu'à trois fois par le fluor
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, trifluorométhyle, alcoxy en C₁-C₄, trifluorométhoxy et oxo, ledit substituant alkyle en C₁-C₄ pouvant pour sa part être substitué par méthoxy, trifluorométhoxy ou éthoxy, et où
R⁷ et R⁸ représentent indépendamment l'un de l'autre dans chaque occurrence individuelle un atome d'hydrogène, un groupe alkyle en C₁-C₄ ou cycloalkyle en C₃-C₆,le groupe alkyle en C₁-C₄ pouvant être substitué par un radical choisi dans la série hydroxy, alcoxy en C₁-C₄, trifluorométhoxy et cycloalkyle en C₃-C₆ ainsi que jusqu'à trois fois par le fluor
et
lesdits groupes cycloalkyle pouvant être substitués jusqu'à deux fois par un radical, qui peut être le même ou différent, choisi dans la série fluoro, alkyle en C₁-C₄, trifluorométhyle, hydroxy, alcoxy en C₁-C₄ et trifluorométhoxy, ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un atome de fluor et
p représente le nombre 0 ou 1,
ainsi que ses sels, produits de solvatation et produits de solvatation des sels.

10. Composé de formule (I) selon la revendication 1, 2, 4 ou 7, dans lequel
le cycle 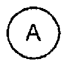 représente un cycle phényle et les groupes adjacents X et CH₂ sont liés à ce cycle phényle en relation 1,3 ou 1,4 l'un par rapport à l'autre,
le cycle 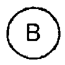 avec le substituant R³ représente un cycle hétéroaryle de formule 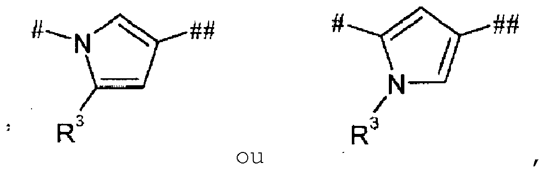 formules dans lesquelles
# désigne le point de liaison au groupe CH₂ contigu
et
## désigne le point de liaison au cycle 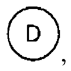
le cycle 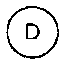 représente un cycle hétéroaryle de formule 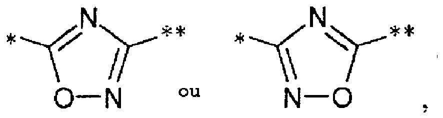 où
* désigne le point de liaison au cycle 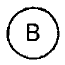
et
** désigne le point de liaison au cycle 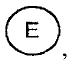
le cycle 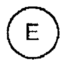 avec les substituants R⁴ et R⁵ représente un cycle phényle de formule 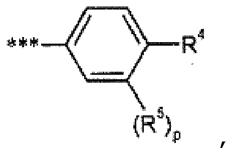 dans laquelle
*** désigne le point de liaison au cycle 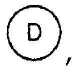
le cycle 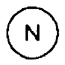 représente un aza-hétérocycle saturé à 4 à 10 chaînons, qui contient au moins un atome d'azote en tant que chaînon formant le cycle et peut en outre contenir un autre hétérochaînon dans
le cycle, choisi dans la série N, 0, S ou S(O)₂,
X représente une liaison ou représente ◆- (CH₂)_{q}N(R⁶)-◆◆, -C(=O)- ou ◆-N(R⁶)-C(=O)-◆◆, où
◆ désigne le point de liaison au cycle 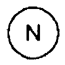
et
◆◆ désigne le point de liaison au cycle 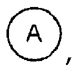
q représente le nombre 0 ou 1 et R⁶ représente un atome d'hydrogène, le groupe méthyle, éthyle, isopropyle, cyclopropyle ou cyclobutyle, R¹ représente un substituant lié à un atome de carbone du cycle 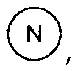 choisi dans la série fluoro, cyano, alkyle en C₁-C₄, hydroxy, alcoxy en C₁-C₄, oxo, amino, monoalkyl(C₁-C₄)amino, dialkyl(C₁-C₄)-amino, cyclopropyle et cyclobutyle, le groupe alkyle en C₁-C₄ pouvant pour sa part être substitué par un radical choisi dans la série hydroxy, alcoxy en C₁-C₄, amino, monoalkyl(C₁-C₄)-amino et dialkyl(C₁-C₄)amino ainsi que jusqu'à trois fois par le fluor
ainsi que ses sels, produits de solvatation et produits de solvatation des sels.
m représente le nombre 0 ou 1,
R² représente un substituant lié à un atome d'azote du cycle 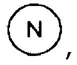 choisi dans la série alkyle en C₁-C₄, alkyl (C₁-C₄) carbonyle, alcoxy (C₁-C₄)-carbonyle, alkyl(C₁-C₄)sulfonyle, cyclopropyle et cyclobutyle,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl (C₁-C₄) amino, dialkyl (C₁-C₄) amino, cycloalkyle en C₃-C₅ et hétérocyclyle à 4 ou 5 chaînons ainsi que jusqu'à trois fois par le fluor,
n représente le nombre 0 ou 1
R³ représente le groupe méthyle,
R⁴ représente un substituant choisi dans la série chloro, pentafluorothio, alkyle en C₁-C₆, triméthylsilyle, -OR⁷, -SR⁷, -S(=O)-R⁷, -S(=O)₂-R⁷, -S(=O)(=NCH₃)-CF₃, cycloalkyle en C₃-C₆ et hétérocyclyle à 4 à 6 chaînons, le groupe alkyle en C₁-C₆ pouvant pour sa part être substitué par un radical choisi dans la série -OR⁷, -NR⁷R⁸, -C(=O)-NR⁷R⁸, cycloalkyle en C₃-C₆ et hétérocyclyle à 4 à 6 chaînons, ainsi que jusqu'à trois fois par le fluor
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, trifluorométhyle, alcoxy en C₁-C₄, trifluorométhoxy et oxo, ledit substituant alkyle en C₁-C₄ pouvant pour sa part être substitué par méthoxy, trifluorométhoxy ou éthoxy, et où
R⁷ et R⁸ représentent indépendamment l'un de l'autre dans chaque occurrence individuelle un atome d'hydrogène, un groupe alkyle en C₁-C₄ ou cycloalkyle en C₃-C₆, le groupe alkyle en C₁-C₄ pouvant être substitué par un radical choisi dans la série hydroxy, alcoxy en C₁-C₄, trifluorométhoxy et cycloalkyle en C₃-C₆ ainsi que jusqu'à trois fois par le fluor
et
lesdits groupes cycloalkyle pouvant être substitués jusqu'à deux fois par un radical, qui peut être le même ou différent, choisi dans la série fluoro, alkyle en C₁-C₄, trifluorométhyle, hydroxy, alcoxy en C₁-C₄ et trifluorométhoxy, ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, 0, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un atome de fluor et
p représente le nombre 0 ou 1,

11. Composé de formule (I) selon la revendication 1, 2, 5 ou 8, dans lequel
le cycle 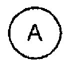 représente un cycle phényle et les groupes adjacents X et CH₂ sont liés à ce cycle phényle en relation 1,3 ou 1,4 l'un par rapport à l'autre,
le cycle 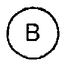 avec le substituant R³ représente un cycle hétéroaryle de formule 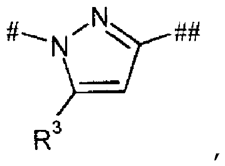 dans laquelle
# désigne le point de liaison au groupe CH₂
contigu et
## désigne le point de liaison au cycle 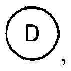
le cycle 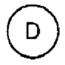 représente un cycle hétéroaryle de formule 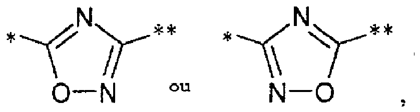 où
* désigne le point de liaison au cycle 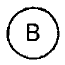
et
** désigne le point de liaison au cycle 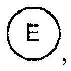
le cycle 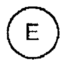 avec les substituants R⁴ et R⁵ représente un cycle phényle de formule 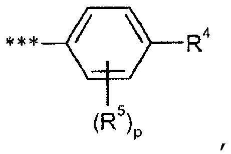 dans laquelle
*** désigne le point de liaison au cycle 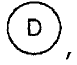 le cycle 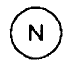 représente un aza-hétérocycle saturé à 4 à 10 chaînons, qui contient au moins un atome d'azote en tant que chaînon formant le cycle et peut en outre contenir un autre hétérochaînon dans le cycle, choisi dans la série N, O, S ou S(O)₂,
X représente une liaison ou représente ◆- (CH₂)_{q}N(R⁶)-◆◆, -C(=O)- ou ◆-N(R⁶)-C(=O)-◆◆, où ◆ désigne le point de liaison au cycle 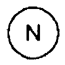 et
◆◆ désigne le point de liaison au cycle 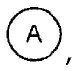
q représente le nombre 0 ou 1 et
R⁶ représente un atome d'hydrogène, le groupe méthyle, éthyle, isopropyle, cyclopropyle ou cyclobutyle,
R¹ représente un substituant lié à un atome de carbone du cycle 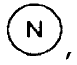 choisi dans la série cyano, alkyle en C₁-C₄, OXO, cyclopropyle et cyclobutyle, le groupe alkyle en C₁-C₄ pouvant pour sa part être substitué par un radical choisi dans la série hydroxy, alcoxy en C₁-C₄, amino, monoalkyl(C₁-C₄)-amino et dialkyl(C₁-C₄)amino ainsi que jusqu'à trois fois par le fluor
R² représente un substituant lié à un atome d'azote du cycle 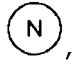 choisi dans la série alkyle en C₁-C₄, alkyl (C₁-C₄) carbonyle, alcoxy (C₁-C₄)-carbonyle, alkyl(C₁₋C₄)sulfonyle, cyclopropyle et cyclobutyle,
le groupe alkyle dans les radicaux alkyle en C₁-C₄, alkyl(C₁-C₄)carbonyle, alcoxy(C₁-C₄)carbonyle et alkyl(C₁-C₄)sulfonyle pouvant pour sa part être substitué par un radical choisi dans la série hydroxy, alcoxy en C₁-C₄, amino, monoalkyl(C₁-C₄)amino, dialkyl (C₁-C₄) amino, cycloalkyle en C₃-C₅ et hétérocyclyle à 4 ou 5 chaînons ainsi que jusqu'à trois fois par le fluor,
m représente le nombre 0 ou 1,
n représente le nombre 0 ou 1,
la somme de m et n étant égale au nombre 1 ou 2,
R³ représente le groupe méthyle,
R⁴ représente un substituant choisi dans la série chloro, pentafluorothio, alkyle en C₁-C₆, triméthylsilyle, -OR⁷, -SR⁷, -S(=O) -R⁷, -S(=O)₂-R⁷, -S(=O)(=NCH₃)-CF₃, cycloalkyle en C₃-C₆ et hétérocyclyle à 4 à 6 chaînons,
le groupe alkyle en C₁-C₆ pouvant pour sa part être substitué par un radical choisi dans la série -OR⁷, -NR⁷R⁸, -C(=O)-NR⁷R⁸, cycloalkyle en C₃-C₆ et hétérocyclyle à 4 à 6 chaînons, ainsi que jusqu'à trois fois par le fluor
et
lesdits groupes cycloalkyle et hétérocyclyle pouvant pour leur part être substitués jusqu'à deux fois par un radical, qui peut être le même ou différent, choisi dans la série fluoro, alkyle en C₁-C₄, trifluorométhyle, alcoxy en C₁-C₄, trifluorométhoxy et oxo, ledit substituant alkyle en C₁-C₄ pouvant pour sa part être substitué par méthoxy, trifluorométhoxy ou éthoxy,
et où
R⁷ et R⁸ représentent indépendamment l'un de l'autre dans chaque occurrence individuelle un atome d'hydrogène, un groupe alkyle en C₁-C₄ ou cycloalkyle en C₃-C₆, le groupe alkyle en C₁-C₄ pouvant être substitué par un radical choisi dans la série hydroxy, alcoxy en C₁-C₄, trifluorométhoxy et cycloalkyle en C₃-C₆ ainsi que jusqu'à trois fois par le fluor
et
lesdits groupes cycloalkyle pouvant être substitués jusqu'à deux fois par un radical, qui peut être le même ou différent, choisi dans la série fluoro, alkyle en C₁-C₄, trifluorométhyle, hydroxy, alcoxy en C₁-C₄ et trifluorométhoxy, ou
R⁷ et R⁸, dans le cas où ils sont tous les deux liés à un atome d'azote, forment ensemble avec cet atome d'azote un hétérocycle à 4 à 6 chaînons, qui peut contenir un autre hétéroatome dans le cycle, choisi dans la série N, O, S ou S(O)₂, et qui peut être substitué jusqu'à deux fois par un radical, qui peut être le même ou différent, choisi dans la série fluoro, alkyle en C₁-C₄, trifluorométhyle, hydroxy, alcoxy en C₁-C₄, oxo et alkyl(C₁-C₄)carbonyle,
R⁵ représente un atome de fluor et
p représente le nombre 0 ou 1,
ainsi que ses sels, produits de solvatation et produits de solvatation des sels.

12. Composé, tel que défini dans l'une quelconque des revendications 1 à 11, destiné au traitement et/ou à la prévention de maladies.

13. Composé, tel que défini dans l'une quelconque des revendications 1 à 11, destiné à l'utilisation dans un procédé pour le traitement et/ou la prévention de maladies cancéreuses ou tumorales.

14. Composé, tel que défini dans l'une quelconque des revendications 1 à 11, destiné à l'utilisation dans un procédé pour le traitement et/ou la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatoïde et de la polycythémie tchouvache.

15. Utilisation d'un composé, tel que défini dans l'une quelconque des revendications 1 à 11, pour la fabrication d'un médicament destiné au traitement et/ou à la prévention de maladies cancéreuses ou tumorales.

16. Utilisation d'un composé, tel que défini dans l'une quelconque des revendications 1 à 11, pour la fabrication d'un médicament destiné au traitement et/ou à la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatoïde et de la polycythémie tchouvache.

17. Médicament contenant un composé, tel que défini dans l'une quelconque des revendications 1 à 11, en association avec un ou plusieurs adjuvants inertes, non toxiques, pharmaceutiquement convenables.

18. Médicament contenant un composé, tel que défini dans l'une quelconque des revendications 1 à 11, en association avec une ou plusieurs autres substances actives.

19. Médicament selon la revendication 17 ou 18, destiné au traitement et/ou la prévention de maladies cancéreuses ou tumorales.

20. Médicament selon la revendication 17 ou 18, destiné au traitement et/ou la prévention de maladies cardiovasculaires ischémiques, de l'insuffisance cardiaque, de l'infarctus du myocarde, de l'arythmie, de l'accident vasculaire cérébral, de l'hypertonie pulmonaire, de maladies fibreuses du rein et du poumon, du psoriasis, de la rétinopathie diabétique, de la dégénérescence maculaire, de la polyarthrite rhumatoïde et de la polycythémie tchouvache.

21. Procédé pour la préparation de composés de formule (I-D) 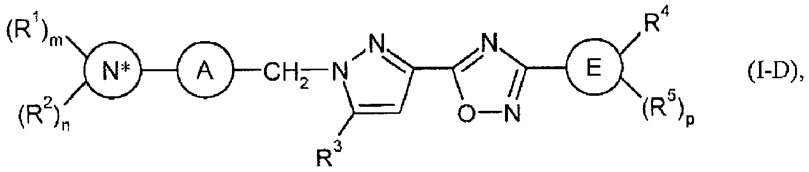 dans laquelle les cycles A et E ainsi que R¹, R², R³, R⁴, R⁵, m, n et p ont chacun les significations indiquées dans les revendications 1 à 11,
et
le cycle N* représente un cycle N lié au cycle A par un atome d' azote formant le cycle, qui est tel que défini dans les revendications 1 à 11,
**caractérisé en ce qu'**on soumet une N'-hydroxyamidine de formule (IX) 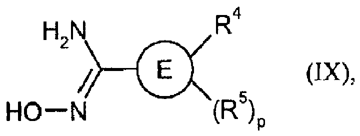 dans laquelle le cycle E ainsi que R⁴, R⁵ et p ont les significations indiquées plus haut,
d'abord soit
[A] à une condensation avec un acide pyrazole-carboxylique (XXVII) 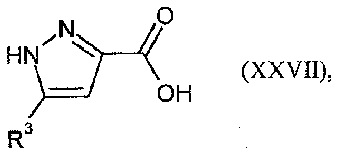 dans laquelle R³ a la signification indiquée plus haut,
conduisant à un dérivé 1,2,4-oxadiazole de formule (XXVIII), 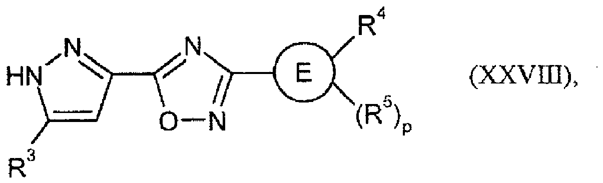 dans laquelle le cycle E ainsi que R³, R⁴, R⁵ et p ont les significations indiquées plus haut,
et ensuite on soumet ce dernier à une alkylation, en présence d'une base, avec un composé de formule (III) 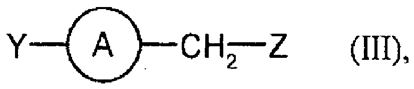 dans laquelle le cycle A a la signification indiquée plus haut,
Y représente un atome de chlore, de brome ou d'iode,
et
Z représente un atome de chlore, de brome, d'iode, le mésylate, le triflate ou le tosylate,
pour aboutir à un composé de formule (XXIX) 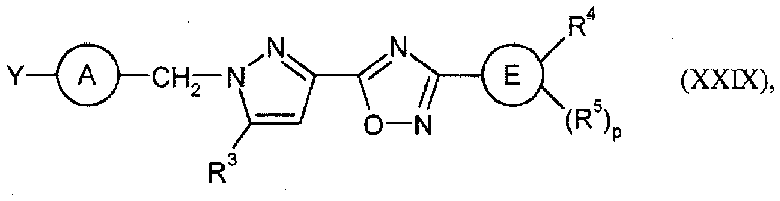 dans laquelle les cycles A et E ainsi que R³, R⁴, R⁵, p et Y ont les significations indiquées plus haut,
soit
[B] à une condensation avec un acide pyrazole-carboxylique de formule (XXX) 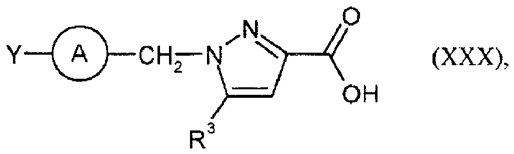 dans laquelle le cycle A ainsi que R³ ont les significations indiquées plus haut
et
Y représente un atome de chlore, de brome ou d'iode,
conduisant au composé de formule (XXIX) 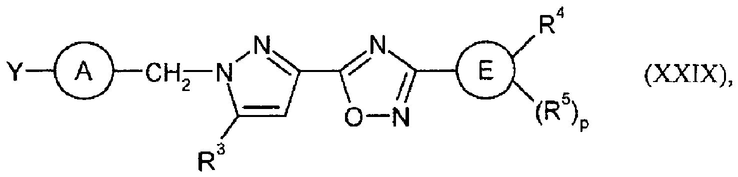 dans laquelle les cycles A et E ainsi que R³, R⁴, R⁵, p et Y ont les significations indiquées plus haut,
et ensuite on fait réagir le composé de formule (XXIX) ainsi obtenu, éventuellement en présence d'un catalyseur au palladium et/ou d'une base, avec un composé de formule (XXXI) 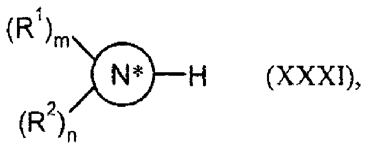 dans laquelle le cycle N* ainsi que R¹, R², m et n ont les significations données plus haut et l'atome d'hydrogène indiqué est lié à un atome d'azote du cycle N^{*}.
